# EUROPEAN PATENT SPECIFICATION

(11) **EP 1 435 957 B1**
(45) Date of publication and mention of the grant of the patent: **17.06.2009**
(21) Application number: 02729056.8
(22) Date of filing: 29.04.2002
(51) Int. Cl.: A61K 31/495, A61K 31/50, A61P 25/00, C07D 487/04

(54) **INHIBITORS OF GSK-3 AND CRYSTAL STRUCTURES OF GSK-3BETA PROTEIN AND PROTEIN COMPLEXES**
INHIBITOREN VON GSK-3 UND KRISTALLSTRUKTUREN VON GSK-3BETA-PROTEIN UND -PROTEINKOMPLEXEN
INHIBITEURS DE GSK-3 ET STRUCTURES CRISTALLINES DE LA PROTEINE GSK-3BETA ET DE COMPLEXES PROTEIQUES

(30) Priority: 30.04.2001 US 287366 P; 08.06.2001 US 297094 P; 27.02.2002 US 361899 P
(43) Date of publication of application: 14.07.2004
(60) Divisional application: 09004787.9
(73) Proprietor: VERTEX PHARMACEUTICALS INCORPORATED, Cambridge, MA 02139-4242 (US)
(72) Inventor: TER HAAR, Ernst, Roslindale, MA 02131 (US); SWENSON, Lovorka, Belmont, MA 02478 (US); GREEN, Jeremy, Burlington, MA 01803 (US); ARNOST, Michael, J., North Andover, MA 01845 (US)
(74) Representative: Vossius & Partner
(86) International application number: PCT/US2002/013511
(87) International publication number: WO 2002/088078

(56) References cited:
- WO-A-02/24893
- WO-A-02/50254
- WO-A-99/21859
- WO-A-03/068932
- WO-A-03/080616
- WO-A1-02/24694
- AOKI M ET AL: "EXPRESSION, PURIFICATION AND CRYSTALIZATION OF HUMAN TAU-PROTEIN KINASE I/GLYCOGEN SYNTHASE KINASE -3BETA" ACTA CRYSTALLOGRAPHICA SECTION D: BIOLOGICAL CRYSTALLOGRAPHY, MUNKSGAARD PUBLISHERS LTD. COPENHAGEN, DK, vol. 56, no. 11, November 2000 (2000-11), pages 1464-1465, XP001097943 ISSN: 0907-4449
- TER HAAR E ET AL: "Structure of GSK3beta reveals a primed phosphorylation mechanism." NATURE STRUCTURAL BIOLOGY. JUL 2001, vol. 8, no. 7, July 2001 (2001-07), pages 593-596, XP008042801 ISSN: 1072-8368
- DAJANI RANA ET AL: "Crystal structure of glycogen synthase kinase 3beta: Structural basis for phosphate-primed substrate specificity and autoinhibition" CELL, CELL PRESS, CAMBRIDGE, NA, US, vol. 105, no. 6, 15 June 2001 (2001-06-15), pages 721-732, XP002193600 ISSN: 0092-8674
- FIOL C J ET AL: "Ordered multisite protein phosphorylation" JOURNAL OF BIOLOGICAL CHEMISTRY, AMERICAN SOCIETY OF BIOLOGICAL CHEMISTS, BALTIMORE, MD, US, vol. 265, no. 11, 15 April 1991 (1991-04-15), pages 6061-6065, XP002090546 ISSN: 0021-9258

## Description

### TECHNICAL FIELD OF INVENTION

The present invention relates to inhibitors of glycogen synthase kinase-3 (GSK-3), a serine/threonine protein kinase, and to methods for producing them. The invention also provides pharmaceutical compositions comprising the inhibitors of the invention. Those compositions are suitable for the treatment and prevention of various disease states, such as diabetes and Alzheimer's disease.

### BACKGROUND OF THE INVENTION

Protein kinases mediate intracellular signal transduction by affecting a phosphoryl transfer from a nucleoside triphosphate to a protein acceptor involved in a signaling pathway. There are a number of kinases and pathways through which extracellular and other stimuli cause a variety of cellular responses to occur inside the cell. Examples of such stimuli include environmental and chemical stress signals (e.g., osmotic shock, heat shock, ultraviolet radiation, bacterial endotoxin, H₂O₂), cytokines (e.g., interleukin-1 (IL-1) and tumor necrosis factor α (TNF-α)), growth factors (e.g., granulocyte macrophage-colony-stimulating factor (GM-CSF), and fibroblast growth factor (FGF). An extracellular stimulus may affect one or more cellular responses related to cell growth, migration, differentiation, secretion of hormones, activation of transcription factors, muscle contraction, glucose metabolism, control of protein synthesis and regulation of cell cycle. Many disease states are associated with abnormal cellular responses triggered by protein kinase-mediated events. These diseases include autoimmune diseases, inflammatory diseases, neurological and neurodegenerative diseases, cancer, cardiovascular diseases, allergies and asthma, Alzheimer's disease and hormone-related diseases.

GSK-3 is a serine/threonine protein kinase and belongs to the superfamily of mitogen-activated protein kinases. MAP kinases are activated by phosphorylation of threonine and/or tyrosine residues in a loop adjacent to the active site. Phosphorylation of MAP kinases is carried out by specific kinases upstream. Activated MAP kinase then phosphorylates the various substrates.

Mammalian cells have α and β isoforms of GSK-3 that are each encoded by distinct genes (Coghlan et al., Chemistry & Biology, 7, pp. 793-803 (2000); Kim and Kimmel, Curr. Opinion Genetics Dev., 10, pp. 508-514 (2000)). The core kinase sequences have 97% similarity, but the protein sequences deviate substantially outside the kinase core (Woodgett, J.R., EMBO J., 9, pp. 2431-8 (1990)). GSK-3α is 63 residues longer at the N-terminal end than GSK-3β, however the N-terminal phosphorylation site in both isoforms (S21 for GSK-3α and S9 for GSK-3β) is embedded in a conserved 7 residue motif. The two isoforms are not redundant as GSK-3β deficiency is lethal in embryogenesis due to severe liver degeneration (Hoeflich, K.P., et al., Nature, 406, pp. 86-90 (2000)).

GSK-3β has multiple phosphorylation sites. The Serine 9 and Tyrosine 216 phosphorylation sites are well described in the literature. Phosphorylation of Tyrosine 216 activates GSK-3β but phosphorylation of Serine 9 inactivates it. GSK-3β is unique among kinases in that it requires prior phosphorylation of its substrates. GSK-3β does not phosphorylate its multiple substrates in the same manner and with the same efficiency but has different modes of phosphorylation. The canonical phosphorylation sequence recognized by GSK-3β, SXXXS, contains two serines separated by three amino acid residues. Multiple copies of this motif can be present in the substrate. Several protein substrates such as glycogen synthase, eIF2b and APC, are first phosphorylated by a different kinase at the P+4 serine in the ₚ₊₄SXXXSₚ motif before GSK-3β phosphorylates the serine in the P position. This is called primed phosphorylation, and is approximately 100 to 1000 times faster than the phosphorylation without priming (Thomas, G.M., et al., FEBS Lett., 458, pp. 247-51 (1999)). Glycogen synthase has multiple serines separated by four residues (residue 640, 644, 648, and 652) and those serines are phosphorylated sequentially by GSK-3β from the C-terminal end, after S656 has been phosphorylated by Casein Kinase II (Woodgett, J.R. and P. Cohen, Biochim. Biophys. Acta, 788, pp. 339-47 (1984); Kuret, J. et al, Eur. J. Biochem., 151, pp. 39-48 (1985)).

Glycogen synthase kinase-3 has been implicated in various diseases including diabetes, Alzheimer's disease, CNS disorders such as manic depressive disorder and neurodegenerative diseases, and cardiomyocete hypertrophy (WO 99/65897; WO 00/38675; and Haq et al., J. Cell Biol., 151, pp. 117 (2000)). These diseases may be caused by, or result in, the abnormal operation of certain cell signaling pathways in which GSK-3 plays a role. GSK-3 phosphorylates and modulates the activity of a number of regulatory proteins. These include glycogen synthase which is the rate limiting enzyme necessary for glycogen synthesis, the microtubule associated protein Tau, the gene transcription factor beta-catenin, the translation initiation factor elF2B, as well as ATP citrate lyase, axin, heat shock factor-1, c-Jun, c-Myc, c-Myb, CREB and CEPBa. These diverse targets implicate GSK-3 in many aspects of cellular metabolism, proliferation, differentiation and development.

In a GSK-3 mediated pathway that is relevant for the treatment of type II diabetes, insulin-induced signaling leads to cellular glucose uptake and glycogen synthesis. Along this pathway, GSK-3 is a negative regulator of the insulin-induced signal. Insulin inactivates GSK-3β via the PKB/Akt pathway, which results in activation of glycogen synthase. (Summers, S.A., et al., J. Biol. Chem., 274, pp. 17934-40 (1999); Ross, S.E., et al., Mol. Cell. Biol., 19, pp. 8433-41 (1999)). The inhibition of GSK-3 leads to increased glycogen synthesis and glucose uptake (Klein et al., PNAS, 93, pp-8455-9 (1996); Cross et al., Biochem. J., 303, pp. 21-26 (1994); Cohen, Biochem. Soc. Trans., 21, pp. 555-567(1993); Massillon et al., Biochem. J. 299, pp. 123-128 (1994)). However, in a diabetic patient where the insulin response is impaired, glycogen synthesis and glucose uptake fail to increase despite the presence of relatively high blood levels of insulin. This leads to abnormally high blood levels of glucose with acute and long term effects that may ultimately result in cardiovascular diseases, renal failure and blindness. In such patients, the normal insulin-induced inhibition of GSK-3 fails to occur. It has also been reported that in patients with type II diabetes, GSK-3 is overexpressed (WO 00/38675). Therapeutic inhibitors of GSK-3 are therefore potentially useful for treating diabetic patients suffering from an impaired response to insulin.

GSK-3 activity has also been associated with Alzheimer's disease. This disease is characterized by the well-known β-amyloid peptide and the formation of intracellular neurofibrillary tangles. The neurofibrillary tangles contain hyperphosphorylated Tau protein where Tau is phosphorylated on abnormal sites. GSK-3 has been shown to phosphorylate these abnormal sites in cell and animal models. Furthermore, inhibition of GSK-3 has been shown to prevent hyperphosphorylation of Tau in cells (Lovestone et al., Current Biology, 4, pp. 1077-86 (1994); Brownlees et al., Neuroreport, 8, pp. 3251-55 (1997)). Therefore, it is believed that GSK-3 activity may promote generation of the neurofibrillary tangles and the progression of Alzheimer's disease.

Another substrate of GSK-3 is beta-catenin which is degraded after phosphorylation by GSK-3. Reduced levels of beta-catenin have been reported in schizophrenic patients and have also been associated with other diseases related to increase in neuronal cell death (Zhong et al., Nature, 395, 698-702 (1998); Takashima et al., PNAS, 90, 7789-93 (1993); Pei et al., J. Neuropathol. Exp., 56, 70-78 (1997)).

GSK-3β is also a component of the Wnt signalling pathway. Activation of the Wnt pathway inhibits GSK-3β, which results in accumulation of cytosolic β-catenin (Yost, C., et al., Cell, 93, pp. 1031-41 (1998)). The cytosolic β-catenin translocates to the cell nucleus, where it associates with LEF/tcf and stimulates the expression of Wnt target genes resulting in cell proliferation (Ding, V.W., et al., J. Biol. Chem., 275, pp. 32475-81 (2000); Waltzer, L. and M. Bienz, Cancer Metastasis Rev. 18, pp. 231-46 (1999); Ikeda, S., et al., EMBO J., 17, pp. 1371-84 (1998); Thomas, G.M., et al., FEBS Lett, 458, pp. 247-51 (1999); Salic, A., et al., Mol. Cell., 5, pp. 523-32 (2000)). The activity of GSK-3β is also down regulated by 7-TM receptors that regulate cAMP levels. cAMP-dependent protein kinase A, binds, phosphorylates and inhibits GSK-3β in response to the adenyl cyclase activator forskolin, or the p-adrenergic receptor activator isoproterenol (Fang, X., et al., Proc. Natl. Acad. Sci. U S A, 97, pp. 11960-5 (2000)).

Small molecule inhibitors of GSK-3 have recently been reported (WO 99/65897 and WO 00/38675). For many of the aforementioned diseases associated with abnormal GSK-3 activity, other protein kinases have also been targeted for treating the same diseases. However, the various protein kinases often act through different biological pathways. Quinazoline derivatives have been reported recently as inhibitors of p38 kinase (WO 00/12497). The compounds are reported to be useful for treating conditions characterized by enhanced p38-α activity and/or enhanced TGF-β activity. While p38 activity has been implicated in a wide variety of diseases, including diabetes, p38 kinase is not reported to be a constituent of an insulin signaling pathway that regulates glycogen synthesis or glucose uptake. Therefore, unlike GSK-3, p38 inhibition would not be expected to enhance glycogen synthesis and/or glucose uptake.

Accordingly, there has been an interest in finding GSK-3 inhibitors that are effective as therapeutic agents due to its important role in diabetes, Alzheimer's disease and other diseases. A challenge has been to find protein kinase inhibitors that act in a selective manner. Since there are numerous protein kinases that are involved in a variety of cellular responses, non-selective inhibitors may lead to unwanted side effects.

In this regard, the three-dimensional structure of the kinase would assist in the rational design of inhibitors. Further, information provided by the X-ray crystal structure of GSK-3β-inhibitor complexes would be extremely useful in iterative drug design of various GSK-3 proteins. The determination of the amino acid residues in GSK-3β binding pockets and the determination of the shape of those binding pockets would allow one to design inhibitors that bind more favorably to this class of enzymes.

### SUMMARY OF THE INVENTION

The present invention addresses this need by providing compounds and pharmaceutical compositions thereof that are effective as protein kinase inhibitors, particularly as inhibitors of GSK-3.

The compounds of the present invention have the general formula I: 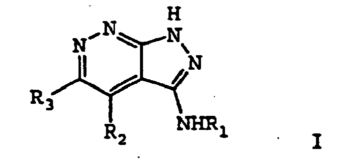 or a pharmaceutically acceptable derivative thereof, wherein:
R₁ is selected from H, aliphatic, RC(O)-, RS(O)ₙ-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heteroaralkyl is optionally substituted;
R₂ and R₃ are each independently selected from H, aliphatic, carbocyclyl, heterocyclyl, aryl, aralkyl, heteroaryl, heteroaralkyl, -N(R)₂, -NRCOR,-NRCO₂R, -NRCO₂R, -S(O)ₙR, -SO₂N(R)₂, -SR, -OR, -CF₃, halo, -NO₂, -CN, -C(O)R, -CO₂R, -OC(O)R, -CON(R)₂, or - OC(O)N(R)₂, wherein said aliphatic, carbocyclyl, heterocyclyl, aryl, aralkyl, heteroaryl, or heteroaralkyl is optionally substituted; or R₂ and R₃ taken together with the intervening atoms optionally form a five- to nine-membered ring that is fused to the pyridazinyl ring of formula I, said fused ring having 0-2 heteroatoms;
each R is independently selected from H, aliphatic, carbocyclyl, heterocyclyl, aryl, aralkyl, heteroaryl, or heteroaralkyl, wherein each member of R except H is optionally substituted; and
n is 1 or 2;
provided that when R₁ is H, R₂ and R₃ are not both unsubstituted phenyl; and when R₁ is H, R₂ and R₃ are other than H, halogen, or an unsubstituted alkyl.

In another embodiment, the invention provides pharmaceutical compositions comprising a GSK-3 inhibitor of this invention. These compositions may be utilized for treating or preventing a variety of GSK-3 mediated disorders, such as autoimmune diseases, inflammatory diseases, metabolic, neurological and neurodegenerative diseases, cardiovascular diseases, allergy, asthma, diabetes, Alzheimer's disease, Huntington's Disease, Parkinson's Disease, AIDS-associated dementia, amyotrophic lateral sclerosis (AML, Lou Gehrig's Disease), multiple sclerosis (MS), schizophrenia, cardiomyocyte hypertrophy, reperfusion/ischemia, and baldness.

The compositions of this invention are also useful for enhancing glycogen synthesis and/or lowering blood levels of glucose and therefore are especially useful for diabetic patients. These compositions are also useful for inhibiting the production of hyperphosphorylated Tau protein, which is useful in halting or slowing the progression of Alzheimer's disease. Another embodiment of this invention relates to the use for inhibiting the phosphorylation of β-catenin, which is useful for treating schizophrenia.

In another embodiment, the invention provides pharmaceutical compositions comprising these compounds.

### BRIEF DESCRIPTION OF THE FIGURES

Figure 1 lists the atomic structure coordinates for unphosphorylated GSK-3β as derived by X-ray diffraction from the crystal.

Figure 2 lists the atomic structure coordinates for phosphorylated GSK-3β.

Figure 3 lists the atomic structure coordinates for phosphorylated GSK-3β-inhibitor1 complex (inhibitor1 is 4,5-Diphenyl-1H-pyrazolo[3,4-c]pyridazin-3-ylamine).

Figure 4 lists the atomic structure coordinates for unphosphorylated GSK-3β-inhibitor2 complex (inhibitor2 is (5-Methyl-2H-pyrazol-3-yl)-(2-pyridin-4-yl-quinazolin-4-yl)-amine)).

Figure 5 lists the atomic structure coordinates for unphosphorylated GSK-3β-inhibitor3 complex (inhibitor 3 is 4-(5-Methyl-2-phenylamino-pyrimidin-4-yl)-1H-pyrrole-2-carboxylic acid (2-hydroxy-1-phenyl-ethyl)-amide) .

Figure 6 lists the atomic structure coordinates for unphosphorylated GSK-3β-inhibitor4 complex (inhibitor 4 is(1H-Indazol-3-yl)-[2-(2-trifluoromethyl-phenyl)-quinazolin-4-yl]-amine).

Figure 7 lists the atomic structure coordinates for phosphorylated GSK-3β in complex with ADP and glycogen synthase peptide.

Figure 8 depicts a ribbon diagram of the overall fold of unphosphorylated GSK-3β. The N-terminal domain corresponds to the β-strand domain and encompasses residues 25 to 138. β-strand 1 was only visible in one of the two molecules in the asymmetric unit and makes hydrogen bonds with β-strand 2 although it is not part of the β-barrel. The α-helical domain corresponds to residues 139 to 349. Key features of the kinase-fold such as the hinge, glycine rich loop and activation-loop are indicated.

Figure 9 depicts a superposition of unphosphorylated GSK-3β (light shade) and activated substrate-bound CDK2 (Protein Data Bank accession number 1QMZ) (dark shade). The α-helical domains of GSK-3β and CDK2 were superimposed in QUANTA by aligning matching residues.

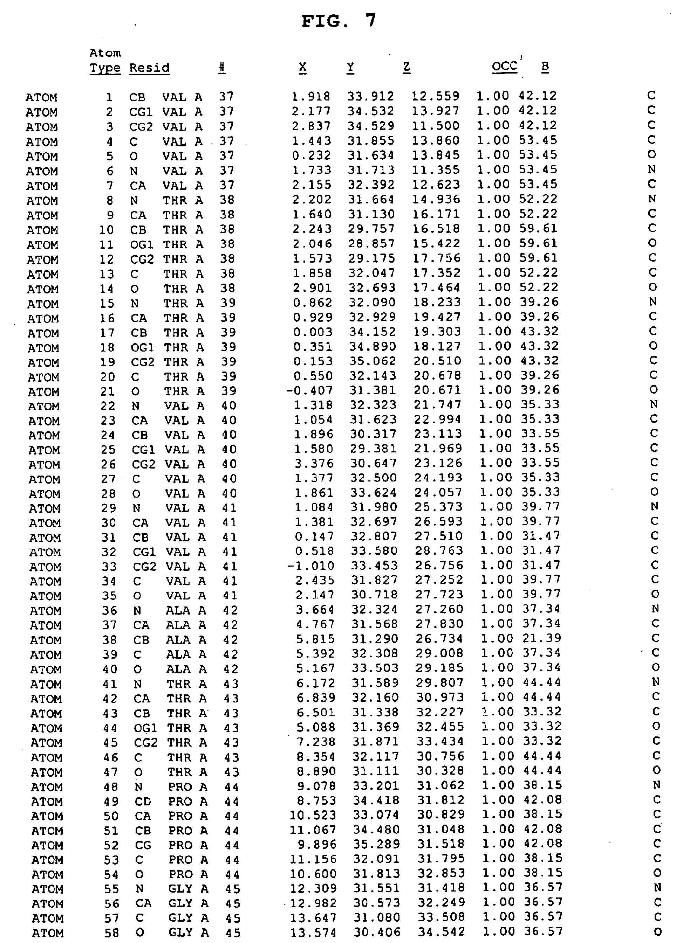

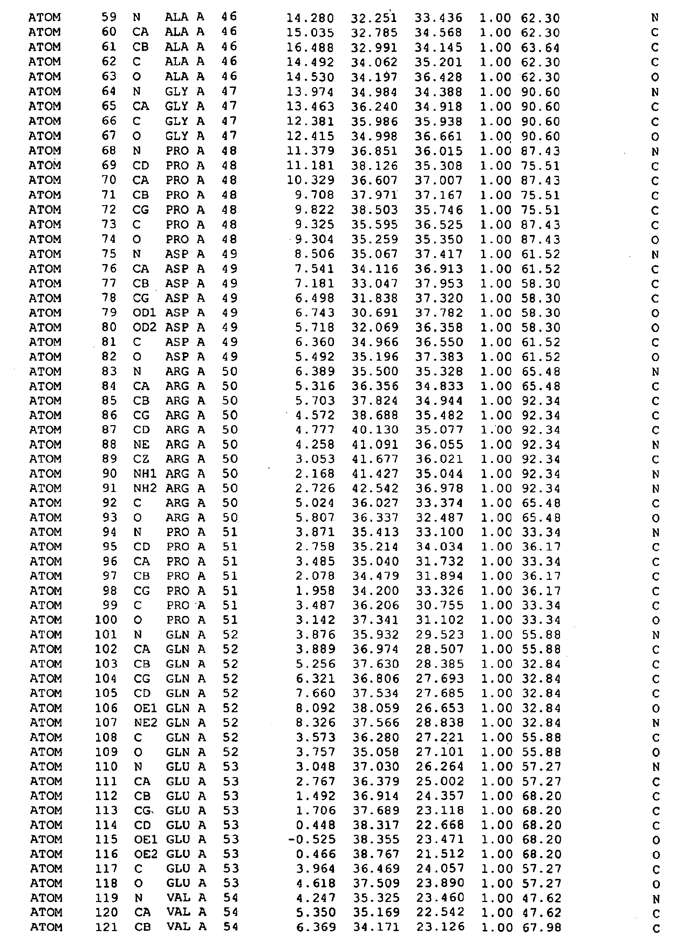

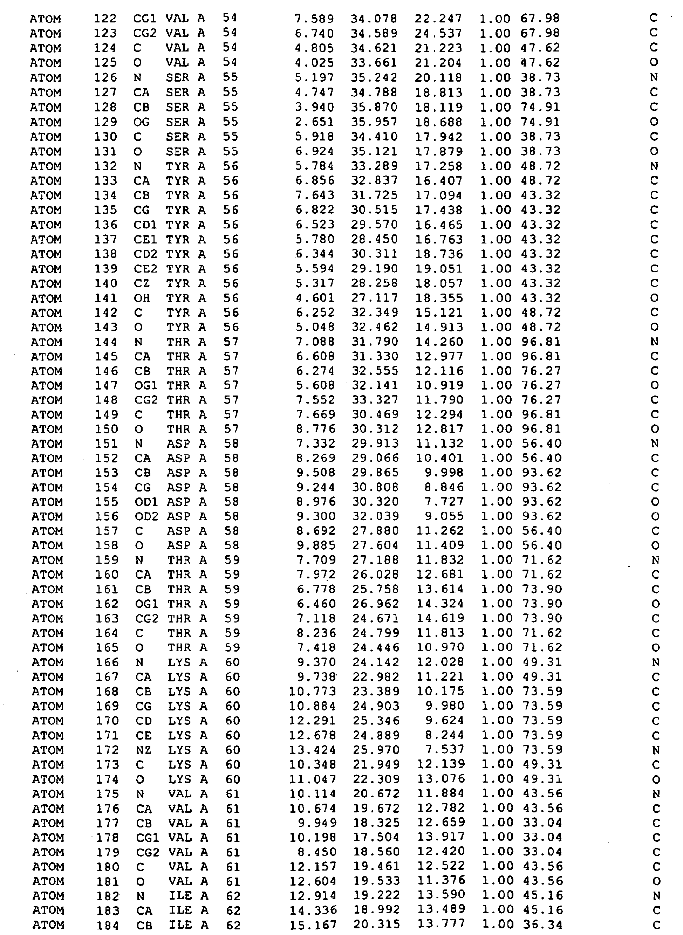

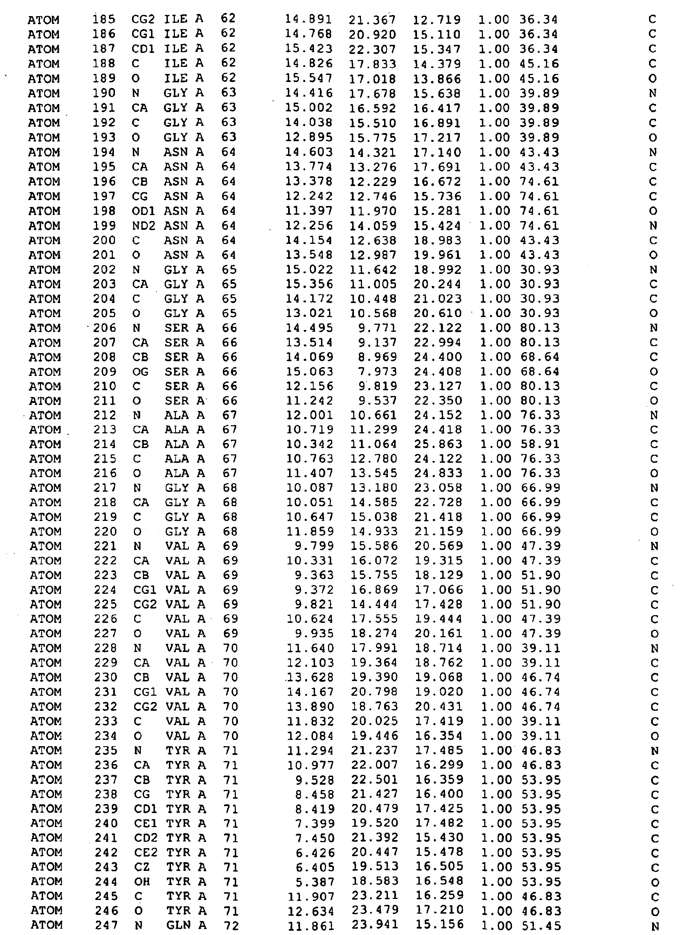

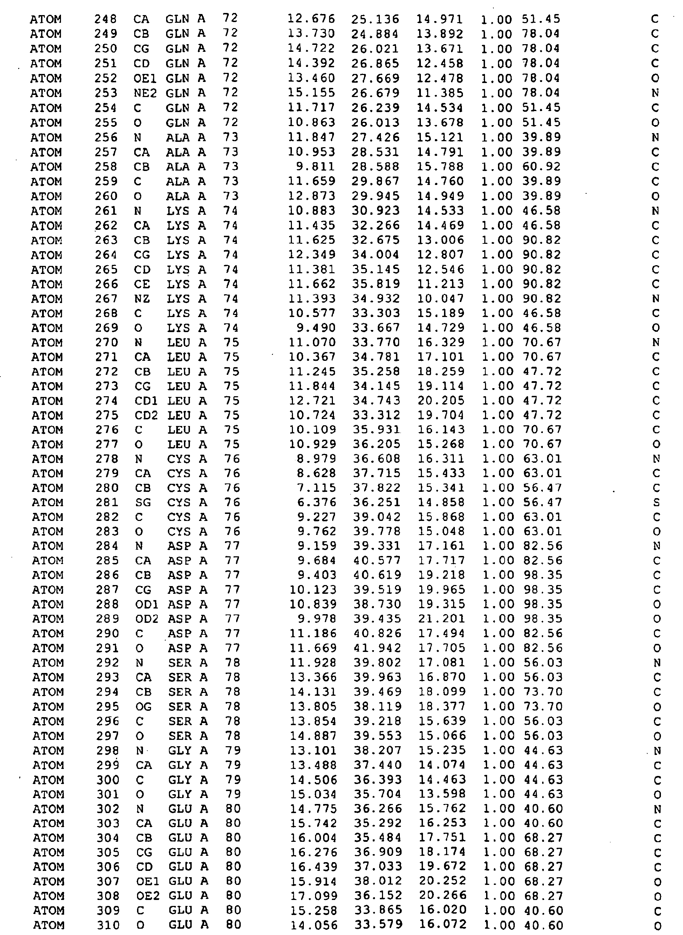

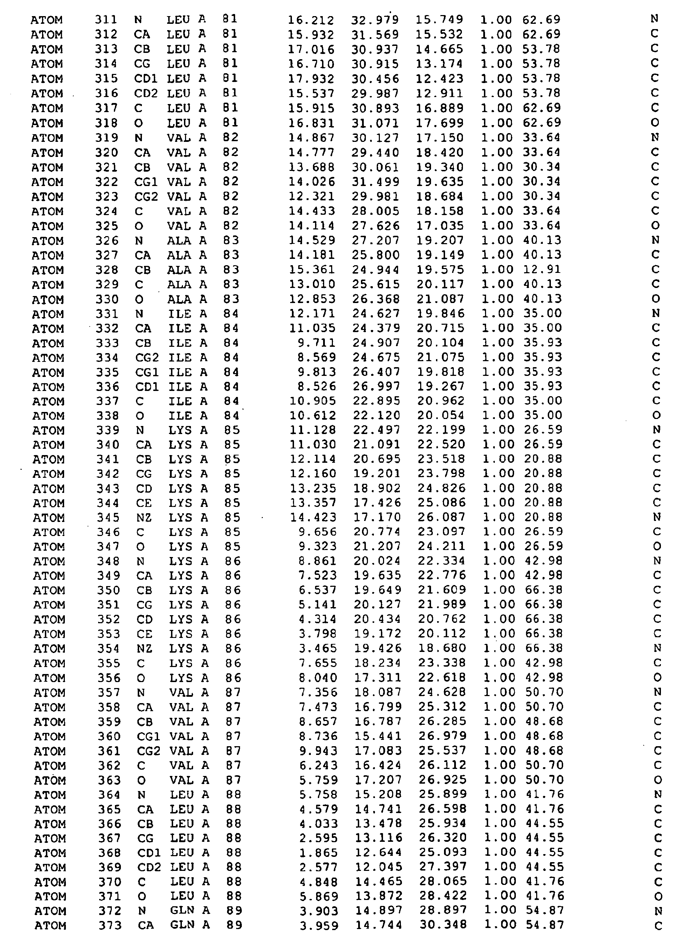

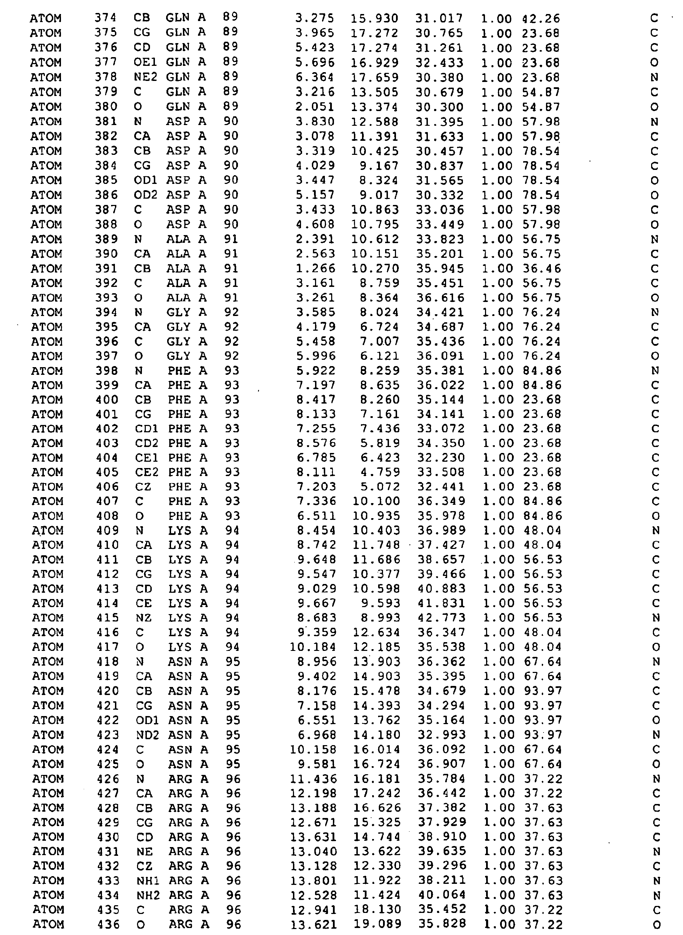

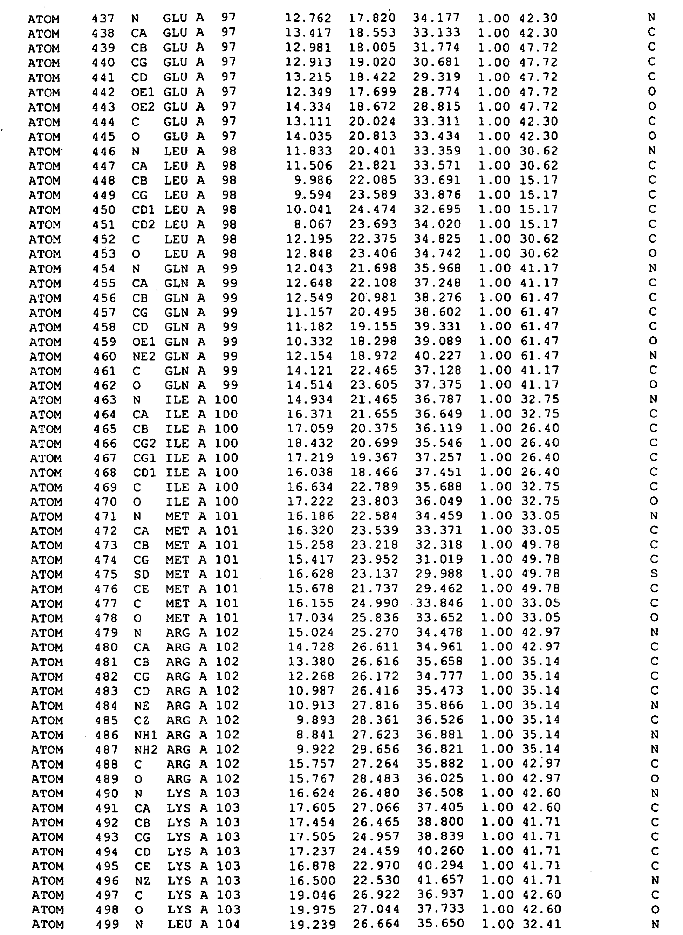

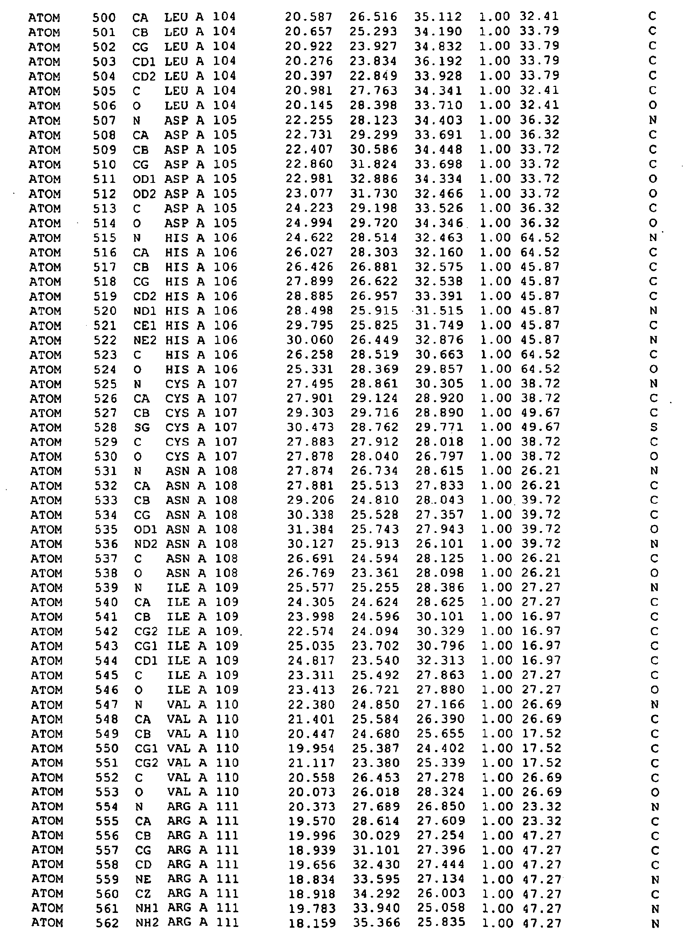

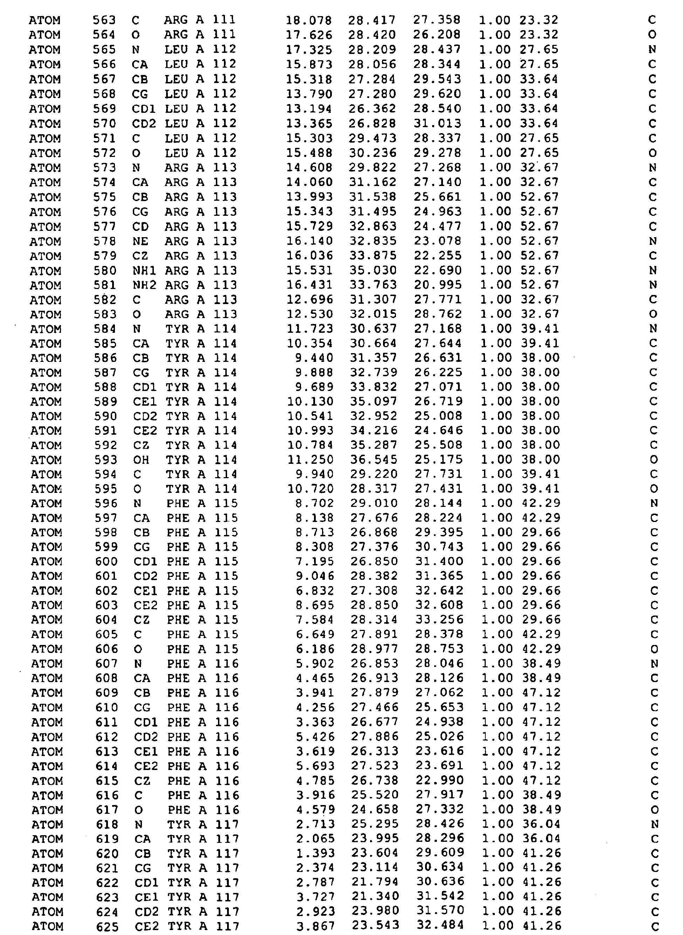

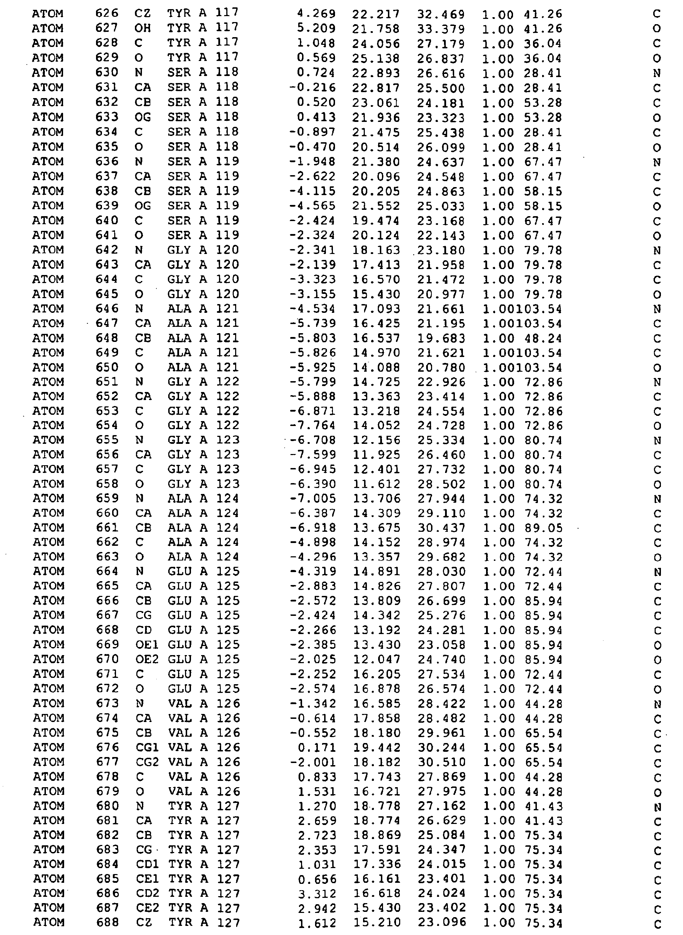

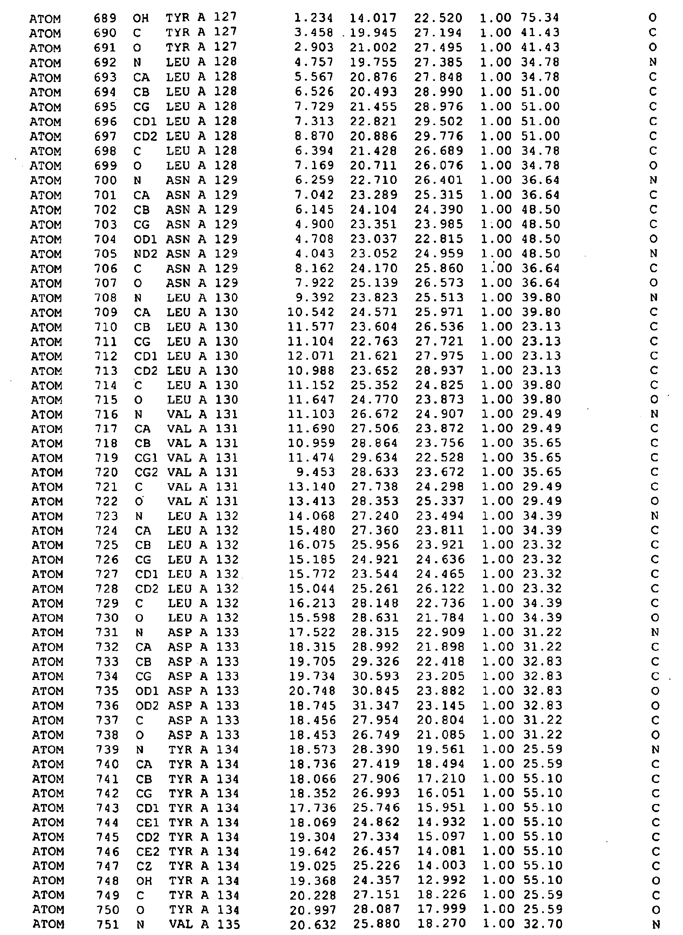

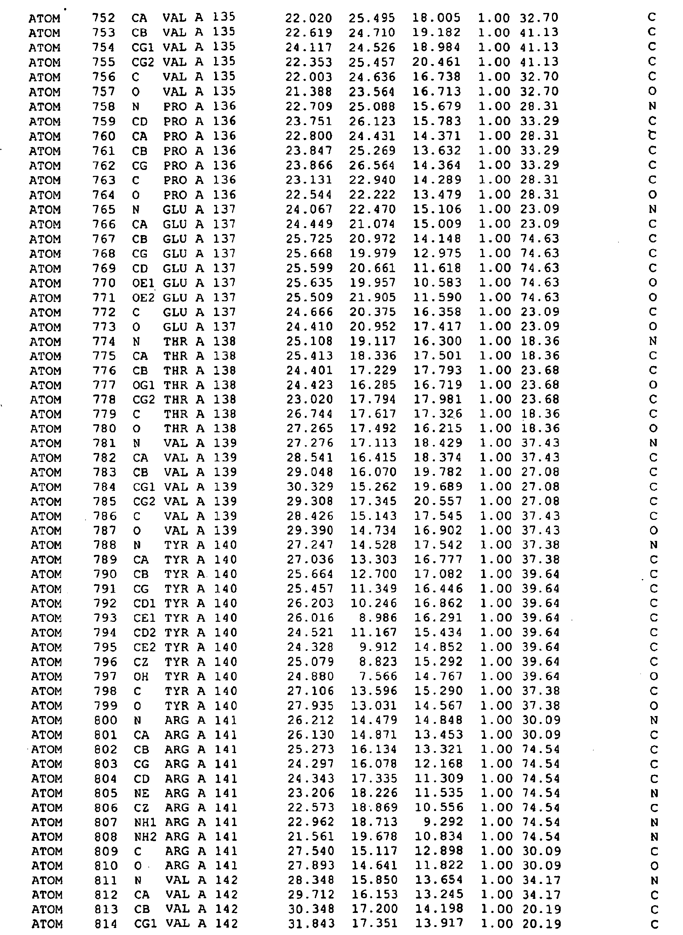

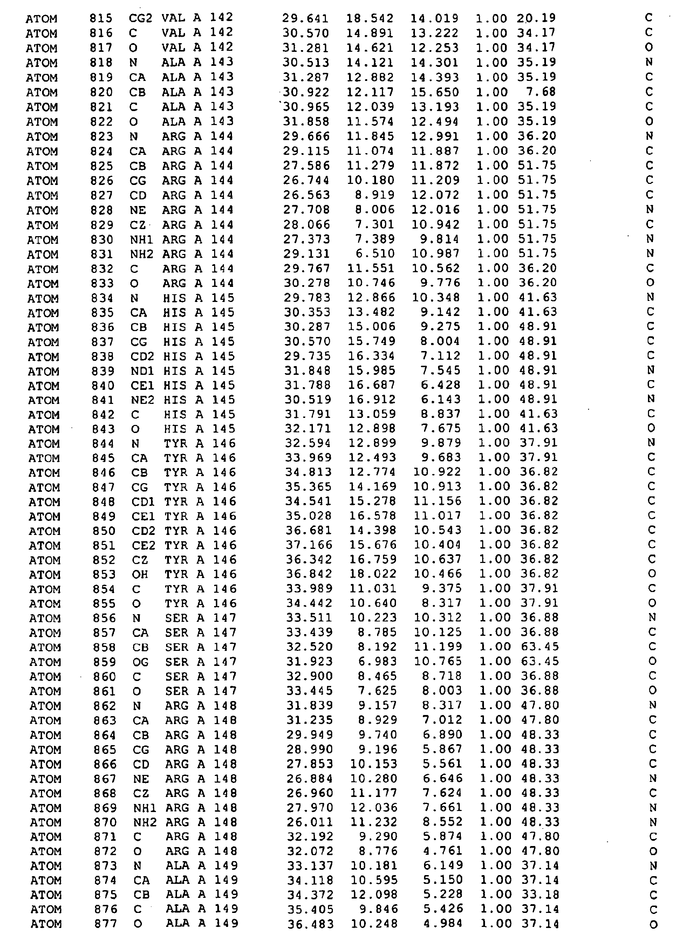

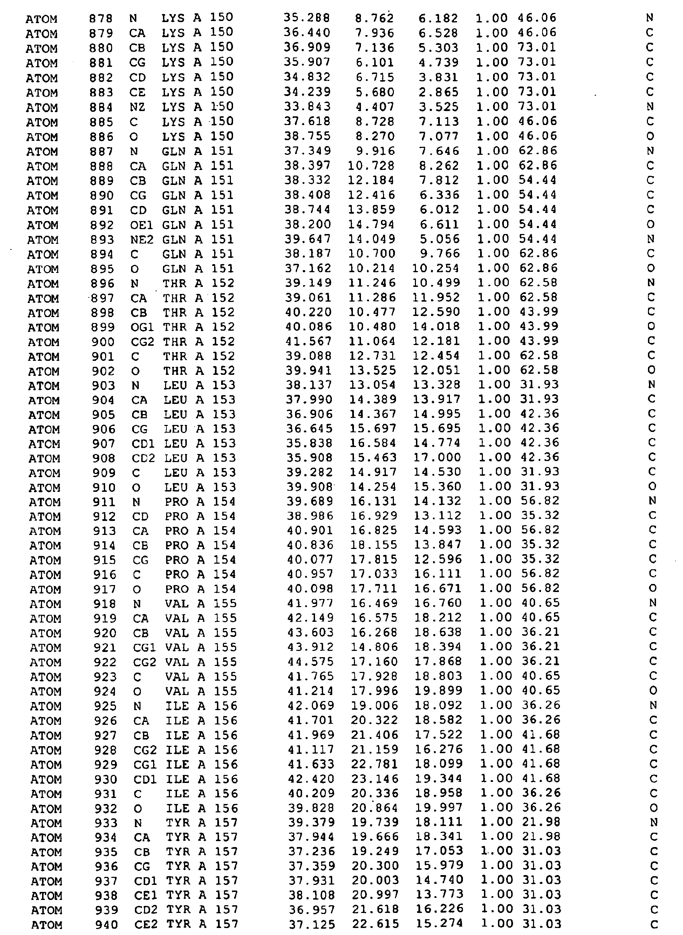

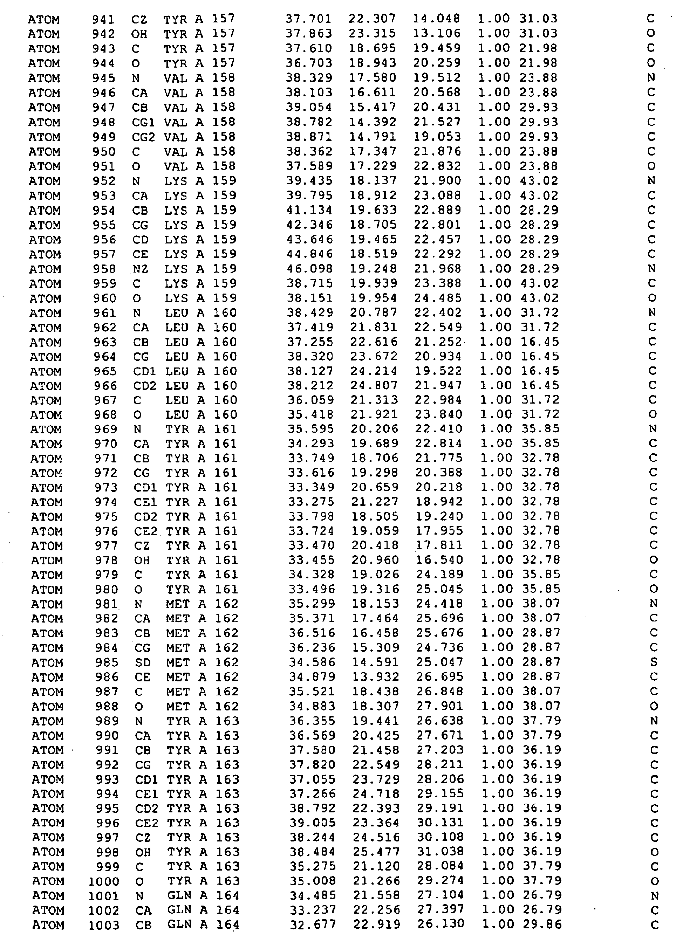

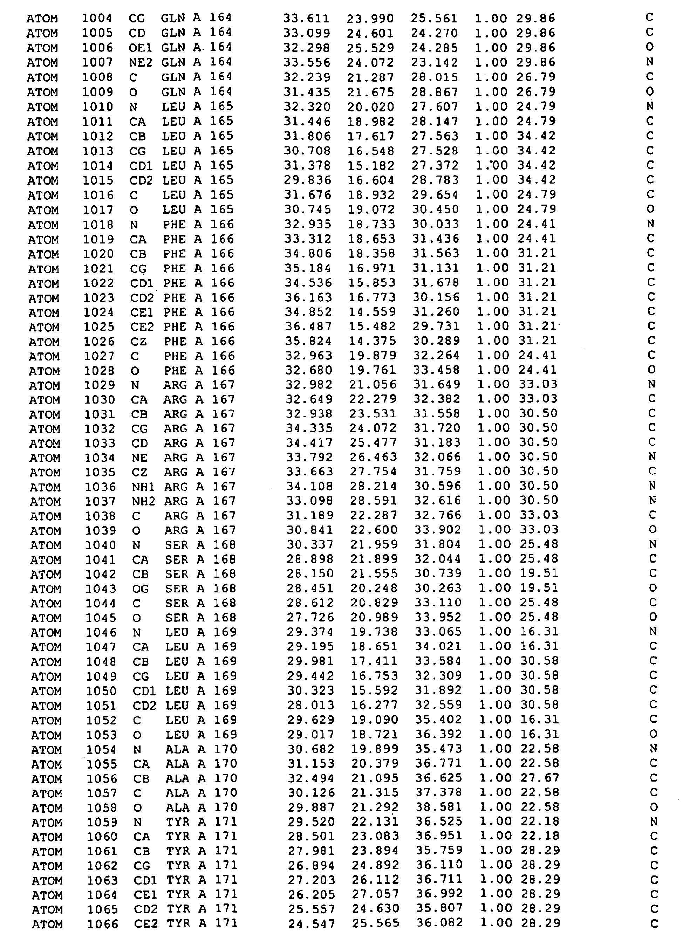

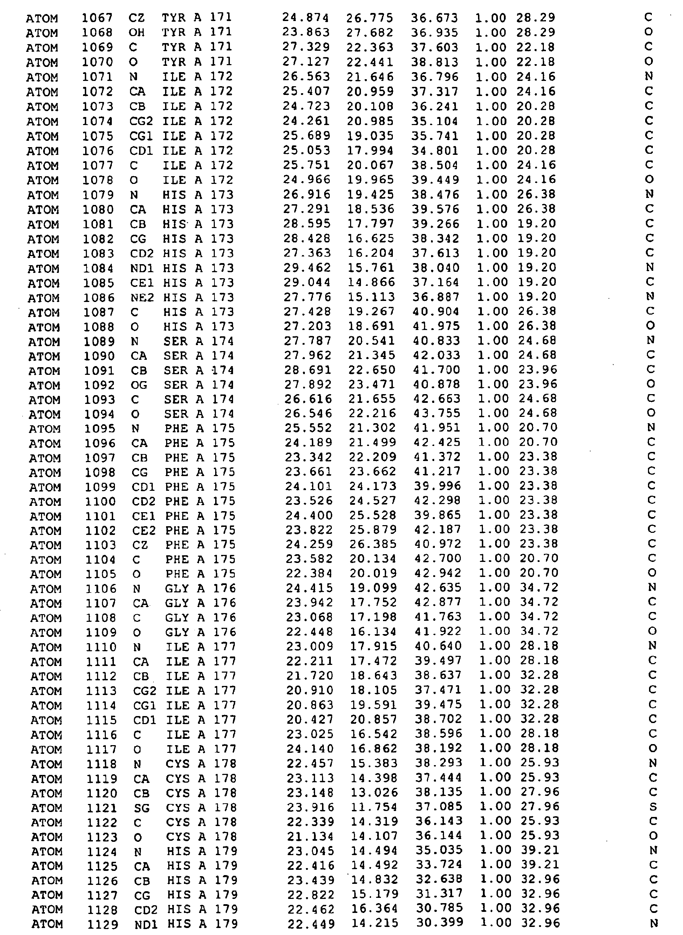

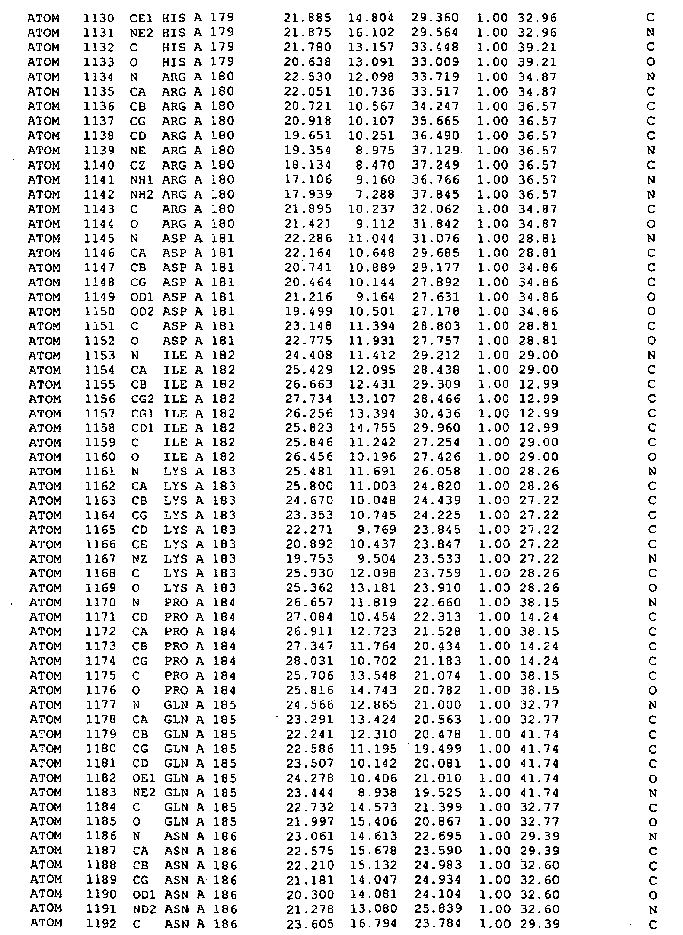

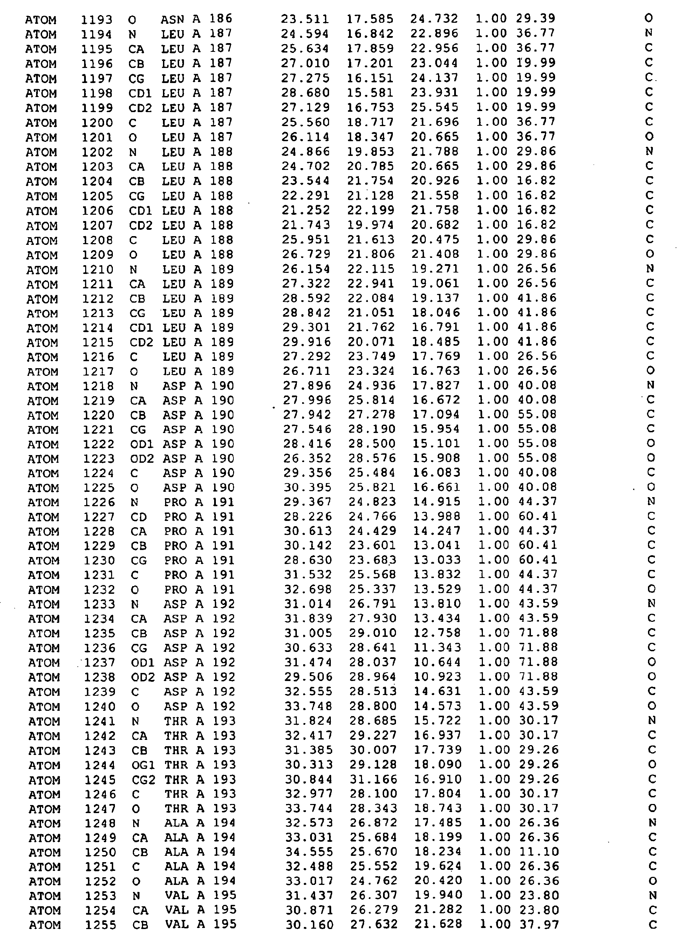

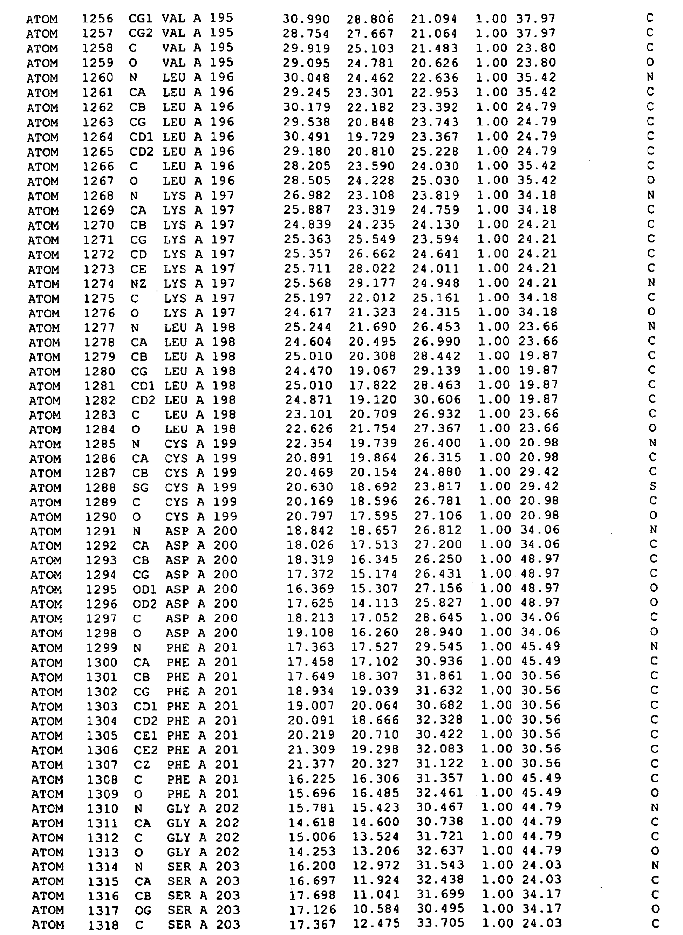

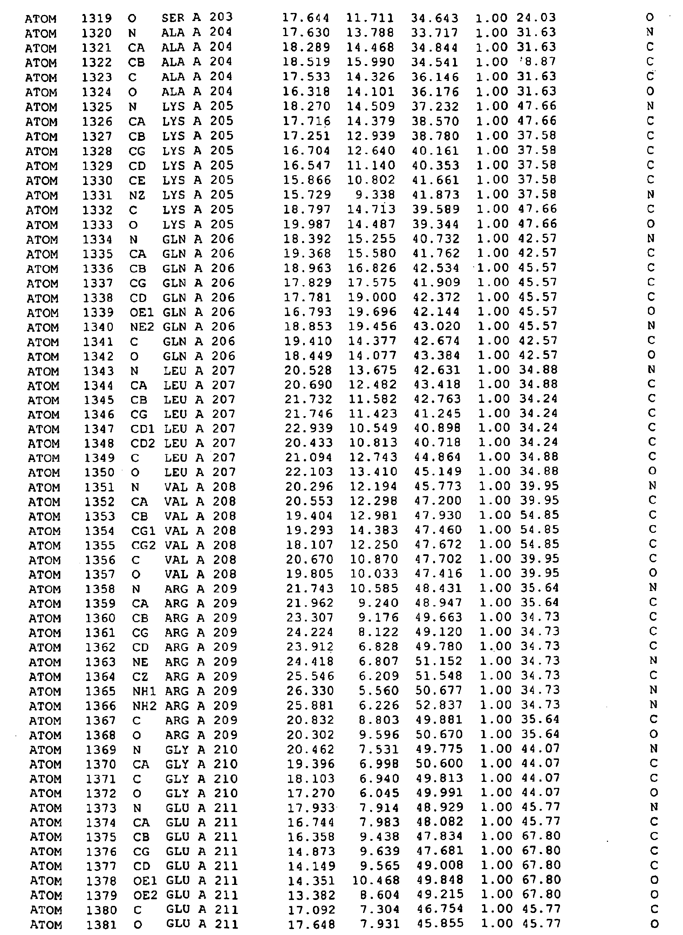

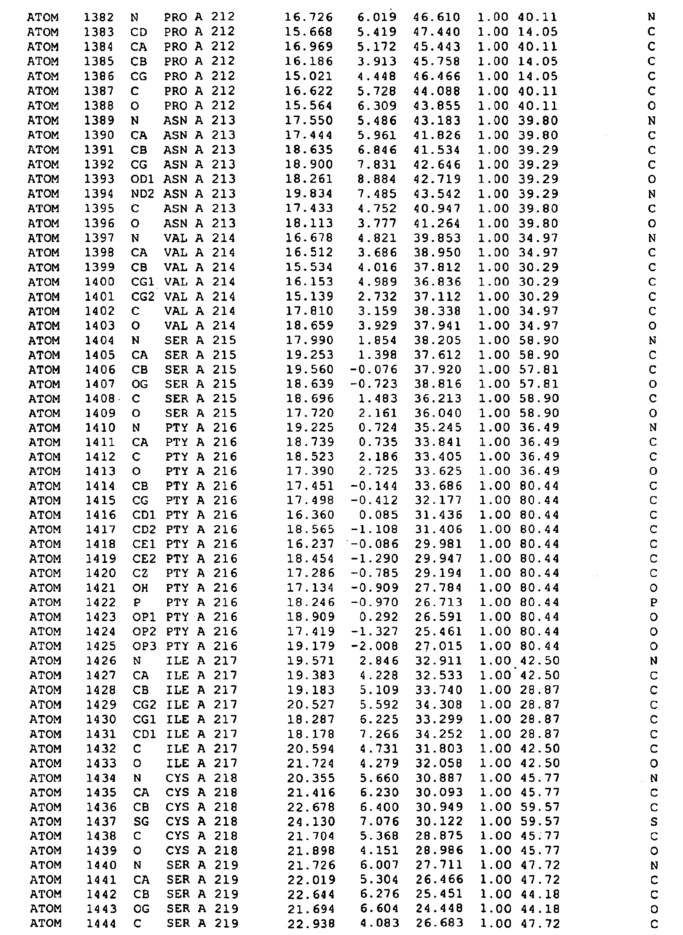

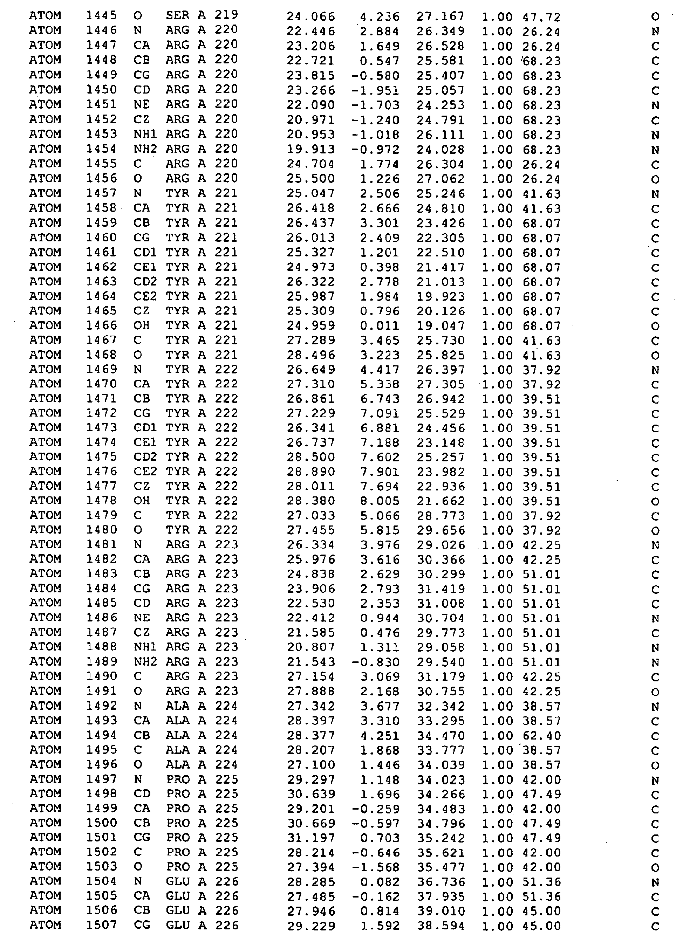

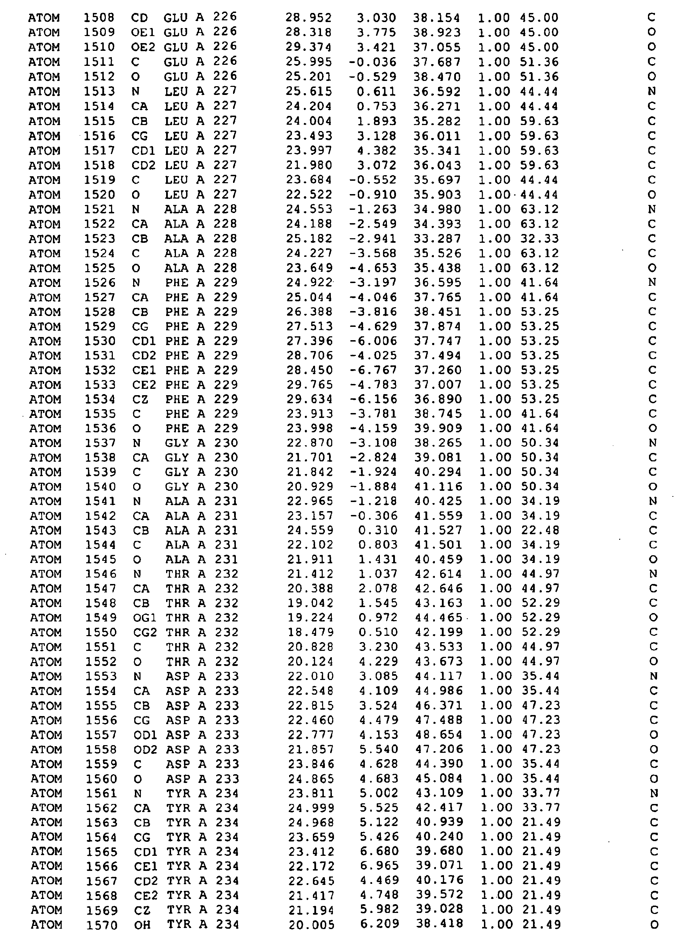

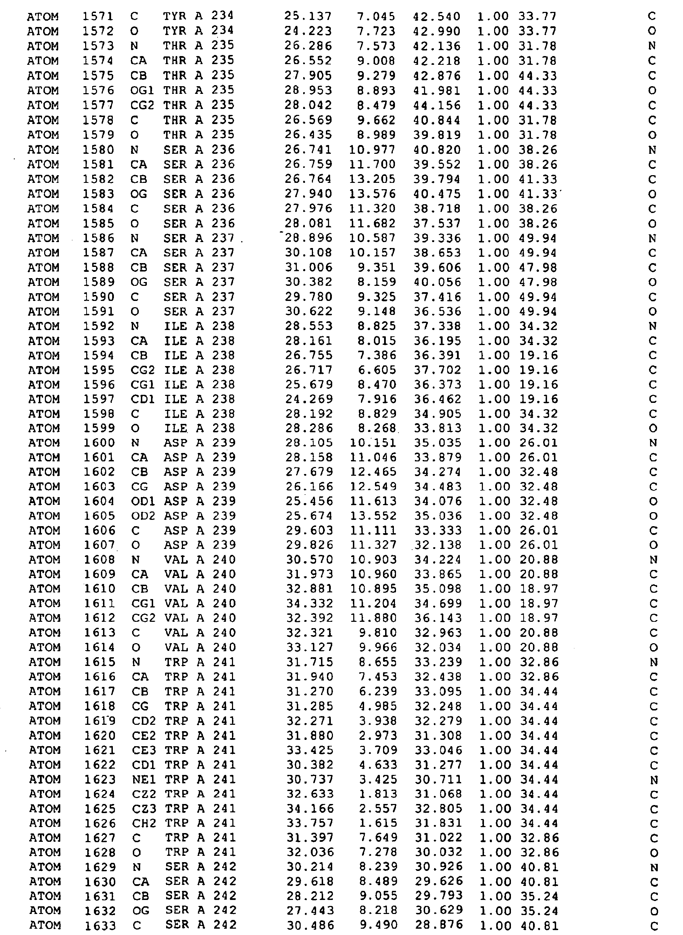

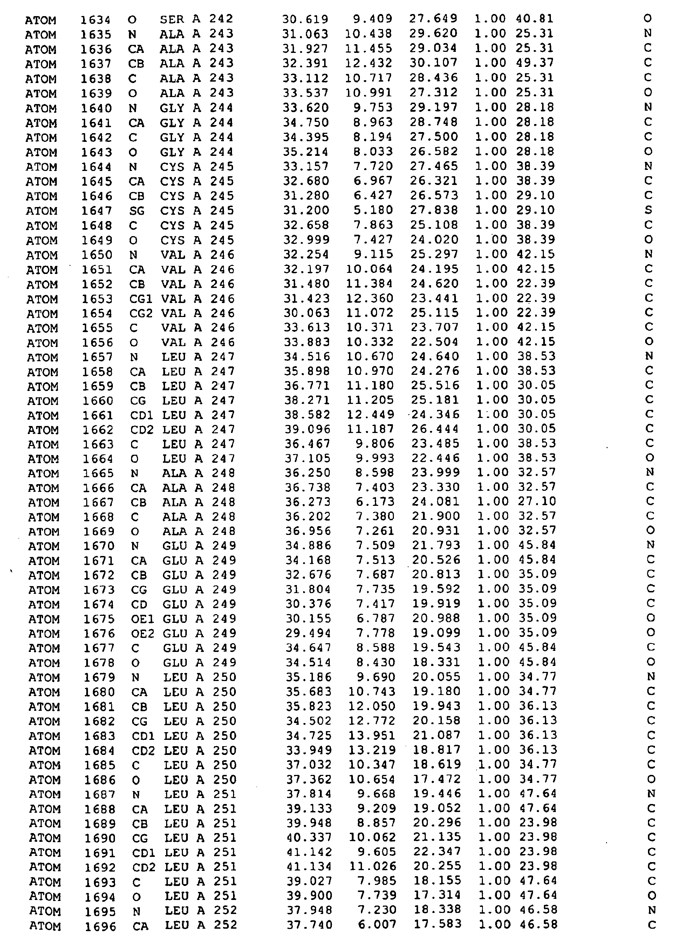

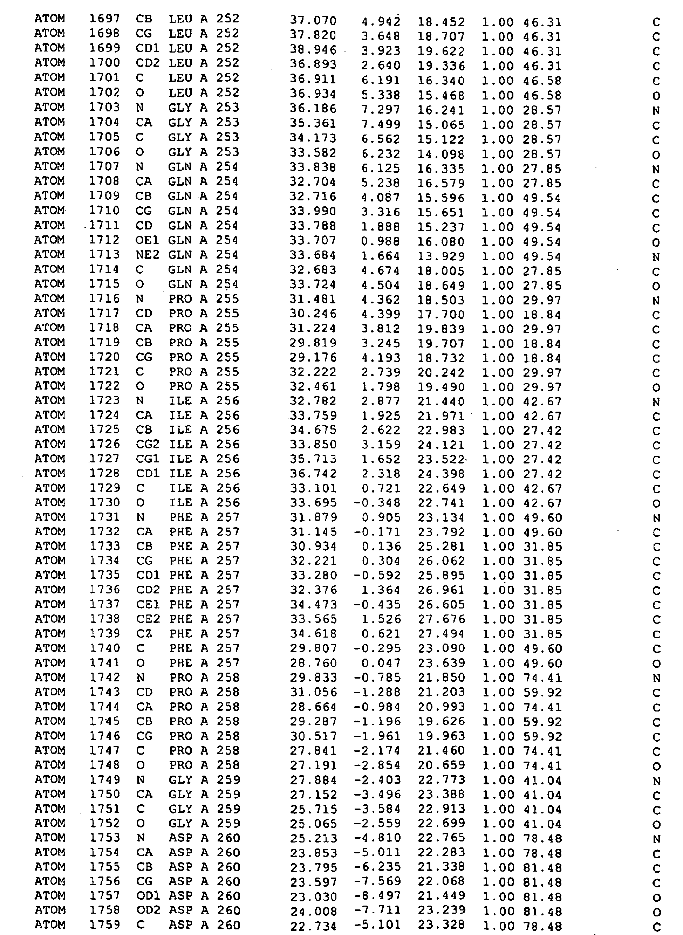

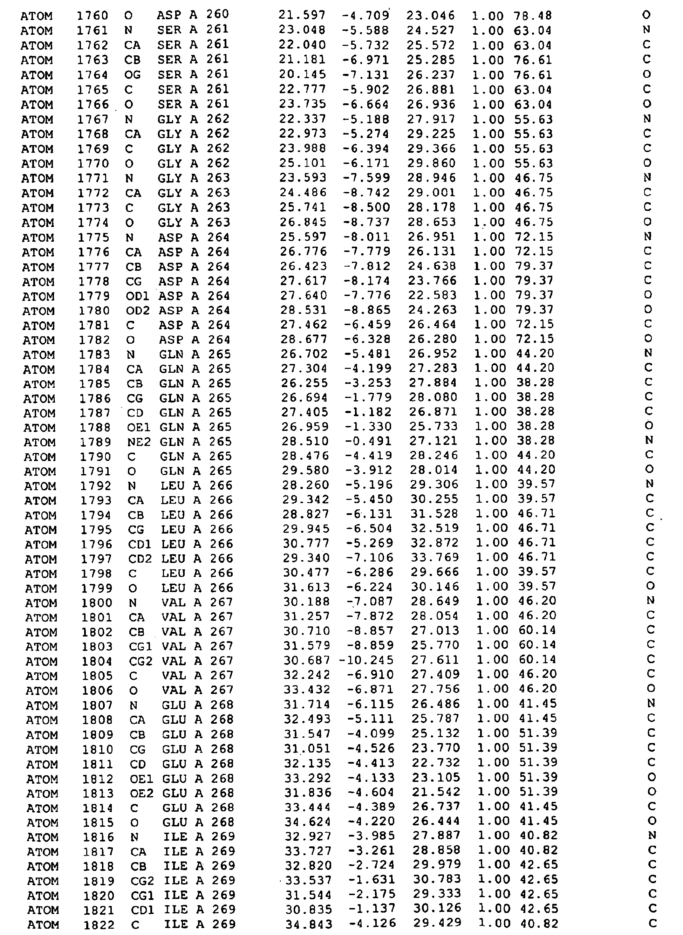

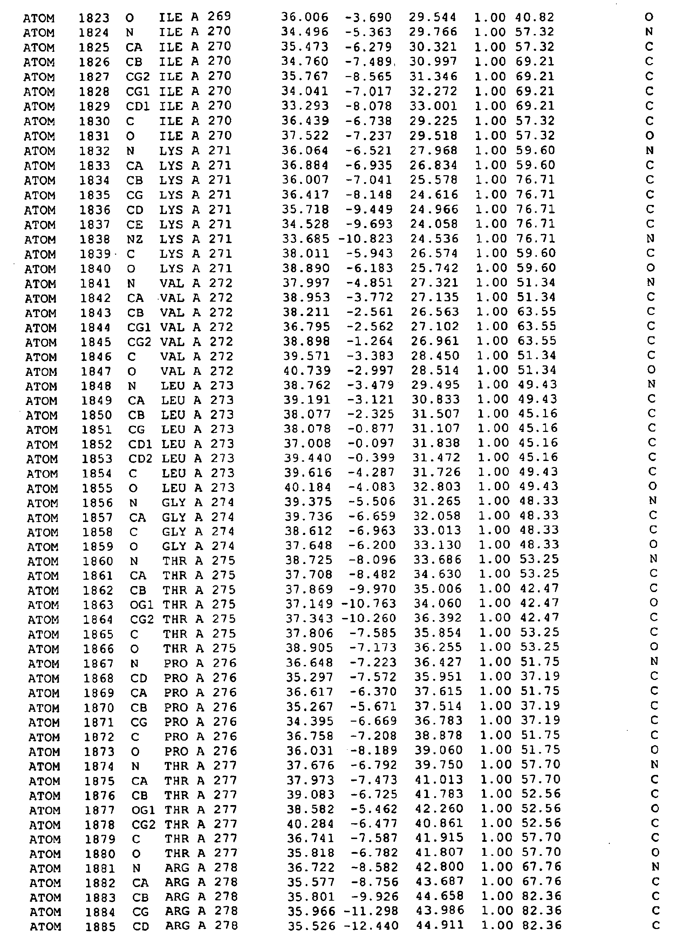

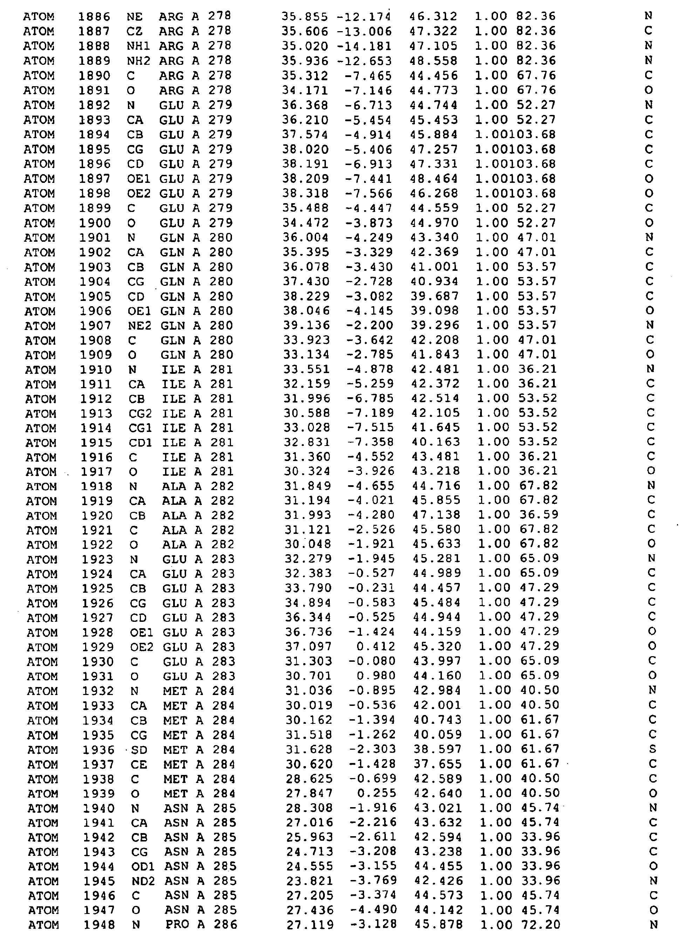

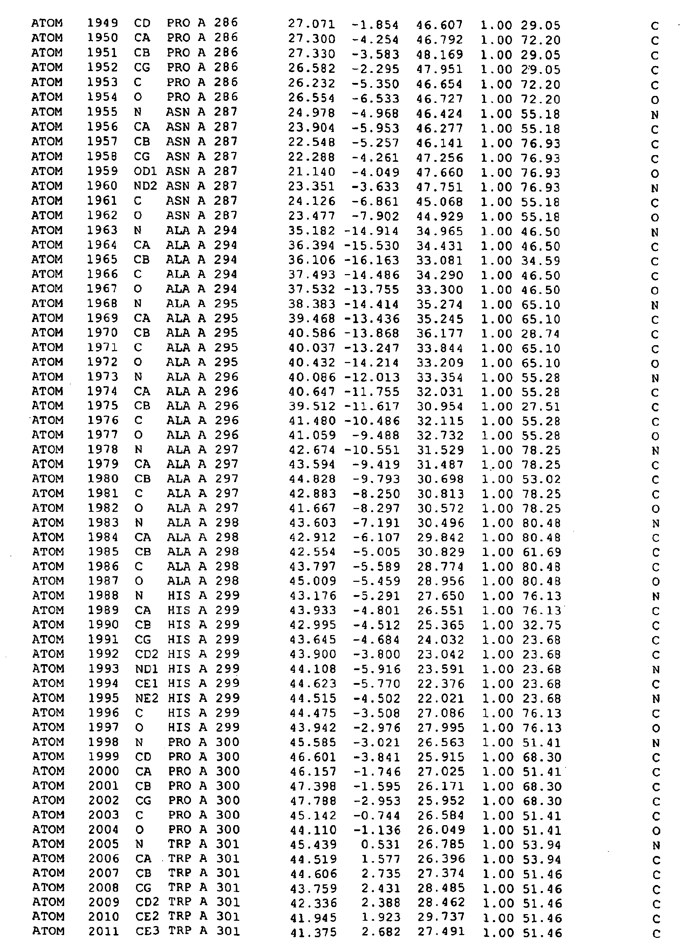

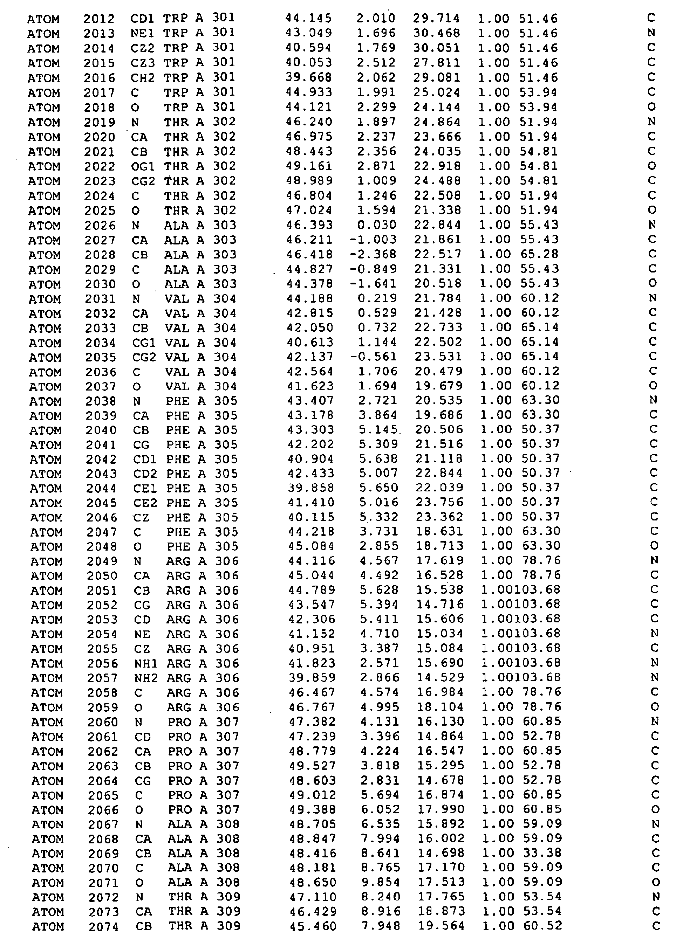

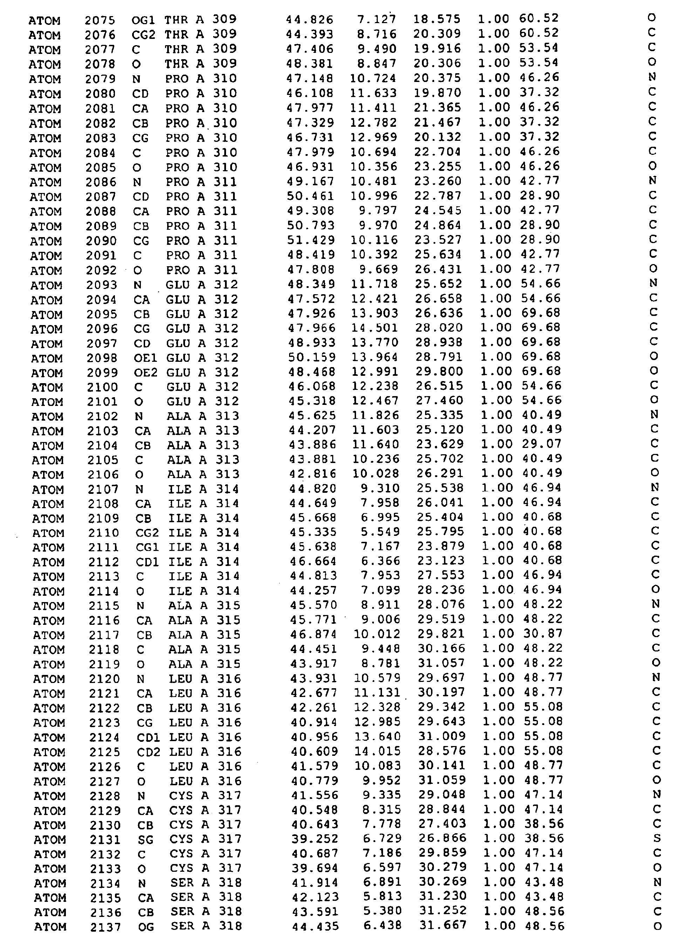

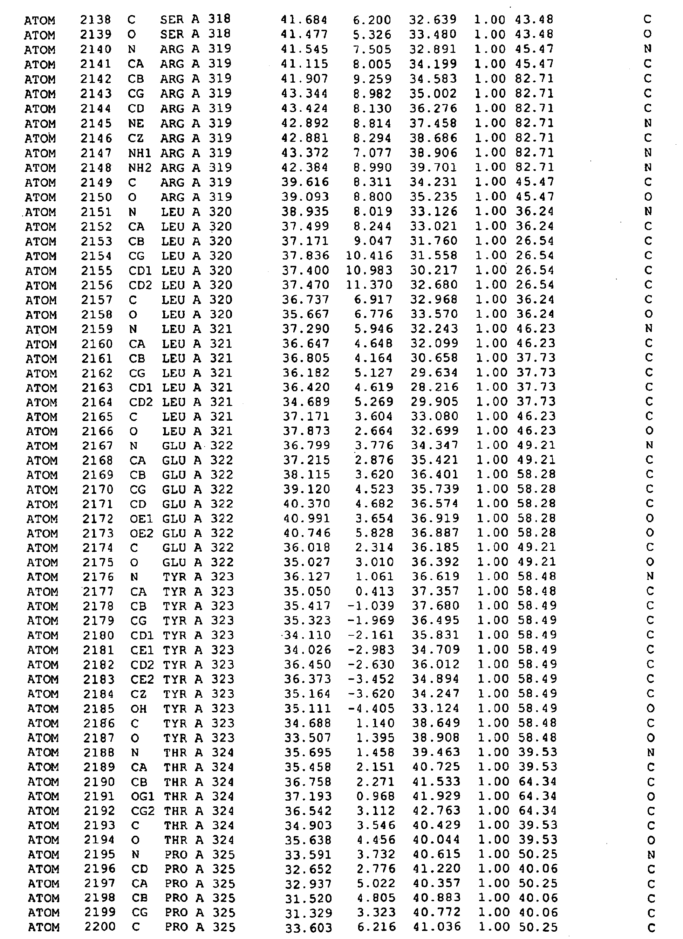

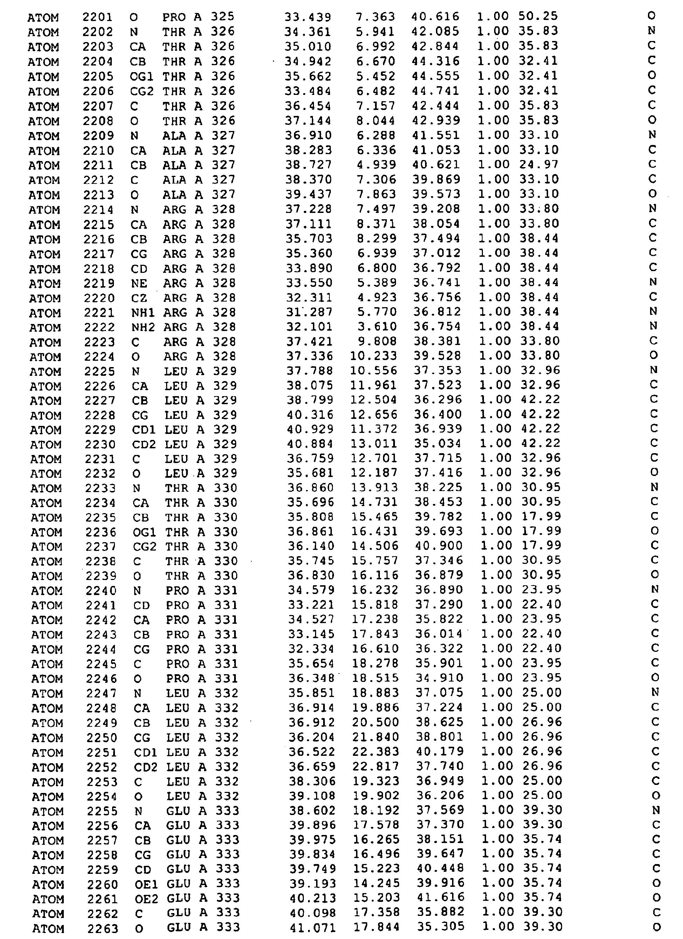

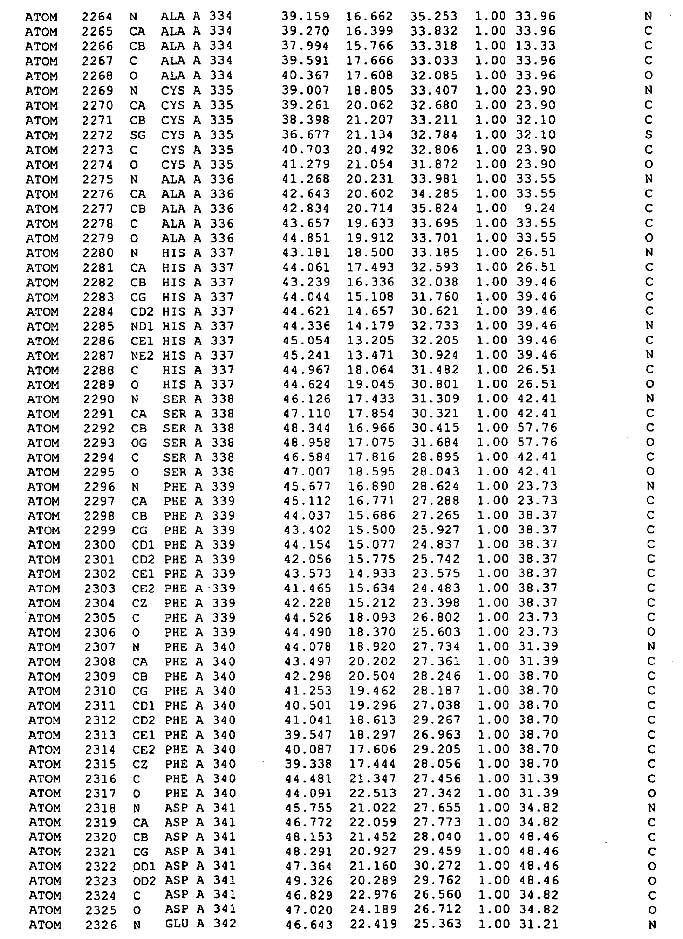

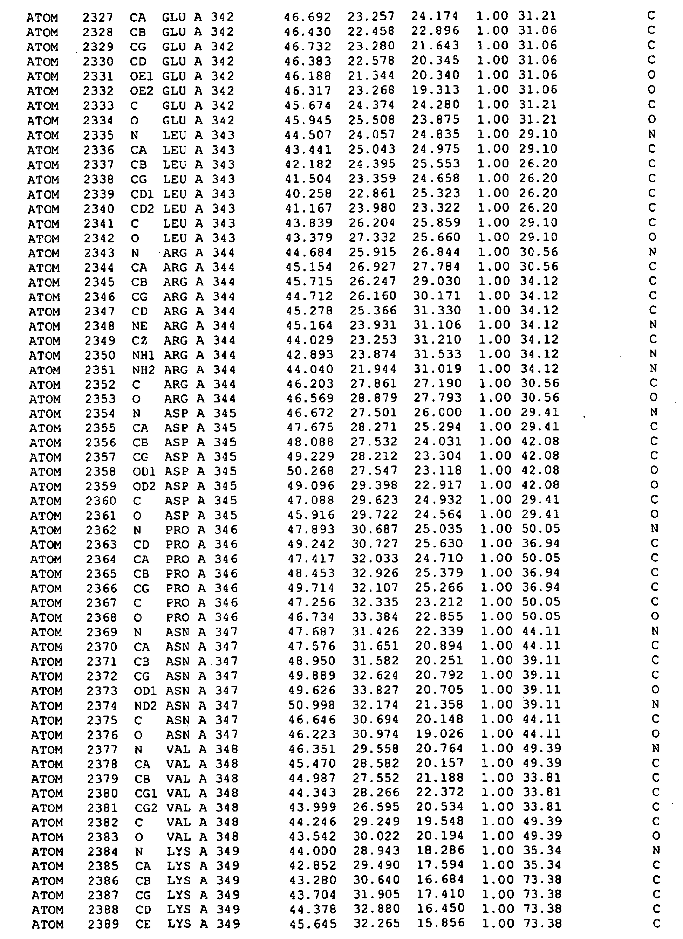

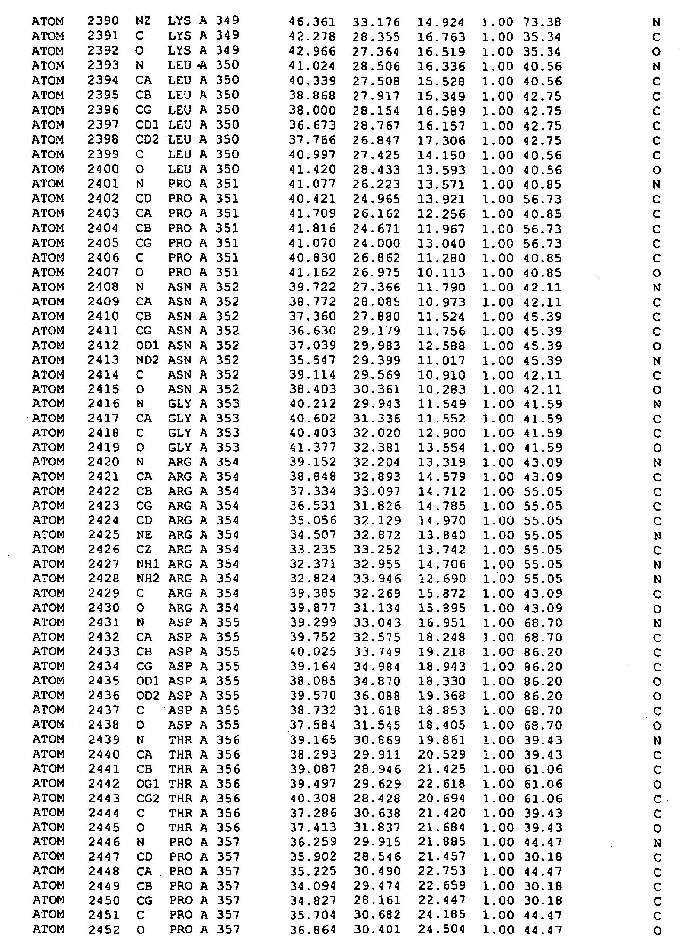

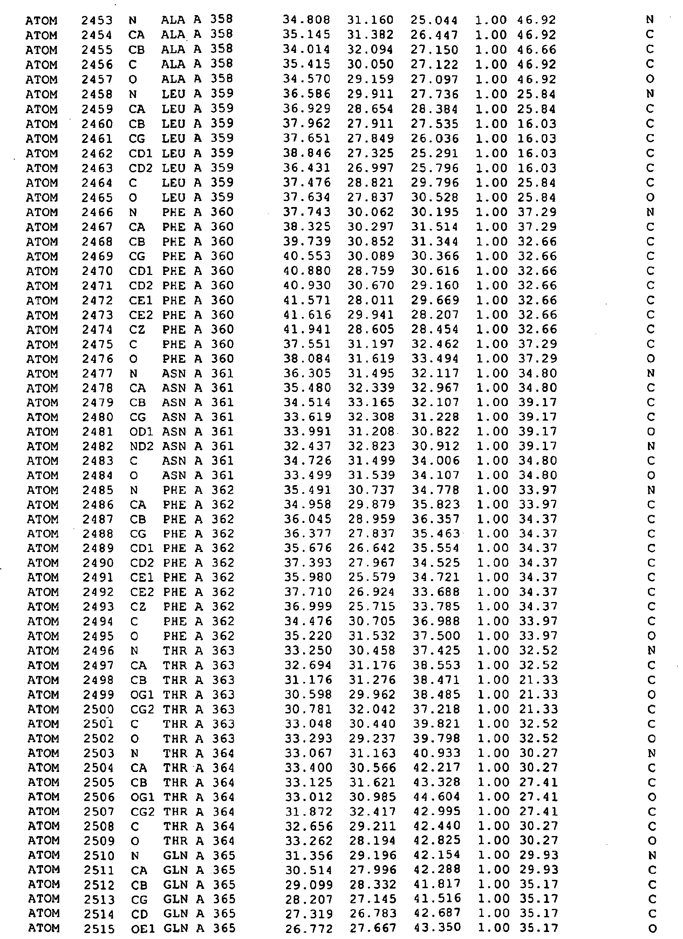

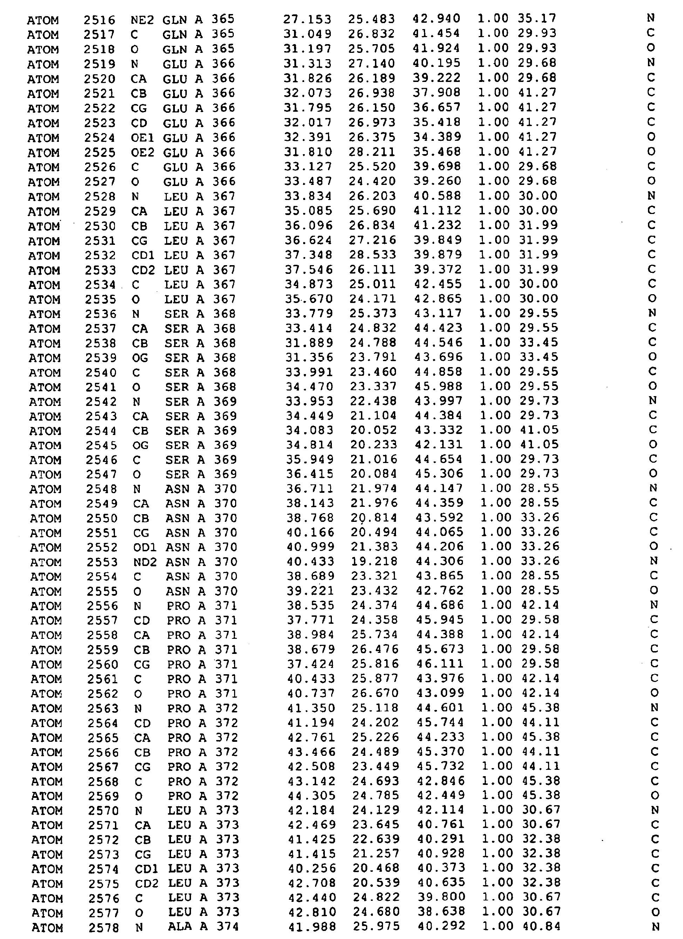

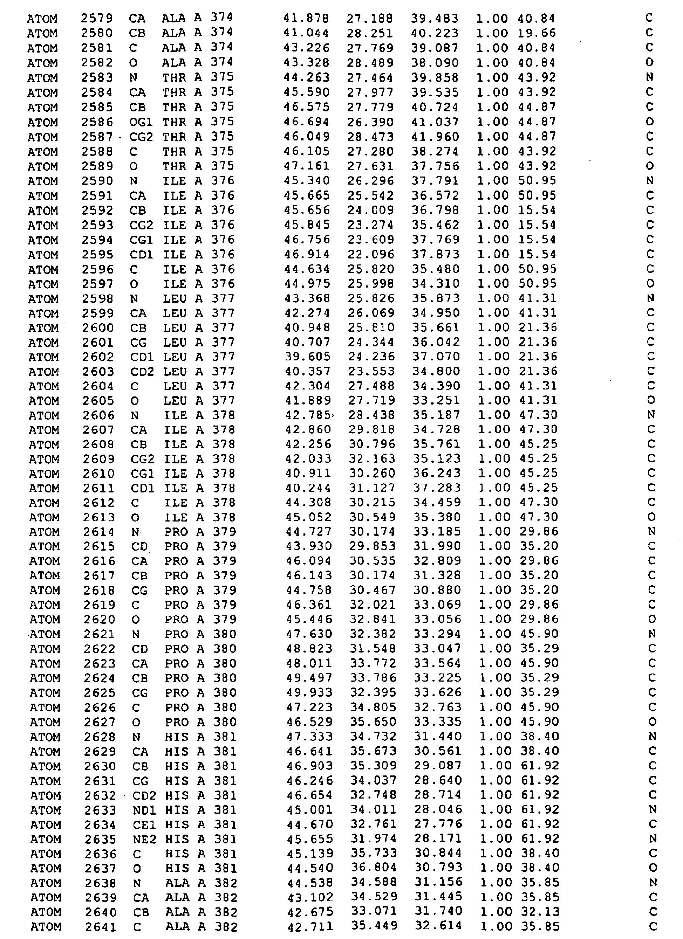

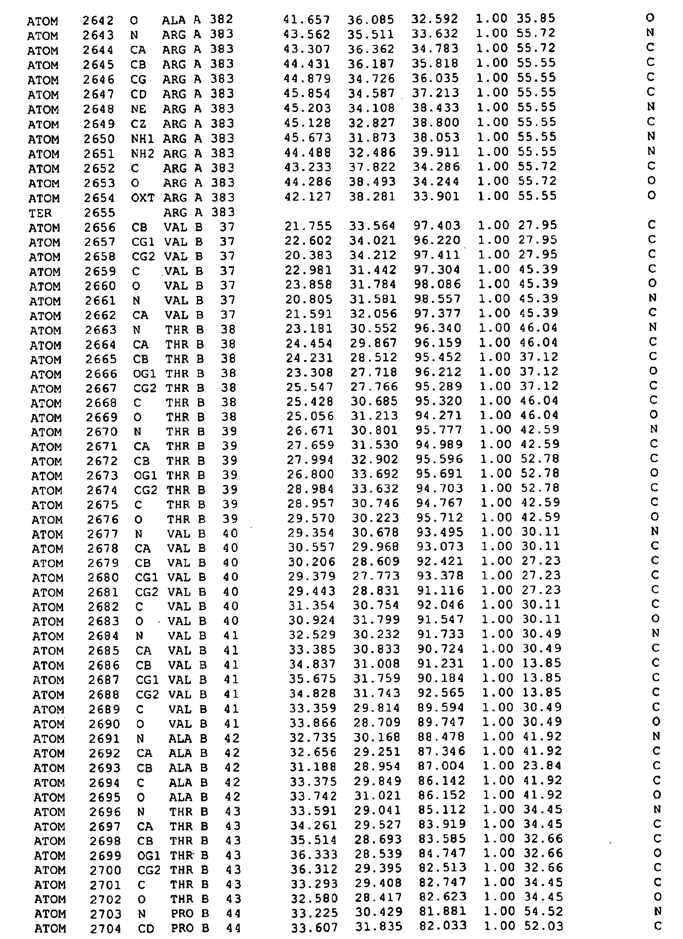

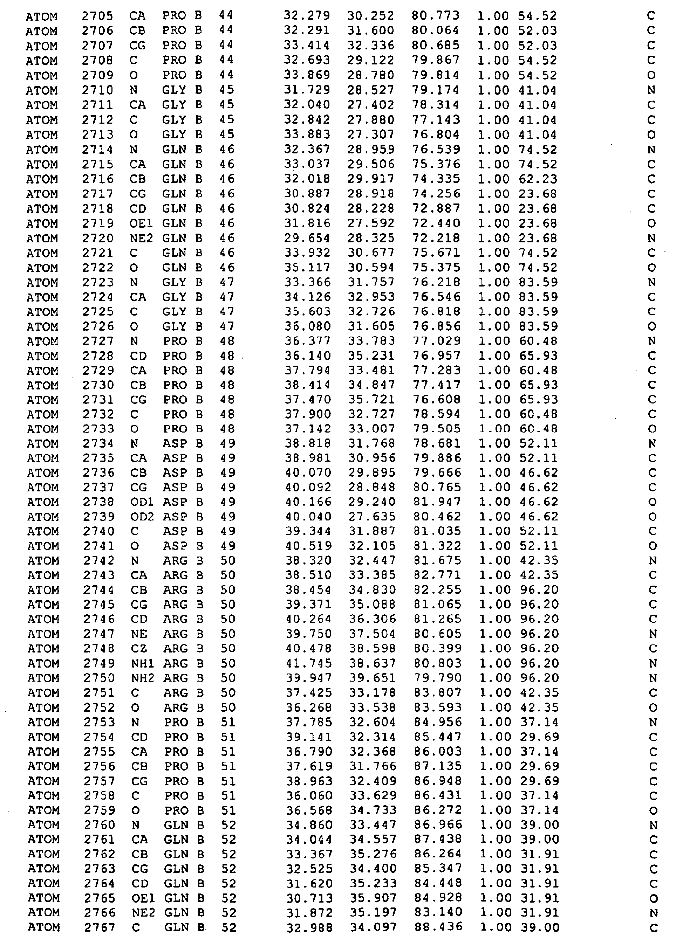

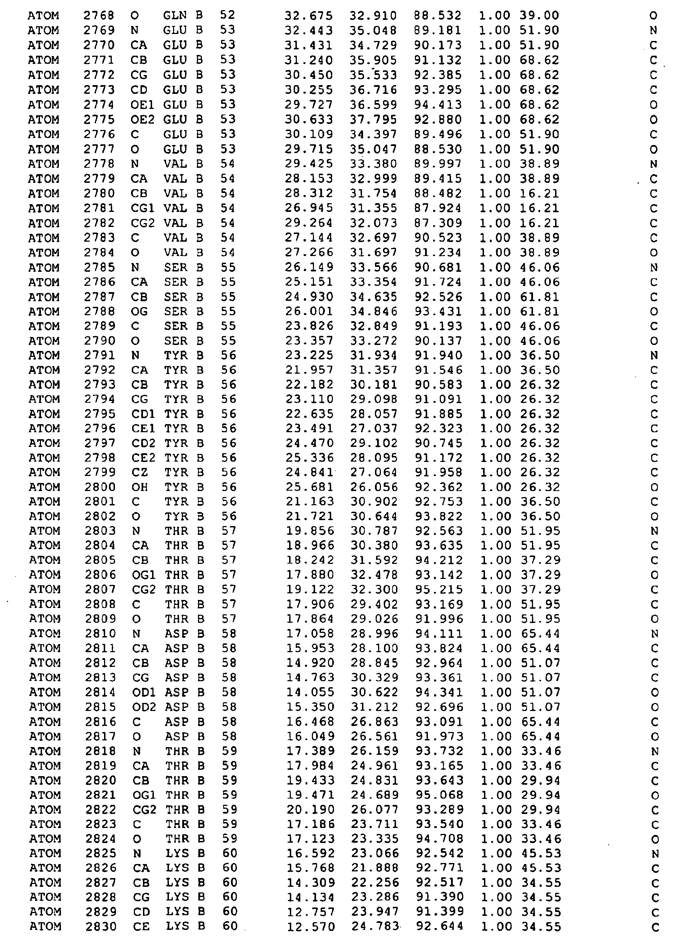

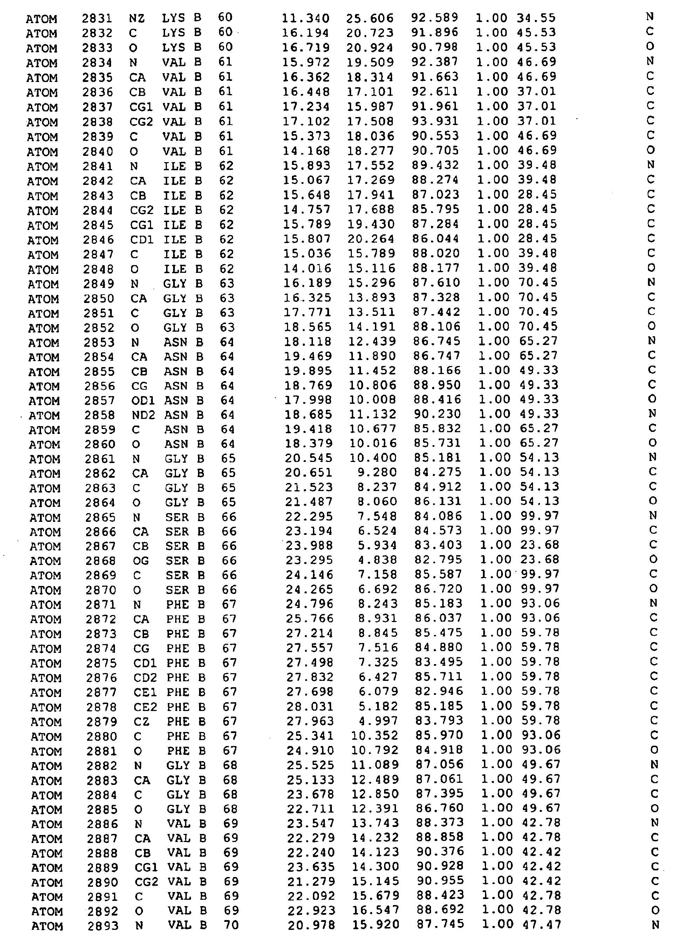

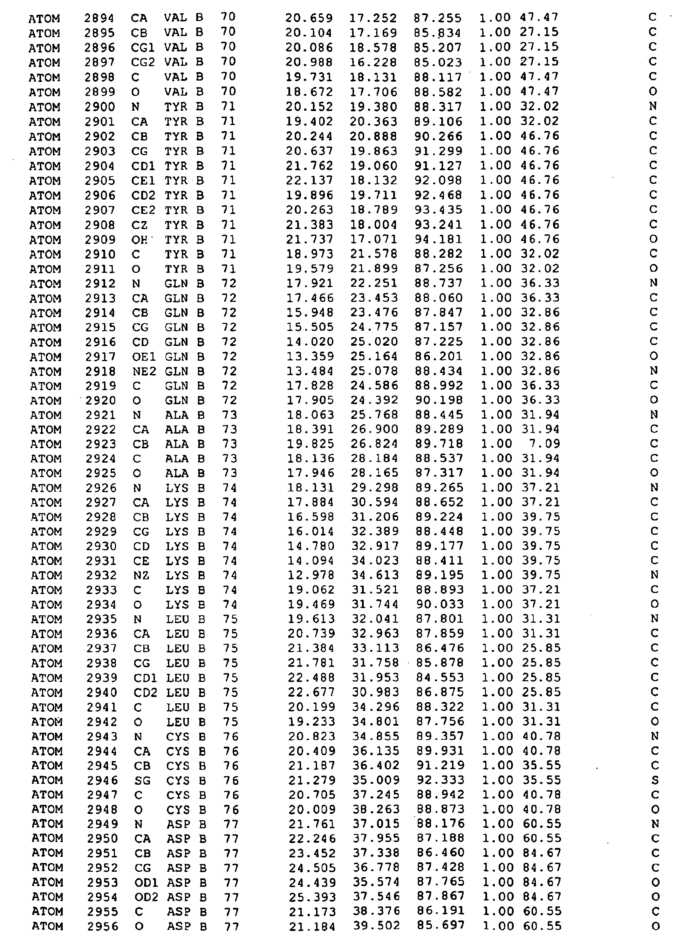

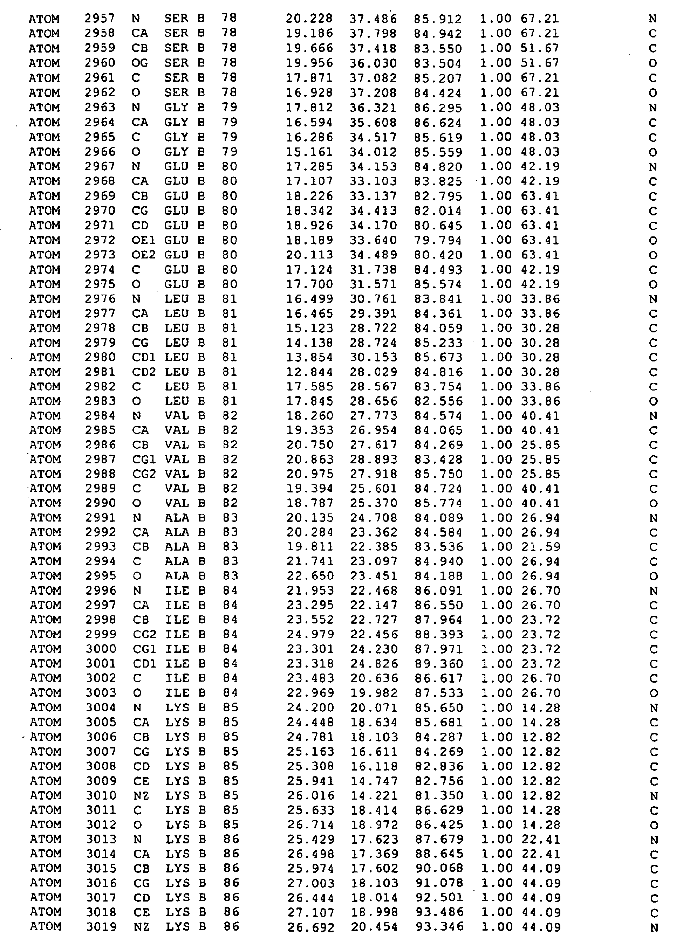

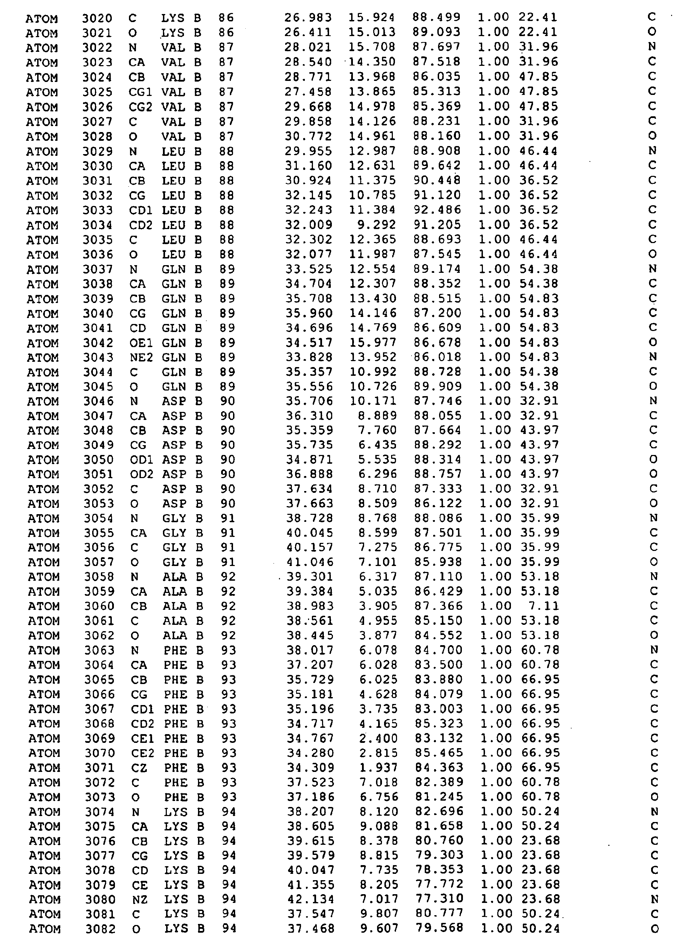

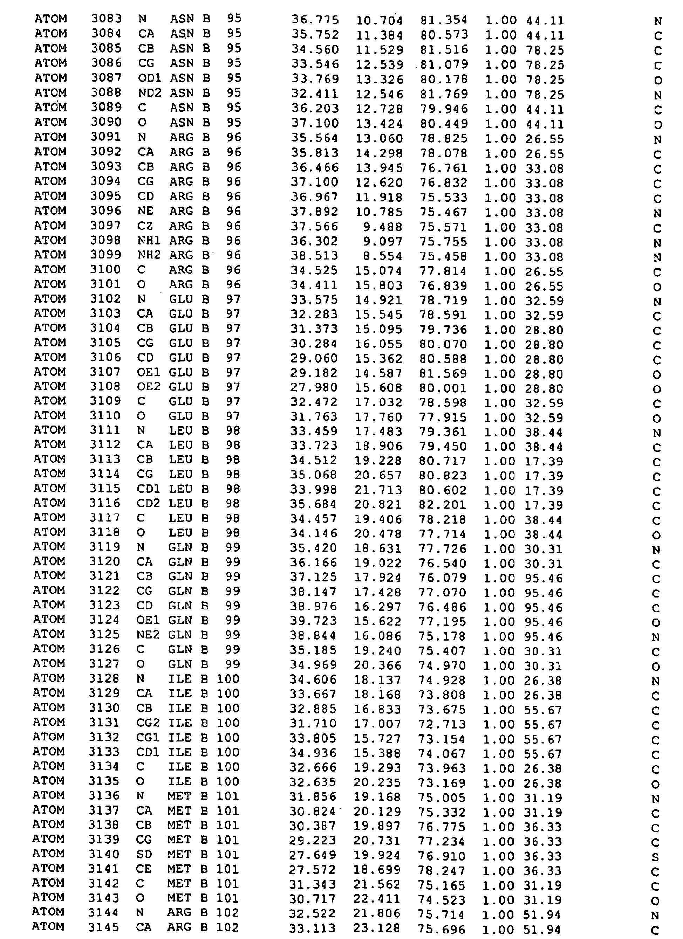

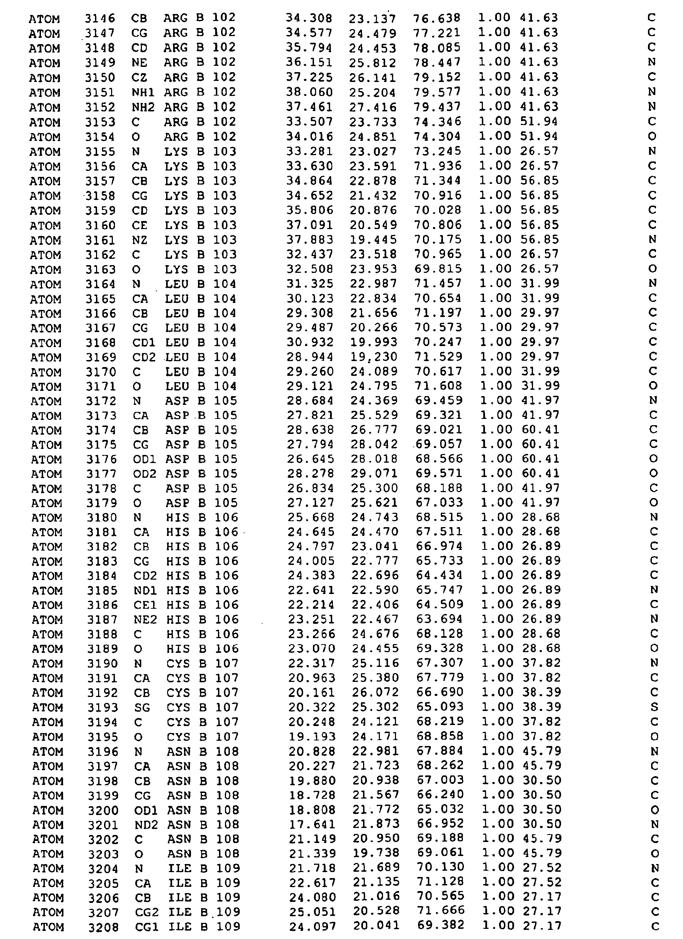

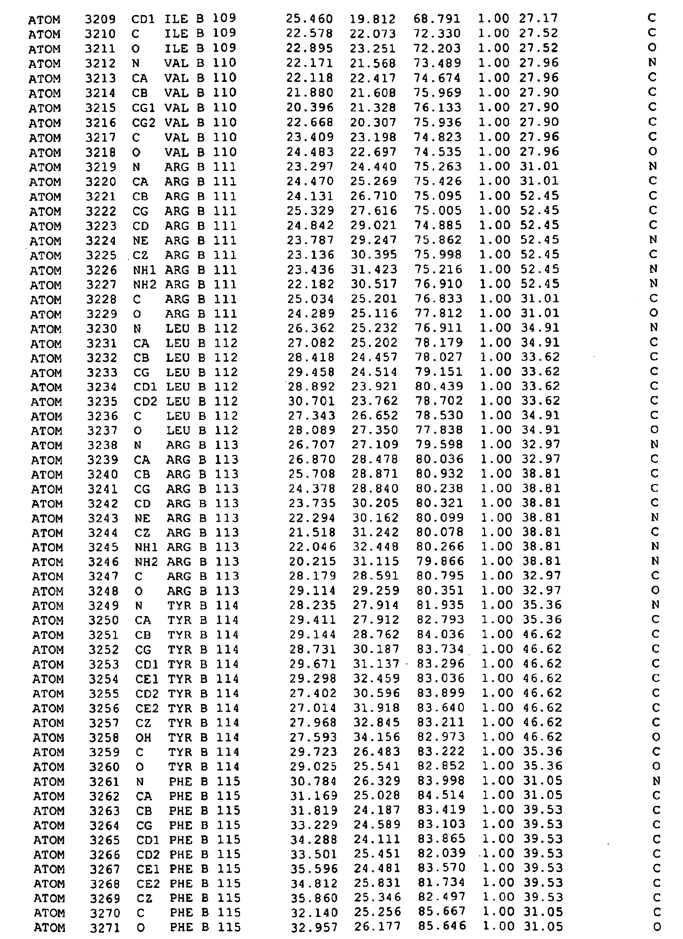

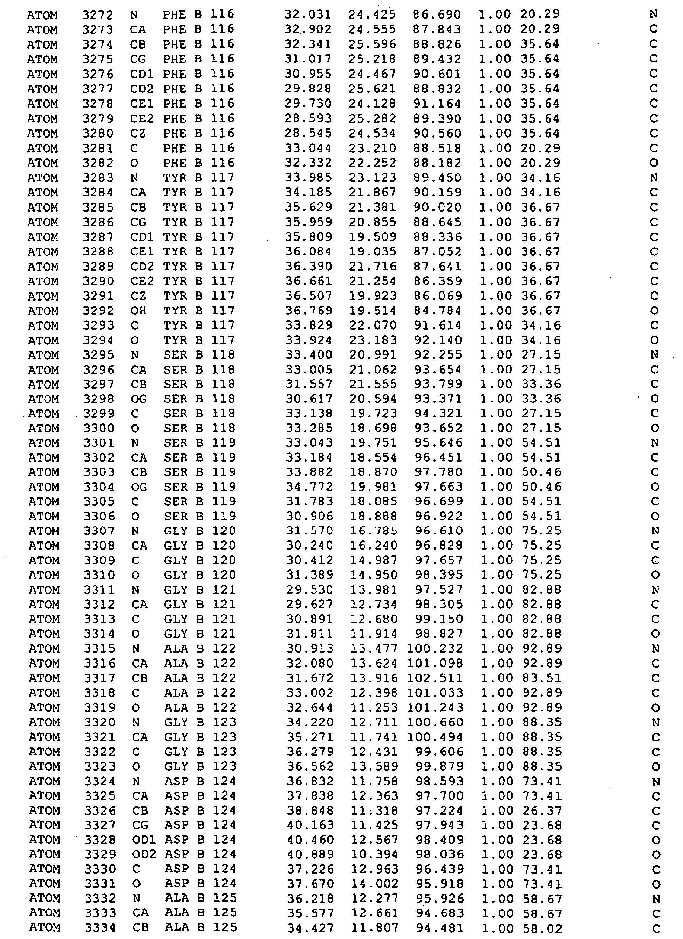

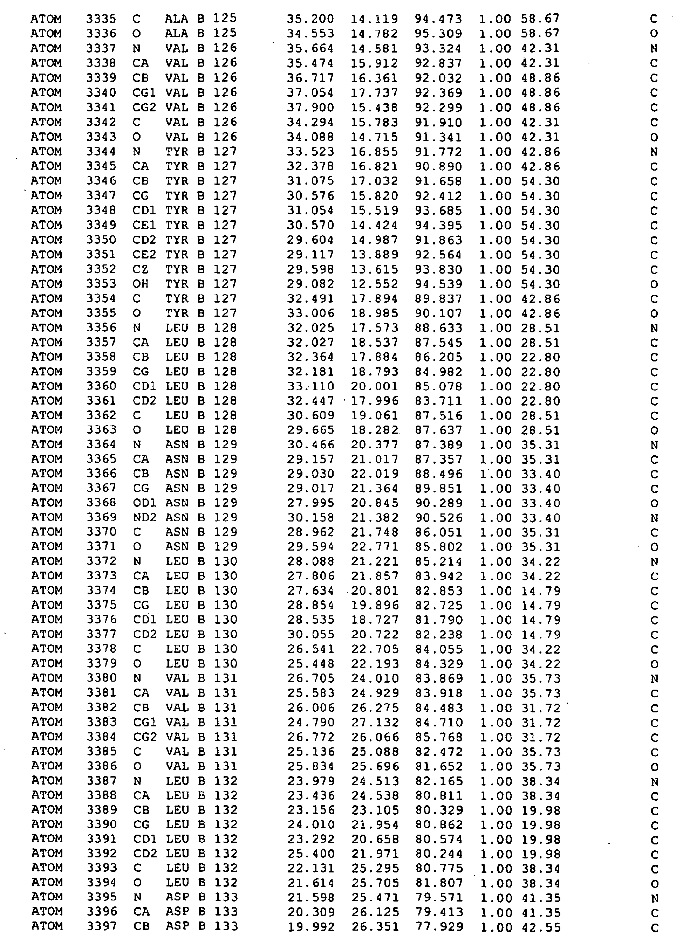

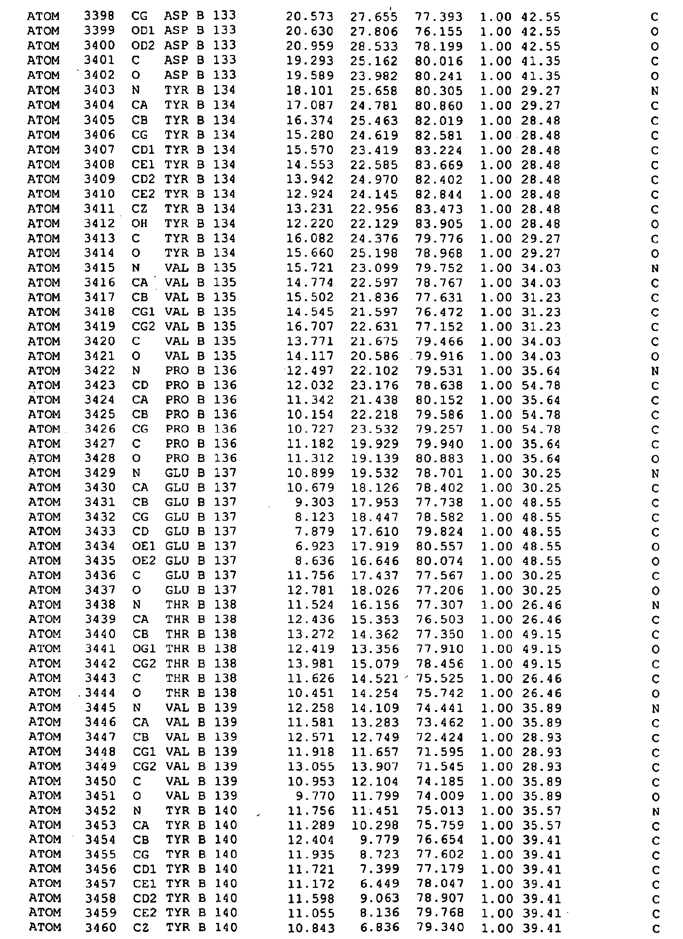

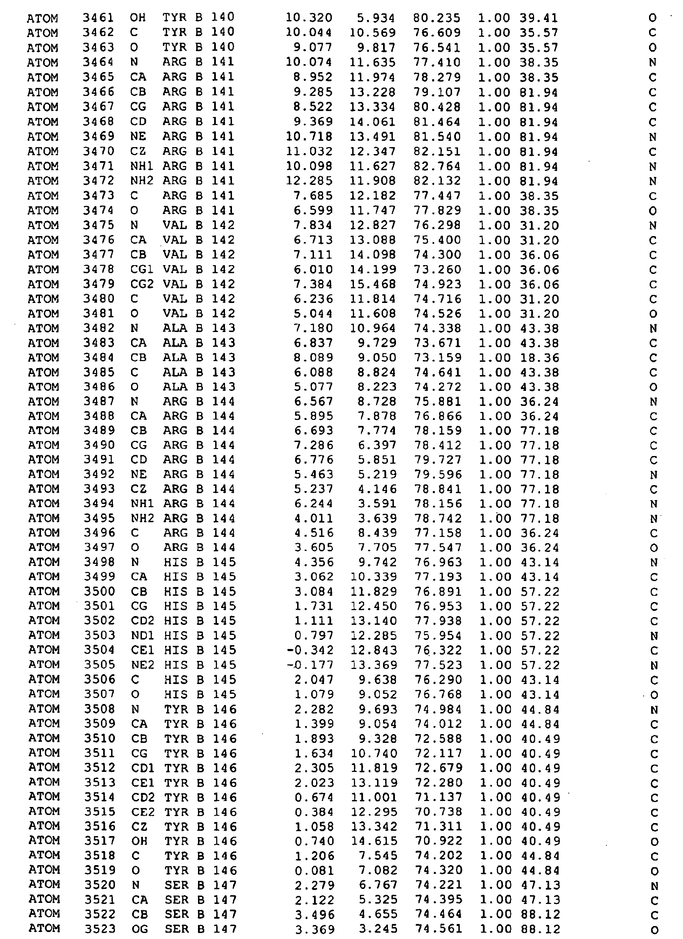

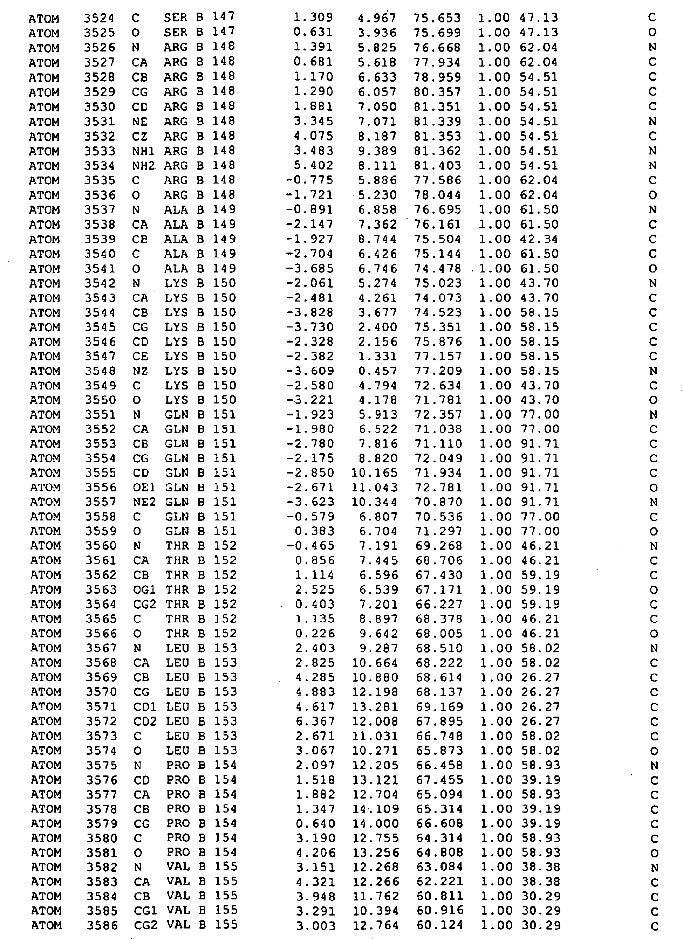

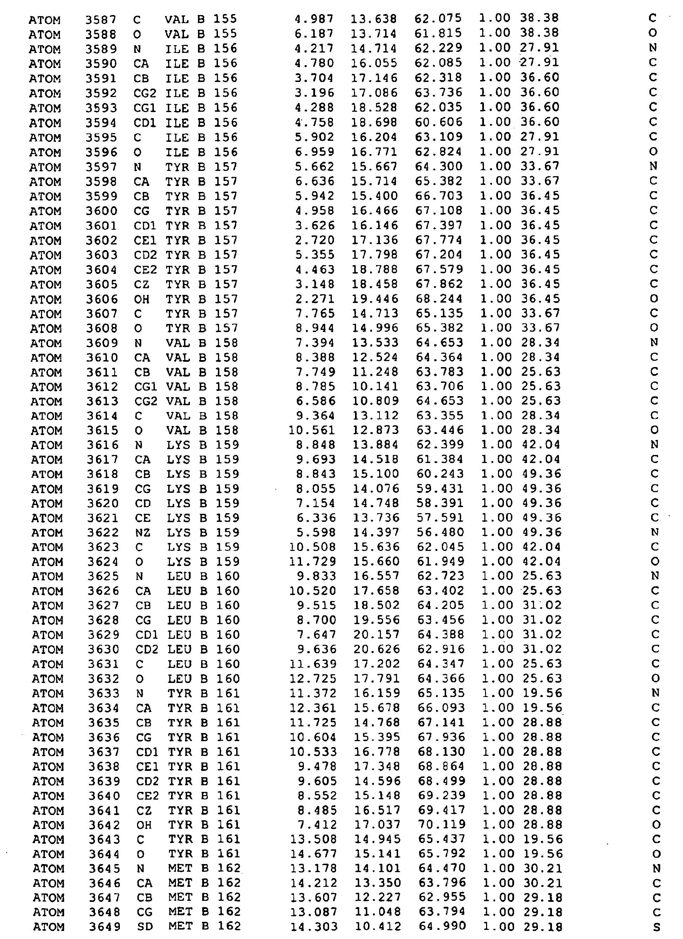

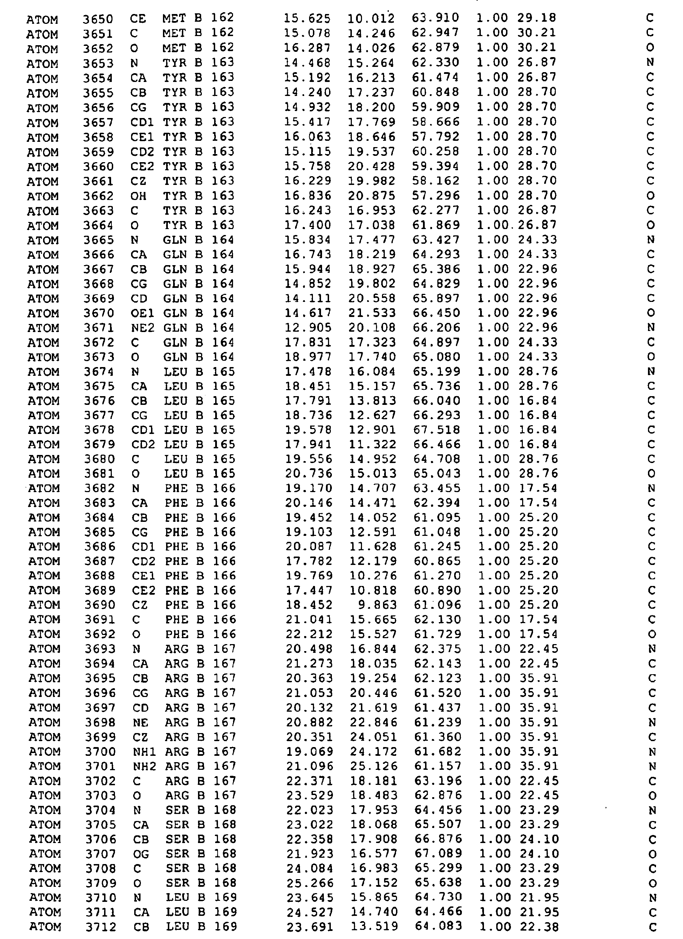

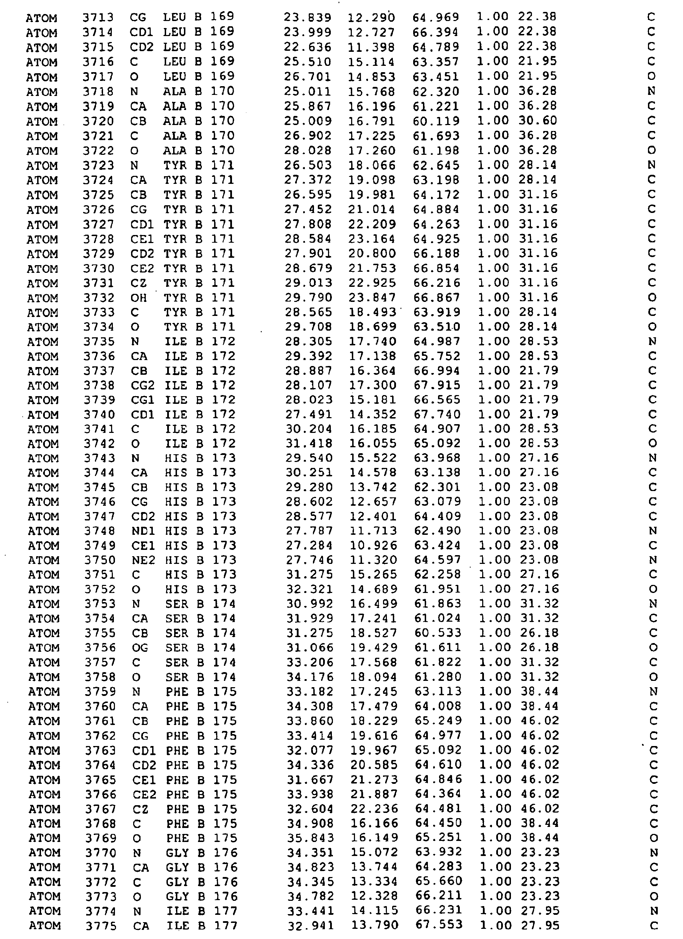

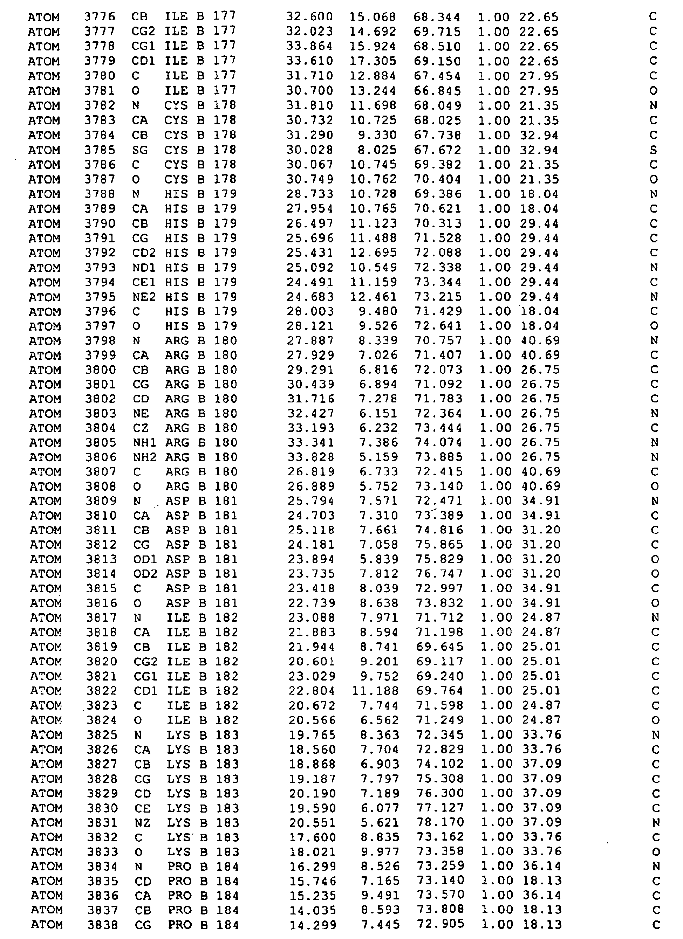

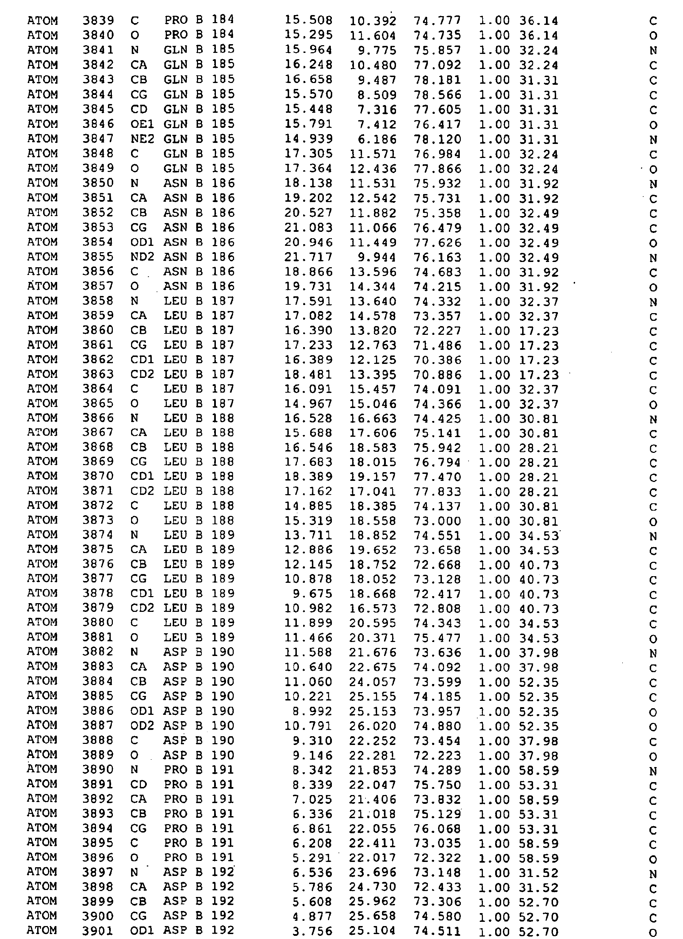

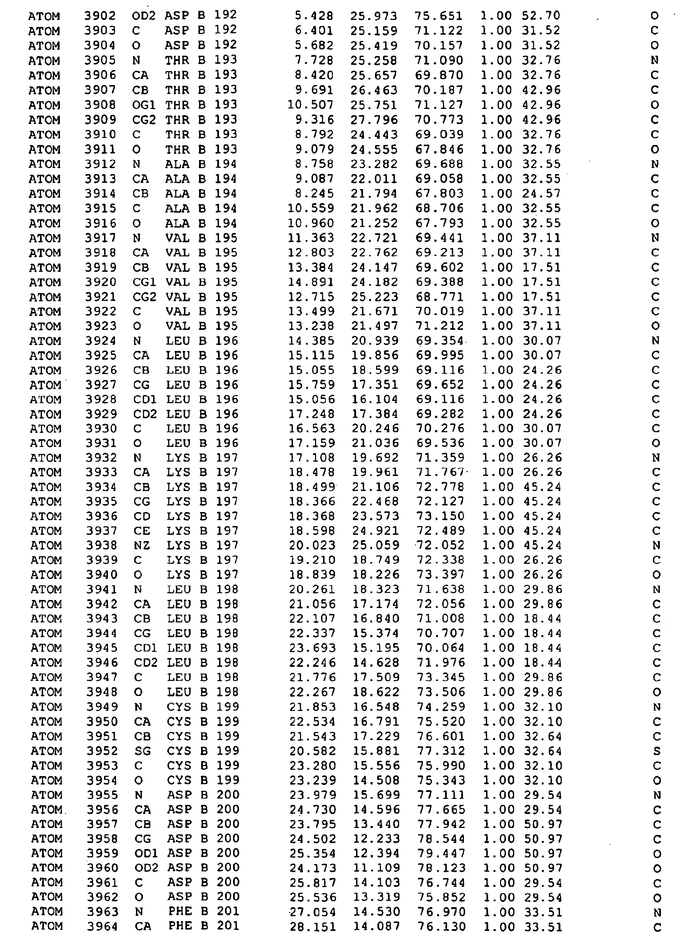

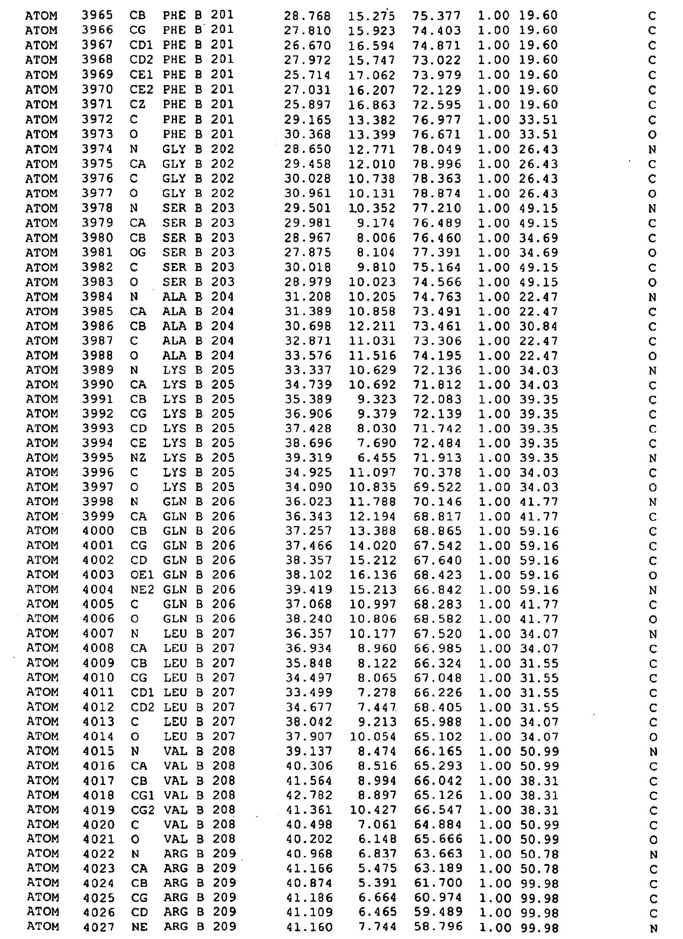

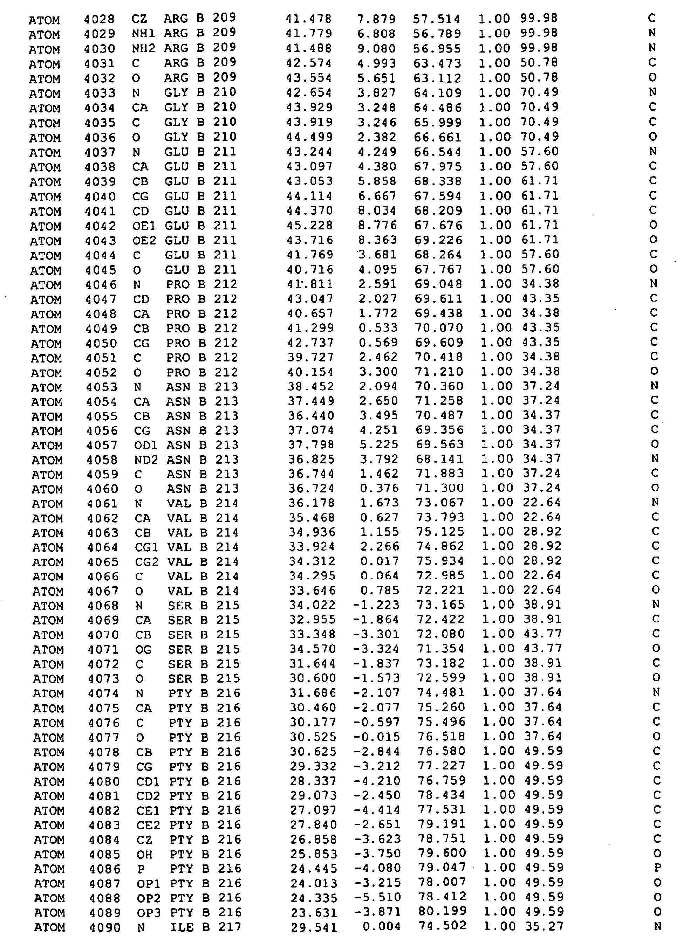

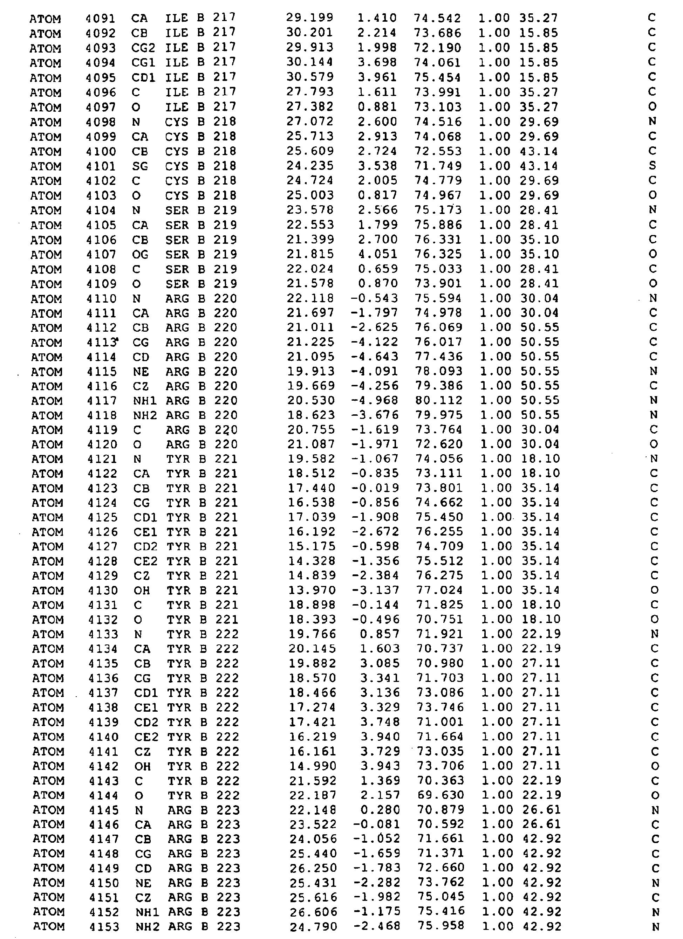

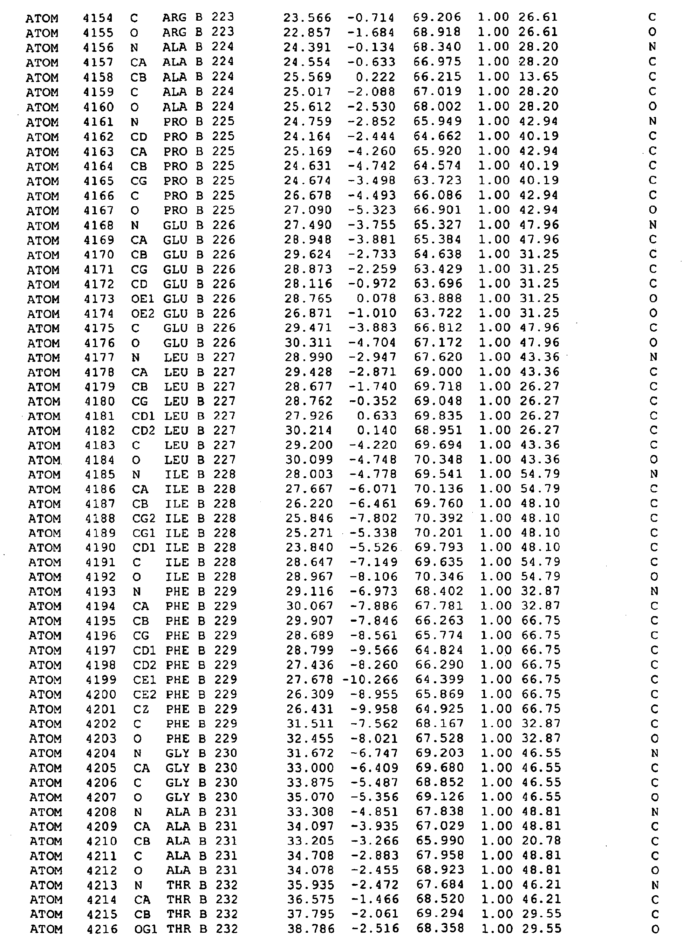

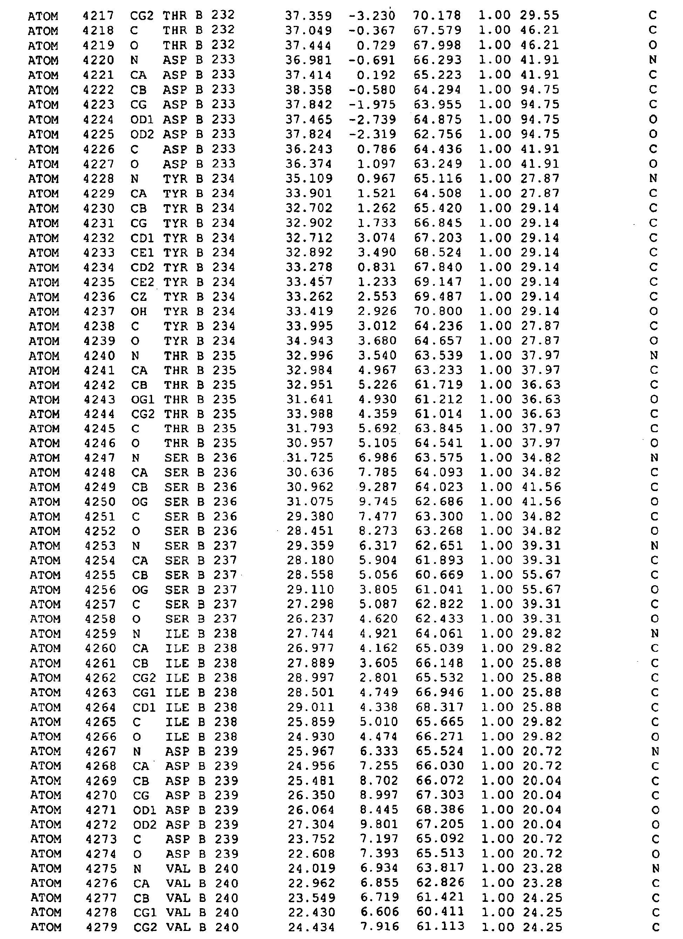

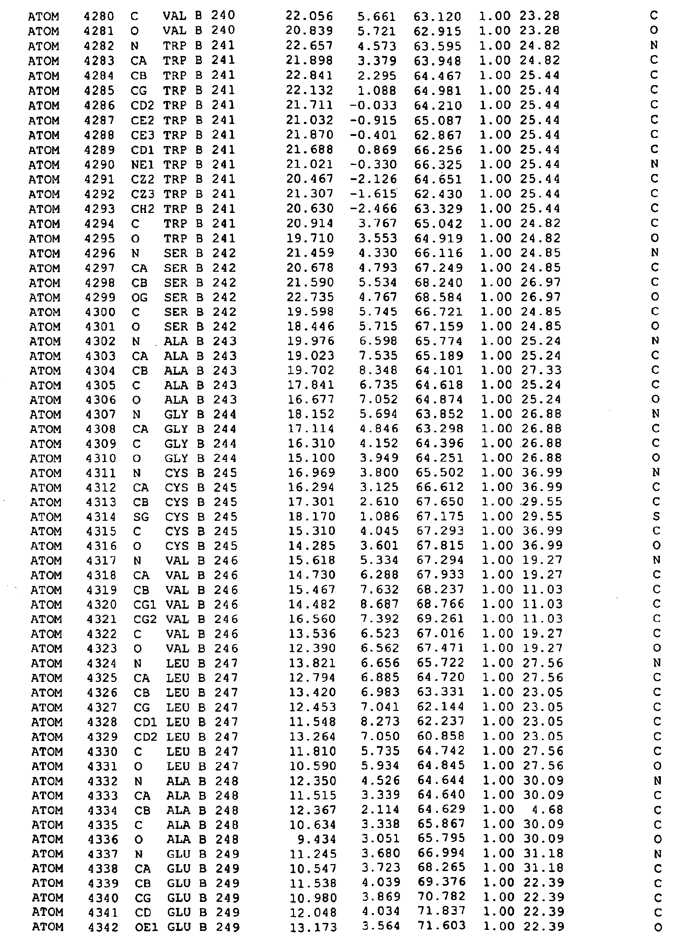

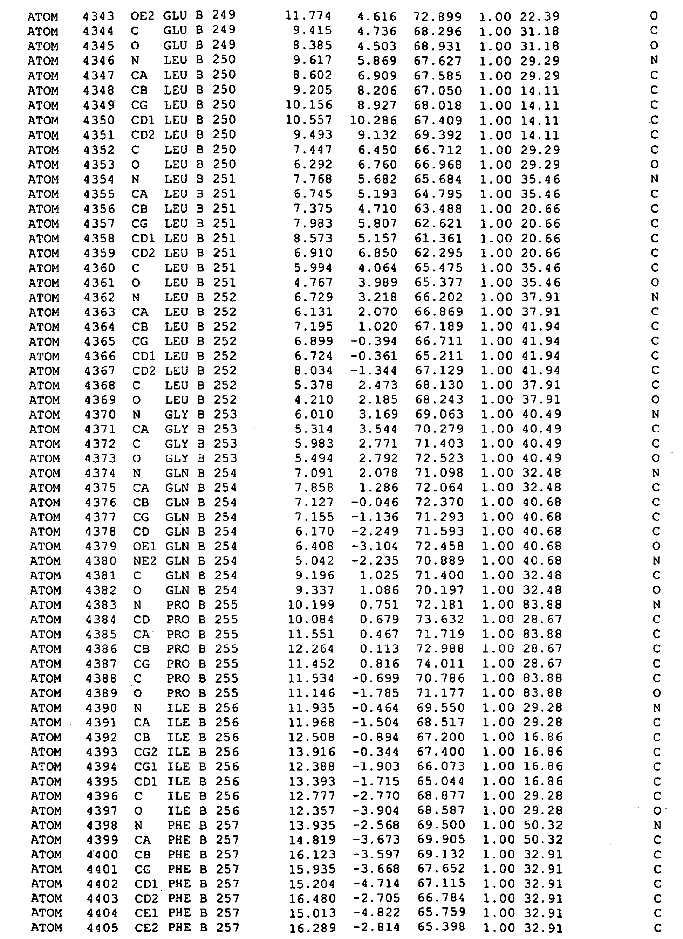

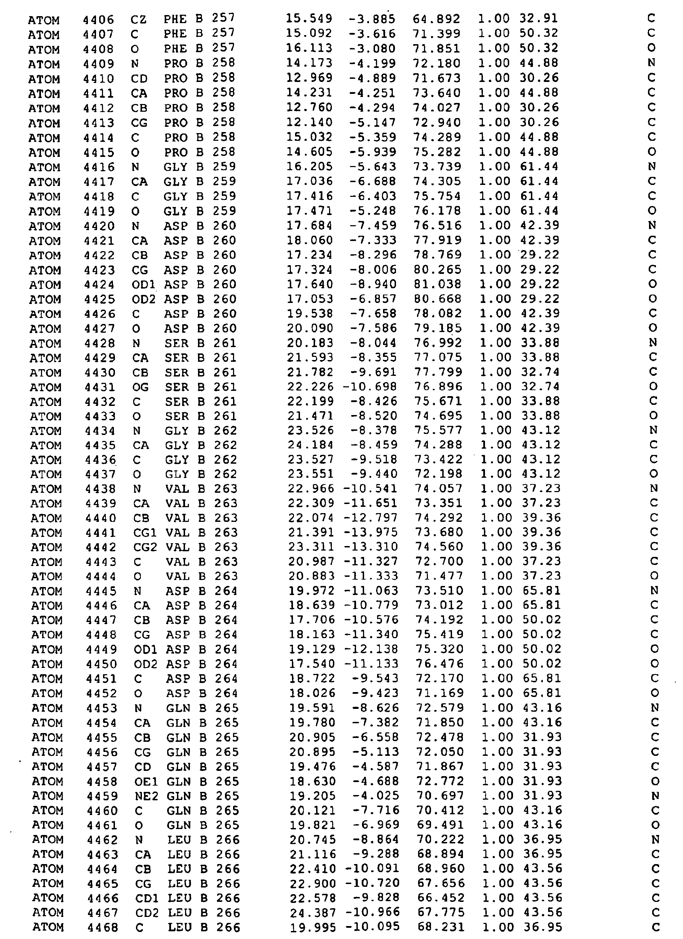

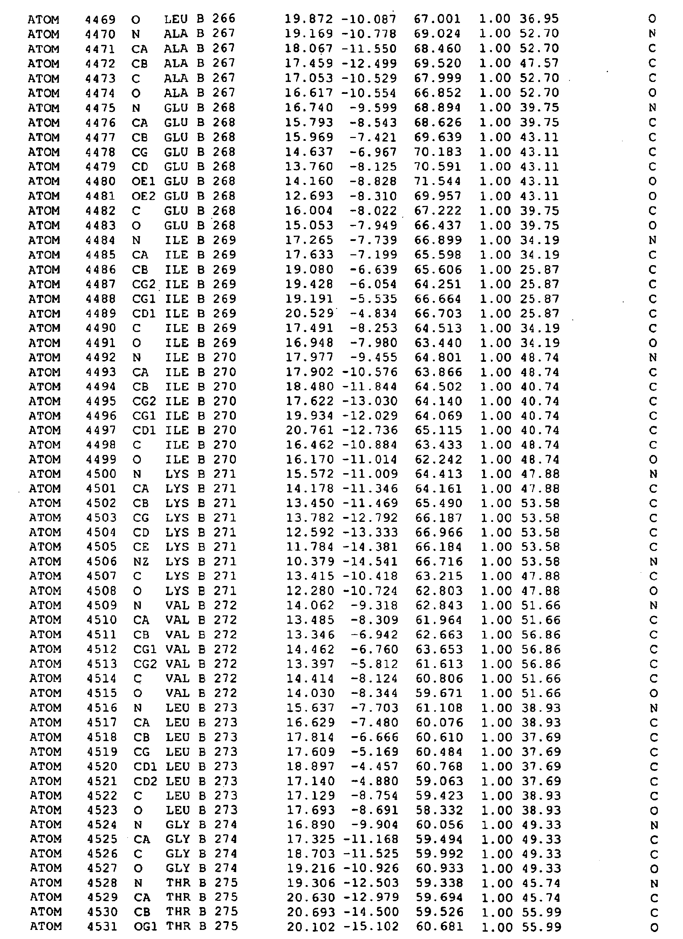

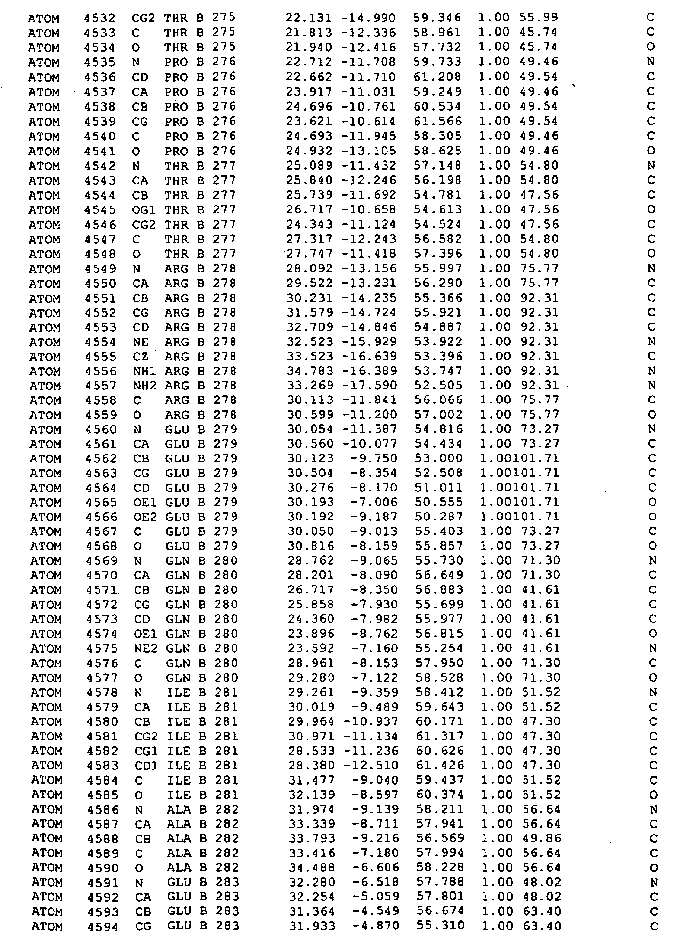

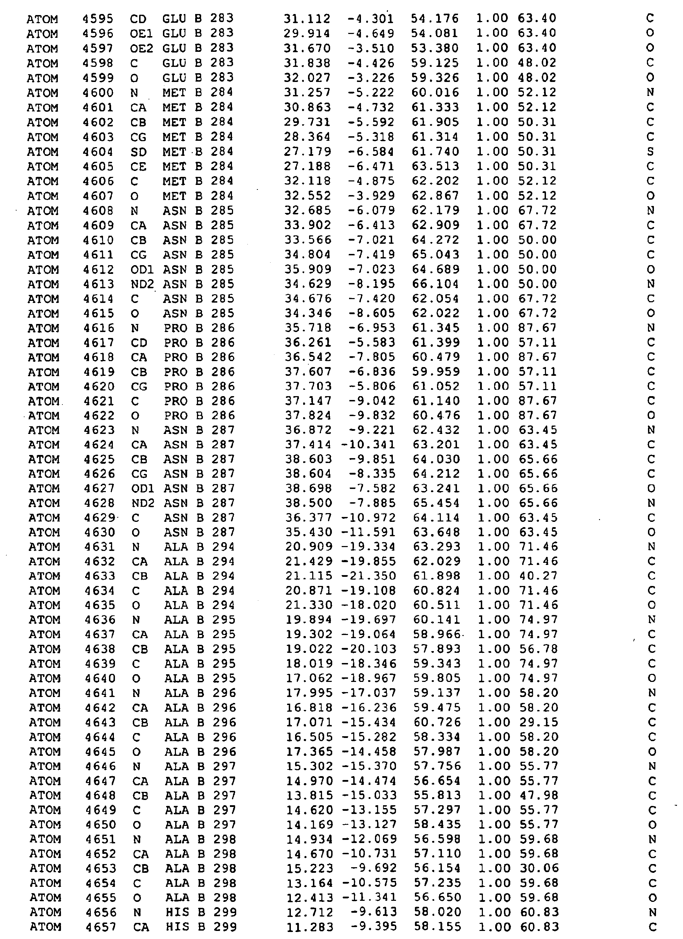

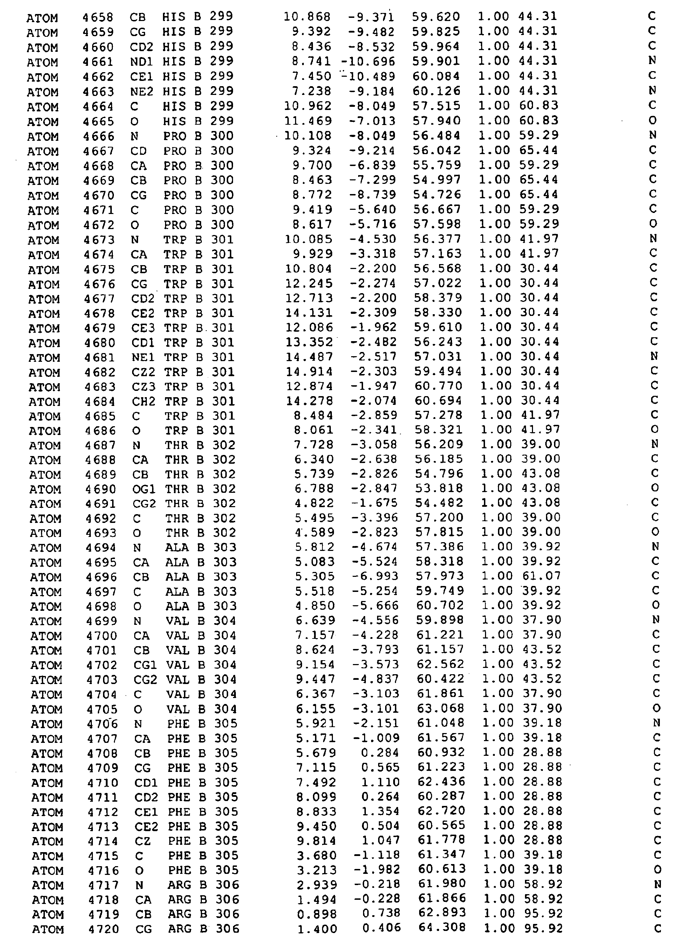

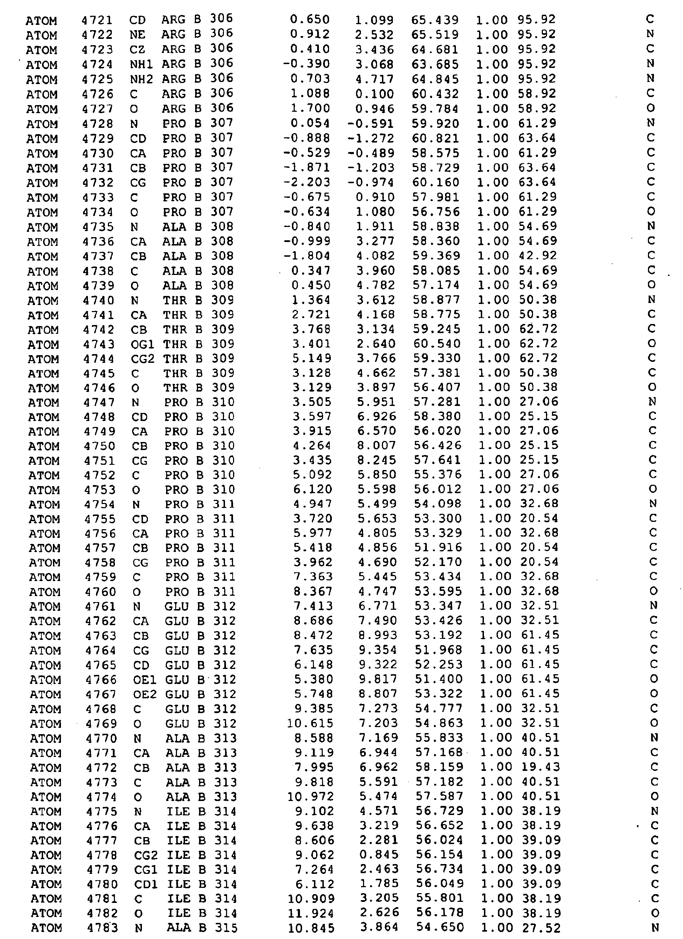

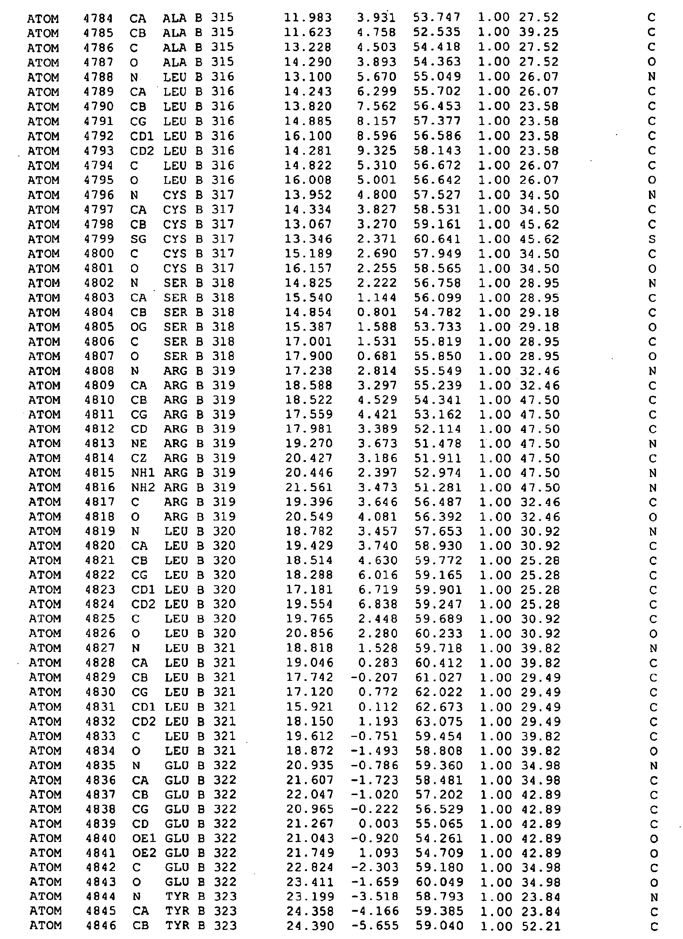

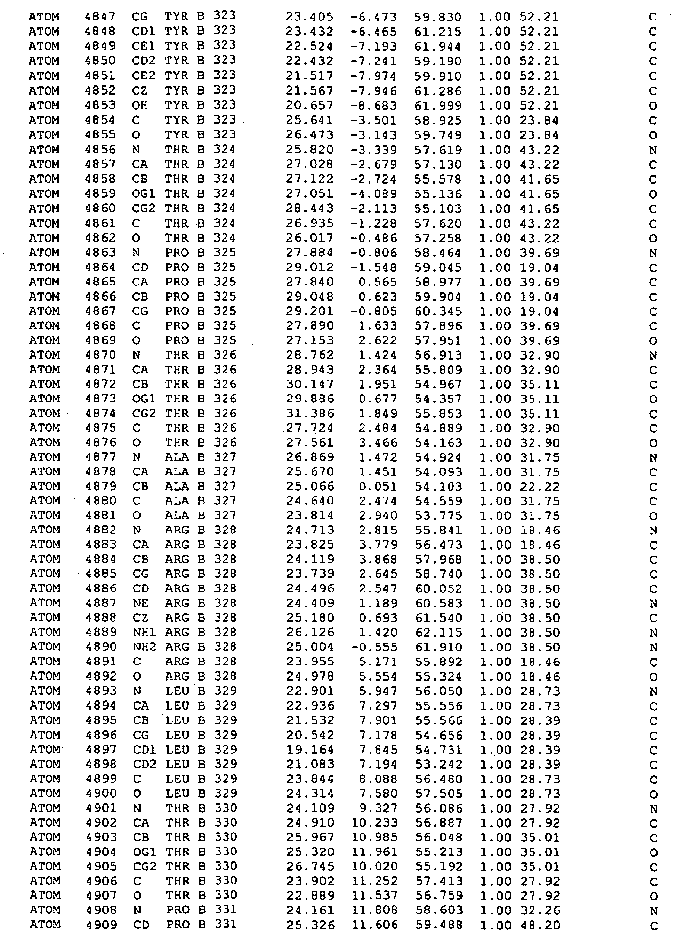

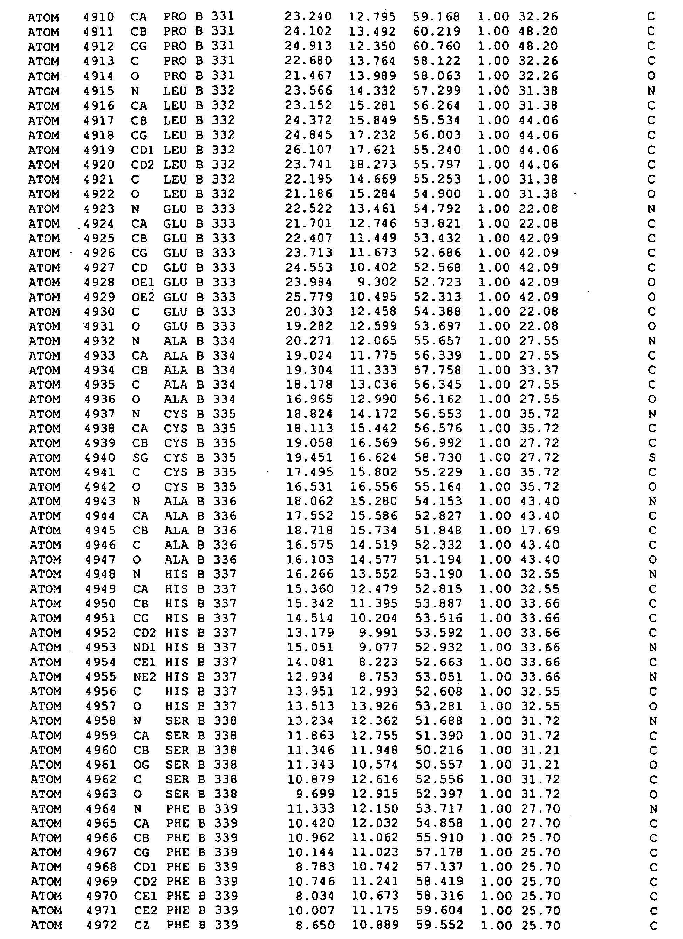

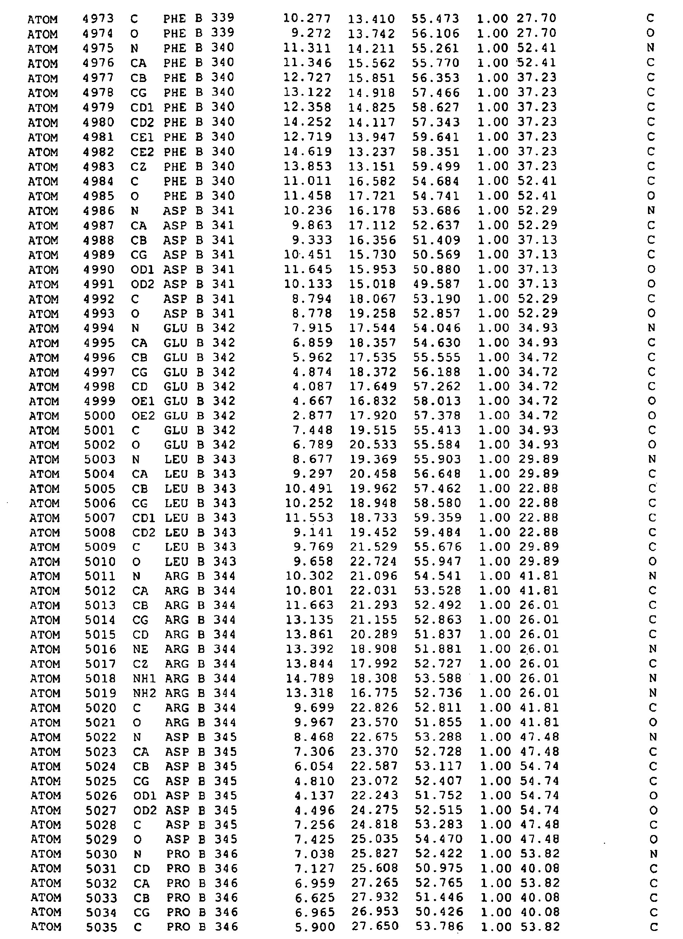

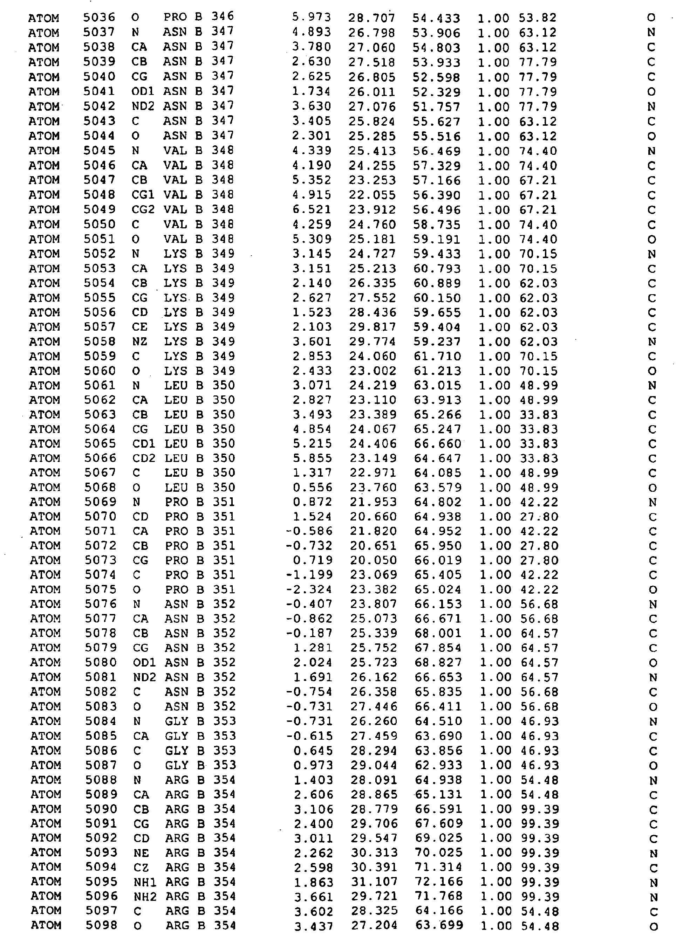

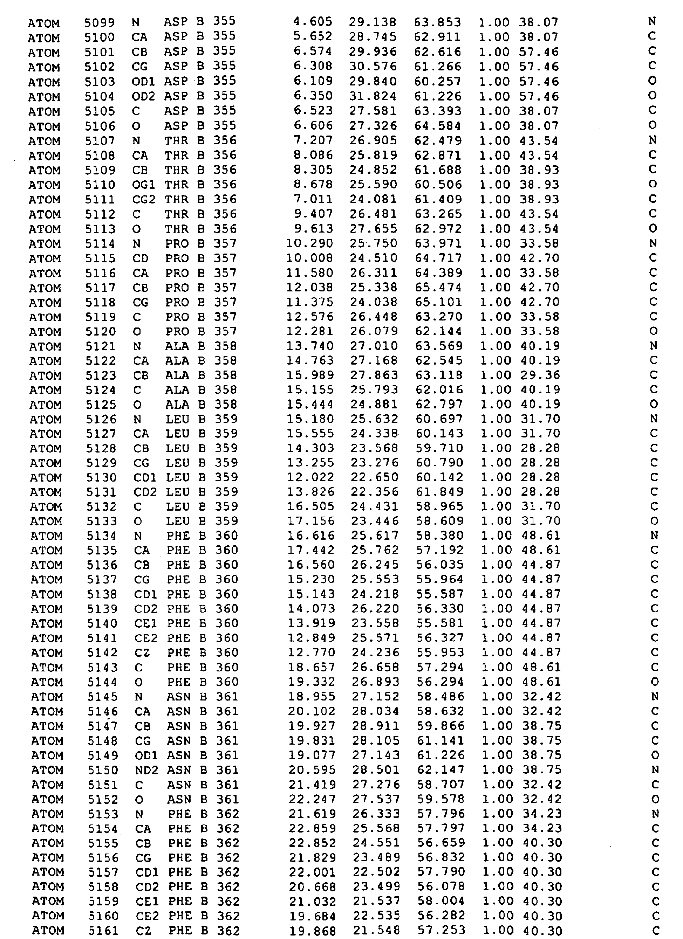

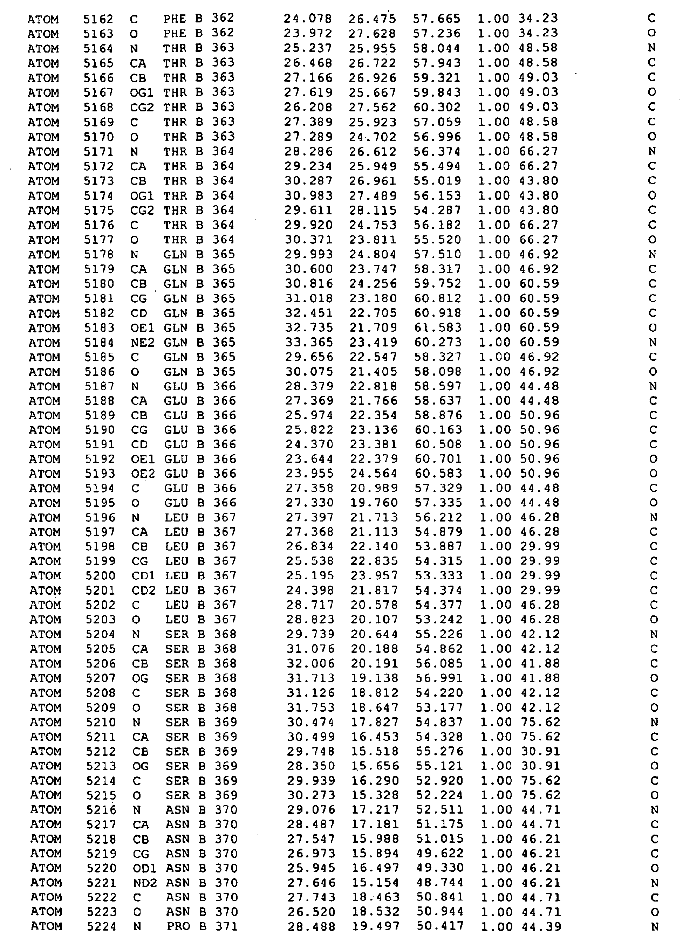

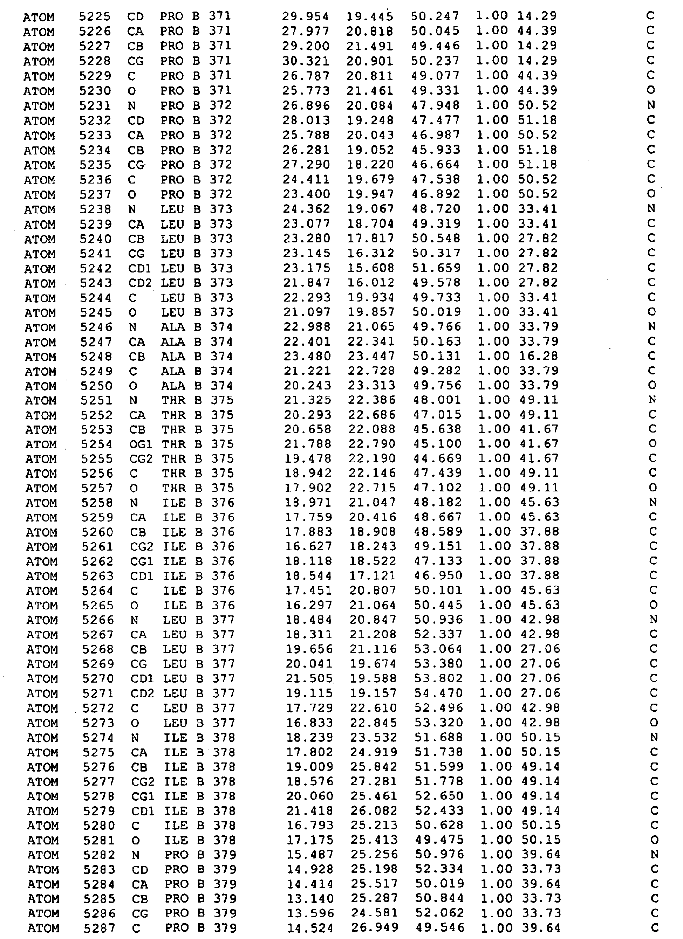

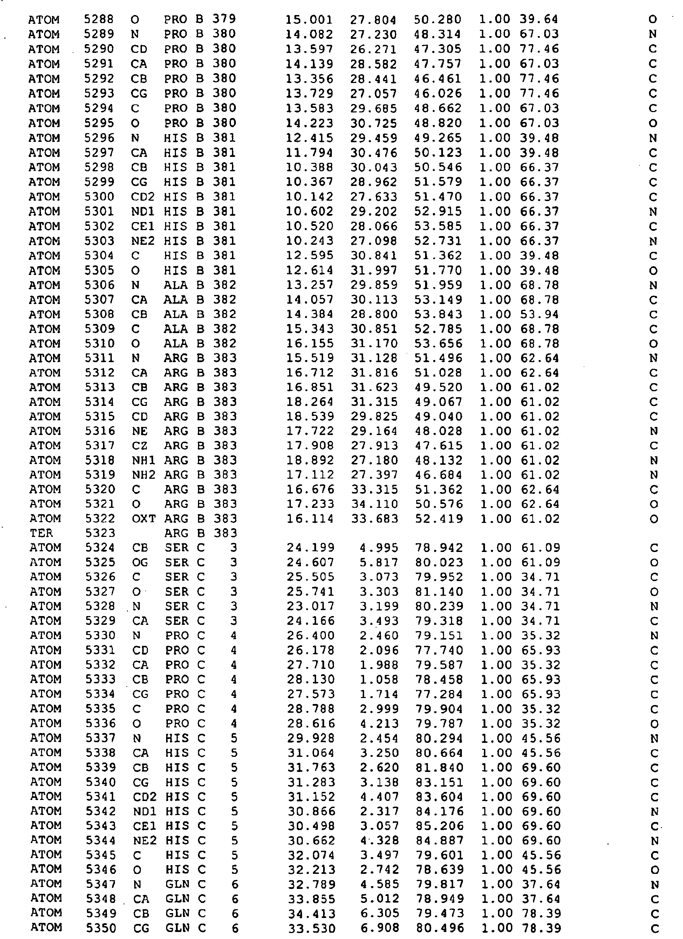

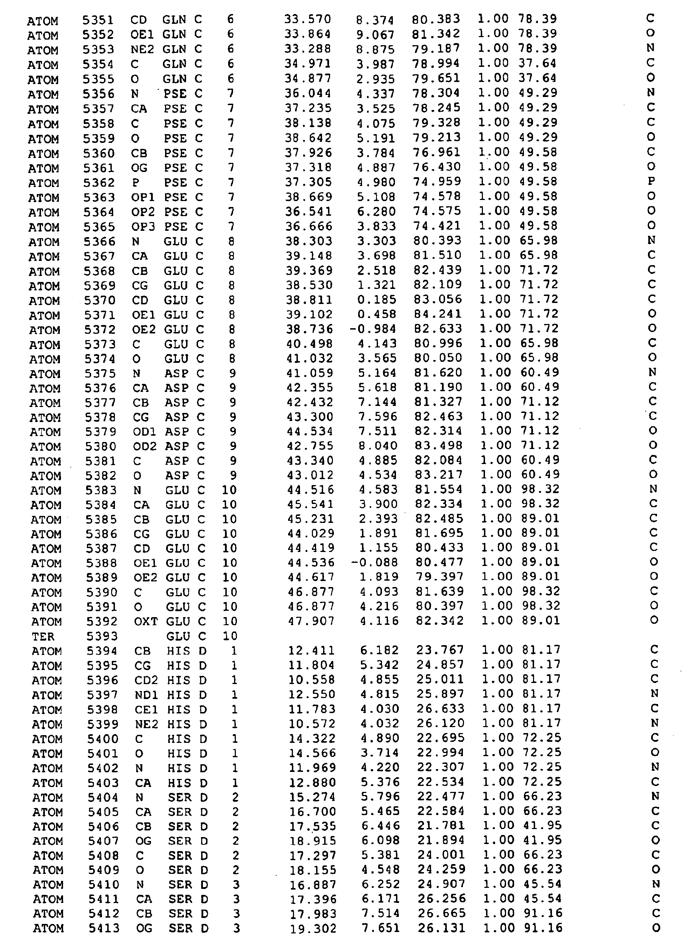

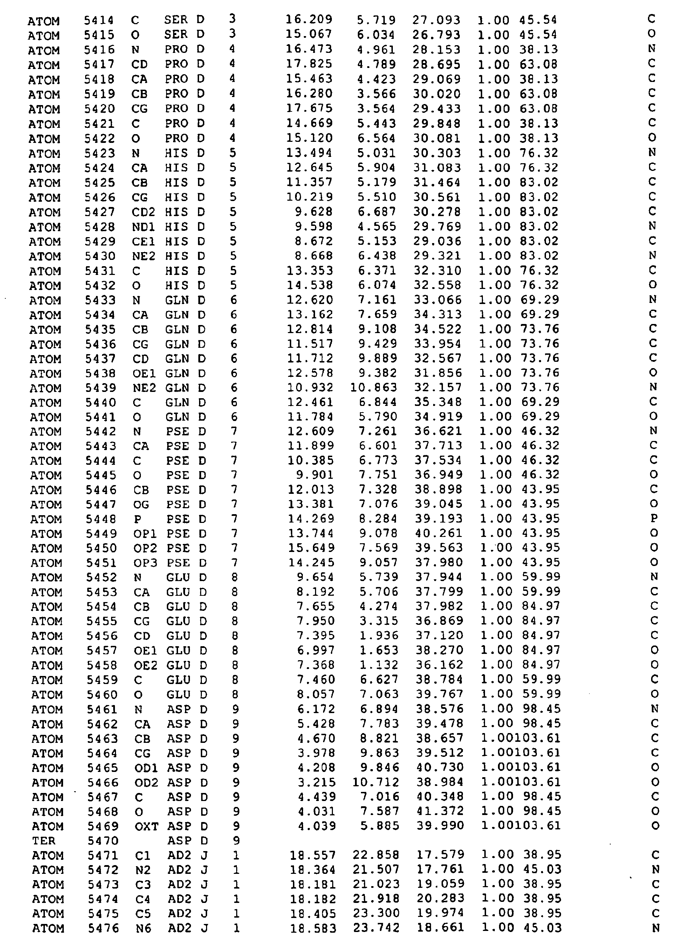

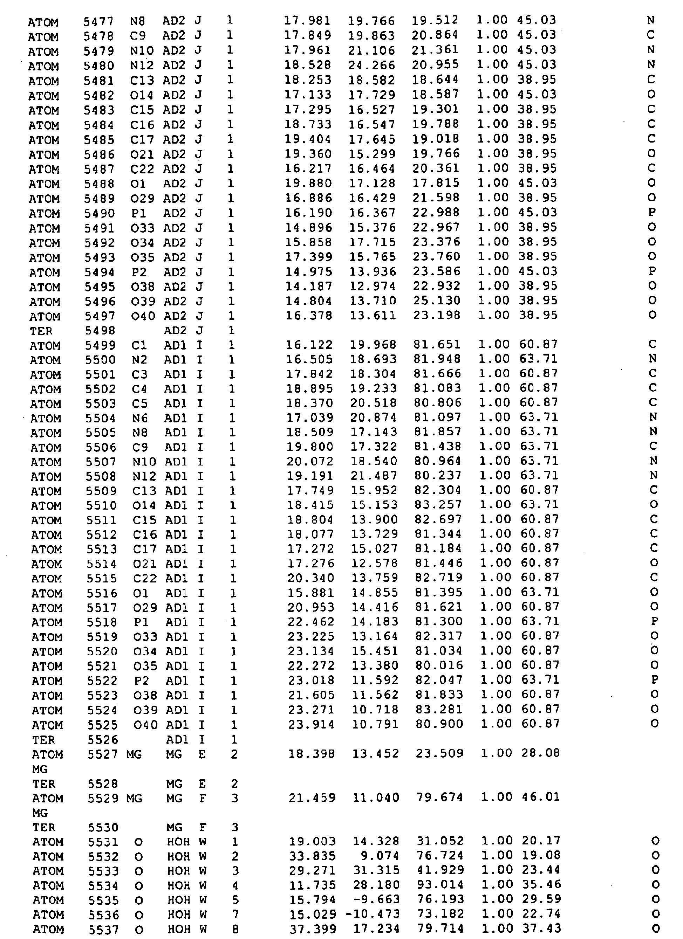

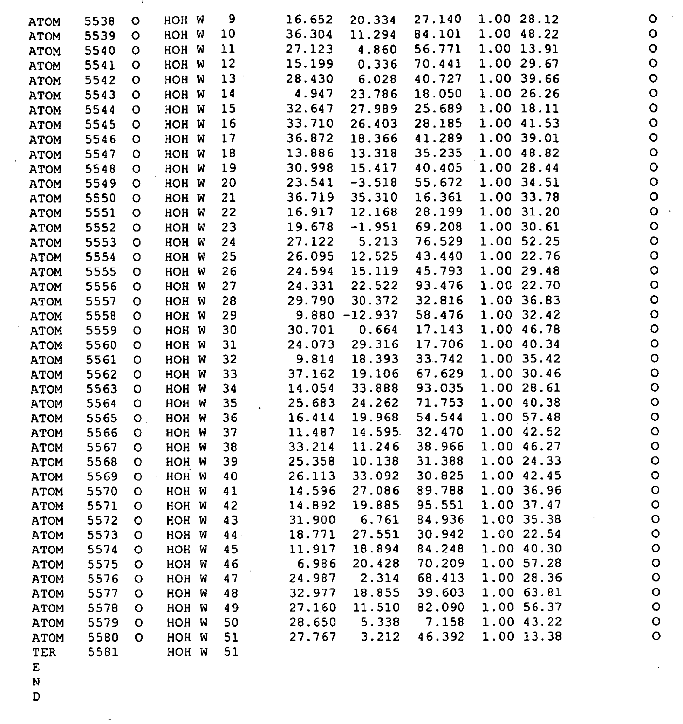

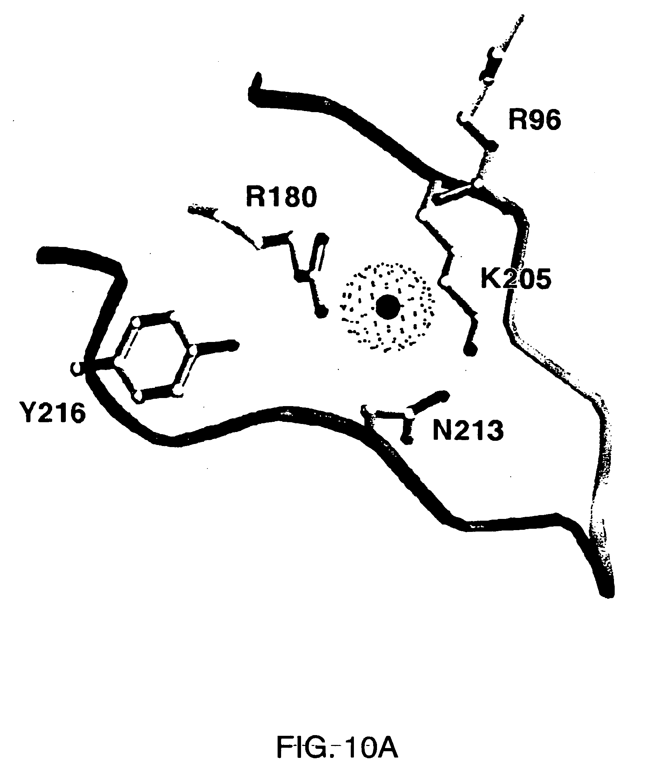

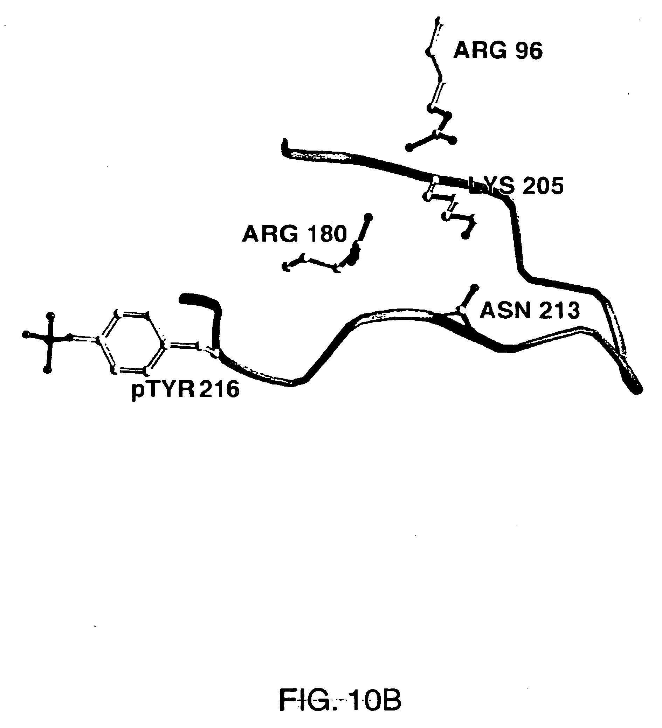

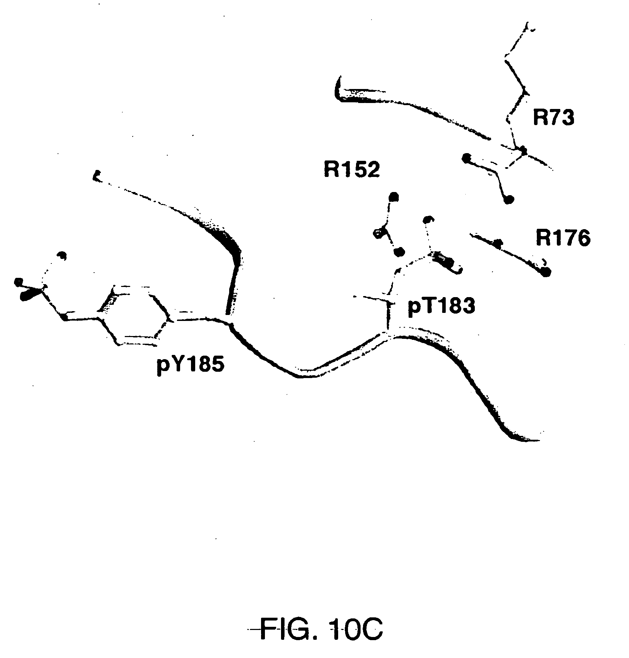

Figure 10 shows a comparison between the activation loops of unphosphorylated GSK-3β (Figure 10A), phosphorylated GSK-3β (Figure 10B), and phosphorylated p38γ (Protein Data Bank accession number 1CM8)(Figure 7C). In Figure 10A, the side chains of residues R96, R180 and K205 are pointing to a phosphate ion that occupies the same position as the phosphate group of the phospho-threonine in activated p38γ (Figure 10C), activated CDK2 and activated ERK2. In Figure 10C, the phosphorylated Y216 is flipped out of the substrate binding groove, which is similar to the position of the phosphorylated Y185 of p38γ.

Figure 11A shows that in the unphosphorylated GSK-3β structure, the GSK-3β substrate-binding groove is occupied by a phosphate ion and residues 260 to 264 of a neighboring GSK-3β molecule.

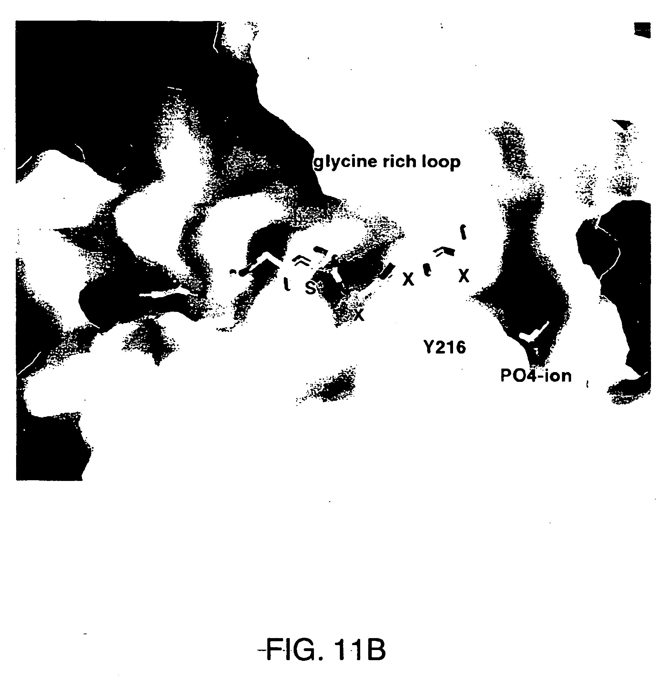

Figure 11B shows a model for the binding of the SXXXpS motif in the GSK-3β substrate-binding groove. The α-helical domains of GSK-3β and activated substrate bound CDK2 (Protein Data Bank accession number 1QMZ) were superimposed to model the positioning of a primed peptide in the GSK-3β substrate-binding groove. The side chains of the peptide residues except for the target serine S* have been removed for clarity. The phosphorylation site S* is positioned in front of the active site. The model estimates how the SXXXpS motif fits in the substrate-binding groove with the three residues bridging the gap between the P and P+4 serines. The phosphate group of the P+4 serine will occupy the same position as the phosphate ion found in the crystal structure.

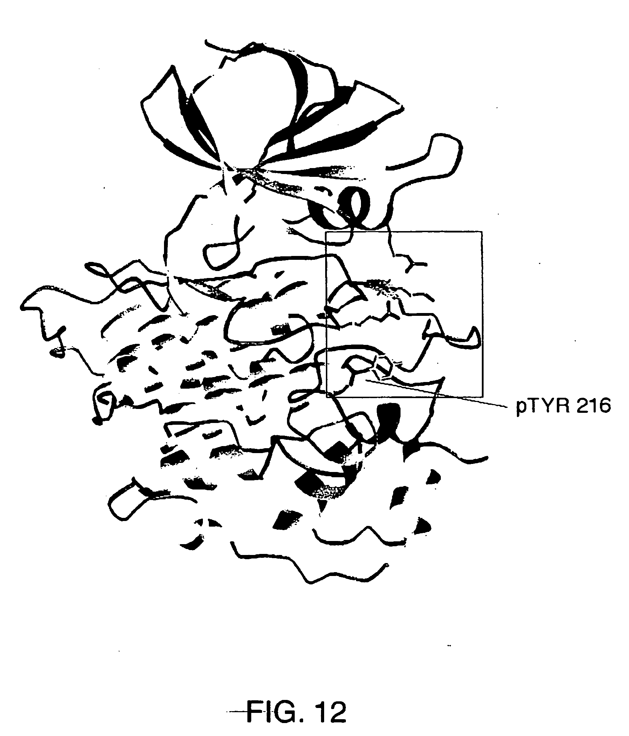

Figure 12 depicts a ribbon diagram of phosphorylated GSK-3β. The activation loop and the phosphorylated Y216 are indicated.

Figure 13 presents the inhibitor2 bound in the active site of unphosphorylated GSK-3β. The hinge and glycine rich loop are indicated.

Figure 14 presents the view of inhibitor1 bound in the active site of phosphorylated GSK-3β.

Figure 15 presents the view of inhibitor3 bound in the active site of unphosphorylated GSK-3β.

Figure 16 presents the conformation of Gln185 in the active site when bound to inhibitor4.

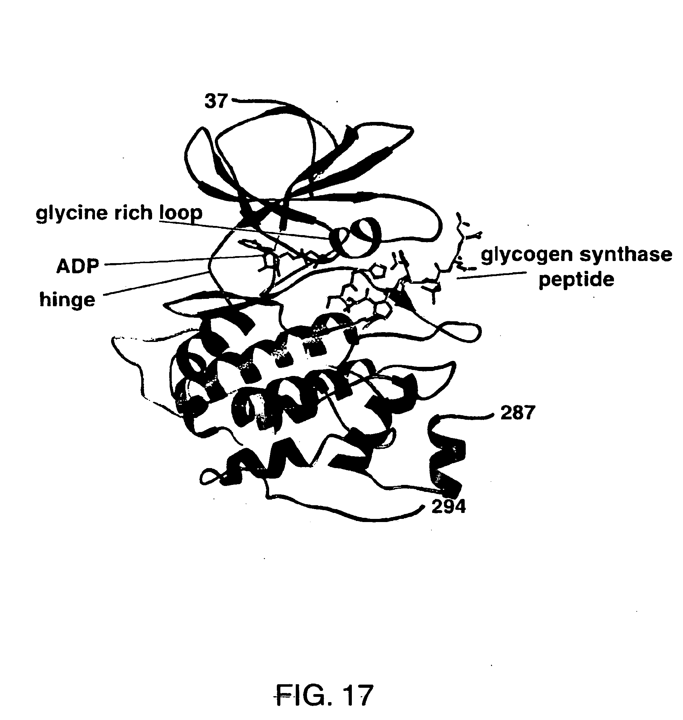

Figure 17 depicts the overall structure of phosphorylated GSK-3β in complex with ADP and glycogen synthase peptide. The N-terminal domain corresponds to the β-strand domain and includes residues 37 to 138. The α-helical domain of the GSK-3β kinase core corresponds to residues 139 to 343. The C-terminal 34 residues are not part of the kinase core but pack against the α-helical domain. Key features of the kinase fold, such as the glycine rich loop, the hinge and the activation loop are indicated. The ADP-Mg complex occupying the active site is shown. The glycogen synthase peptide (residues 650 to 658) bound in the substrate binding groove is also shown here.

Figure 18 depicts the β-strand domain rotation induced by ADP and glycogen synthase peptide. Superposition of the α-helical domains of unphosphorylated (dark shade), phosphorylated apo-GSK3 (gray shade) and phosphorylated ADP peptide bound GSK-3β (light shade). The β-strand domain of the phosphorylated GSK-3β-ADP-peptide complex, rotated 6.5 Å in comparison to unphosphorylated and phosphorylated apo-GSK-3. The loop between β-3 and α-C (residues 87 to 95) moved 13 Å to contact the glycogen synthase peptide (not shown).

Figure 19A depicts a stereo view of the active site occupied by the ADP-Mg complex. The adenine base is surrounded by hydrophobic residues and makes two hydrogen bonds with the backbone of the hinge region. The catalytic residues involved in the phosphate transfer to the P-site serine surround the two non-transferable ADP phosphates.

Figure 19B is a schematic drawing of the hydrogen bond network that connects the ADP-Mg complex to the P-site serine via the catalytic residues.

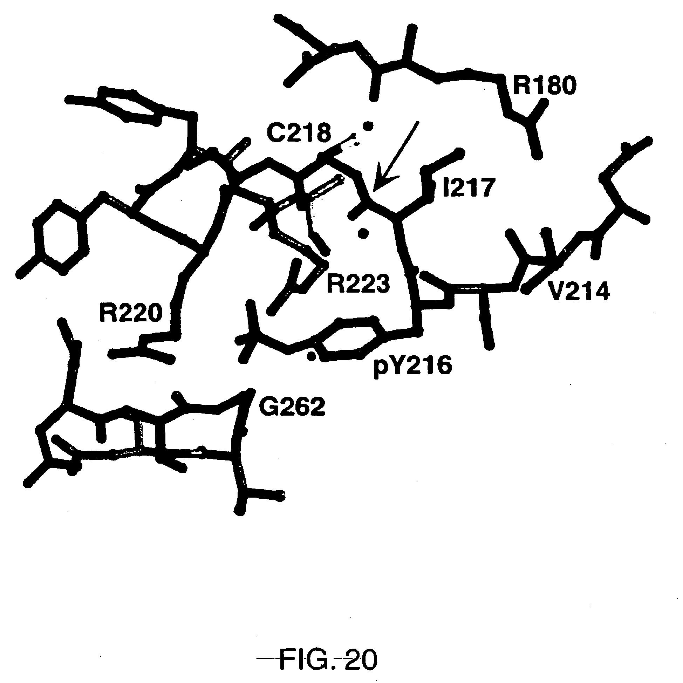

Figure 20 depicts the environment of phosphotyrosine 216 in the structure of phosphorylated GSK-3β in complex with ADP and glycogen synthase peptide. The phosphate moiety binds two arginine side chains (Arg 220 and Arg 223) resulting in charge neutralization. The arrow indicated the 180 degree flip of I217 backbone carbonyl.

Figure 21 depicts the glycogen synthase peptide in the substrate binding groove. The phosphoserine (pSer 656) binds Arg 96, Arg 180 and Lys 205, which results in the proper alignment of the α-helical and β-stand domains. pTyr 216 moves its side chain out of the substrate binding groove to make contact with the side chains of Arg 220 and Arg 223.

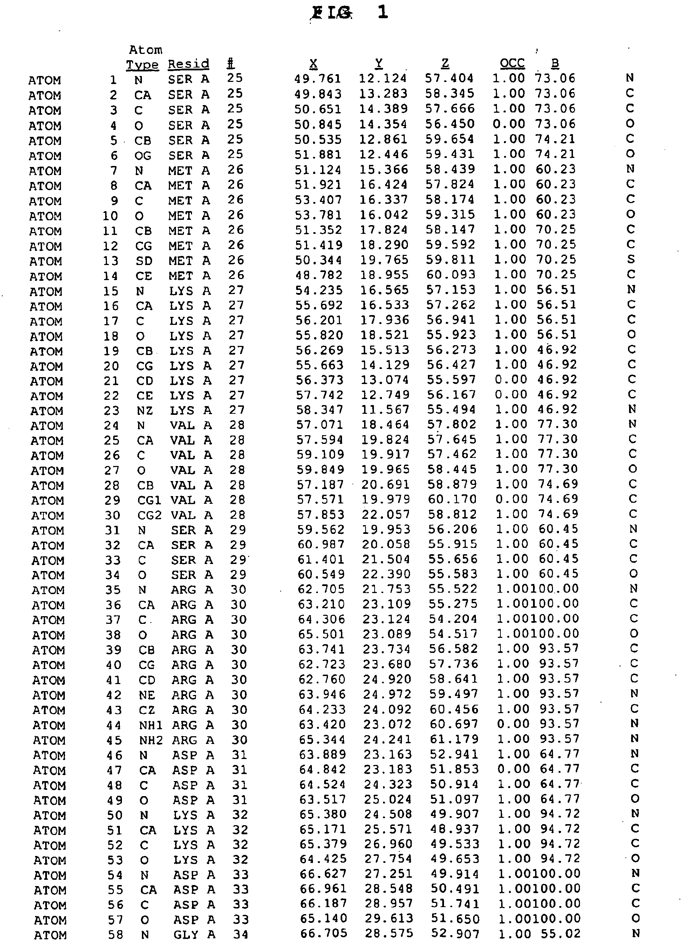

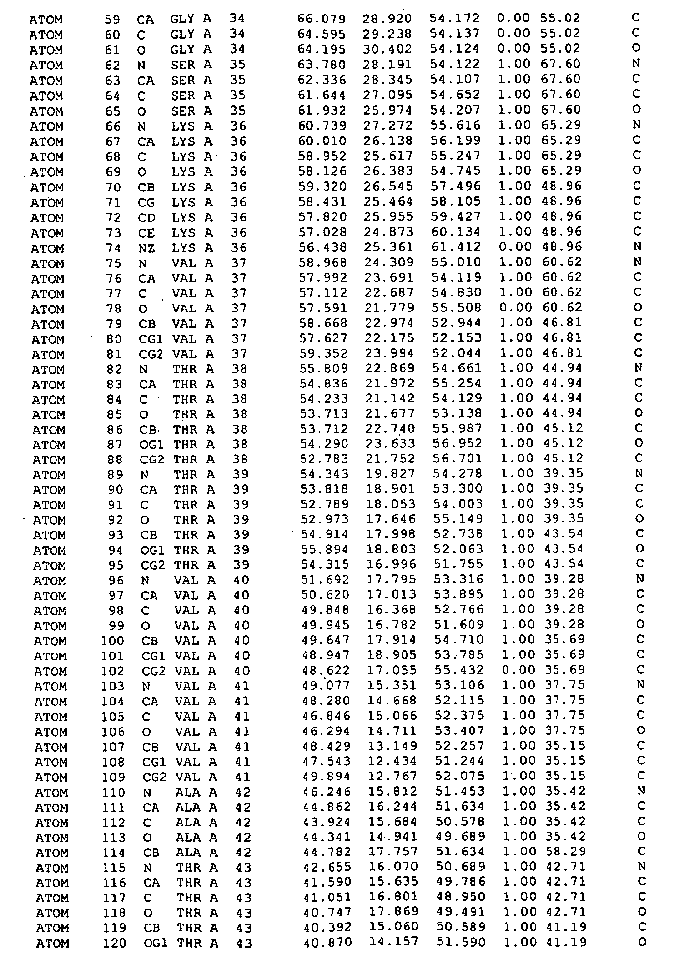

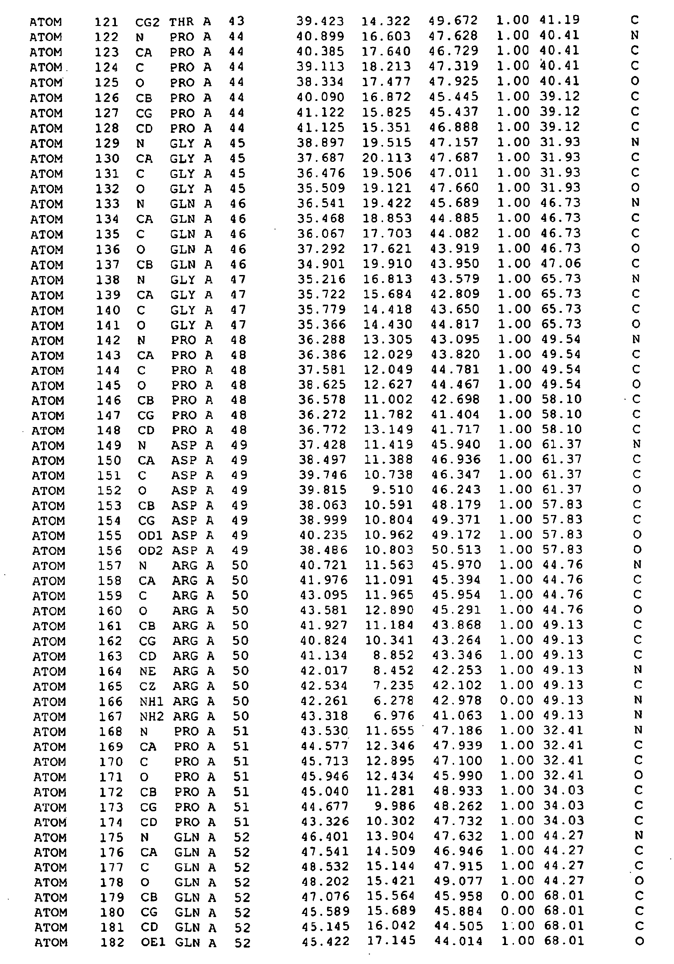

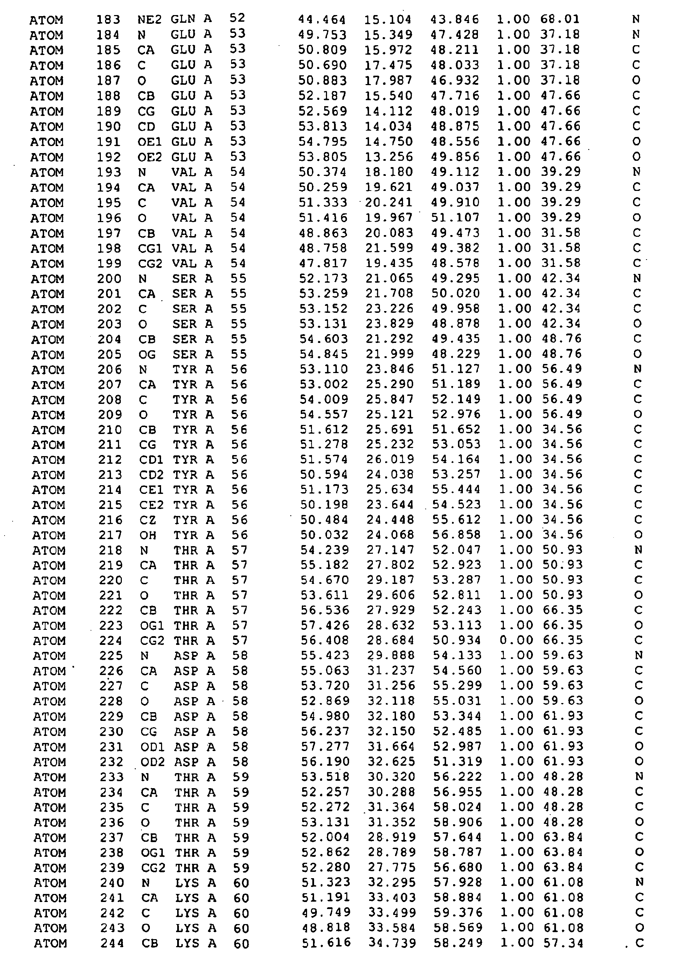

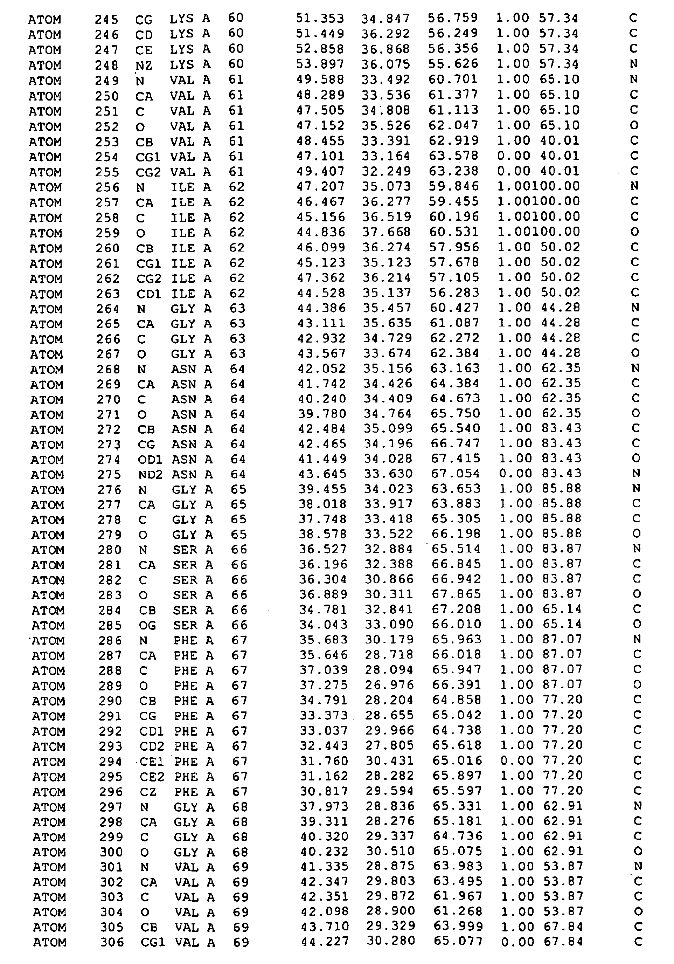

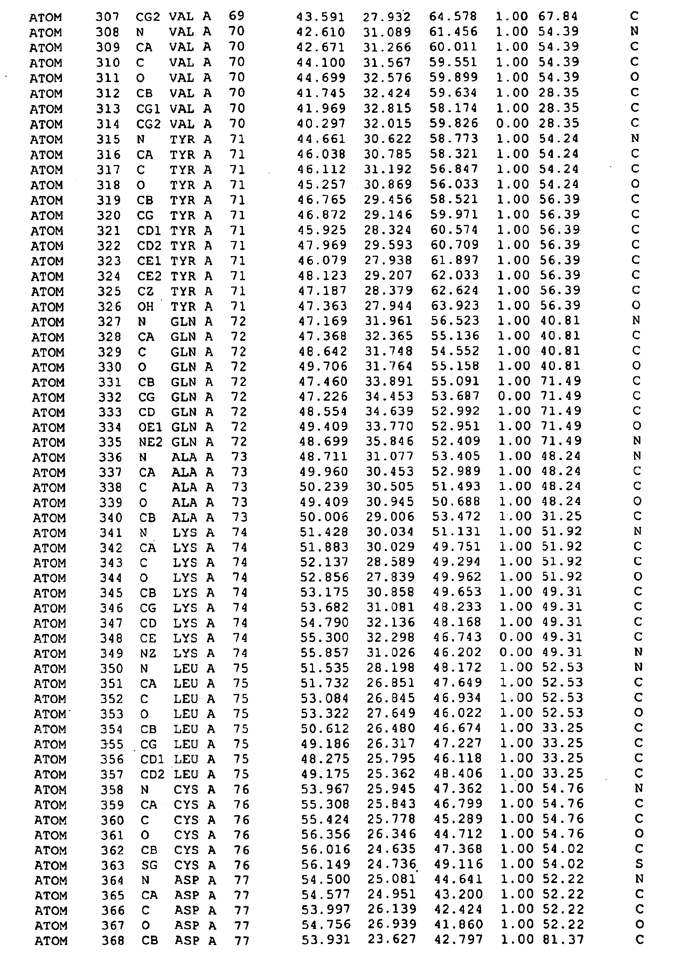

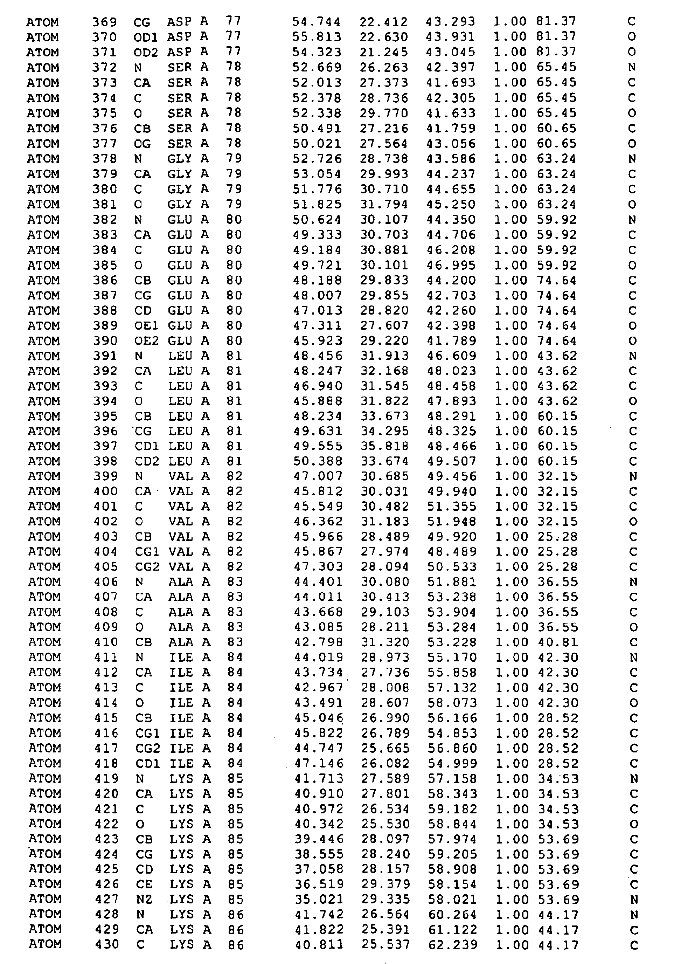

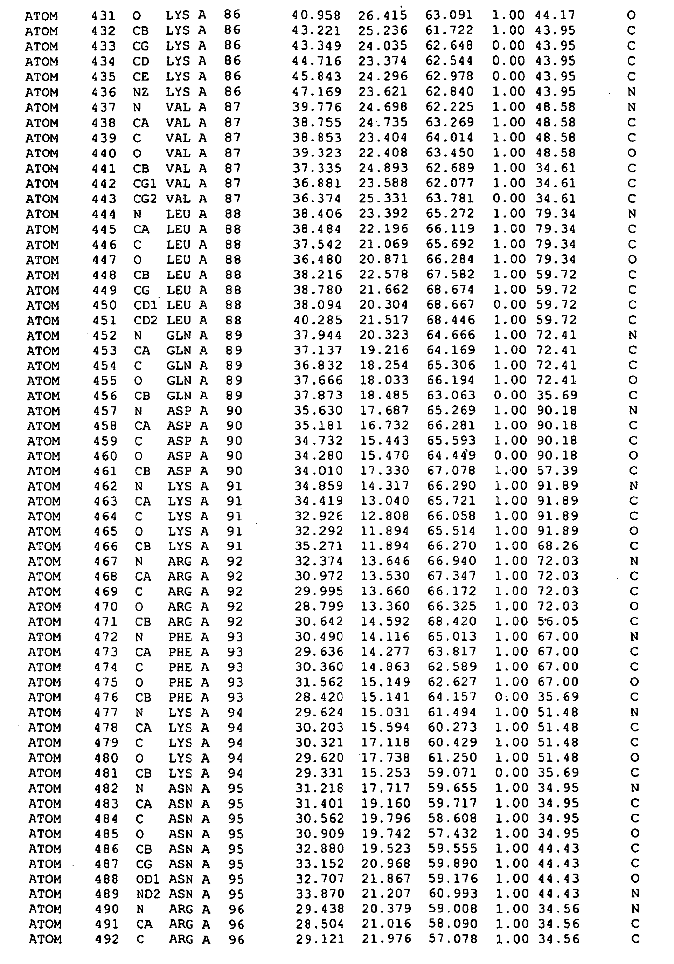

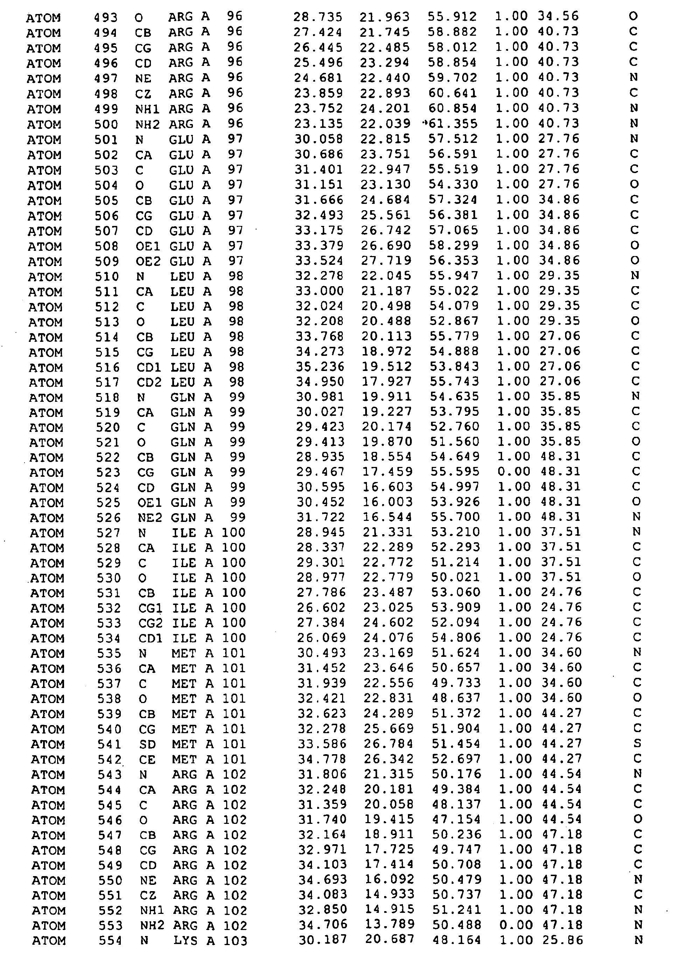

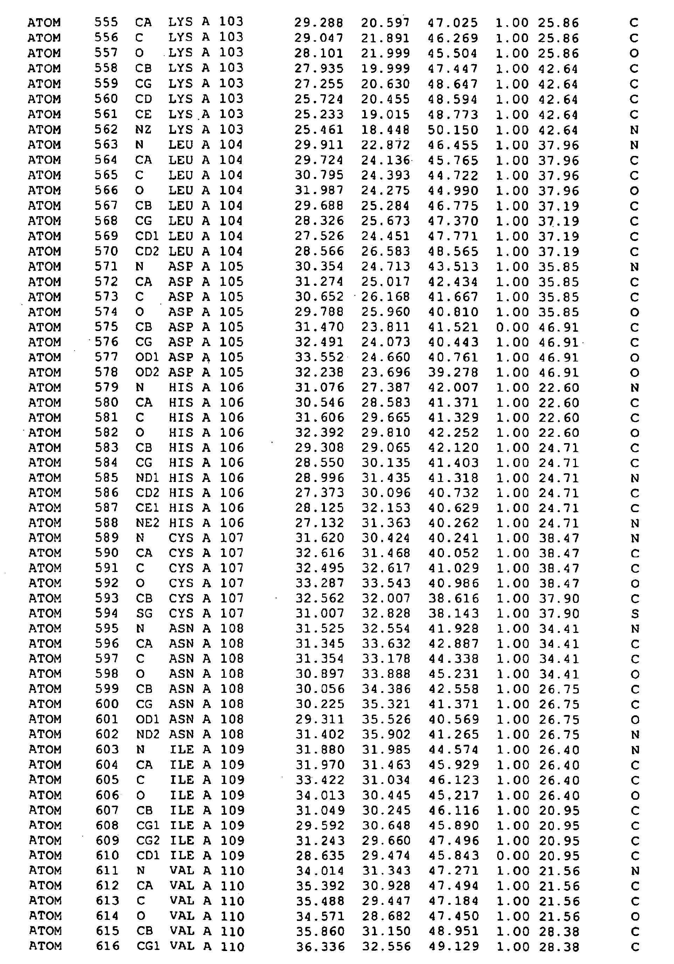

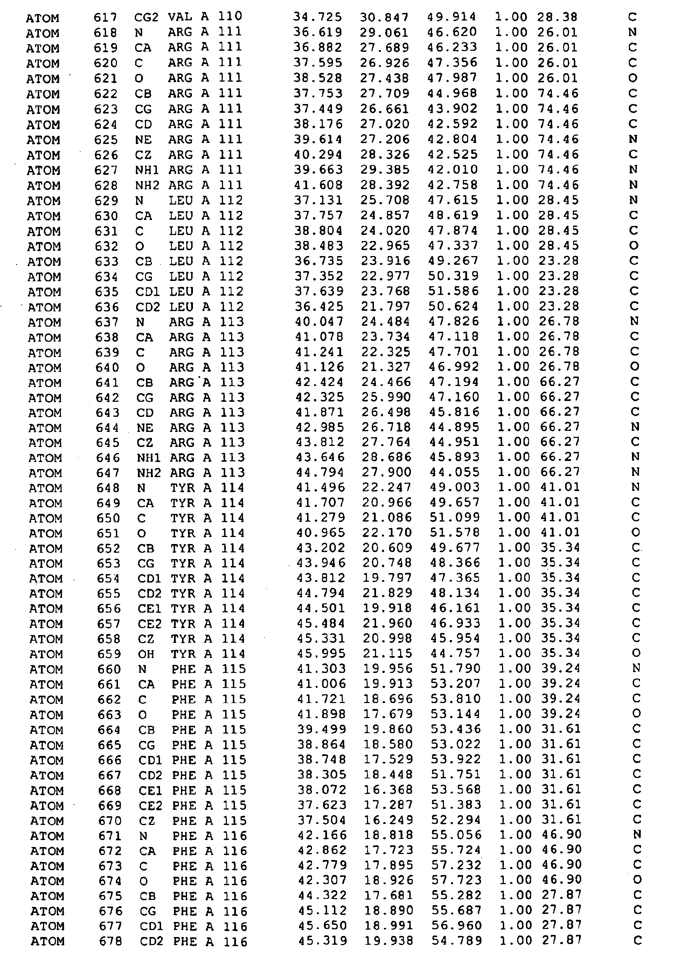

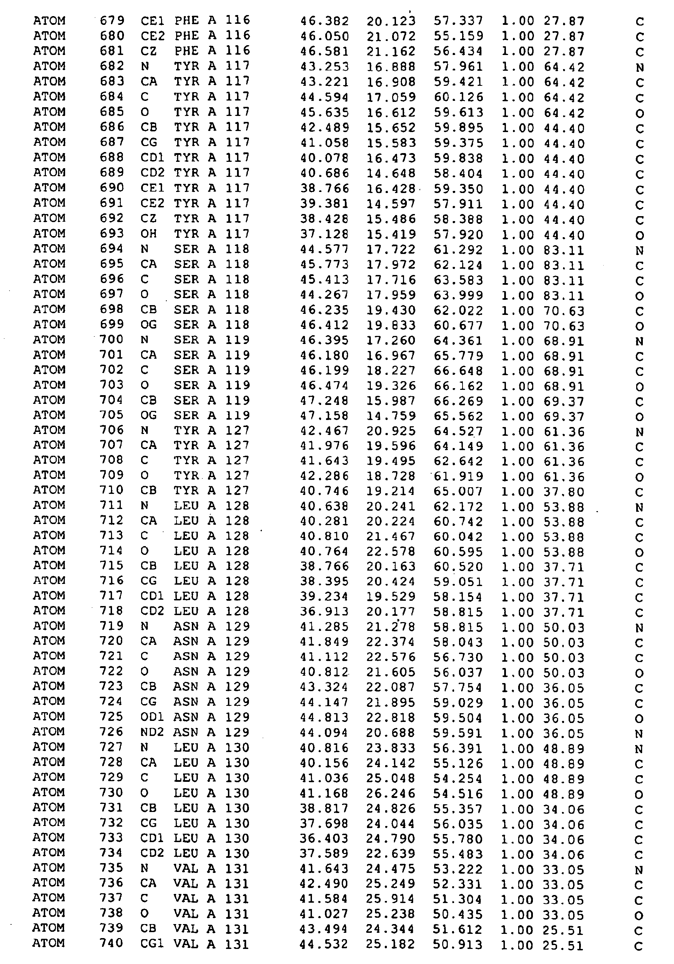

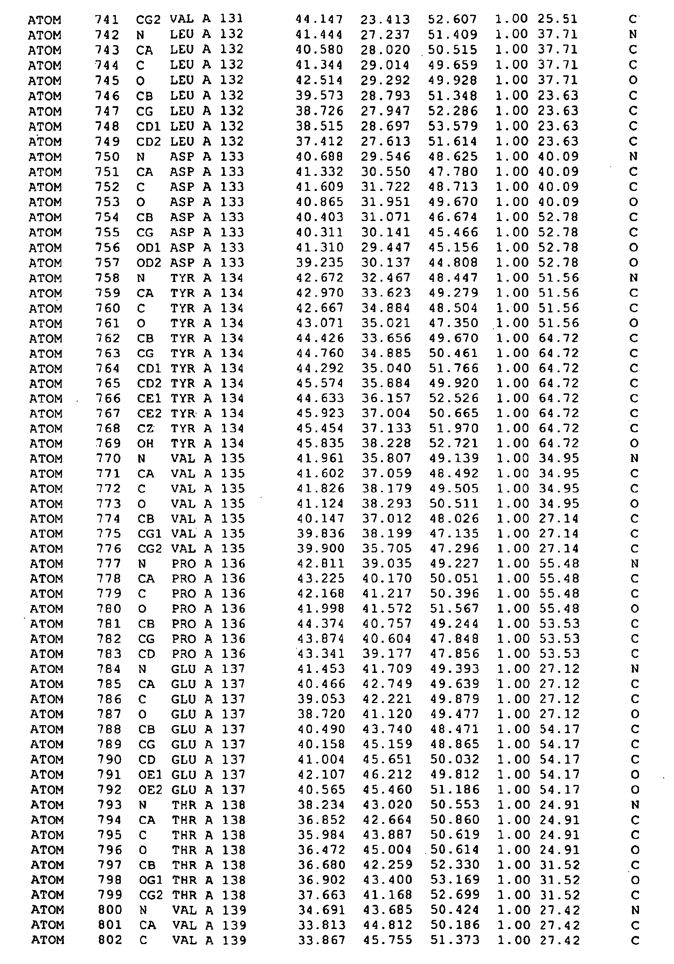

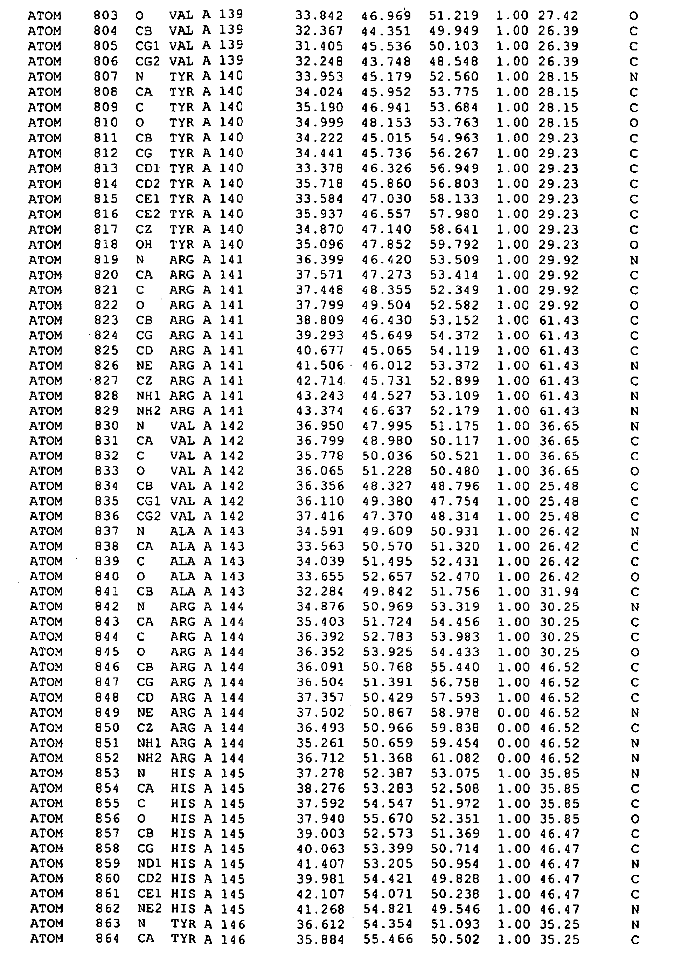

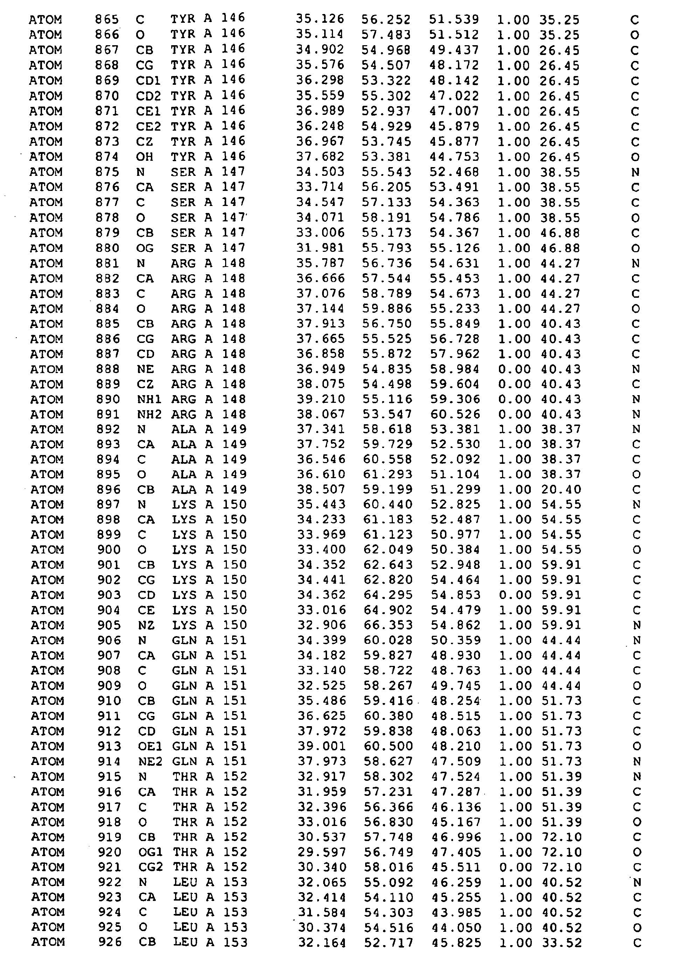

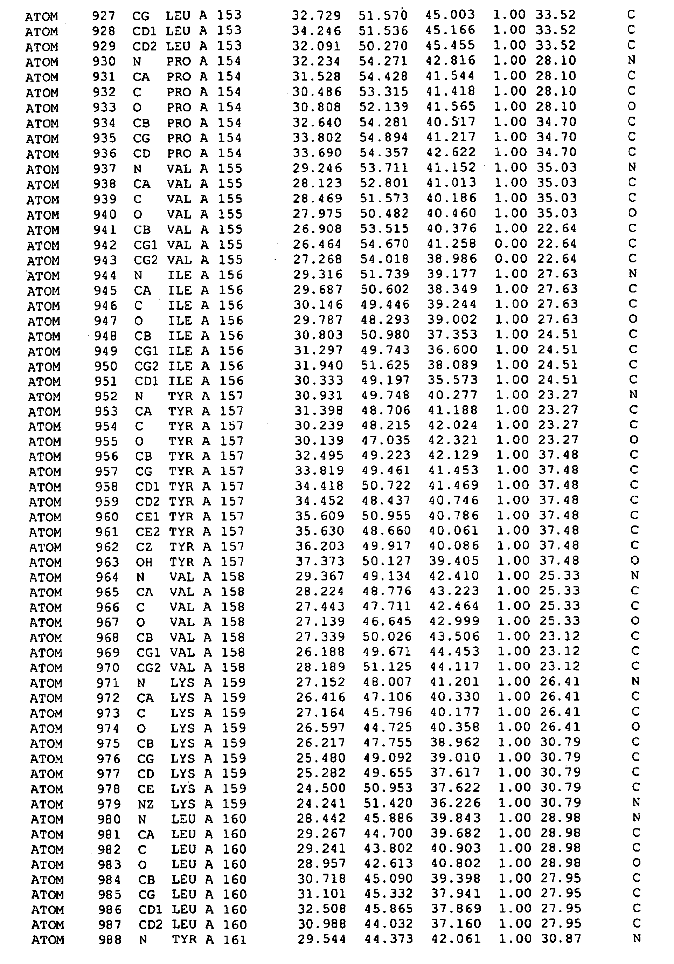

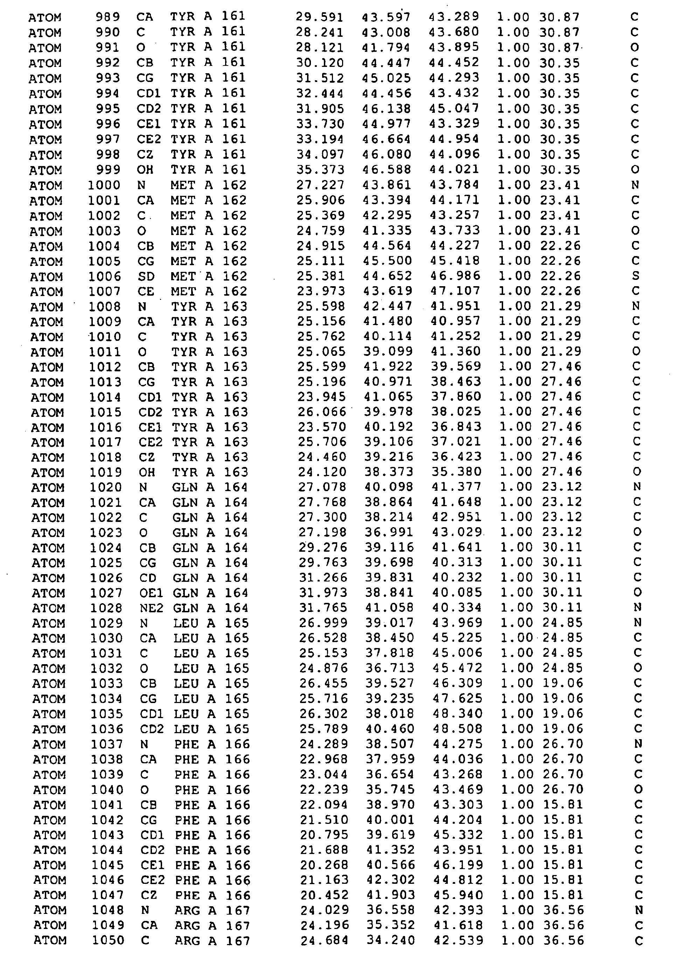

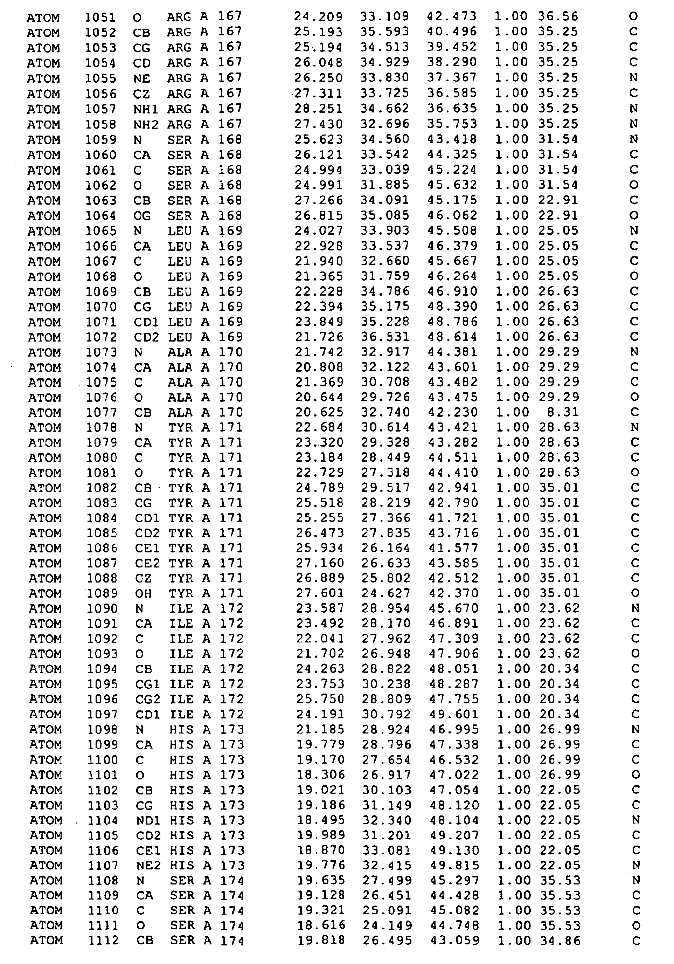

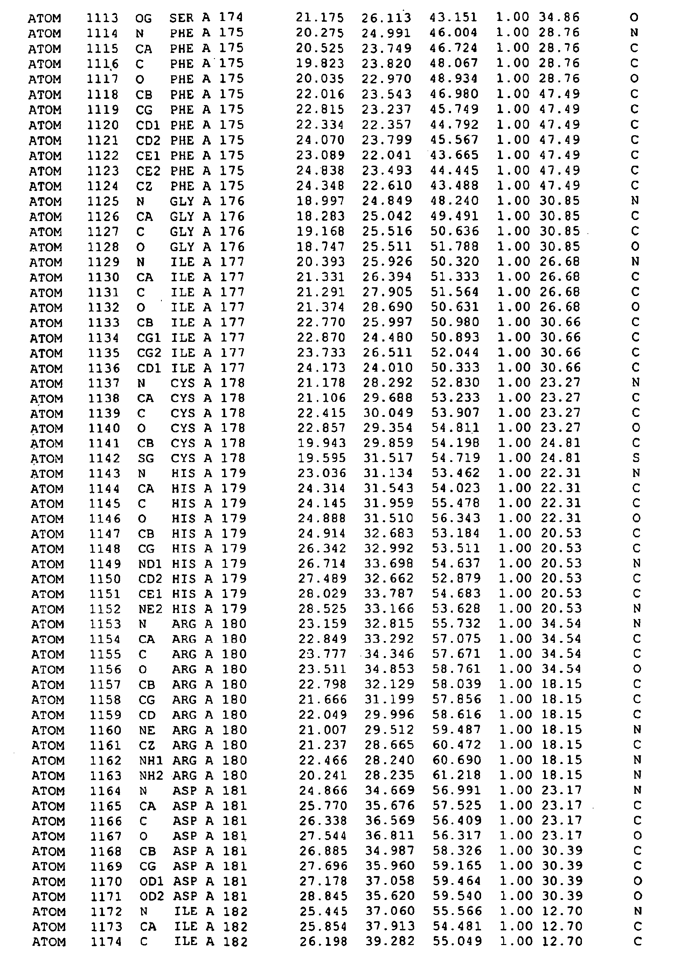

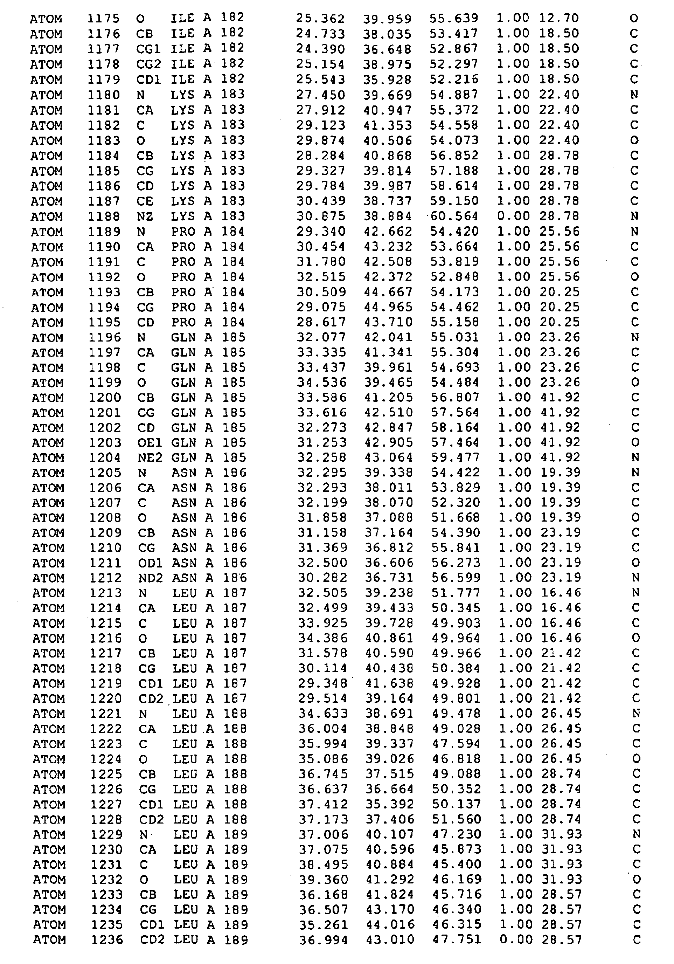

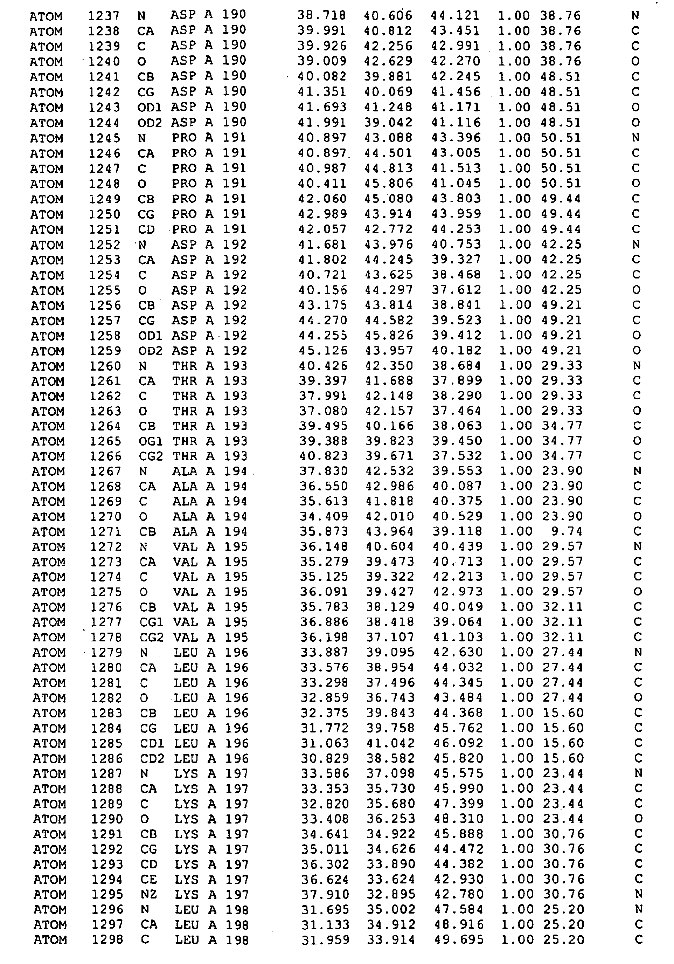

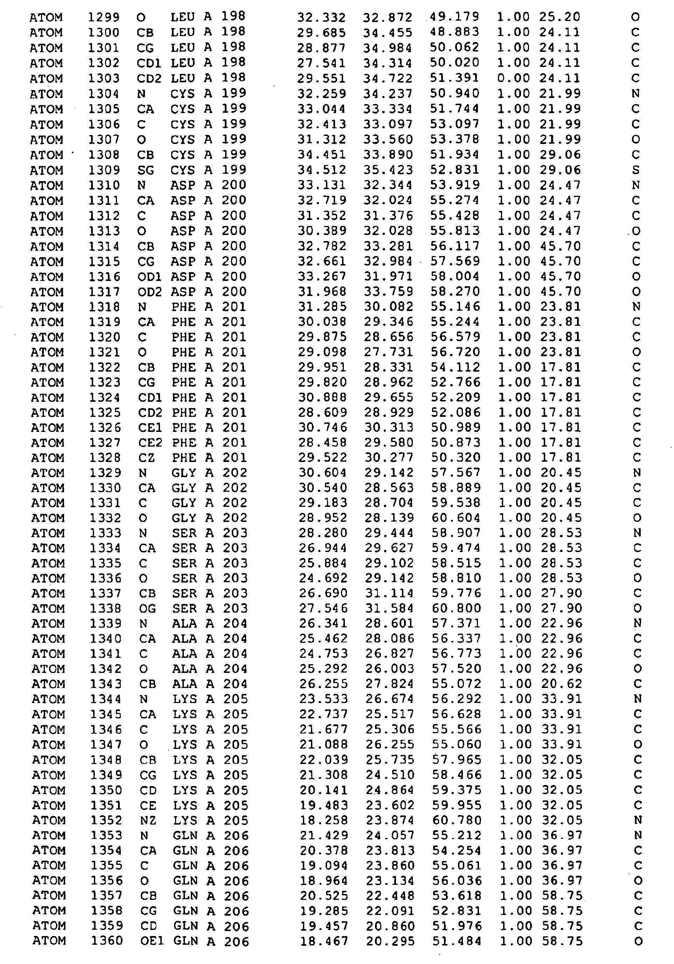

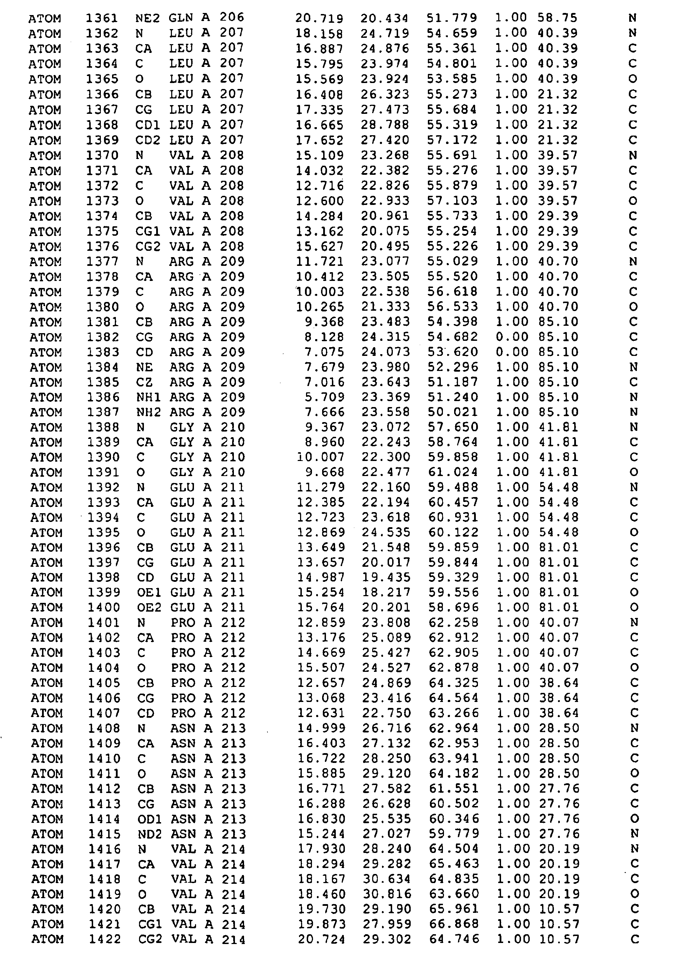

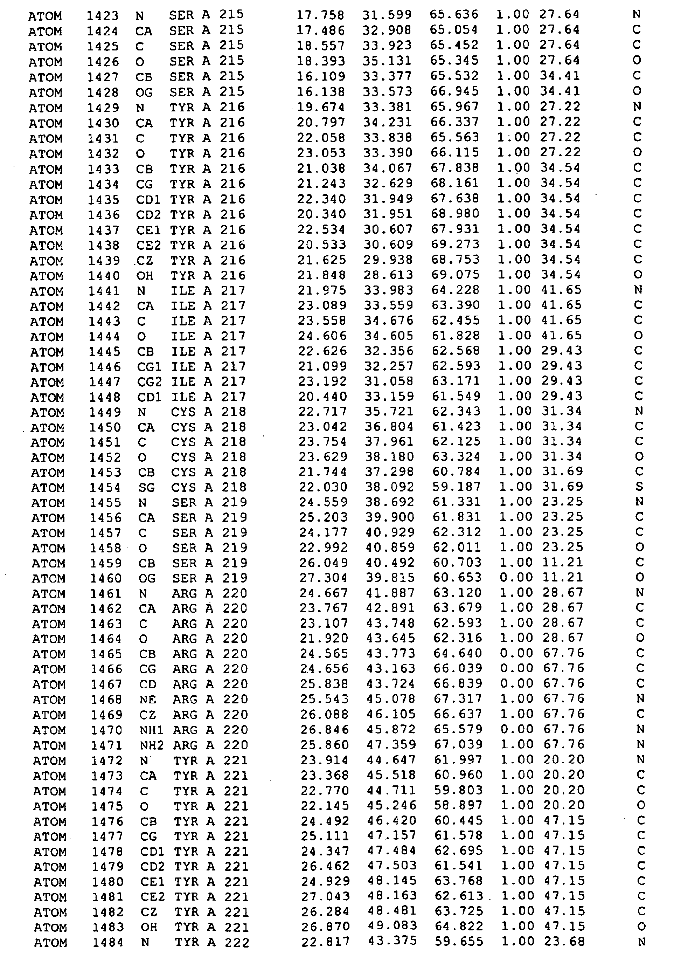

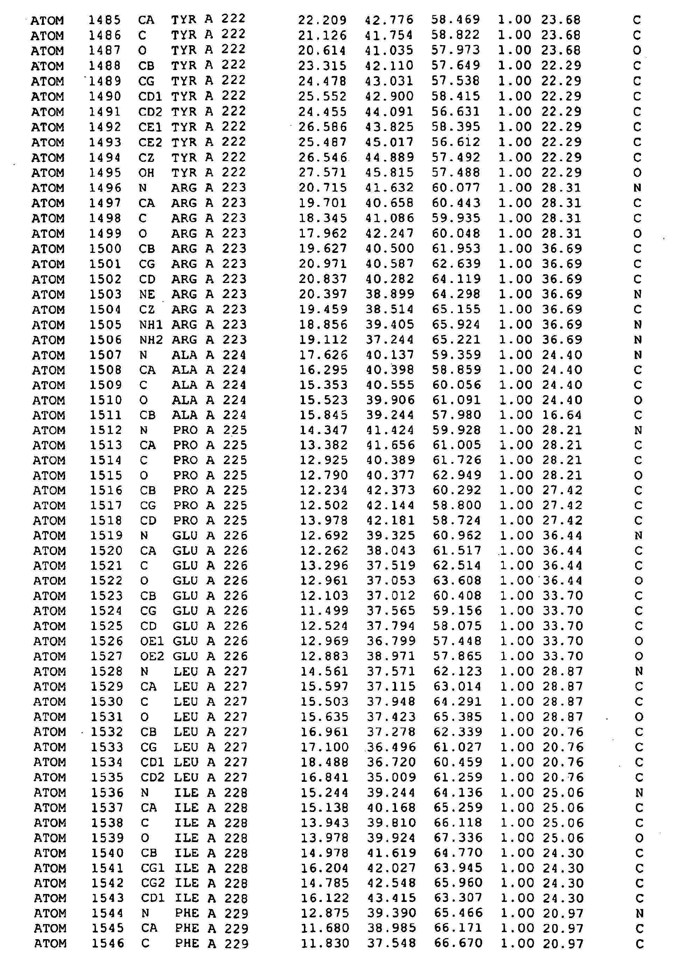

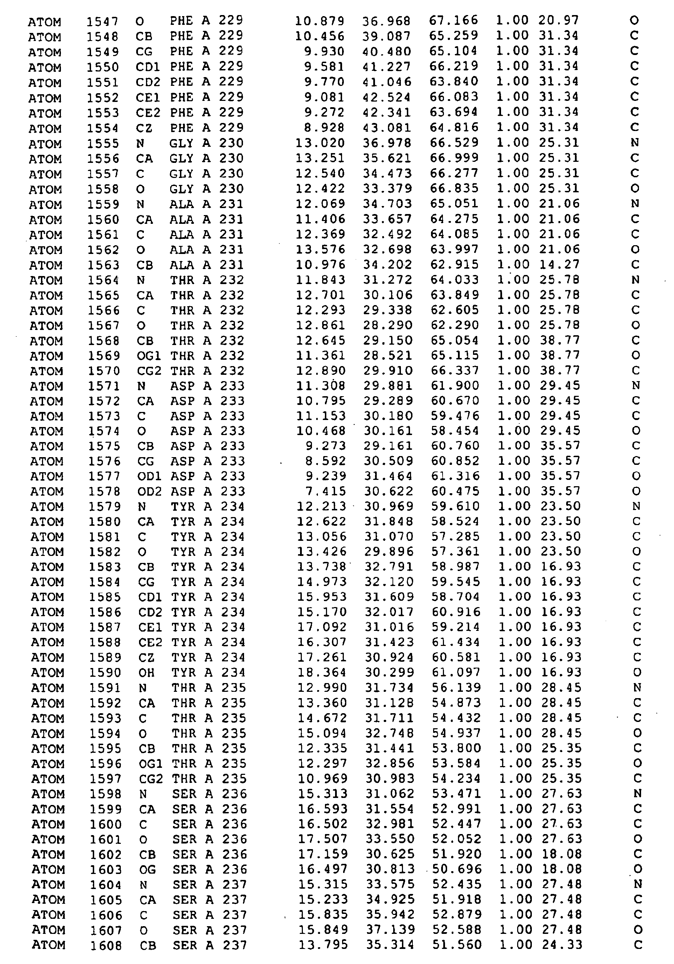

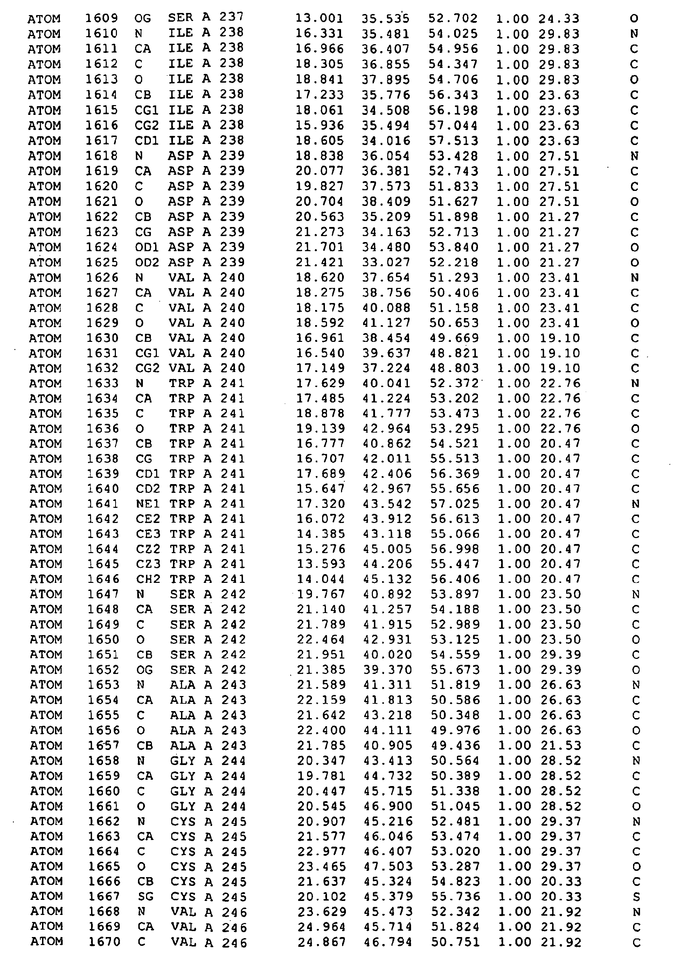

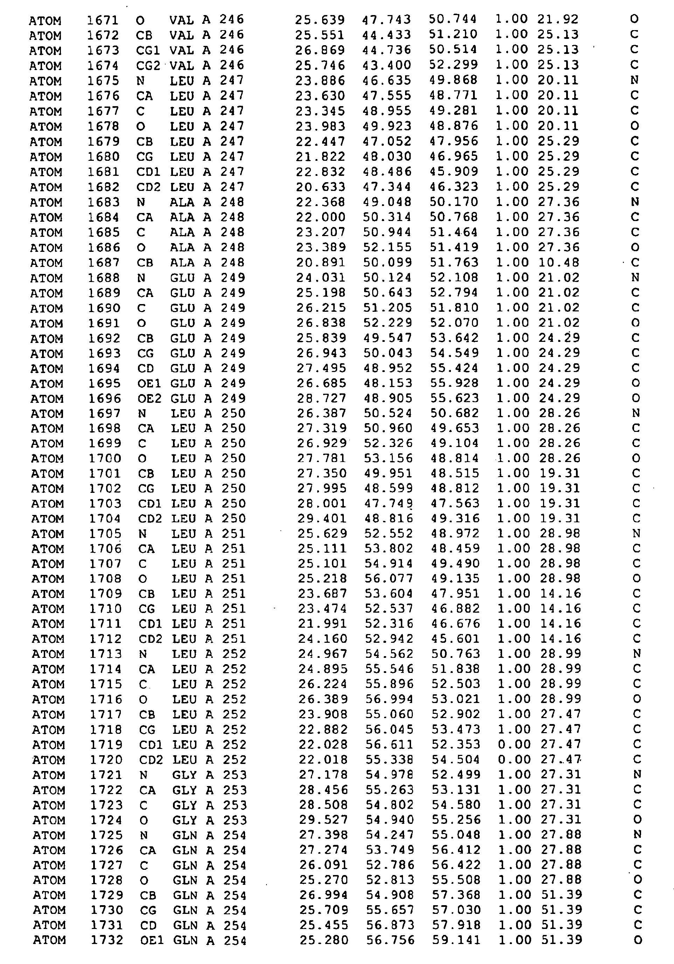

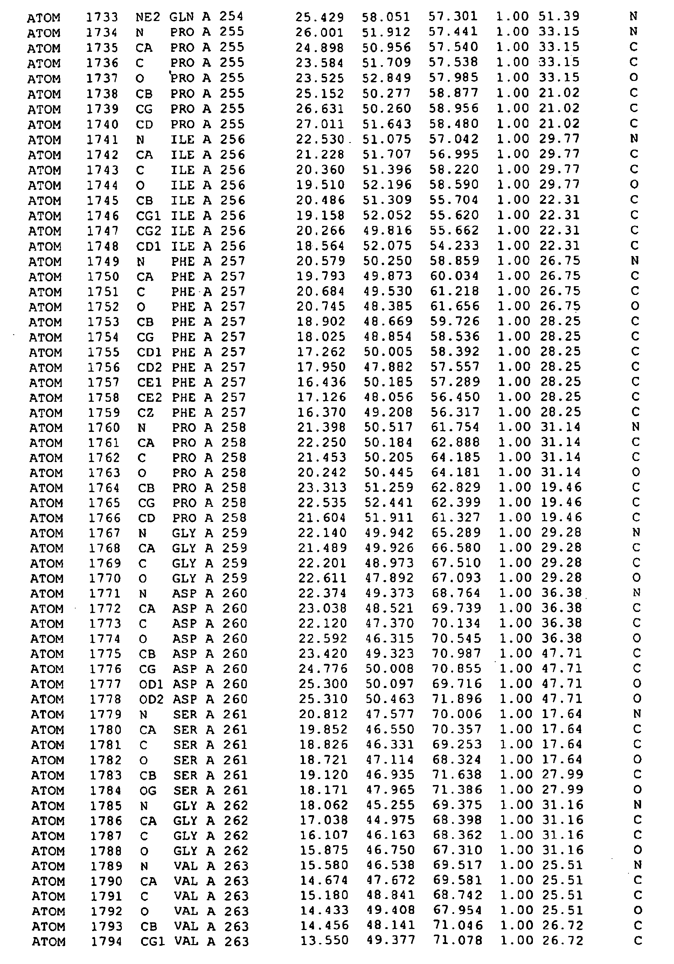

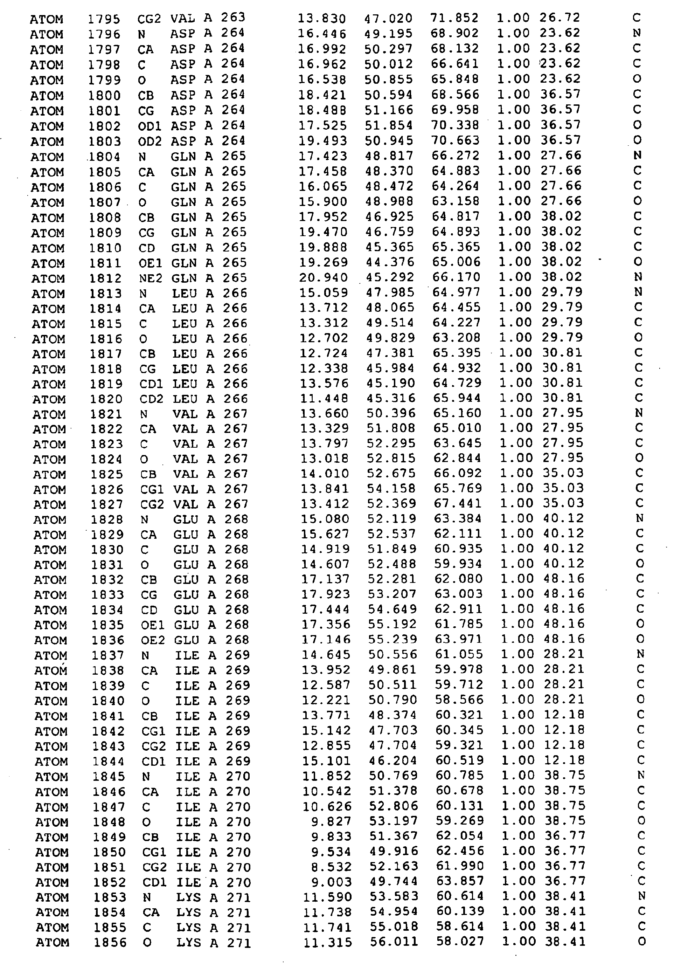

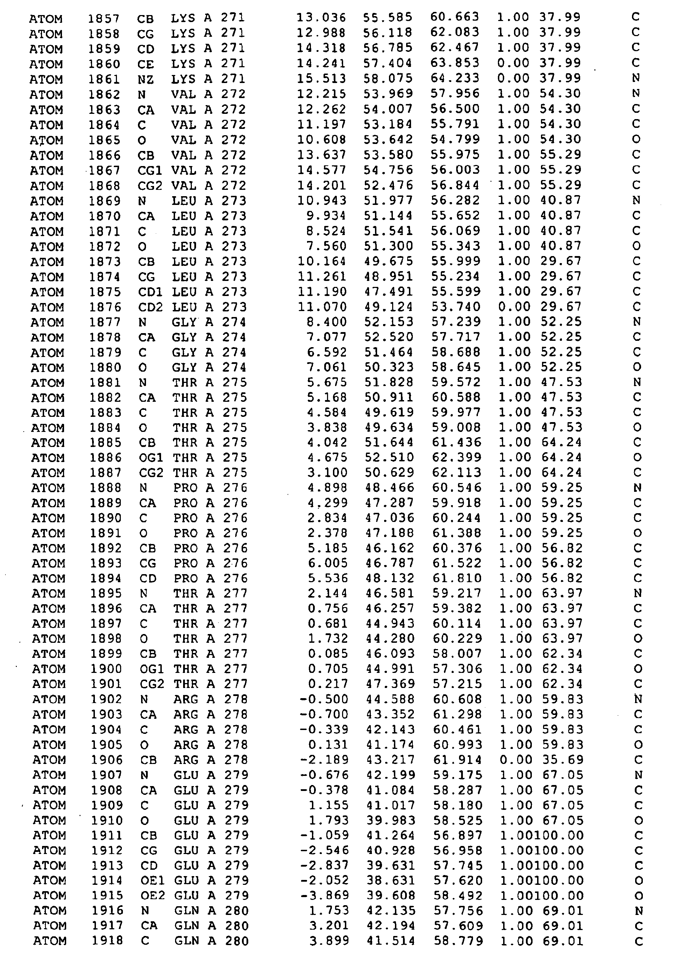

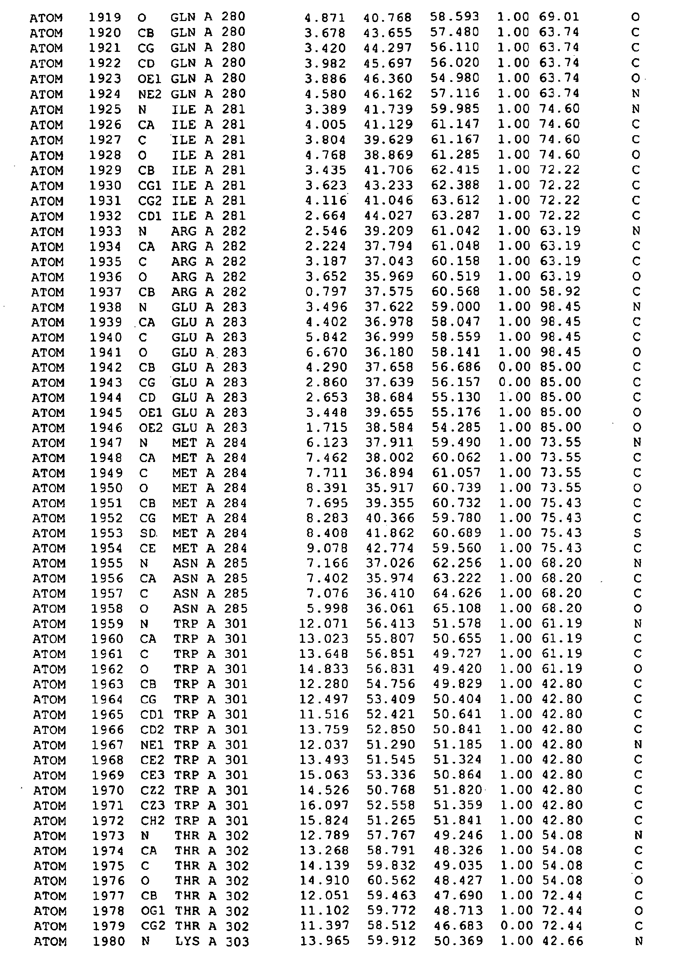

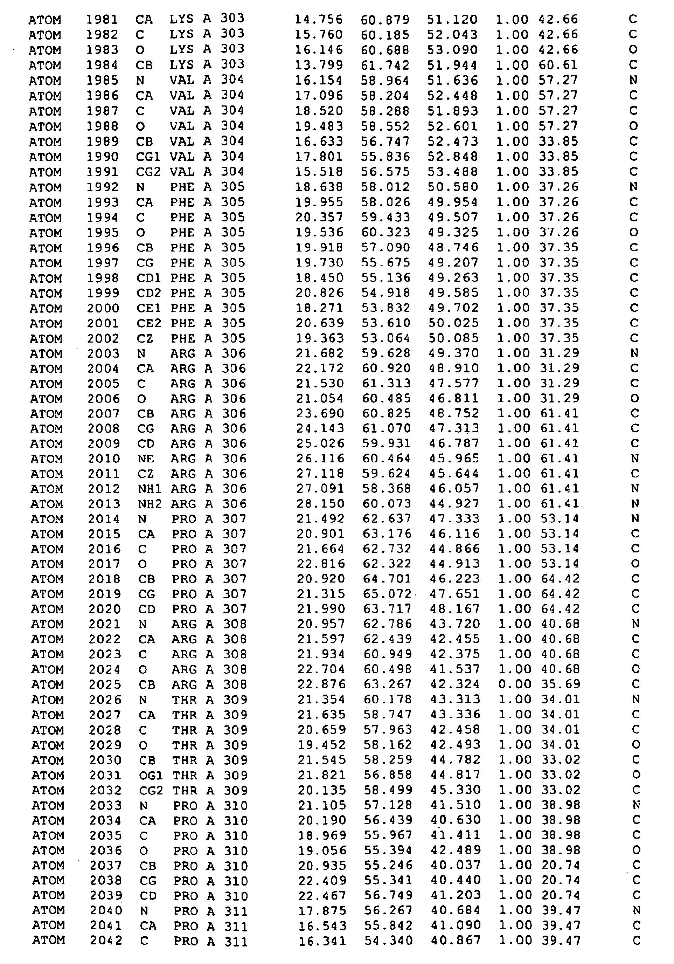

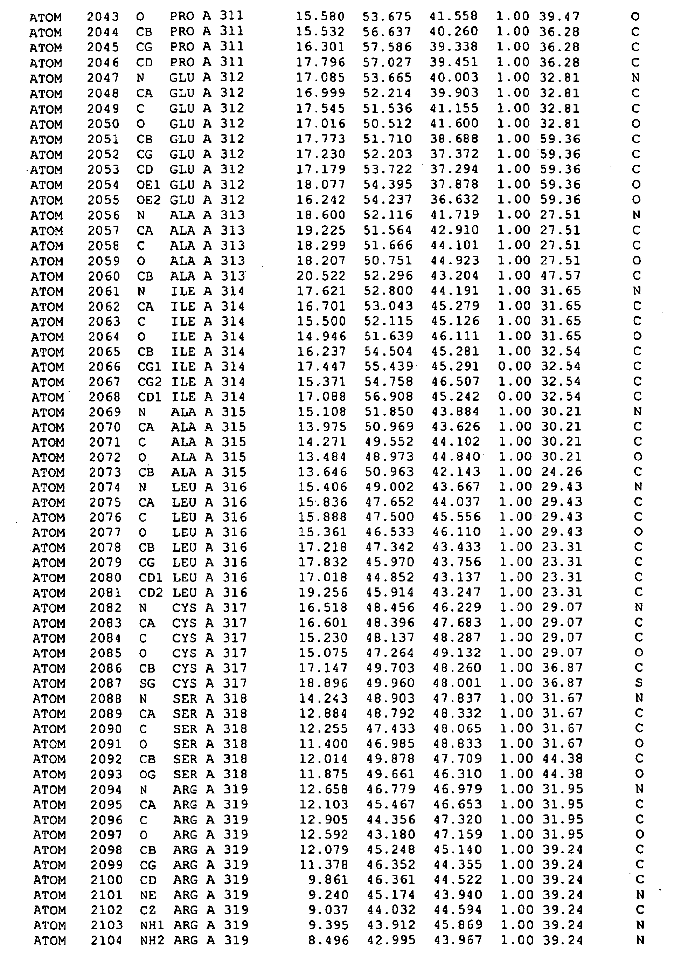

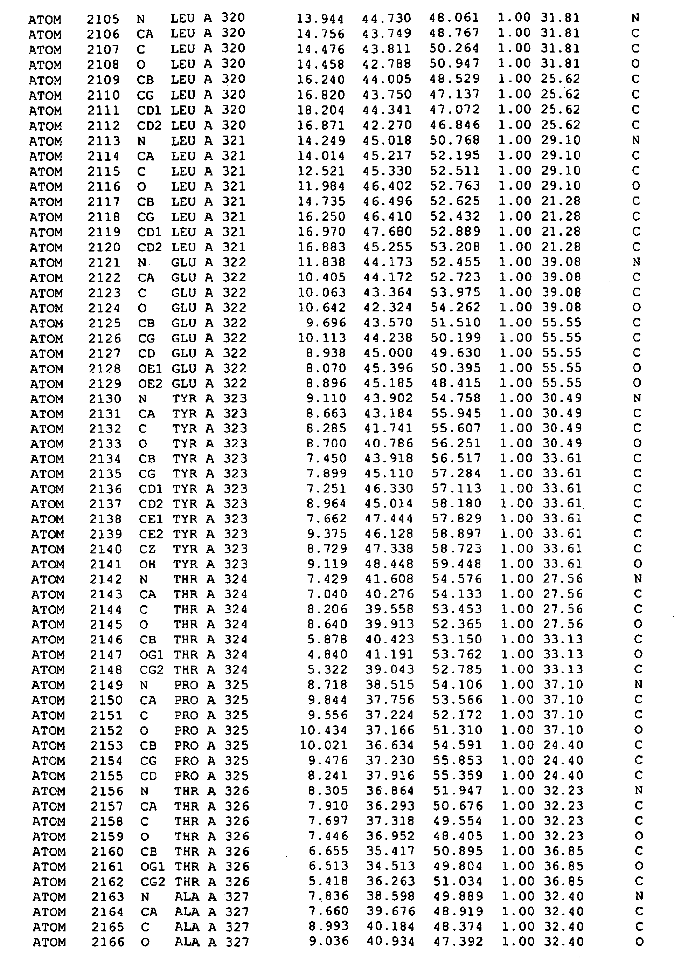

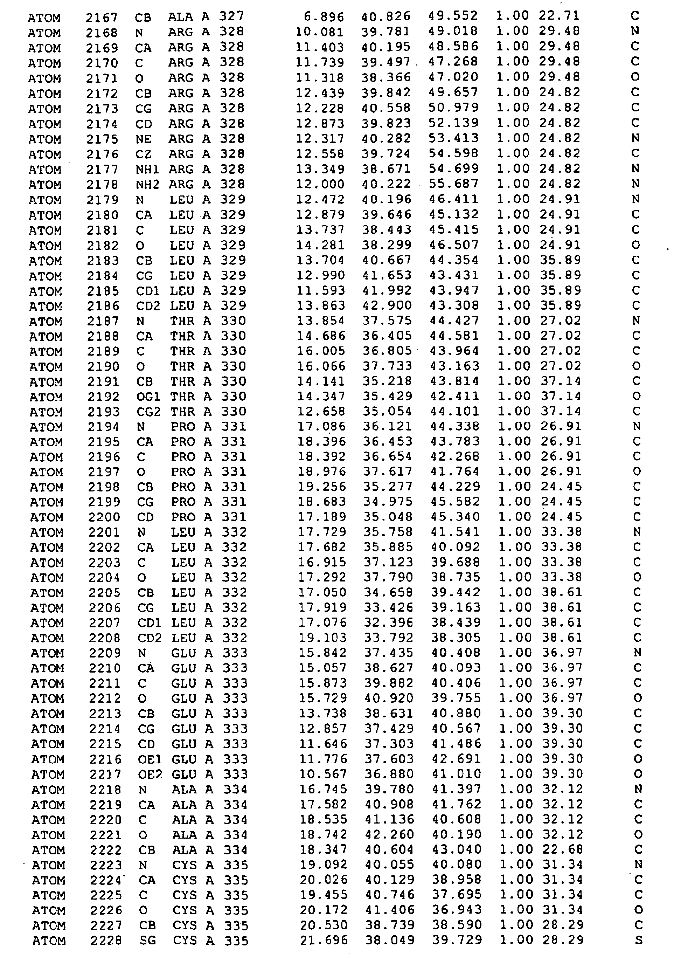

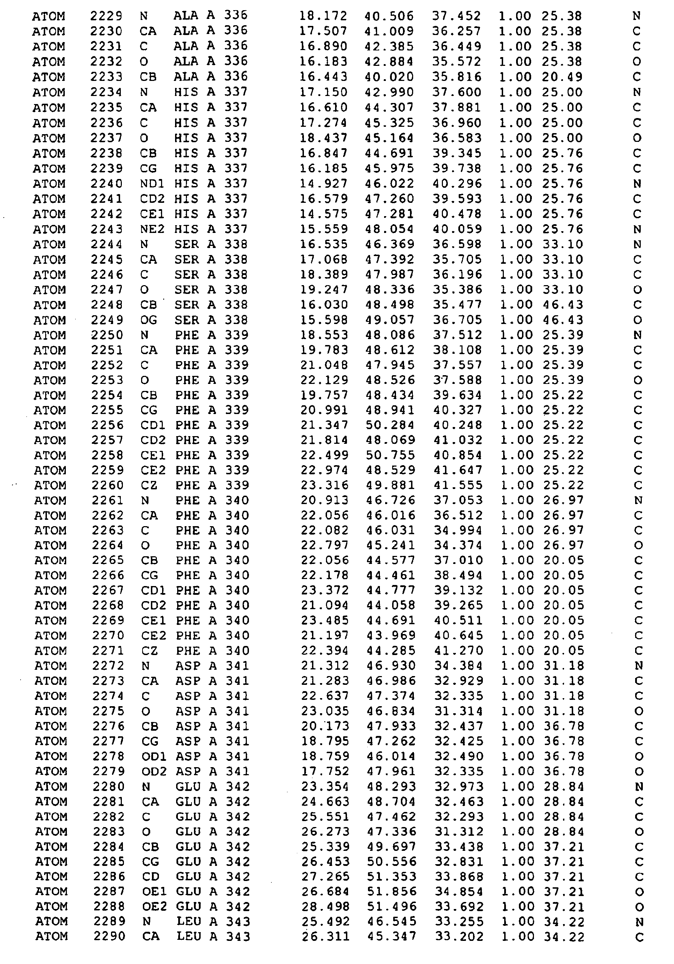

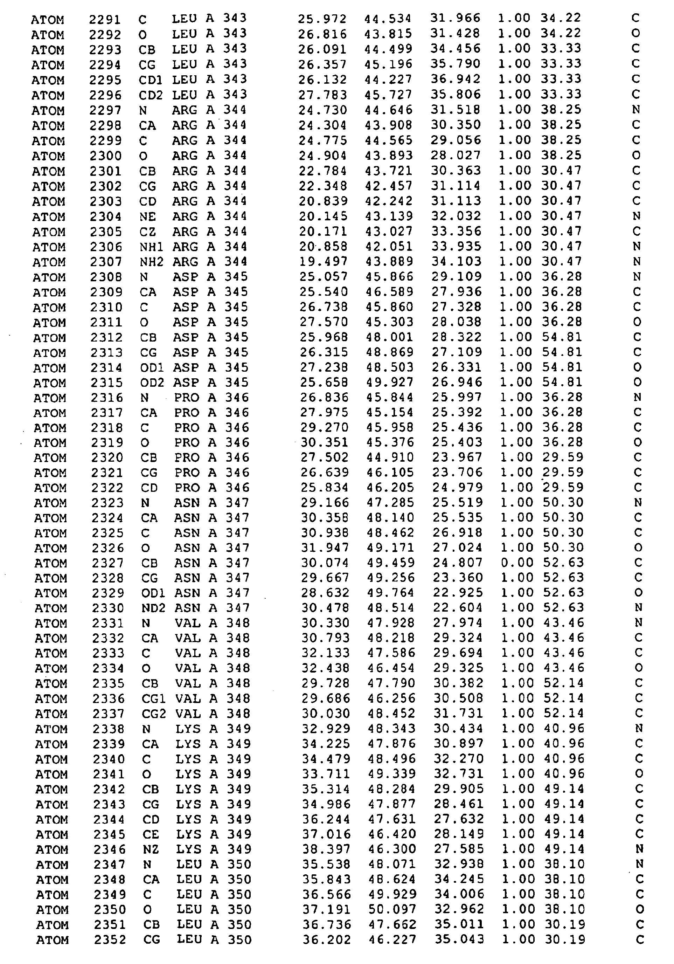

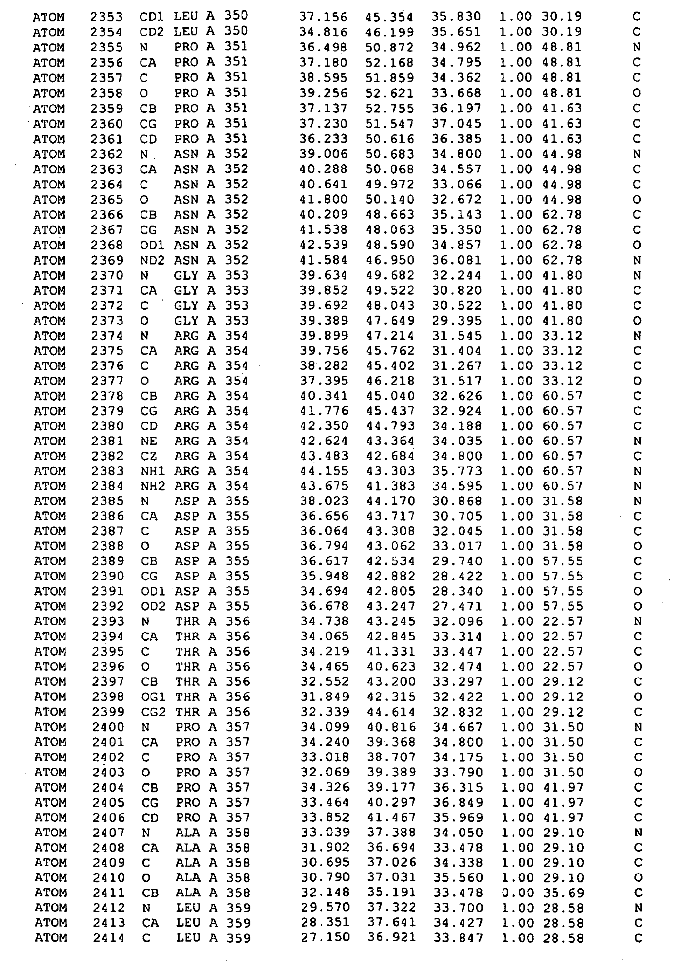

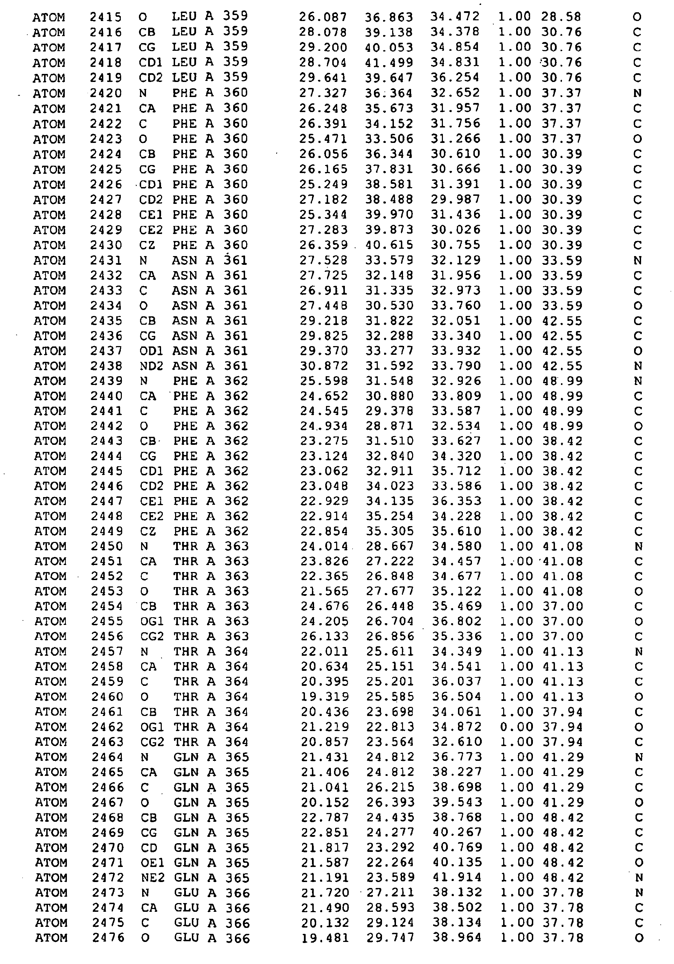

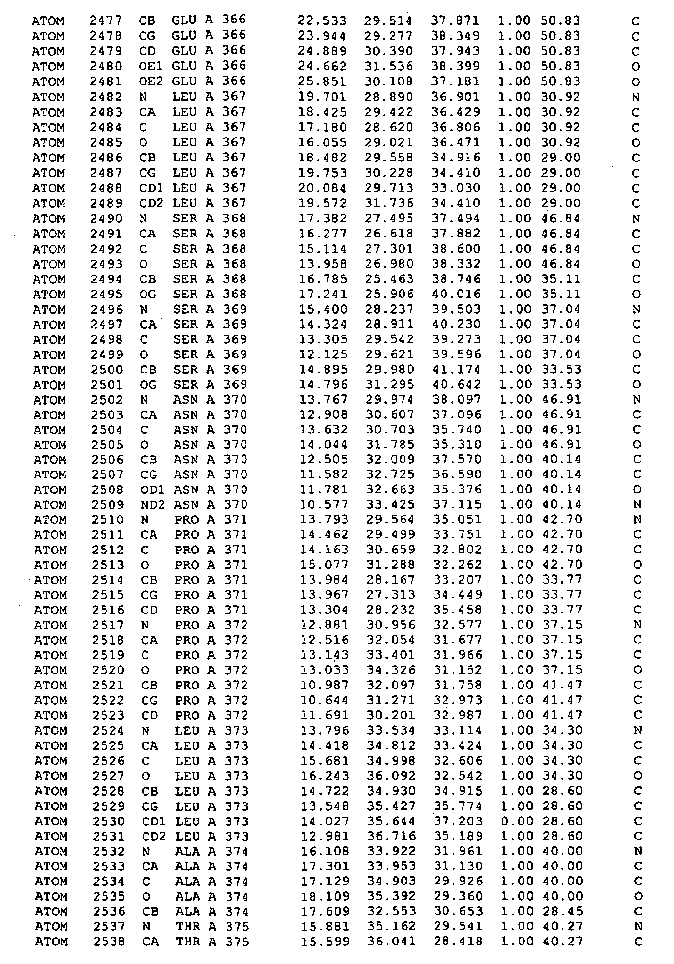

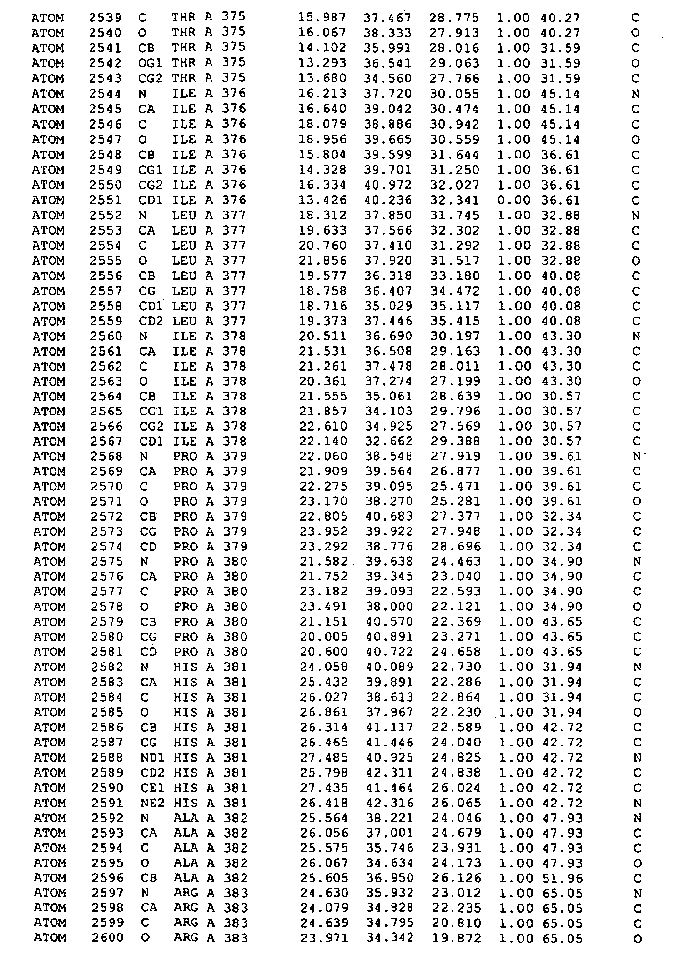

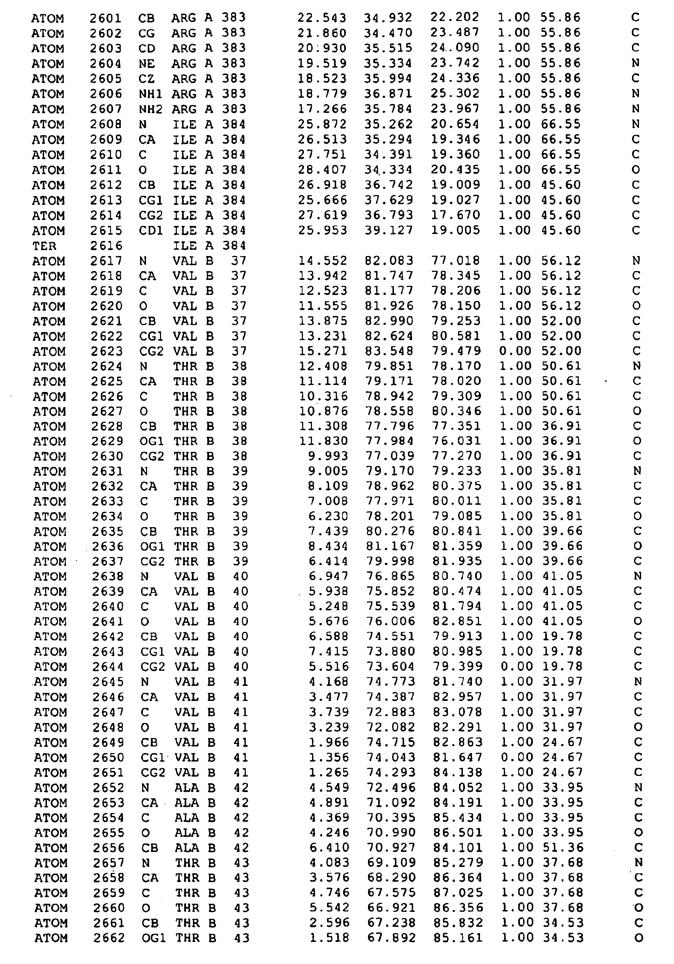

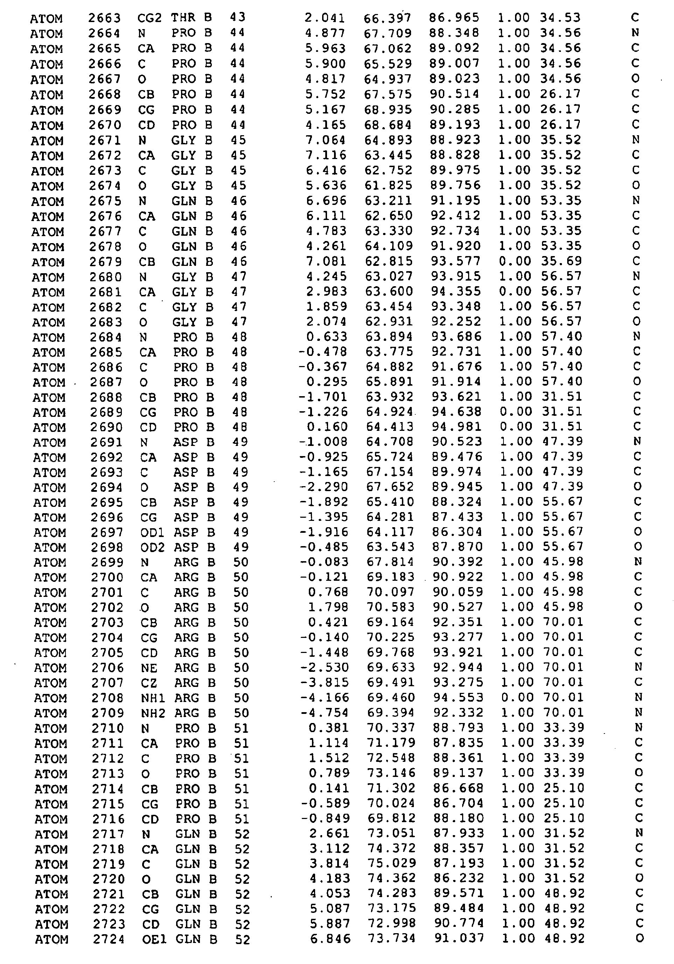

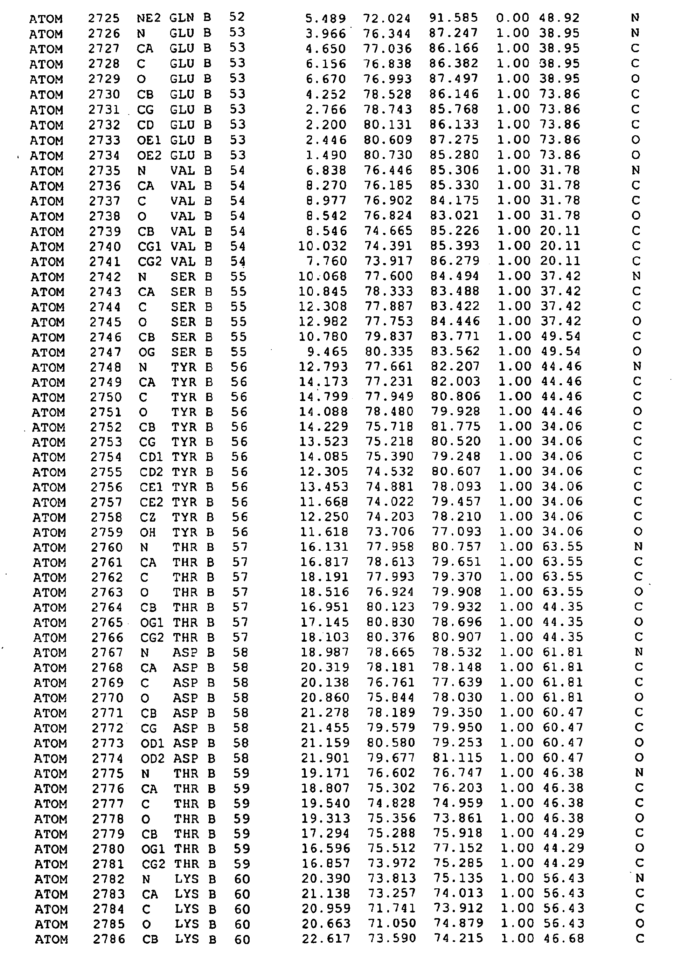

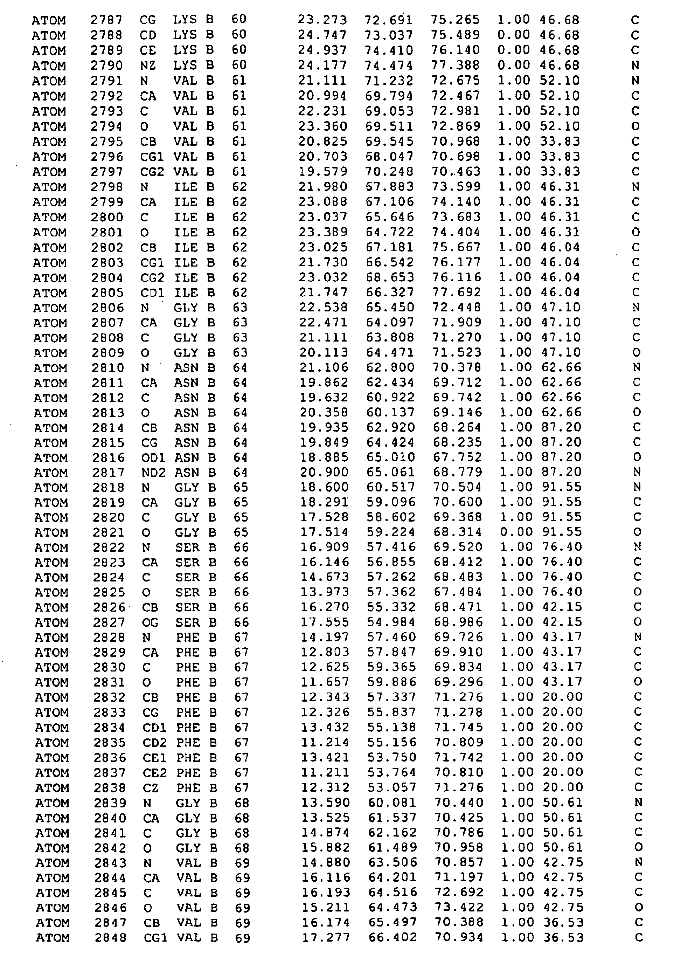

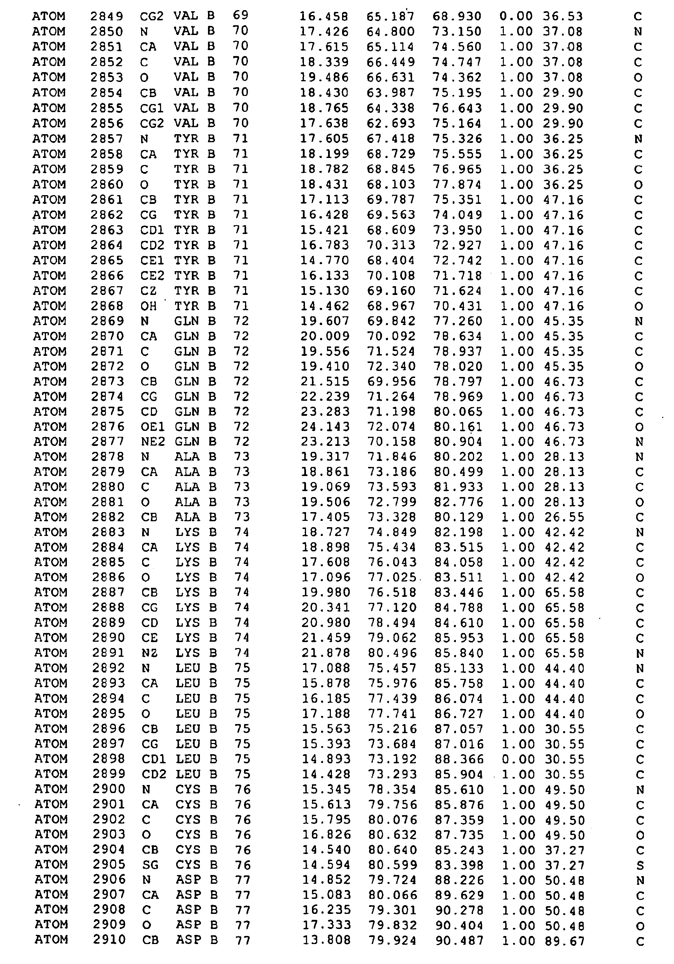

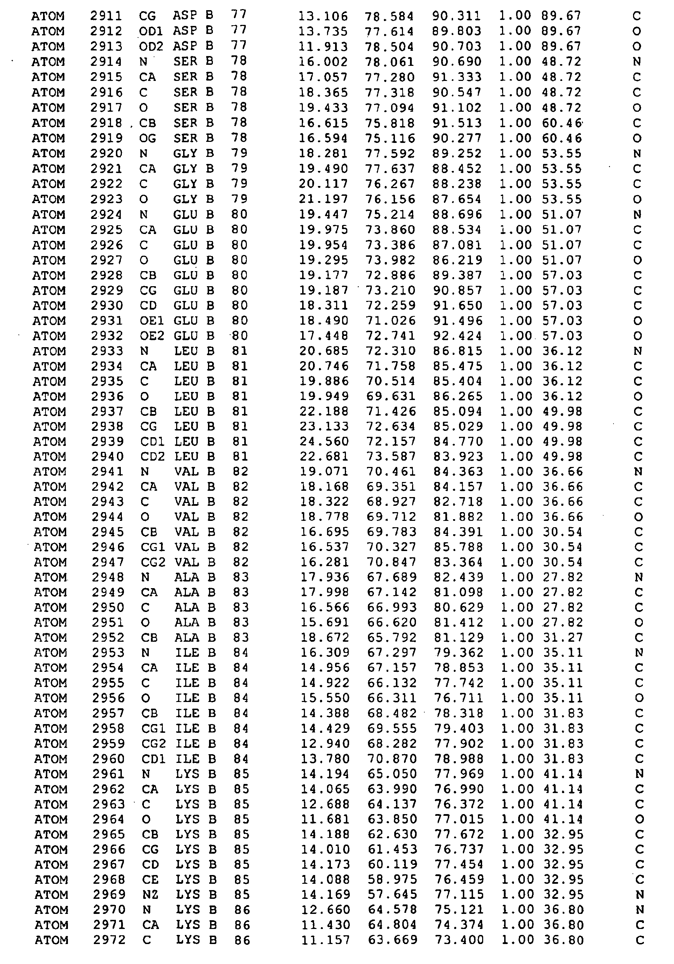

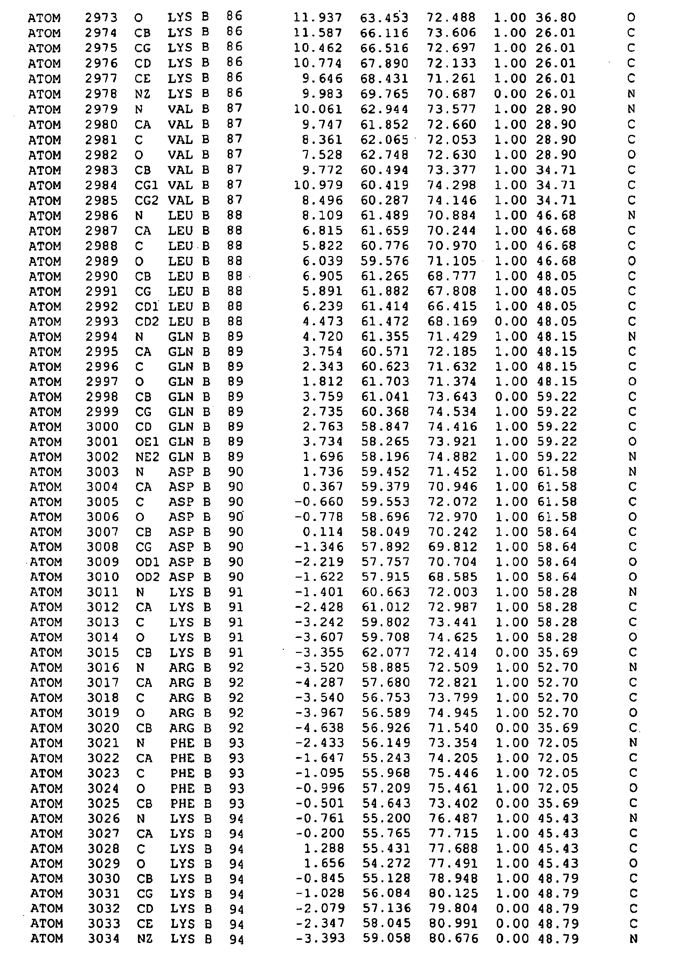

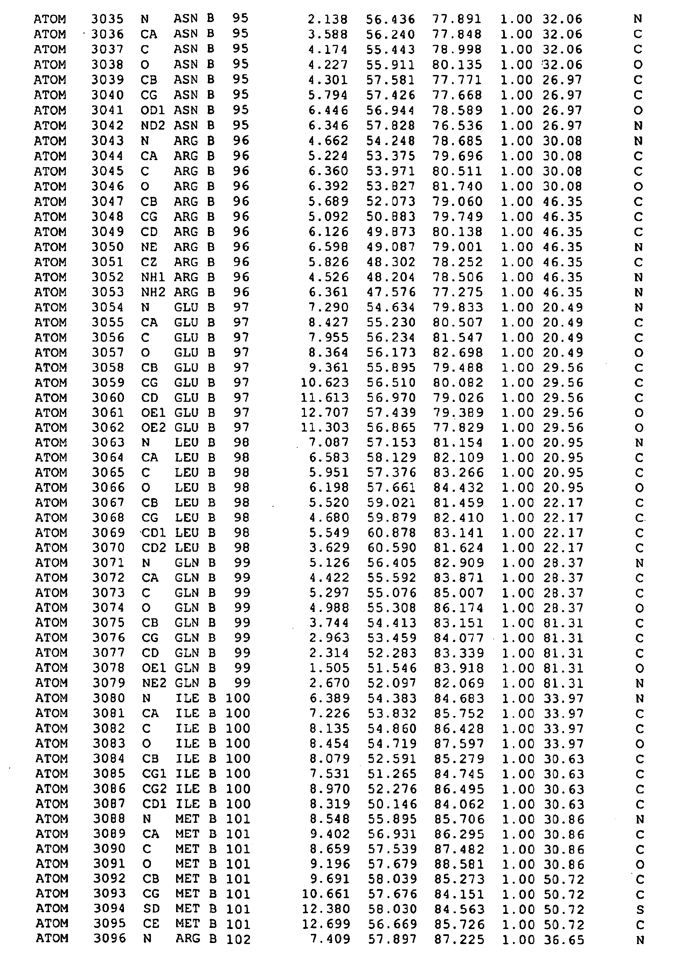

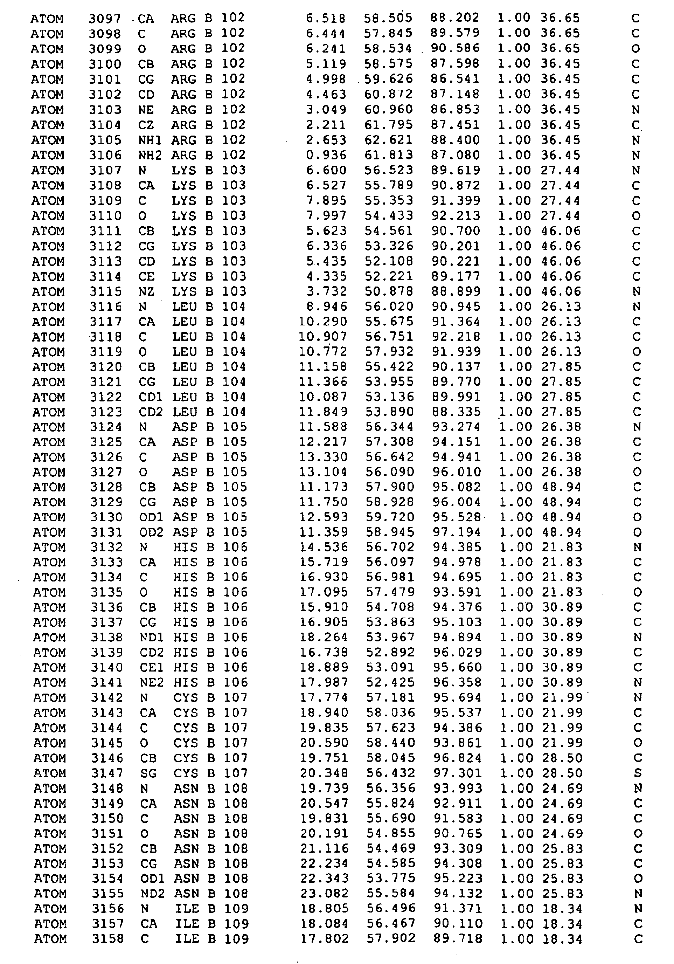

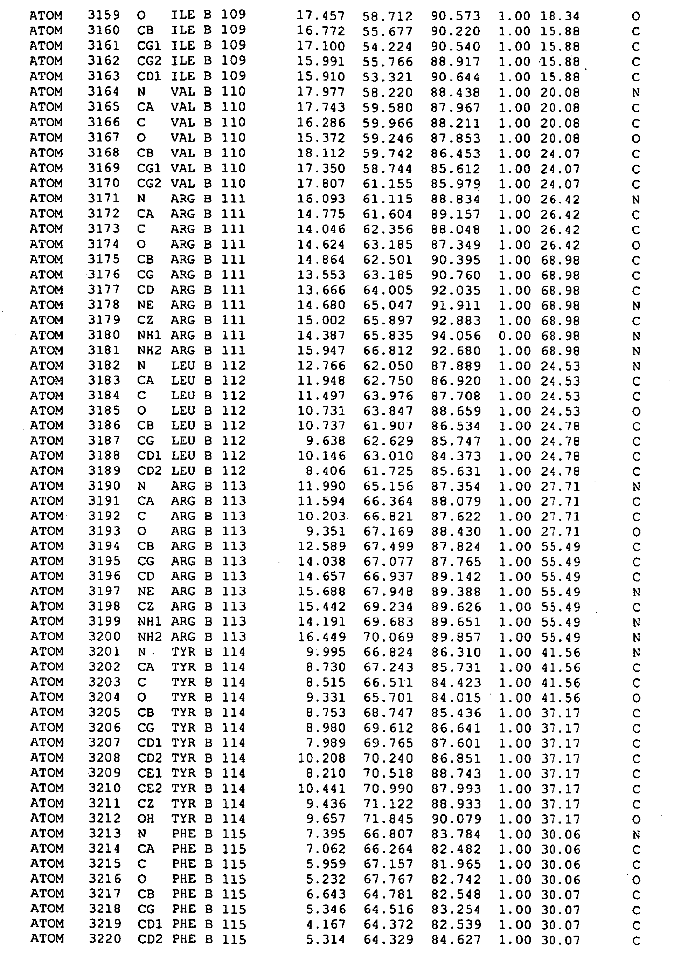

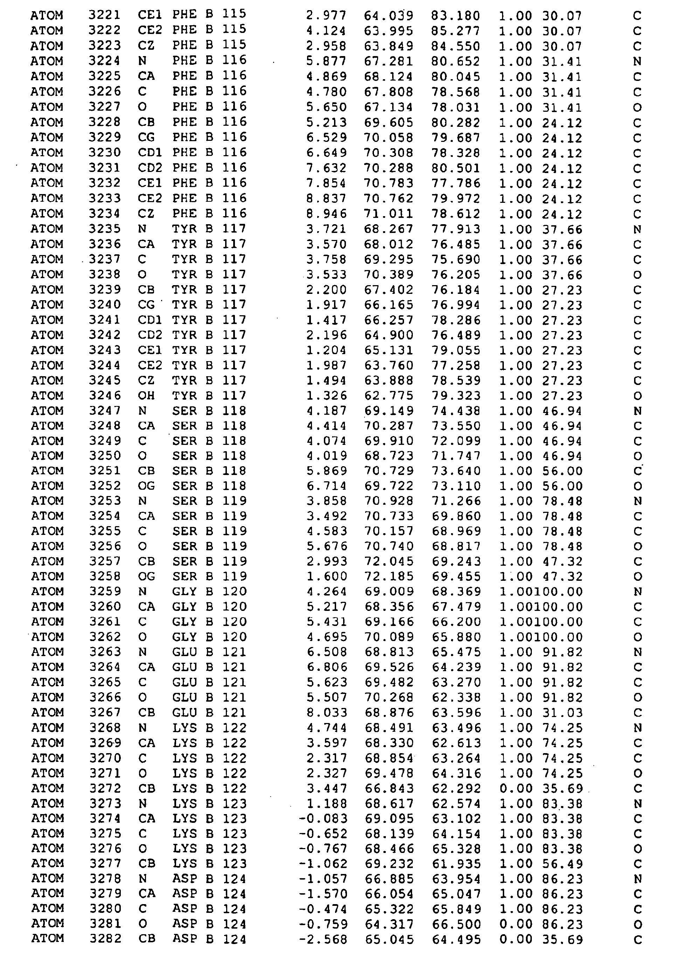

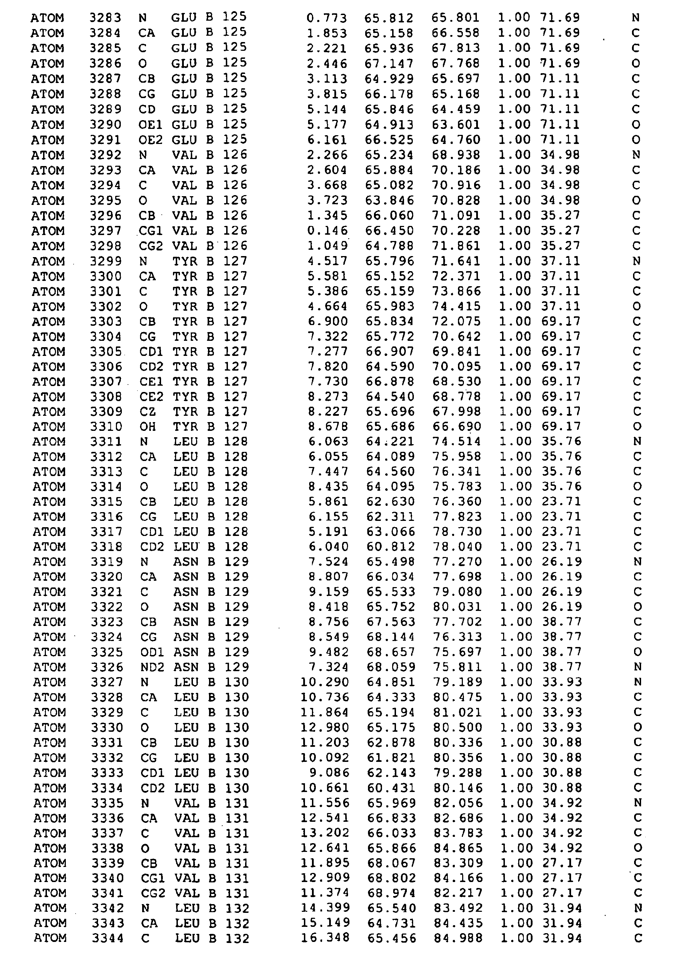

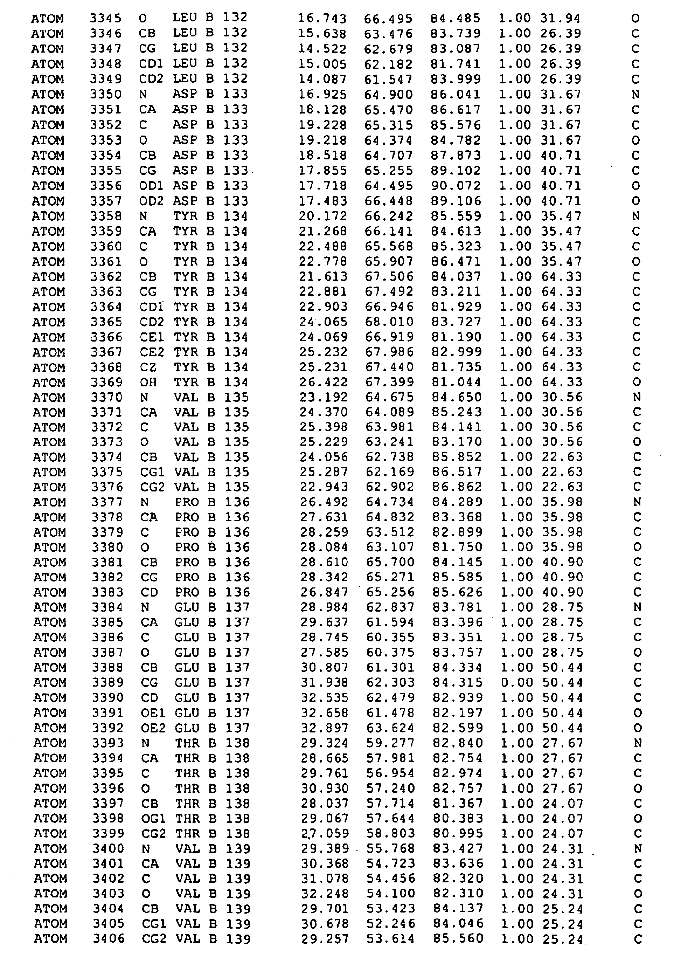

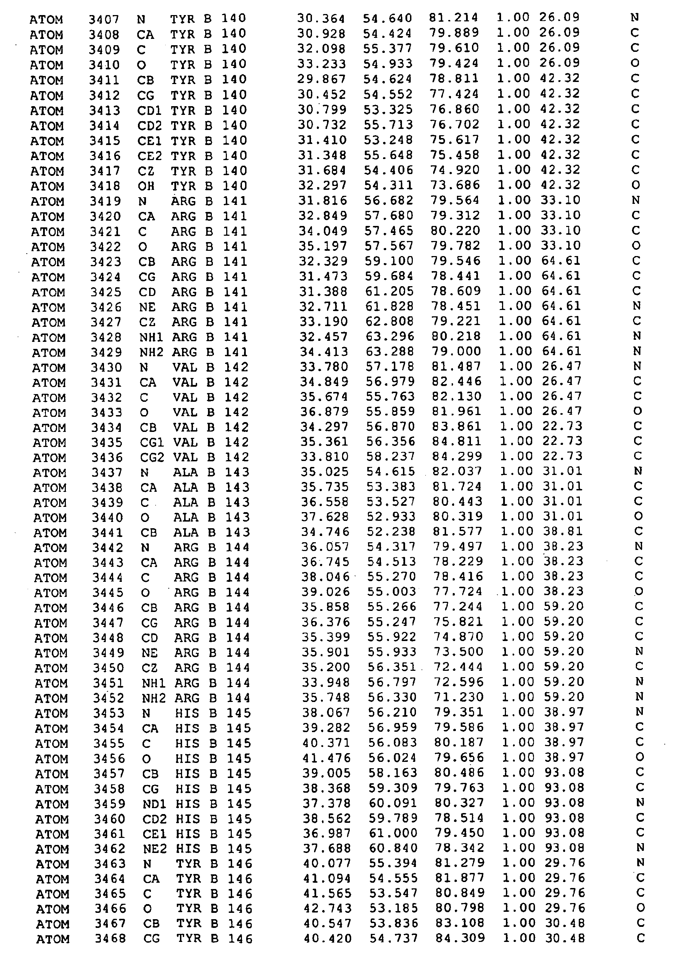

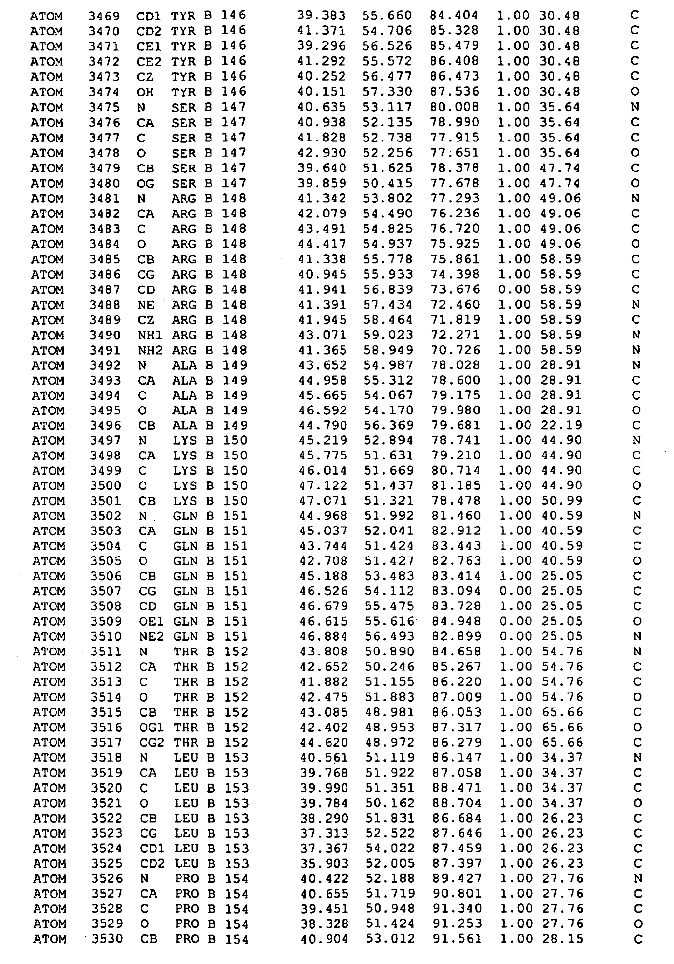

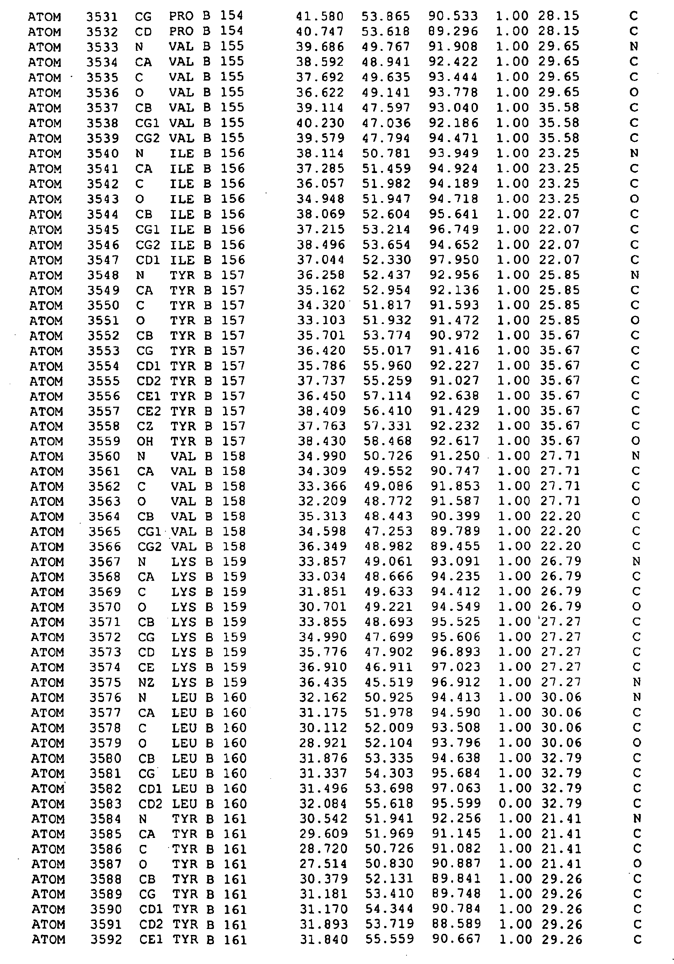

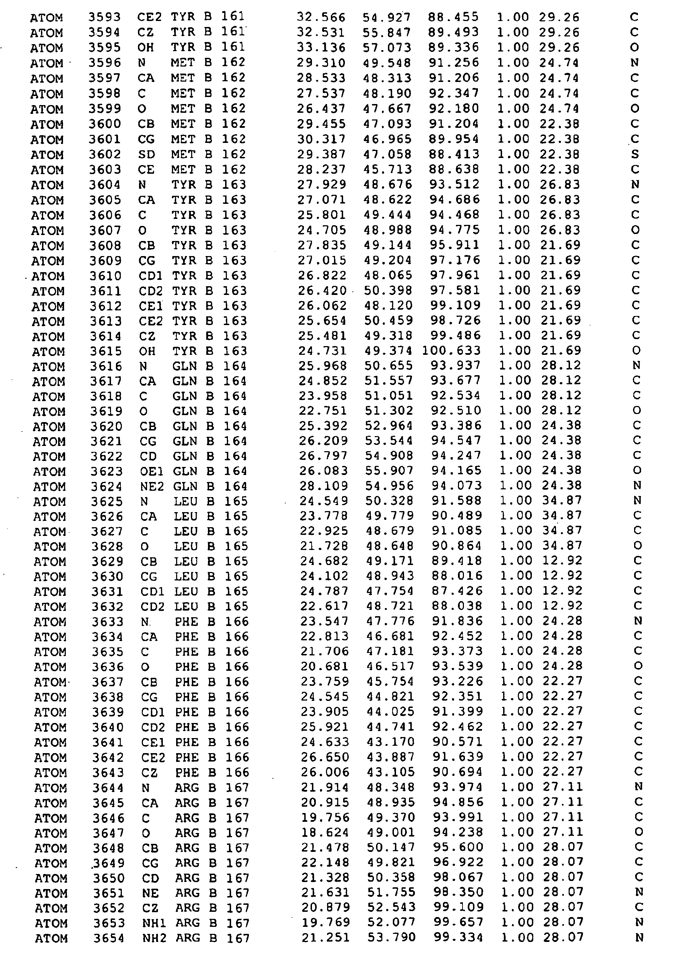

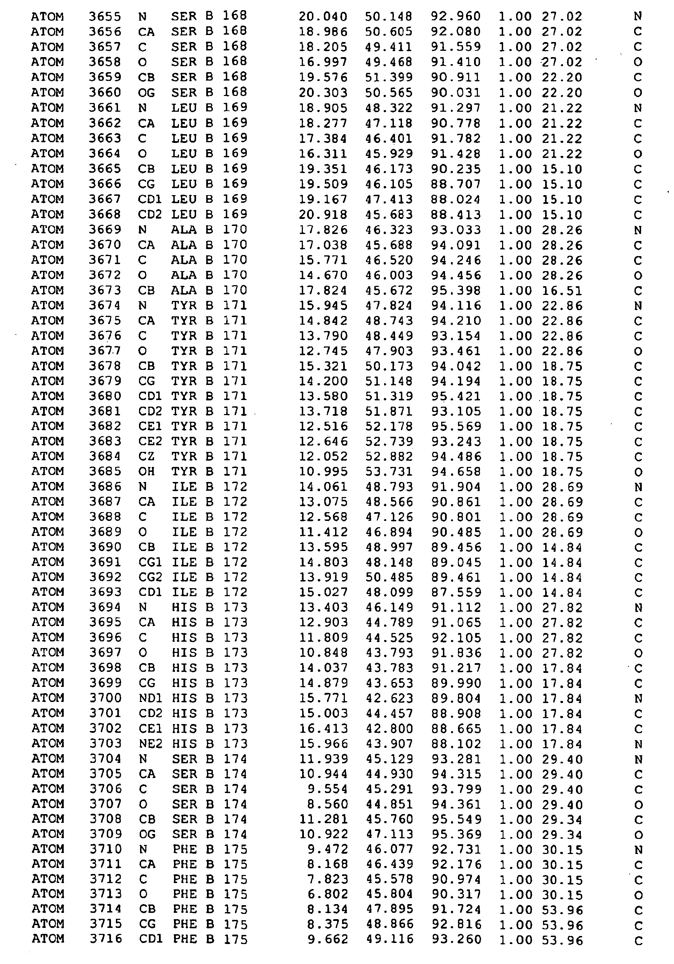

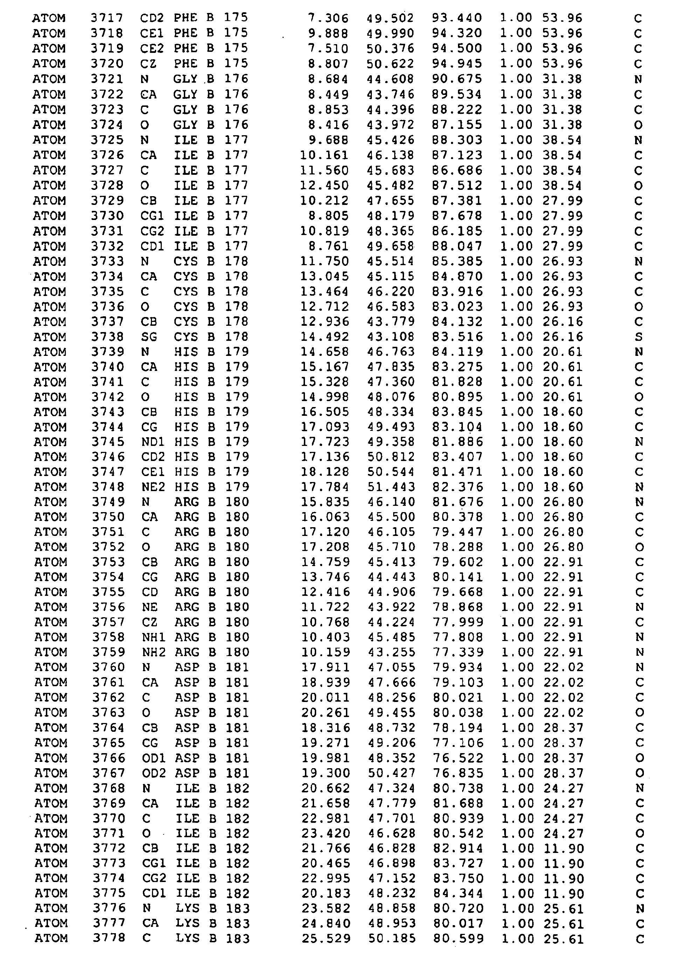

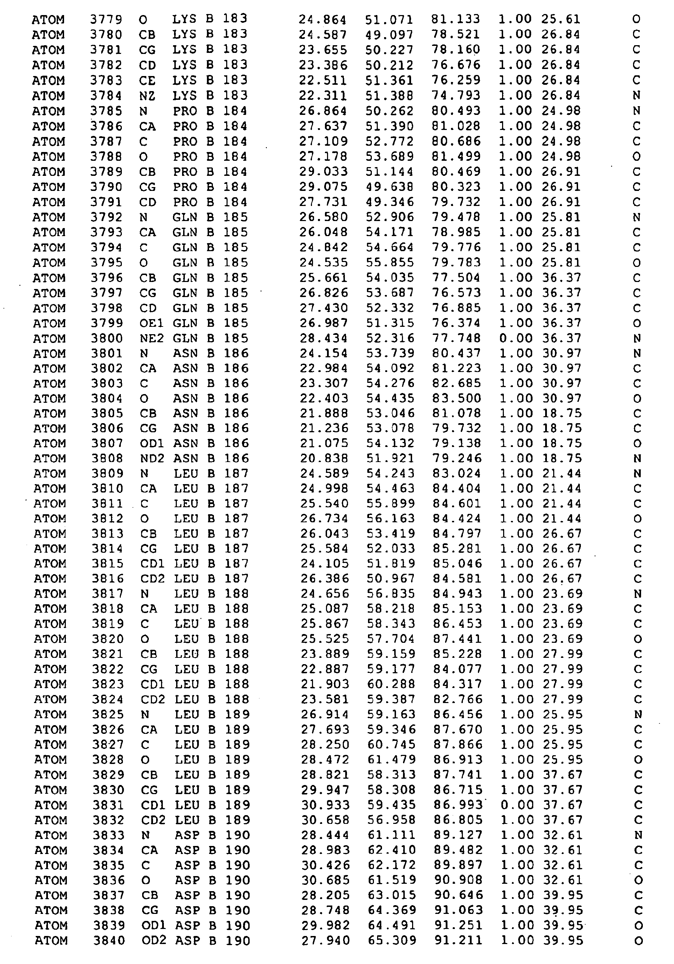

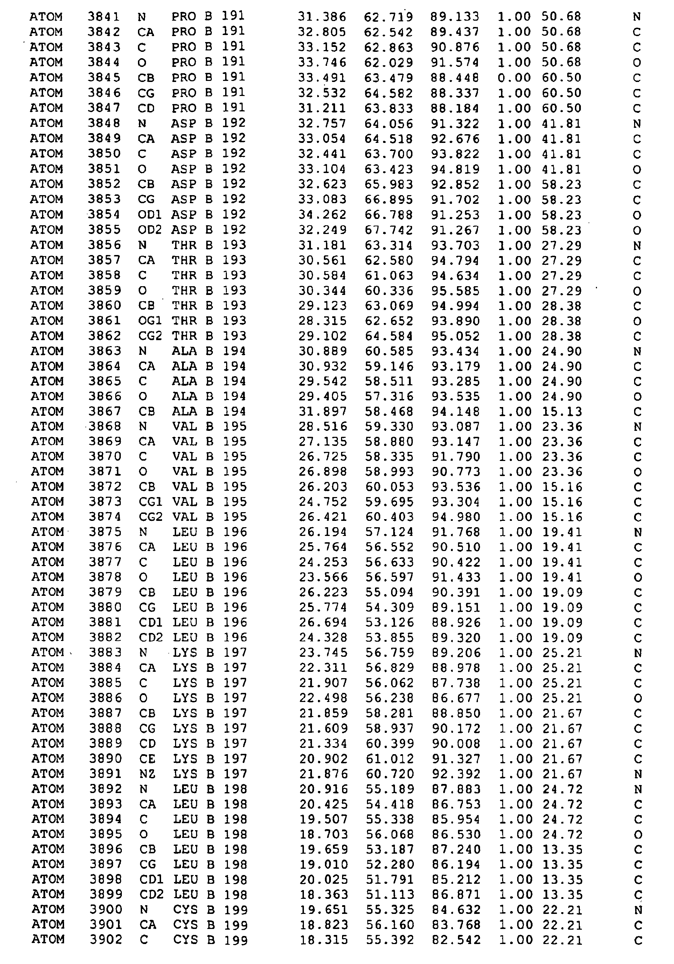

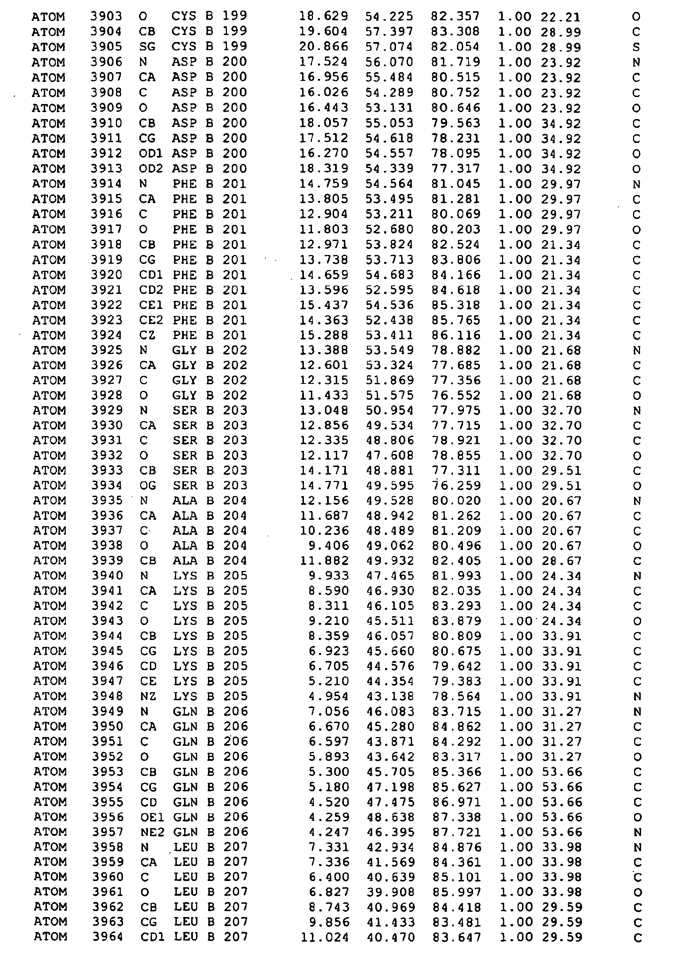

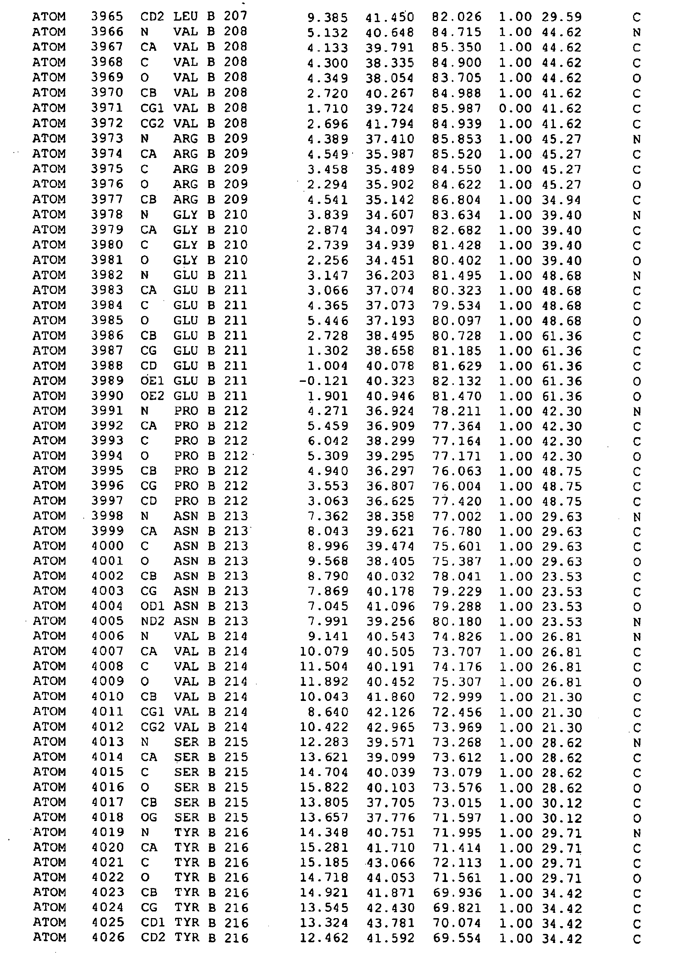

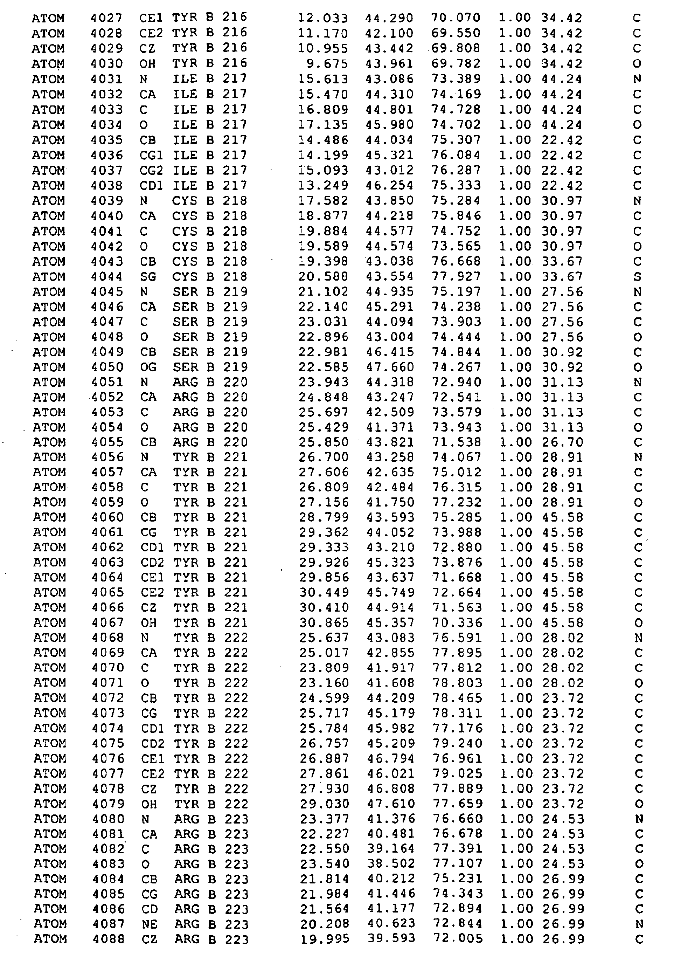

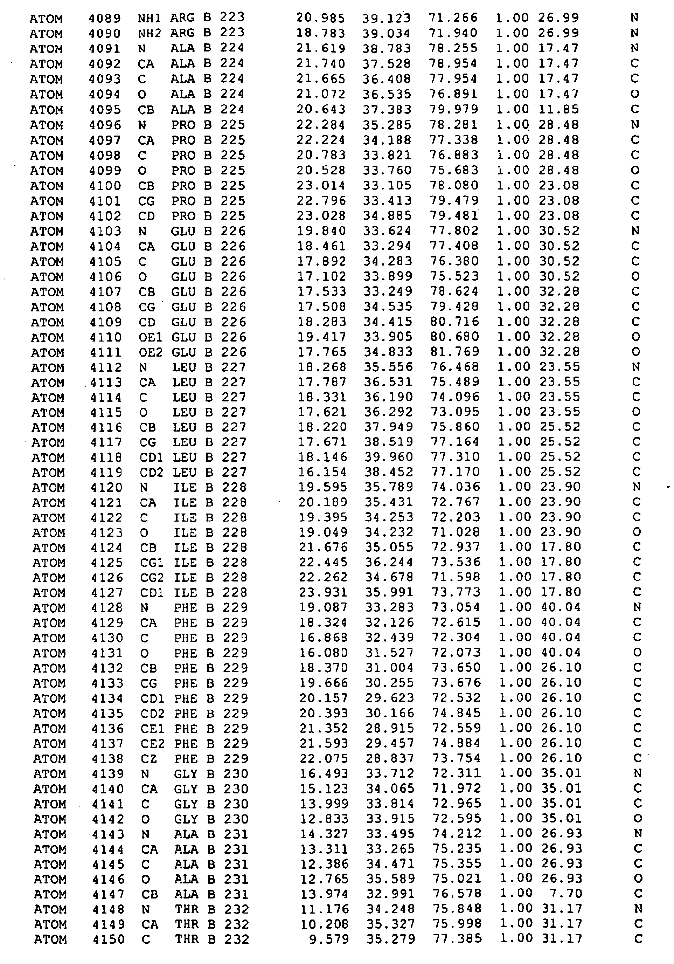

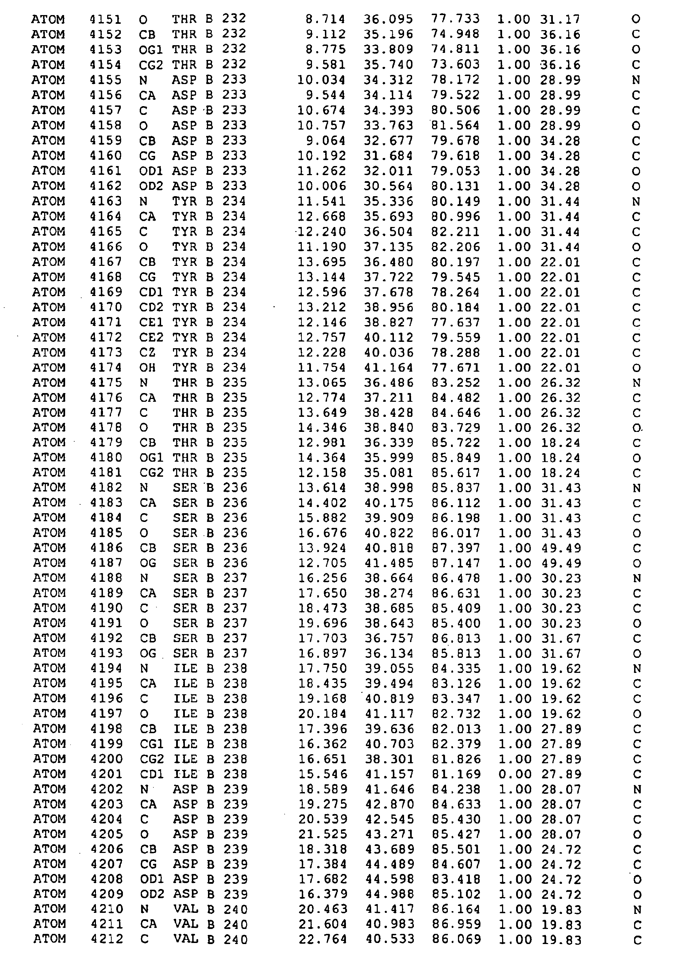

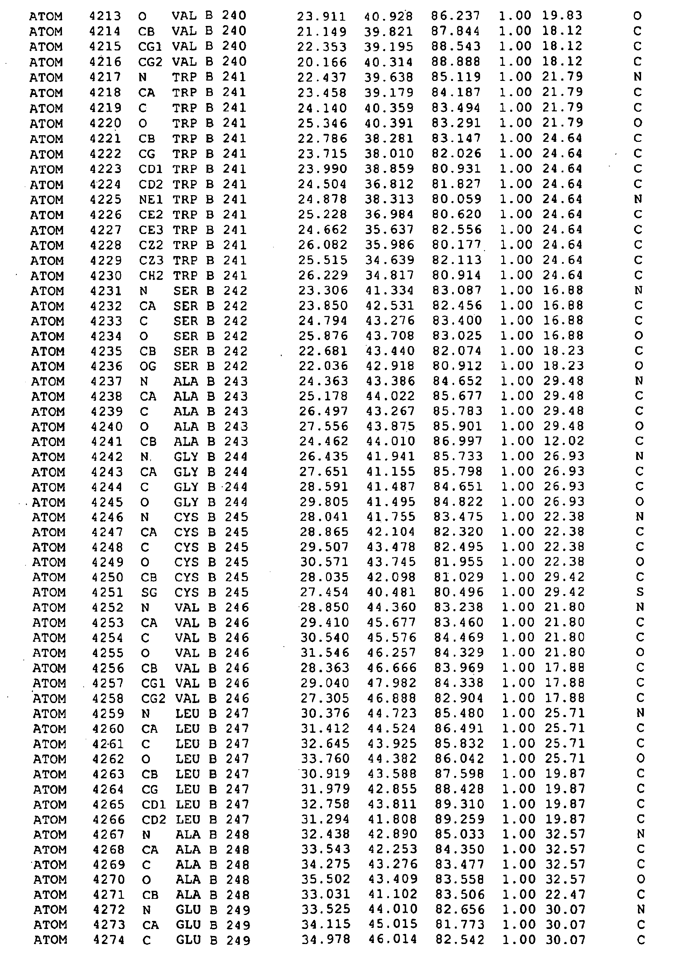

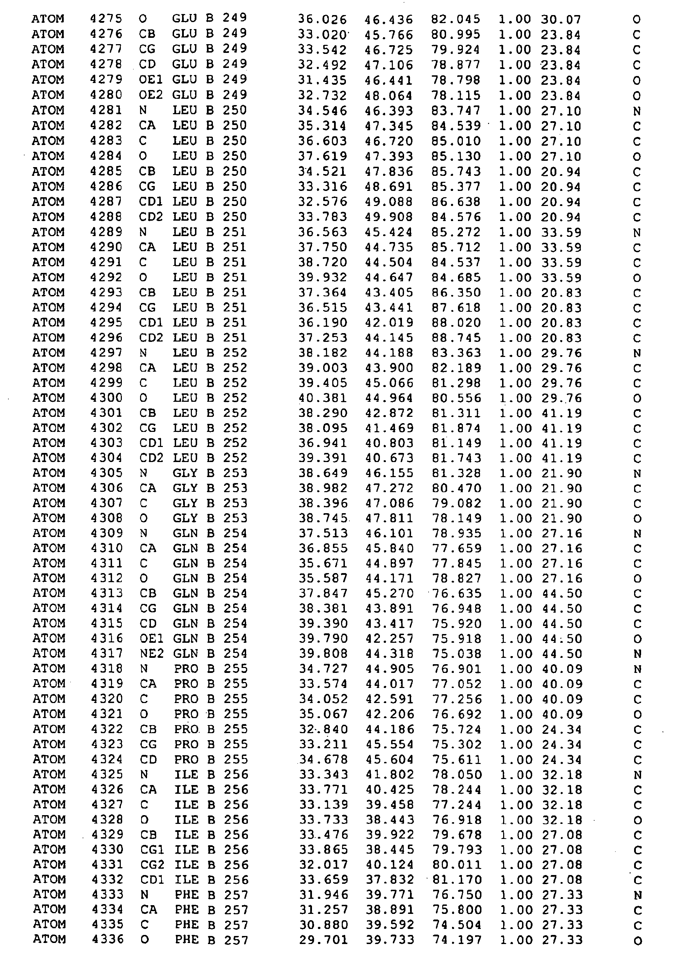

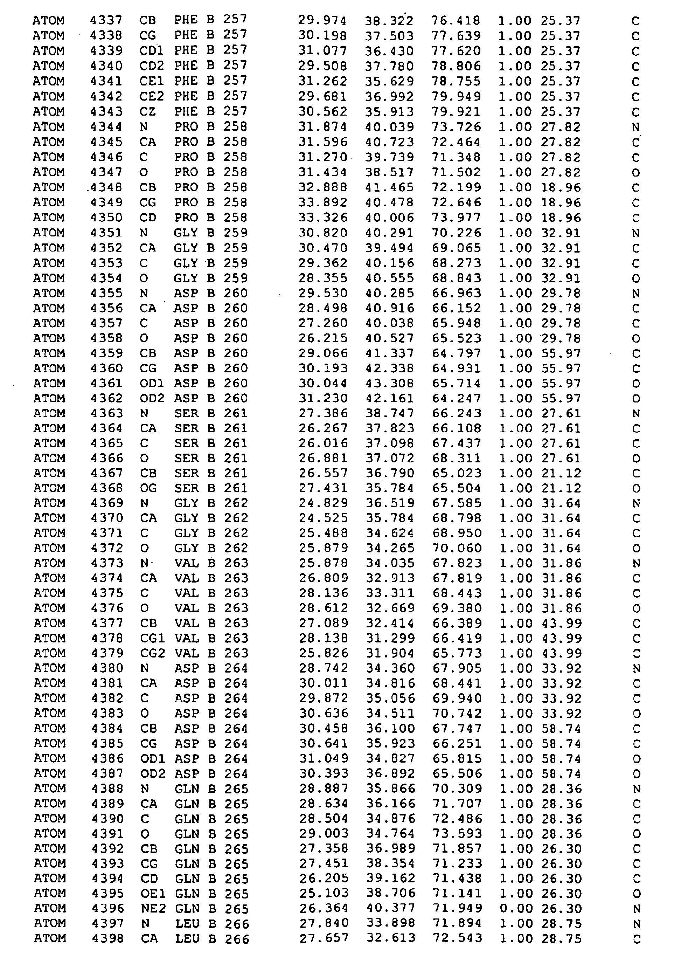

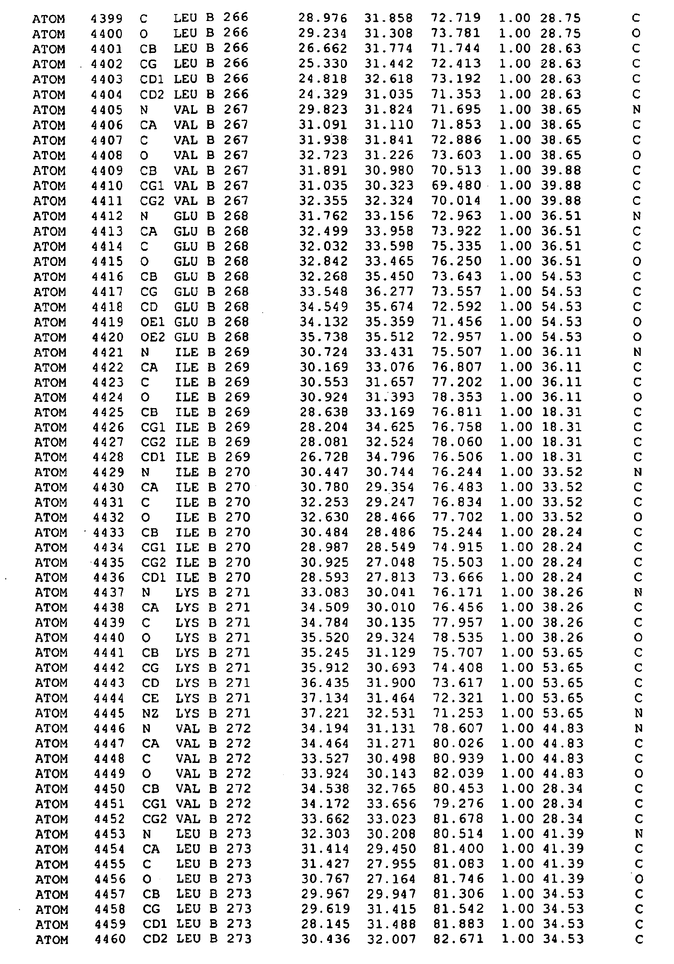

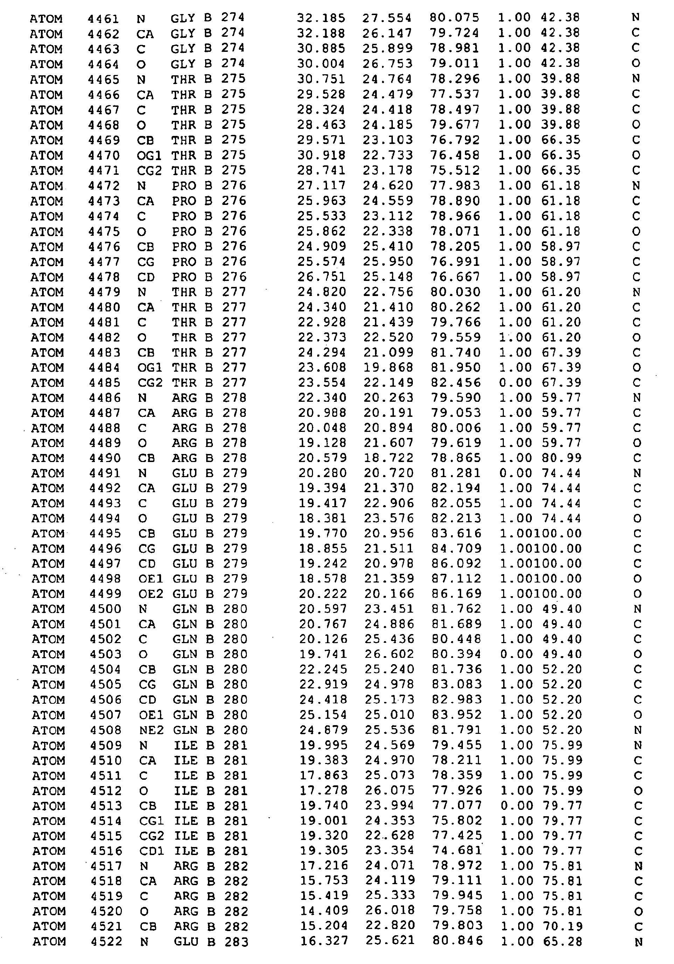

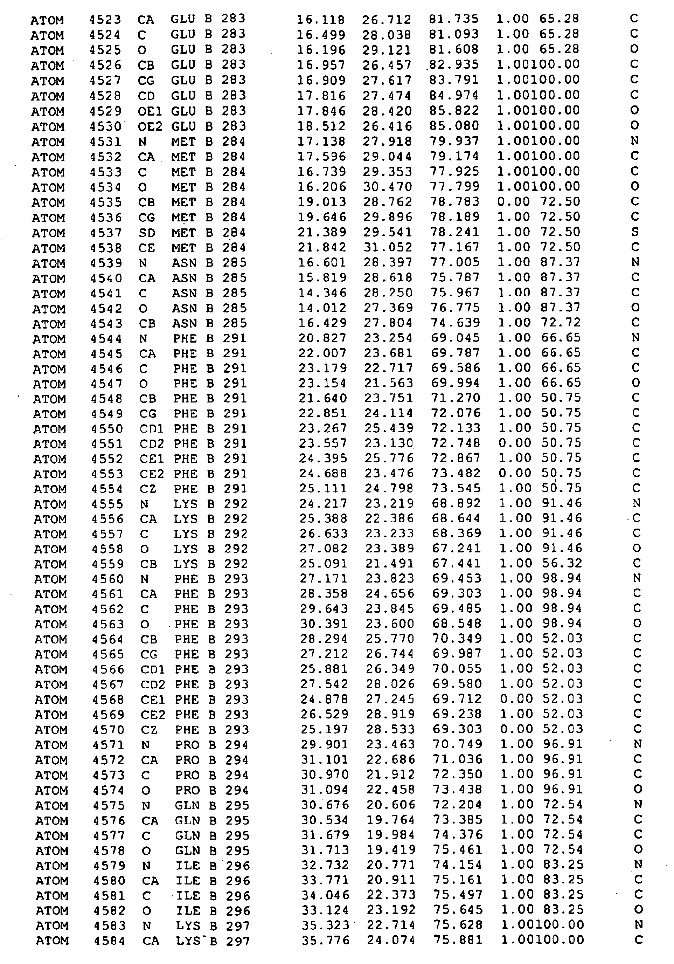

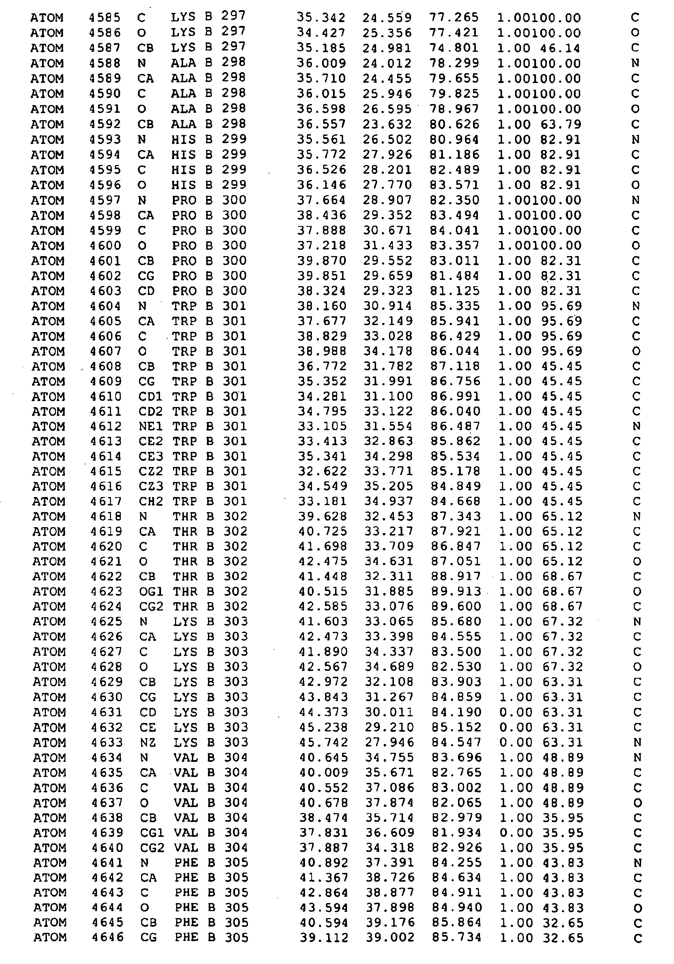

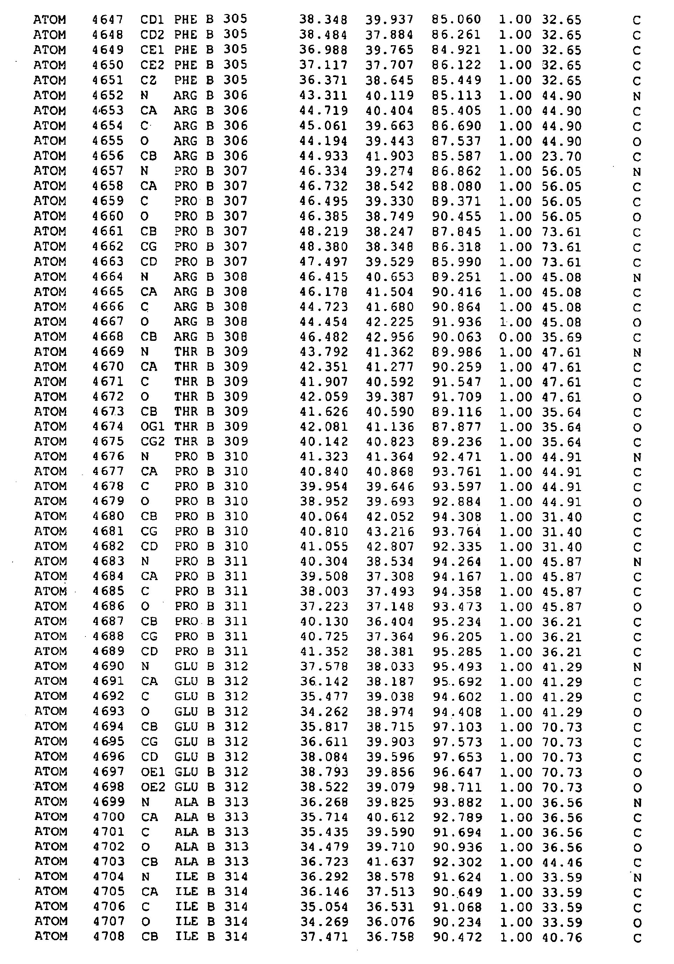

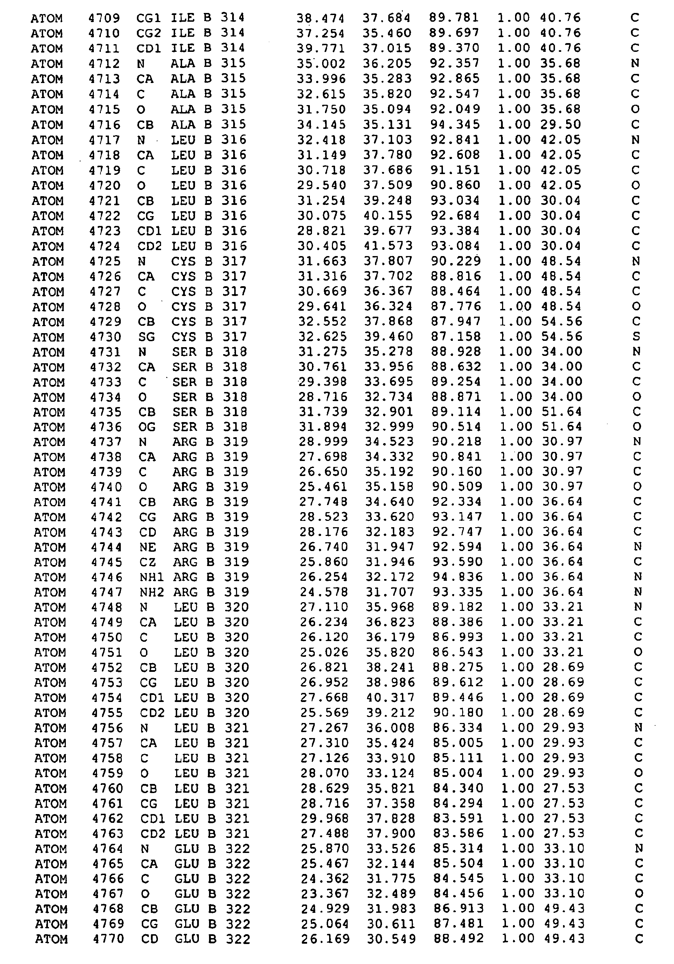

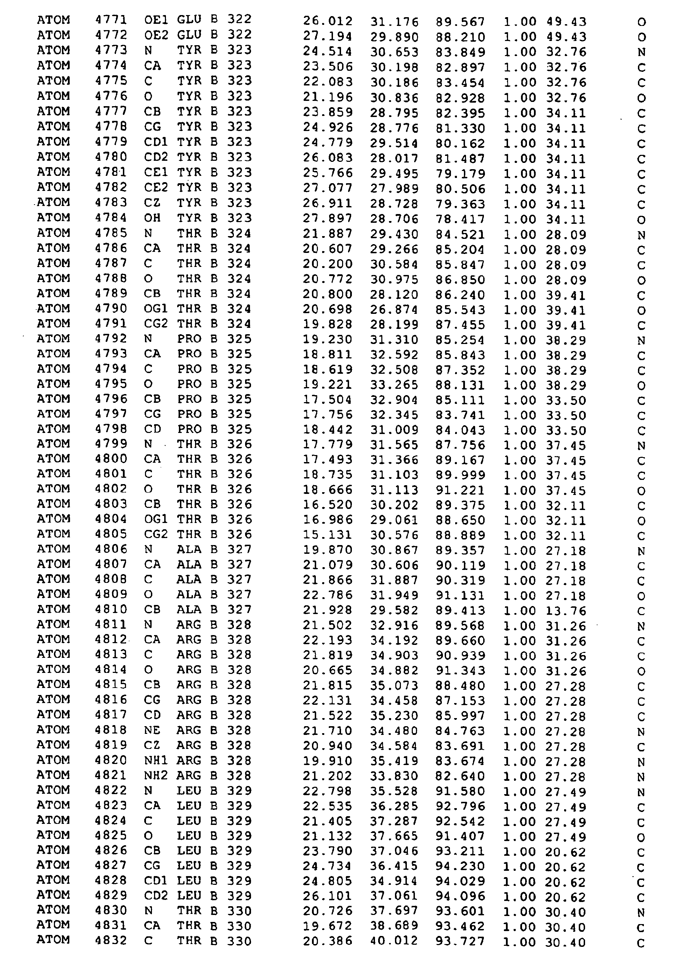

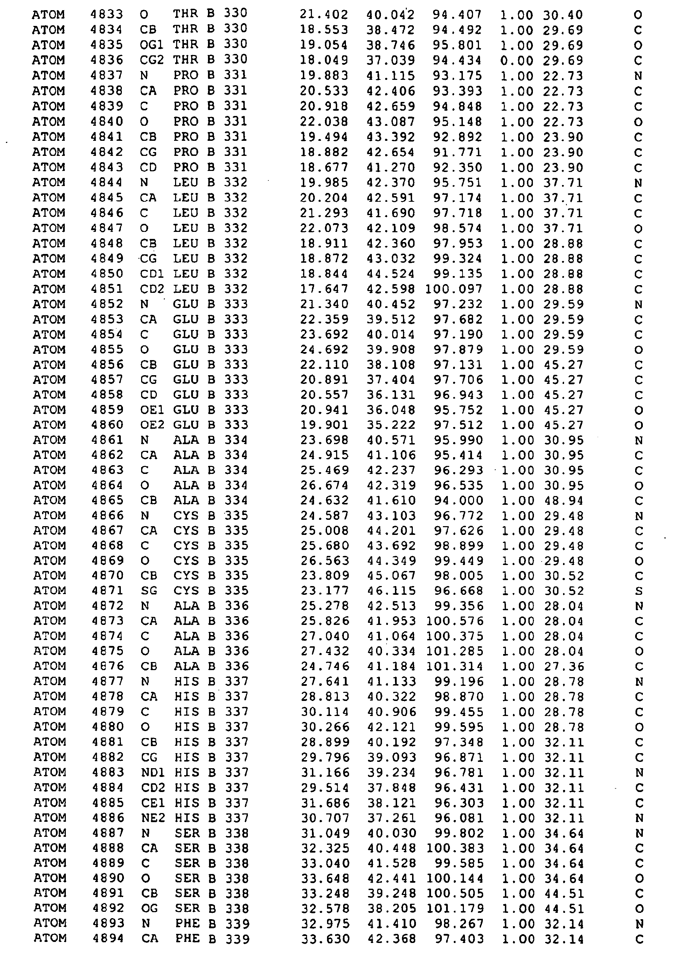

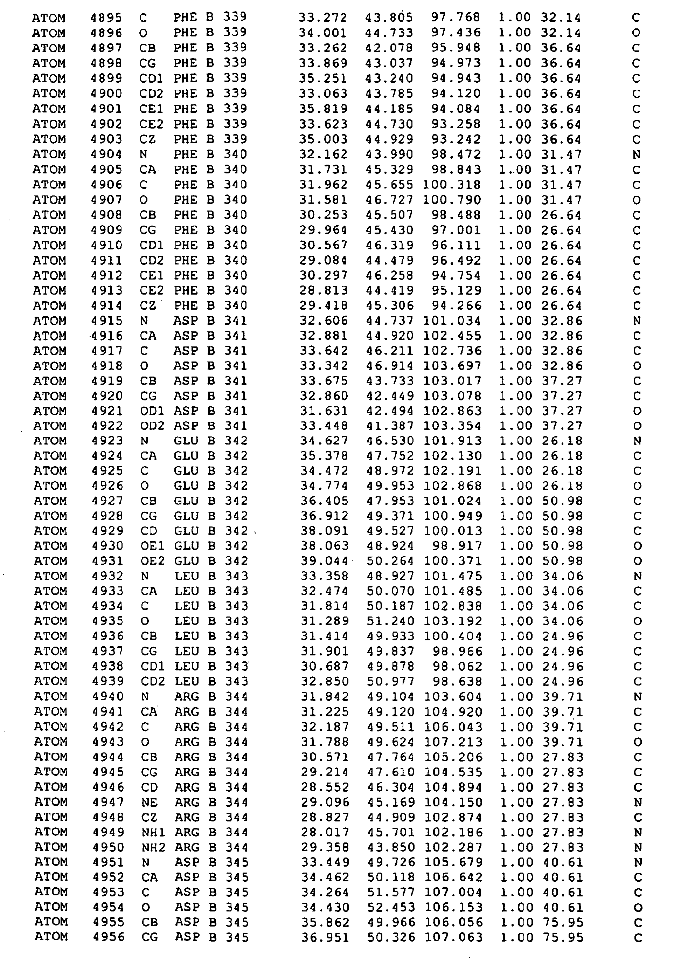

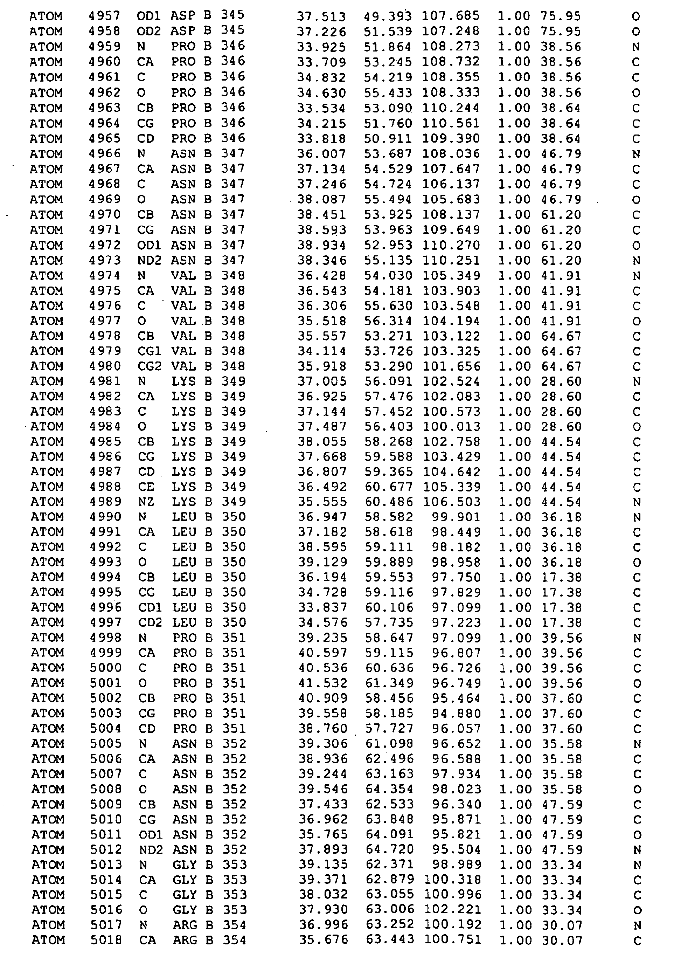

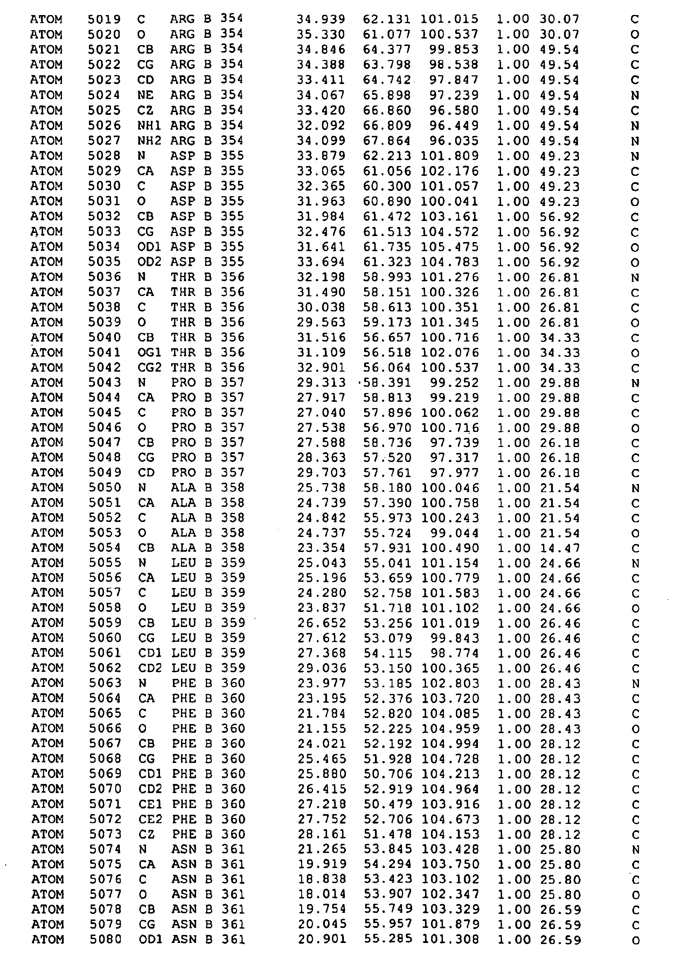

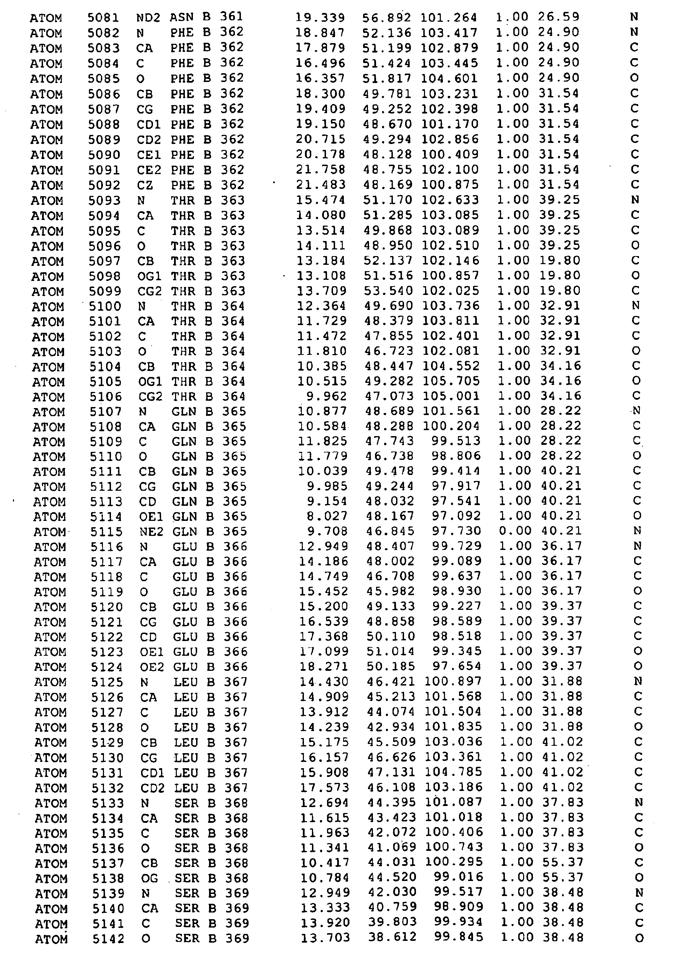

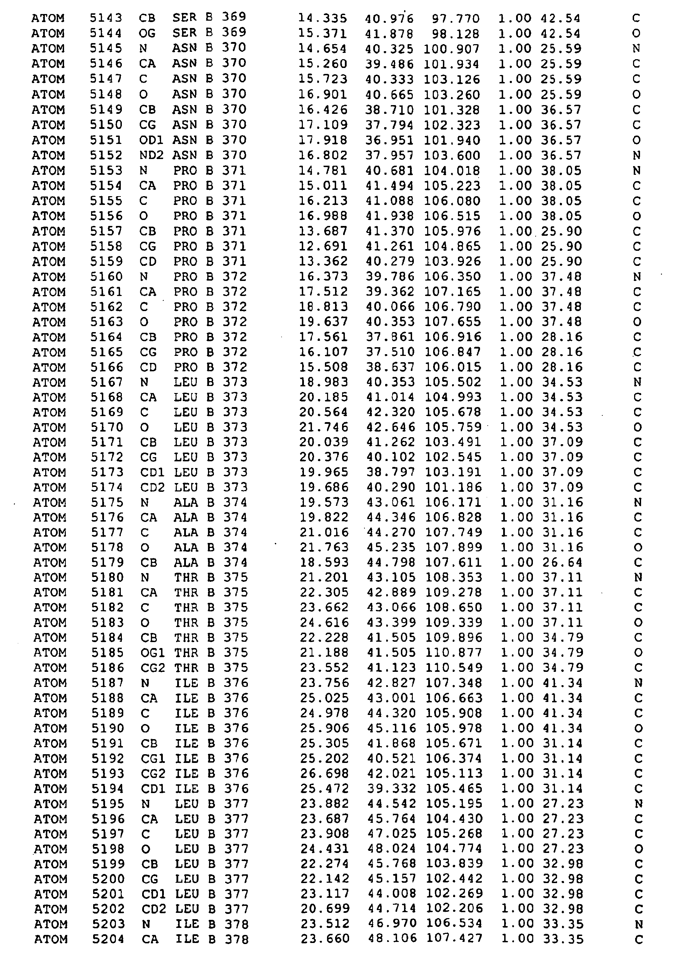

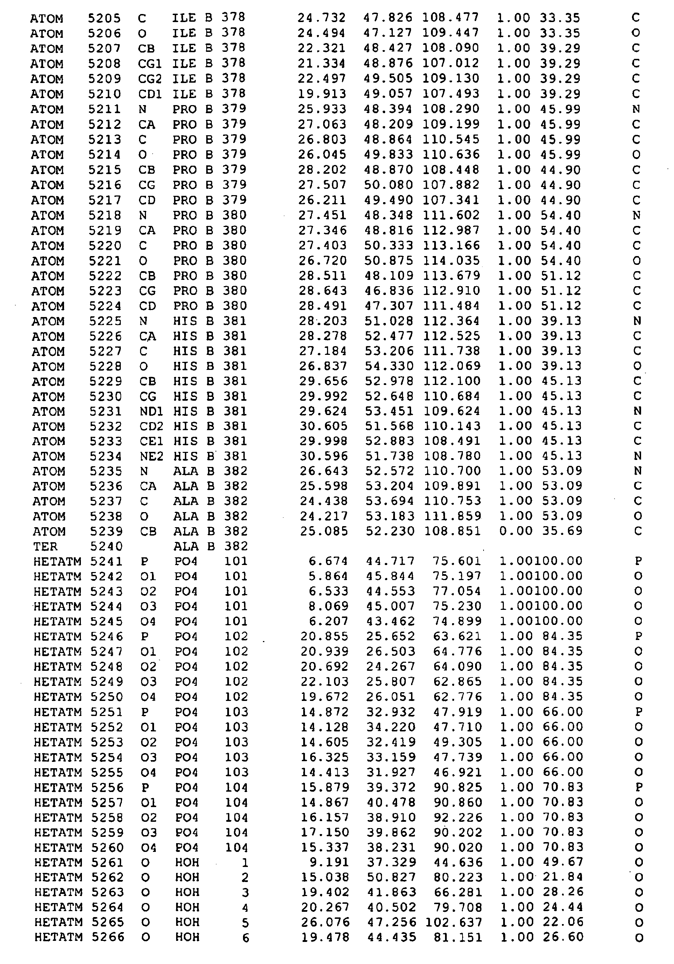

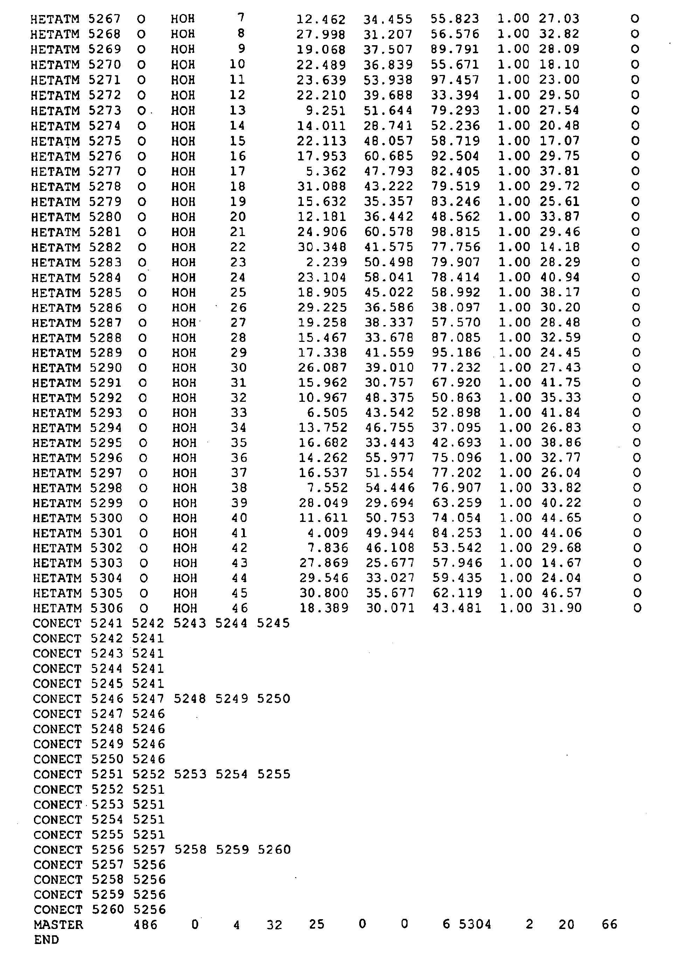

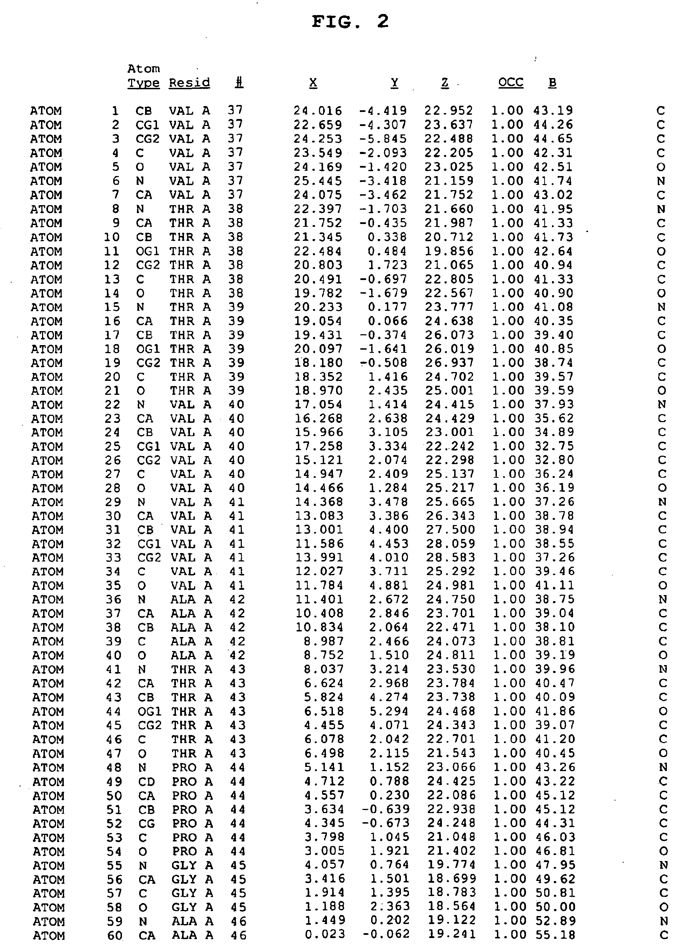

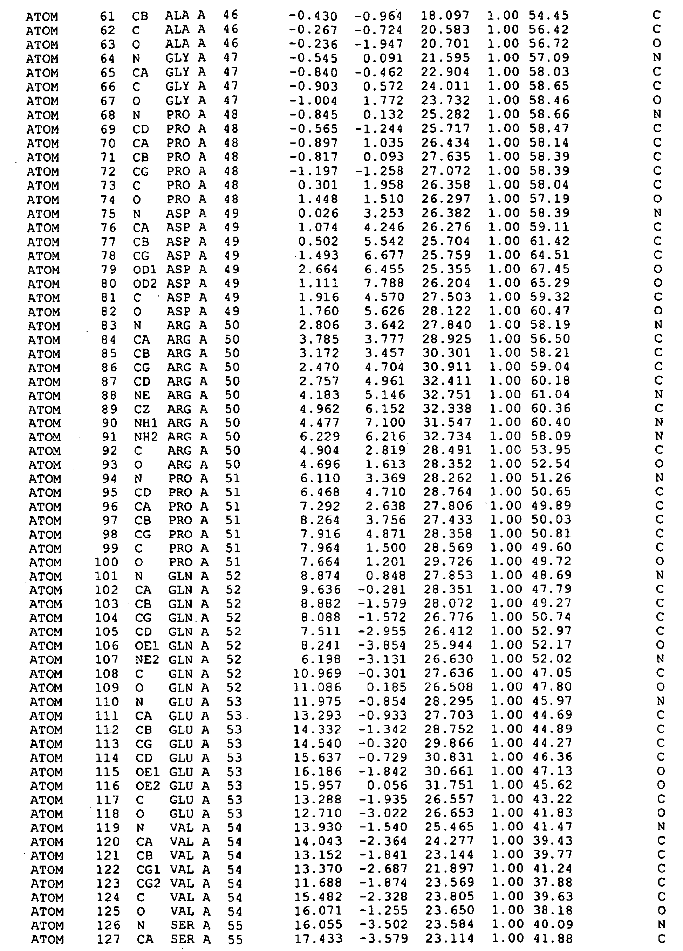

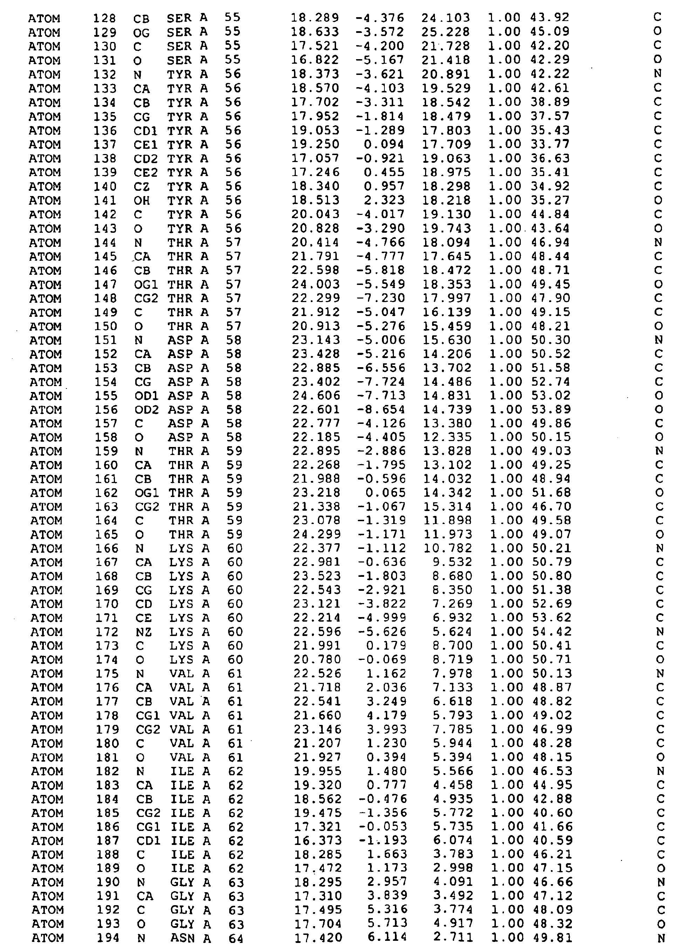

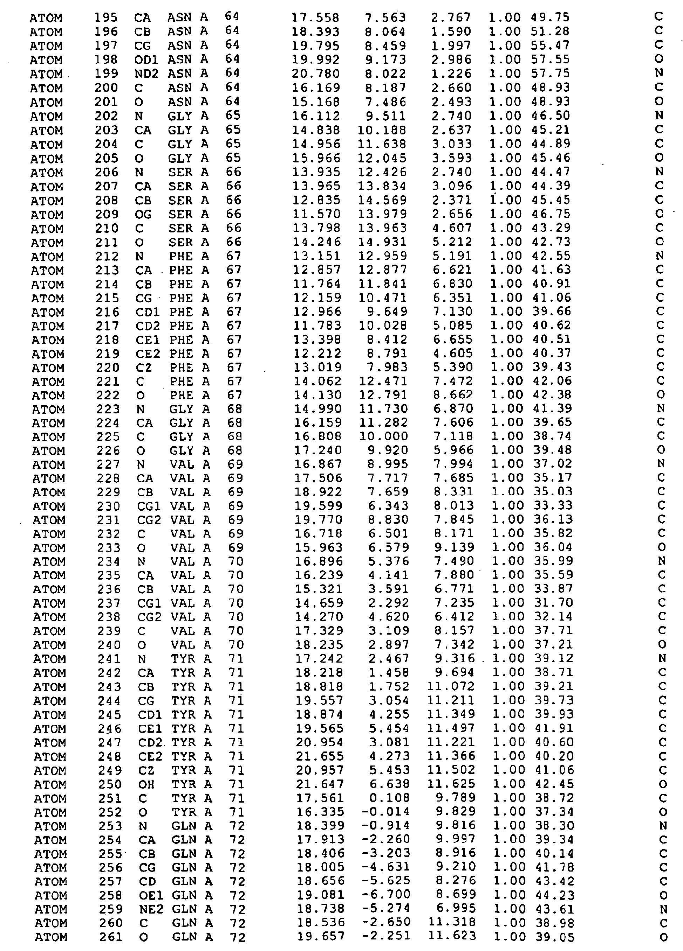

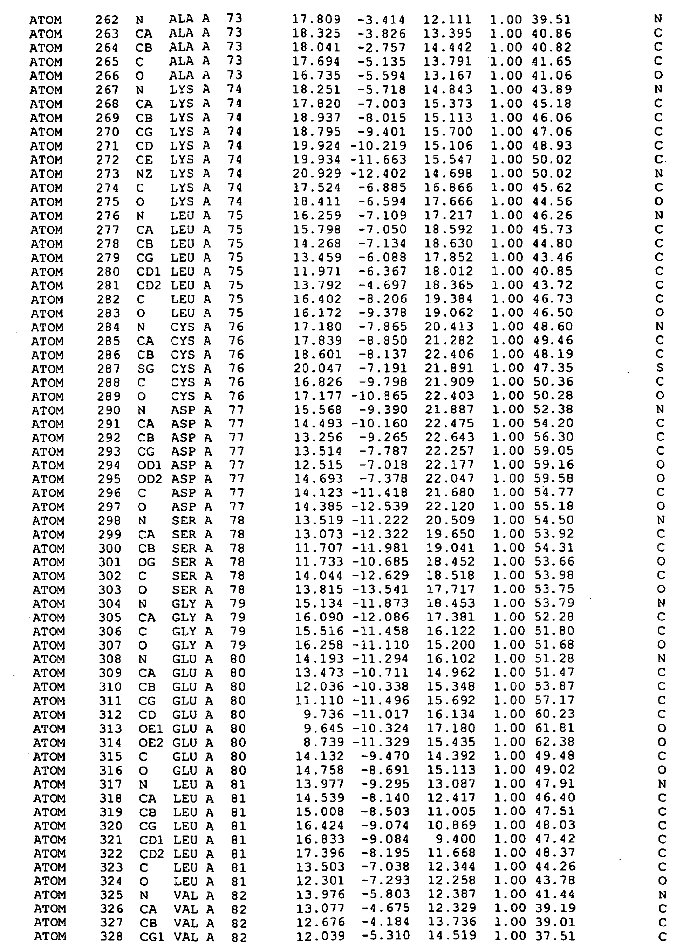

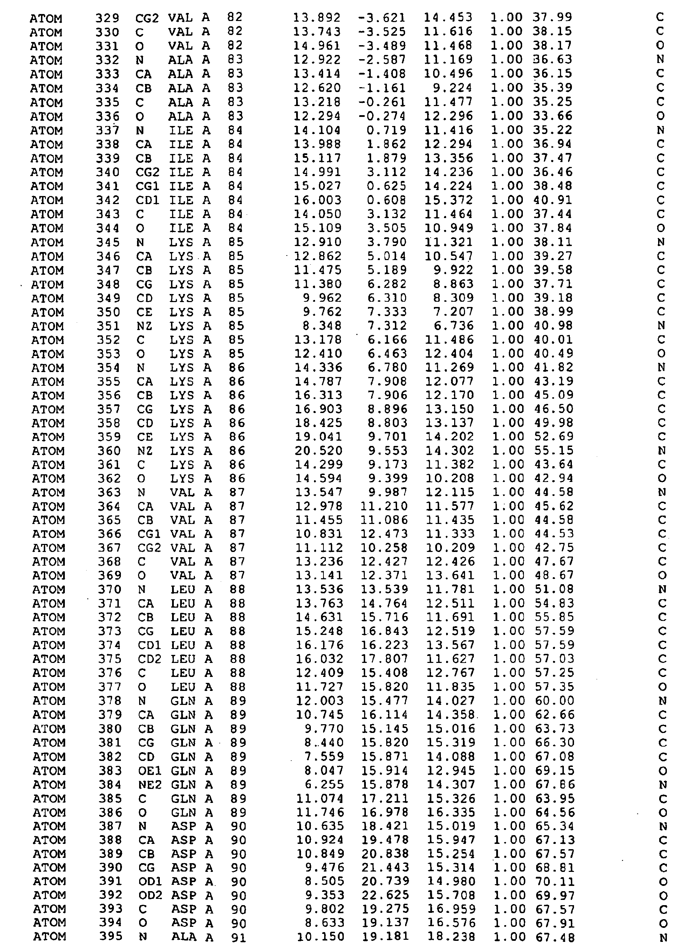

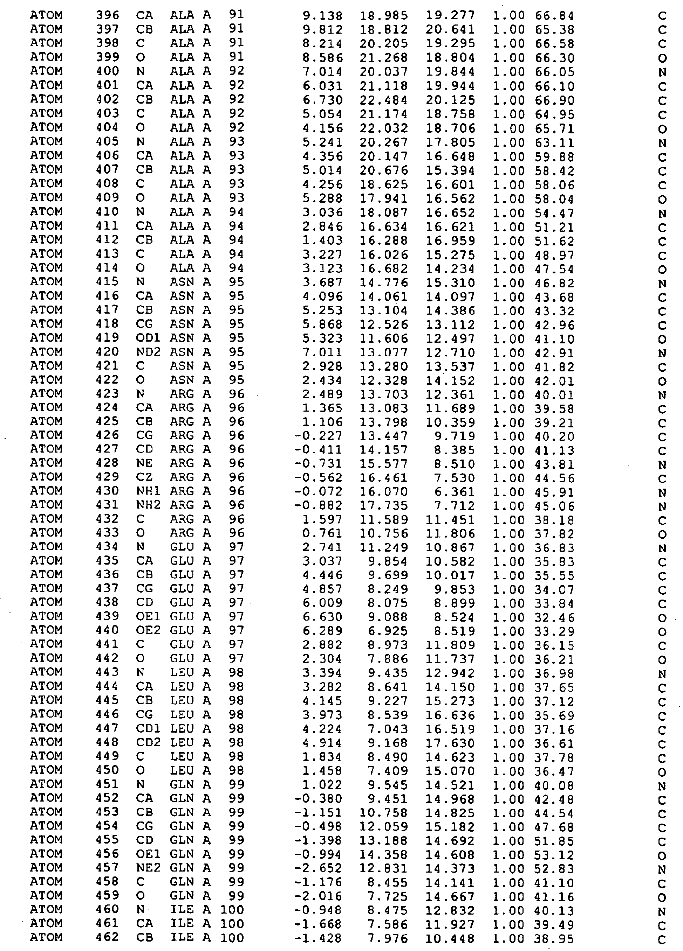

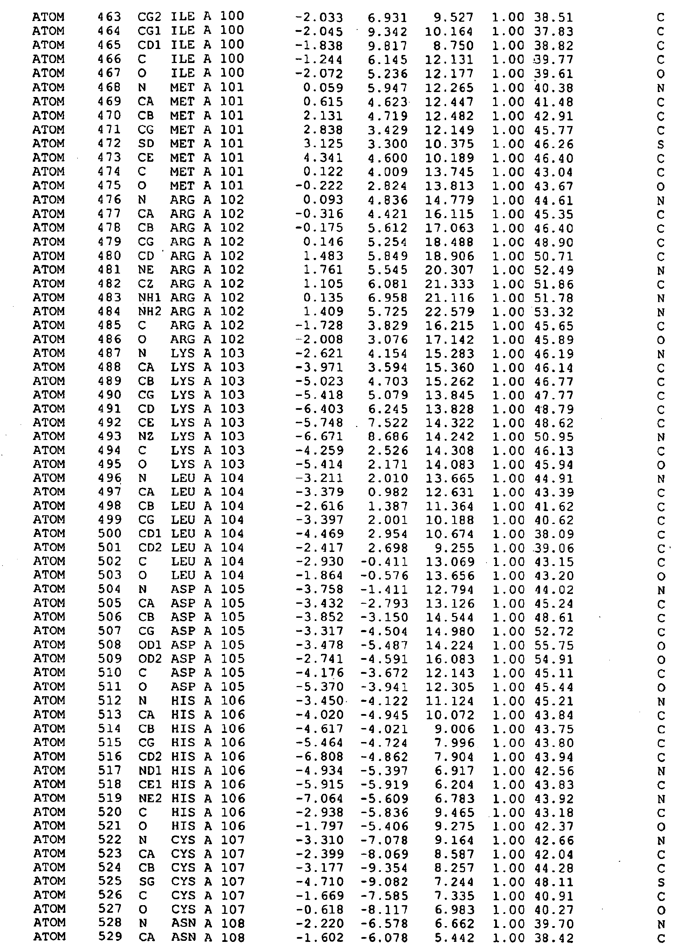

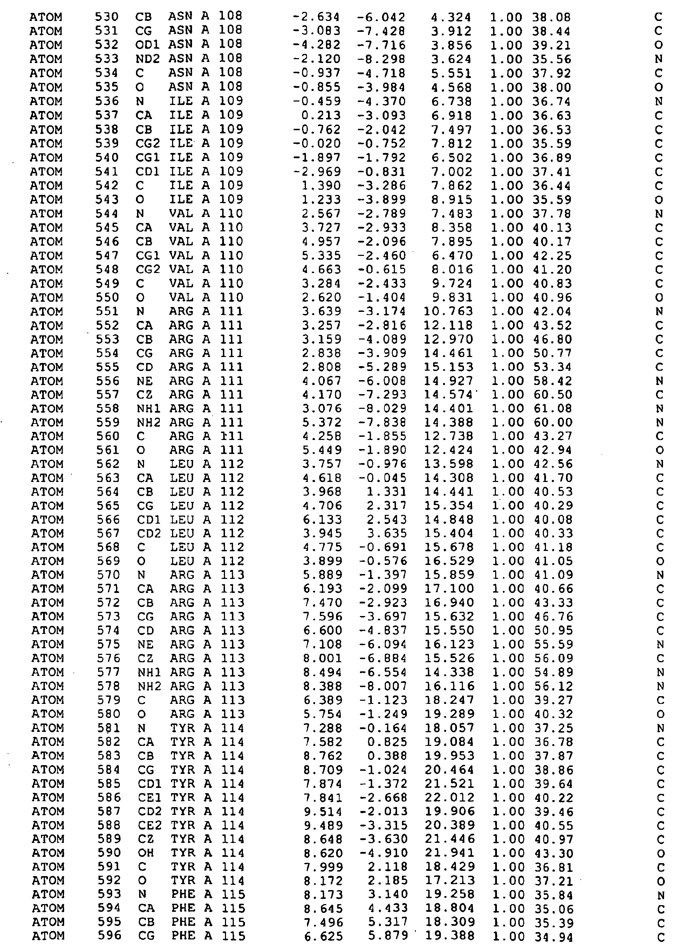

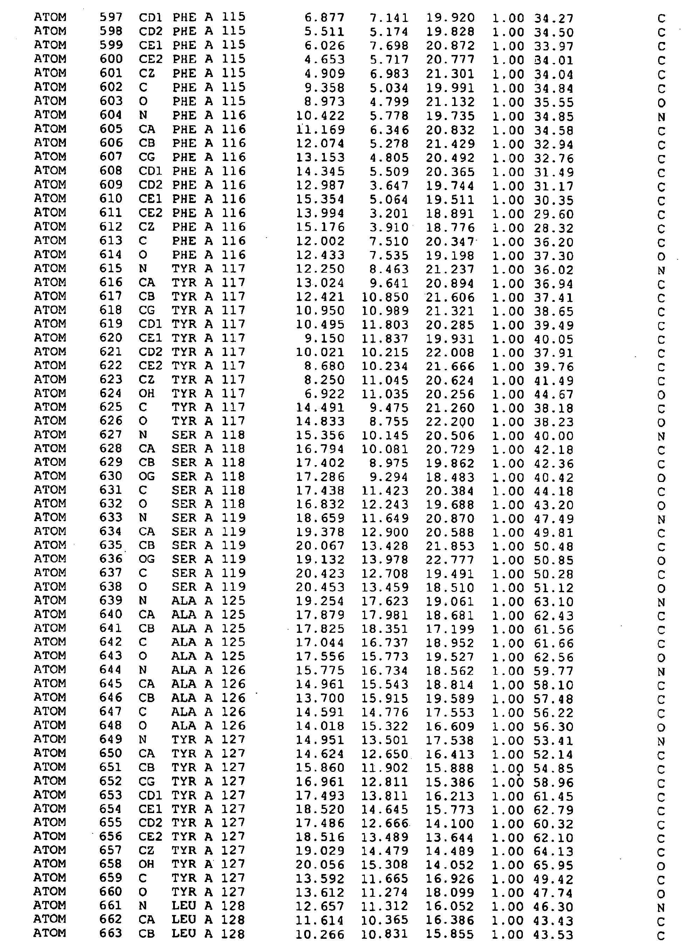

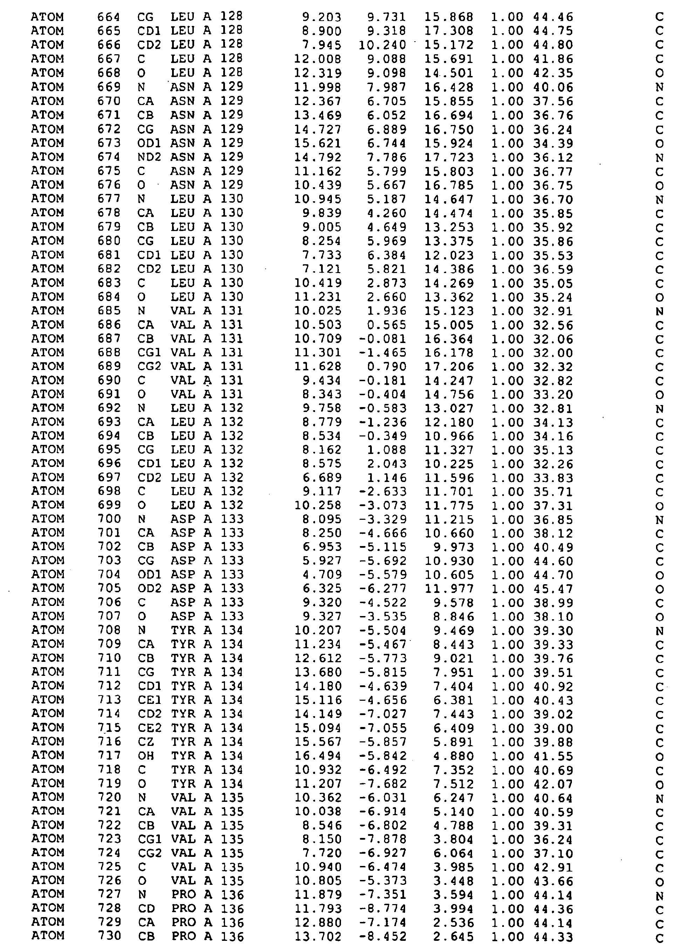

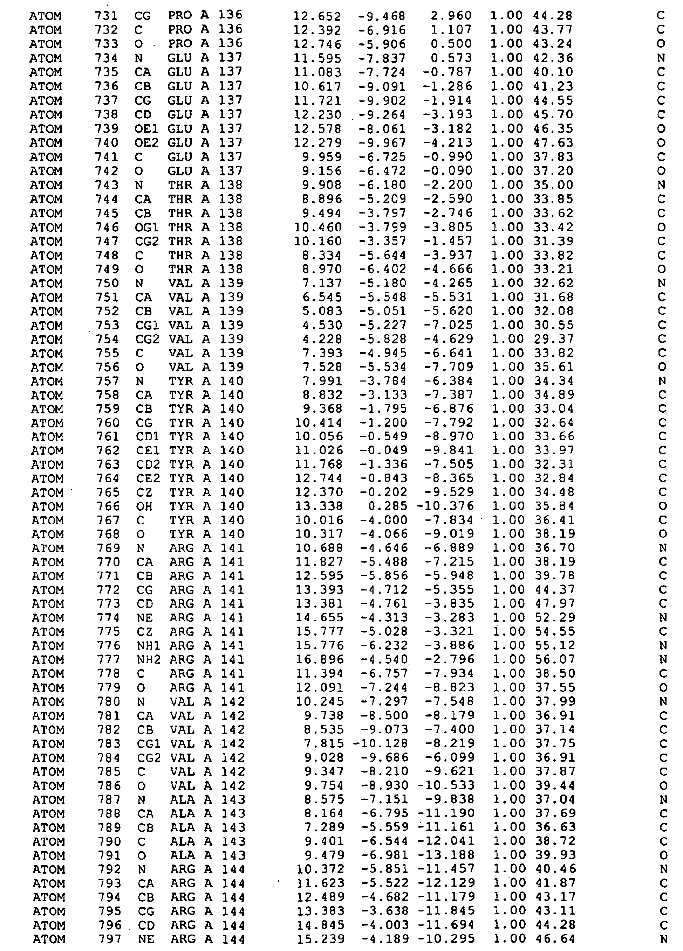

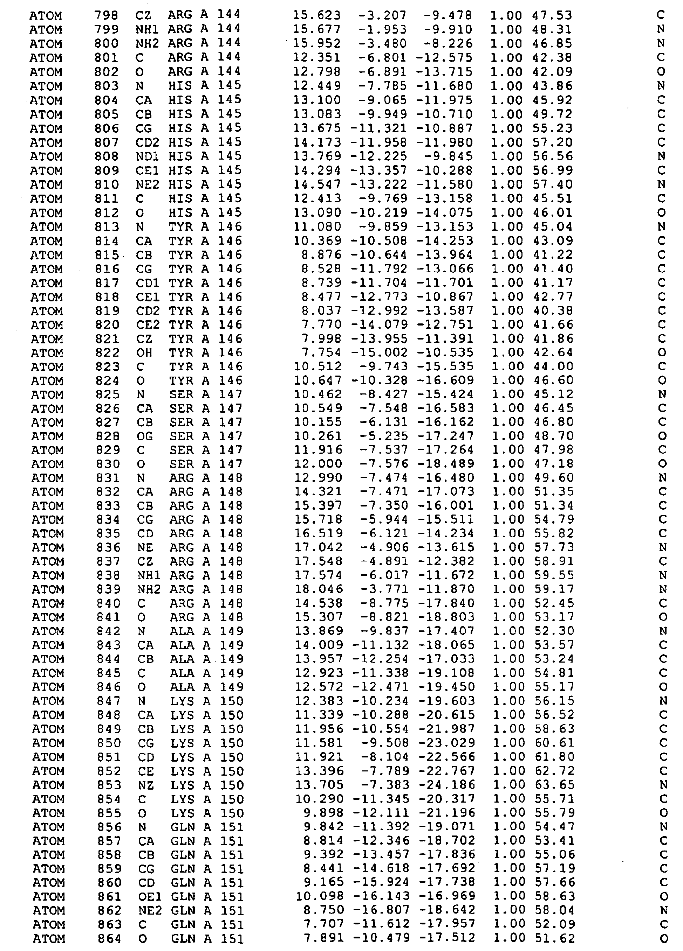

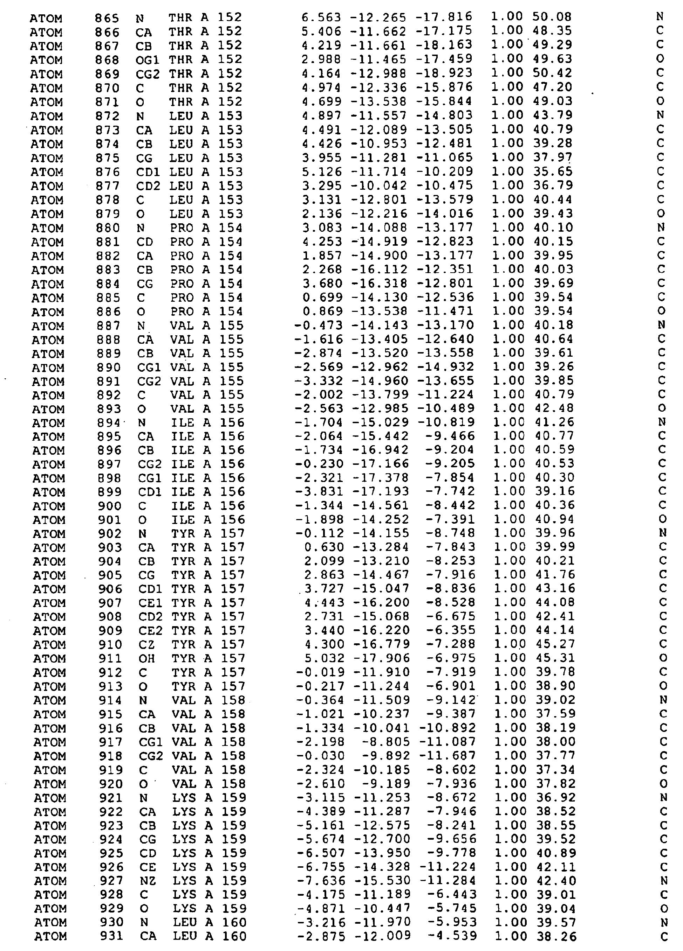

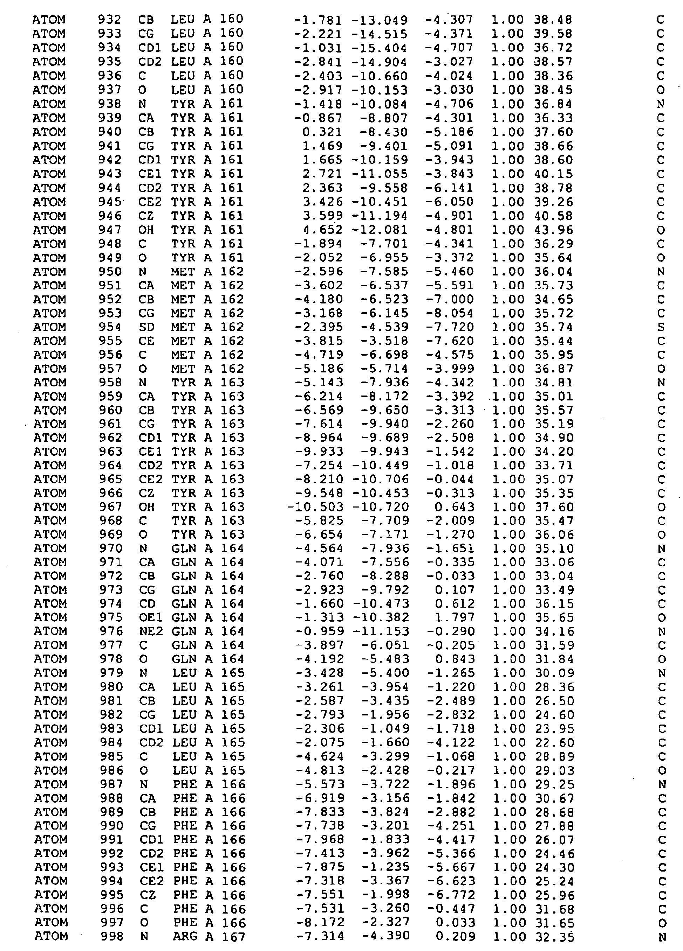

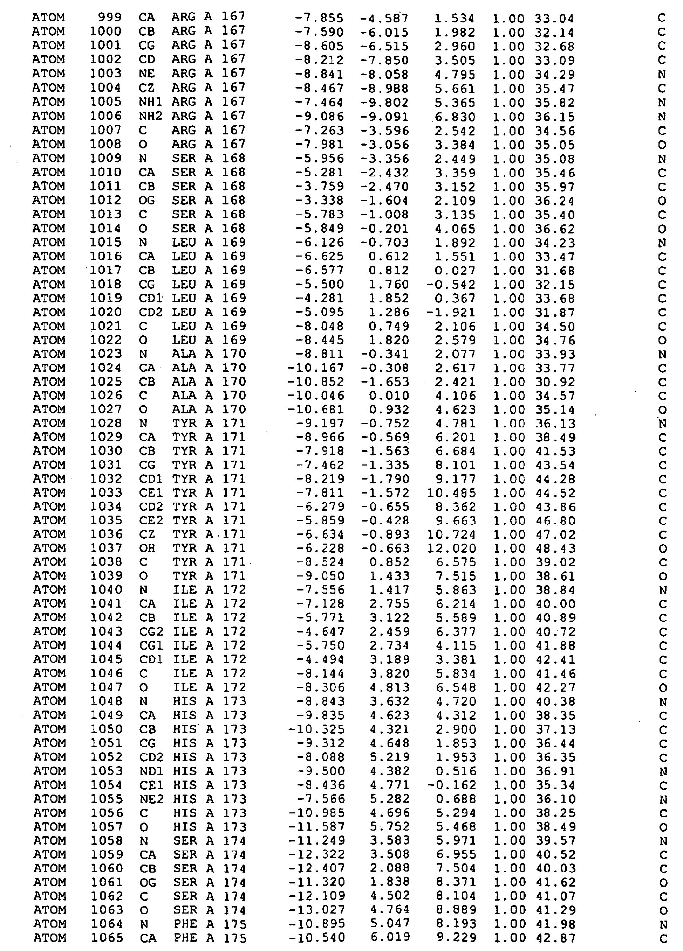

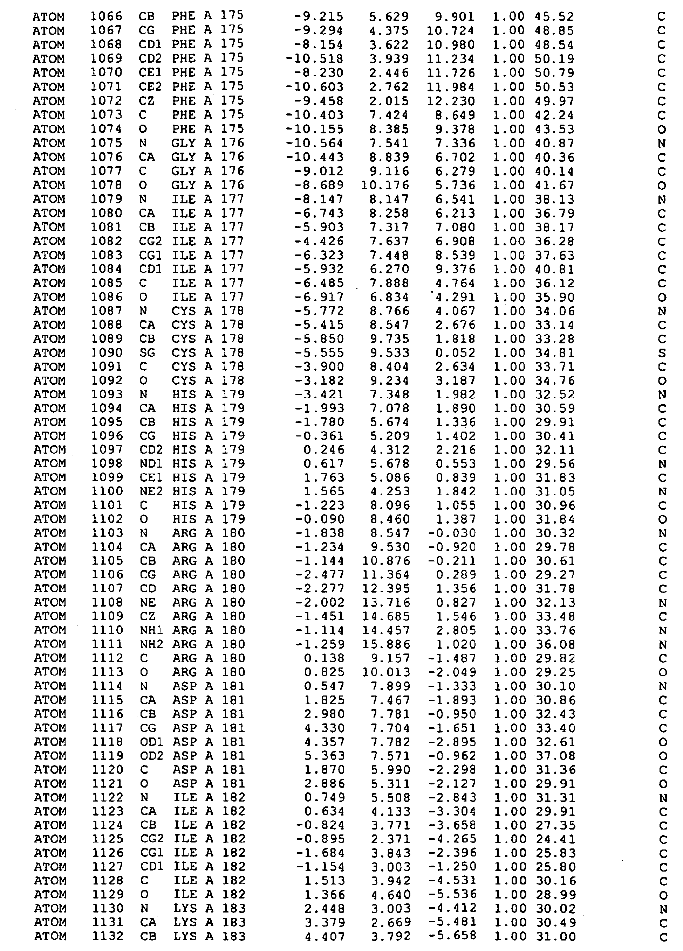

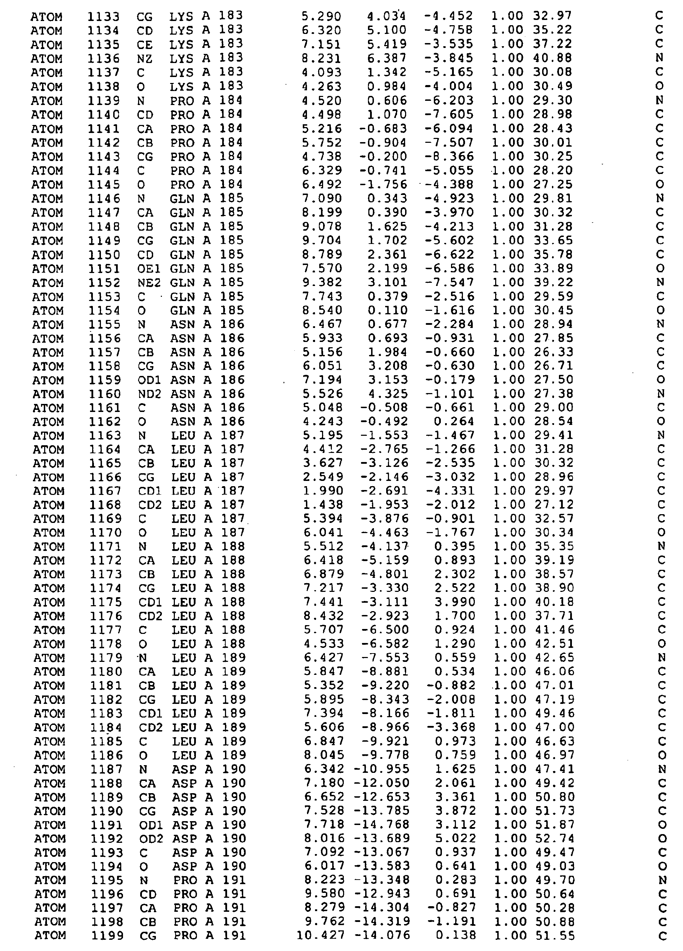

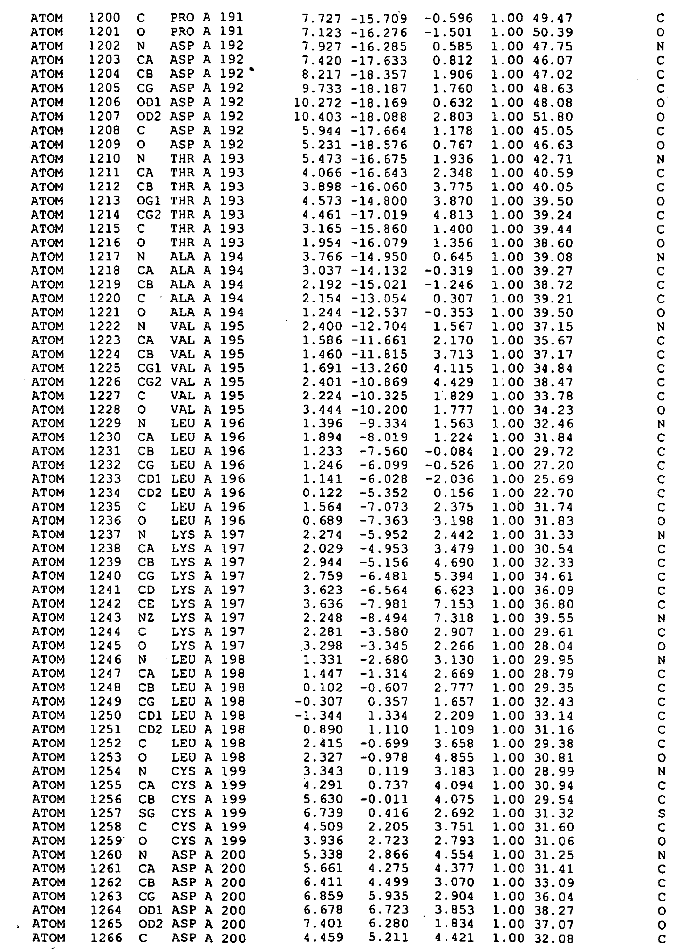

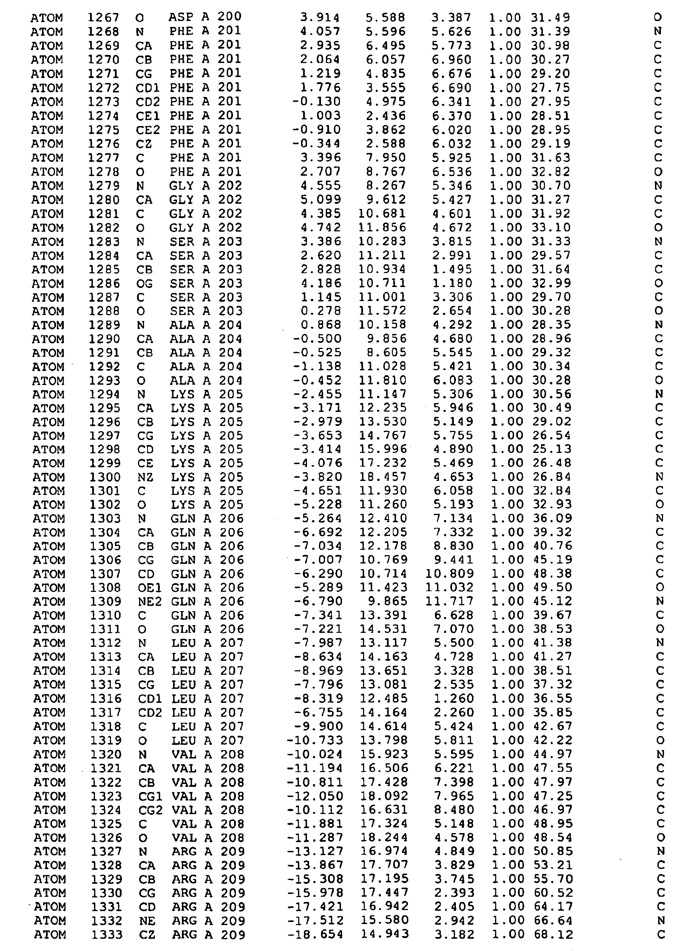

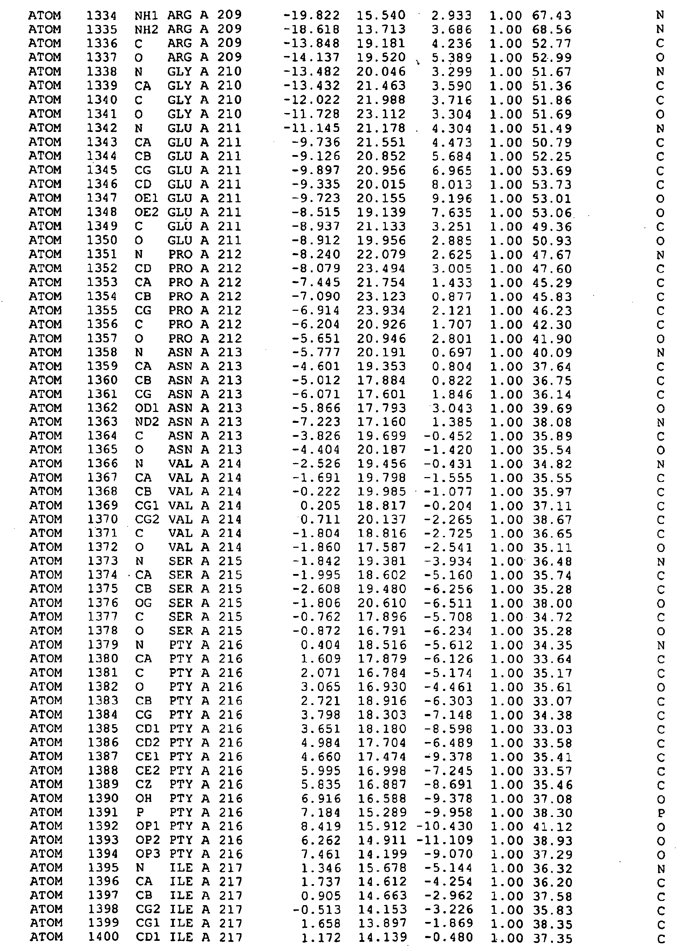

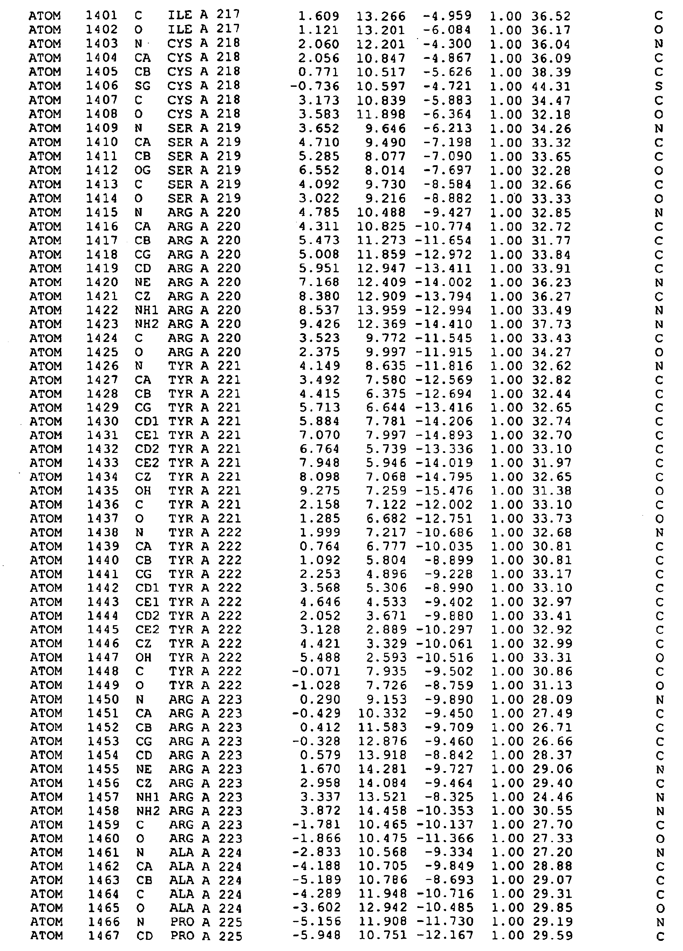

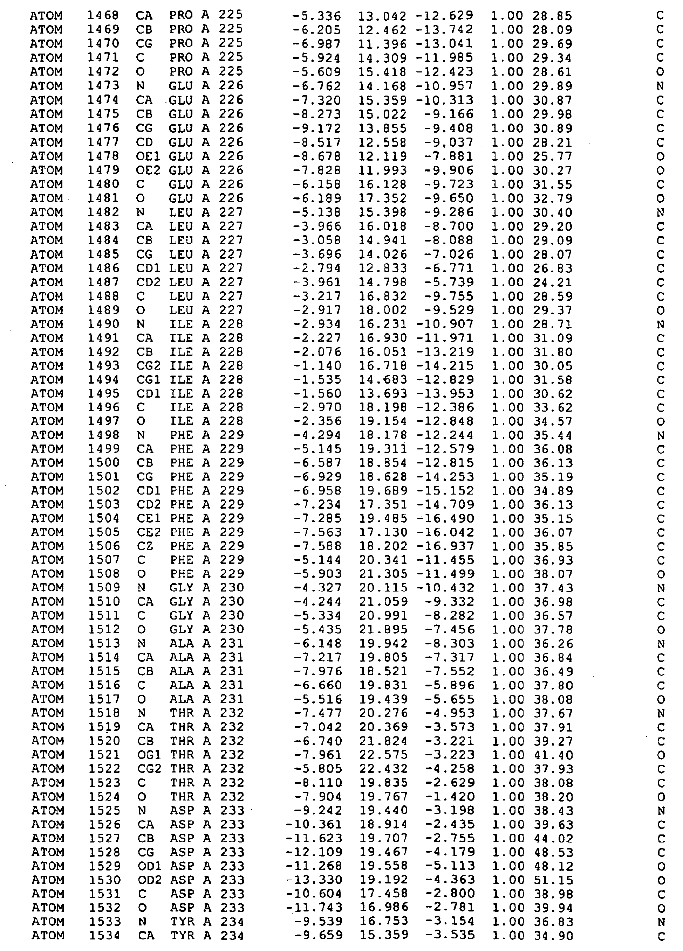

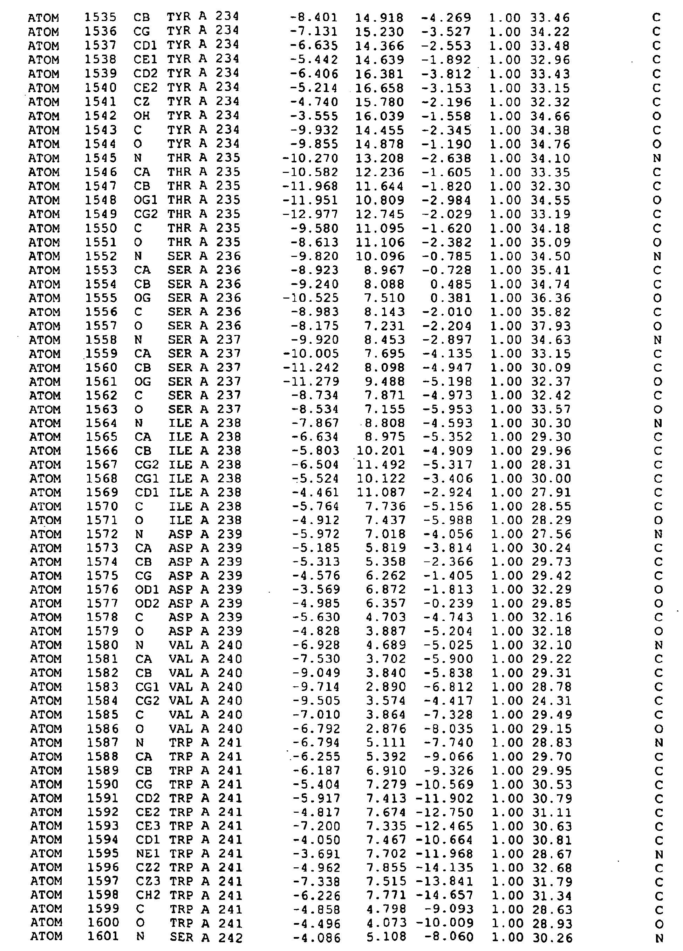

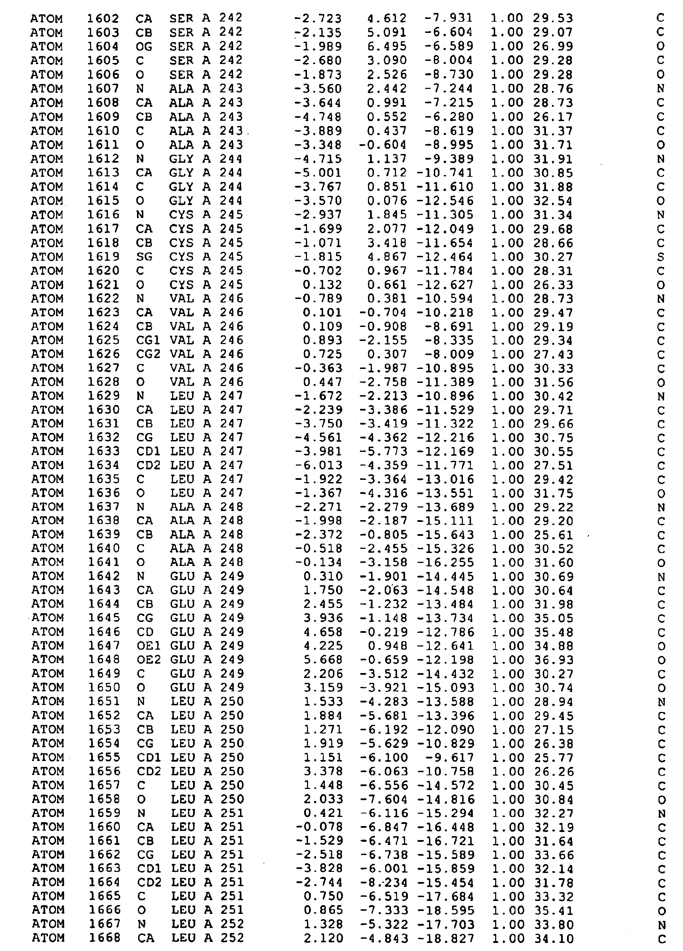

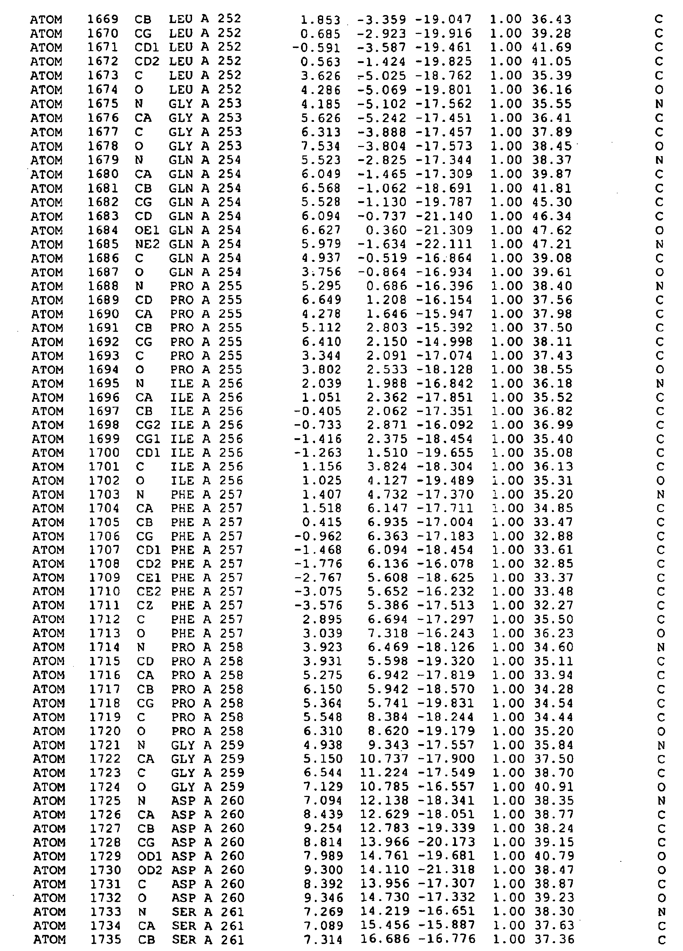

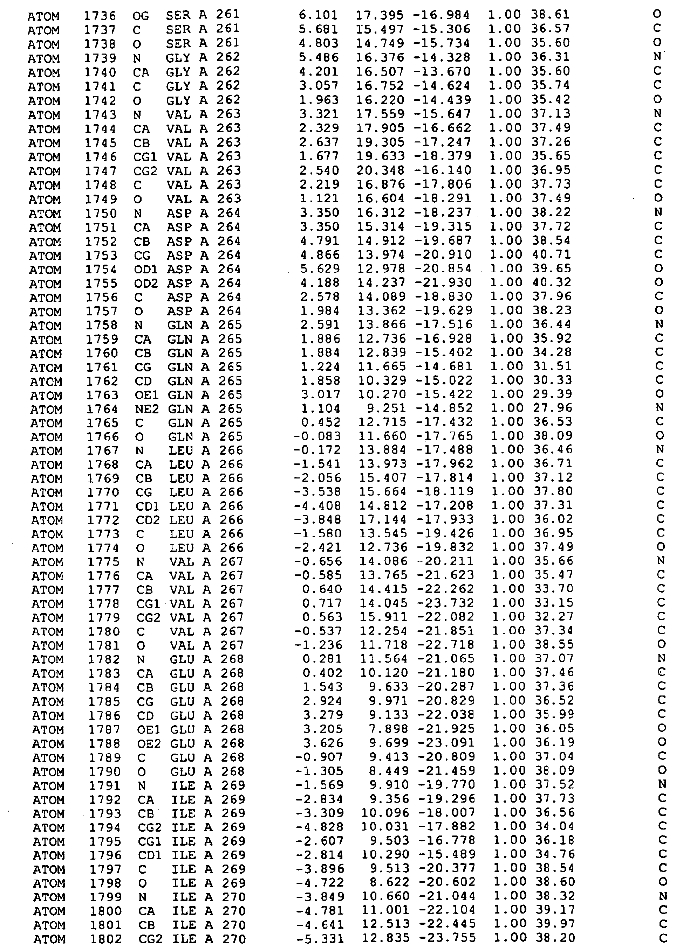

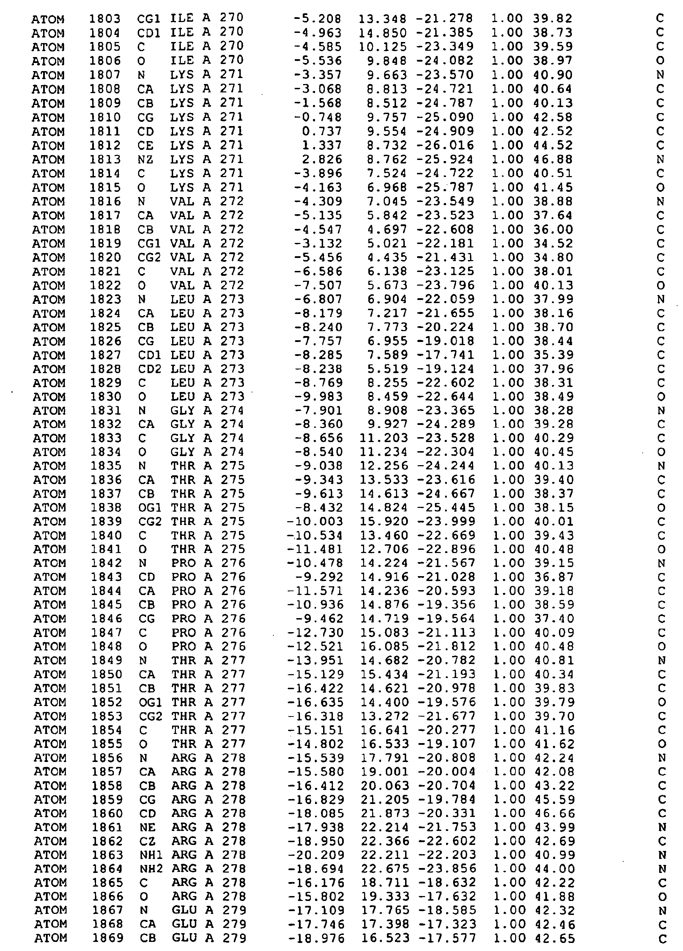

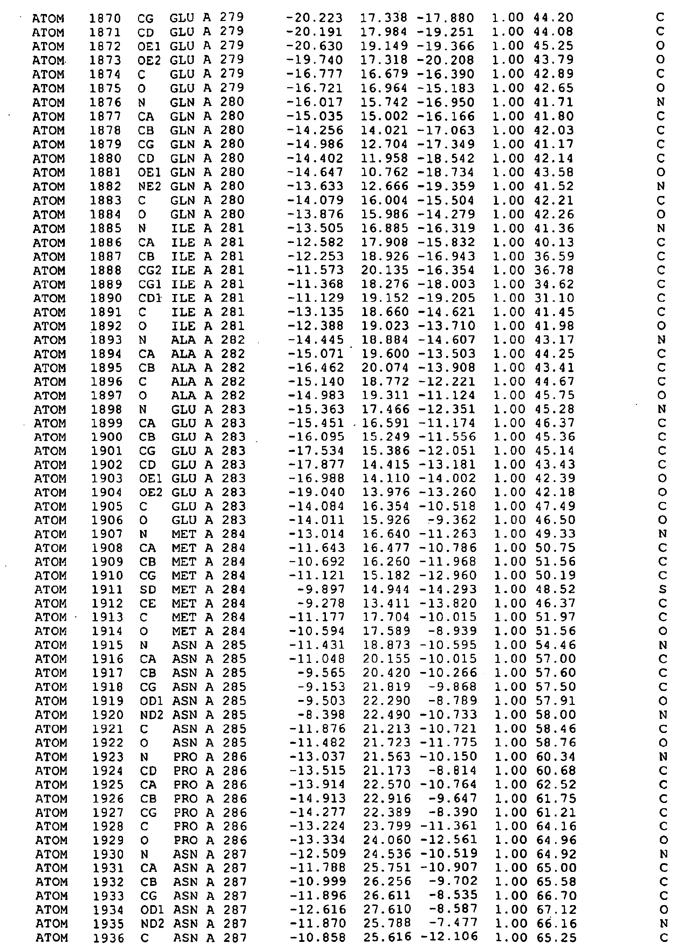

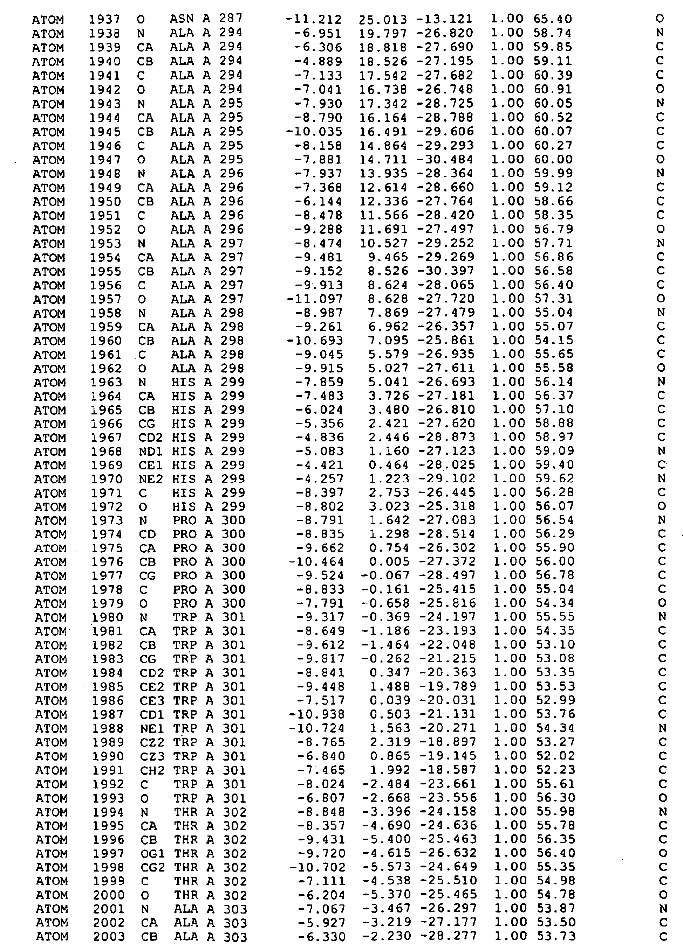

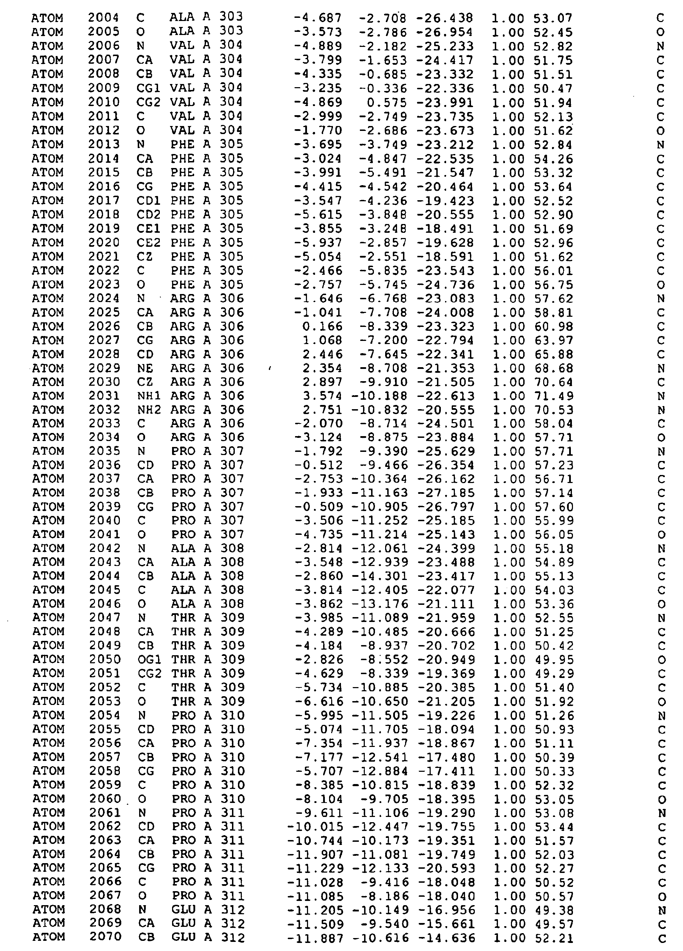

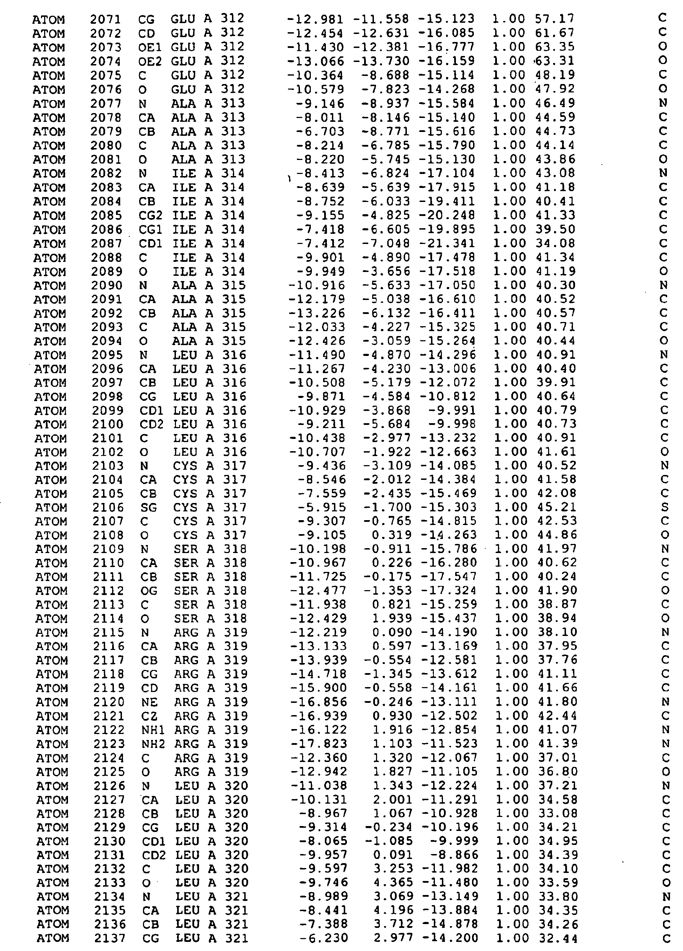

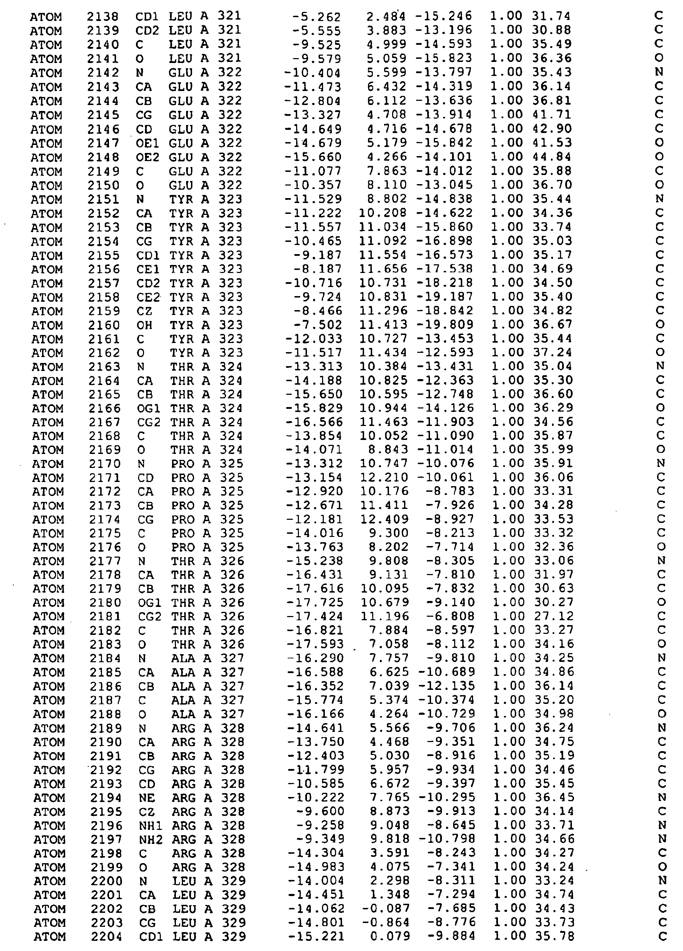

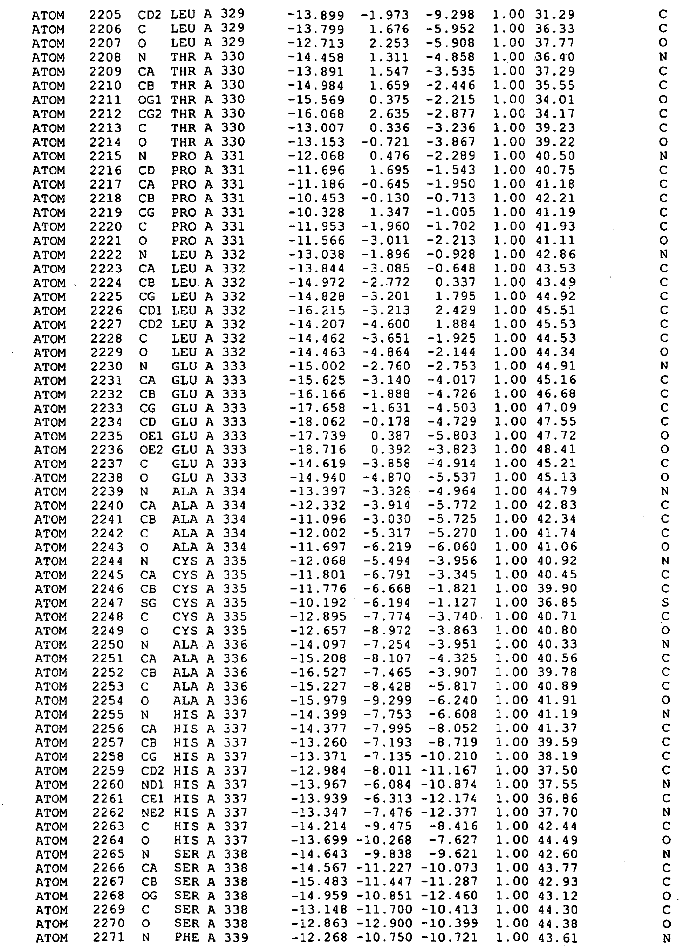

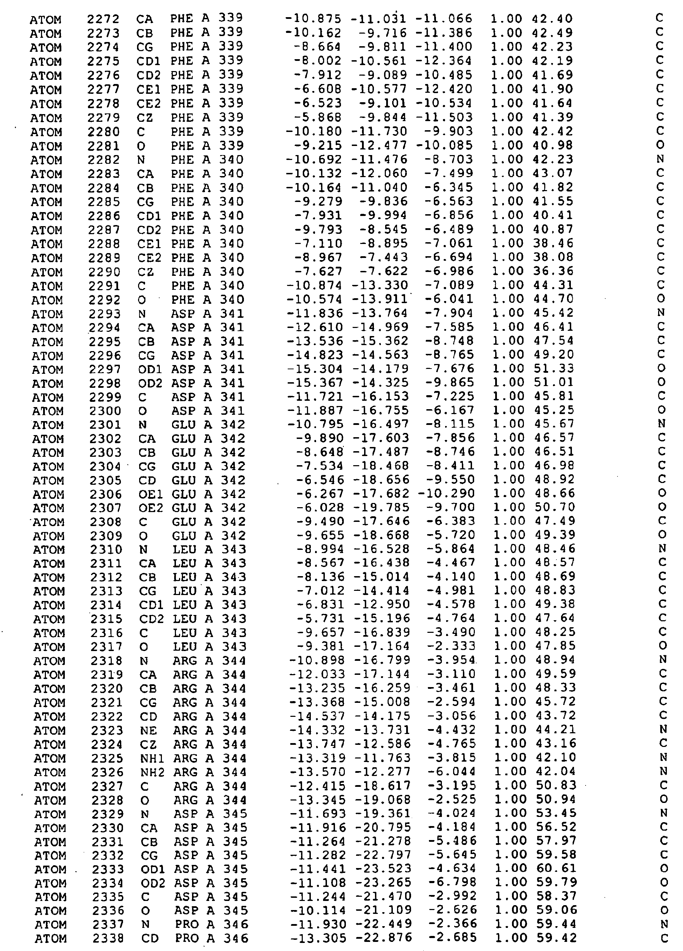

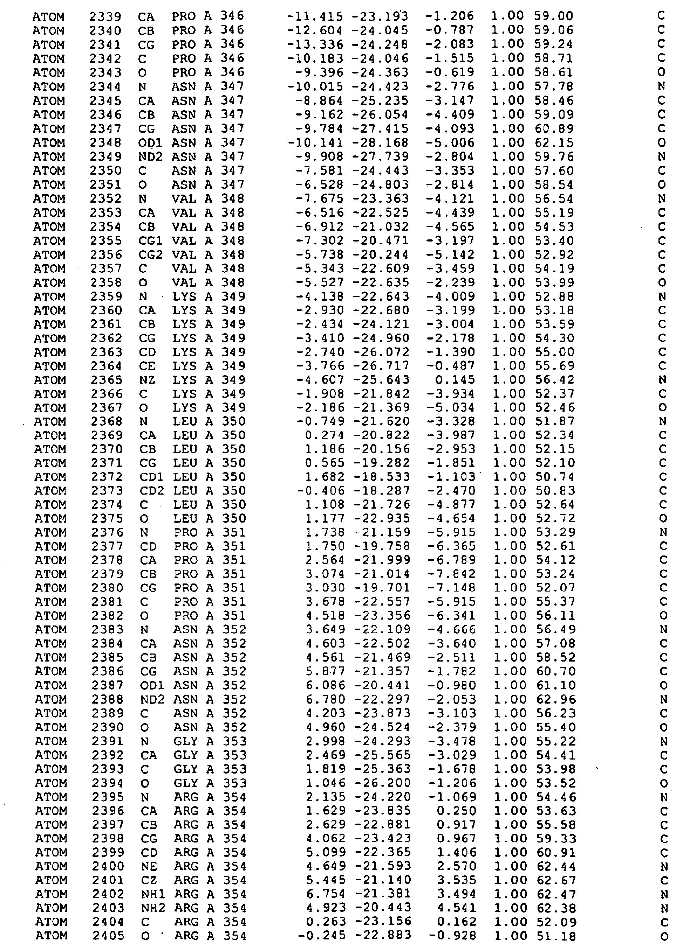

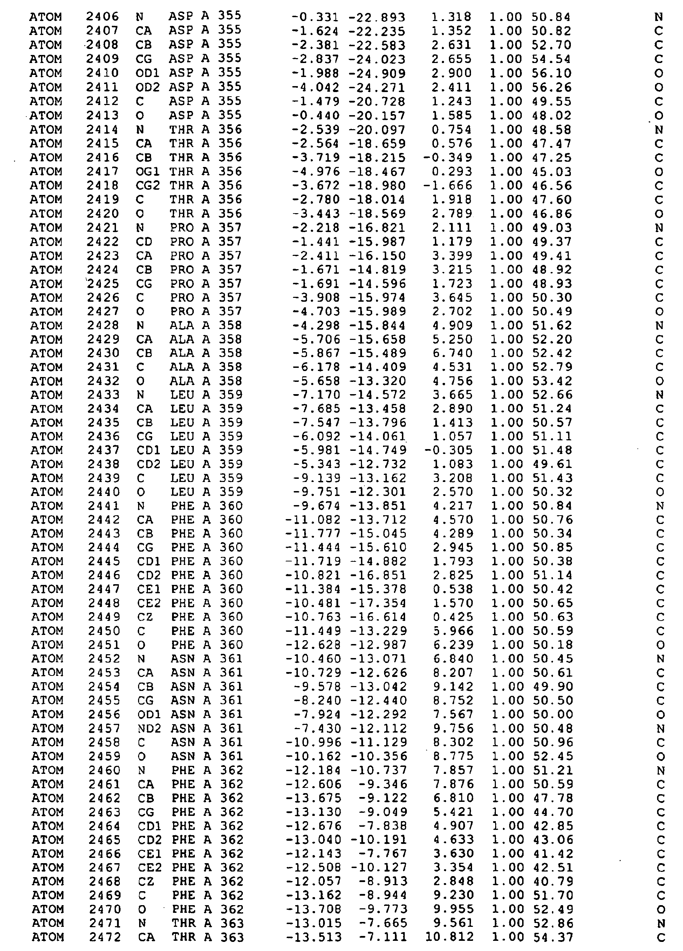

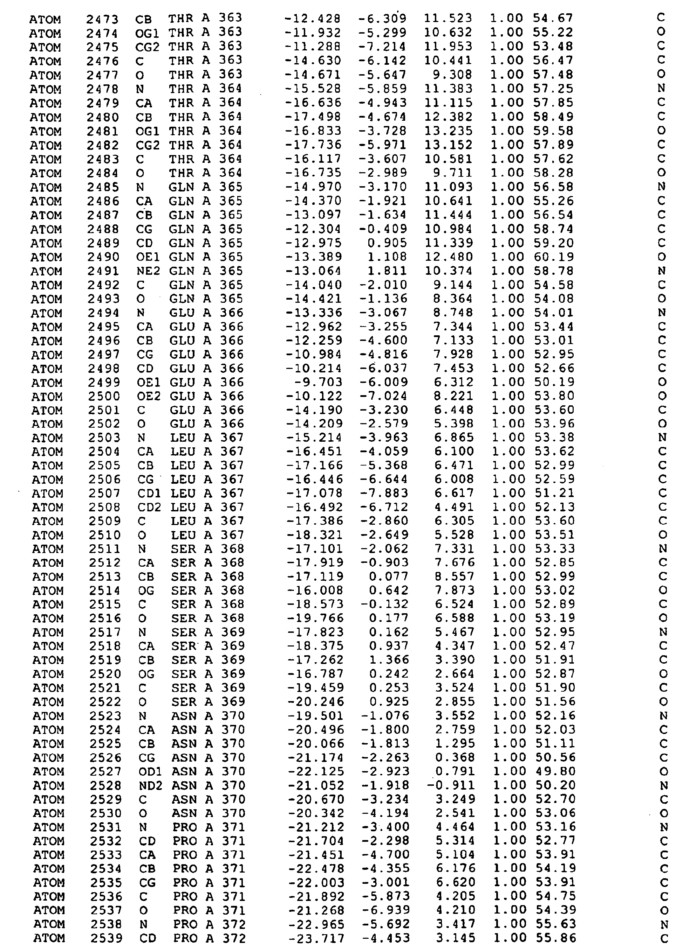

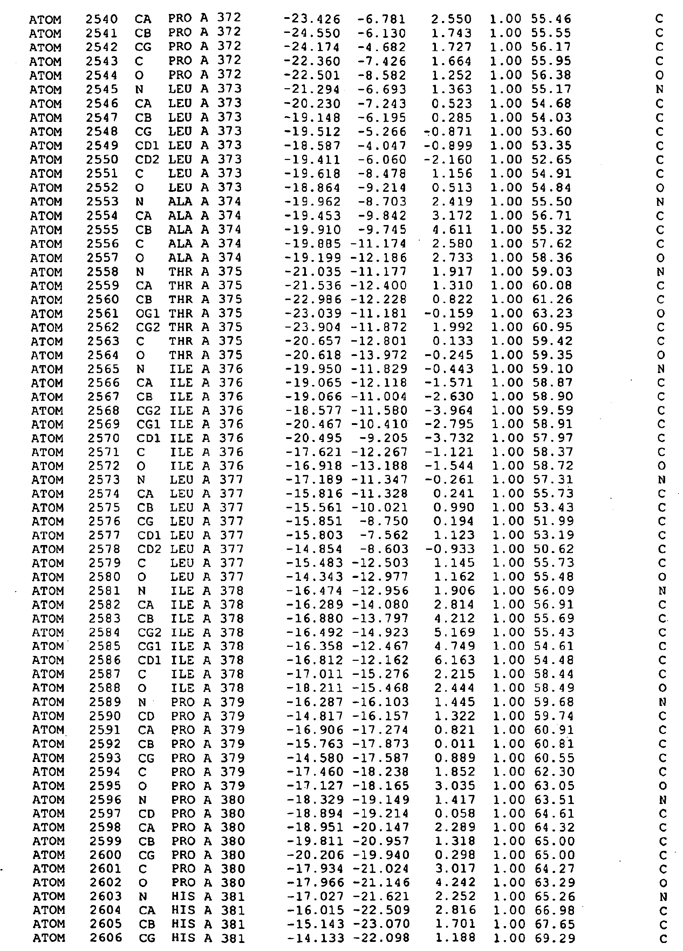

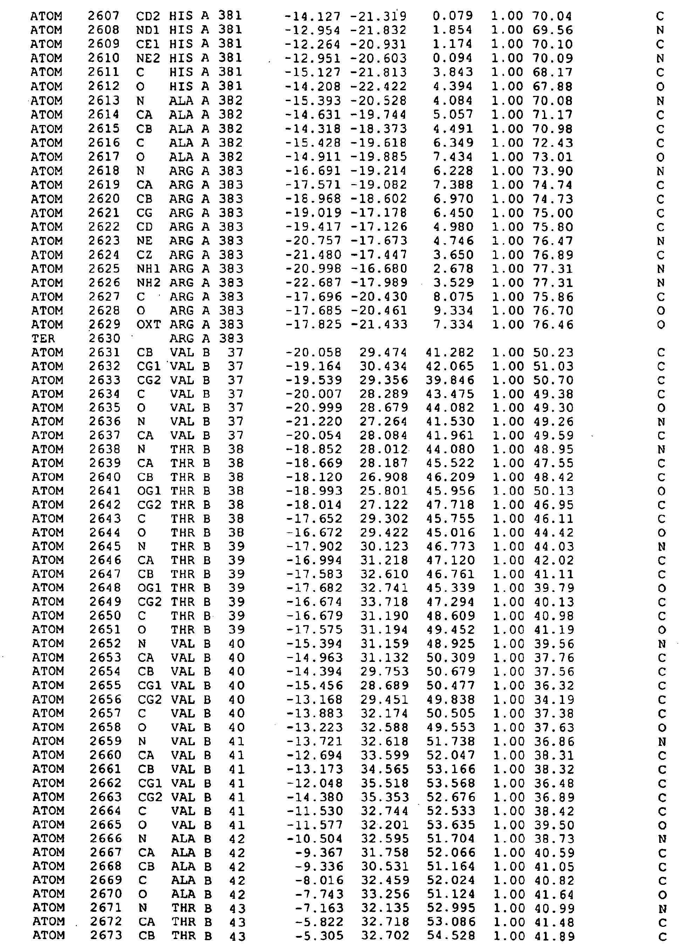

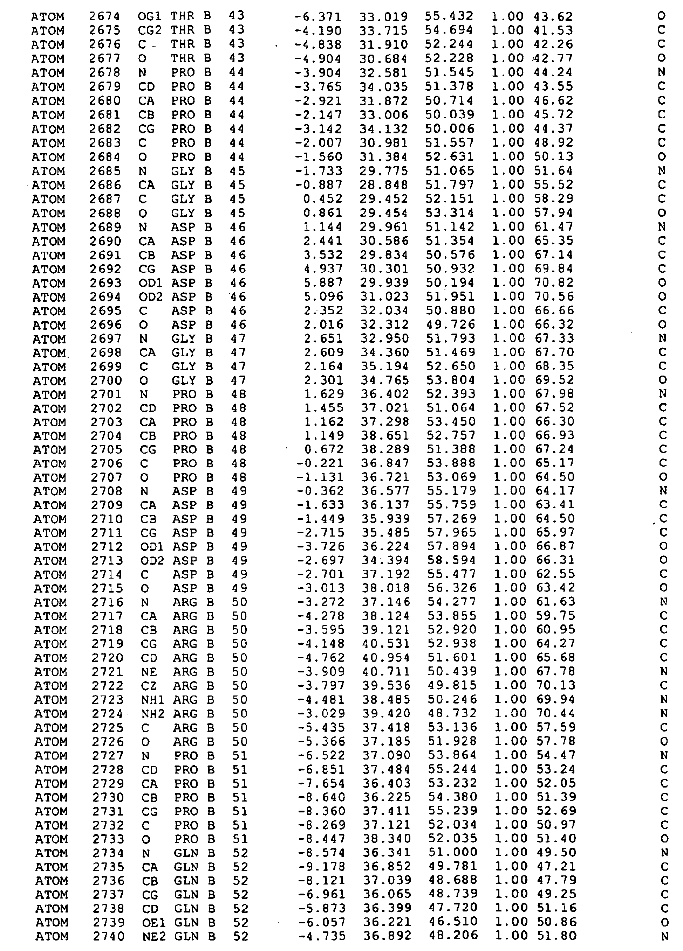

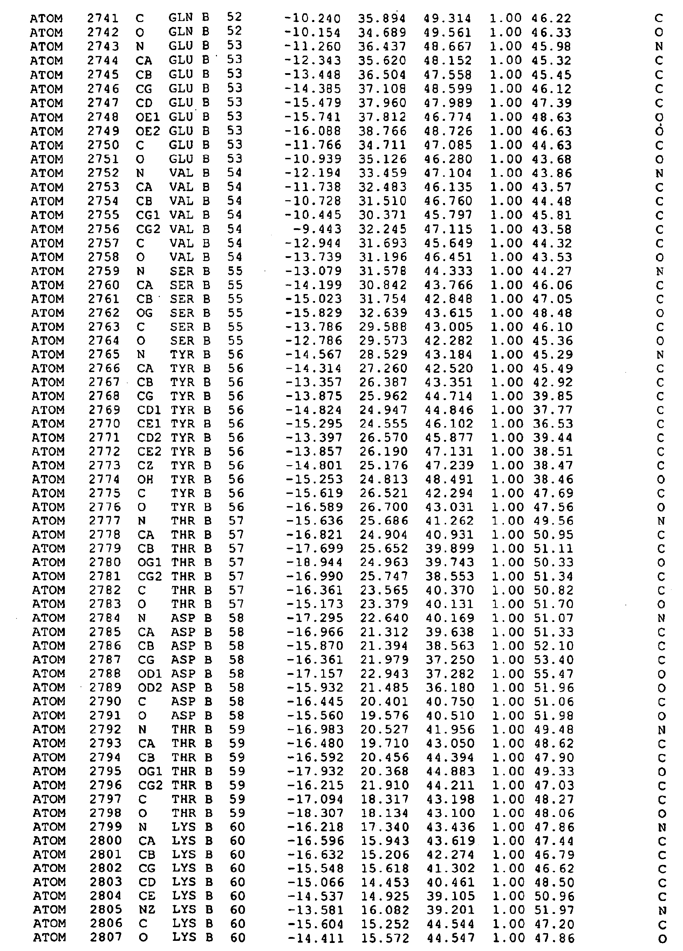

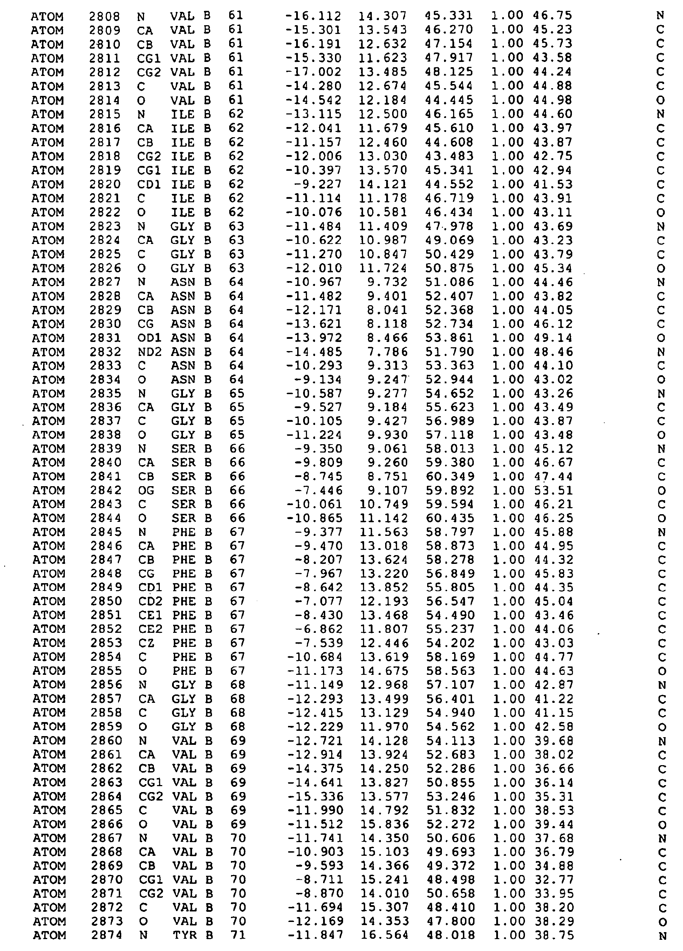

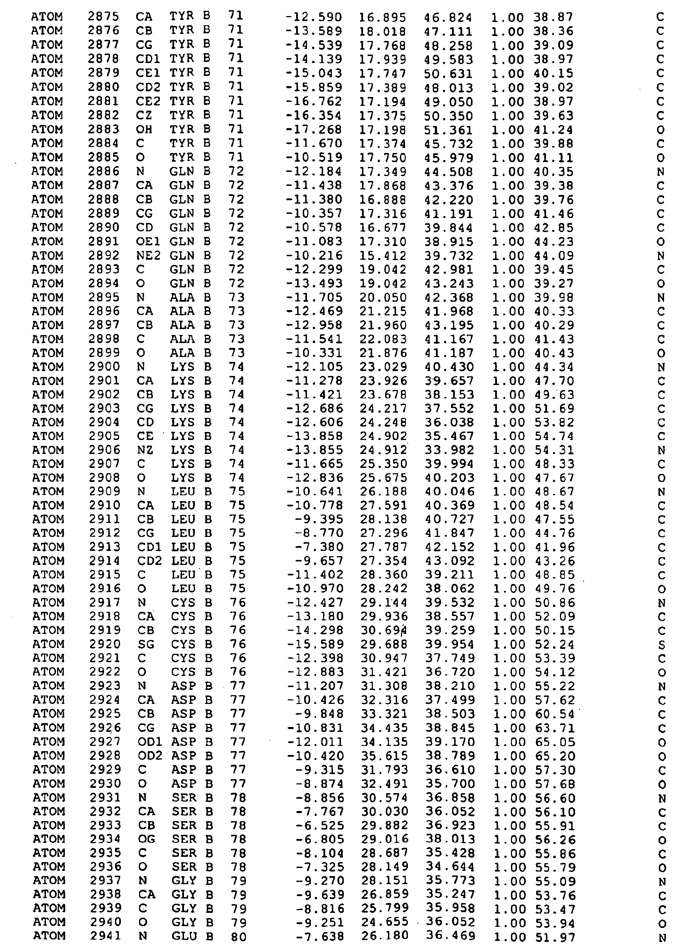

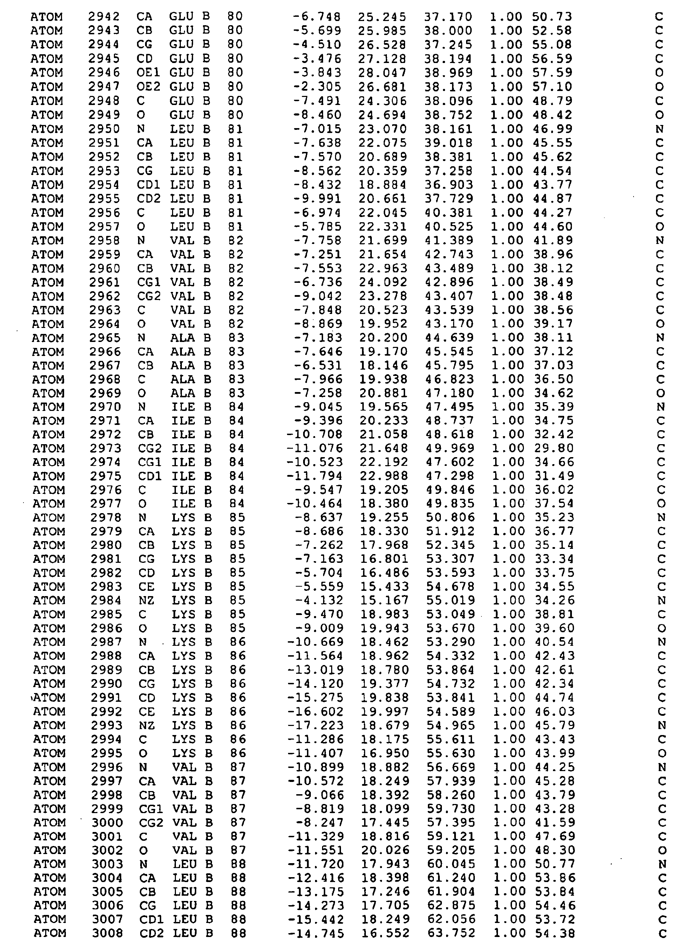

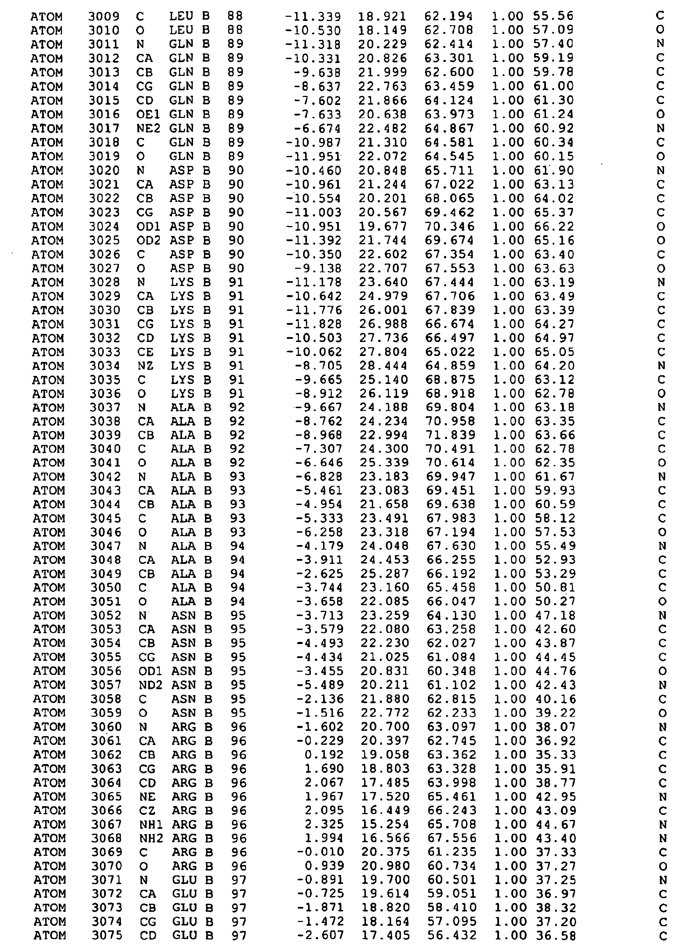

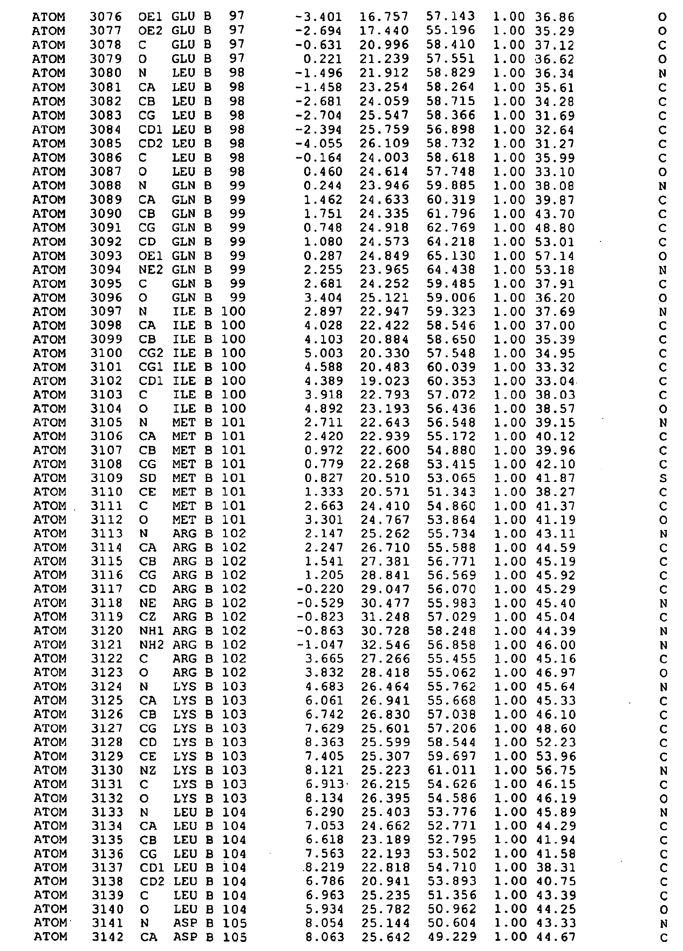

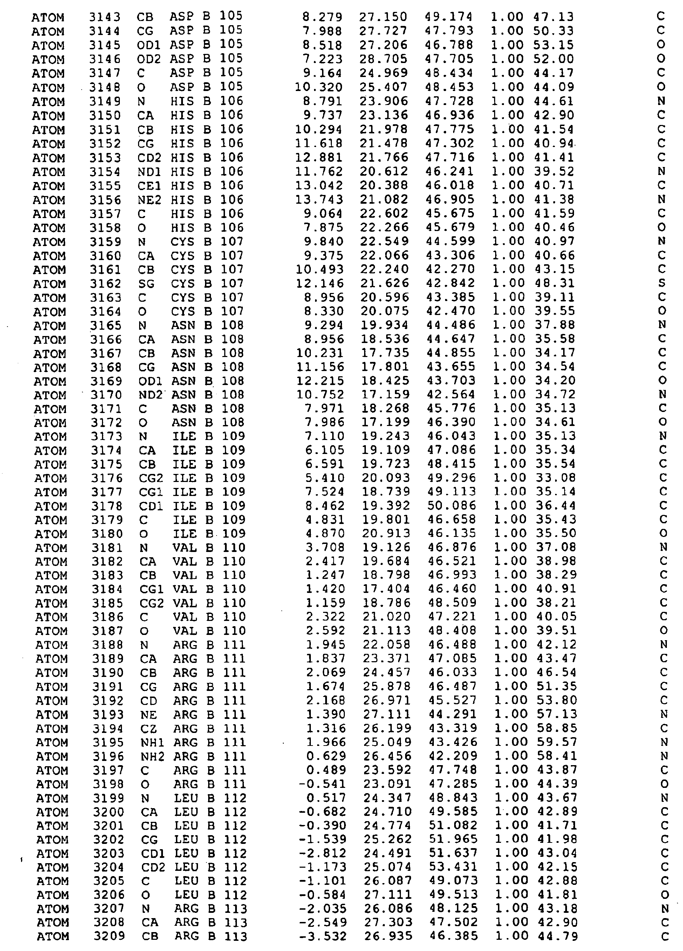

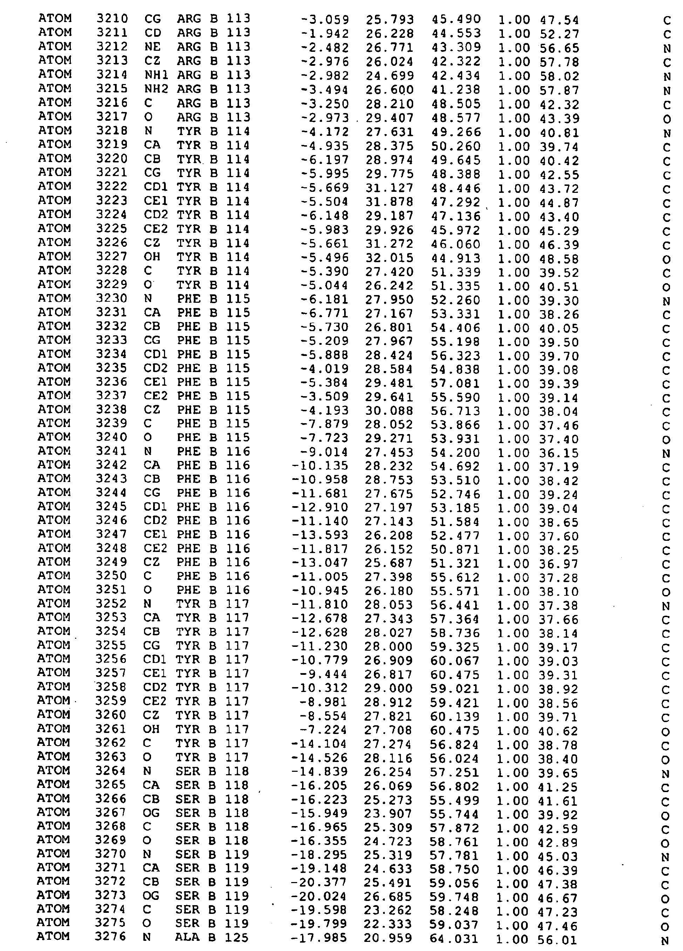

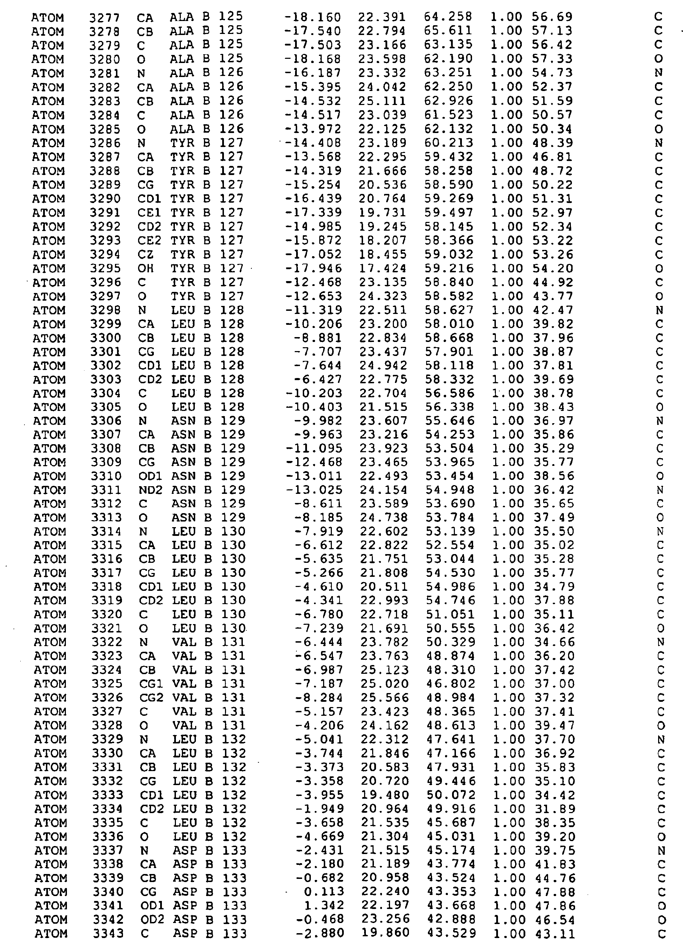

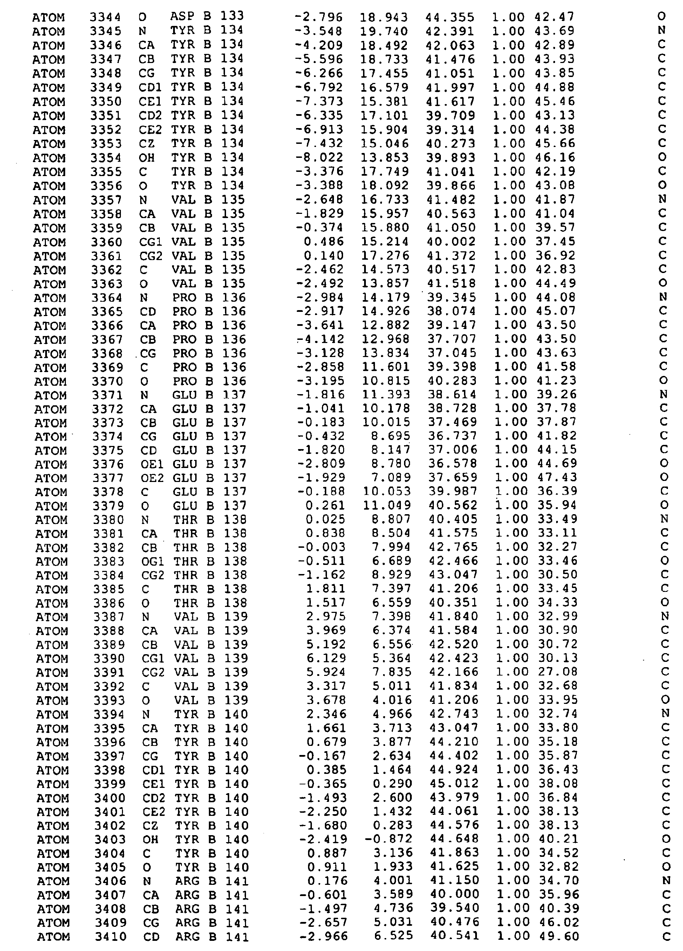

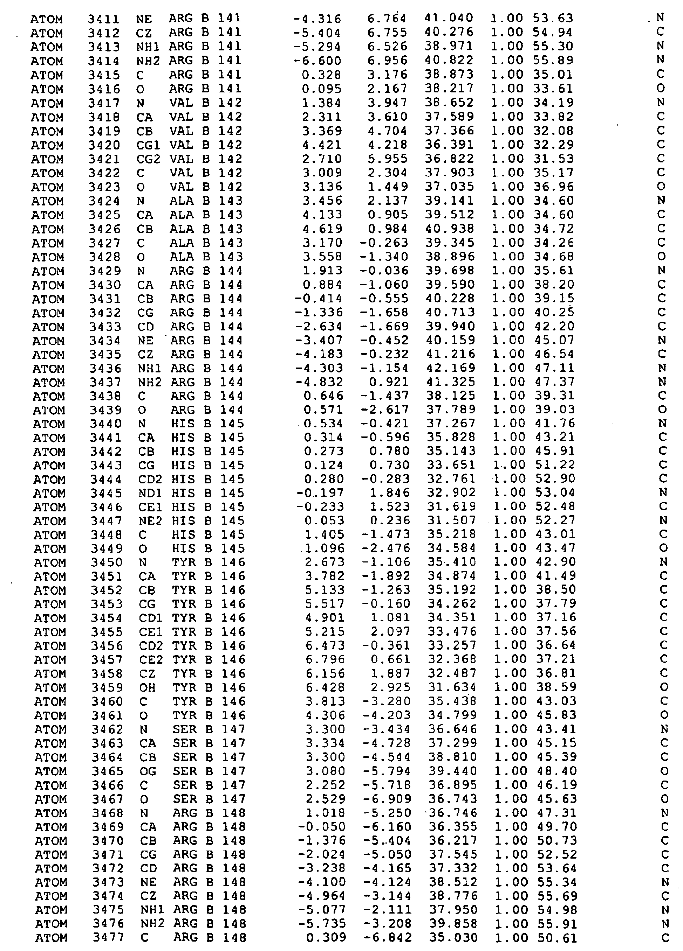

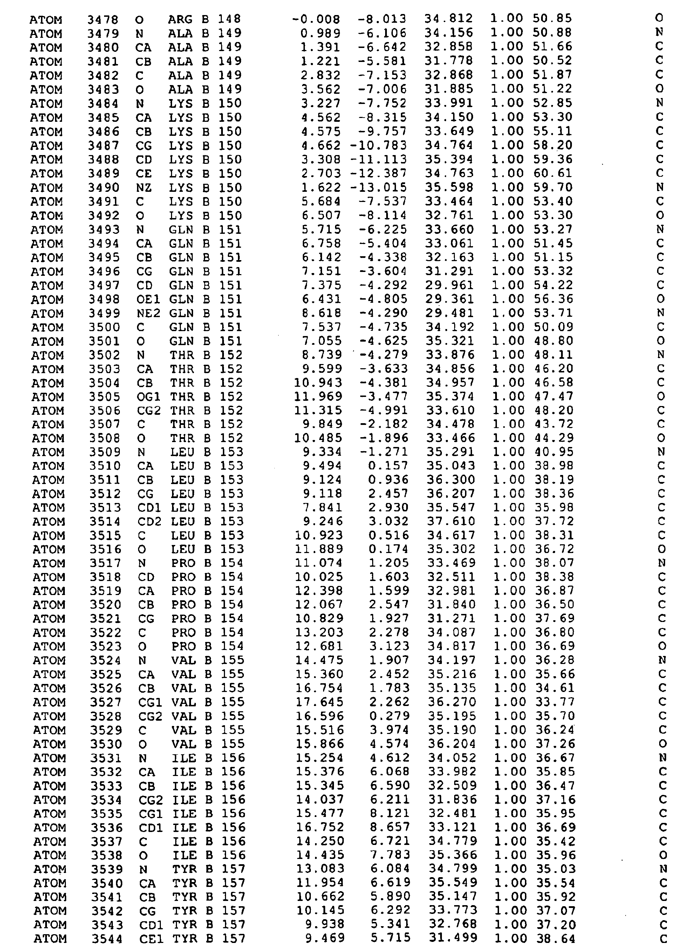

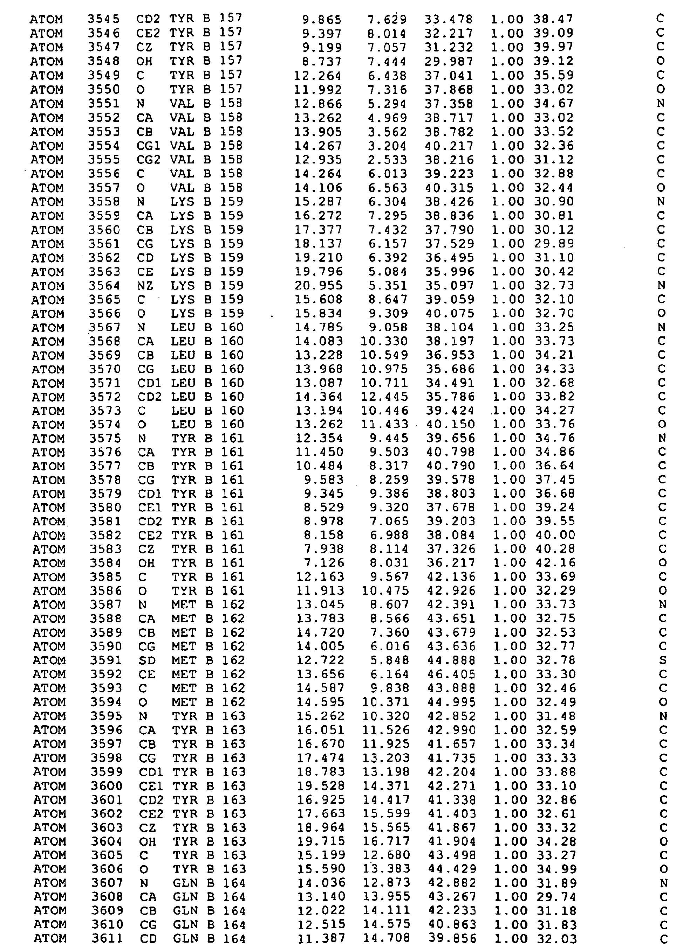

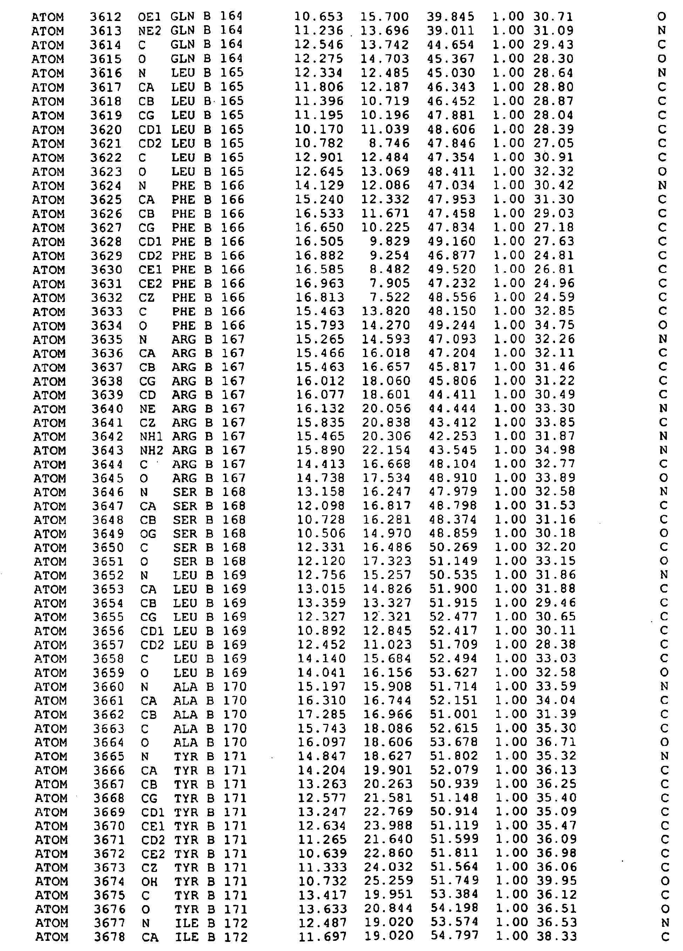

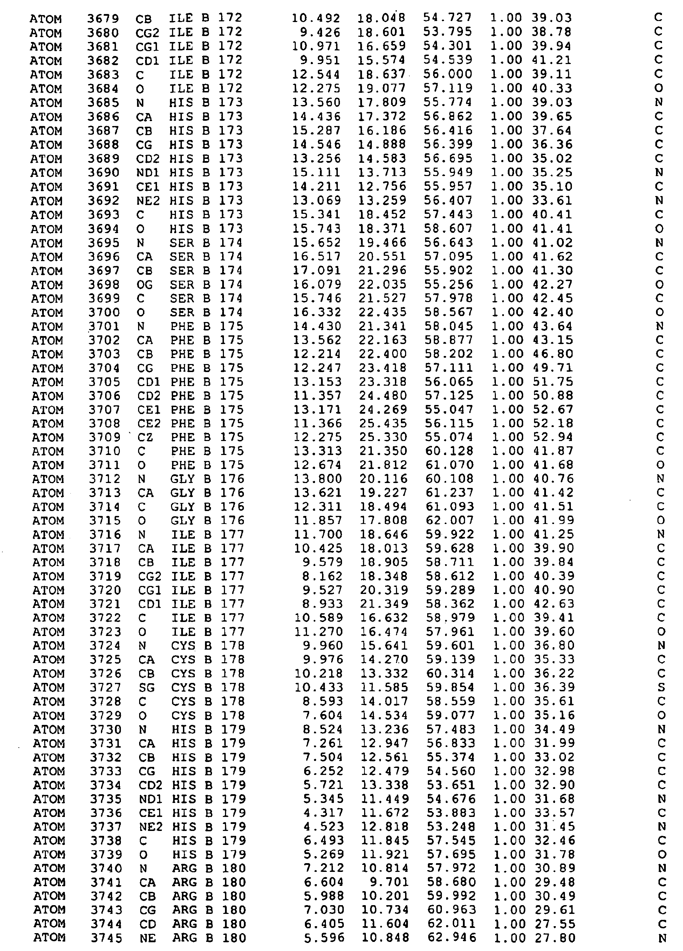

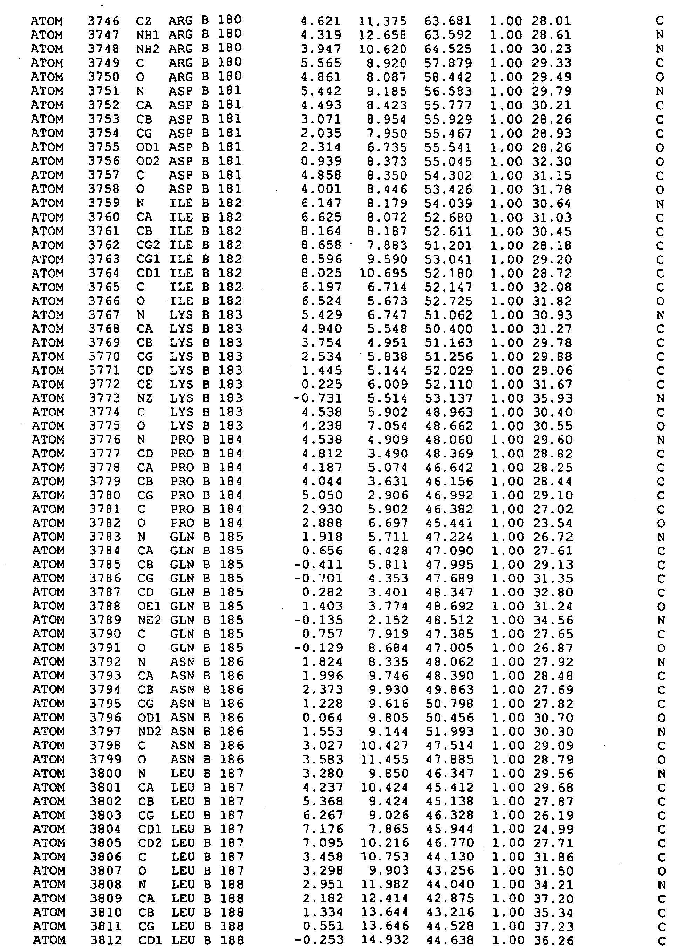

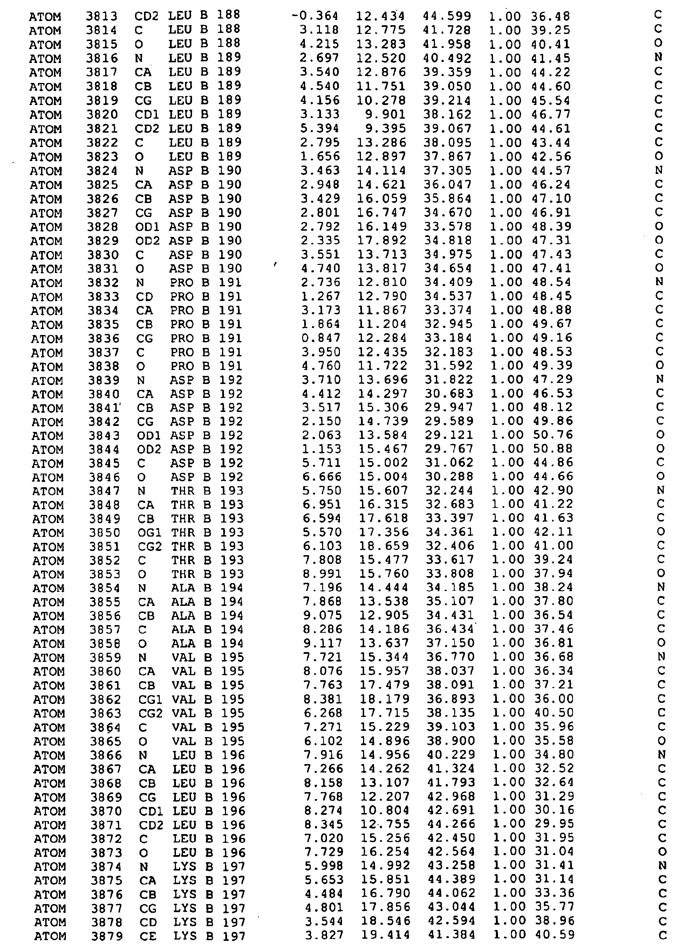

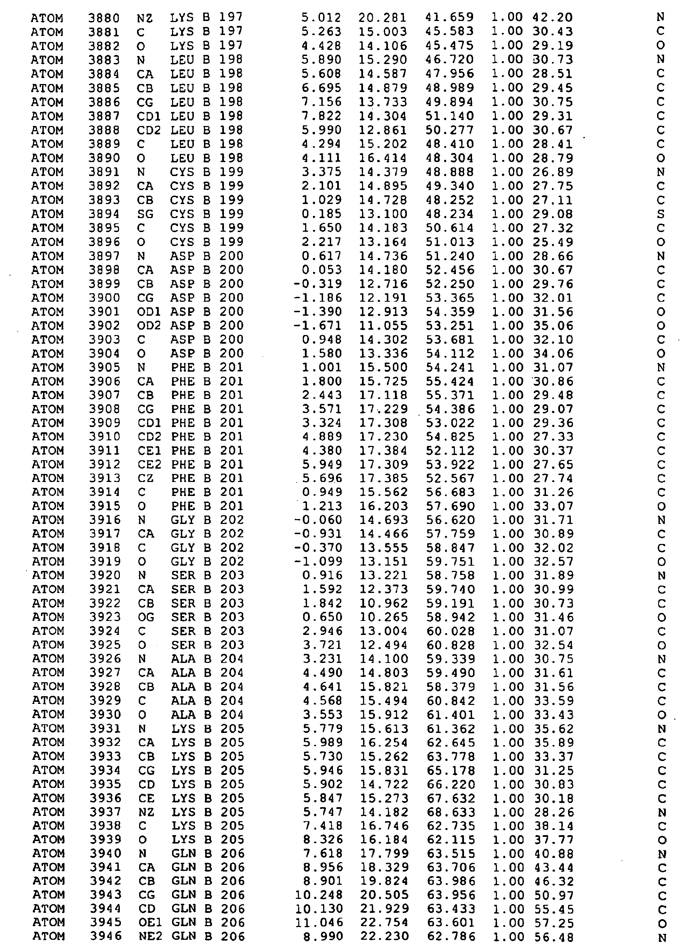

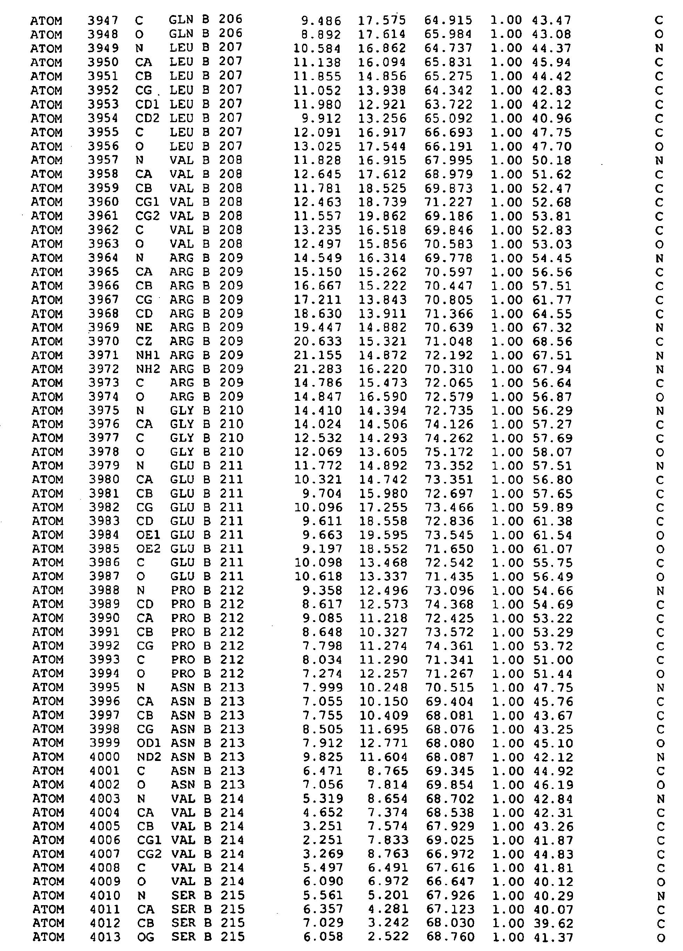

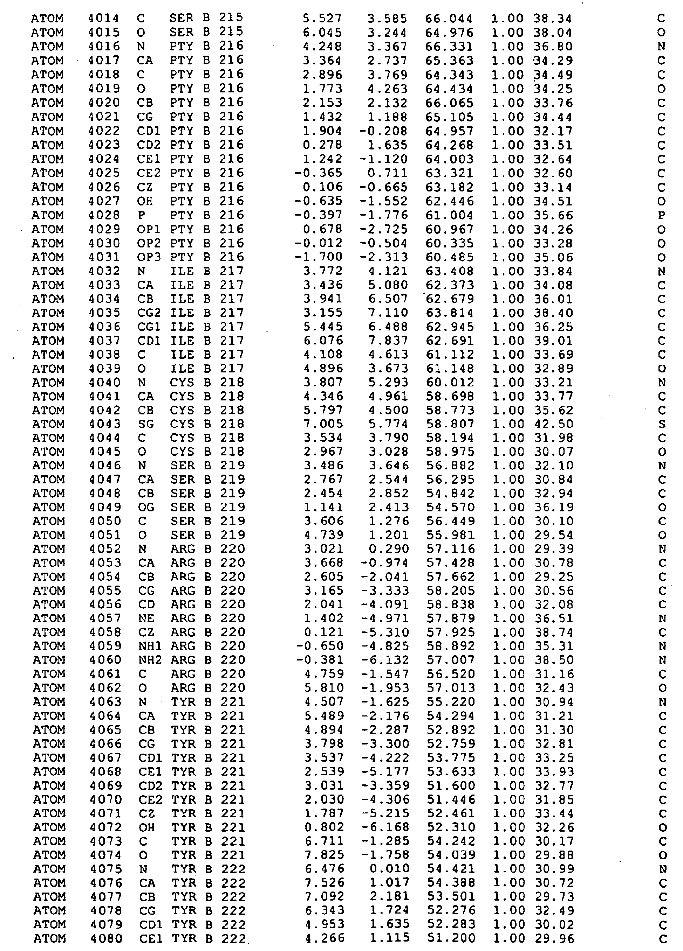

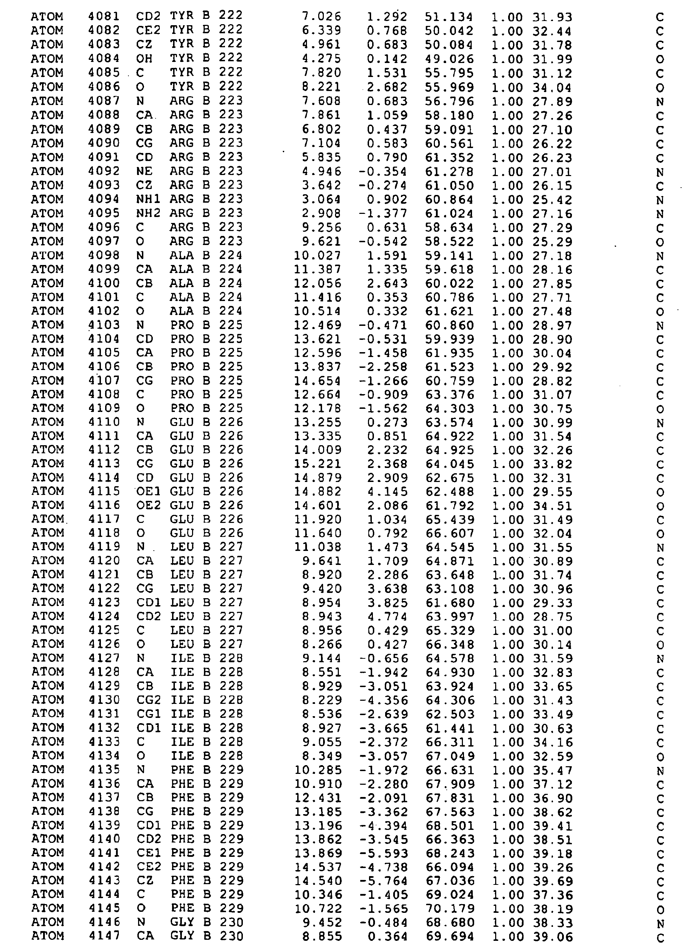

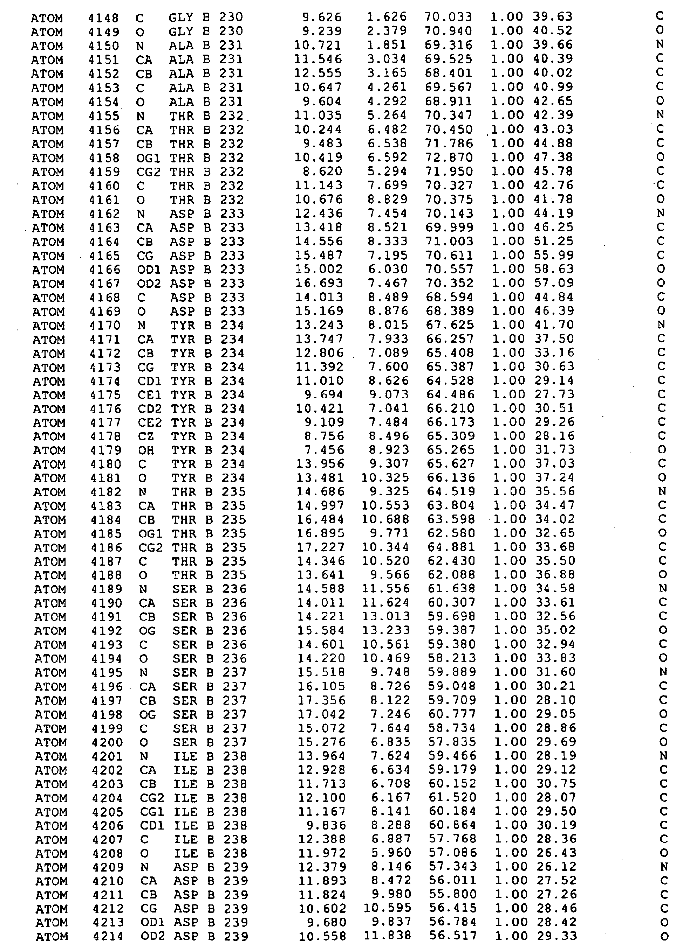

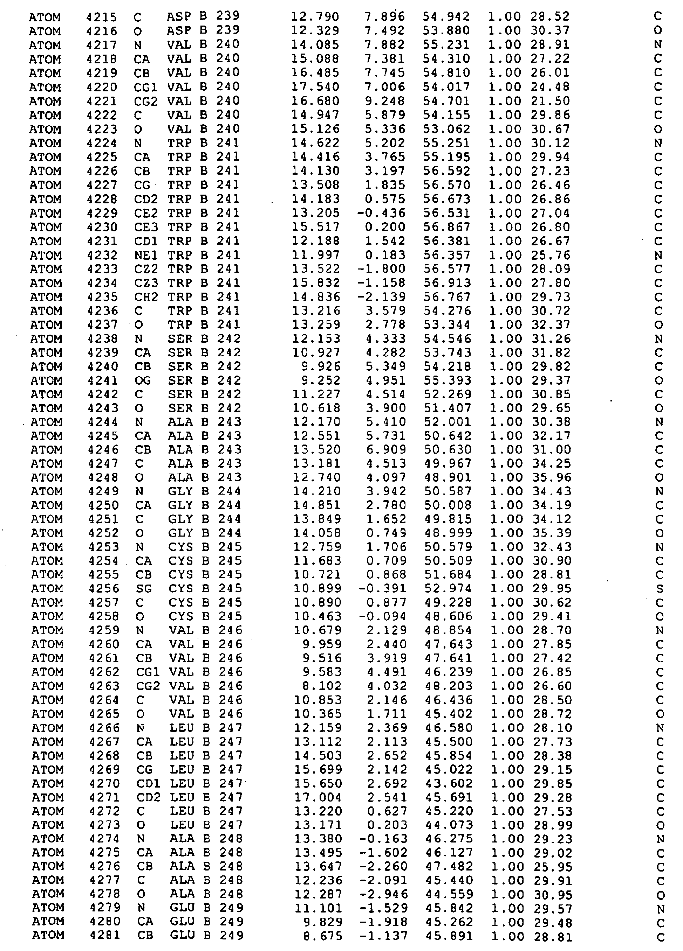

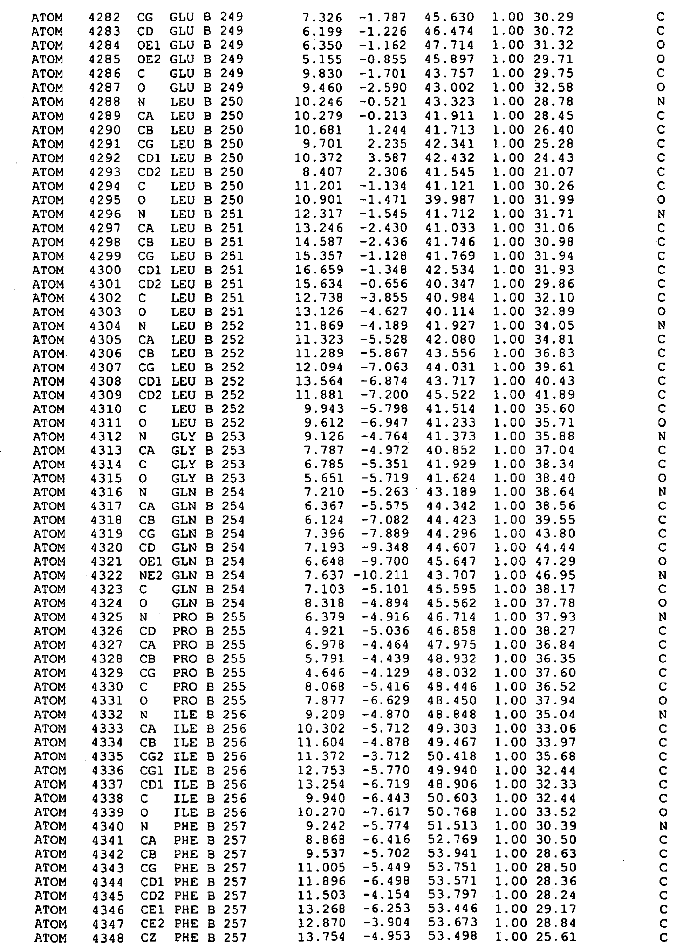

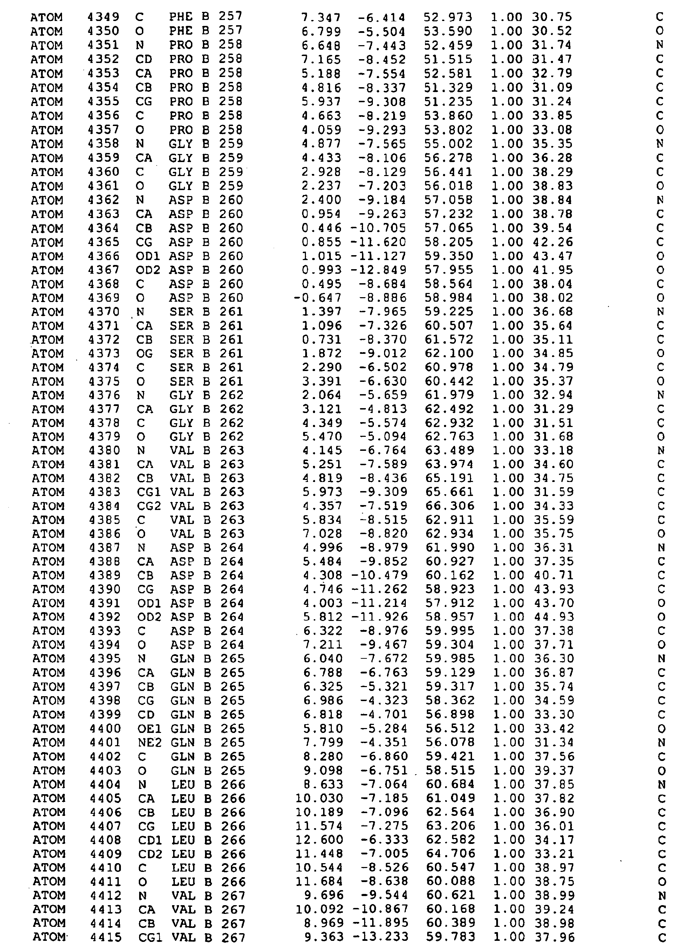

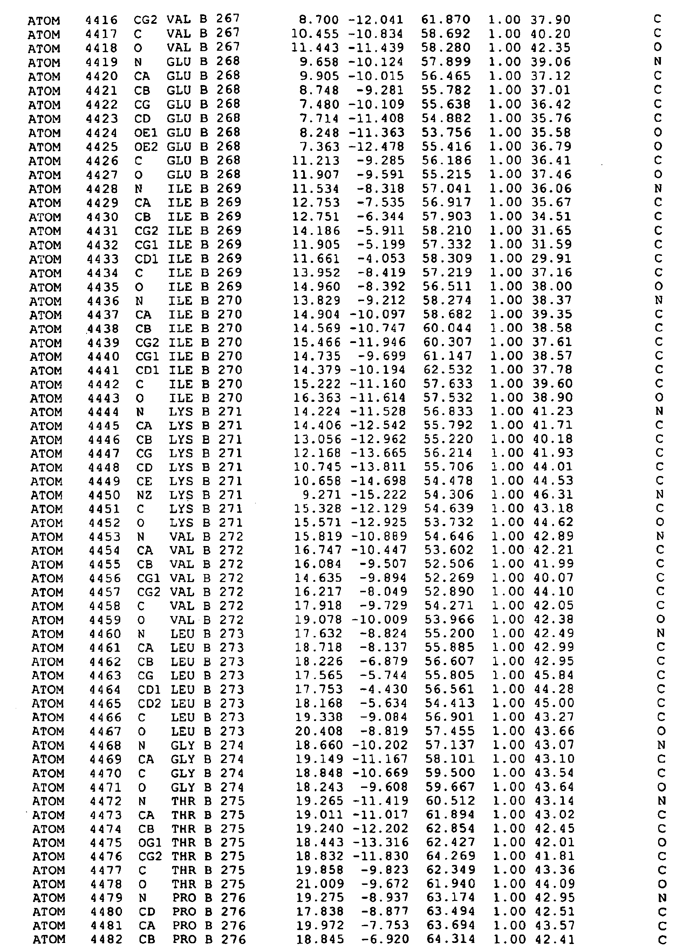

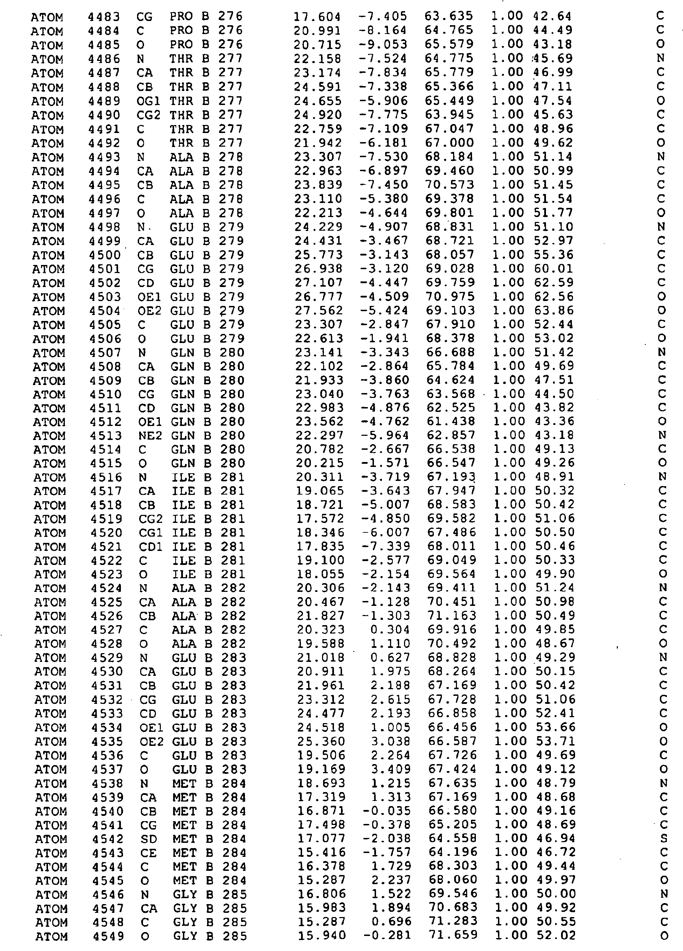

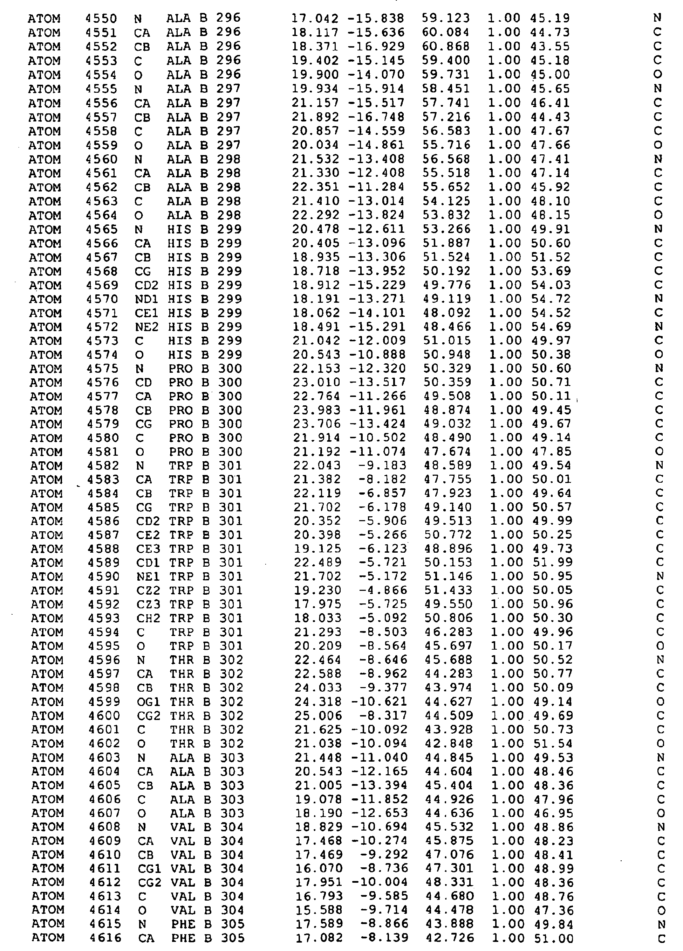

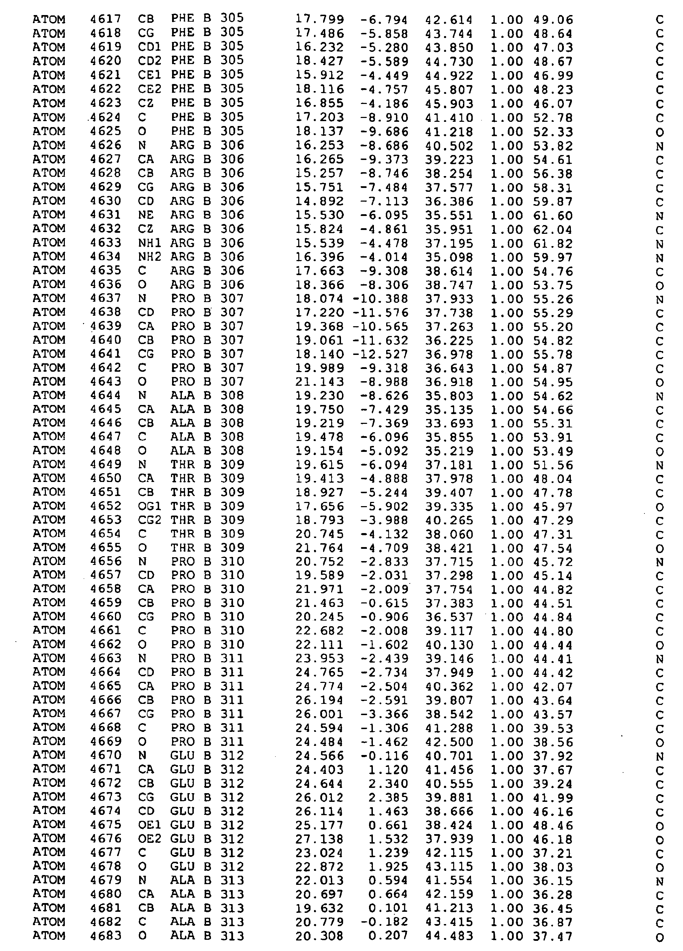

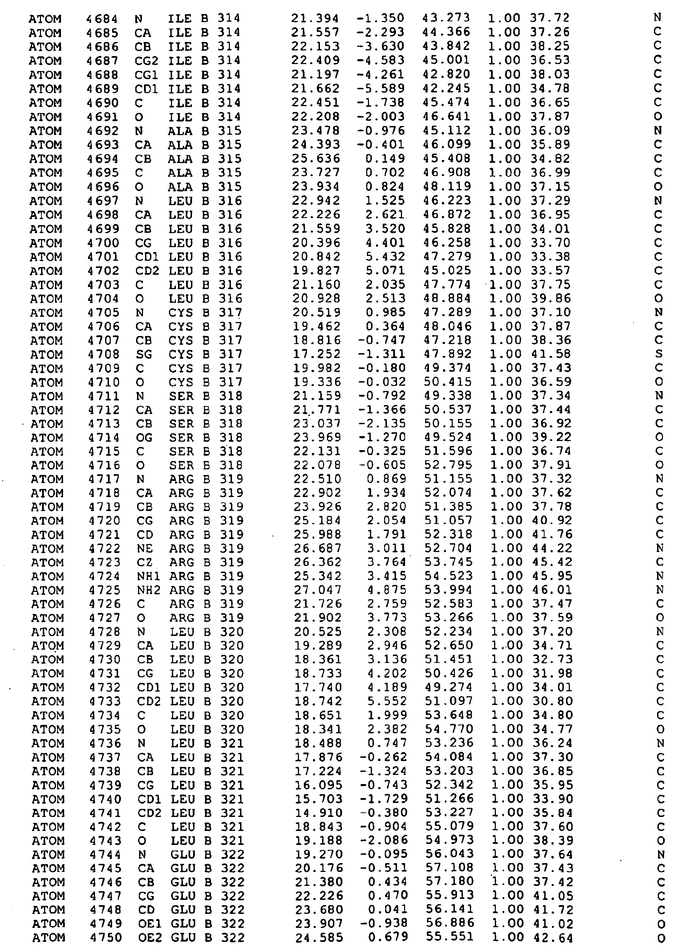

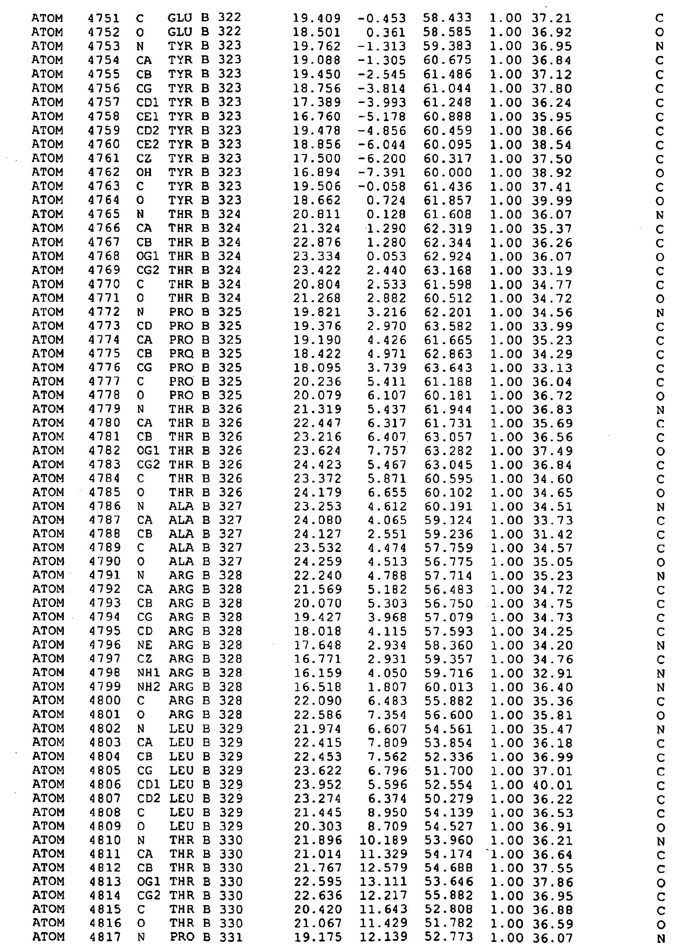

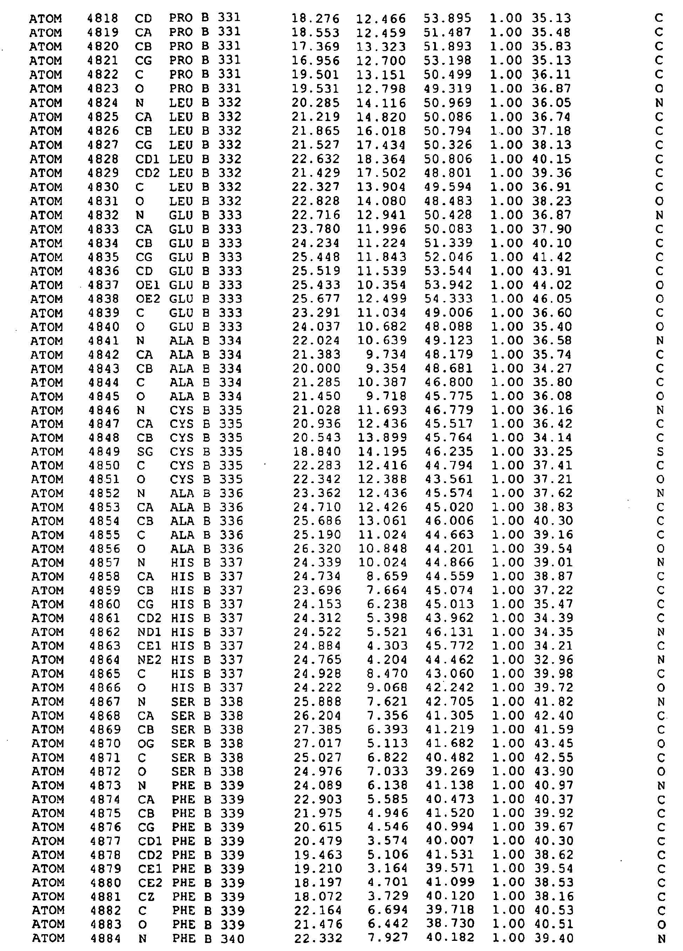

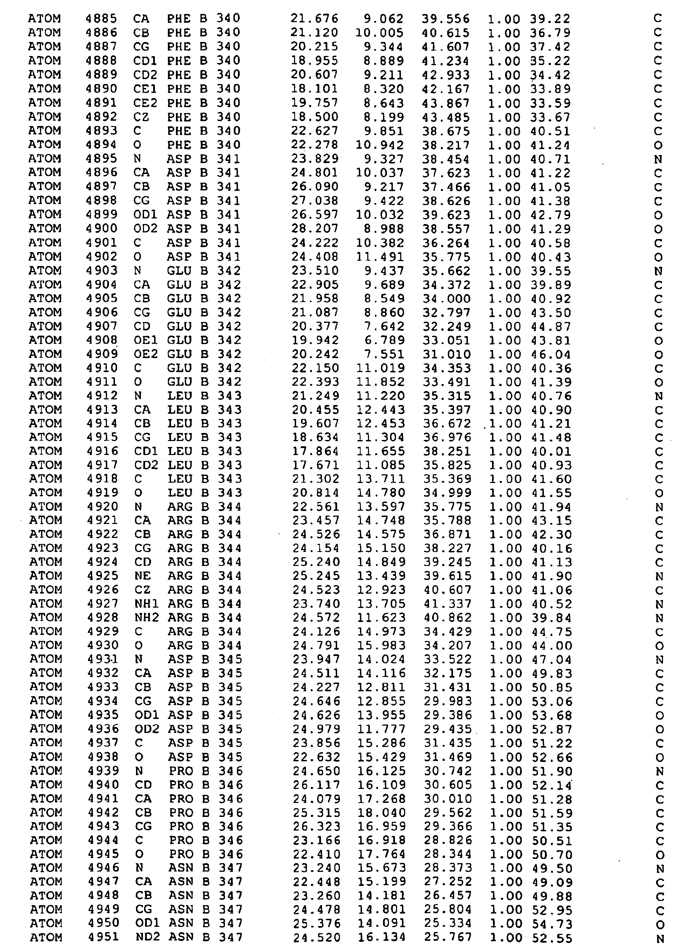

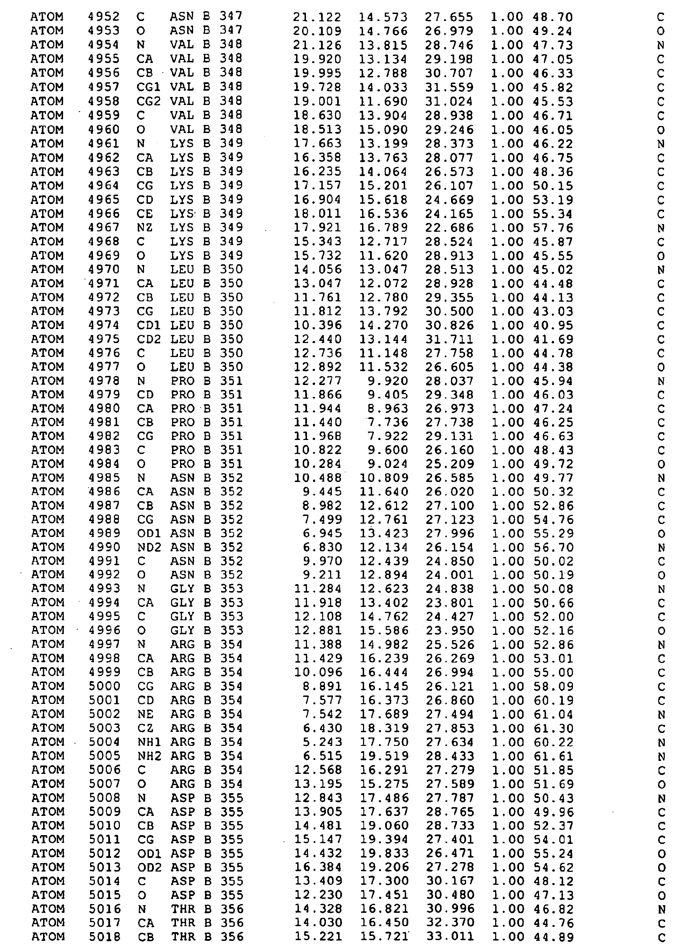

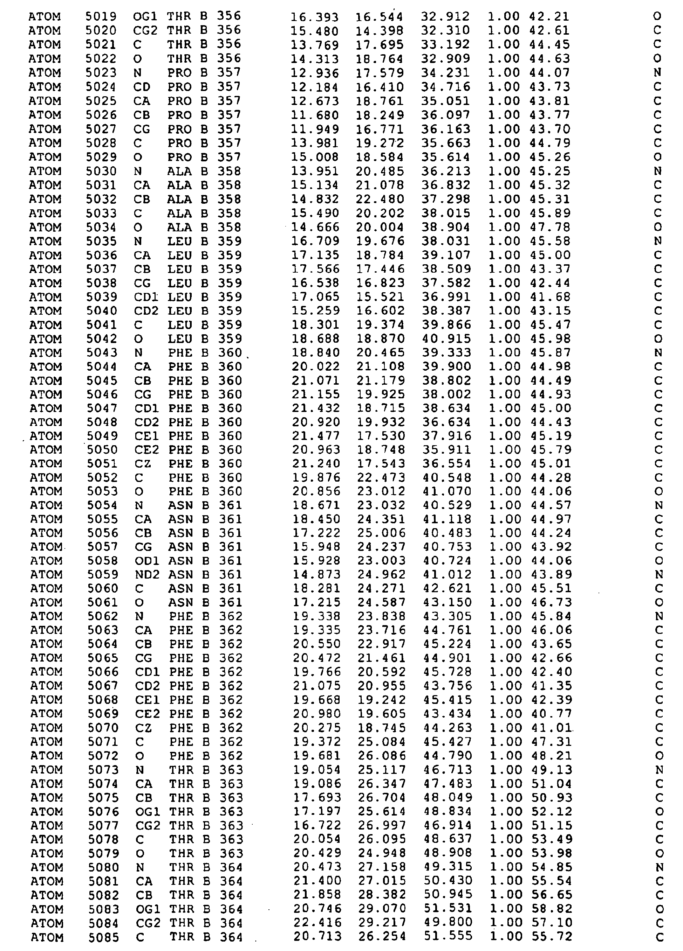

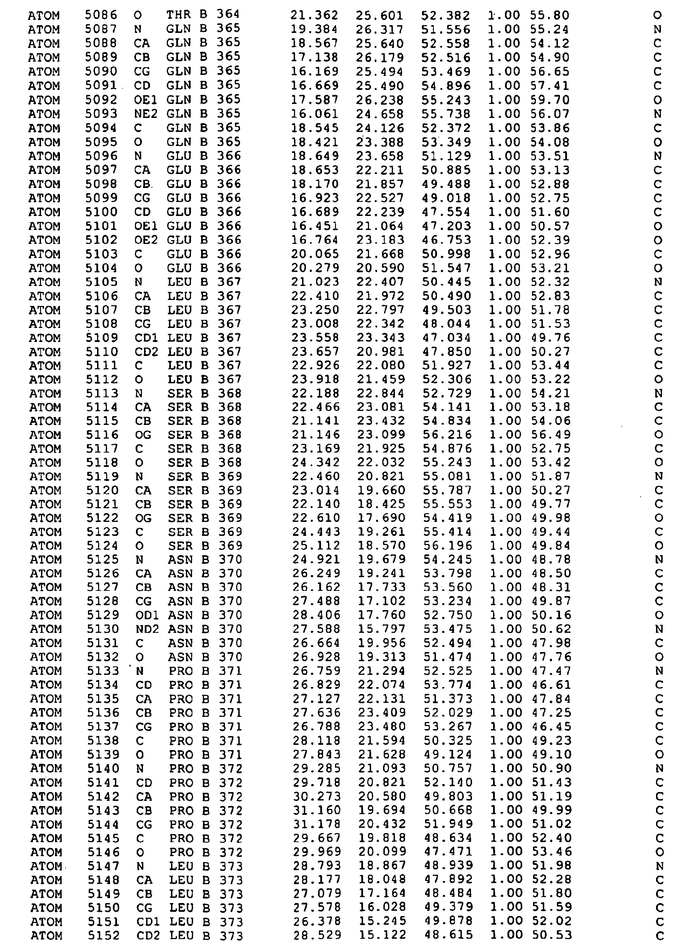

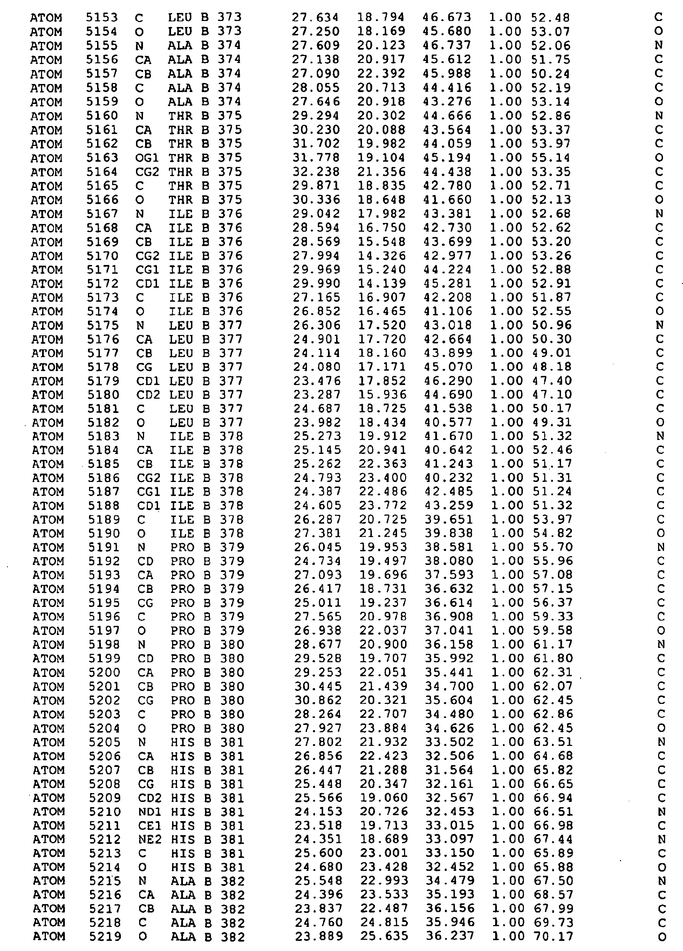

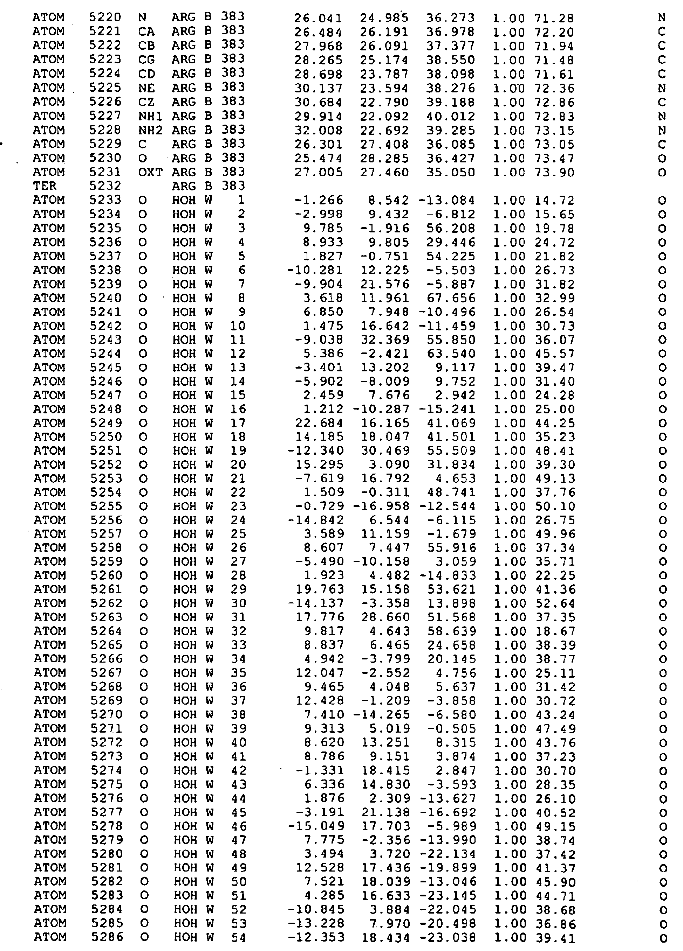

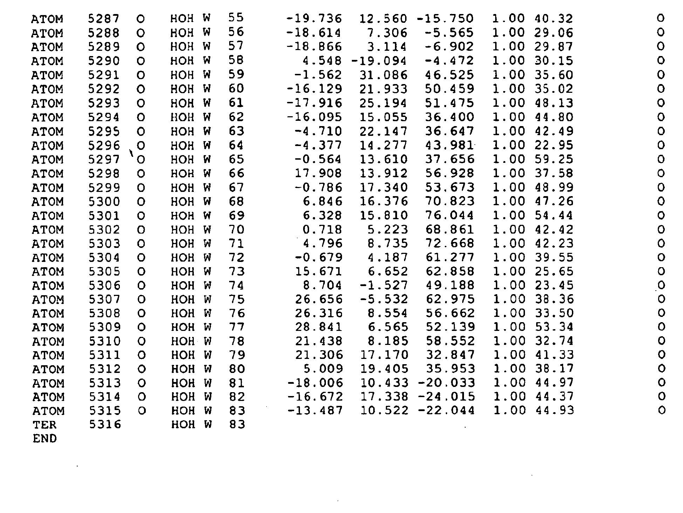

The following abbreviations are used in Figures 1-5:
"Atom type" refers to the element whose coordinates are measured. The first letter in the column defines the element.
"Res" refers to the amino acid residue in the molecular model.
"X, Y, Z" crystallographically define the atomic position of the element measured.
"B" is a thermal factor that measures movement of the atom around its atomic center.
"Occ" is an occupancy factor that refers to the fraction of the molecules in which each atom occupies the position specified by the coordinates. A value of "1" indicates that each atom has the same conformation, i.e., the same position, in all molecules of the crystal.

### DETAILED DESCRIPTION OF THE INVENTION

In order that the invention described herein may be more fully understood, the following detailed description is set forth.

Throughout the specification, the word "comprise" or variations such as "comprises" or "comprising" will be understood to imply the inclusion of a stated integer or groups of integers but not the exclusion of any other integer or groups of integers.

The following abbreviations are used throughout the application:

| | | | | | |
|---|---|---|---|---|---|
| A = | Ala = | Alanine | T = | Thr = | Threonine |
| V = | Val = | Valine | C = | Cys = | Cysteine |
| L = | Leu = | Leucine | Y = | Tyr = | Tyrosine |
| I = | Ile = | Isoleucine | N = | Asn = | Asparagine |
| P = | Pro = | Proline | Q = | Gln = | Glutamine |
| F = | Phe = | Phenylalanine | D = | Asp = | Aspartic Acid |
| W = | Trp = | Tryptophan | E = | Glu = | Glutamic Acid |
| M = | Met = | Methionine | K = | Lys = | Lysine |
| G = | Gly = | Glycine | R = | Arg = | Arginine |
| S = | Ser = | Serine | H= | His = | Histidine |
| pS= | Phosphorylated Serine | | pTy= | Phosphorylated | |
| | | | | Tyrosine | |

As used herein, the following definitions shall apply unless otherwise indicated. Also, combinations of substituents or variables are permissible only if such combinations result in stable compounds.

The term "about" when used in the context of RMSD values takes into consideration the standard error of the RMSD value, which is ± 0.1 Å.

The term "active site" refers to the area in the protein kinase where the nucleotide binds. This site is located at the interface of the C-terminal α-helical and N-terminal β-strand domain, and is bordered by the glycine rich loop and the hinge (See, Xie et al., Structure, 6, pp. 983-991 (1998), incorporated herein by reference).

The term "aliphatic" refers to straight chain or branched hydrocarbons that are completely saturated or that contain one or more units of unsaturation. For example, aliphatic groups include substituted or unsubstituted linear or branched alkyl, alkenyl and alkynyl groups. Unless indicated otherwise, the term "aliphatic" encompasses both substituted and unsubstituted hydrocarbons. The term "alkyl", used alone or as part of a larger moiety, refers to both straight and branched saturated chains containing one to twelve carbon atoms. The terms "alkenyl" and "alkynyl", used alone or as part of a larger moiety, encompass both straight and branched chains containing two to twelve carbon atoms and at least one unit of unsaturation. An alkenyl group contains at least one carbon-carbon double bond and an alkynyl group contains at least one carbon-carbon triple bond.

The term "correspond to" or "corresponding amino acids" when used in the context of amino acid residues that correspond to GSK-3β amino acids refers to particular amino acids or analogues thereof in a protein that correspond to amino acids in the GSK-3β protein. The corresponding amino acid may be an identical, mutated, chemically modified, conserved, conservatively substituted, functionally equivalent or homologous amino acid when compared to the GSK-3β amino acid to which it corresponds.

Methods for identifying a corresponding amino acid are known in the art and are based upon sequence, structural alignment, its functional position or a combination thereof as compared to the GSK-3β protein. For example, corresponding amino acids may be identified by superimposing the backbone atoms of the amino acids in GSK-3β and the protein using well known software applications, such as QUANTA (Molecular Simulations, Inc., San Diego, CA ©2000). The corresponding amino acids may also be identified using sequence alignment programs such as the "bestfit" program available from the Genetics Computer Group which uses the local homology algorithm described by Smith and Waterman in Advances in Applied Mathematics 2, 482 (1981).

The term "aryl", alone or in combination with other terms, refers to monocyclic or polycyclic aromatic carbon ring systems having five to fourteen members. Examples of aryl groups include, but are not limited to, phenyl (Ph), 1-naphthyl, 2-naphthyl, 1-anthracyl and 2-anthracyl. The term "aralkyl" refers to an alkyl group substituted by an aryl. Examples of aralkyl groups include benzyl and phenethyl.

The term "associating with" refers to a condition of proximity between a chemical entity or compound, or portions thereof, and a binding pocket or binding site on a protein. The association may be non-covalent -- wherein the juxtaposition is energetically favored by hydrogen bonding or van der Waals or electrostatic interactions -- or it may be covalent.

The term "ATP analogue" refers to a compound derived from Adenosine-5'-triphosphate(ATP). The analogue can be ADP, or non-hydralysable, for example, Adenylyl Imidodiphosphate (AMPPNP). AMPPNP can be in complex with Magnesium or Manganese ions.

The term "binding pocket" refers to a region of a molecule or molecular complex, that, as a result of its shape, favorably associates with another chemical entity or compound.

The term "biological sample" includes, without limitation, cell cultures or extracts thereof; biopsied material obtained from a mammal or extracts thereof; and blood, saliva, urine, feces, semen, tears, or other body fluids or extracts thereof.

The term "carbocylyl" or "carbocyclic", alone or in combination with any other term, refers to monocyclic or polycyclic non-aromatic carbon ring systems, which may contain a specified number of carbon atoms, preferably from 3 to 12 carbon atoms, which are completely saturated or which contain one or more units of unsaturation. A carbocyclic ring system may be monocyclic, bicyclic or tricyclic. A carbocylyl ring may be fused to another ring, such as an aryl ring or another carbocyclic ring. Examples of carbocyclic rings could include cyclohexyl, cyclopentyl, cyclobutyl, cyclopropyl, cyclohexenyl, cyclopentenyl, indanyl, tetrahydronaphthyl and the like.

The term "chemically feasible or stable" refers to a compound structure that is sufficiently stable to allow manufacture and administration to a patient by methods known in the art. Typically, such compounds are stable at a temperature of 40°C or less, in the absence of moisture or other chemically reactive conditions, for at least one week.

The term "chemical entity" refers to chemical compounds, complexes of at least two chemical compounds, and fragments of such compounds or complexes. The chemical entity can be, for example, a ligand, a substrate, nucleotide triphosphate, a nucleotide, an agonist, antagonist, inhibitor, antibody, peptide, protein or drug. In one embodiment, the chemical entity is selected from the group consisting of an ATP and an inhibitor for the active site. In one embodiment, the inhibitor is 4,5-Diphenyl-1H-pyrazolo[3,4-c]pyridazin-3-ylamine, (5-Methyl-2H-pyrazol-3-yl)-(2-pyridin-4-yl-quinazolin-4-yl)-amine, 4-(5-Methyl-2-phenylamino-pyrimidin-4-yl)-1H-pyrrole-2-carboxylic acid (2-hydroxy-1-phenyl-ethyl)-amide, (1H-Indazol-3-yl)-[2-(2-trifluoromethyl-phenyl)-quinazolin-4-yl]-amine and an ATP analogue such as MgAMP-PNP (adenylyl imidodiphosphate) or ADP. In one embodiment, the chemical entity is selected from the group consisting of a peptide substrate or inhibitor for the substrate binding groove.

The term "crystallization solution" refers to a solution that promotes crystallization of macromolecules. The crystallization solution may contain a precipitant, a buffer, salt, stabilizer, a polyionic agent, a detergent, a lanthanide ion or reducing agent. One of ordinary skilled in the art may adjust the components of the crystallization solution to find a condition suitable for the macromolecule of interest.

The term "conservative substitutions" refers to residues that are physically or functionally similar to the corresponding reference residues. That is, a conservative substitution and its reference residue have similar size, shape, electric charge, chemical properties including the ability to form covalent or hydrogen bonds, or the like. Preferred conservative substitutions are those fulfilling the criteria defined for an accepted point mutation in Dayhoff et al., Atlas of Protein Sequence and Structure, 5, pp. 345-352 (1978 & Supp.). Examples of conservative substitutions are substitutions including but not limited to the following groups: (a) valine, glycine; (b) glycine, alanine; (c) valine, isoleucine, leucine; (d) aspartic acid, glutamic acid; (e) asparagine, glutamine; (f) serine, threonine; (g) lysine, arginine, methionine; and (h) phenylalanine, tyrosine.

The term "complex" refers to a protein associated with a chemical entity.

The term "domain" refers to a structural unit of the GSK-3β protein or homologue. The domain can comprise a binding pocket, or a sequence or structural motif. In GSK-3β, the protein is separated into two domains, the N-terminal domain which is predominantly β strands and the C-terminal domain which is predominantly α helical.

The term "generating a three-dimensional structure" refers to plotting the structure coordinates in three-dimensional space. This can be achieved through commercially available software. The three-dimensional structure may be used to perform computer modeling, fitting operations, or displayed as a three-dimensional graphical representation.

The term "GSK-3β inhibitor-binding pocket" or "GSK-3β ATP-binding pocket" refers to a binding pocket of a molecule or molecular complex defined by the structure coordinates of a certain set of amino acid residues present in the GSK-3β structure, as described below. This binding pocket is in an area in the GSK-3β protein where the ATP or inhibitor for the active site binds.

The term "GSK-3β-like" refers to all or a portion of a molecule or molecular complex that has a commonality of shape to all or a portion of the GSK-3β protein. For example, in the GSK-3β-like inhibitor binding pocket, the commonality of shape is defined by a root mean square deviation of the structure coordinates of the backbone atoms between the amino acids in the GSK-3β-like inhibitor-binding pocket and the GSK-3β amino acids in the GSK-3β inhibitor-binding pocket (as set forth in any one of Figure 1-7). Depending on the set of GSK-3β amino acids that define the GSK-3β inhibitor-binding pocket, one skilled in the art would be able to locate the corresponding amino acids that define a GSK-3β-like inhibitor-binding pocket in a protein based on sequence or structural homology.

The term "GSK-3-mediated condition" or "state" refers to any disease or other deleterious condition or state in which GSK-3, in particular GSK-3, is known to play a role. Such diseases or conditions include, without limitation, diabetes, Alzheimer's disease, Huntington's Disease, Parkinson's Disease, AIDS-associated dementia, amyotrophic lateral sclerosis (AML), multiple sclerosis (MS), schizophrenia, cardiomycete hypertrophy, reperfusion/ischemia, and baldness.

The term "halogen" or "halo" means F, Cl, Br, or I.

The term "heteroatom" means N, O, or S and shall include any oxidized form of nitrogen and sulfur, such as N(O), S(O), S(O)₂ and the quaternized form of any basic nitrogen.

The term "heterocyclic" or "heterocyclyl" refers to non-aromatic saturated or unsaturated monocyclic or polycyclic ring systems containing one or more heteroatoms and with a ring size of three to fourteen. One having ordinary skill in the art will recognize that the maximum number of heteroatoms in a stable, chemically feasible heterocyclic ring is determined by the size of the ring, degree of unsaturation, and valence. In general, a heterocyclic ring may have one to four heteroatoms so long as the heterocyclic ring is chemically feasible and stable and may be fused to another ring, such as a carbocyclic, aryl or heteroaryl ring, or to another heterocyclic ring. A heterocyclic ring system may be monocyclic, bicyclic or tricyclic. Also included within the scope of within the scope of the term "heterocyclic" or "heterocyclyl", as used herein, is a group in which one or more carbocyclic rings are fused to a heteroaryl.

Examples of heterocyclic rings include, but are not limited to, 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl.

The term "heteroaryl", alone or in combination with any other term, refers to monocyclic or polycyclic aromatic ring systems having five to fourteen members and one or more heteroatoms. One having ordinary skill in the art will recognize that the maximum number of heteroatoms in a stable, chemically feasible heteroaryl ring is determined by the size of the ring and valence. The term "heteroaralkyl" refers to an alkyl group substituted by a heteroaryl. Also explicitly included within the scope of the term "heteroaralkyl" are alkenyl or alkynyl groups substituted by a heteroaryl. In general, a heteroaryl ring may have one to four heteroatoms. Heteroaryl groups include, without limitation, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl. The term "heteroaryl ring", "heteroaryl group", or "heteroaralkyl" also refers to rings that are optionally substituted.

Examples of fused polycyclic heteroaryl and aryl ring systems in which a carbocyclic aromatic ring or heteroaryl ring is fused to one or more other rings include, tetrahydronaphthyl, benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, and the like.

An aryl, aralkyl, heteroaryl, or heteroaralkyl group may contain one or more independently selected substituents. Examples of suitable substituents on the unsaturated carbon atom of an aryl or heteroaryl group include halogen, CF₃, -R', -OR', -OH, -SH, -SR', protected OH (such as acyloxy), -NO₂, -CN, -NH₂, -NHR', -N(R')₂, -NHCOR', -NHCONH₂, -NHCONHR' , -NHCON(R')₂, -NRCOR', -NHCO₂H, -NHCO₂R', -CO₂R' , -CO₂H, -COR', -CONH₂, -CONHR' , -CON(R')₂, -S(O)₂H, -S(O)₂R', -SO₂NH₂, -S(O)H, -S(O)R', -SO₂NHR', -SO₂N(R')₂, -NHS(O)₂H, or -NHS(O)₂R', where R' is selected from H, aliphatic, carbocyclyl, heterocyclyl, aryl, aralkyl, heteroaryl, or heteroaralkyl and each R' is optionally substituted with halogen, nitro, cyano, amino, -NH-(unsubstituted aliphatic), -N-(unsubstituted aliphatic)₂, carboxy, carbamoyl, hydroxy, -O-(unsubstituted aliphatic), -SH, -S-(unsubstituted aliphatic), CF₃, -SO₂NH₂, unsubstituted aliphatic, unsubstituted carbocyclyl, unsubstituted heterocyclyl, unsubstituted aryl, unsubstituted aralkyl, unsubstituted heteroaryl, or unsubstituted heteroaralkyl.

An aliphatic group or a non-aromatic heterocyclic ring may contain one or more substituents. Examples of suitable substituents on the saturated carbon of an aliphatic group or of a non-aromatic heterocyclic ring include those listed above for the unsaturated carbon as well as the following: =O, =S, =NNHR', =NN(R')₂, =N-OR', =NNHCOR', =NNHCO₂R', =NNHSO₂R', =N-CN, or =NR', wherein R' is as defined above. Guided by this specification, the selection of suitable substituents is within the knowledge of one skilled in the art.

A substitutable nitrogen on a heteroaryl or a non-aromatic heterocyclic ring is optionally substituted. Suitable substituents on the nitrogen include R", COR", S(O)₂R", and CO₂R", where R" is H, an aliphatic group or a substituted aliphatic group.

The term "motif" refers to a group of amino acids in the protein that defines a structural compartment or carries out a function in the protein, for example, catalysis, structural stabilization or phosphorylation. The motif may be conserved in sequence, structure and function when. The motif can be contiguous in primary sequence or three-dimensional space. Examples of a motif include but are not limited to SXXXS motif, phosphorylation lip or activation loop, the glycine-rich phosphate anchor loop, the catalytic loop, the DFG loop and the APE motif (See, Xie et al., Structure, 6, pp. 983-991 (1998)).

The term "homologue of GSK-3β" or "GSK-3β homologue" refers to a molecule that is homologous to GSK-3β by structure or sequence, but retains the kinase activity of GSK-3. In one embodiment, the homologue has at least 80%, 90% or 95% sequence homology to GSK-3β. The homologue can be GSK-3α, GSK-3β from another species, with conservative substitutions, conservative additions or deletions thereof; human GSK-3β with conservative substitutions, conservative additions or deletions. For example, the GSK-3β can be full length protein (amino acids 1-420 of SEQ ID NO: 1); a truncated protein with amino acids 7-420, 25-381, 37-381 of SEQ ID NO: 1; the full length protein with conservative substitutions; the truncated protein with conservative mutations.

The term "part of a binding pocket" refers to less than all of the amino acid residues that define the binding pocket. The structure coordinates of residues that constitute part of a binding pocket may be specific for defining the chemical environment of the binding pocket, or useful in designing fragments of an inhibitor that may interact with those residues. For example, the portion of residues may be key residues that play a role in ligand binding, or may be residues that are spatially related and define a three-dimensional compartment of the binding pocket. The residues may be contiguous or non-contiguous in primary sequence.

Part of the binding pocket can be at least two amino acid residues. Part of the inhibitor-binding pocket can be GSK-3β amino acids D133 and V135, GSK-3β amino acids K85, L132, D133 and V135, GSK-3β amino acids K85, M101, V110, L130 and L132, GSK-3β amino acids I62, V135, P136, T138 and L188, GSK-3β amino acids V70, V110, L188 and C199, GSK-3β amino acids F67, V70, Q185 and C199. Part of a substrate binding pocket can be GSK-3β amino acids D90, K91, R92, F93, K94, GSK-3β amino acids R96, R180, K205, N213 and Y234, GSK-3β amino acids R96; R180 and K205, GSK-3β amino acids S66, F67 and F93, or GSK-3β amino acids Y216, I217, C218, S219, R220 and R223.

The term "part of a GSK-3β protein" refers to less than all of the amino acid residues of a GSK-3β protein. Part of a GSK-3β protein can define the binding pockets, domains or motifs of the protein. The structure coordinates of residues that constitute part of a GSK-3β protein may be specific for defining the chemical environment of the protein, or useful in designing fragments of an inhibitor that may interact with those residues. The portion of residues may also be residues that are spatially related and define a three-dimensional compartment of a binding pocket, motif or domain. The residues maybe contiguous or non-contiguous in primary sequence. For example, the portion of residues may be key residues that play a role in ligand or substrate binding, catalysis or structural stabilization.

The term "pharmaceutically acceptable carrier, adjuvant, or vehicle" refers to a non-toxic carrier, adjuvant, or vehicle that may be administered to a patient, together with a compound of this invention, and which does not destroy the pharmacological activity thereof .

The term "patient" includes human and veterinary subjects.

The term "peptide comprising a phosphorylation sequence" refers to a peptide comprising the ZXXXY motif. Z can be serine or threonine. Y can be serine, threonine or valine. X can be any amino acid residue. Z or Y can be phosphorylated or unphosphorylated. The phosphorylation of Y facilitates the phosphorylation of X by GSK-3β. Examples of peptide substrates comprising a phosphorylation sequence include but are not limited to, ASVPPS, PSPSLS, LSRHSS, SSPHQS, DSRAGS, LSRRPS, PTPPPT,PTPVPS, KSPVVS, VSGDTS, QSYLDS, DSGIHS, HSGATT, TTTAPS, TSANDS, DSEQQS, SSPLPS, PSSPLS and CTPTDV.

Pharmaceutically acceptable salts of the compounds of this invention include those derived from pharmaceutically acceptable inorganic and organic acids and bases. Examples of suitable acid salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, formate, fumarate, glucoheptanoate, glycerophosphate, glycolate, hemisulfate, heptanoate, hexanoate, hydrochloride, hydrobromide, hydroiodide, 2-hydroxyethanesulfonate, lactate, maleate, malonate, methanesulfonate, 2-naphthalenesulfonate, nicotinate, nitrate, oxalate, palmoate, pectinate, persulfate, 3-phenylpropionate, phosphate, picrate, pivalate, propionate, salicylate, succinate, sulfate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Salts derived from appropriate bases include alkali metal (e.g., sodium and potassium), alkaline earth metal (e.g., magnesium), ammonium and N⁺(C₁₋₄ alkyl)₄ salts. This invention also envisions the quaternization of any basic nitrogen-containing groups of the compounds disclosed herein. Water or oil-soluble or dispersible products may be obtained by such quaternization.

The term "protein kinase-mediated condition" or "state" refers to any disease or other deleterious condition or state in which a protein kinase is known to play a role. Such conditions include, without limitation, autoimmune diseases, inflammatory diseases, metabolic, neurological and neurodegenerative diseases, cardiovasclular diseases, allergy and asthma.

The term "root mean square deviation" or "RMSD" refers to the square root of the arithmetic mean of the squares of the deviations from the mean. It is a way to express the deviation or variation from a trend or object. For purposes of this invention, the "root mean square deviation" defines the variation in the backbone of a protein from the backbone of GSK-3β or a binding pocket portion thereof, as defined by the structure coordinates of GSK-3β described herein. It would be readily apparent to those skilled in the art that the calculation of RMSD involves standard error.

The term "soaked" refers to a process in which the crystal is transferred to a solution containing the compound of interest.

The term "structure coordinates" refers to Cartesian coordinates derived from mathematical equations related to the patterns obtained on diffraction of a monochromatic beam of X-rays by the atoms (scattering centers) of a protein or protein complex in crystal form. The diffraction data are used to calculate an electron density map of the repeating unit of the crystal. The electron density maps are then used to establish the positions of the individual atoms of the enzyme or enzyme complex.

The term "substantially all of a GSK-3β binding pocket" or "substantially all of a GSK-3β protein" refers to all or almost all of the amino acids in the GSK-3β binding pocket or protein. For example, substantially all of a GSK-3β binding pocket can be 100%, 95%, 90%, 80%, 70% of the residues defining the GSK-3β binding pocket or protein.

The term "substrate binding groove" refers to an area in a protein kinase where the substrate binds. The substrate binding groove is located at the interface of the β-strand and α-helical domain, and positioned between the activation loop and β-strand domain. Examples of substrates include but are not limited to glycogen synthase, β-catenin, elongation initiation factor 2B ε subunit, cAMP-responsive element binding protein, CCAAT/enhancer binding protein α, microtuble associated protein Tau, axin, Dd-STATa and Cyclin D1.

The term "substrate binding pocket" refers to a binding pocket of a molecule or molecular complex defined by the structure coordinates of a certain set of amino acid residues present in the GSK-3β structure, as described below. This binding pocket is in an area in the GSK-3β protein where the substrate binding groove is located.

The term "sufficiently homologous to GSK-3β" refers to a protein that has a sequence homology of at least 20% compared to GSK-3β protein. In one embodiment, the sequence homology is at least 40%.

### Inhibitors of GSK-3

One object of the instant invention is to provide compounds having formula (I): 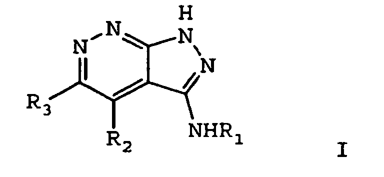 or a pharmaceutically acceptable derivative thereof, wherein:
R₁ is selected from H, aliphatic, RC(O)-, RS(O)ₙ-, ROC(O)-, carbocyclyl, heterocyclyl, aryl, aralkyl, heteroaryl, or heteroaralkyl; wherein said aliphatic, carbocyclyl, heterocyclyl, aryl, aralkyl, heteroaryl, or heteroaralkyl is optionally substituted;
R₂ and R₃ are each independently selected from H, aliphatic, carbocyclyl, heterocyclyl, aryl, aralkyl, heteroaryl, heteroaralkyl, -N(R)₂, -NRCOR, -NRCO₂R, -NRSO₂R, -S(O)ₙR, -SO₂N(R)₂, -SR, -OR, -CF₃, halo, -NO₂, -CN, -C(O)R, -CO₂R, -OC(O)R, -CON(R)₂, or -OC(O)N(R)₂, wherein said aliphatic, carbocyclyl, heterocyclyl, aryl, aralkyl, heteroaryl, or heteroaralkyl is optionally substituted; or R₂ and R₃ taken together with the intervening atoms optionally form a five- to nine-membered ring that is fused to the pyridazinyl ring of formula I, said fused ring having 0-2 heteroatoms;
each R is independently selected from H, aliphatic, carbocyclyl, heterocyclyl, aryl, aralkyl, heteroaryl, or heteroaralkyl, wherein each member of R except H is optionally substituted; and
n is 1 or 2;
   provided that when R₁ is H, R₂ and R₃ are not both unsubstituted phenyl; and when R₁ is H, R₂ and R₃ are other than H, halogen, or an unsubstituted alkyl.

It will be apparent to one skilled in the art that certain compounds of this invention may exist in tautomeric forms, all such tautomeric forms of the compounds being within the scope of the invention.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the invention. Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon are within the scope of this invention.

In a preferred embodiment of the invention, R₁ is H, RC(O)-, or aralkyl, wherein R is as defined above. In a more preferred embodiment, R₁ is H, aliphatic-C(O)-, aryl-C(O)-, or aralkyl. In an even more preferred embodiment, R₁ is H, CH₃C(O)-, PhC(O)-, or PhCH₂-.

In another preferred embodiment, R₂ and R₃ are independently H, aryl or heteroaryl. In another preferred embodiment, R₂ and R₃ are independently H, aryl, carbocyclyl, heterocyclyl, or heteroaryl. In another embodiment, R₂ and R₃ are independently aryl, carbocyclyl, heterocyclyl, or heteroaryl. Preferably, R₂ and R₃ are independently H, phenyl, naphthyl, pyridyl, thienyl, furanyl, pyrimidinyl, benzodioxolyl, or cyclohexyl, any of which except H is optionally substituted. More preferably, R₂ and R₃ are independently phenyl, naphthyl, pyridyl, thienyl, furanyl, pyrimidinyl, benzodioxolyl, or cyclohexyl, any of which is optionally substituted. Even more preferably, the substituents on phenyl, naphthyl, pyridyl, thienyl, furanyl, pyrimidinyl, benzodioxolyl, or cyclohexyl are selected from halo, alkyl, -CN, -NO₂, -SO₂NH₂, -SO₂NH-(alkyl), -SO₂N(alkyl)₂, -O-alkyl, -NH₂, -N-alkyl, -N-(alkyl)₂, -CONH₂, -CONH(alkyl), -CONH(alkyl)₂, -O-phenyl, or -S-alkyl.

In another preferred embodiment, when R₁ is a large group, R₂ is a small group. A small group refers to hydrogen or a moiety that contains 3 carbons or less, such as methyl, ethyl, or propyl. A large group refers to a moiety that contains 4 or more carbons.

In another preferred embodiment, R₁ is H, and R₂ and R₃ are independently H or an optionally substituted phenyl.

A more preferred embodiment of the invention is shown in formula Ia: 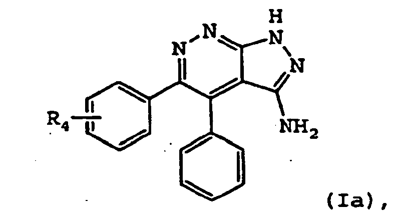 wherein R₄ is halo. In an even more preferred embodiment, R₄ is F.

Representative examples of compounds of the present invention are shown below in Table 1.

**TABLE 1**

| | | | |
|---|---|---|---|
| 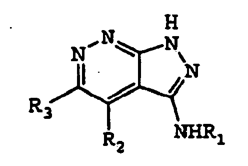 | | | |

| Compound No. | R₁ | R₂ | R₃ |
|---|---|---|---|
| Ref. 1 | H | 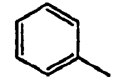 | 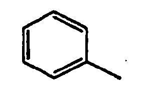 |
| 2 | H | 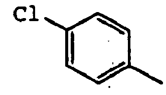 | 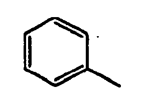 |
| 3 | H | 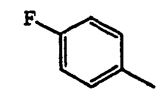 | 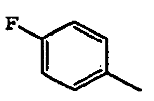 |
| 4 | H | 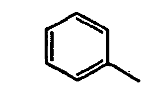 | 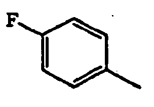 |
| 5 | H | 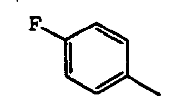 | 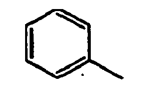 |
| 6 | H | 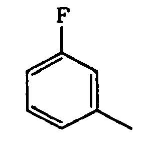 | 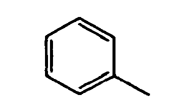 |
| 7 | H | 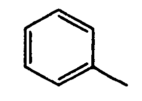 | 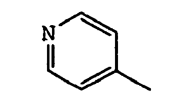 |
| 8 | H | 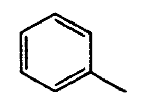 | 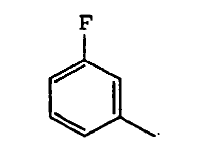 |
| 9 | 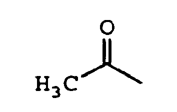 | 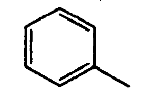 | 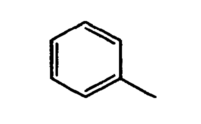 |
| 10 | 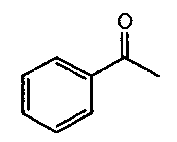 | 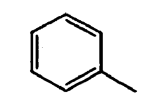 | 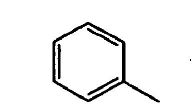 |
| 11 | H | 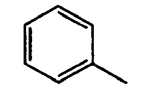 | 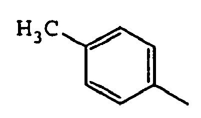 |
| 12 | H | 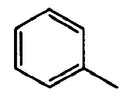 | 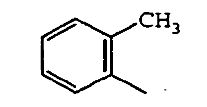 |
| 13 | H | 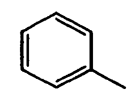 | 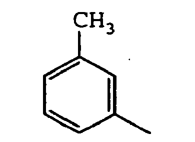 |
| 14 | H | 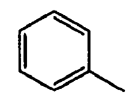 | 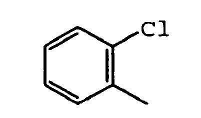 |
| 15 | H | 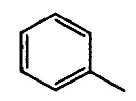 | 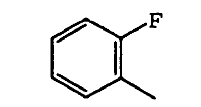 |
| 16 | H | 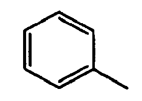 | 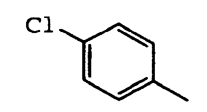 |
| 17 | H | 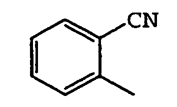 | 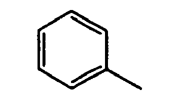 |
| 18 | H | 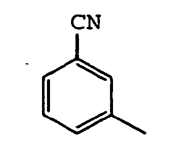 | 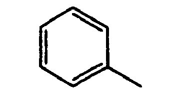 |
| 19 | H | 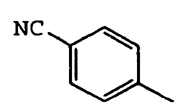 | 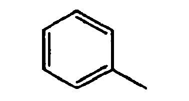 |
| 20 | H | 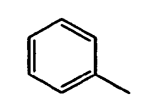 | 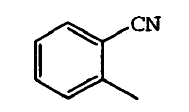 |
| 21 | H | 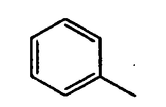 | 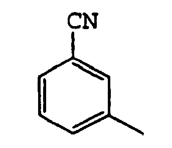 |
| 22 | H | 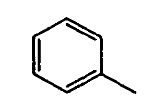 | 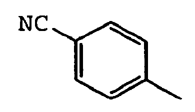 |
| 23 | H | 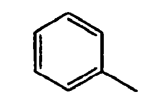 | 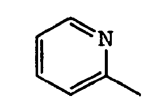 |
| 24 | H | 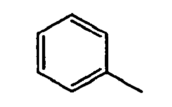 | 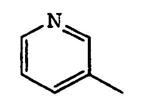 |
| 25 | 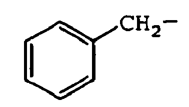 | 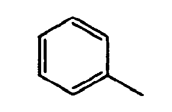 | 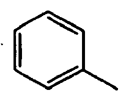 |
| 26 | H | 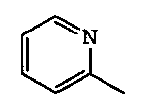 | 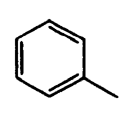 |
| 27 | H | 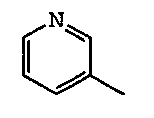 | 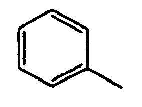 |
| 28 | H | 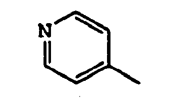 | 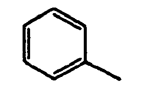 |
| 29 | H | 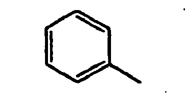 | 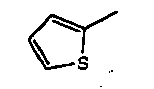 |
| 30 | H | 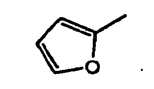 | 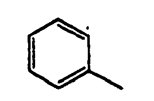 |
| 31 | H | 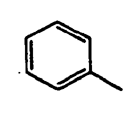 | 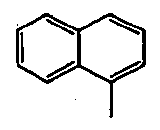 |
| Ref. 32 | H | 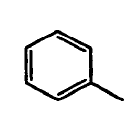 | H |
| Ref. 33 | H | H | 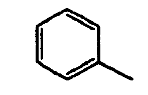 |
| 35 | H | 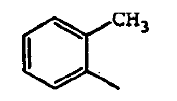 | 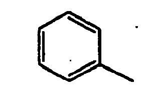 |
| 36 | H | 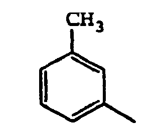 | 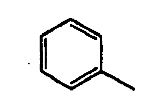 |
| 37 | H | 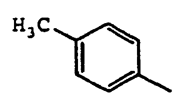 | 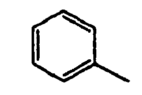 |
| 38 | H | 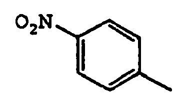 | 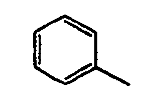 |
| 39 | H | 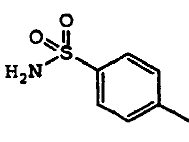 | 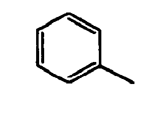 |
| 40 | H | 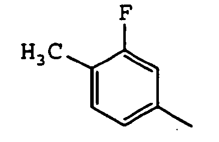 | 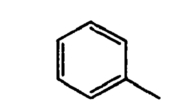 |
| 41 | H | 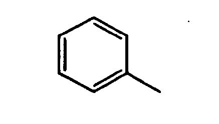 | 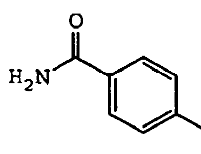 |
| 42 | H | 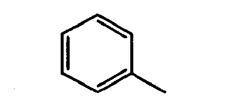 | 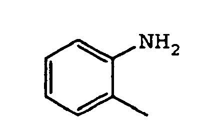 |
| 43 | H | 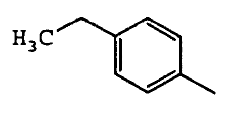 | 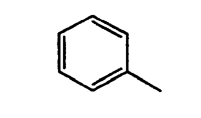 |
| 44 | H | 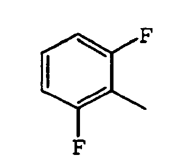 | 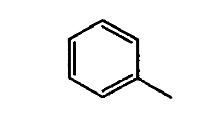 |
| 45 | H | 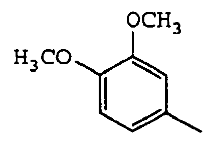 | 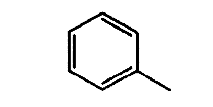 |
| 46 | H | 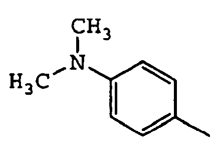 | 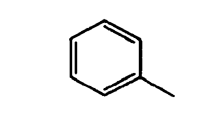 |
| 47 | H | 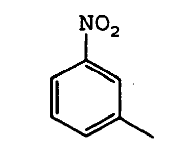 | 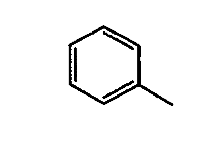 |
| 48 | H | 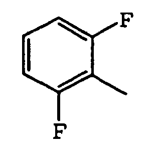 | 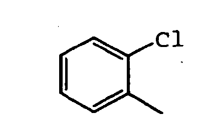 |
| 49 | H | 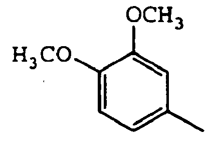 | 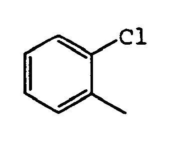 |
| 50 | H | 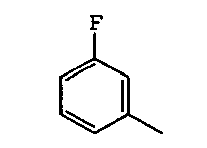 | 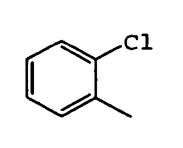 |
| 51 | H | 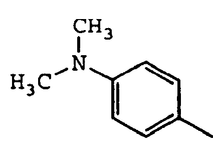 | 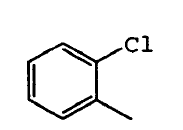 |
| 52 | H | 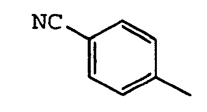 | 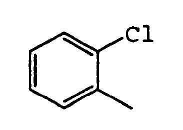 |
| 53 | H | 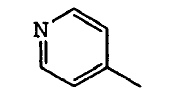 | 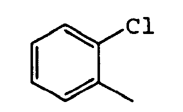 |
| 54 | H | 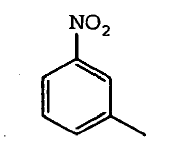 | 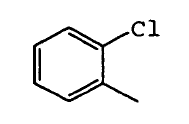 |
| 55 | H | 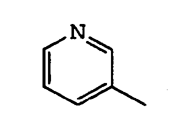 | 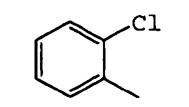 |
| 56 | H | 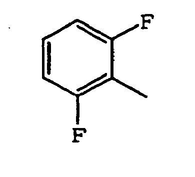 | 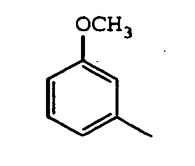 |
| 57 | H | 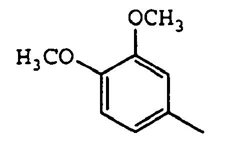 | 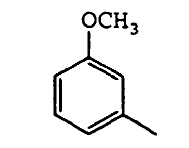 |
| 58 | H | 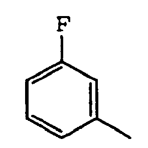 | 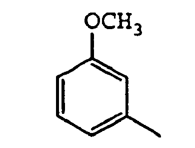 |
| 59 | H | 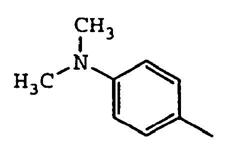 | 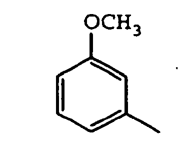 |
| 60 | H | 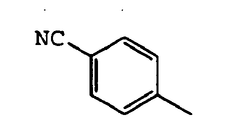 | 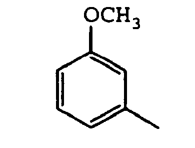 |
| 61 | H | 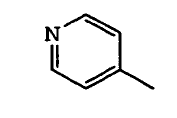 | 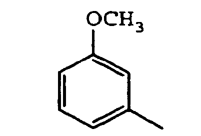 |
| 62 | H | 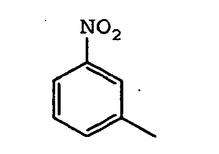 | 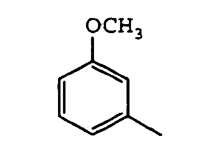 |
| 63 | H | 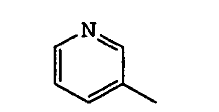 | 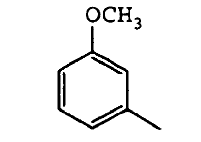 |
| 64 | H | 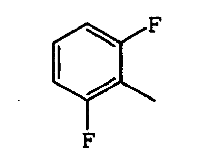 | 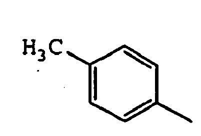 |
| 65 | H | 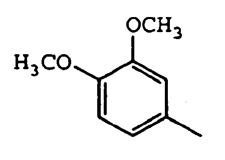 | 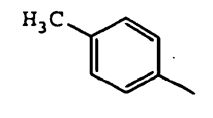 |
| 66 | H | 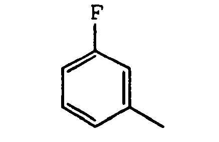 | 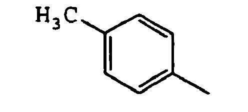 |
| 67 | H | 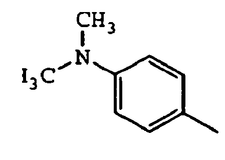 | 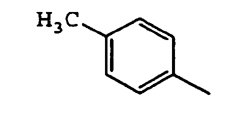 |
| 68 | H | 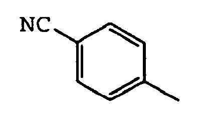 | 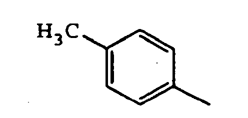 |
| 69 | H | 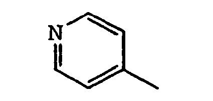 | 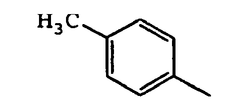 |
| 70 | H | 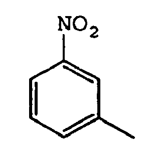 | 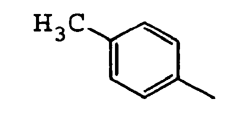 |
| 71 | H | 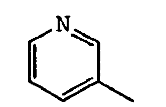 | 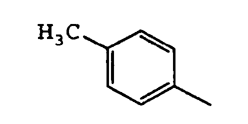 |
| 72 | H | 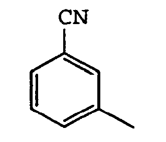 | 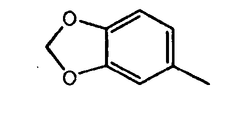 |
| 73 | H | 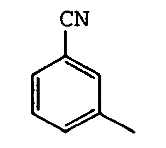 | 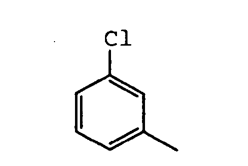 |
| 74 | H | 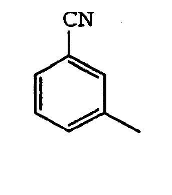 | 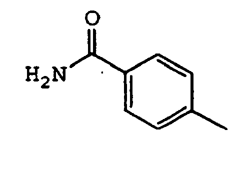 |
| 75 | H | 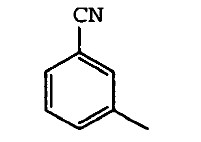 | 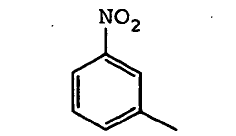 |
| 76 | H | 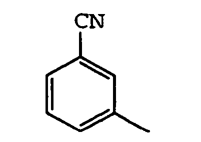 | 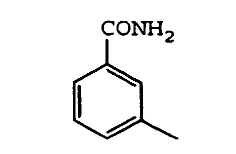 |
| 77 | H | 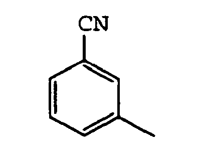 | 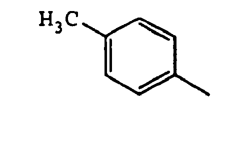 |
| 78 | H | 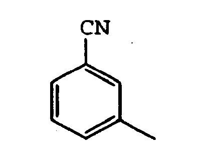 | 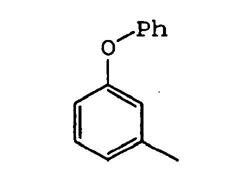 |
| 79 | H | 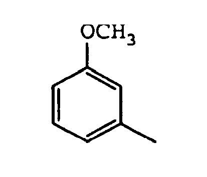 | 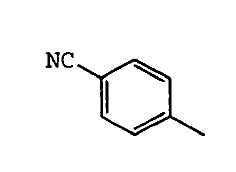 |
| 80 | H | 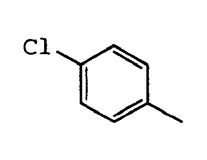 | 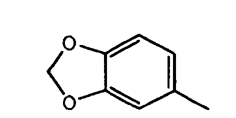 |
| 81 | H | 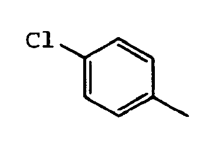 | 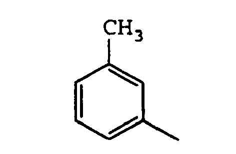 |
| 82 | H | 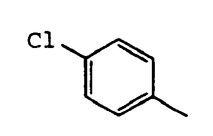 | 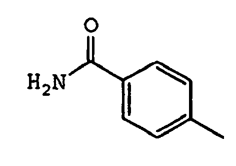 |
| 83 | H | 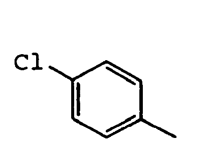 | 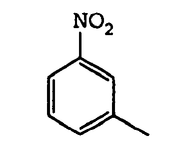 |
| 84 | H | 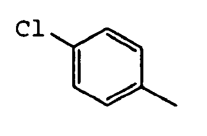 | 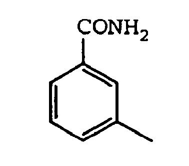 |
| 85 | H | 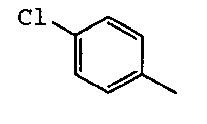 | 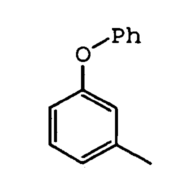 |
| 86 | H | 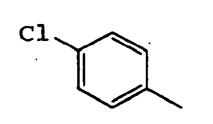 | 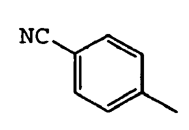 |
| 87 | H | 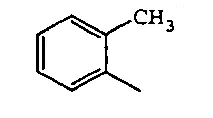 | 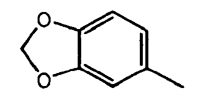 |
| 88 | H | 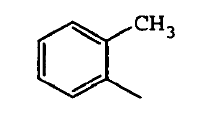 | 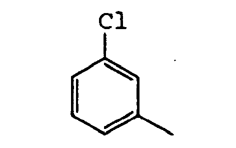 |
| 89 | H | 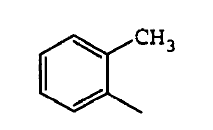 | 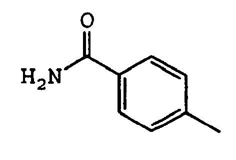 |
| 90 | H | 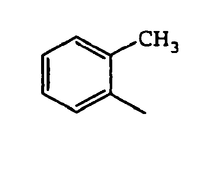 | 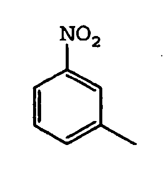 |
| 91 | H | 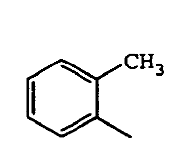 | 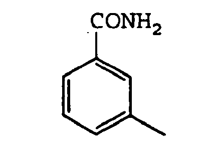 |
| 92 | H | 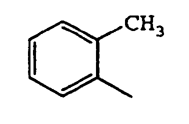 | 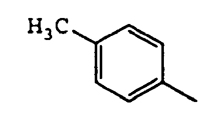 |
| 93 | H | 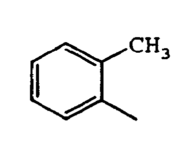 | 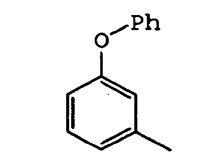 |
| 94 | H | 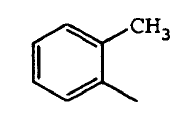 | 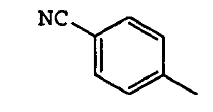 |
| 95 | H | 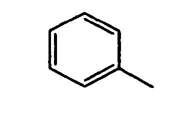 | 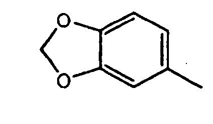 |
| 96 | H | 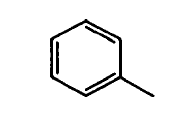 | 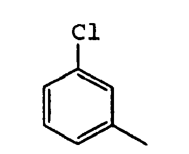 |
| 97 | H | 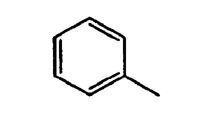 | 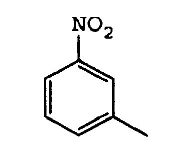 |
| 98 | H | 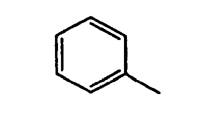 | 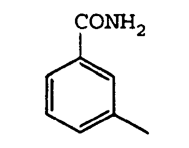 |
| 99 | H | 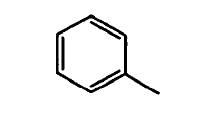 | 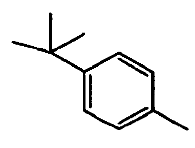 |
| 100 | H | 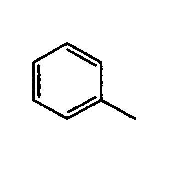 | 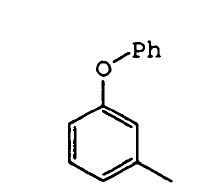 |
| 101 | H | 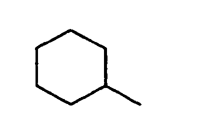 | 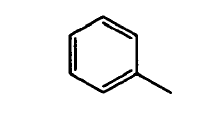 |
| 102 | H | 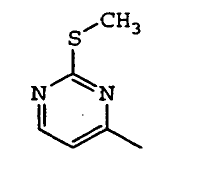 | 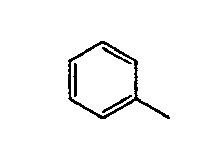 |

In a more preferred embodiment, the compound of the present invention is 3-amino-4-phenyl-5-(3-fluorophenyl)-1H-pyrazolo[3,4-c] pyridazine (Compound 8).

### Methods for Producing GSK-3 Inhibitors

The compounds of this invention generally may be prepared from known starting materials, following methods known to those skilled in the art for analogous compounds, as illustrated by general Scheme I and the synthetic examples described below. References that may be useful in making the present compounds include El-Gendy, A.M. et al., Asian J. Chem. , 1, 376 (1989) ; Deeb, A. and Said, S.A., Collect. Czech. Chem. Comm., 50, 2795 (1990) ; and Shalaby, A.A. J. Prakt. Chemie, 332, 104 (1990) .

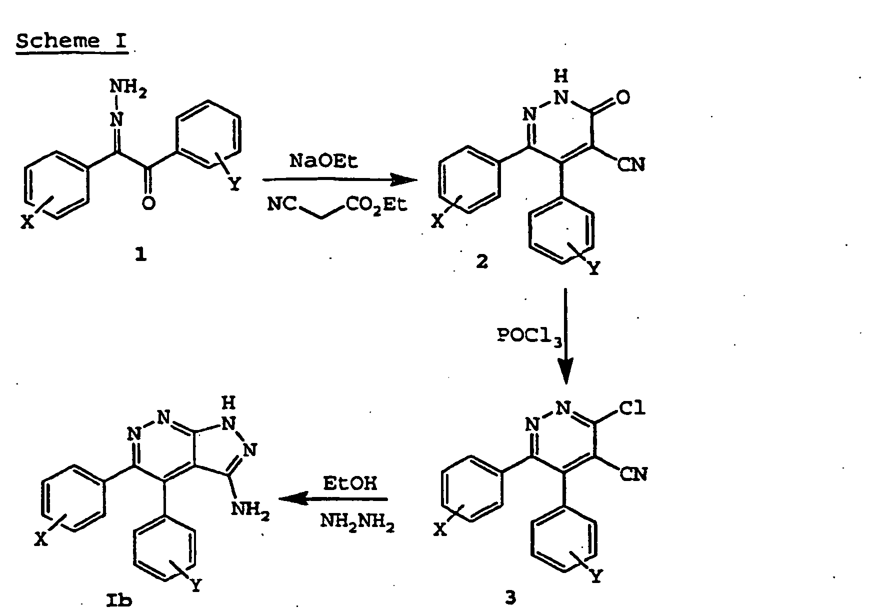

Scheme I shows a general approach for making the present compounds. The unsymmetrical diaryl keto hydrazones (1) were prepared from the corresponding substituted deoxybenzoins as described in United States Patent 4,009,022. The substituted deoxybenzoins were readily synthesized following methods known in the art, for instance, those described in Hill, D.T. et al, J. Het. Chem., 28, 1181 (1991); Rieke, R.D. et al, J. Org. Chem., 56, 1445 (1991); Fujisowa, T. et al, Chem. Lett., 1135 (1981); and Iyoda, M. et al, Tet. Lett., 26, 4777 (1985). To an ethanol solution of diaryl keto hydrazone (1), ethyl cyanoacetate (excess) and sodium ethoxide in tetrahydrofuran (THF) were added. The mixture was refluxed for 6 hours. After cooling, the solvent was removed under vacuum and the residue was taken up in dichloromethane (CH₂Cl₂), washed with 0.1 M HCl and water and dried with sodium sulfate. After filtering, the solvent was removed under vacuum and the product, 4-cyano-5,6-diaryl 2(1H) pyridazinone (2) was purified by chromatography on silica gel (5:95 methanol/dichloromethane).

Purified 4-cyano-5,6-diaryl 2(1H) pyridazinone (2) was added to POCl₃ and heated to 100°C for 5-6 hours. After cooling, the reaction mixture was poured onto ice and stirred for one hour. The resultant 3-chloro-4-cyano-5,6-diaryl pyridazine (3) was filtered off, washed with water, air dried and used in the next step without further purification. Purified 3-chloro-4-cyano-5,6-diaryl pyridazine (3) was further refluxed with 2 equivalents of anhydrous hydrazine in ethanol for several hours. Upon cooling, the product Ib would sometimes precipitate out, in which case compound Ib was purified by recrystallizing from ethanol. Otherwise purification of Ib was achieved by chromatography on silica gel (5:95 methanol/dichloromethane).

One having ordinary skill in that art may synthesize other compounds of this invention following the teachings of the specification using reagents that are readily synthesized or commercially available.

The present invention provides detailed methods of producing representative compounds of the present invention as described in Examples 1-17 below.

The activity of the compounds as protein kinase inhibitors, for example, as GSK-3 inhibitors, may be assayed *in vitro, in vivo* or in a cell line. *In vitro* assays include assays that determine inhibition of either the phosphorylation activity or ATPase activity of activated GSK-3. Alternate in vitro assays quantitate the ability of the inhibitor to bind to GSK-3. Inhibitor binding may be measured by radiolabelling the inhibitor prior to binding, isolating the inhibitor/GSK-3 complex and determining the amount of radiolabel bound. Alternatively, inhibitor binding may be determined by running a competition experiment where new inhibitors are incubated with GSK-3 bound to known radioligands.

### Pharmaceutical Compositions

According to another embodiment of the invention, the protein kinase inhibitors, particularly GSK-3 inhibitors, or derivatives/salts thereof may be formulated into compositions. In a preferred embodiment, the composition is a pharmaceutical composition. In one embodiment, the composition comprises an amount of the protein kinase inhibitor effective to inhibit GSK-3 in a biological sample or in a patient. In another embodiement, the pharmaceutical compositions, which comprise an amount of the protein kinase inhibitor effective to treat or prevent a GSK-3-mediated condition and a pharmaceutically acceptable carrier, adjuvant, or vehicle, may be formulated for administration to a patient.

The amount effective to inhibit GSK-3 is one that inhibits the kinase activity of GSK-3 at least 50%, more preferably at least 60% or 70%, even more preferably at least 80% or 90%, and most preferably at least 95%, where compared to the GSK-3 activity of the enzyme in the absence of an inhibitor. Any method may be used to determine inhibition. See, e.g., Example 18.

Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, favoring or coloring agents may also be added.

Alternatively, the pharmaceutical compositions of this invention may be administered in the form of suppositories for rectal administration. These can be prepared by mixing the agent with a suitable nonirritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

In addition to the compounds of this invention, pharmaceutically acceptable derivatives or prodrugs of the compounds of this invention may also be employed in compositions to treat or prevent the above-identified diseases or disorders.

The amount of the protein kinase inhibitor that may be combined with the carrier materials to produce a single dosage form will vary:depending upon the patient treated and the particular mode of administration. Preferably, the compositions should be formulated so that a dosage of between 0.01 - 100 mg/kg body weight/day of the inhibitor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of inhibitor will also depend upon the particular compound in the composition.

### Treatment and Prevention of Disease

One aspect of this invention relates to the use of a therapeutically effective amount of a compound of formula I or a pharmaceutically acceptable derivatives thereof for the preparation of a medicament for treating a disease state in patients that is alleviated by treatment with a GSK-3 inhibitor.

Another aspect of this invention relates to the use of a therapeutically effective amount of a compound of formula I or a pharmaceutically acceptable derivatives thereof for the preparation of a medicament for inhibiting GSK-3 activity in a patient, Another use relates to enhancing glycogen synthesis and/or lowering blood levels of glucose in a patient in need thereof. This use is especially useful for diabetic patients. Another use relates to inhibiting the production of hyperphosphorylated Tau protein, which is useful in halting or slowing the progression of Alzheimer's disease. Another use relates to inhibiting the phosphorylation of β-catenin, which is useful for treating schizophrenia.

Depending upon the particular protein kinase-mediated condition to be treated or prevented, additional drugs, which are normally administered to treat or prevent that condition, may be administered together with the inhibitors of this invention. For example, in the treatment of diabetes, other anti-diabetic agents may be combined with the GSK-3 inhibitors of this invention to treat diabetes. These agents include, without limitation, insulin, in injectable or inhalation form, glitazones, and sulfonyl ureas.

Those additional agents may be administered separately from the protein kinase inhibitor-containing composition, as part of a multiple dosage regimen. Alternatively, those agents may be part of a single dosage form, mixed together with the protein kinase inhibitor of this invention in a single composition.

Another method of this invention relates to inhibiting GSK-3 activity in a biological sample, which method comprises contacting the biological sample with the GSK-3 inhibitor of formula I or a pharmaceutically acceptable derivative or prodrug thereof, or a pharmaceutical composition thereof, in an amount effective to inhibit GSK-3.

### Crystallizable Compositions and Crystals of GSK-3β Protein and Protein complexes

The application discloses a crystallizable composition comprising unphosphorylated GSK-3β protein or its homologue and phosphate ions. The crystallizable composition may further comprise between about 5 to 25% v/v of precipitant polyethylene glycol, a buffer that maintains pH between about 4.0 and 8.0; and optionally, a reducing agent of 1-20 mM. The crystallizable composition may comprise unphosphorylated GSK-3β protein, 15% PEG 3350, 50 mM Na/KPO₄ at pH 4.1 and 10 mM DTT.

The application discloses a crystallizable composition comprising phosphorylated GSK-3β protein or its homologue. The crystallizable composition may further comprise between about 5-25% v/v polyethylene glycol, a buffer that maintains pH between about 6.0 and 8.5, and 1-10% dimethyl sulfoxide(DMSO). The crystallizable composition may comprise phosphorylated GSK-3β protein, between about 7-10% PEG 3350, 100 mM Tris HCl and 5% DMSO.

The application discloses a crystallizable composition comprising GSK-3β protein or its homologue and a chemical entity. The GSK-3β protein may be phosphorylated or unphosphorylated. The chemical entity may be selected from the group consisting of an inhibitor for the active site, a nucleotide triphosphate, an ATP, a substrate or inhibitor for the substrate binding groove, or a peptide comprising a phosphorylation sequence. The inhibitor for the active site may be selected from the group consisting of 4,5-Diphenyl-1H-pyrazolo[3,4-c]pyridazin-3-ylamine; (5-Methyl-2H-pyrazol-3-yl)-(2-pyridin-4-yl-quinazolin-4-yl)-amine; 4-(5-Methyl-2-phenylamino-pyrimidin-4-yl)-1H-pyrrole-2-carboxylic acid (2-hydroxy-1-phenyl-ethyl)-amide; (1H-Indazol-3-yl)-[2-(2-trifluoromethyl-phenyl)-quinazolin-4-yl]-amine and an ATP analogue. The crystallizable composition may further comprise between about 5-25% v/v polyethylene glycol, and between about 0.1-1 M ammonium fluoride, ammonium formate, potassium formate or potassium fluoride. The peptide comprising a phosphorylation sequence may be HSSPHQpSEDEEE. The crystallizable composition may comprise phosphorylated GSK-3β protein, HSSPHQpSEDEEE, between about 10-15% v/v polyethylene glycol, and 50 mM ammonium fluoride.

The GSK-3β protein or its homologue is preferably 85-100% pure prior to forming the crystallizable composition.

The application discloses a crystal composition comprising unphosphorylated GSK-3β protein or its homologue and phosphate ions. The application discloses a crystal composition comprising unphosphorylated GSK-3β protein or its homologue and a chemical entity. Preferably, the chemical entity is an inhibitor for the active site, an ATP analogue or nucleotide triphosphate. Preferably, the crystal has a unit cell dimension of a= 83 Å b= 86 Å c= 178 Å, α = β = γ = 90° and belongs to space group P2₁2₂2₁. It will be readily apparent to those skilled in the art that the unit cells of the crystal compositions may deviate ± 1-2 Å from the above cell dimensions depending on the deviation in the unit cell calculations.

The application also discloses a crystal composition comprising phosphorylated GSK-3β protein or its homologue with or without a chemical entity. Preferably, the chemical entity is an inhibitor for the active site, an ATP analogue or nucleotide triphosphate. Preferably, the unit cell dimensions of the crystal is a= 64 Å b=67 Å c= 67 Å α=100° β= 103° γ= 89.8° or a= 64 Å b=67 Å c= 67 Å α= 80° β= 77°, γ= 89.8° and belongs to the space group P1. The chemical entity may be a substrate or inhibitor to the substrate binding groove, or a peptide comprising a phosphorylation sequence. Preferably, the chemical entity is HSSPHQpSEDEEE and the unit cell dimensions of the crystal is a= 75 Å b=108 Å c= 121 Å α=β=γ= 90° and belongs to the space group P2₁2₁2₁. It will be readily apparent to those skilled in the art that the unit cells of the crystal compositions may deviate ± 1-2 Å or ± 1-2° from the above cell dimensions depending on the deviation in the unit cell calculations.

As used herein, the GSK-3β protein in the crystallizable or crystal compositions can be the full length GSK-3β protein (amino acids 1-420 of SEQ ID NO: 1); a truncated GSK-3β protein with amino acids 7-420, 25-381, 37-381 of SEQ ID NO: 1; the full length protein with conservative substitutions; said truncated protein with conservative mutations. The GSK-3β protein may be produced from the baculovirus system. The unphosphorylated GSK-3β protein is not phosphorylated at any of the phosphorylation sites. The phosphorylated GSK-3β protein is phosphorylated at any of the phosphorylation sites, for example, at Serine 9 or Tyrosine 216. Preferably, the protein is phosphorylated at Tyrosine 216.

The GSK-3β protein or its homologue may be produced by any well-known method, including synthetic methods, such as solid phase, liquid phase and combination solid phase/liquid phase syntheses; recombinant DNA methods, including cDNA cloning, optionally combined with site directed mutagenesis; and/or purification of the natural products. The protein may be overexpressed from a baculovirus system or E. Coli system.

The application also discloses a method of obtaining a crystal of a GSK-3β protein complex or GSK-3β homologue protein complex comprising a chemical entity that binds to the substrate-binding groove, comprising the steps of:
a) producing and purifying a GSK-3β protein;
b) mixing a crystallization solution with the protein complex to produce a crystallizable composition; and
c) subjecting the composition to conditions which promote crystallization.

Conditions for promoting crystallization include, for example, apparatuses and devices for forming crystals, for example, a hanging drop, sitting drop, dialysis or microtube batch device, will promote crystallization. (U.S. patent 4,886,646, 5,096,676, 5,130,105, 5,221,410 and 5,400,741; Pav et al., Proteins: Structure, Function, and Genetics, 20, pp. 98-102 (1994)) The hanging drop or sitting drop methods produce crystals by vapor diffusion. The hanging drop or sitting drop which contains the crystallizable composition is equilibrated against a reservoir containing a higher concentration of precipitant. As the drop approaches equilibrium with the reservoir, the protein becomes saturated in solution and crystals form. One of ordinary skilled in the art would be able to vary the crystallization conditions disclosed above and identify other crystallization conditions that would produce crystals for GSK-3 protein or its homologues with or without a chemical entity. Such variations may include adjusting the pH, salt type or concentration, precipitant type or concentration, crystallization temperature, protein concentration. One may also use high throughput crystallization assays to assist in finding or optimizing the crystallization condition.

### Binding Pockets of GSK-3β Protein, Protein Complexes or Homologues thereof

As discussed above, applicants have disclosed the three-dimensional X-ray crystal structures of unphosphorylated GSK-3β, phosphorylated GSK-3β, unphosphorylated GSK-3β-inhibitor complexes, phosphorylated GSK-3β-inhibitor complex and phosphorylated GSK-3β-ADP-peptide complex. The crystal structure of GSK-3β presented here is within the GSK-3 subfamily. The application will be useful for inhibitor design. The atomic coordinate data is presented in figures 1-7.

In order to use the structure coordinates generated for the unphosphorylated and phosphorylated GSK-3β, their complexes or one of its binding pockets or GSK-3β-like binding pocket thereof, it is often times necessary to convert them into a three-dimensional shape. This is achieved through the use of commercially available software that is capable of generating three-dimensional structures of molecules or portions thereof from a set of structure coordinates.

Binding pockets, also referred to as binding sites in this application, are of significant utility in fields such as drug discovery. The association of natural ligands or substrates with the binding pockets of their corresponding receptors or enzymes is the basis of many biological mechanisms of action. Similarly, many drugs exert their biological effects through association with the binding pockets of receptors and enzymes. Such associations may occur with all or part of the binding pocket. An understanding of such associations will help lead to the design of drugs having more favorable associations with their target receptor or enzyme, and thus, improved biological effects. Therefore, this information is valuable in designing potential inhibitors of the binding pockets of biologically important targets. The binding pockets disclosed in this application will be important for drug design.

The structure coordinates described above may be used to derive the torsion angles of the side chains (S.C. Lovell et al, Proteins: Structure, Function, and Genetics, 40, 389-408, (2000)). For example, in Glutamine, χ1 defines the torsion angle between N, Cα, Cβ, Cγ; χ2 defines the torsion angle between Cα, Cβ, Cγ, Cδ; and χ3 defines the torsion angle between Cβ, Cγ, Cδ, Oε.

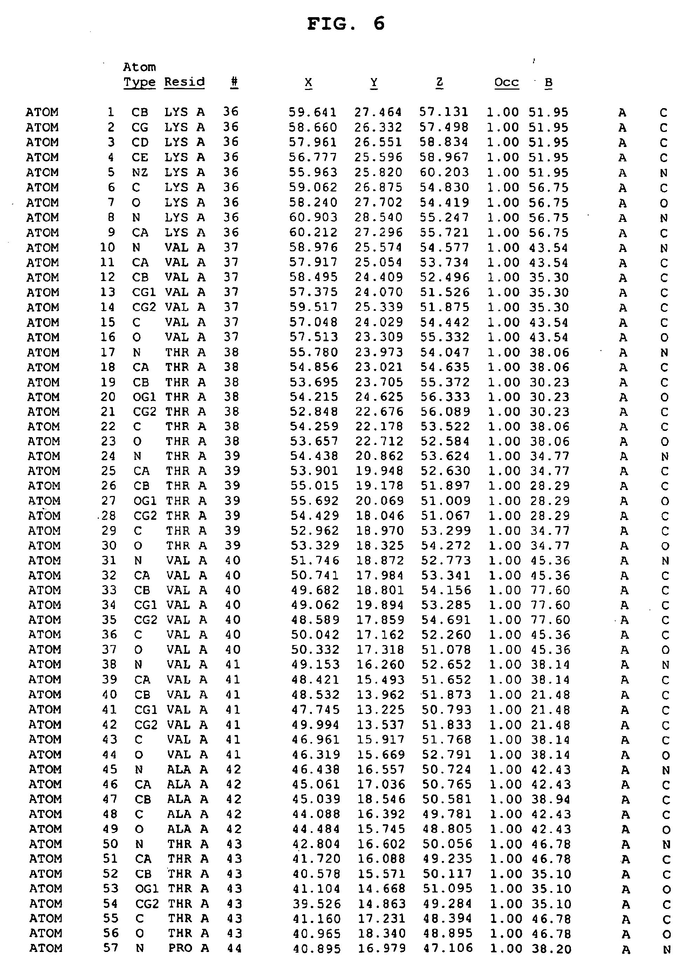

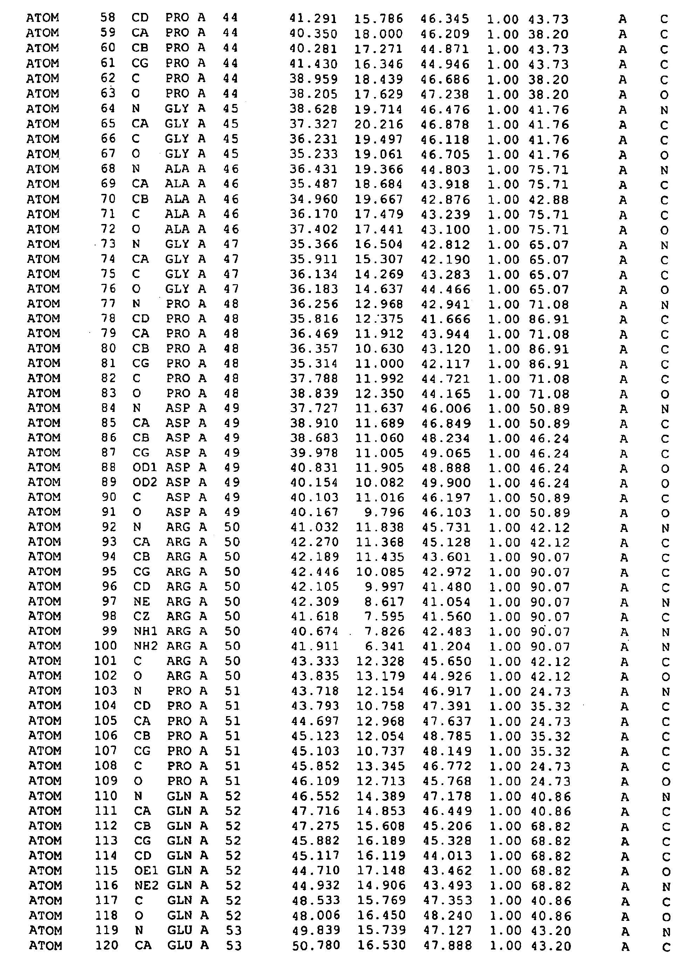

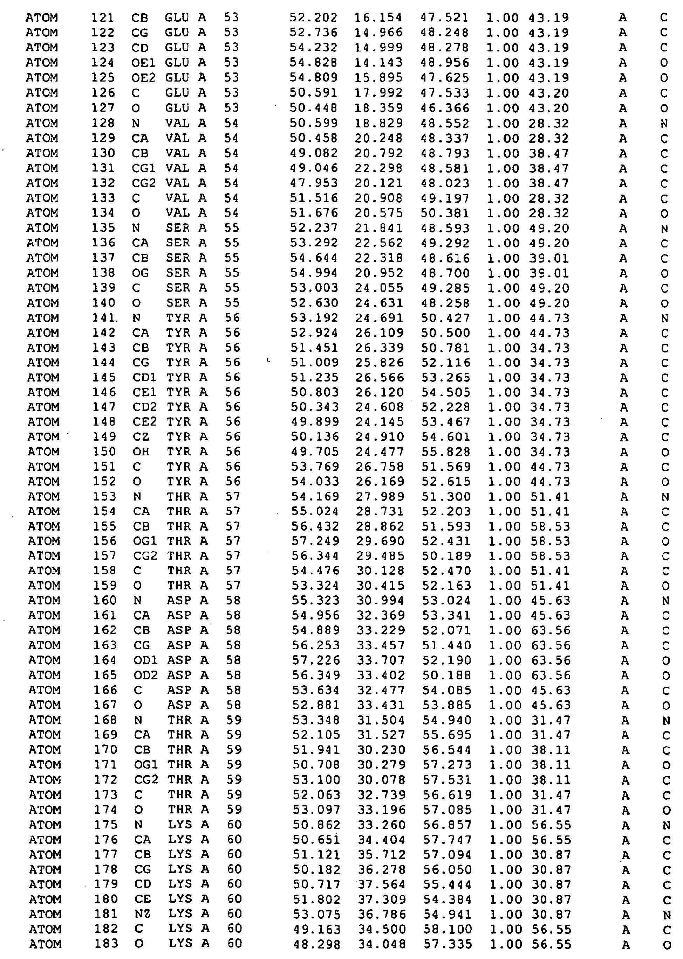

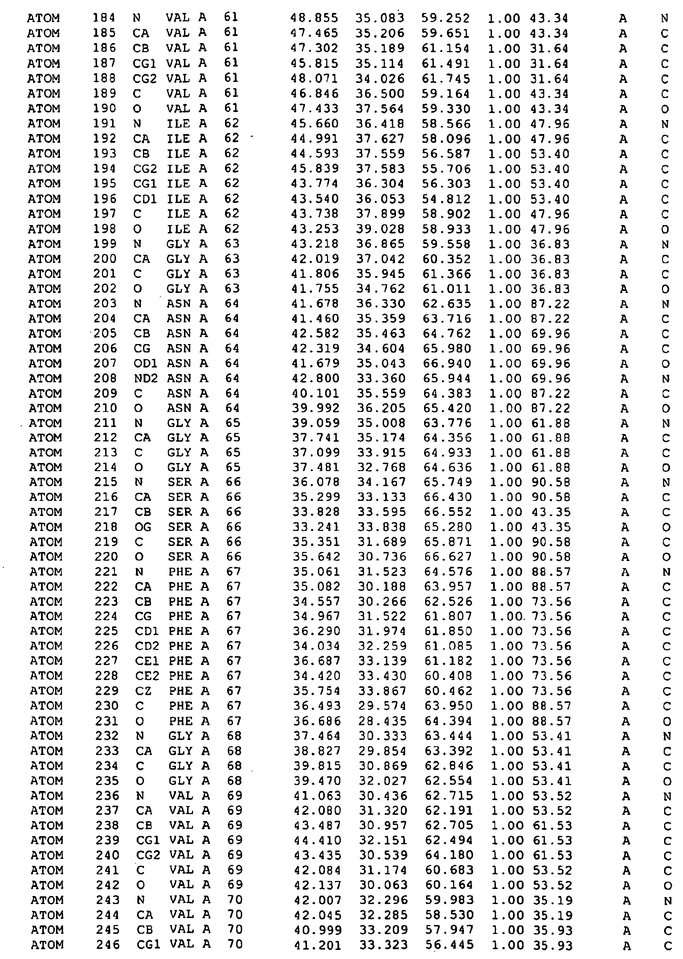

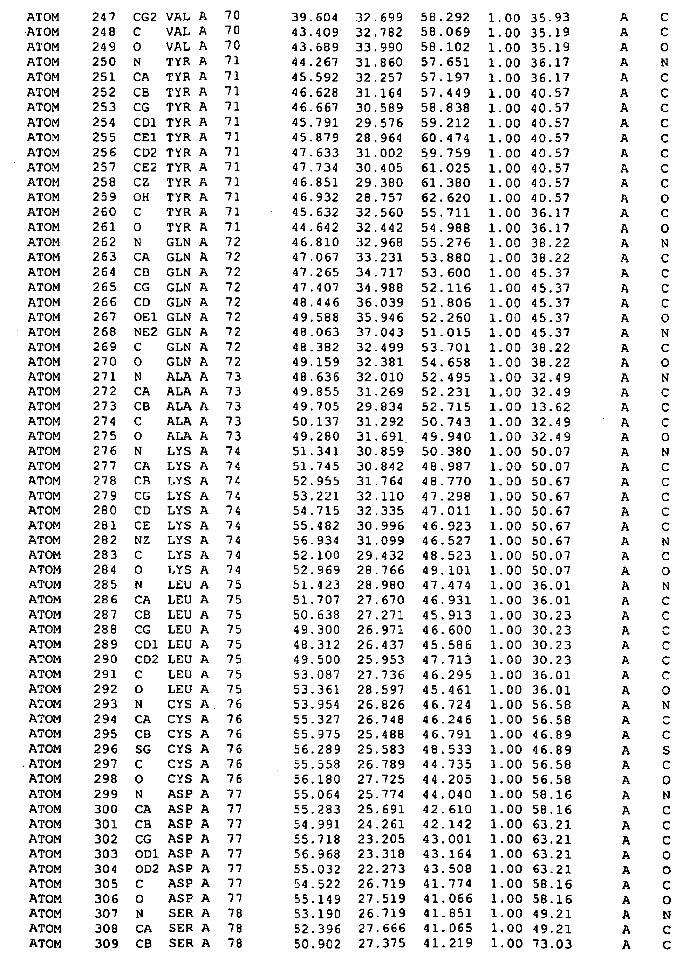

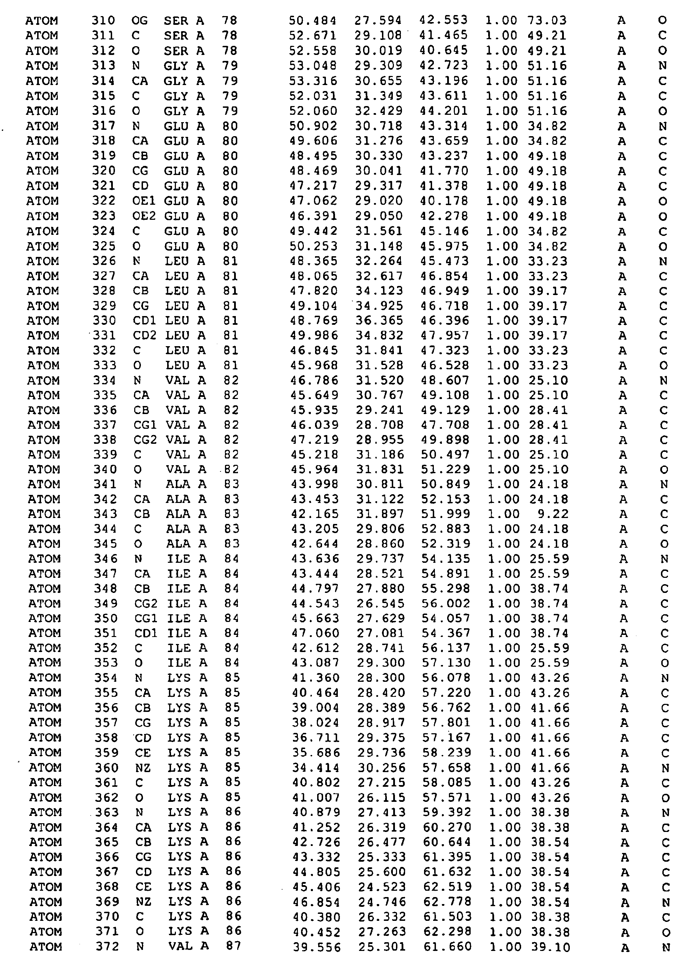

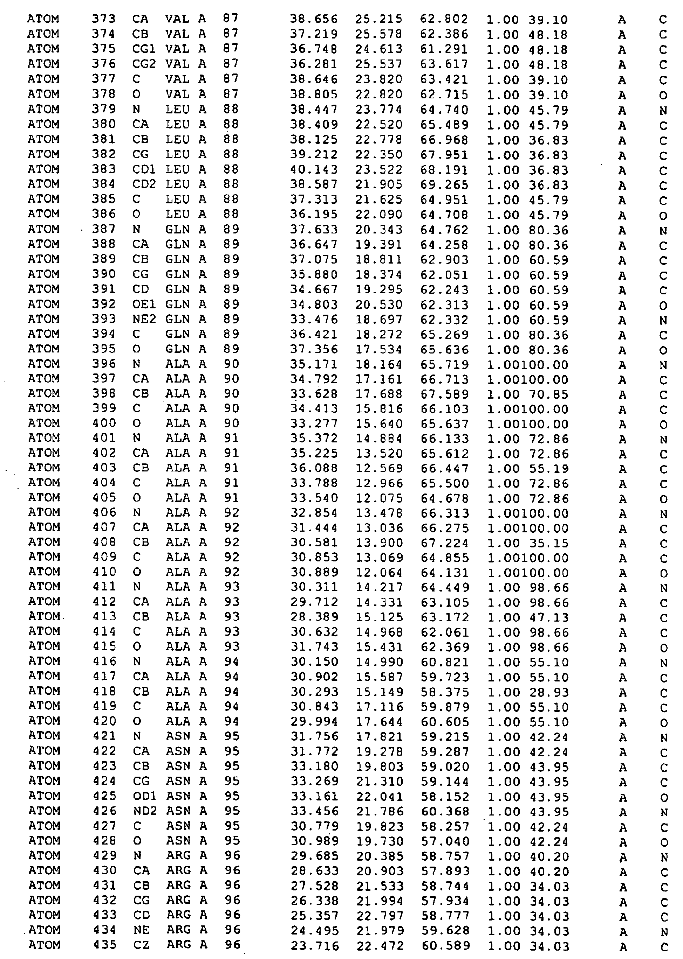

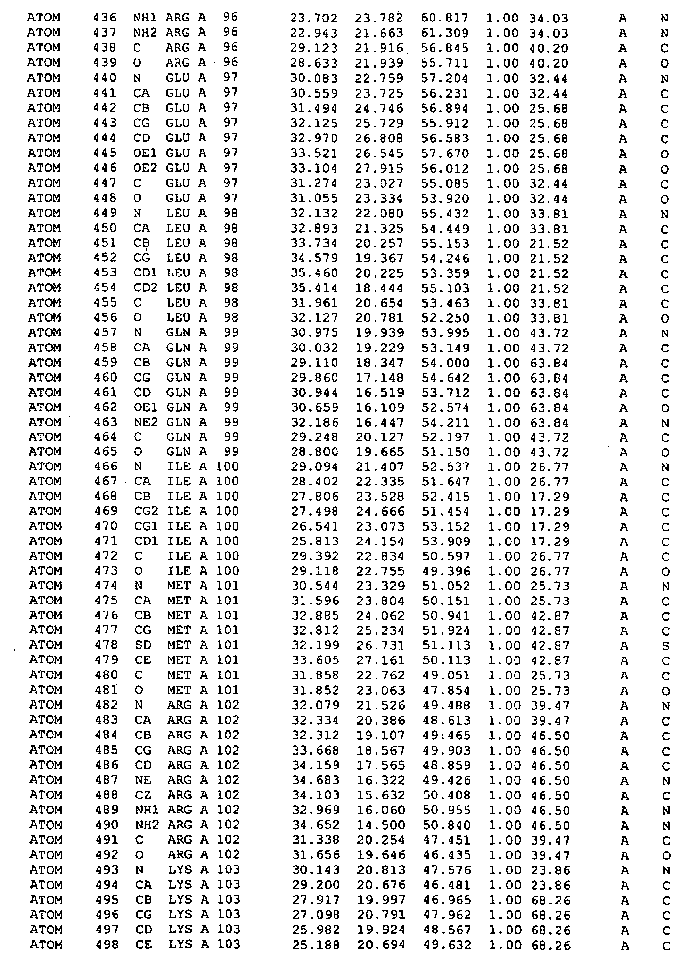

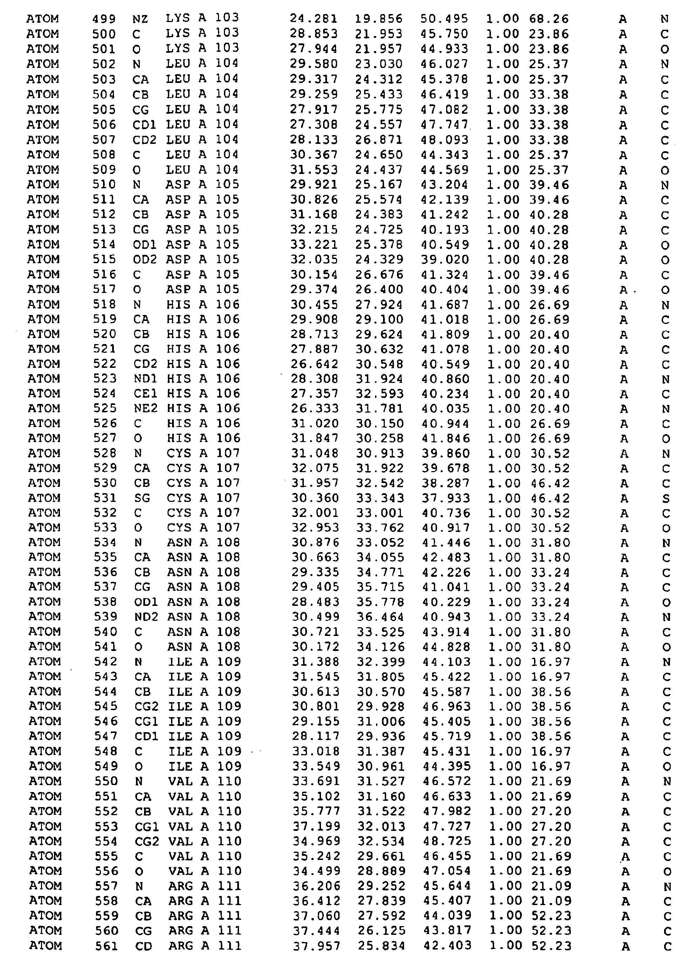

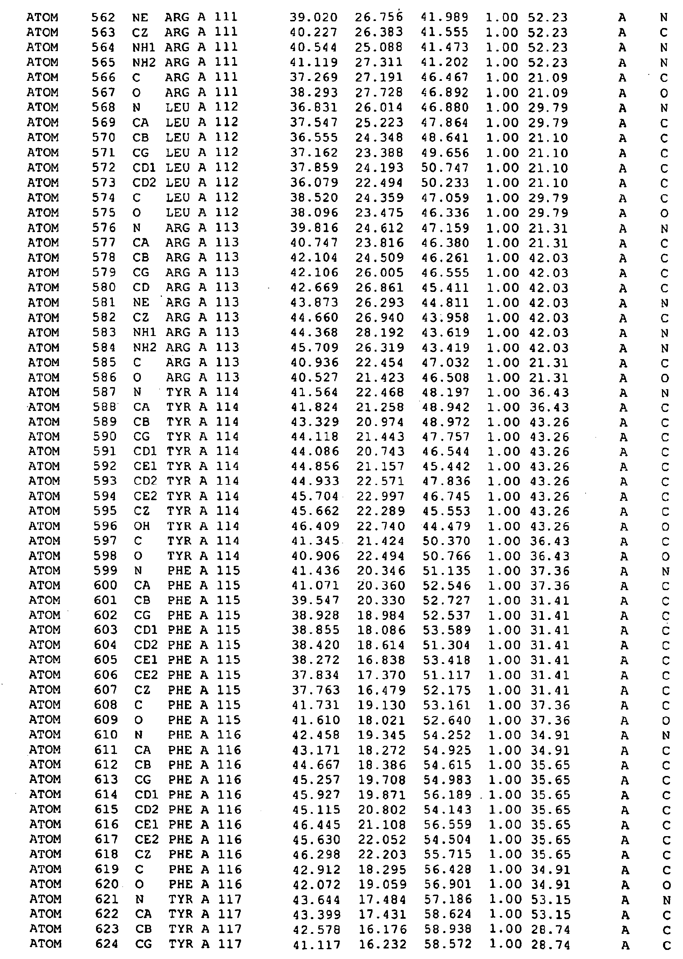

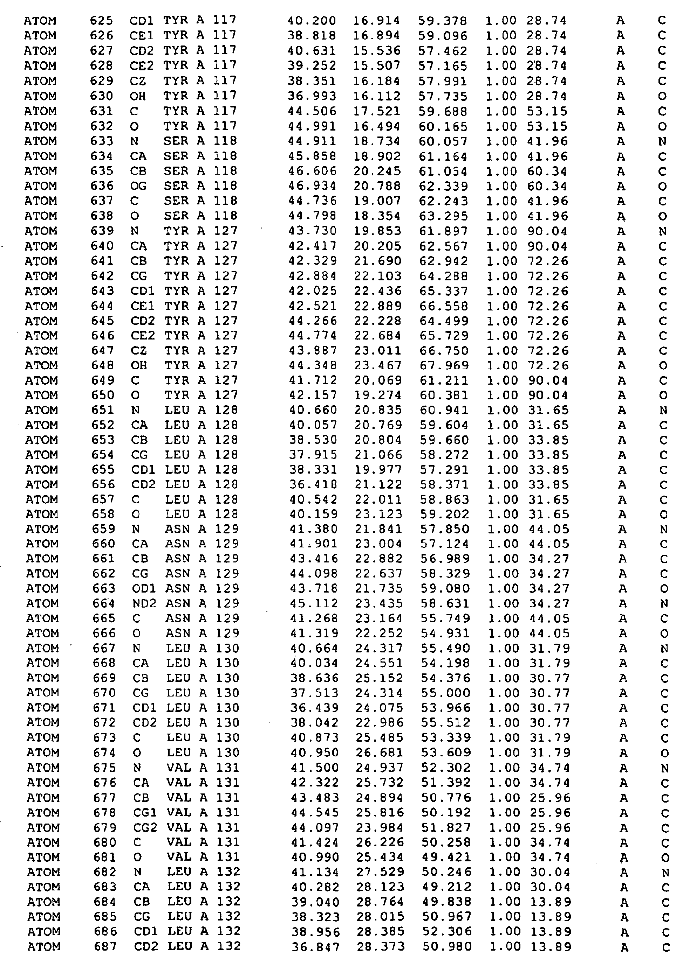

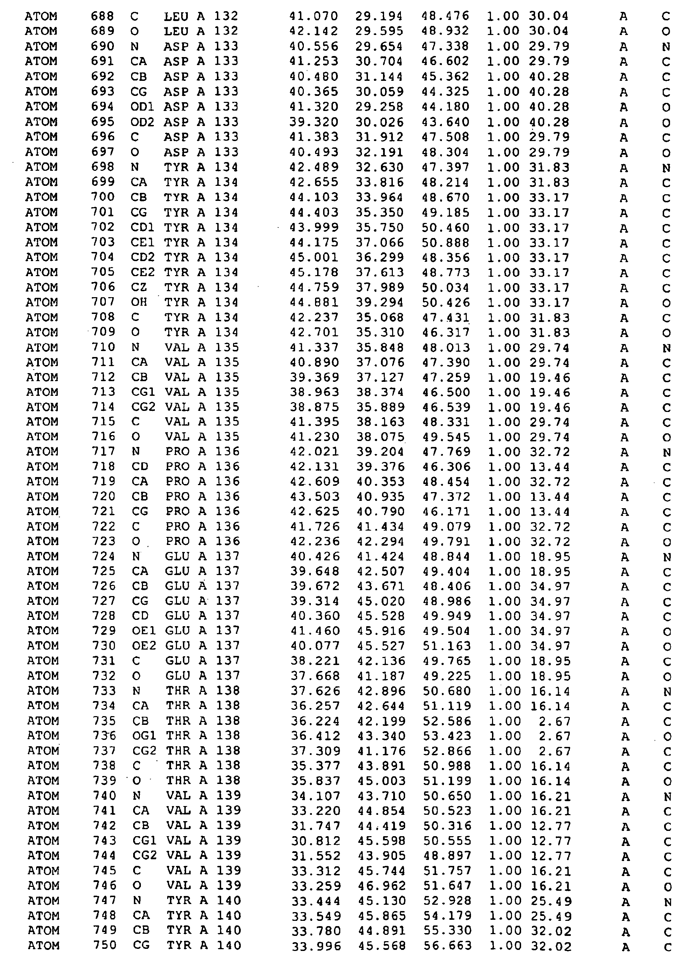

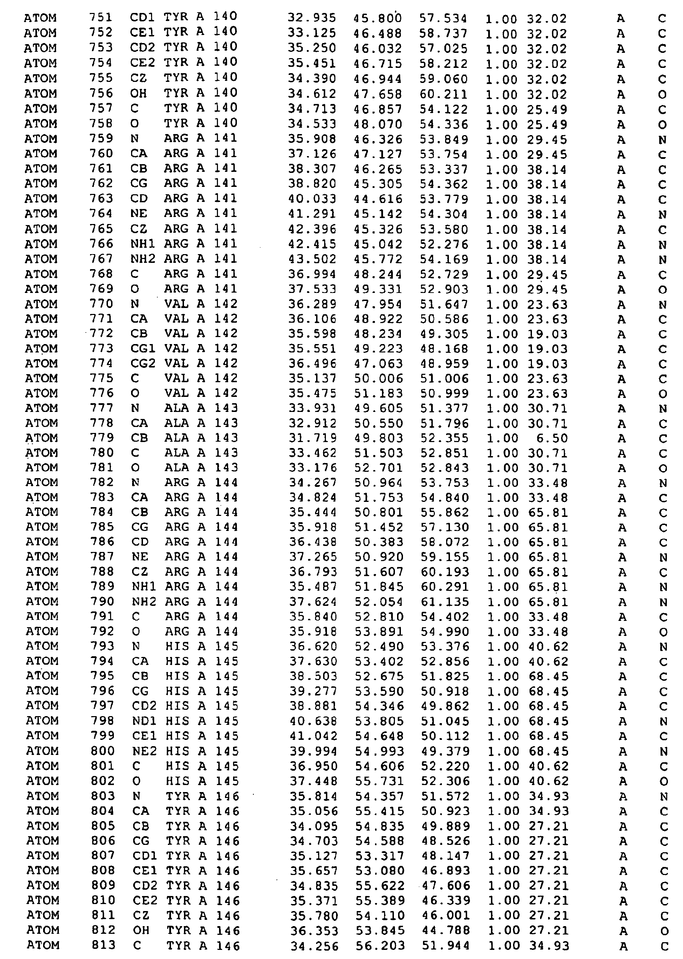

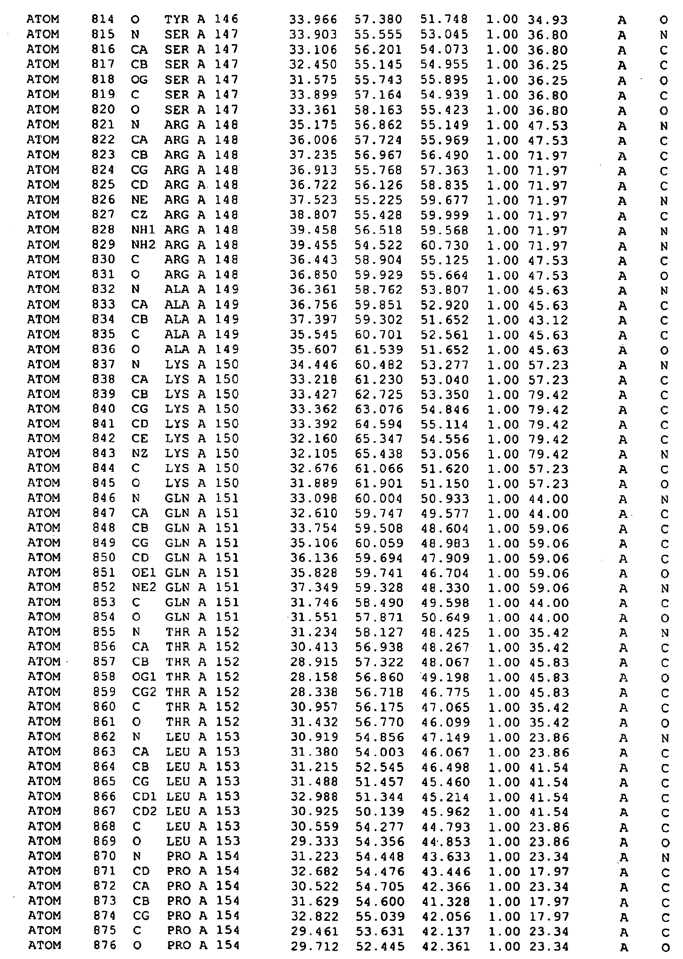

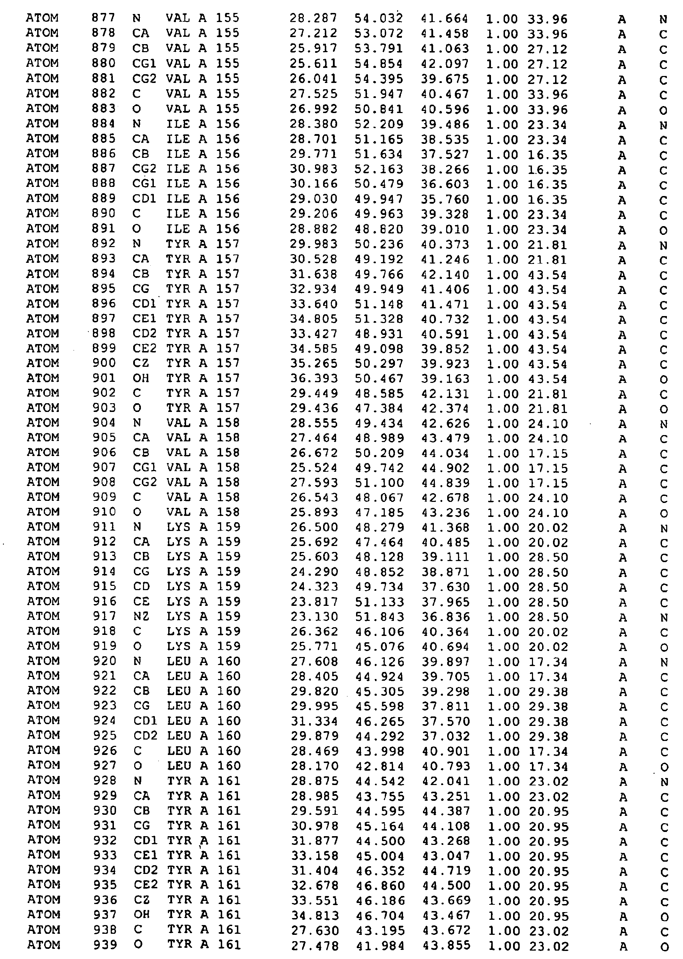

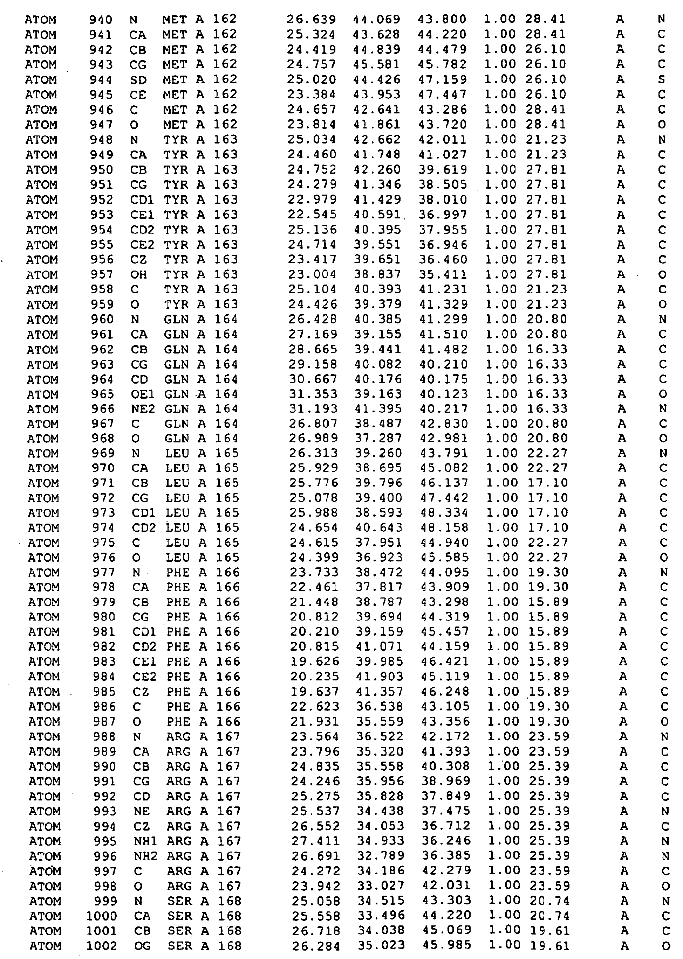

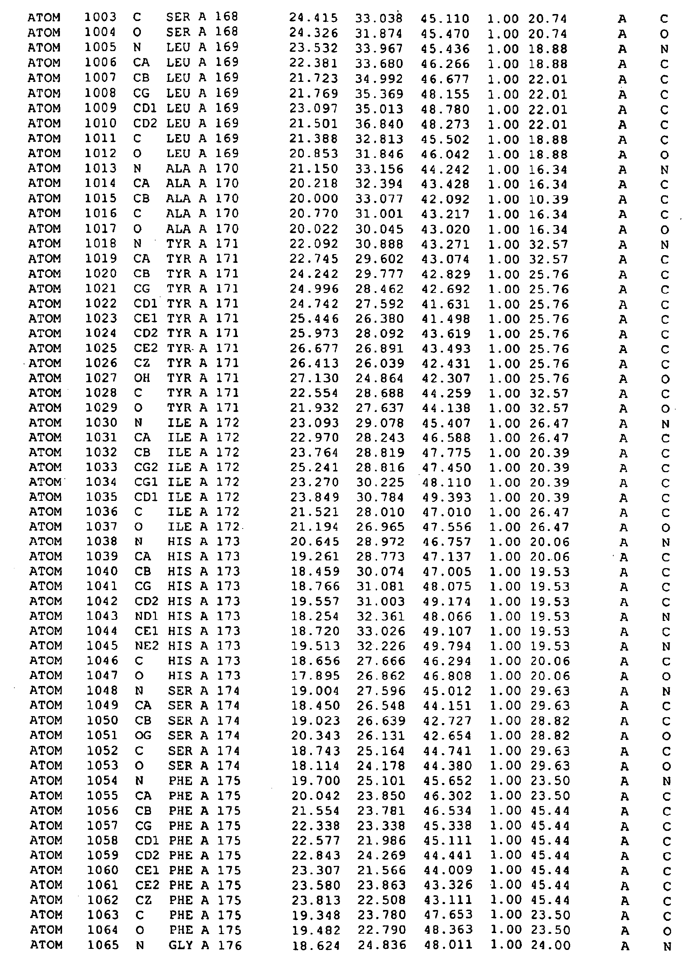

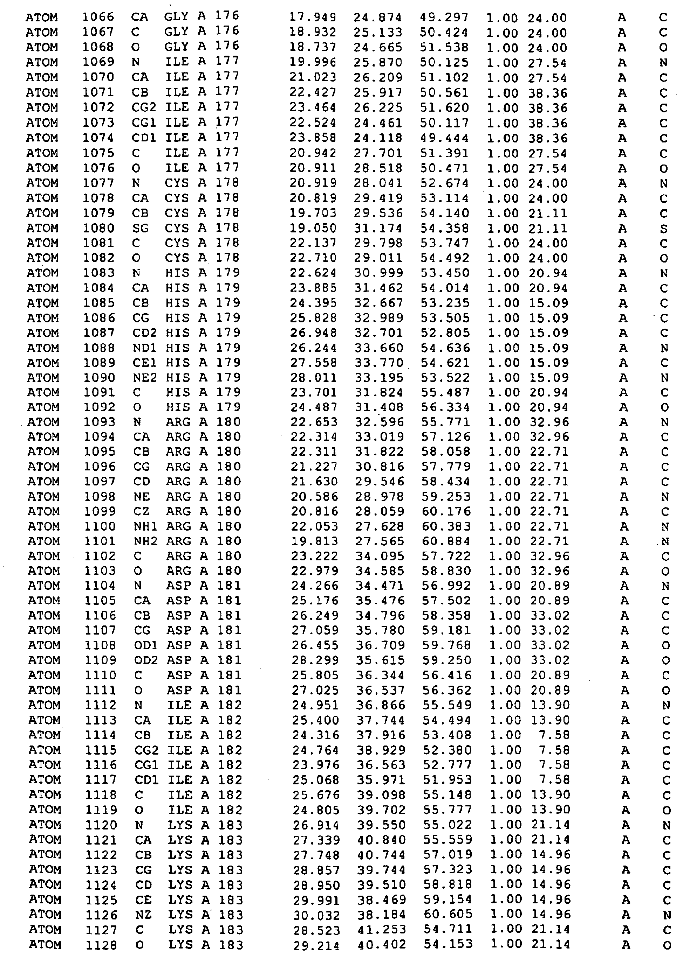

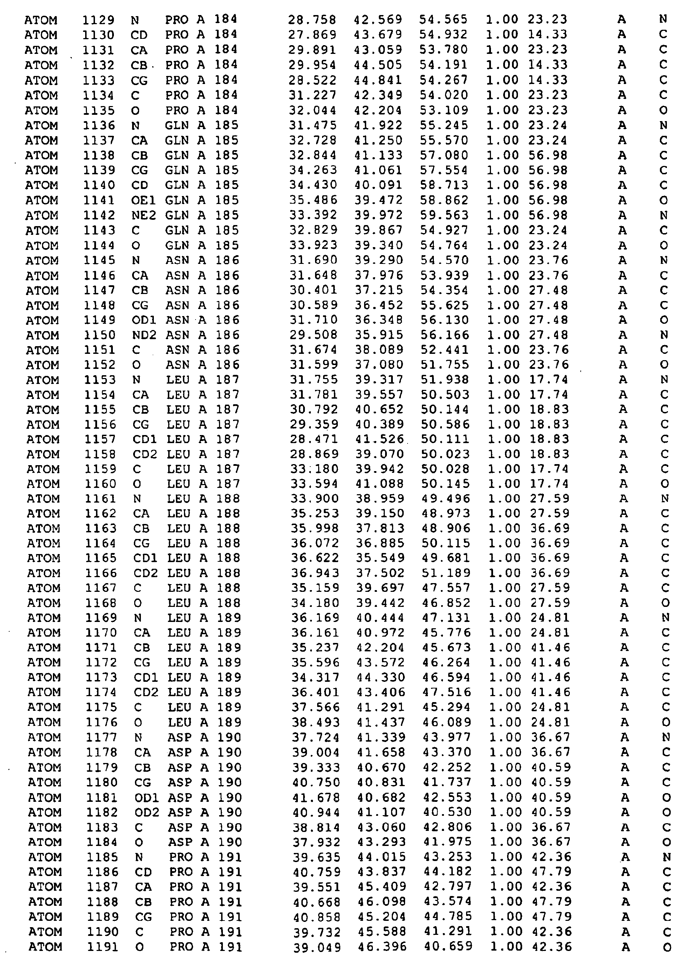

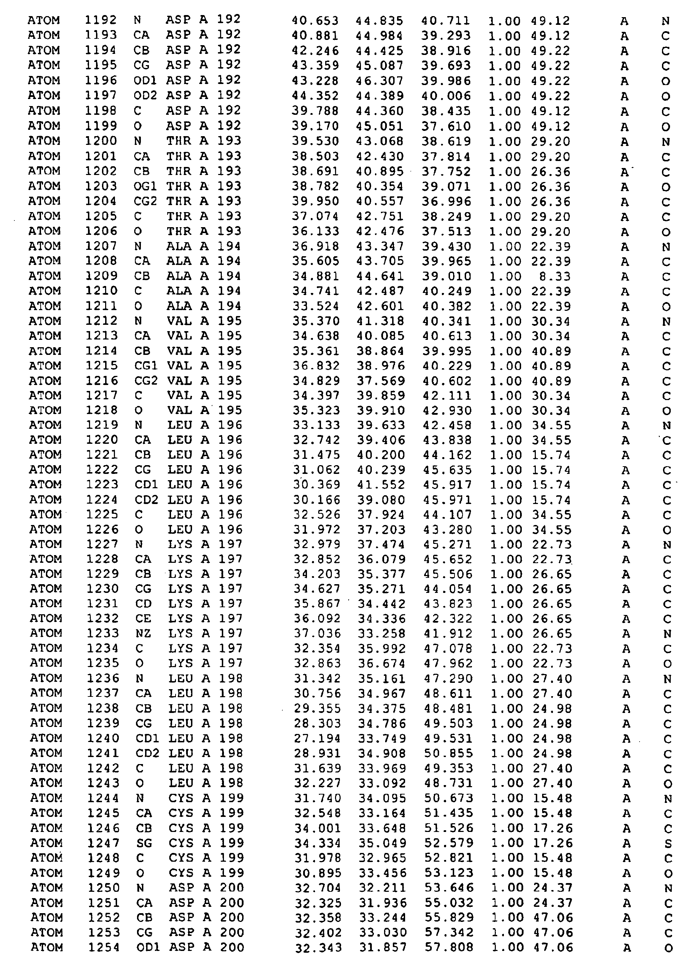

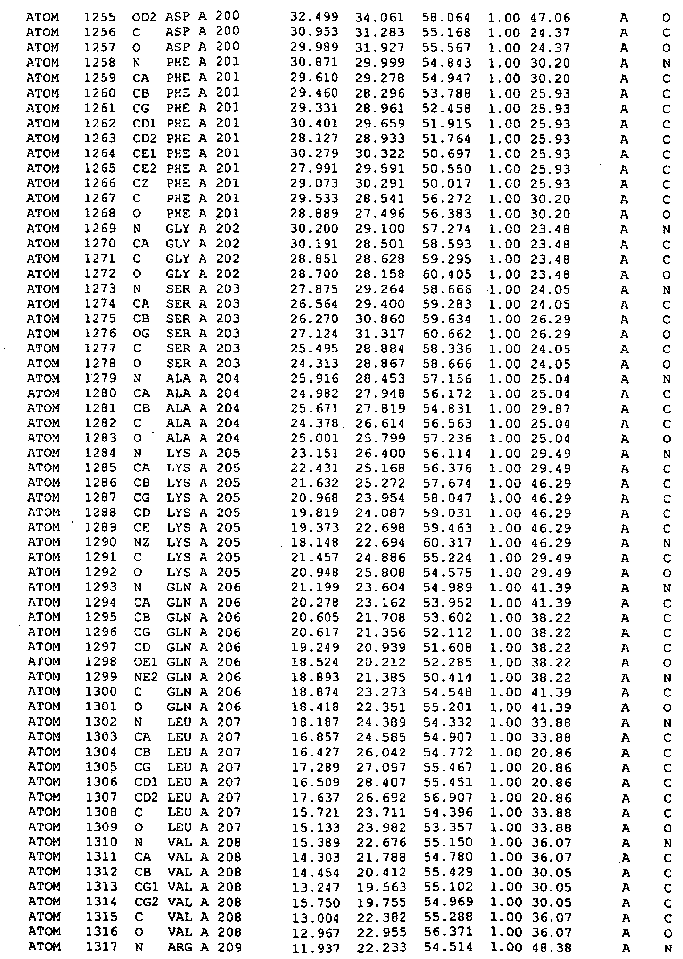

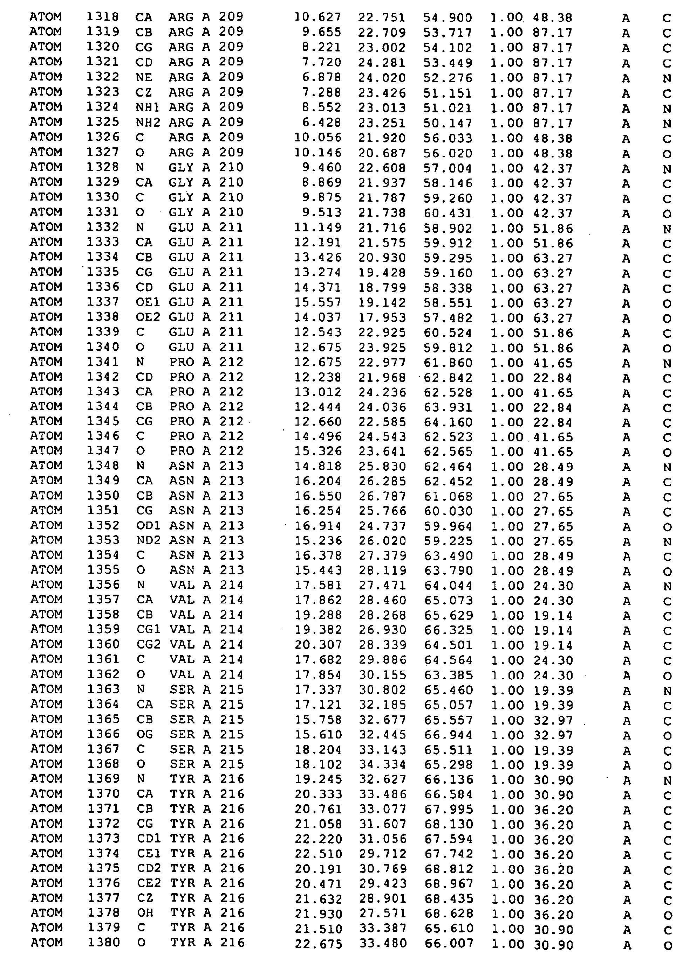

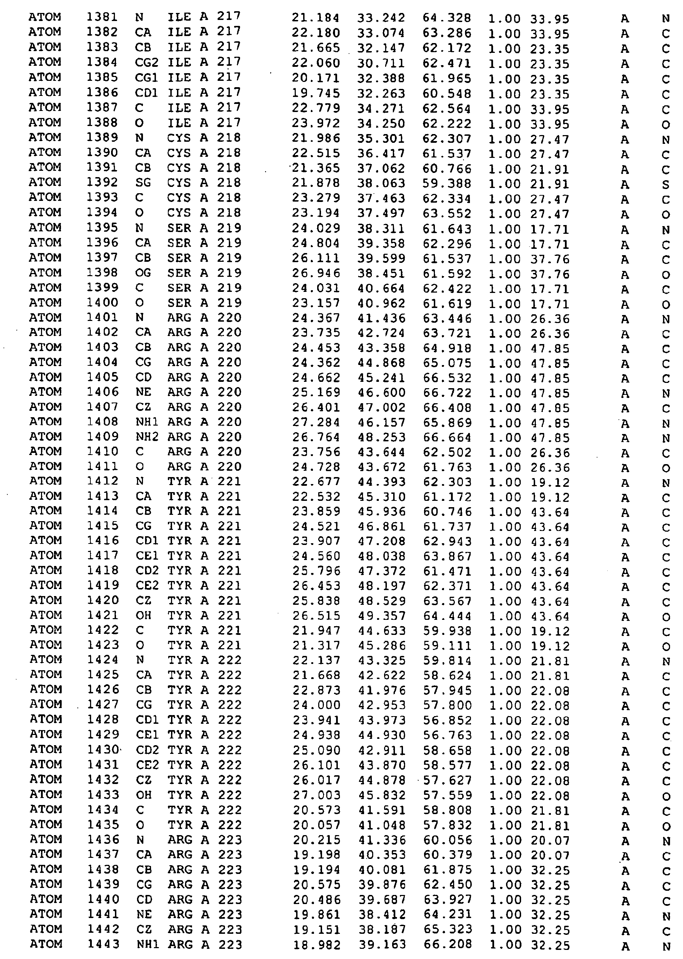

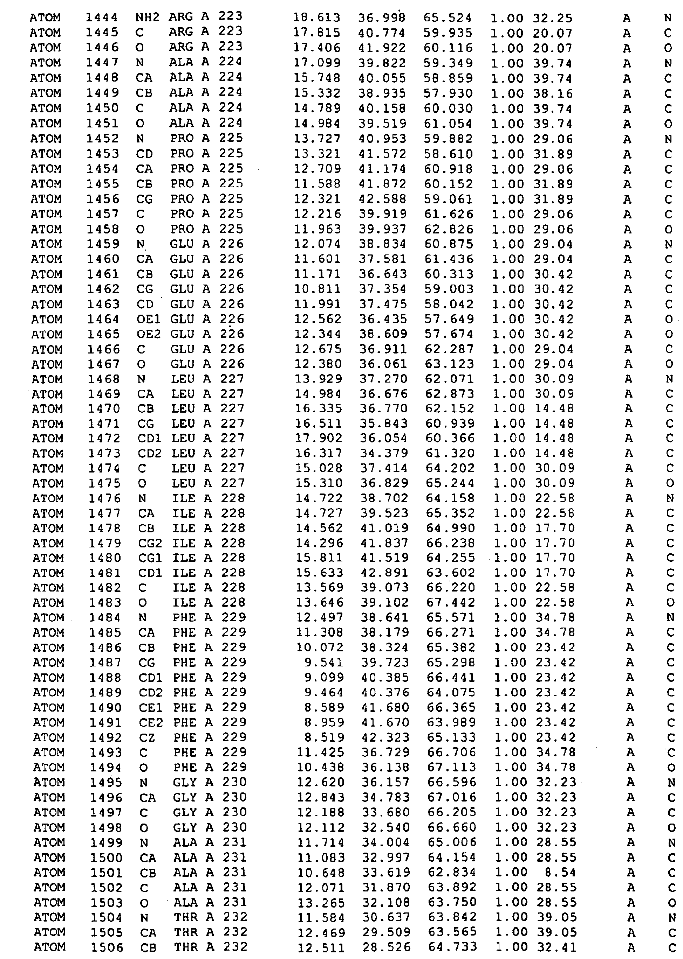

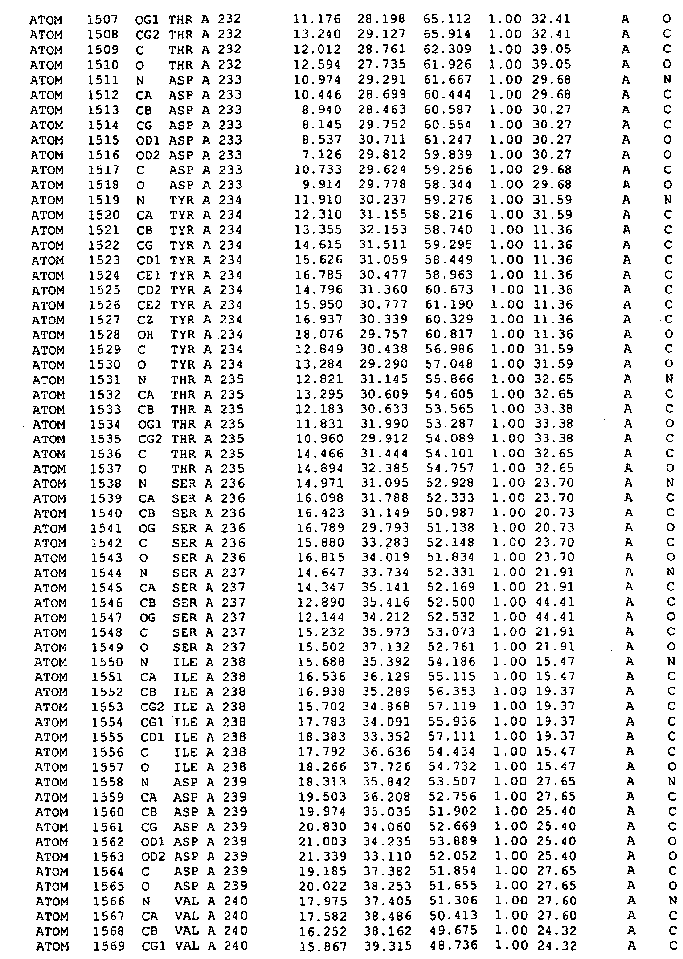

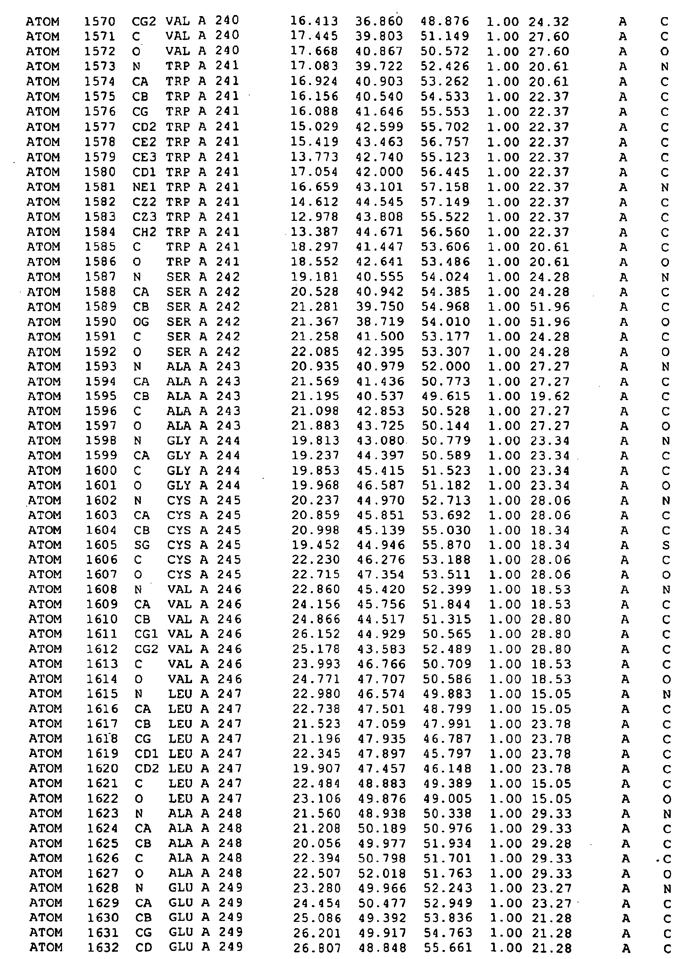

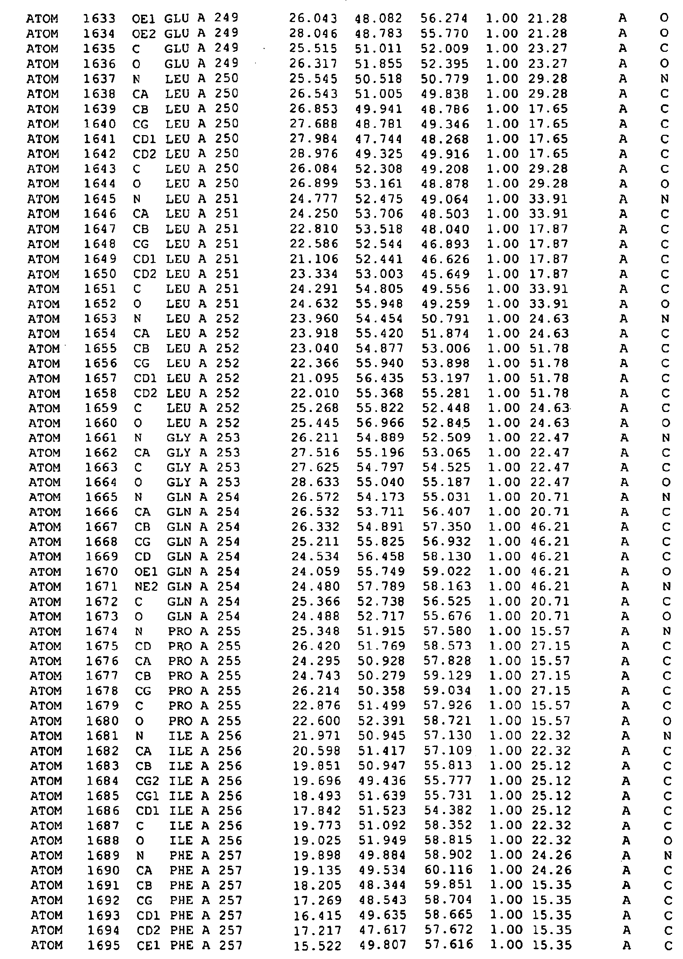

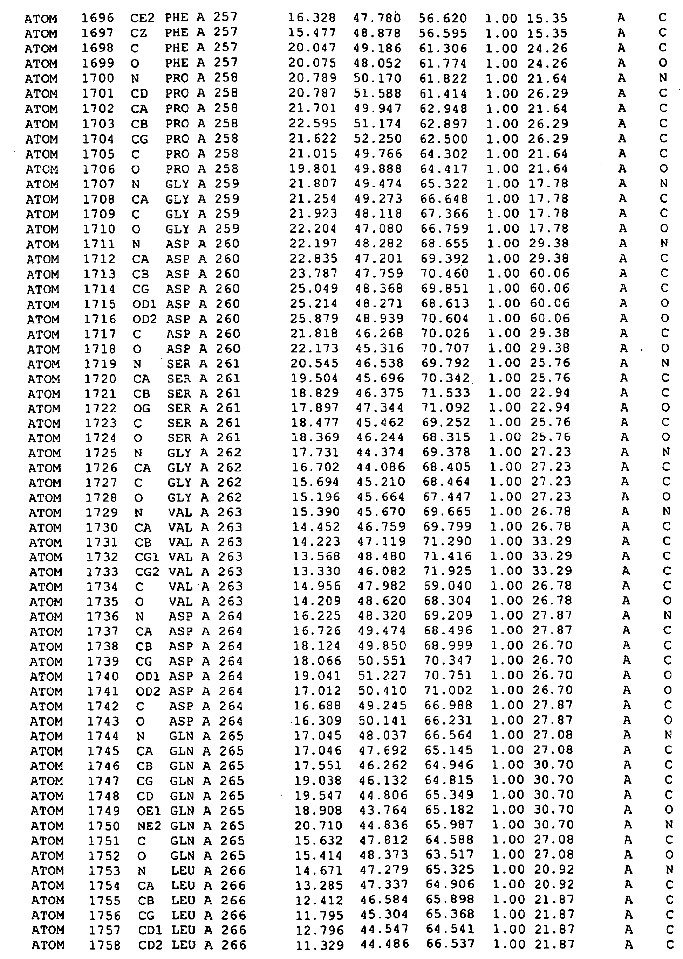

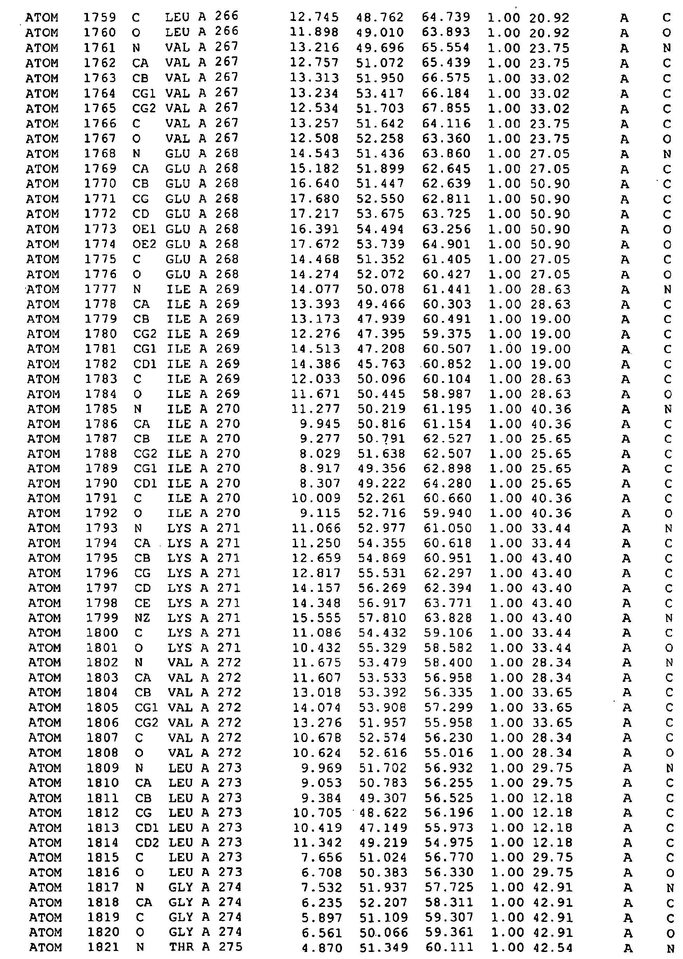

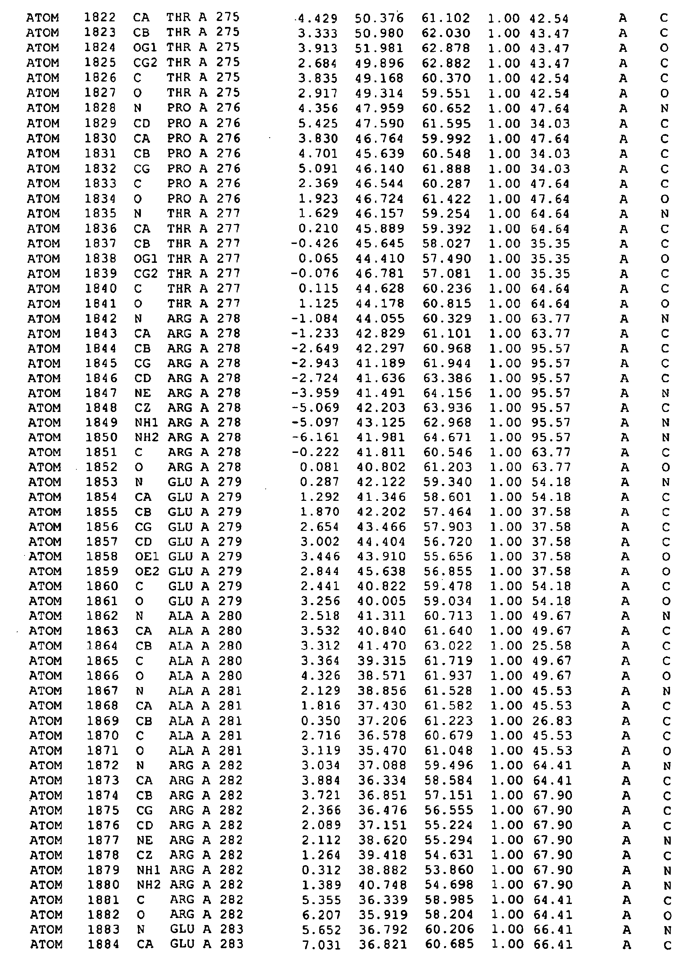

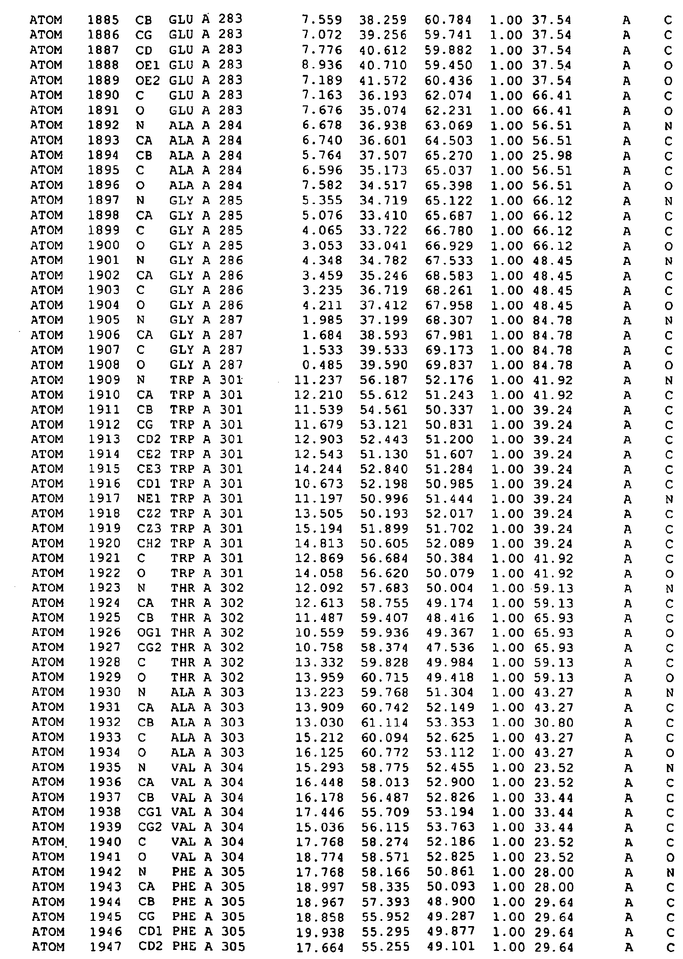

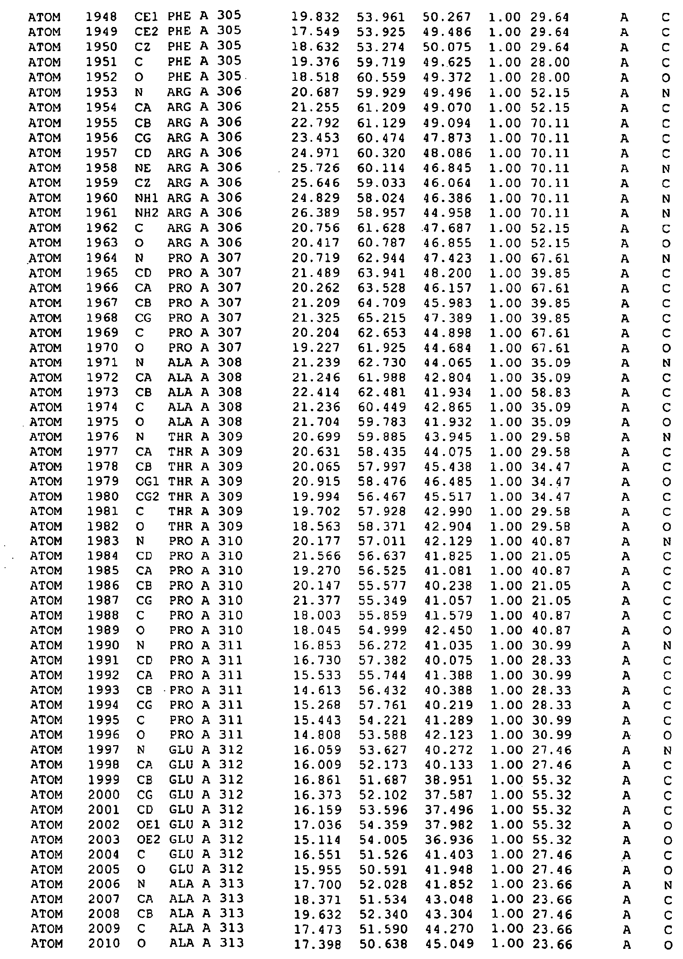

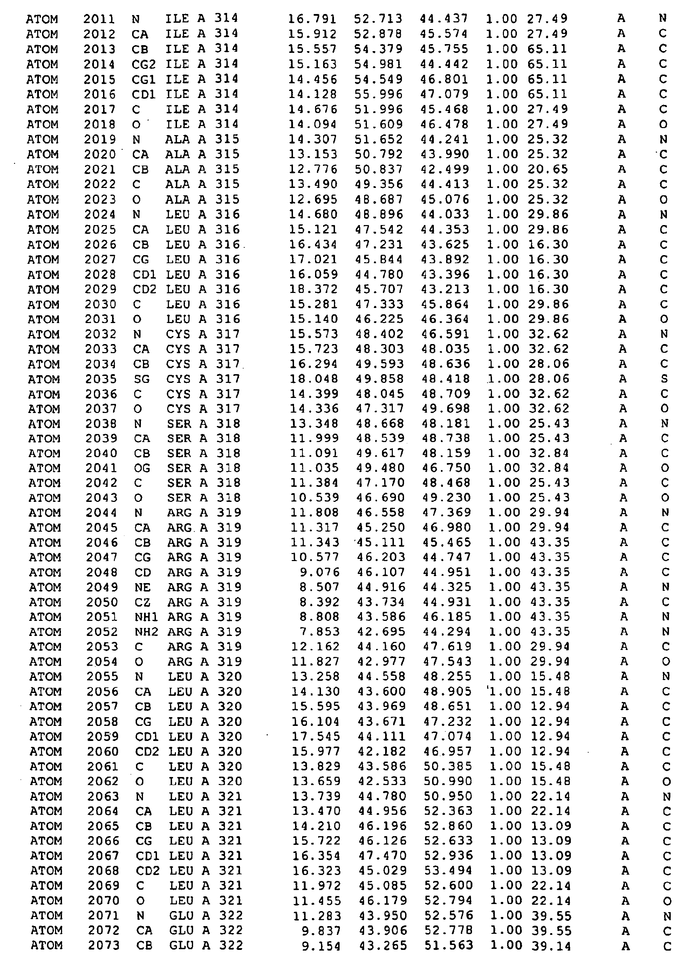

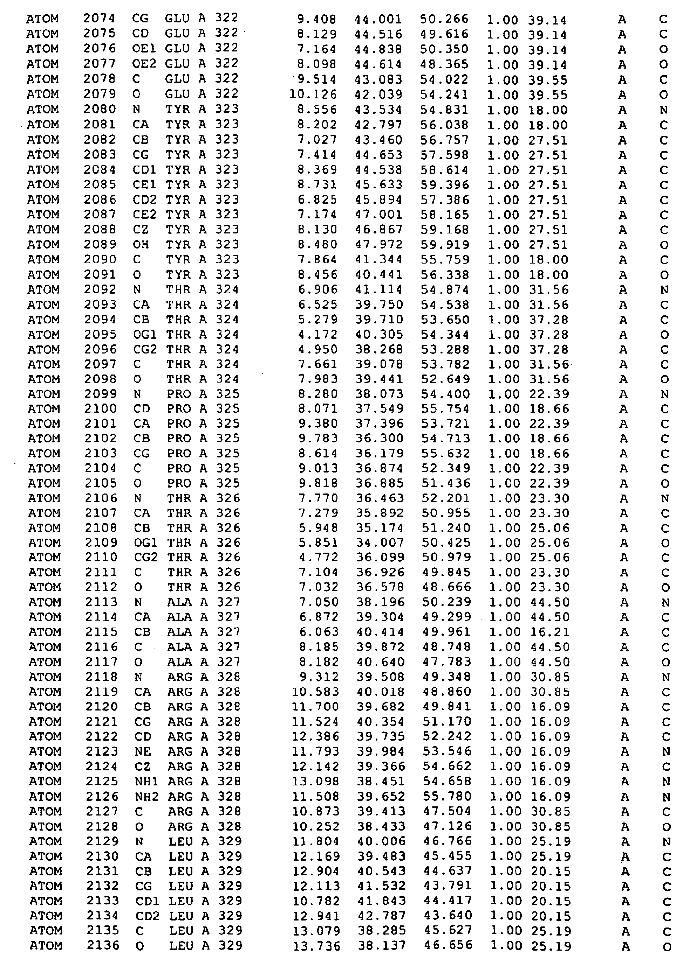

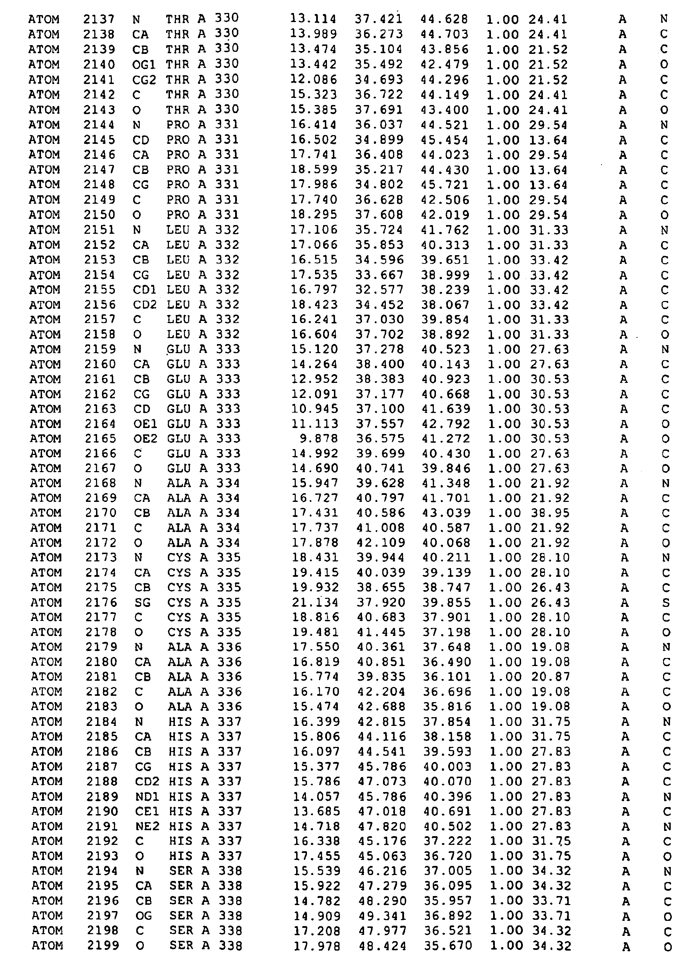

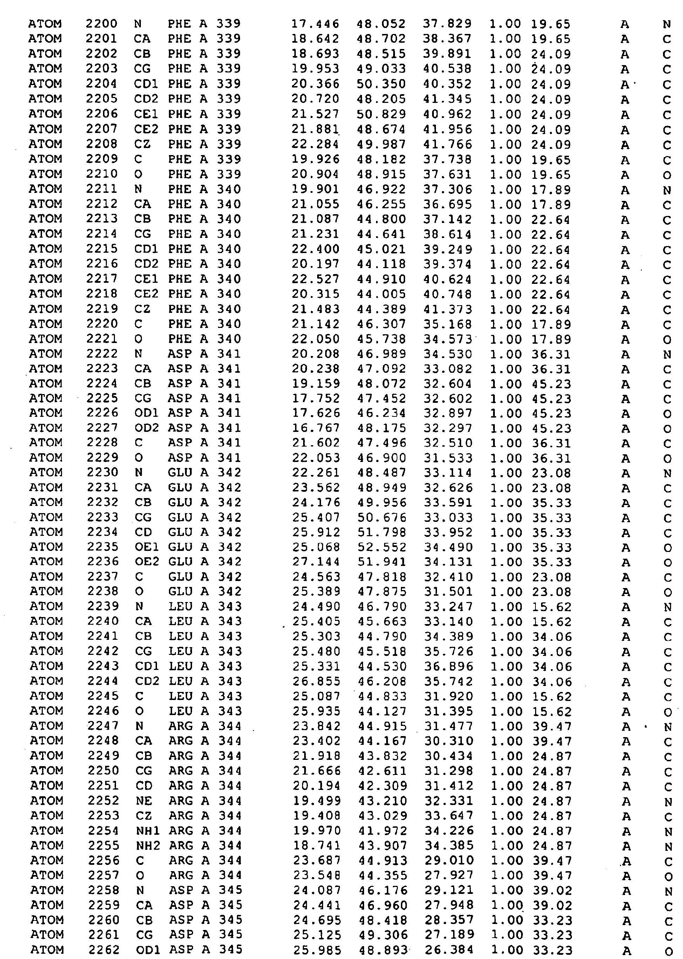

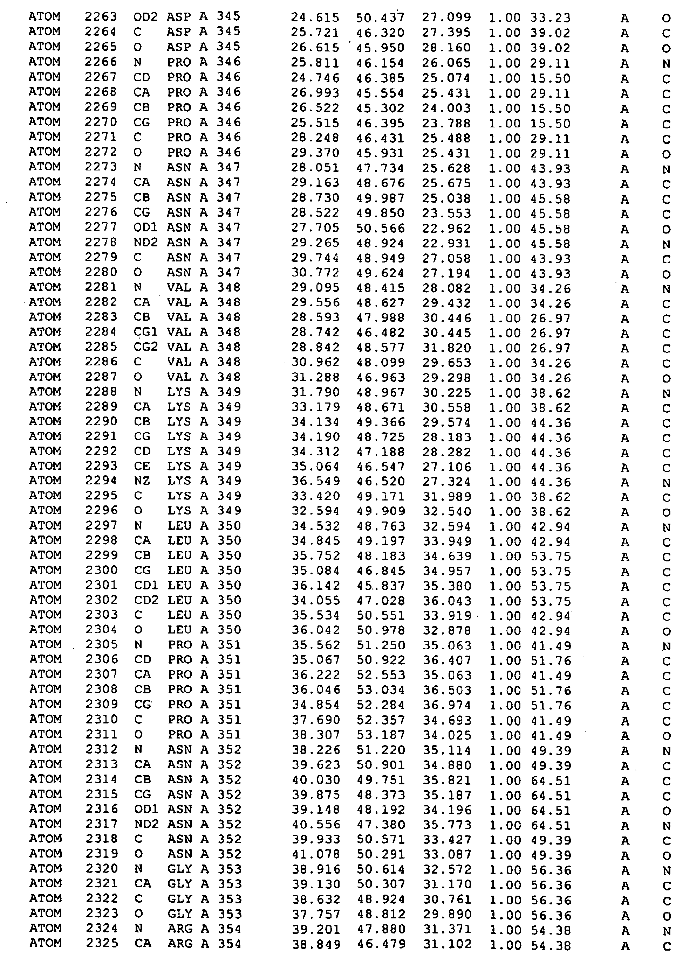

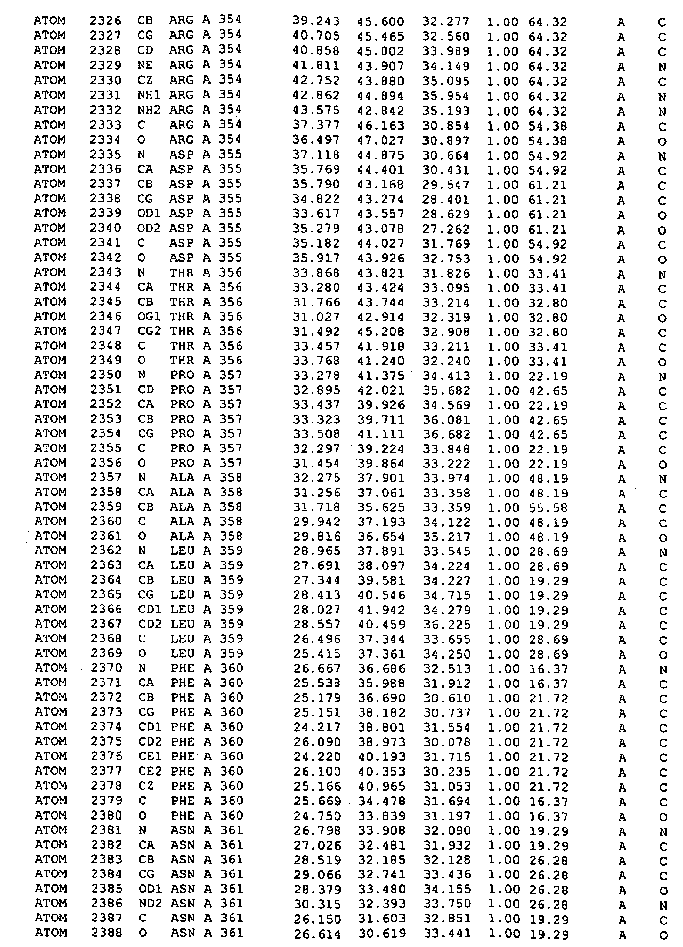

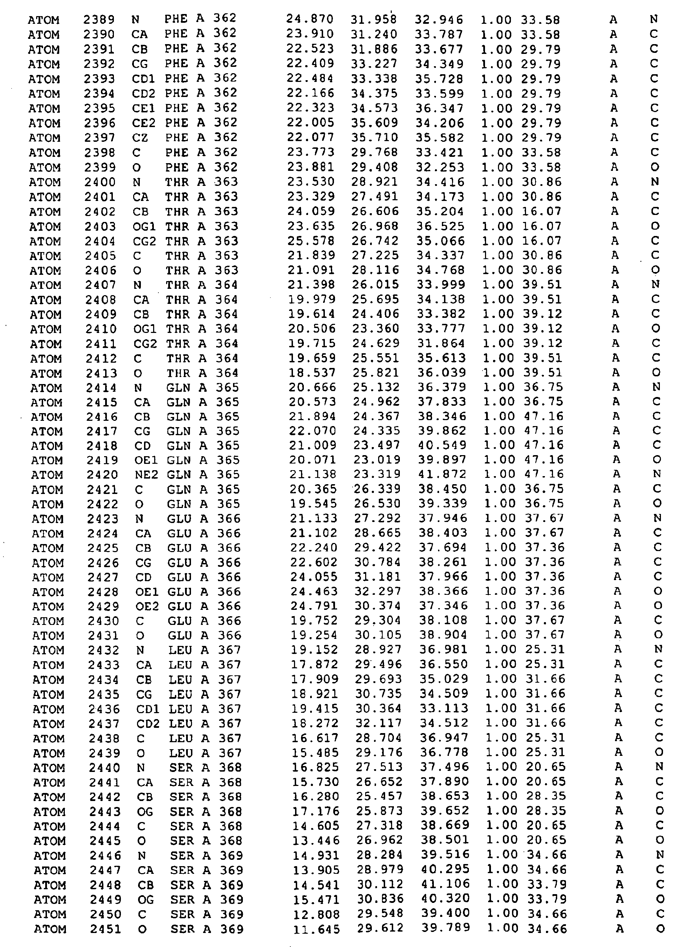

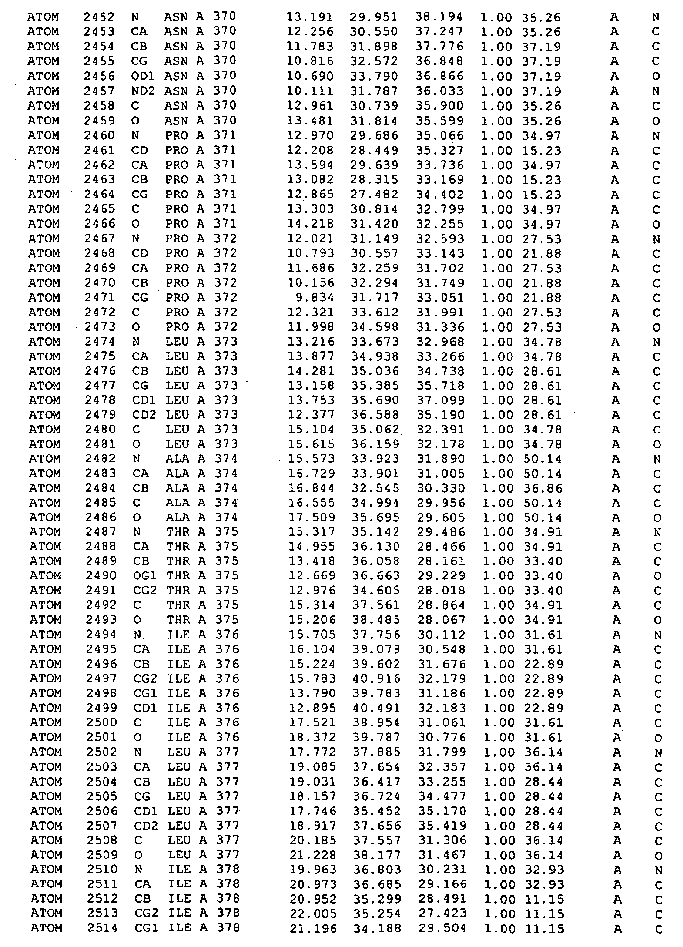

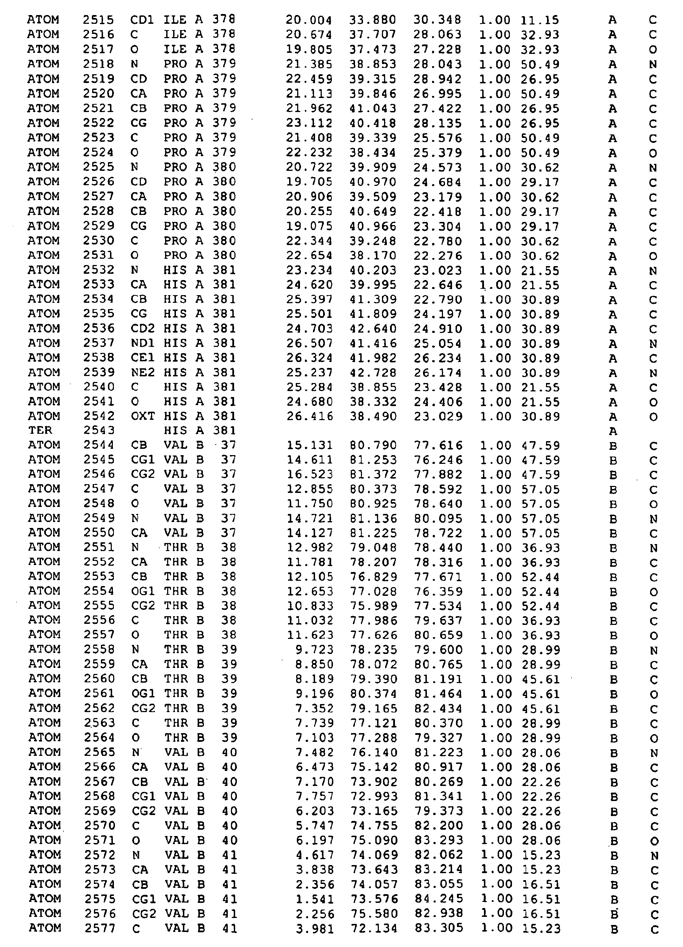

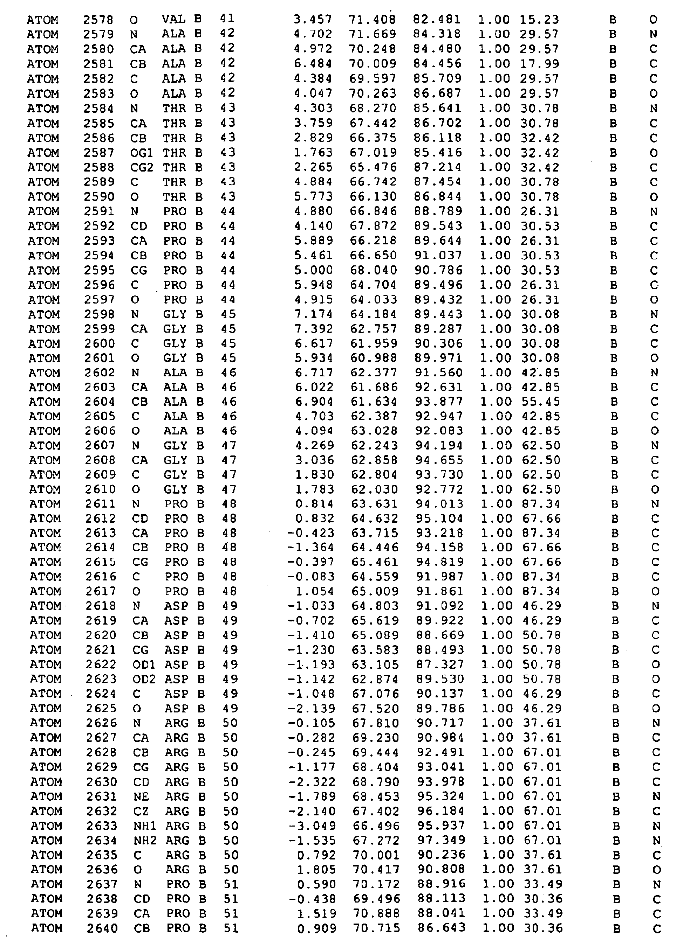

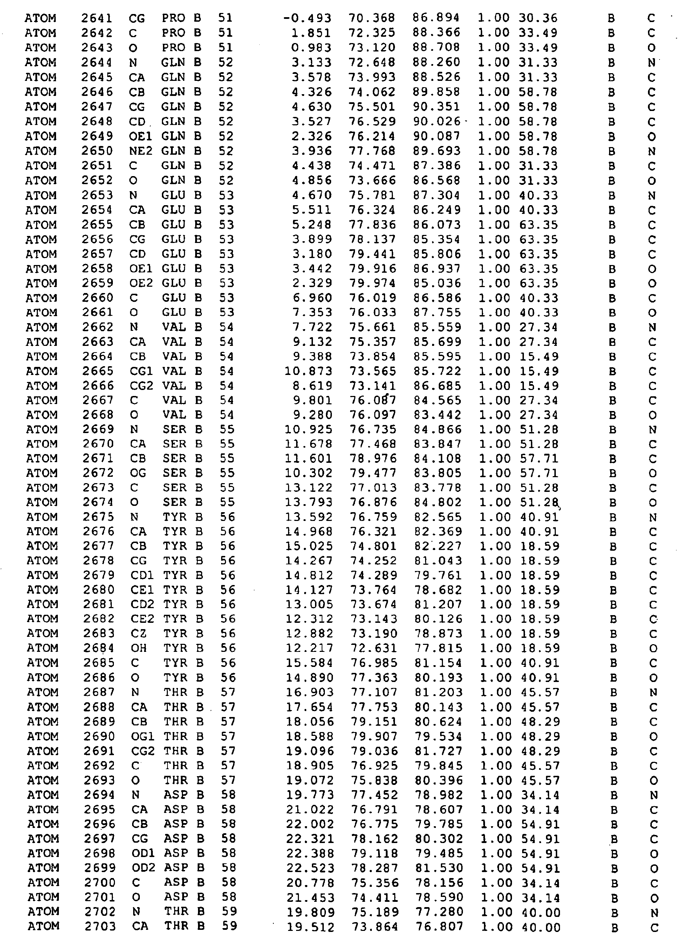

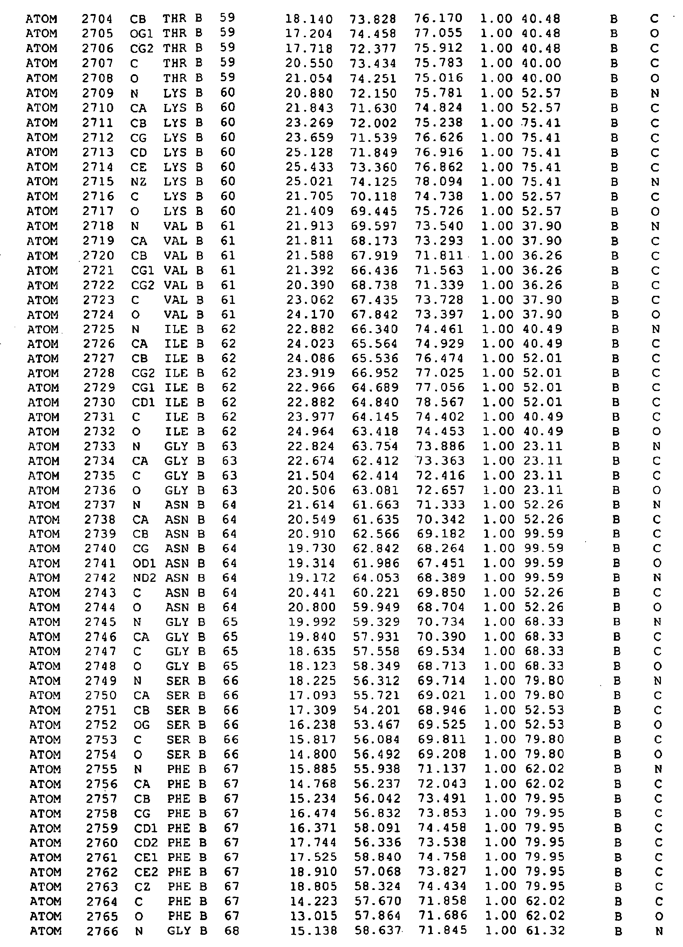

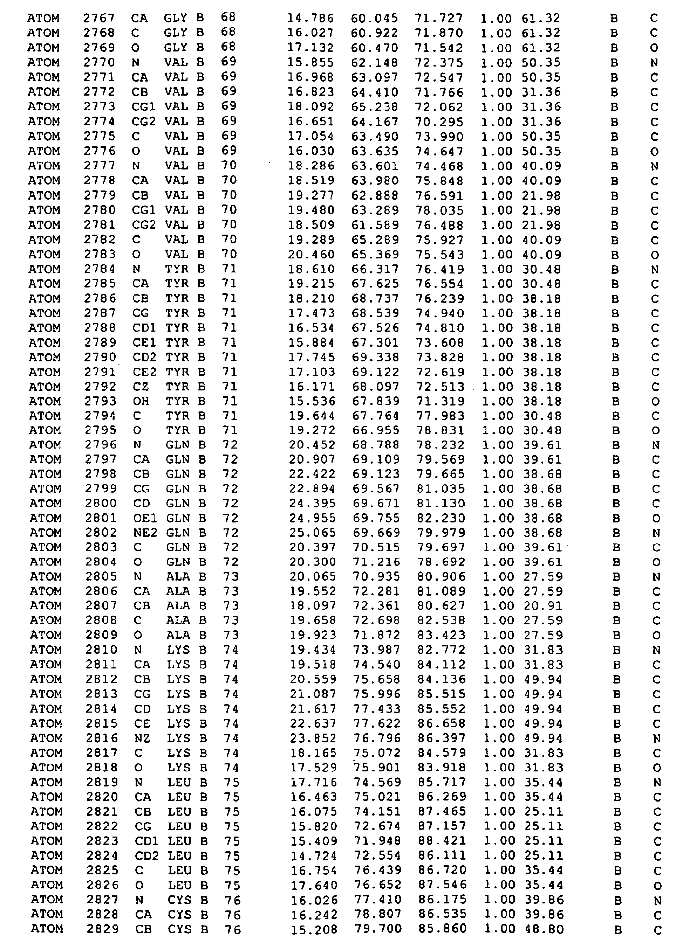

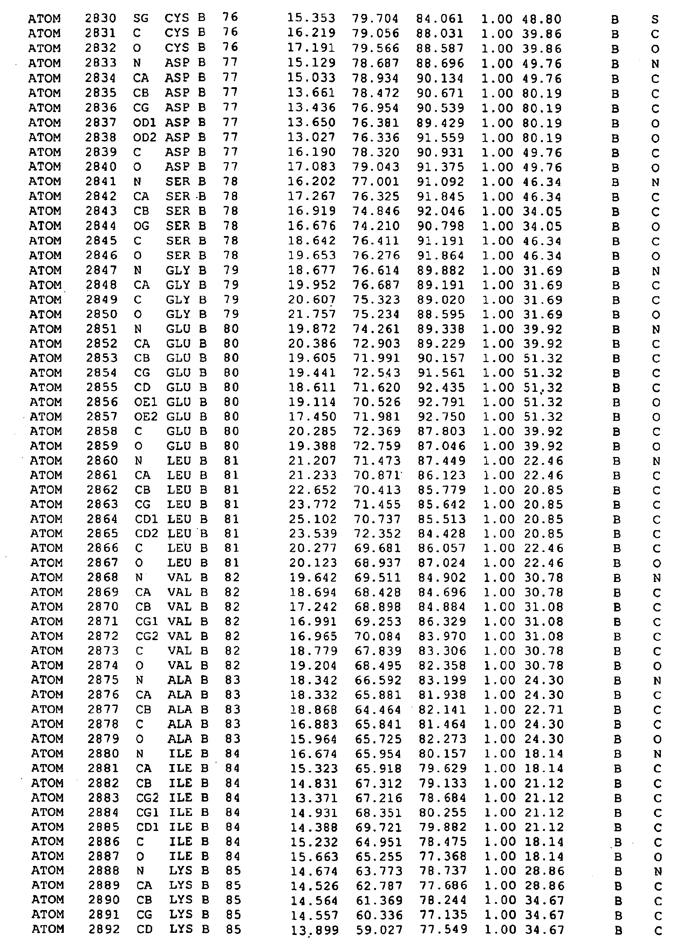

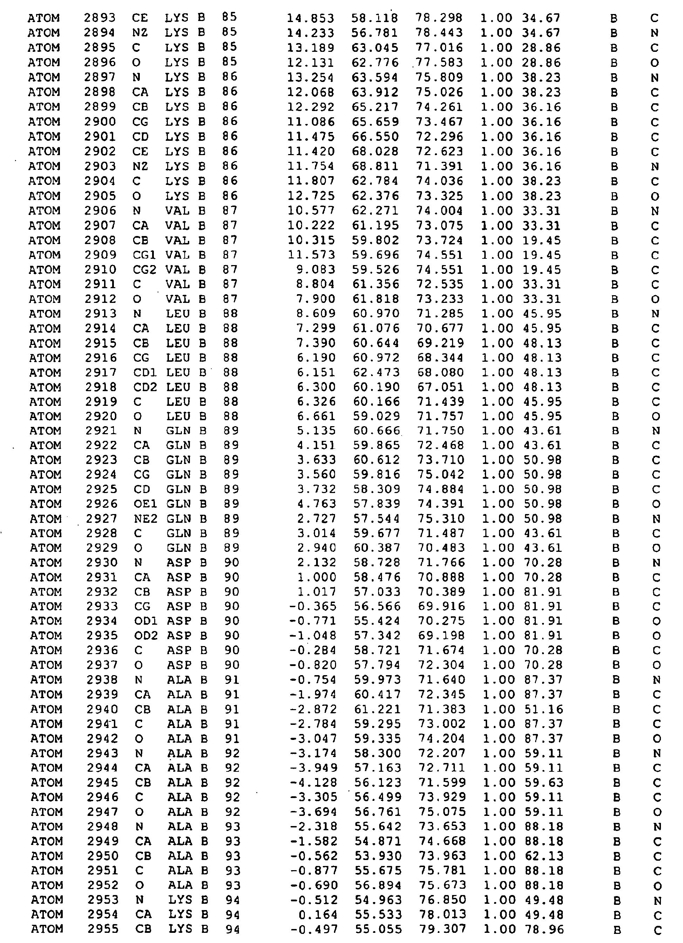

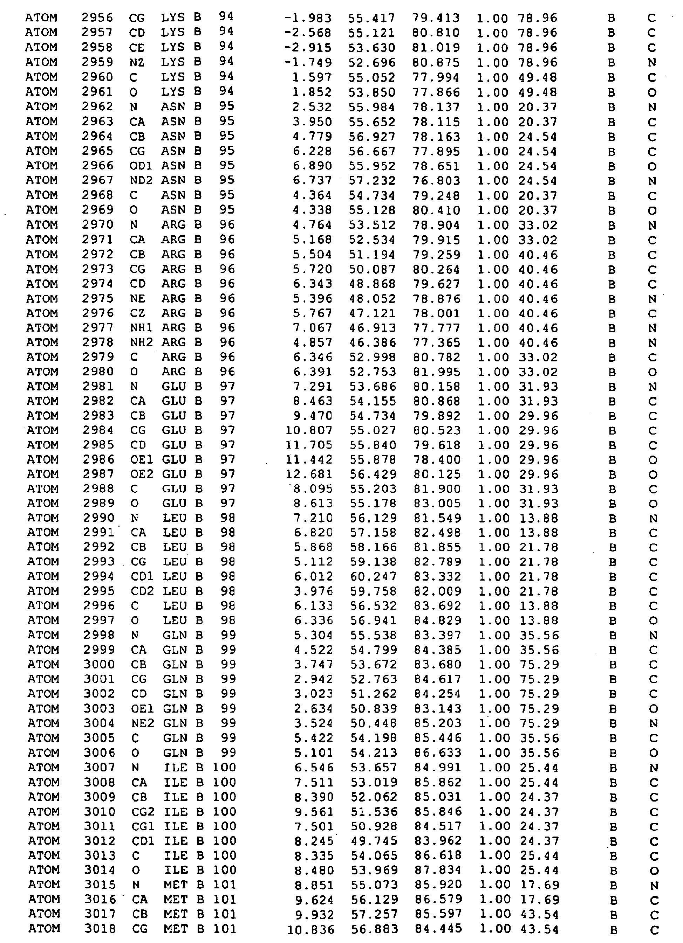

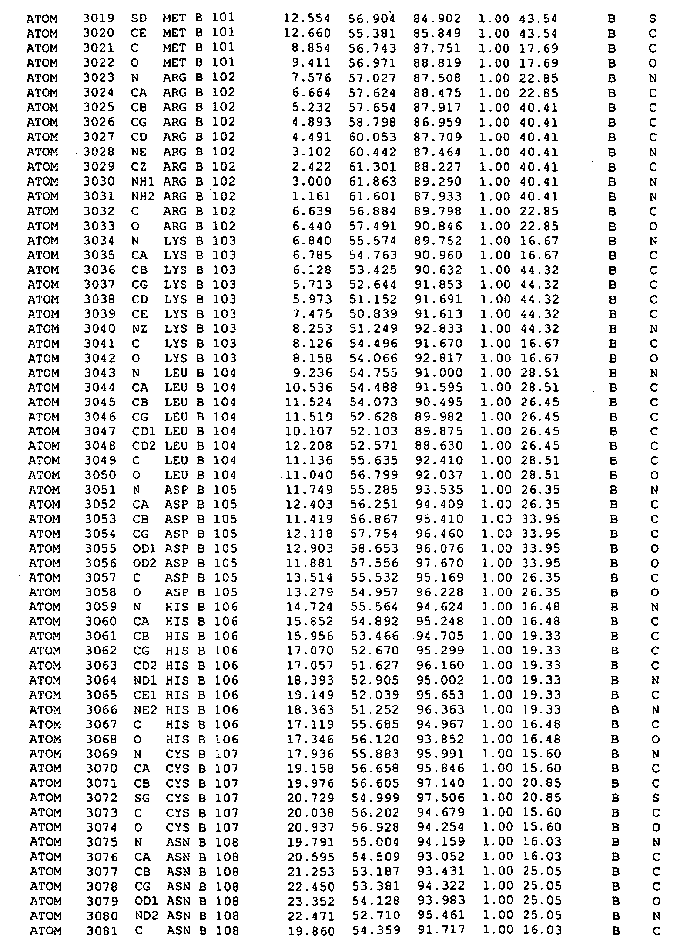

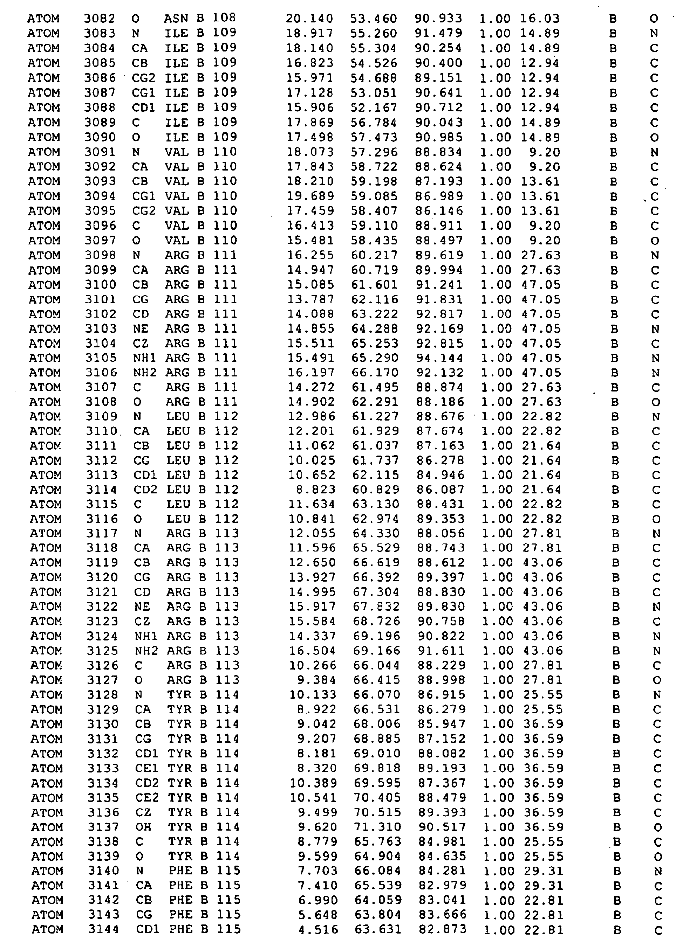

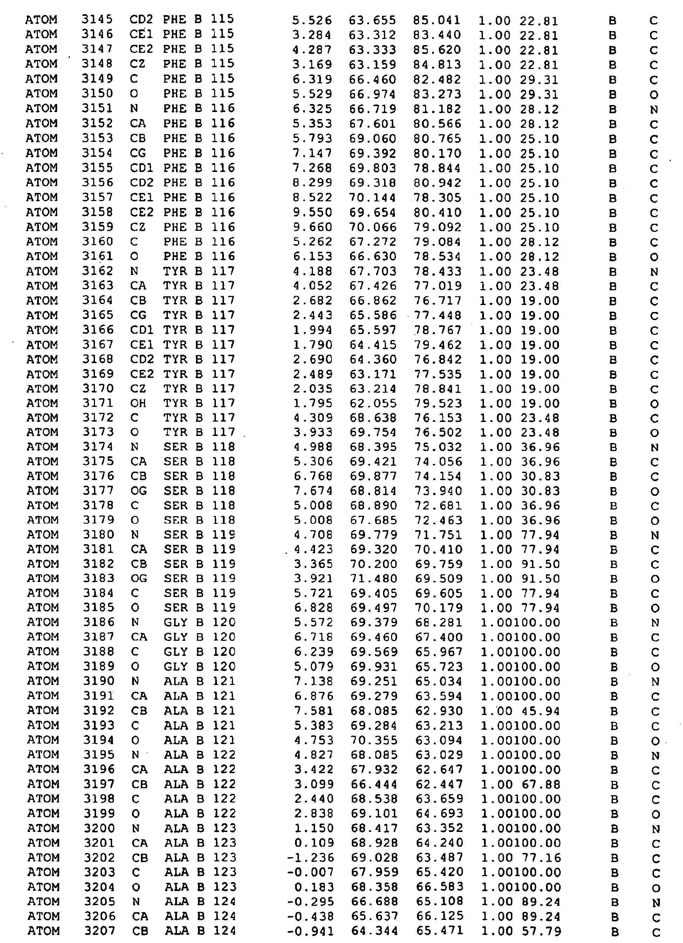

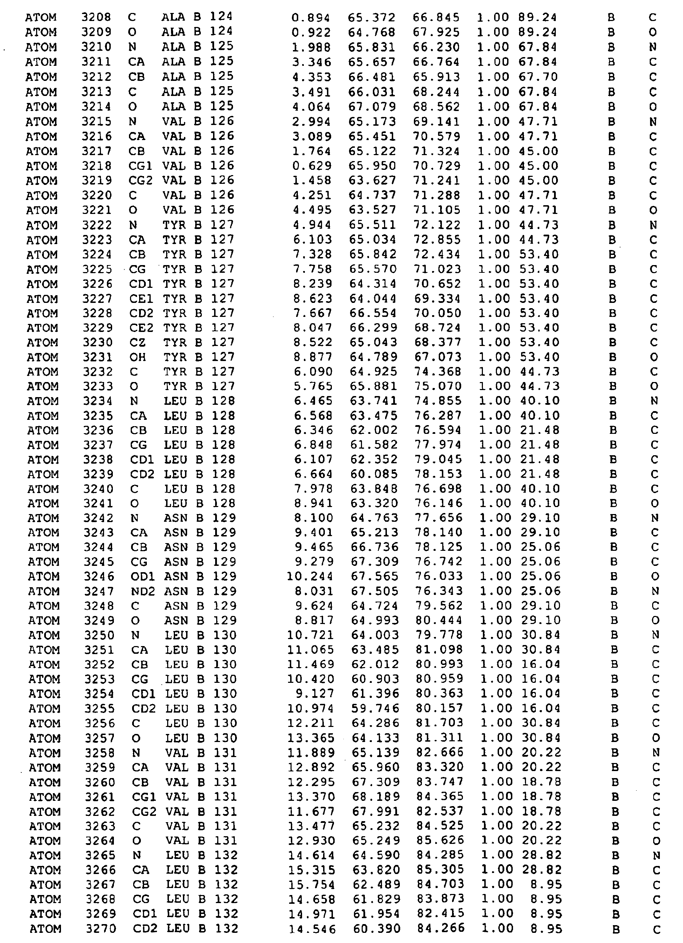

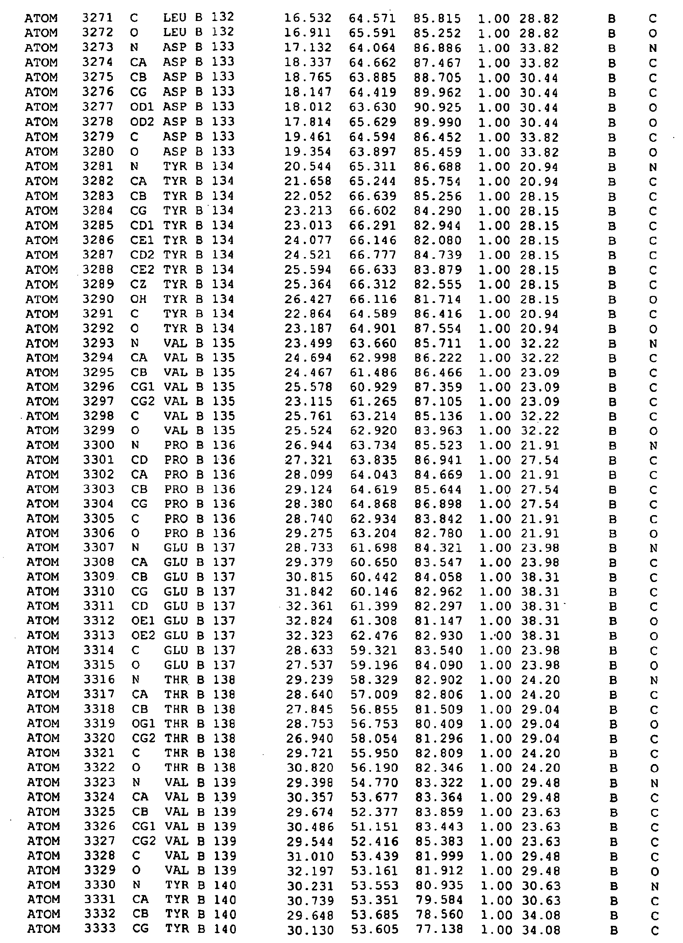

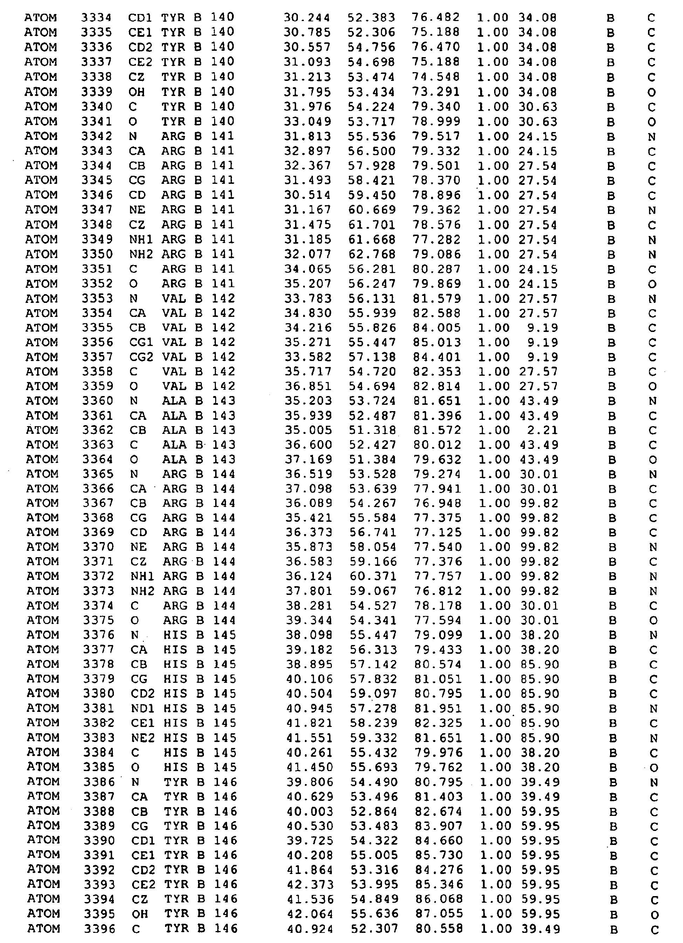

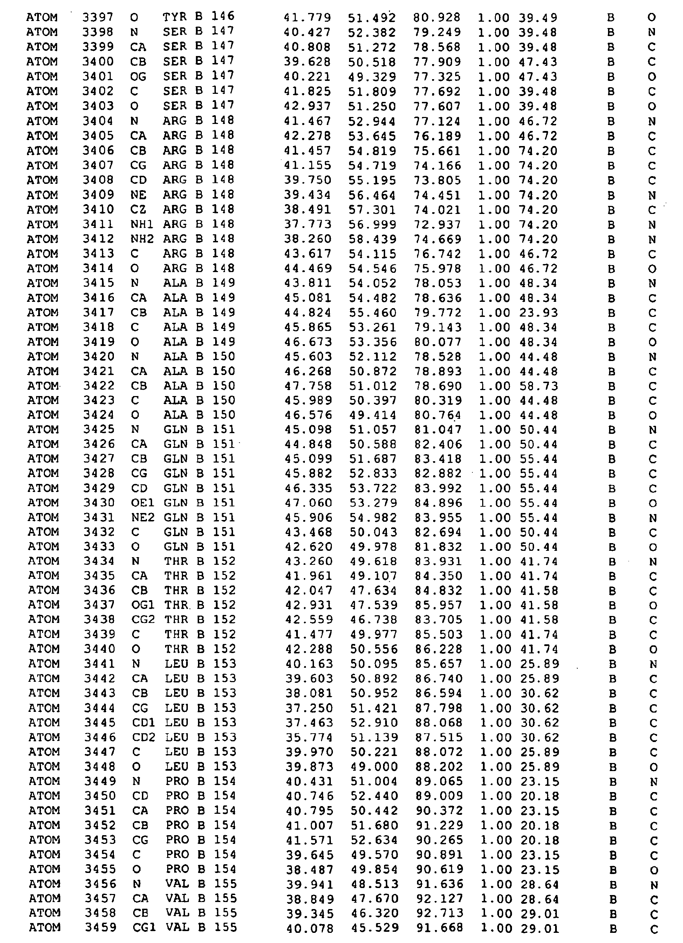

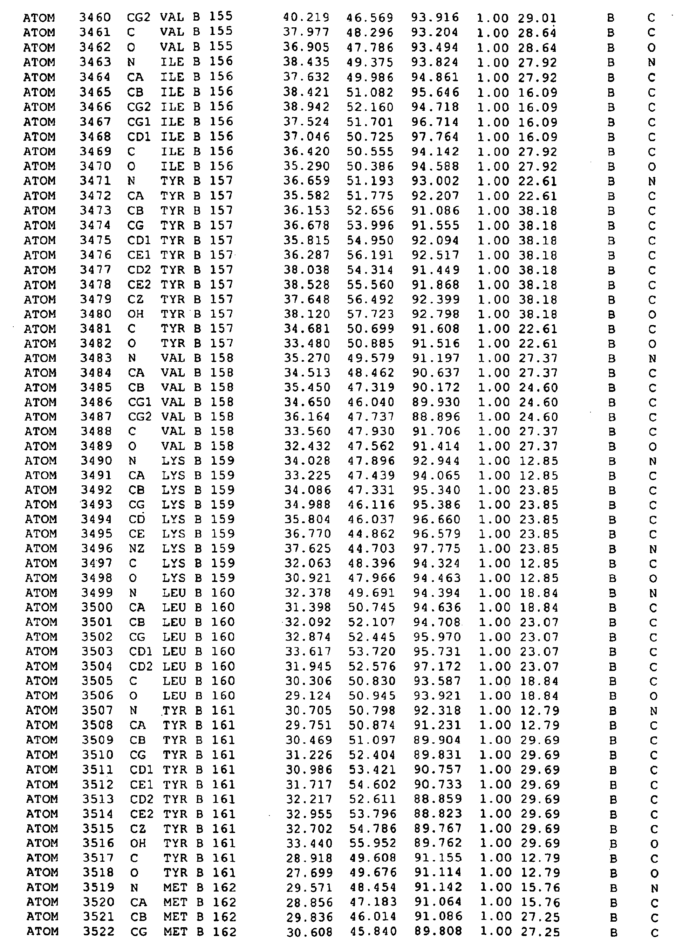

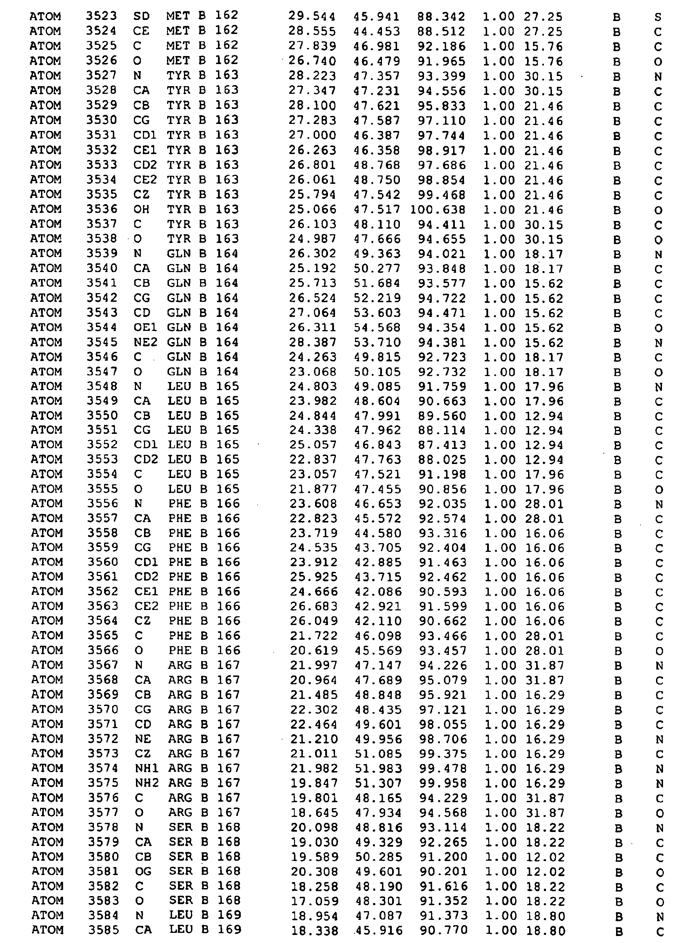

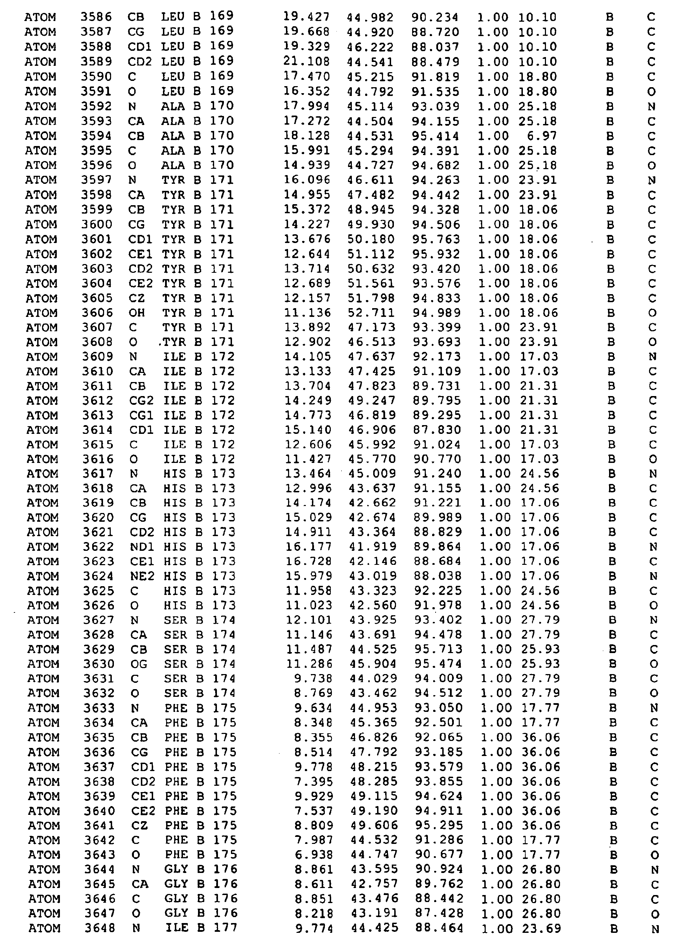

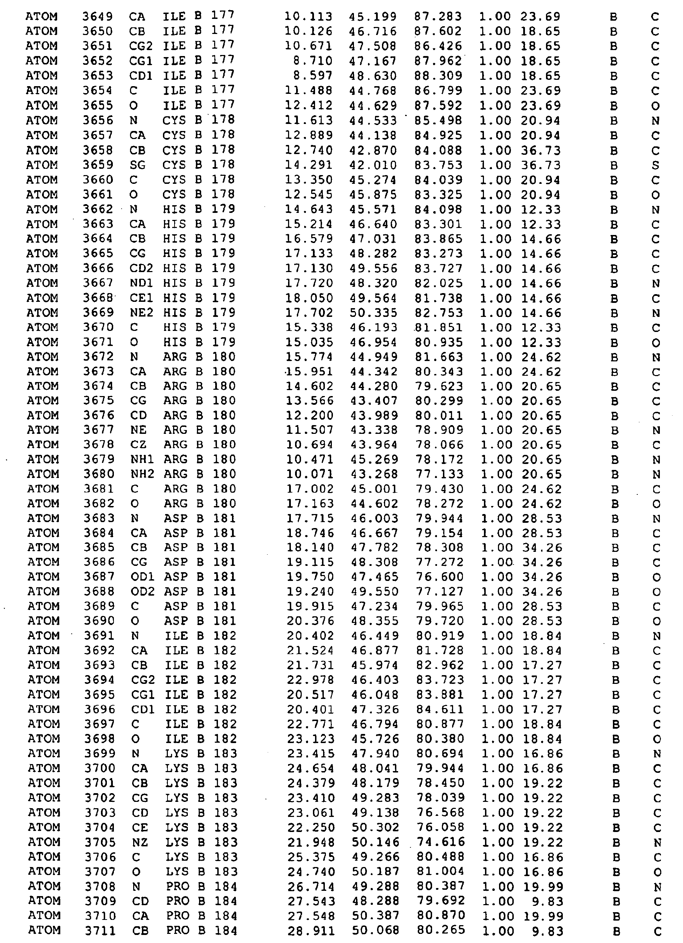

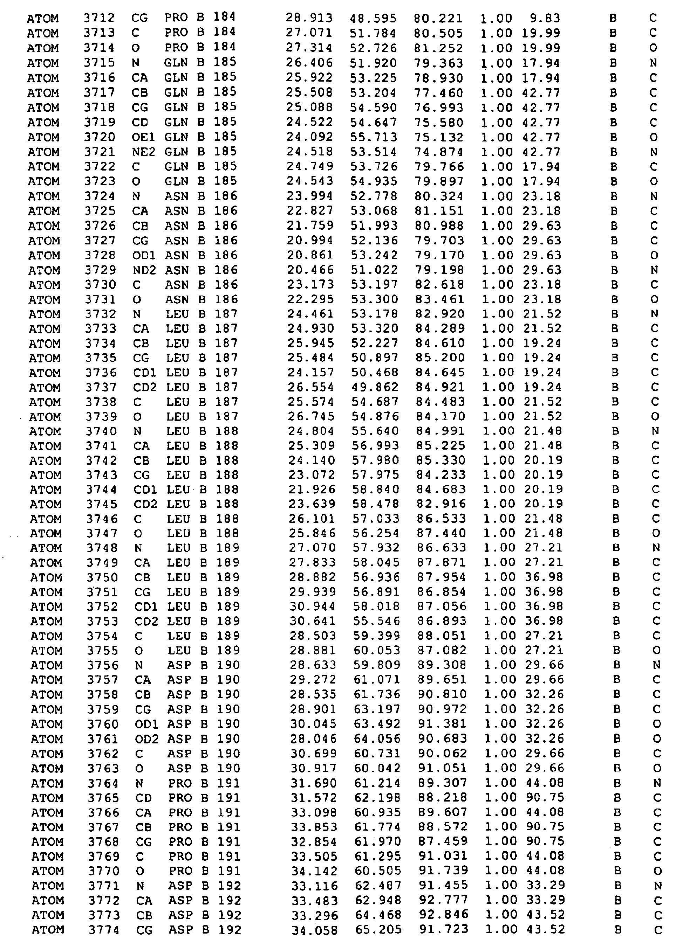

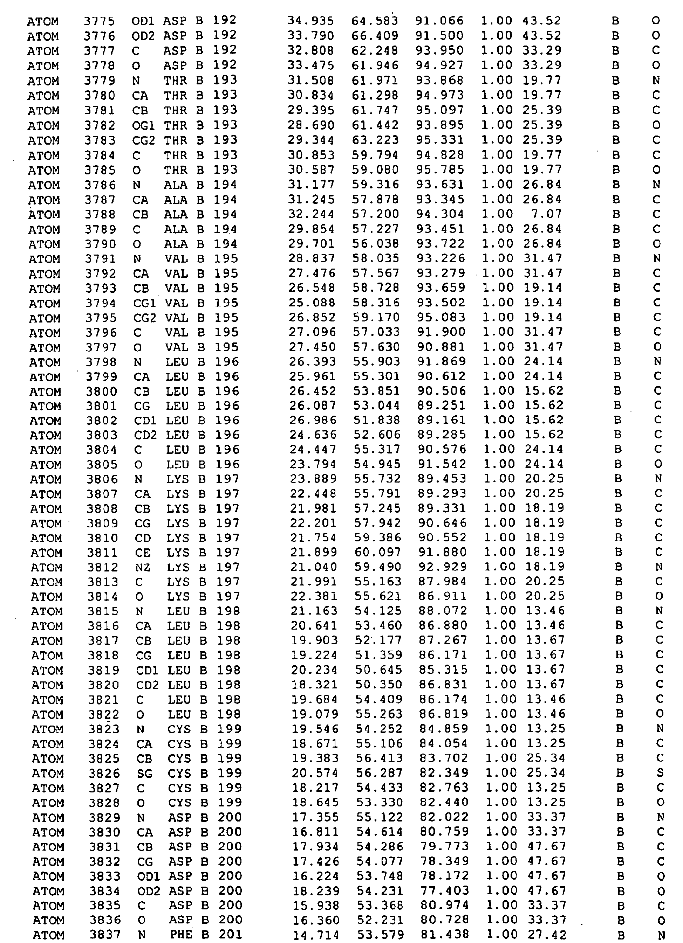

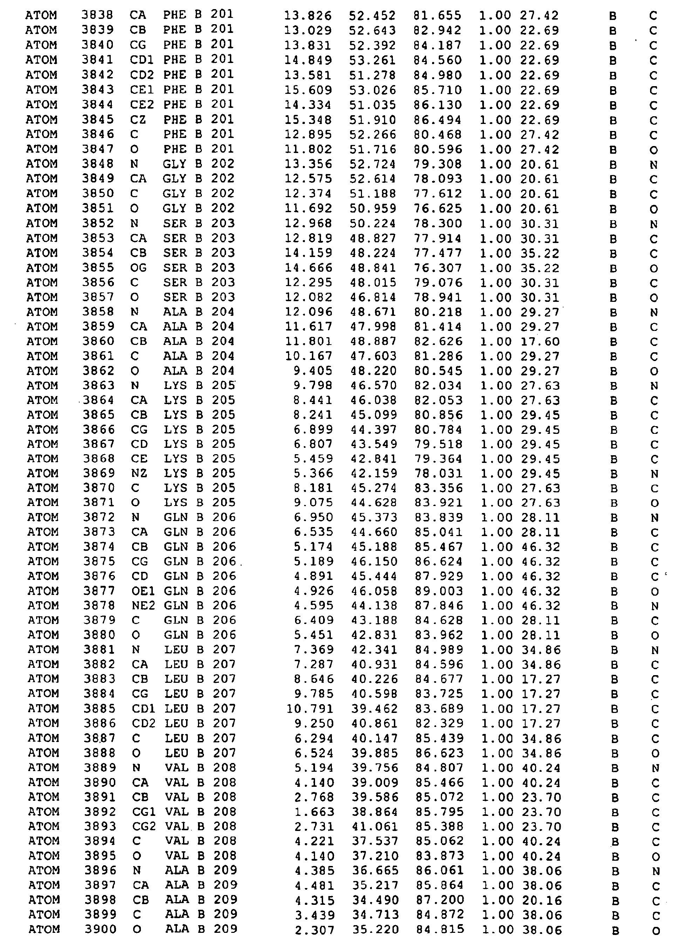

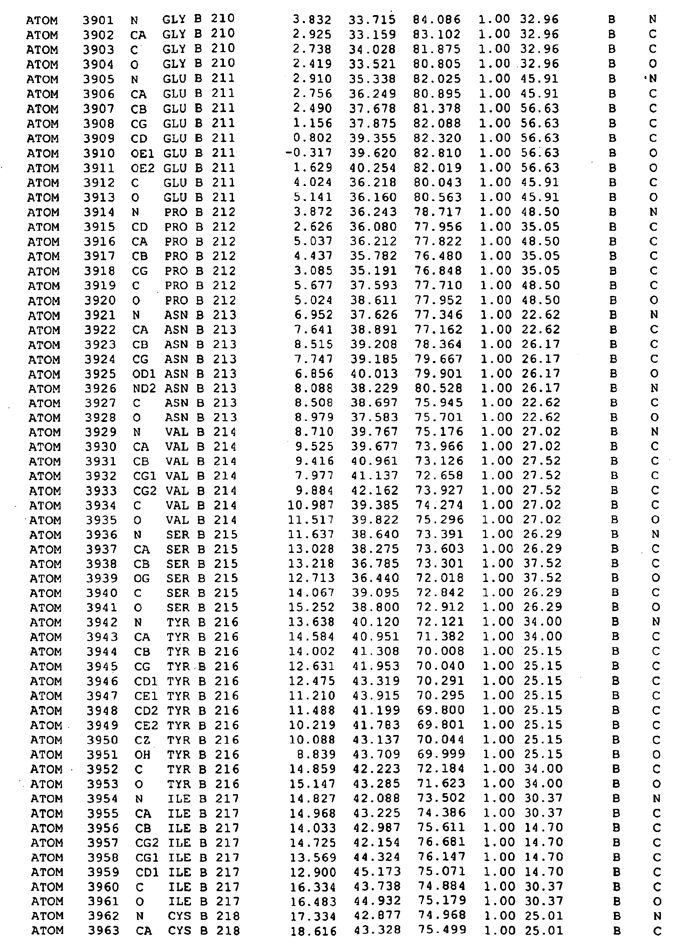

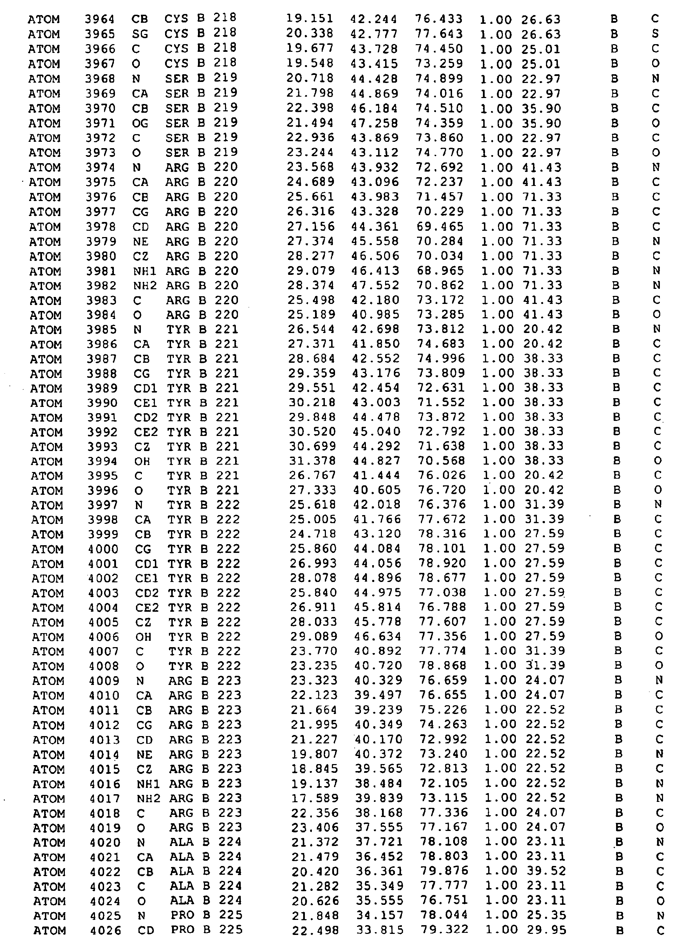

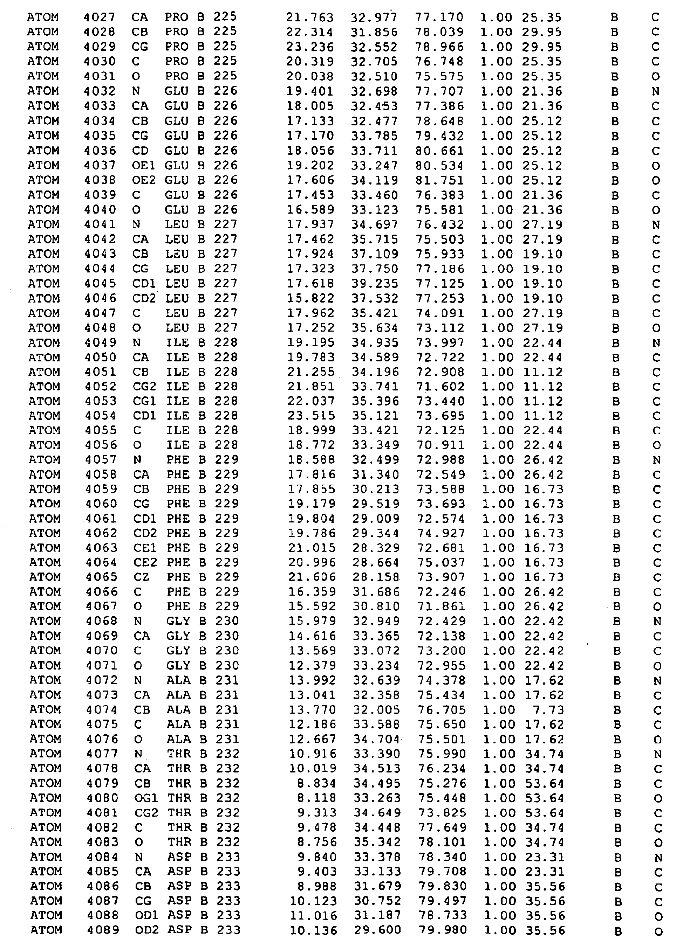

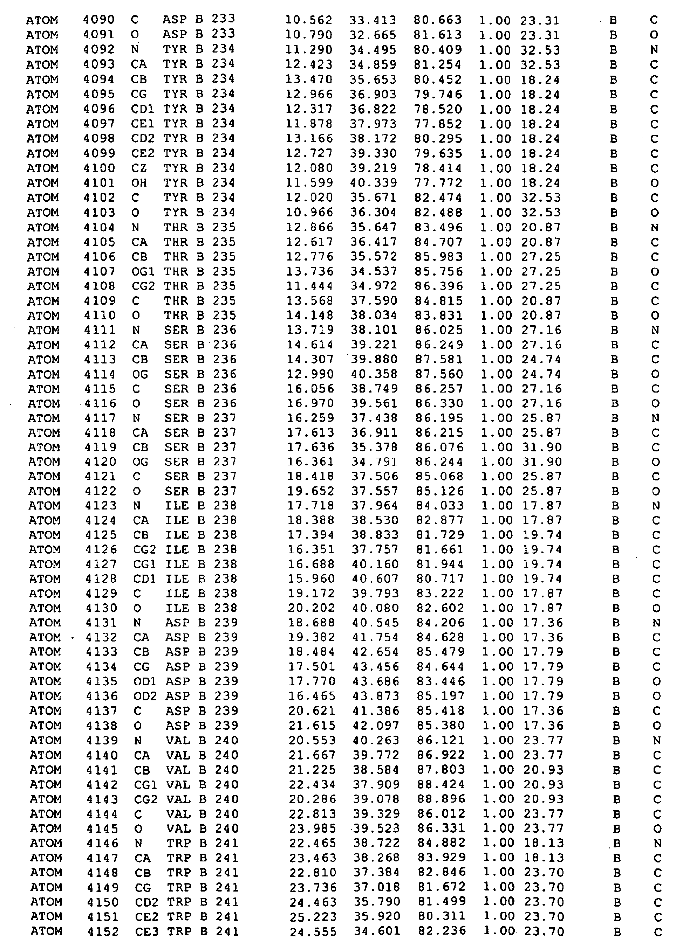

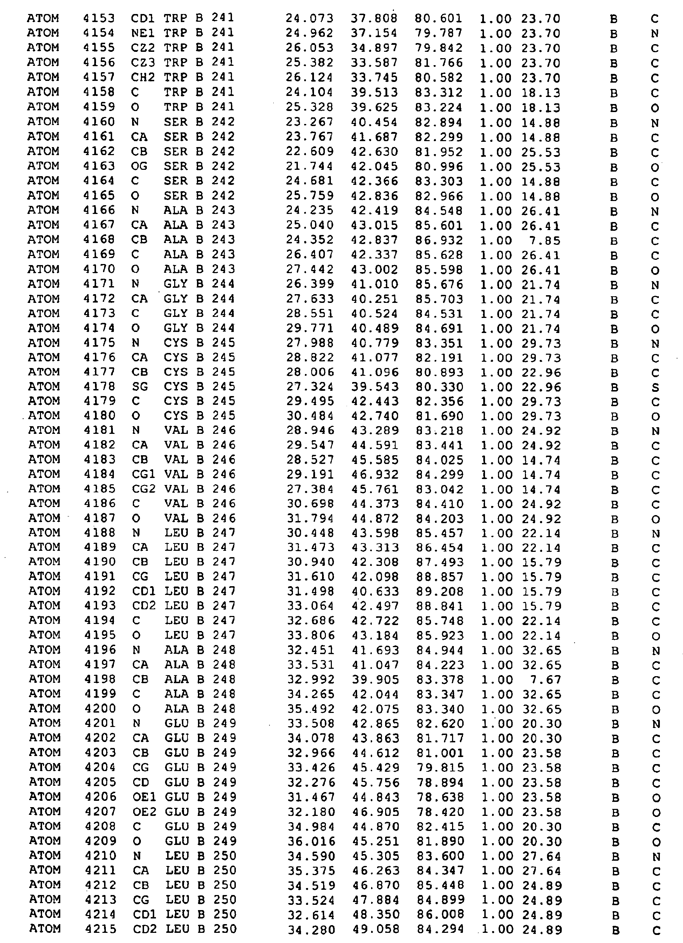

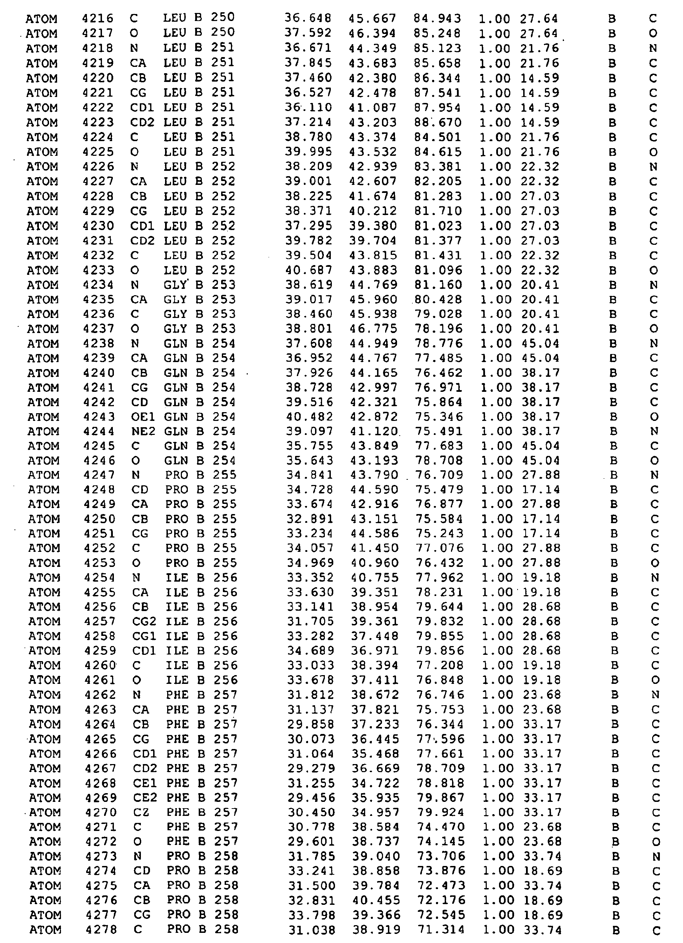

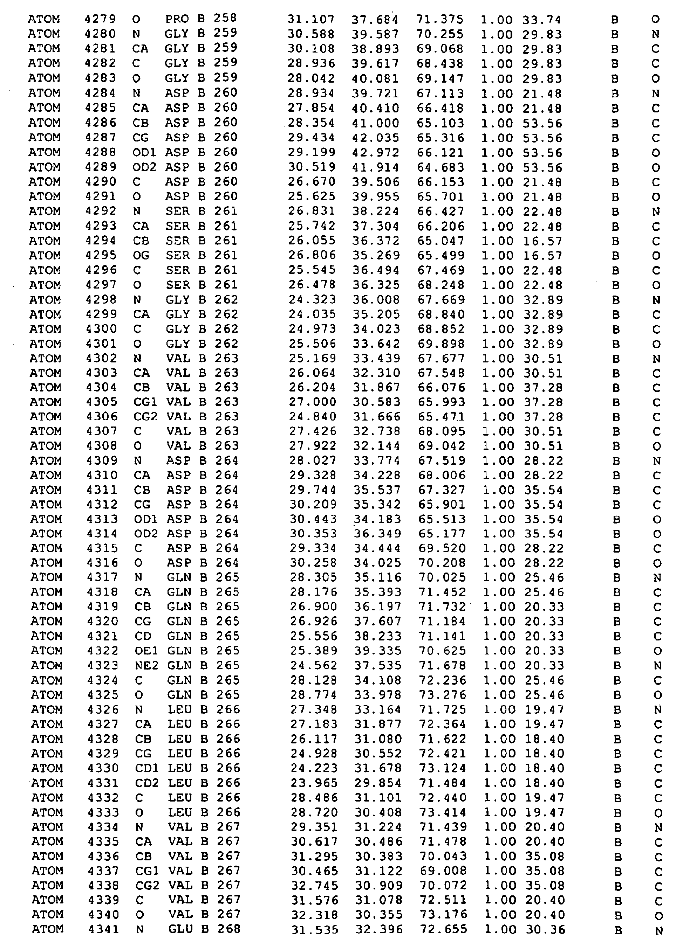

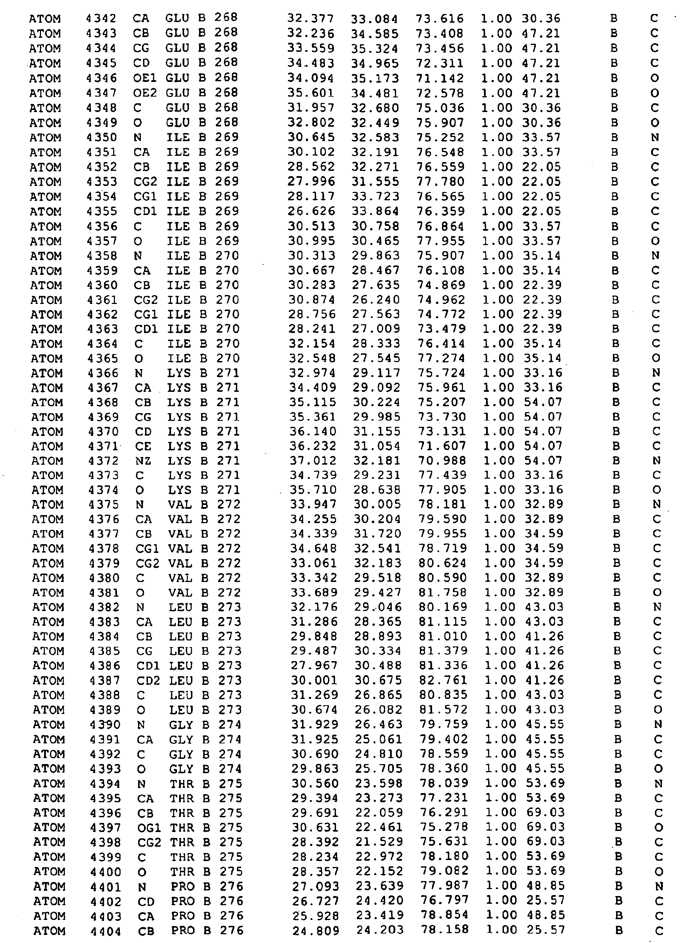

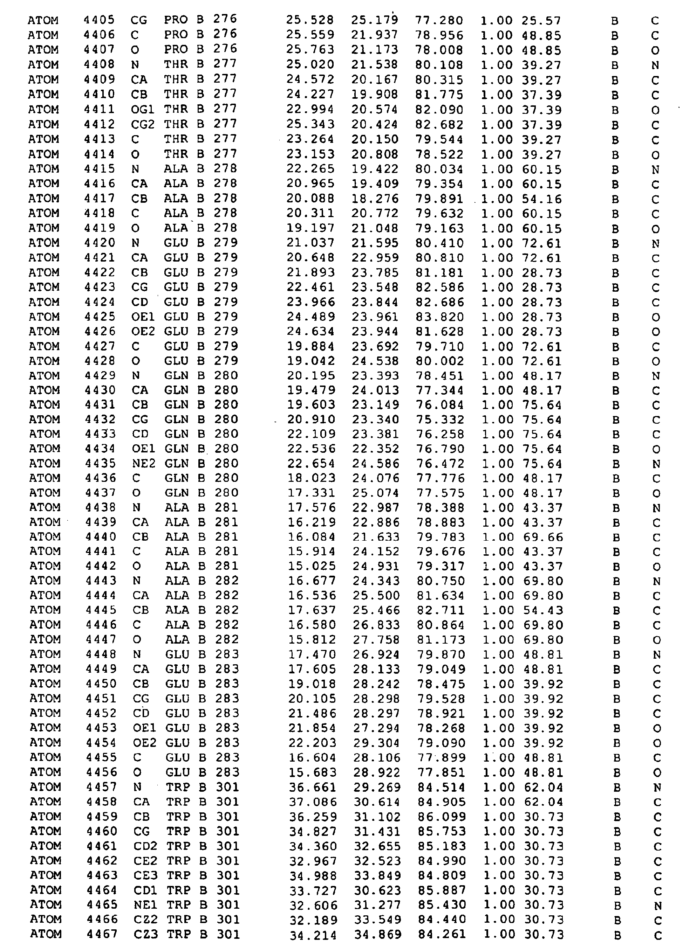

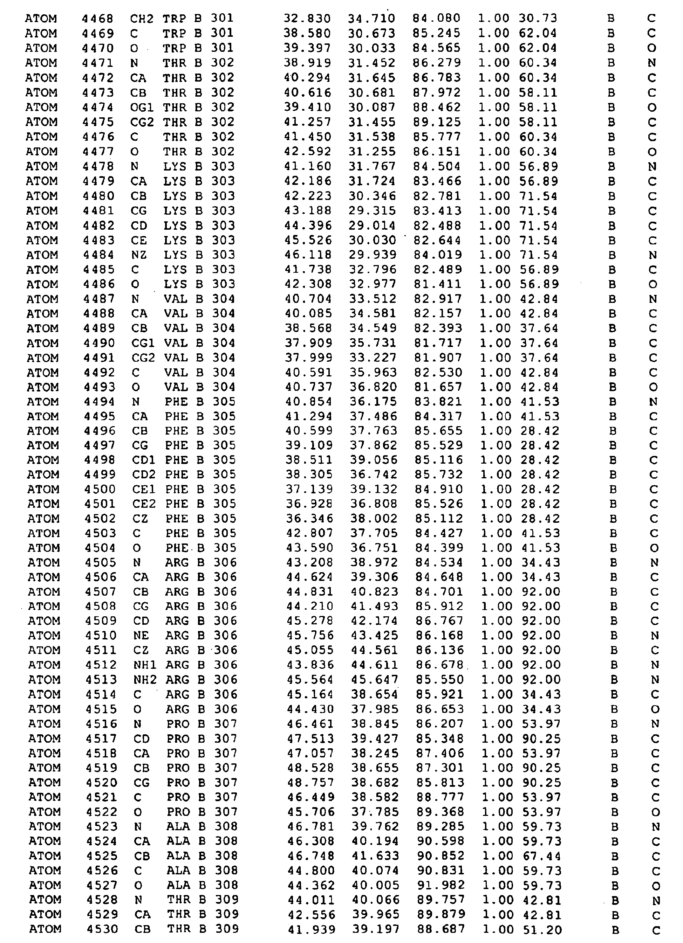

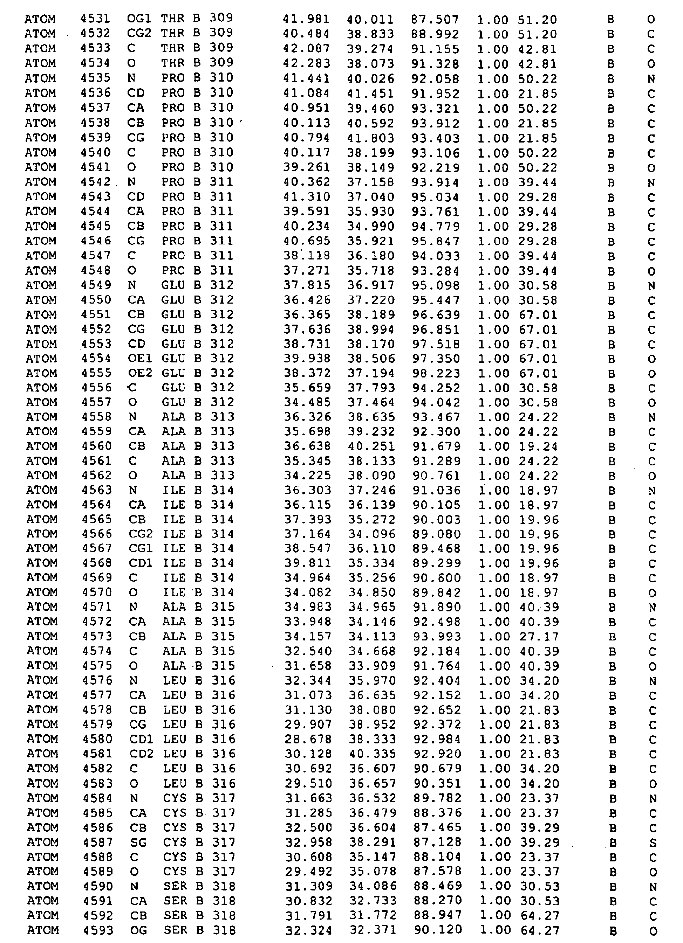

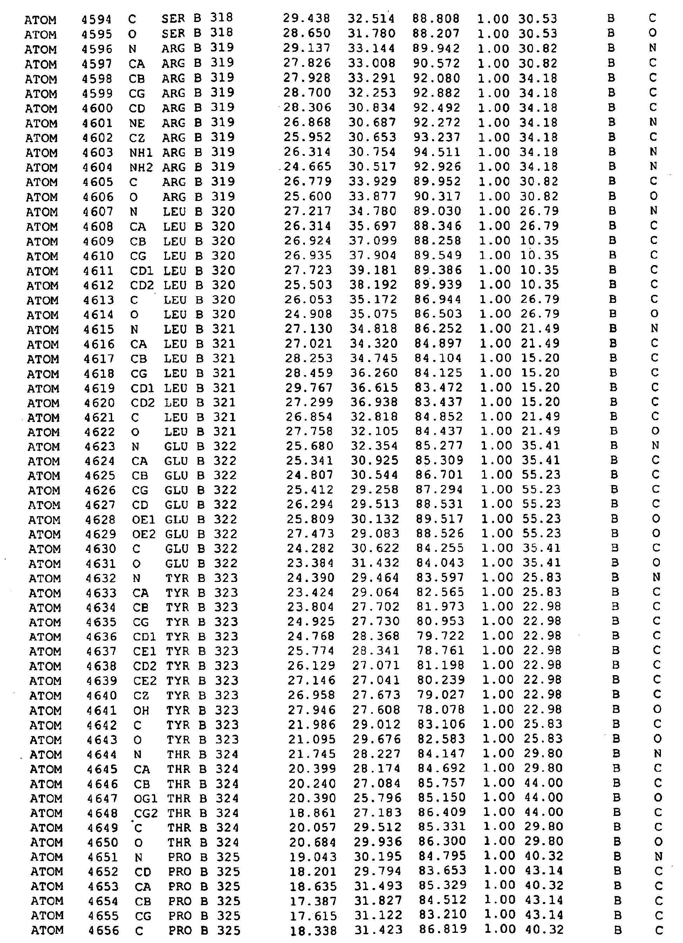

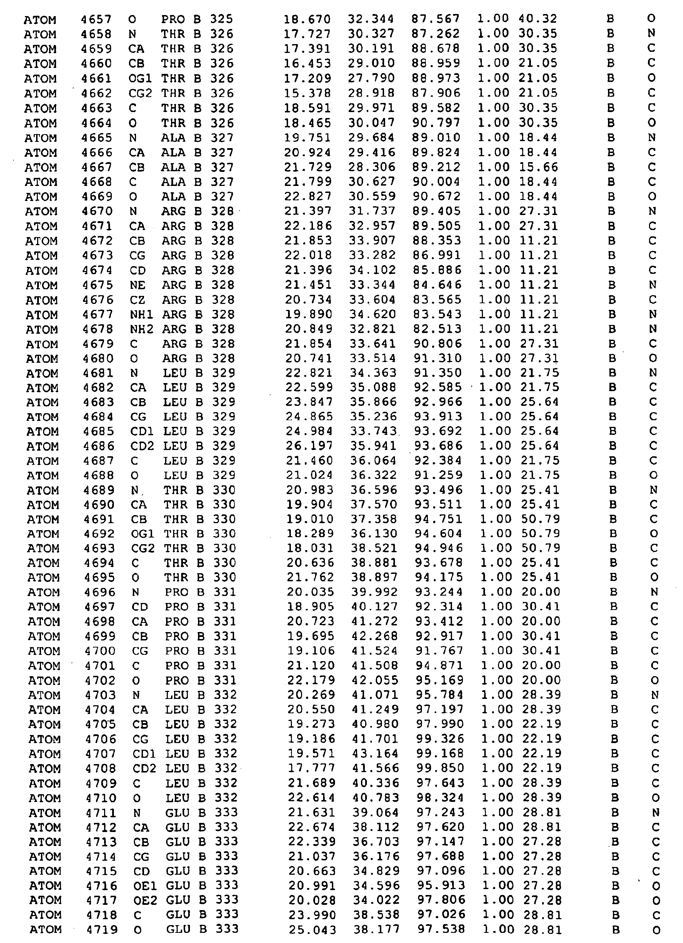

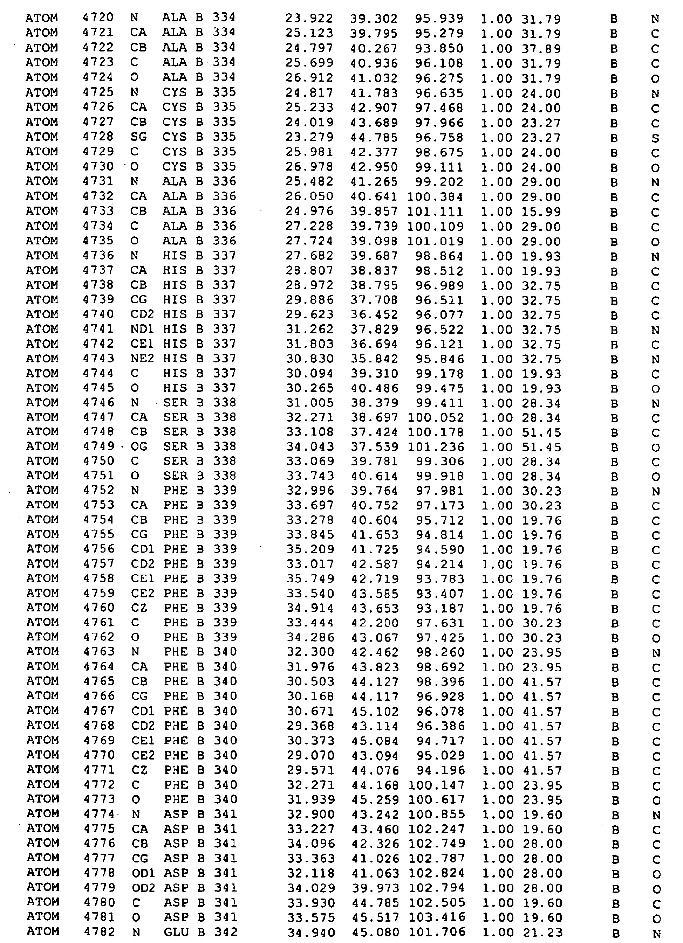

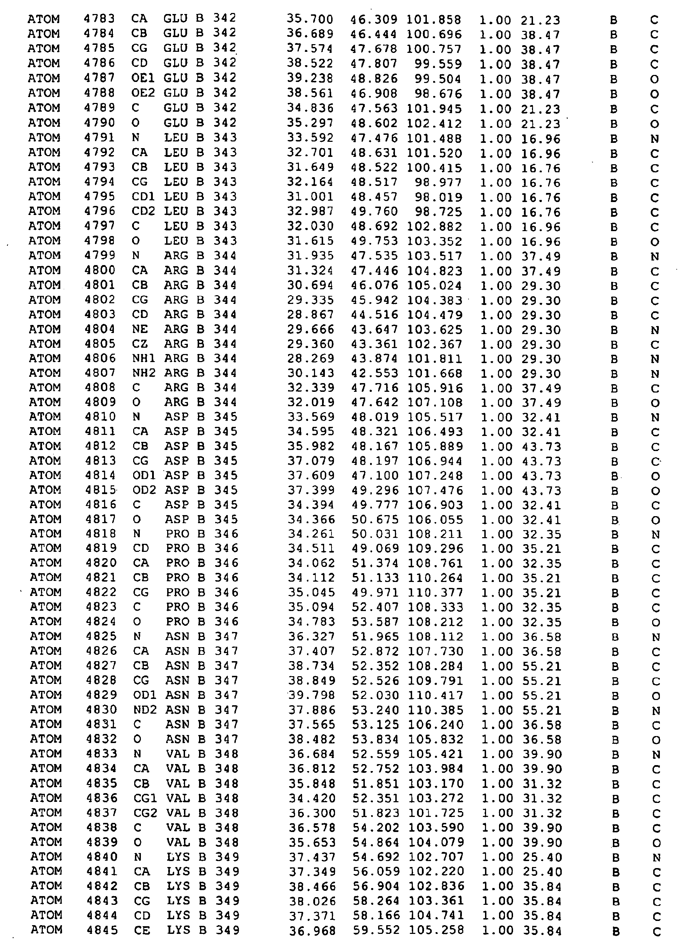

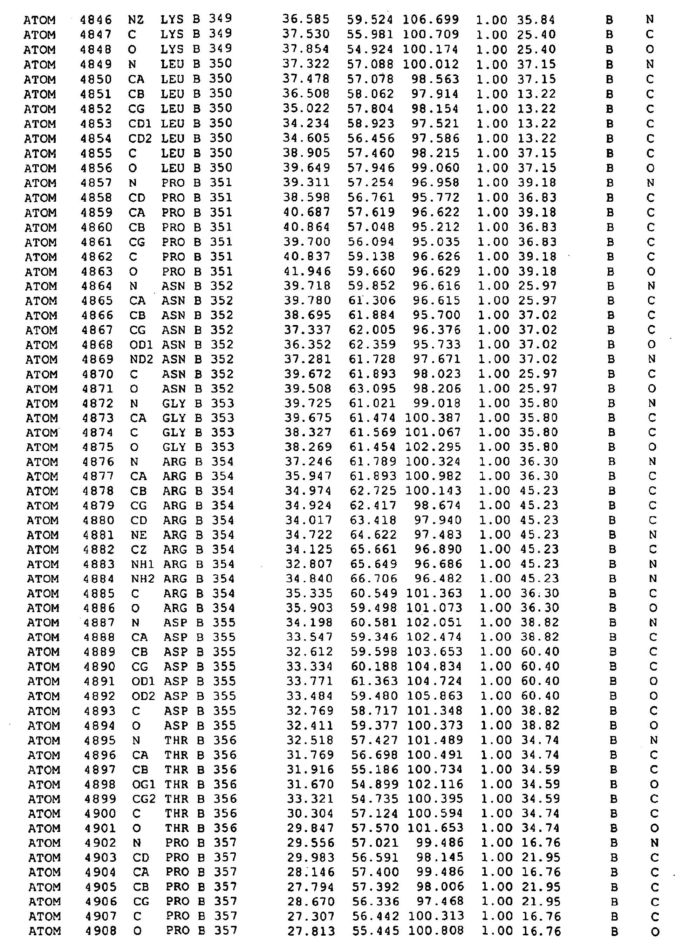

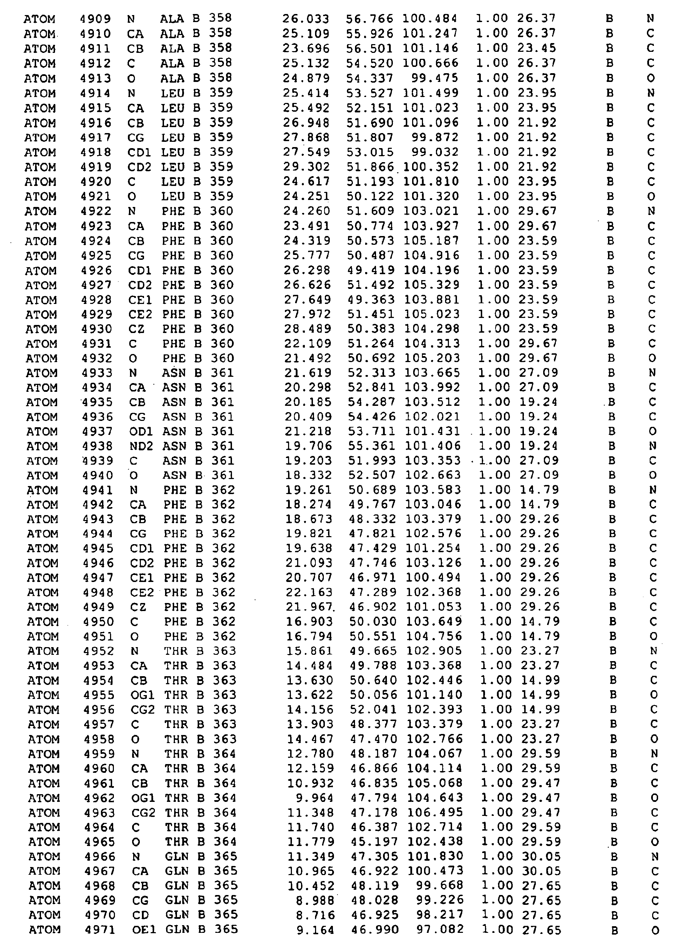

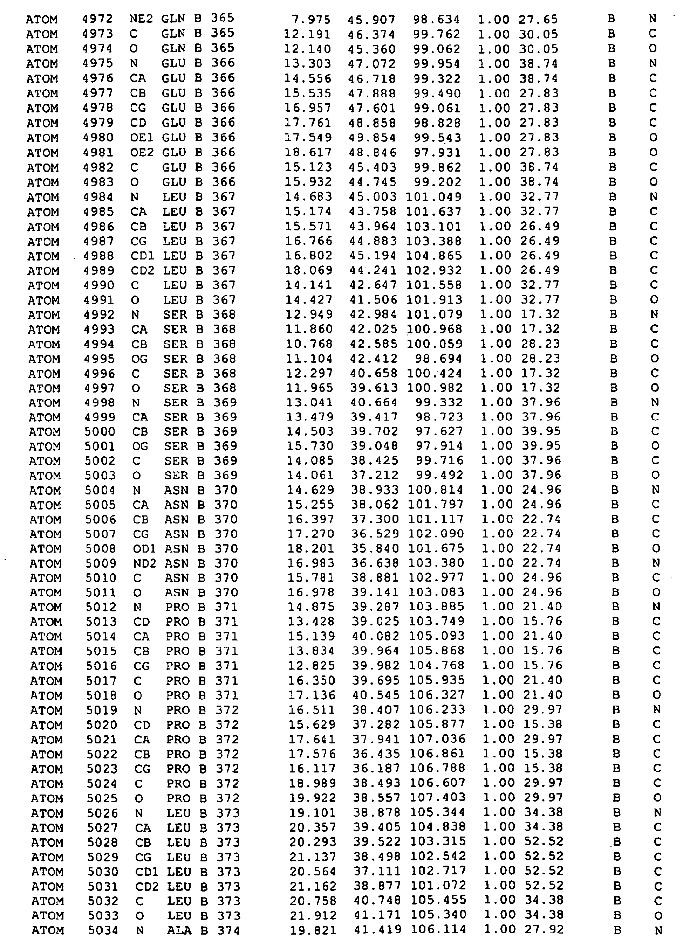

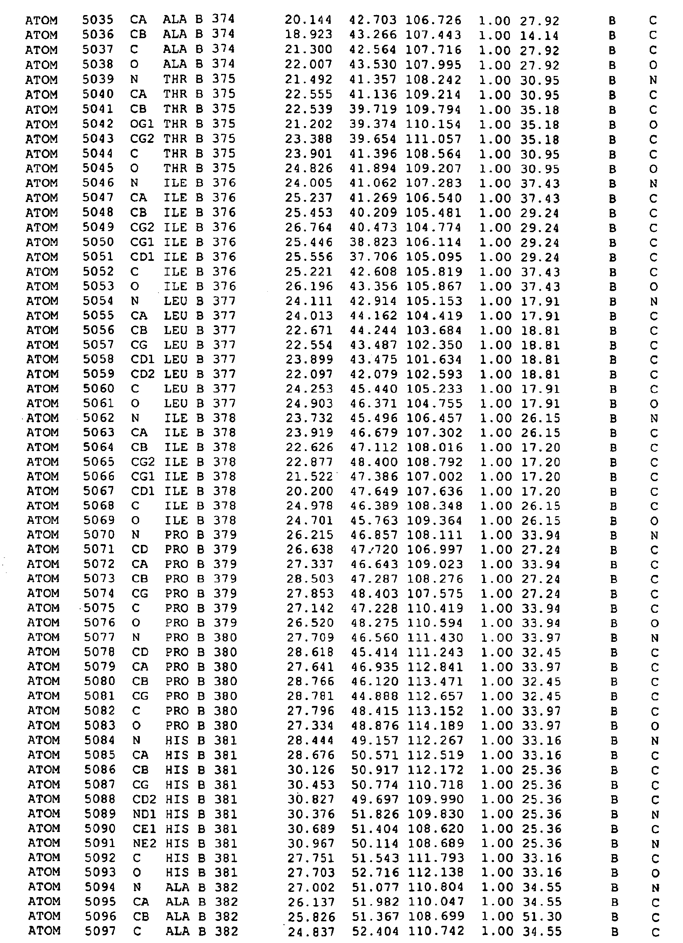

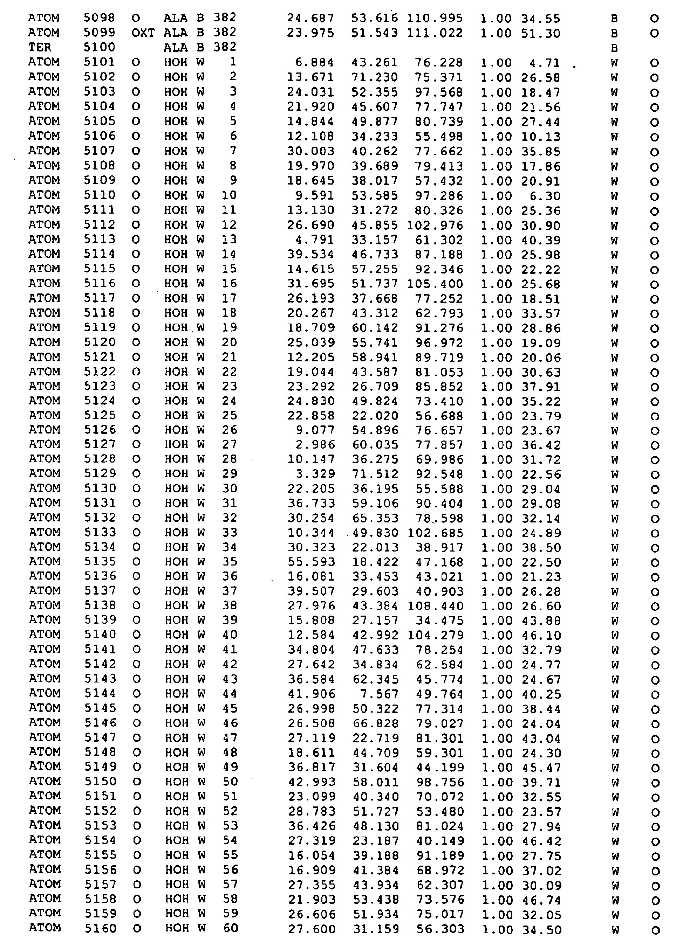

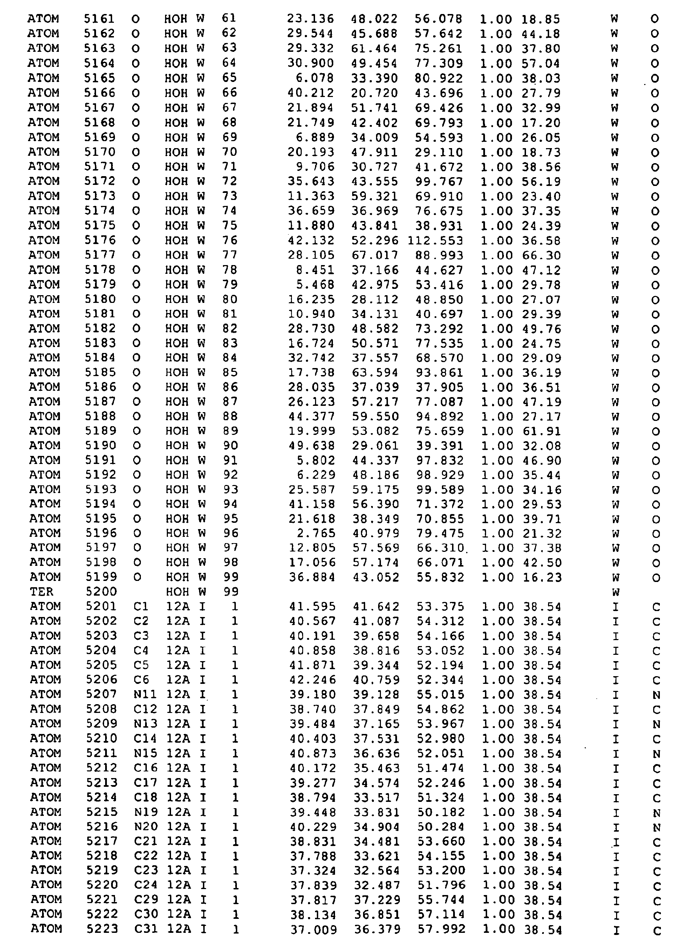

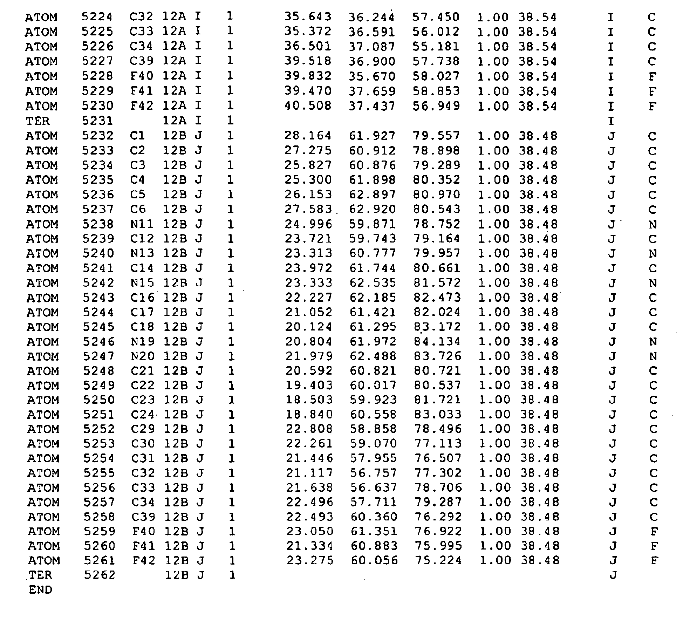

Surprisingly, it has now been found that for GSK-3β-inhibitor4 complex (Figure 6), the conformation of Gln185 is very different from the conformations reported for glutamines at this position in unphosphorylated, phosphorylated GSK-3β, GSK-3β-ADP-peptide complex and other protein kinases. A glutamine side chain is able to adopt different conformations, depending on its chemical environment. In the case of Gln185, the molecule that occupies the active site influences the conformation of the side chain of glutamine. When the molecule that occupies the GSK-3β active site contains an ortho-substituted phenyl ring and that ring is within 3.9 Å of Ile 62, Phe 67, Val 70, Asn 186 and Asp 200, the glutamine side chain adopts a conformation with a χ1 angle of -176.4° and a χ2 angle of 174°. Taking into consideration the steric hindrance of nearby residues, χ1 of Gln185 can range from 123' to 180°, χ2 can range from -174° to -180° and 106° to 180°. The χ1 can also range from -100° to -180°, and χ2 can range from -151° to -180° and 126° to 180°.

In order to compare the conformations of GSK-3β and other protein kinases at a particular amino acid site, such as Gln185, along the polypeptide backbone, well-known procedures may be used for doing sequence alignments of the amino acids. Such sequence alignments allow for the equivalent sites to be compared. One such method for doing a sequence alignment is the "bestfit" program available from Genetics Computer Group which uses the local homology algorithm described by Smith and Waterman in Advances in Applied Mathematics 2; 482 (1981) .

Equivalents of the Gln185 residue of GSK-3 may also be identified by its functional position. Gln185 is located on the α-helical domain of the GSK-3β kinase domain in front of the active site. It is positioned five residues after the conserved RD motif (Arg 180, Asp 181) and just before the beginning of beta-strand β7 (Bax, B. et al. Structure, 9, pp 1143-1152, (2001)).

A comparison of the torsion angles between Gln185 in the GSK-3β or GSK-3β complexes and those of corresponding glutamines in other kinases are illustrated in Table 2. The torsion angles were determined by the program QUANTA.

**TABLE 2**

| Protein | χ1(°) | χ2(°) | χ3(°) |
|---|---|---|---|
| GSK-3β-peptide-ADP | -60.0 | 83.3 | -25.7 |
| Phosphorylated GSK-3β | -59.9 | 83.2 | -22.5 |
| Unphosphorylated GSK-3β | -60.9 | 63.0 | 88.7 |
| GSK-3β-inhibitor1 | -67.1 | 113.9 | -72.2 |
| GSK-3β-inhibitor2 | -60.7 | 90.1 | 98 |
| GSK-3β-inhibitor3 | -63.3 | -158.5 | 179.8 |
| GSK-3β-inhibitor4 | -176.4 | -174.0 | -3.3 |
| CDK2_inhibitor ¹ | 87.4 | -172.5 | 73.4 |
| CDK2_cyclin A ² | -98.1 | -59.2 | 71.2 |

| | | | |
|---|---|---|---|
| 1: Cyclin-Dependant Kinase 2 in complex with oxindole inhibitor. Davis et al., Science, 291, 134 (2001); Protein Data Bank Accession number 1FW. 2: Phosphorylated Cyclin-Dependent Kinase-2 bound to Cyclin A. Russo et al., Nat. Struct. Biol., 3, 696 (1996); Protein Data Bank Accession number 1JST. | | | |

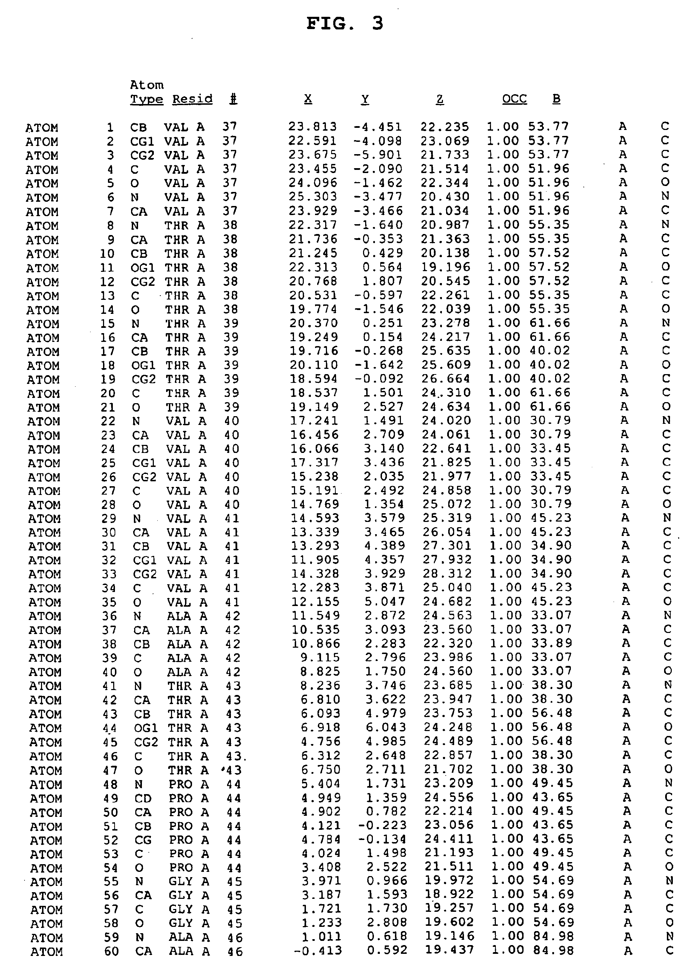

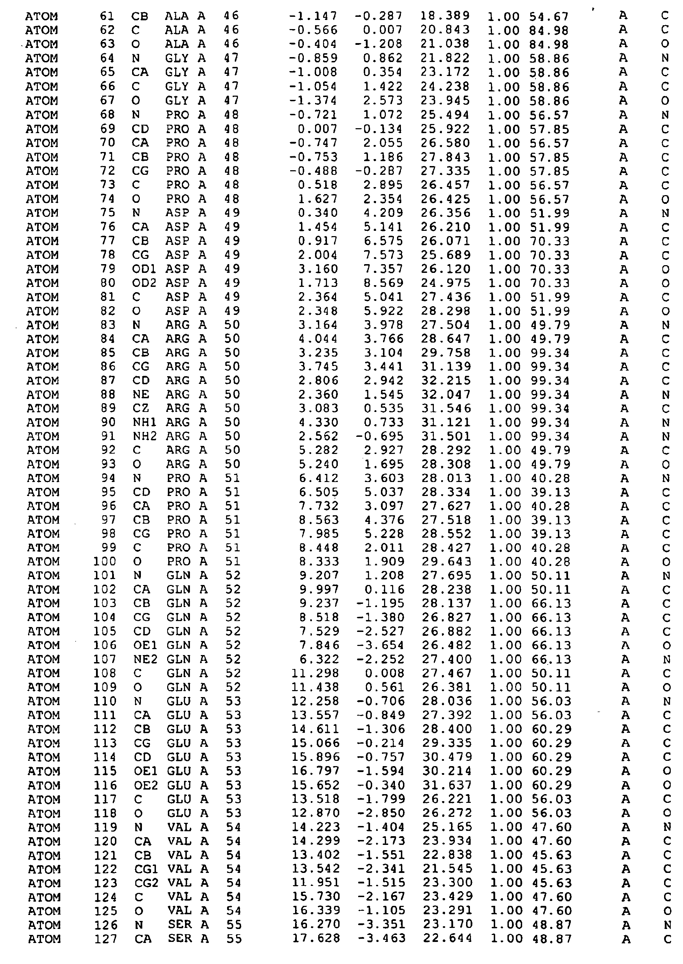

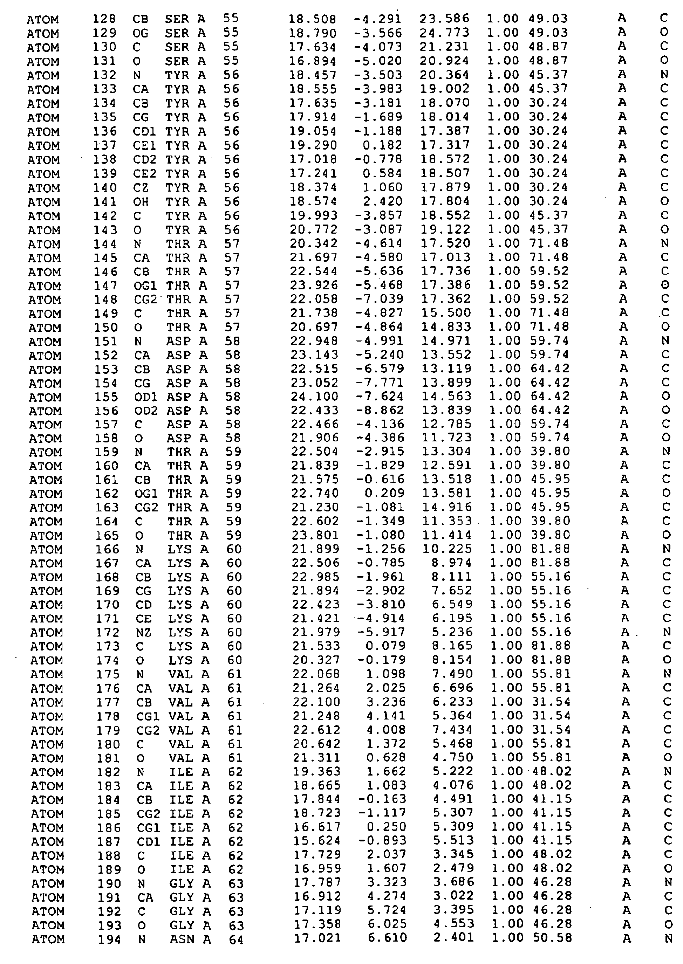

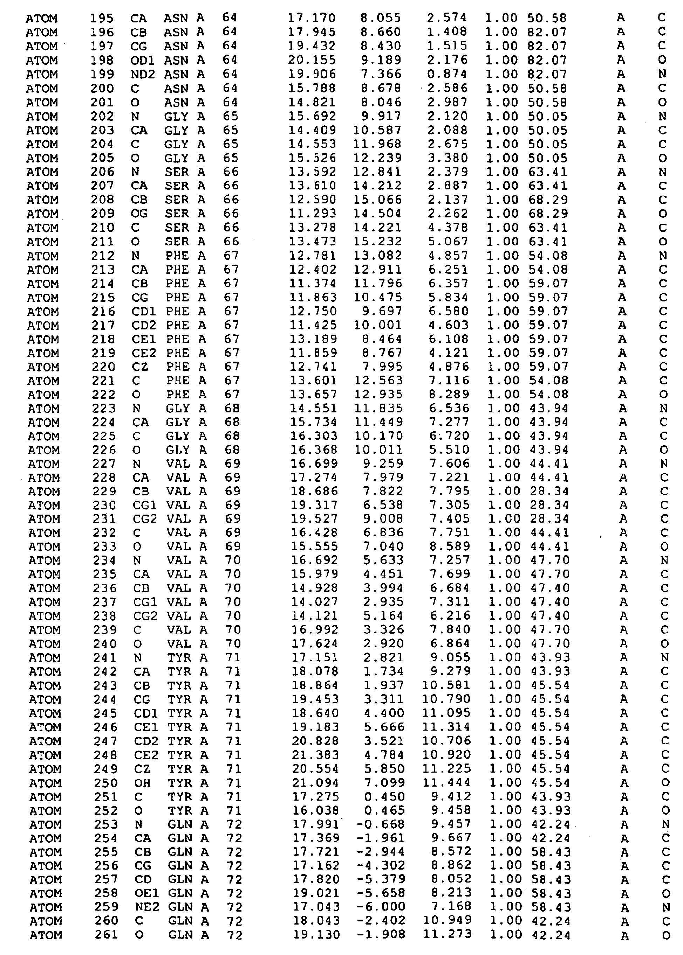

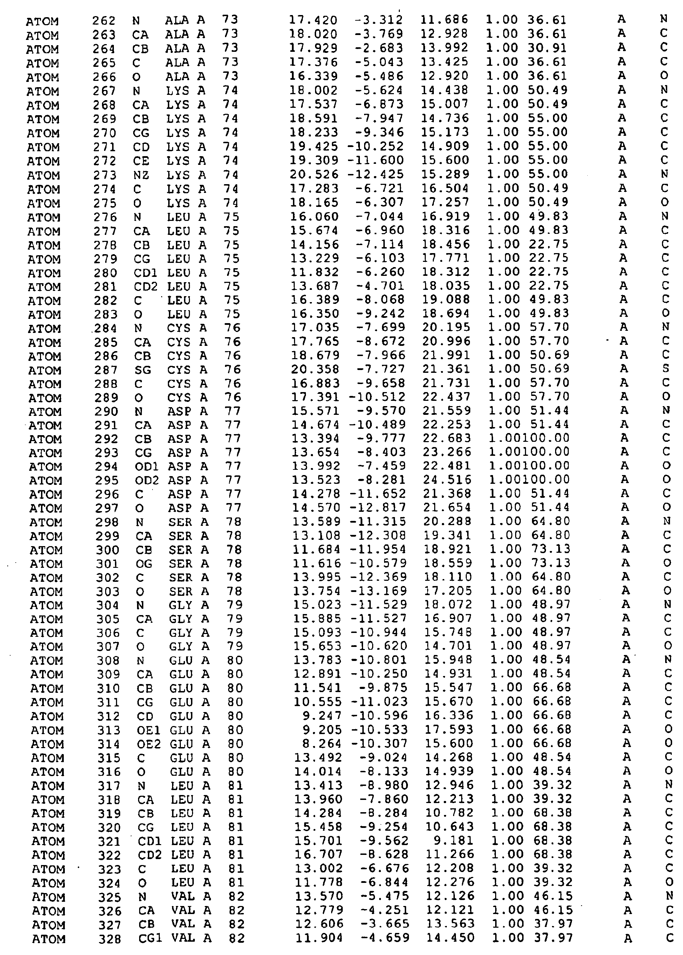

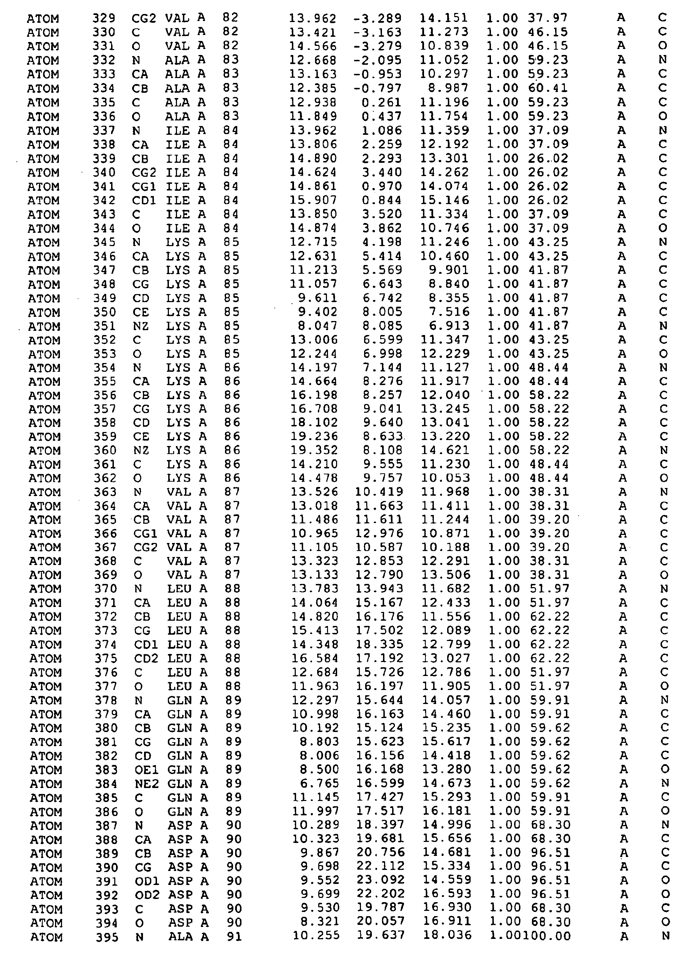

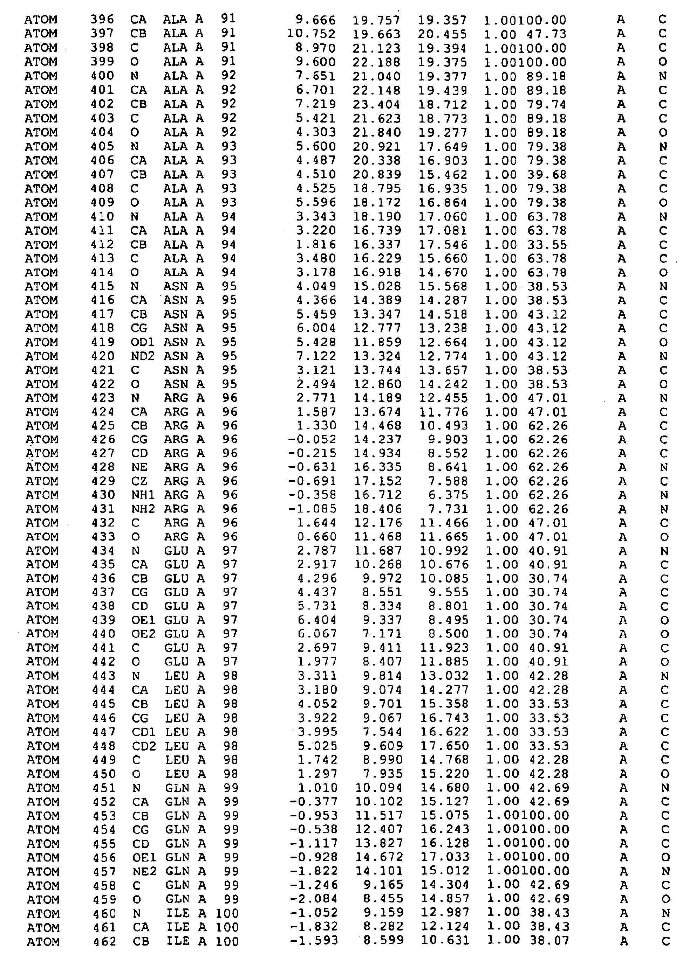

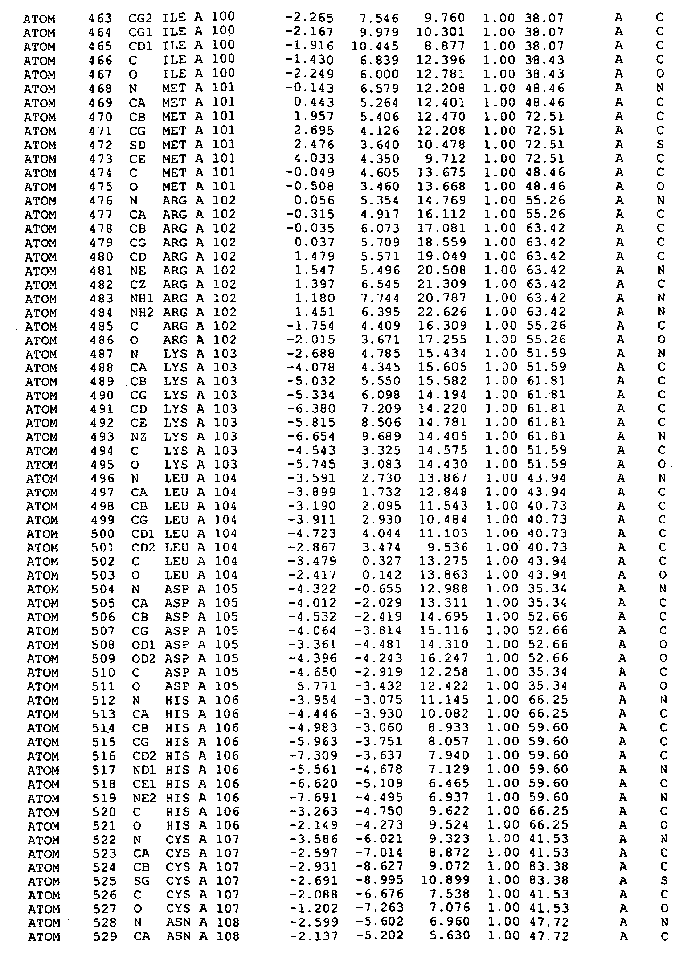

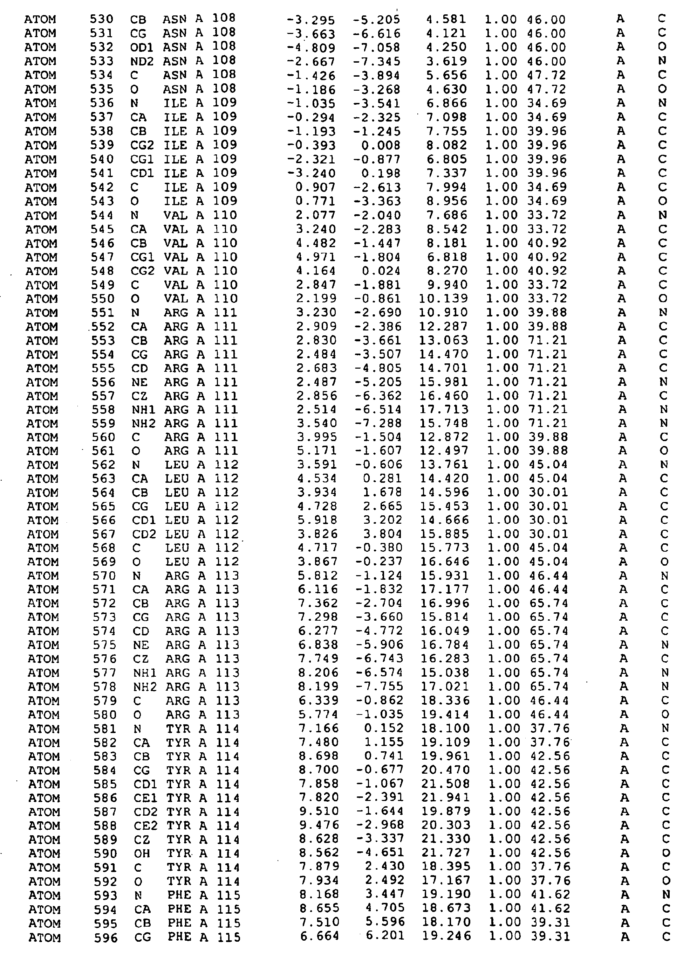

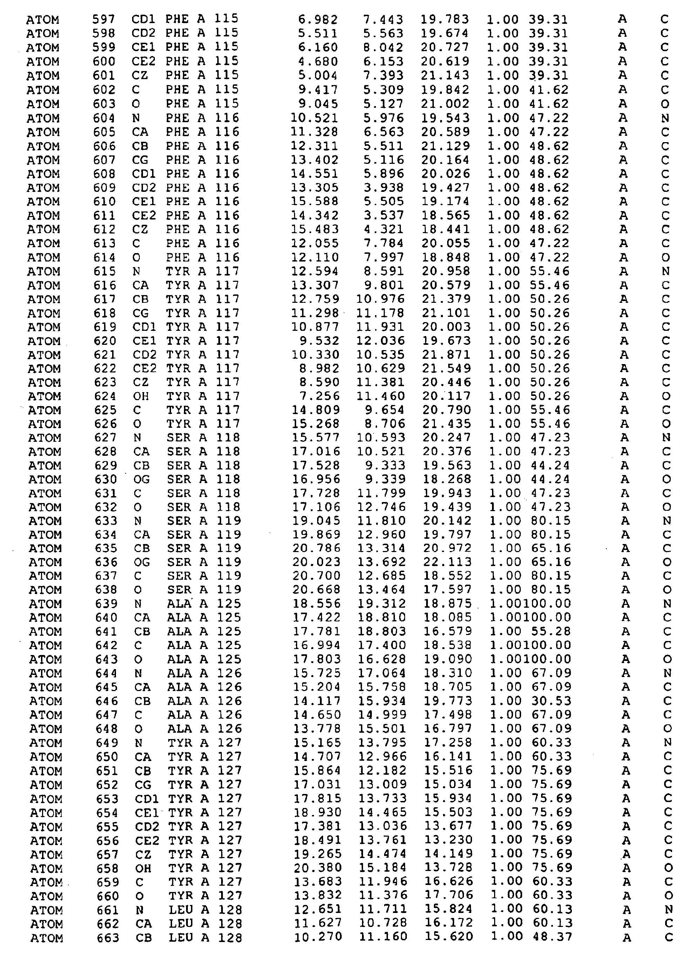

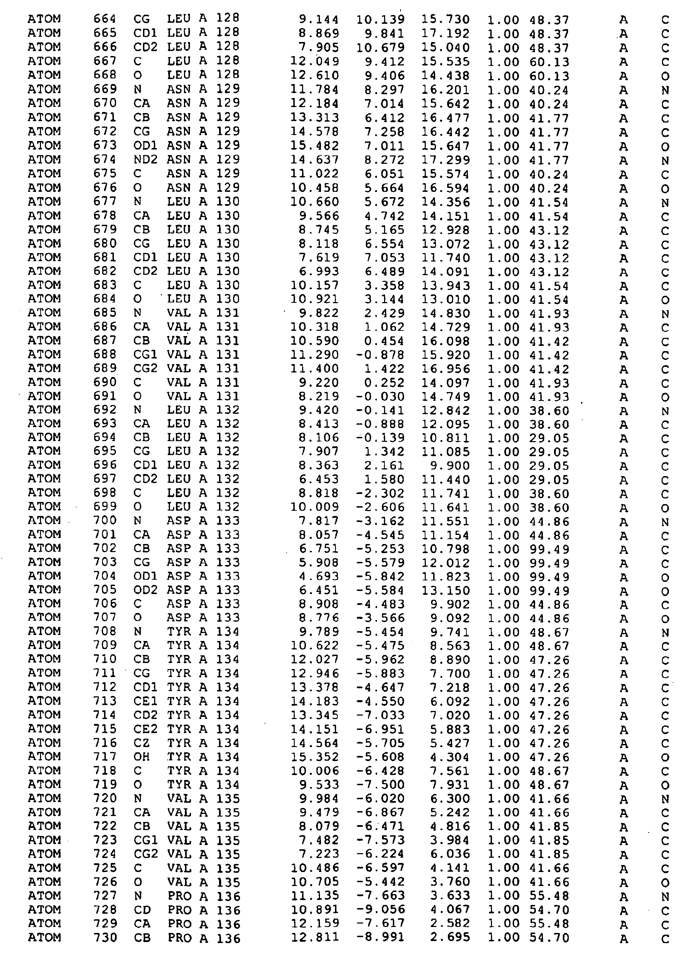

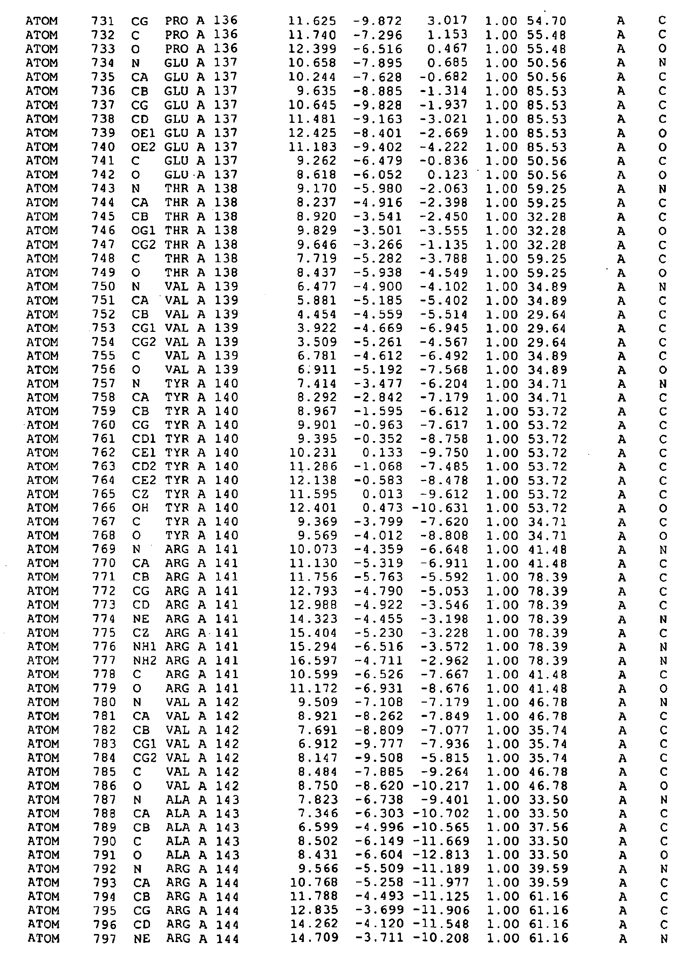

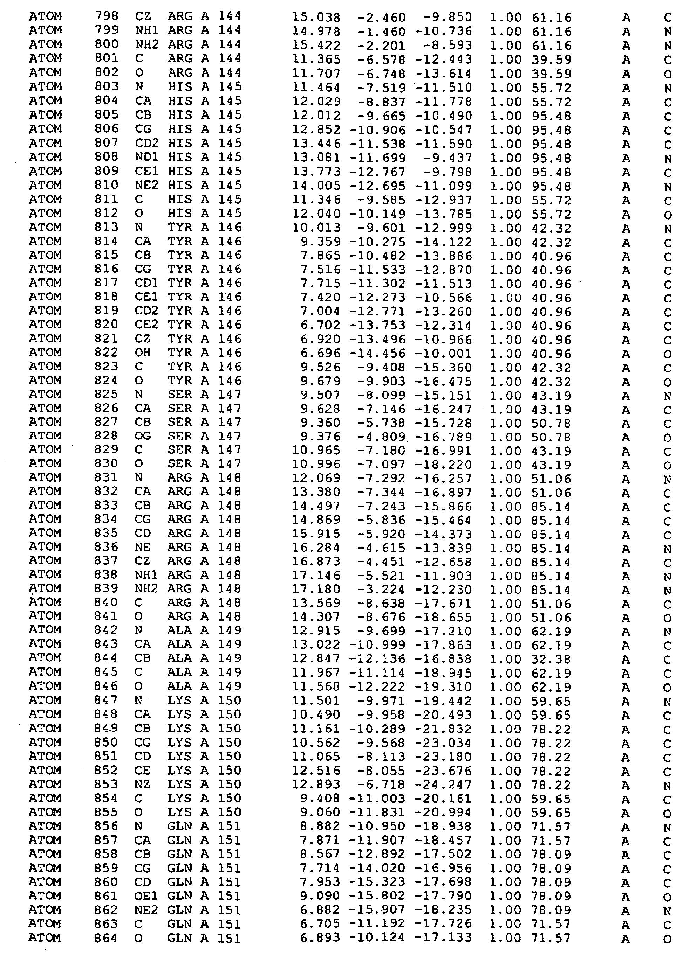

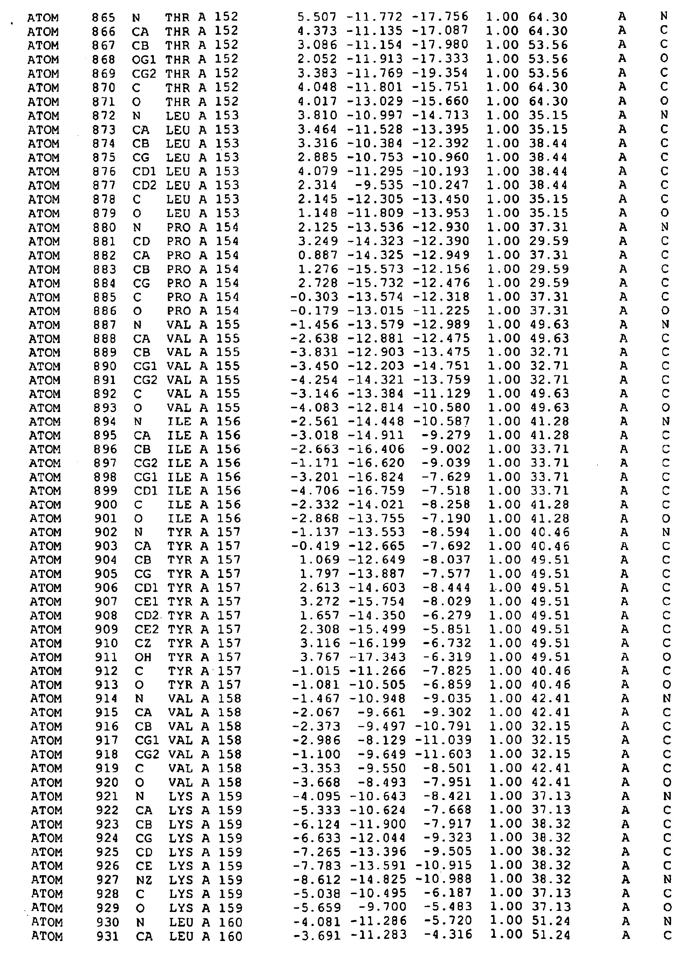

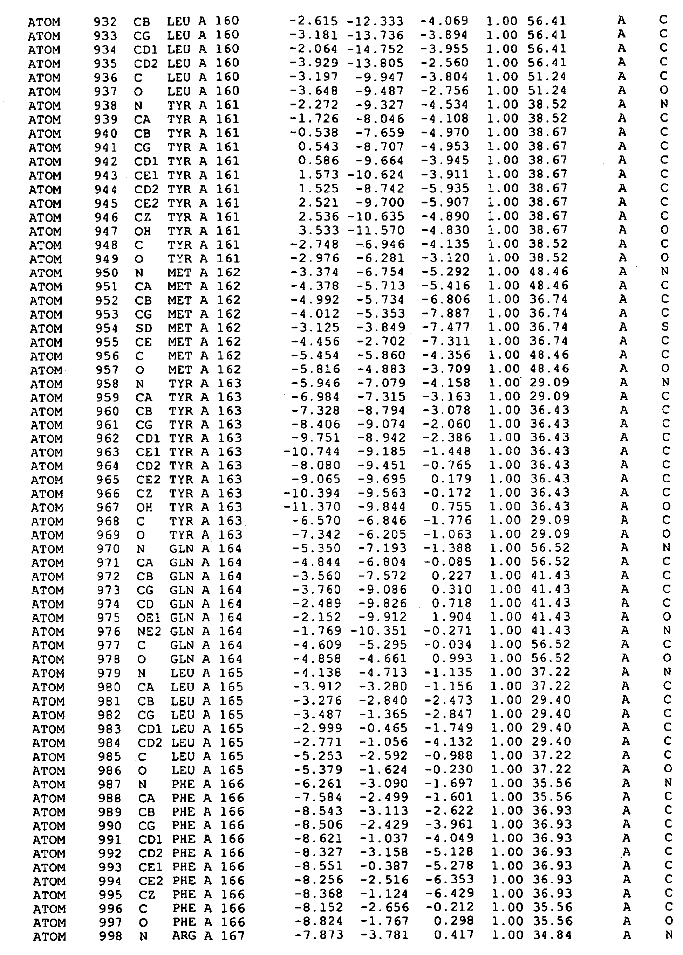

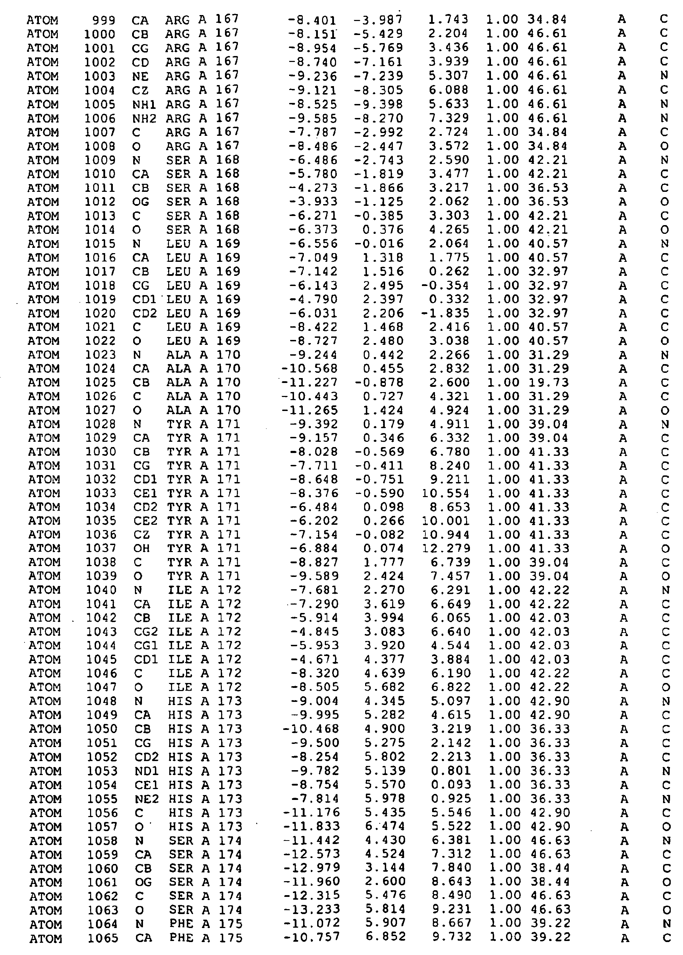

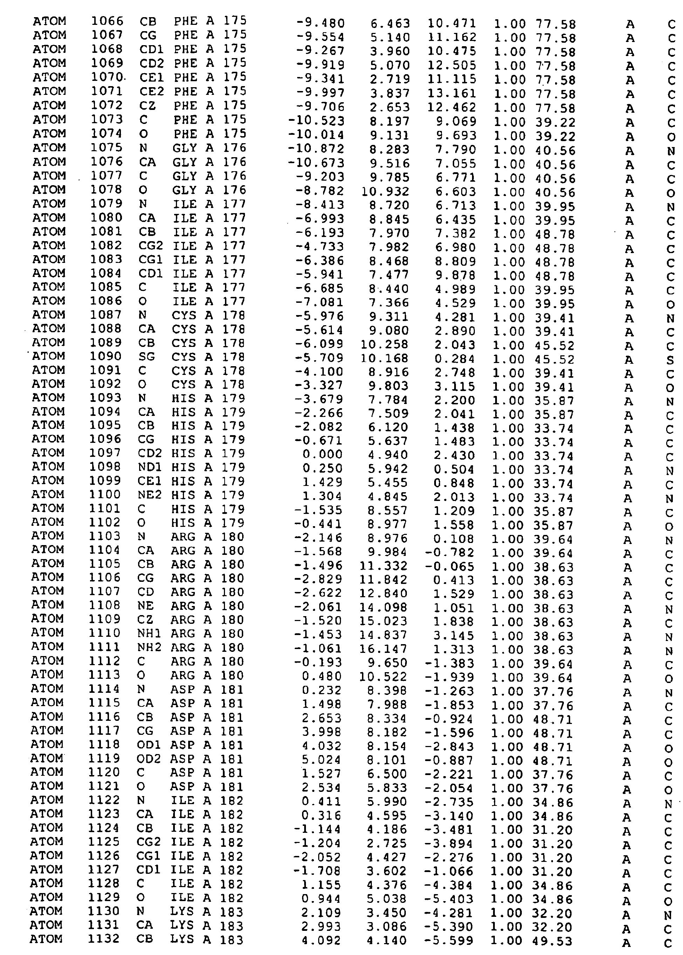

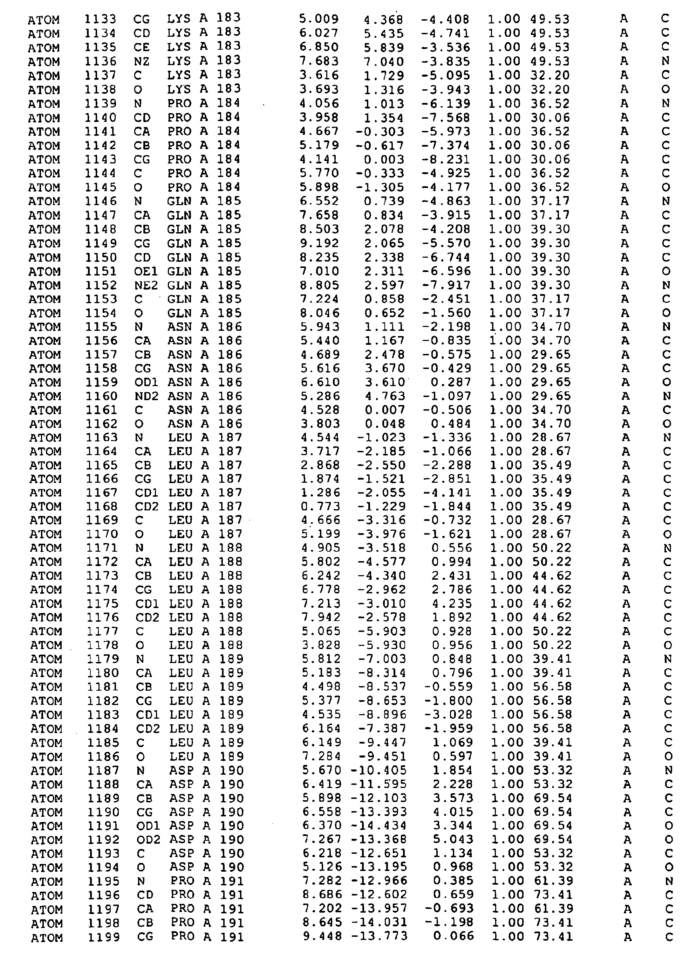

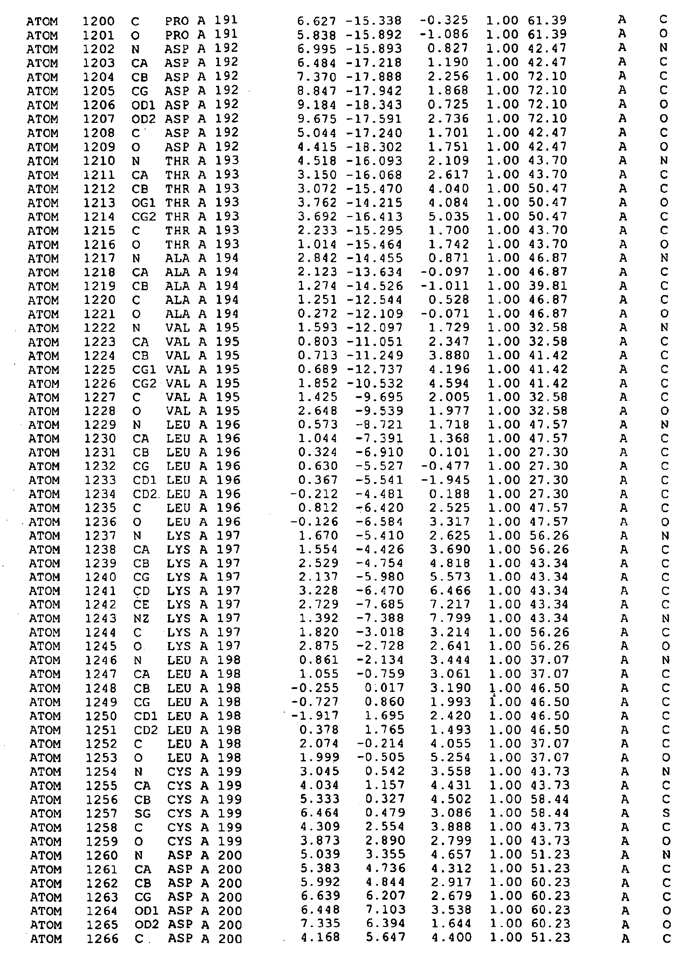

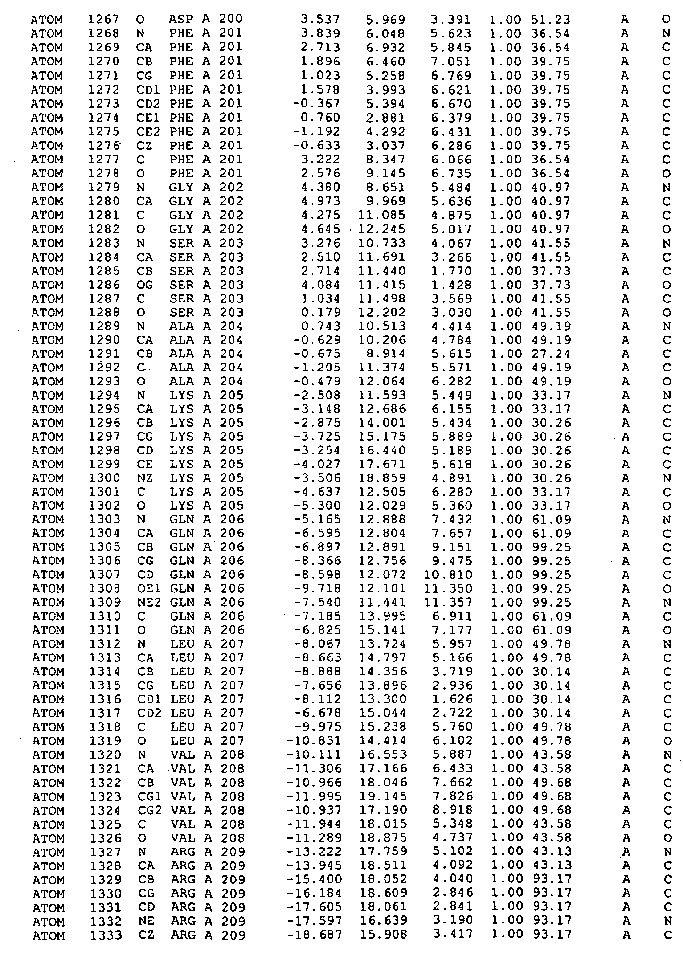

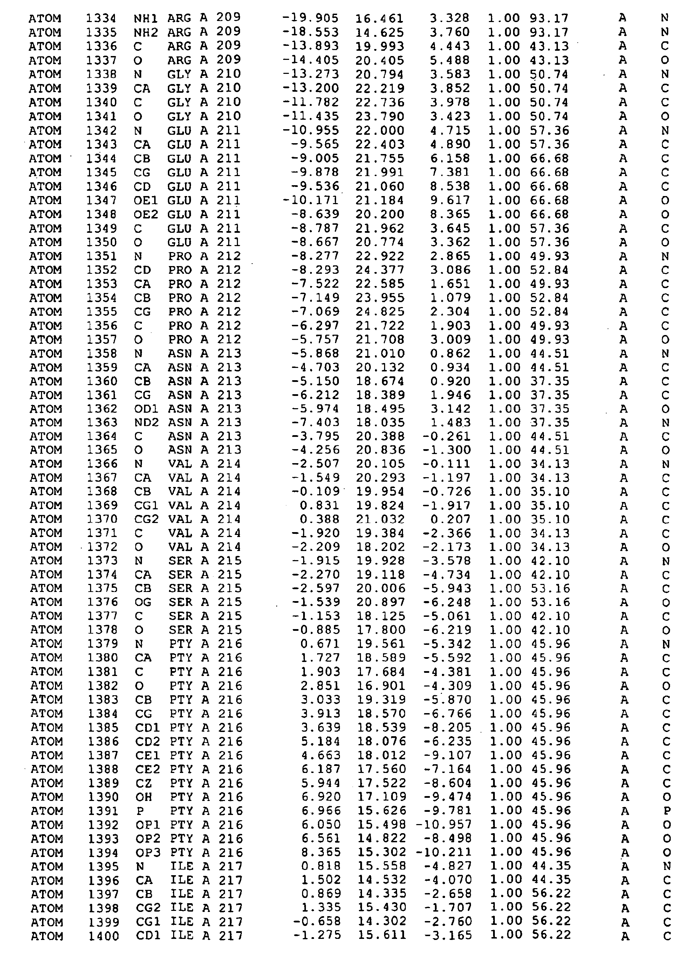

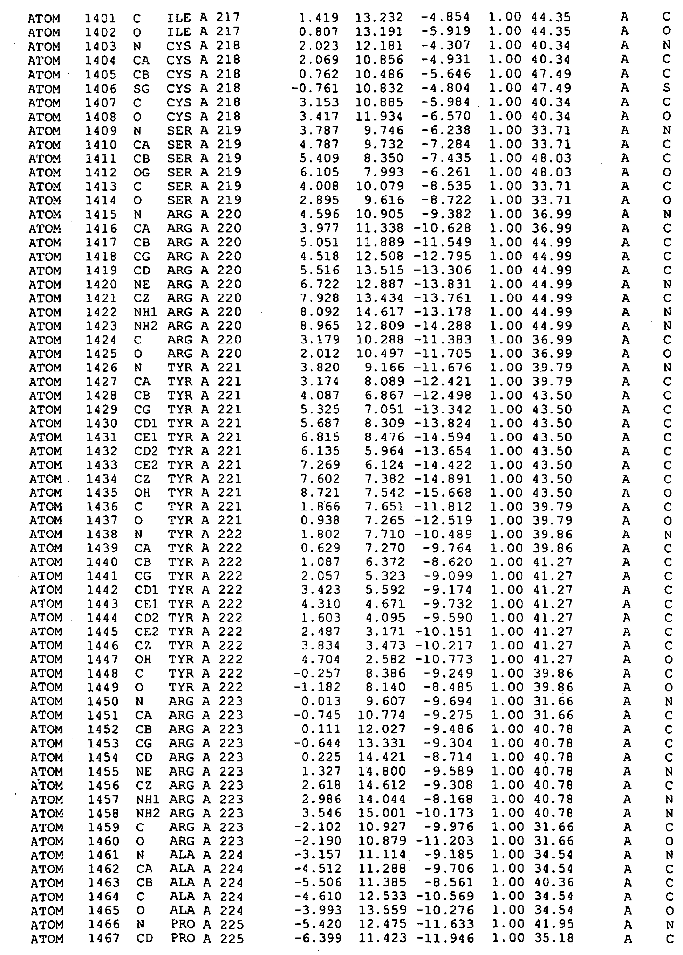

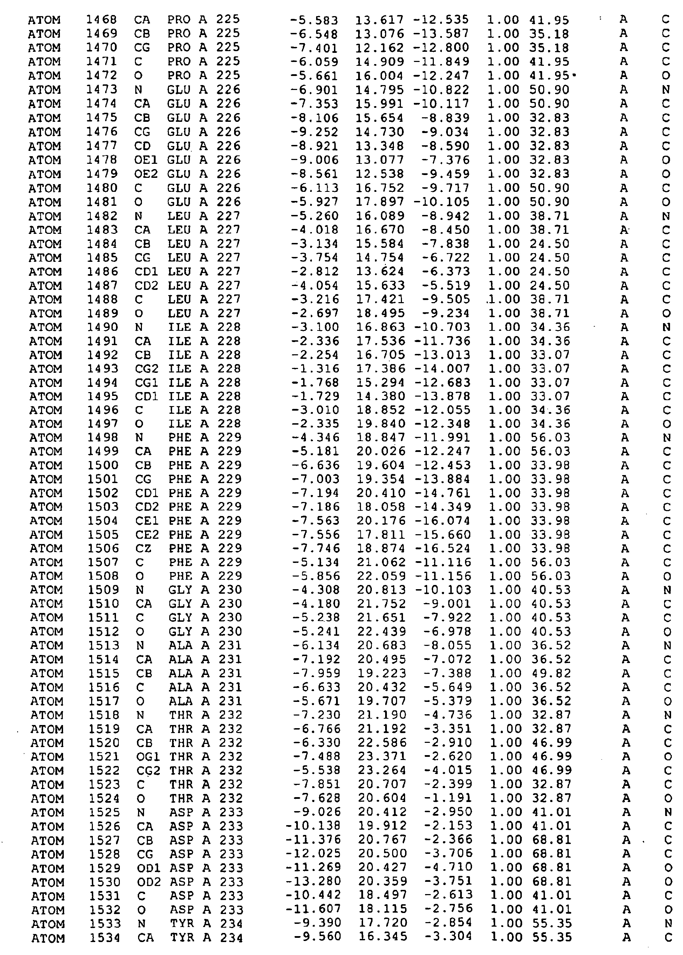

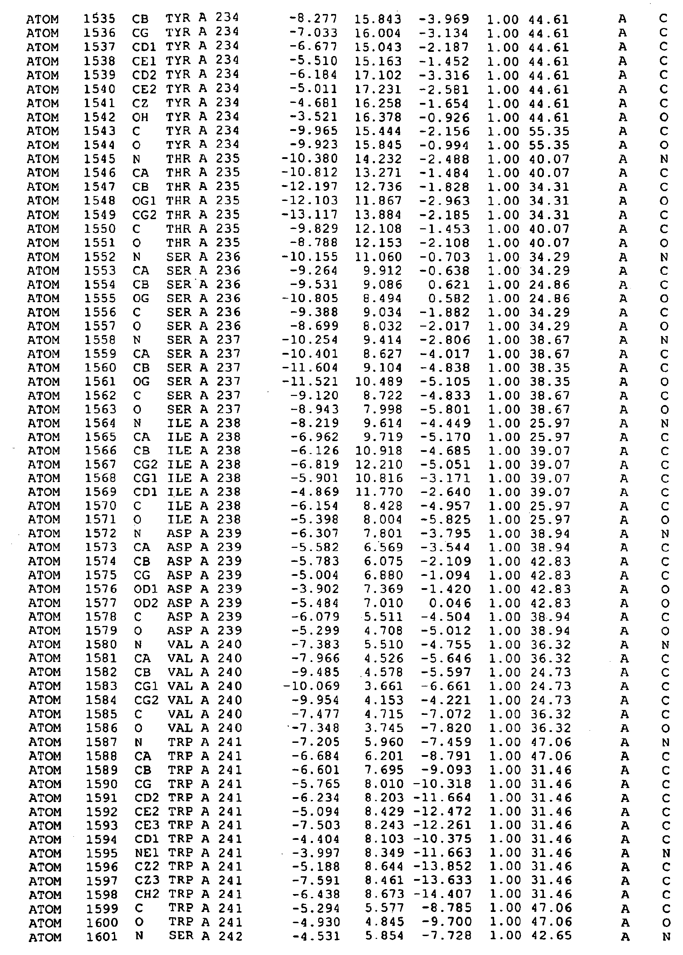

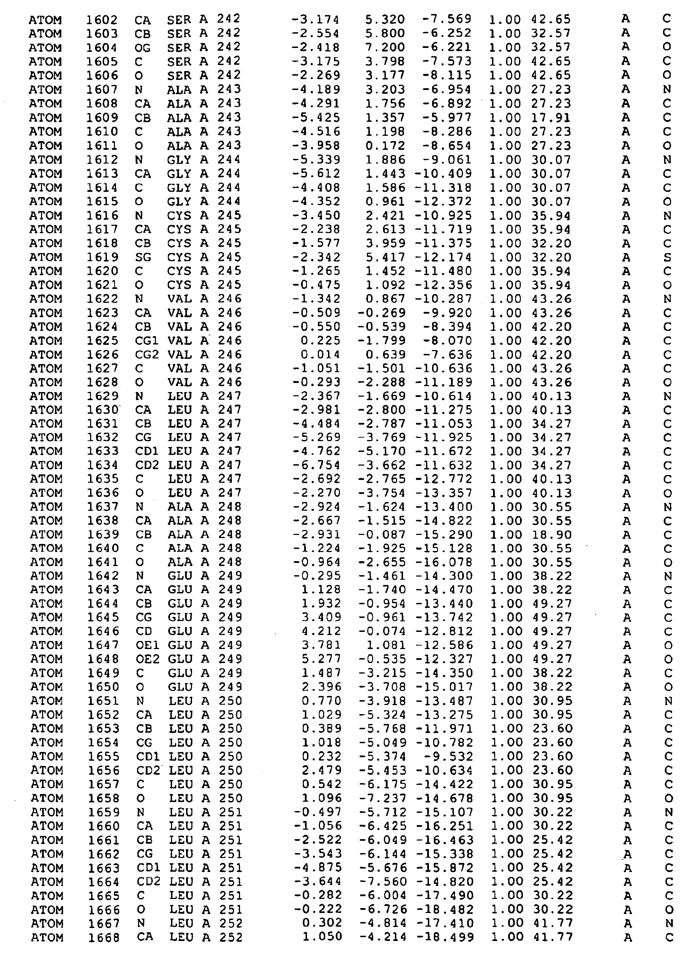

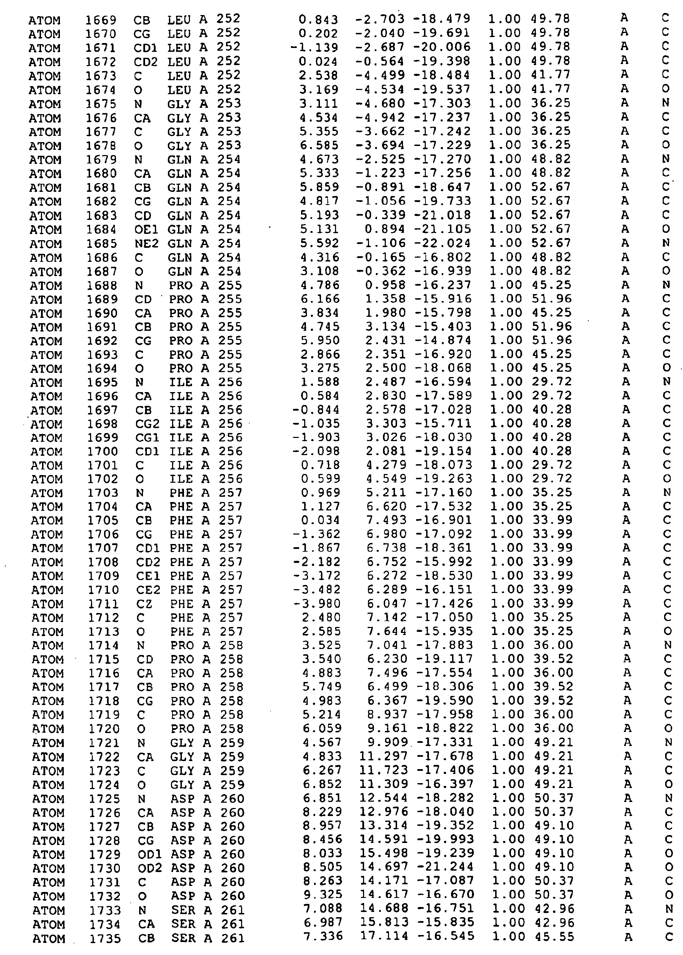

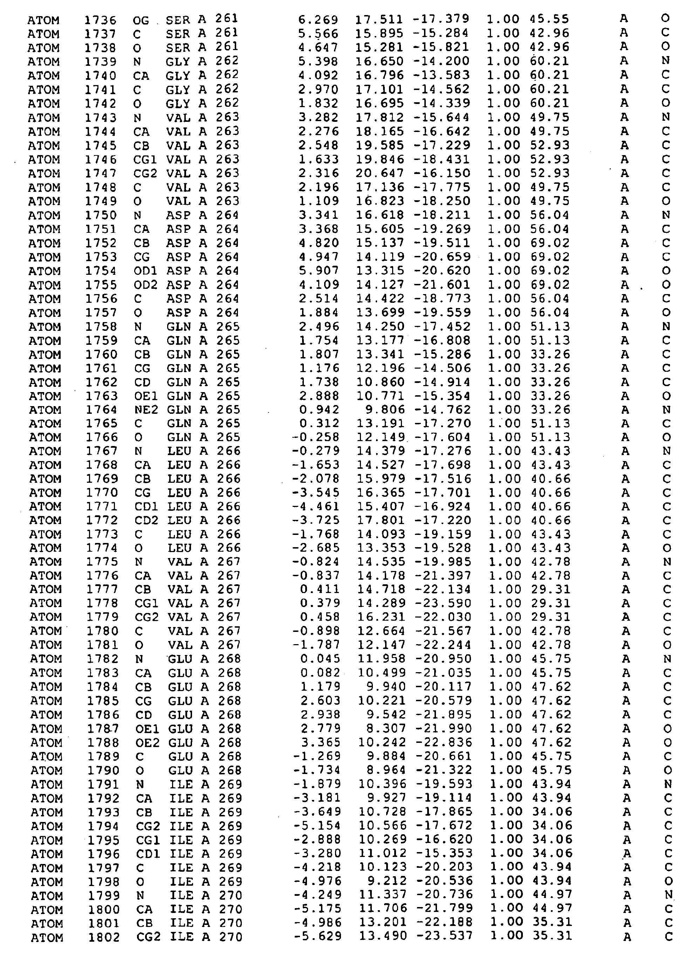

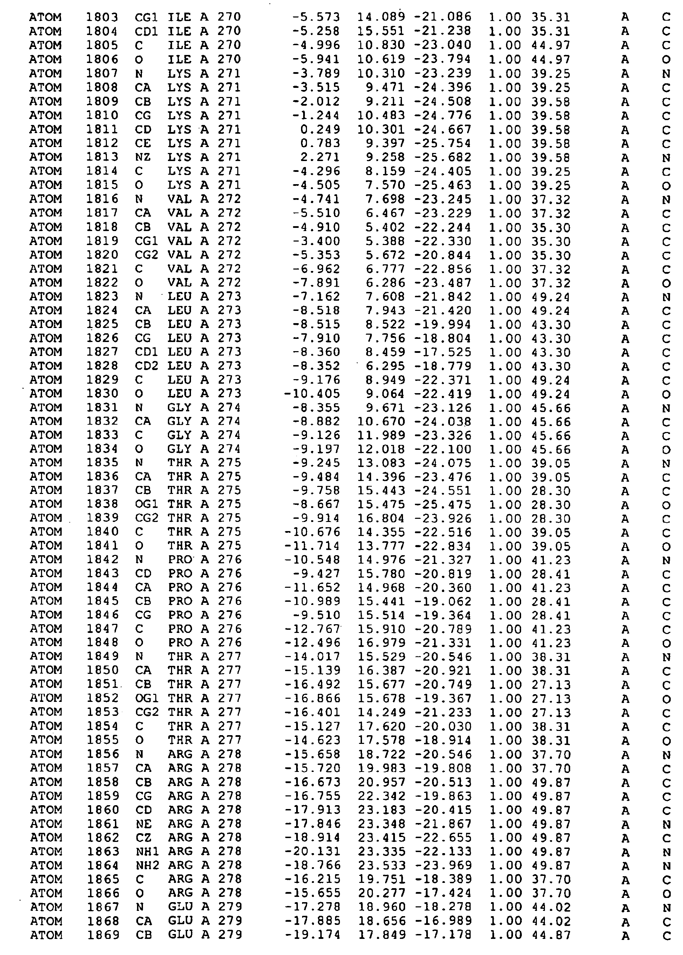

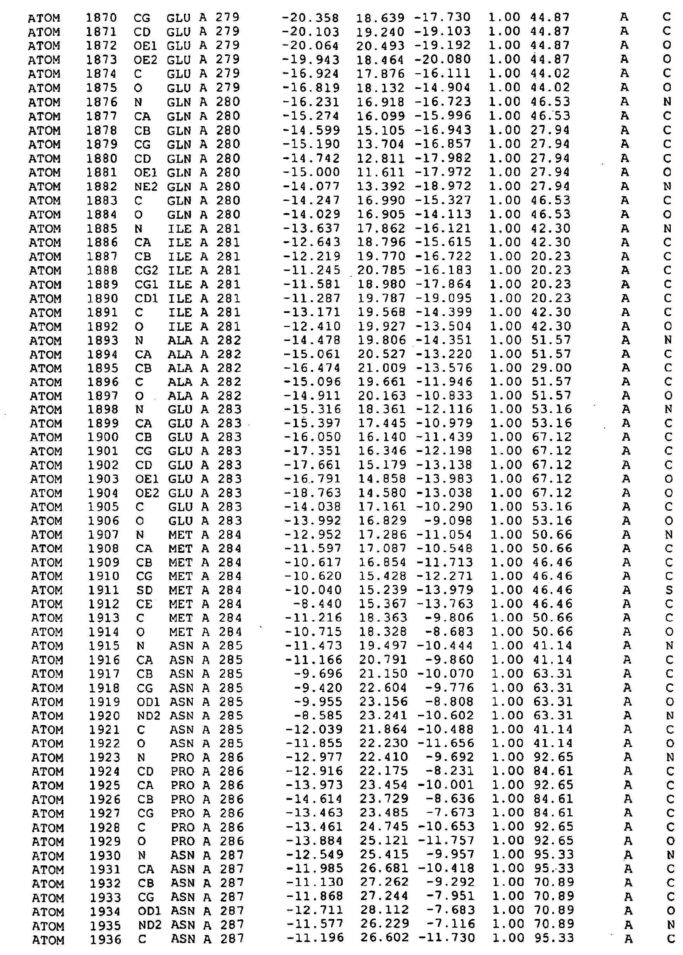

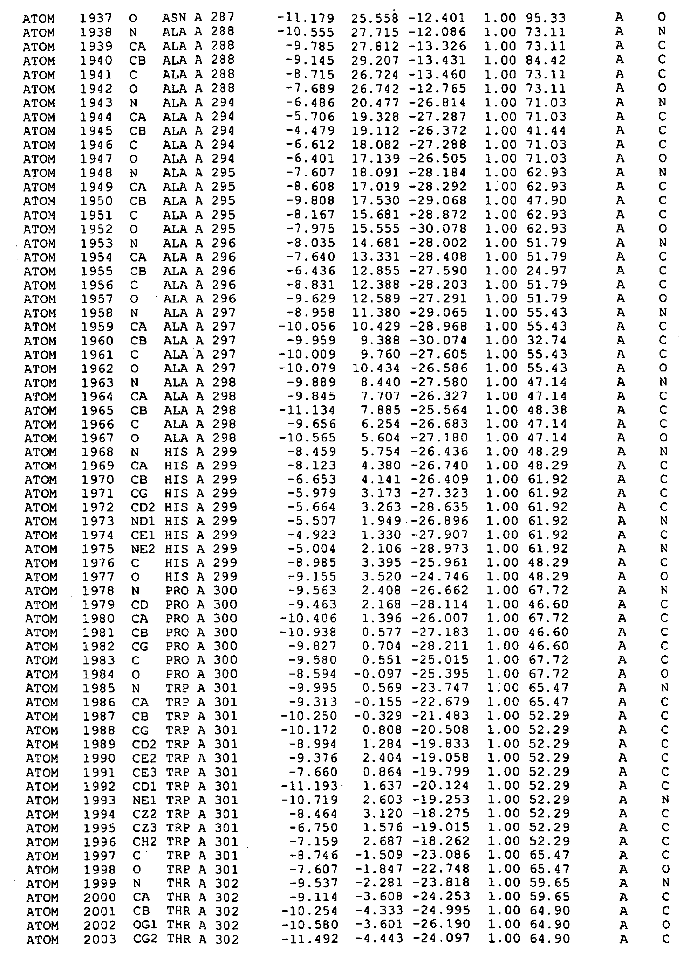

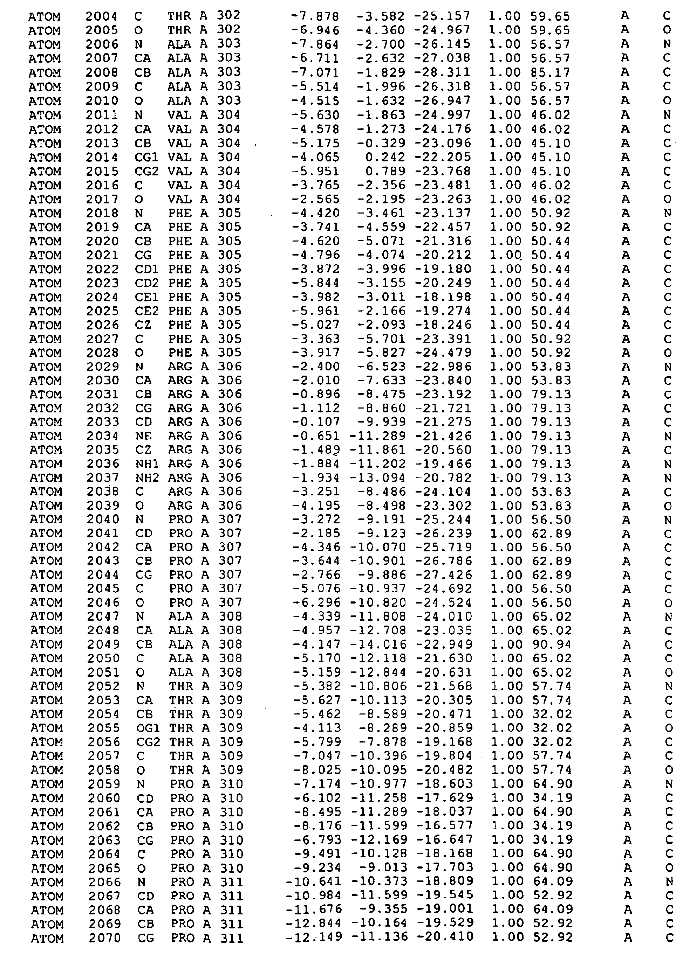

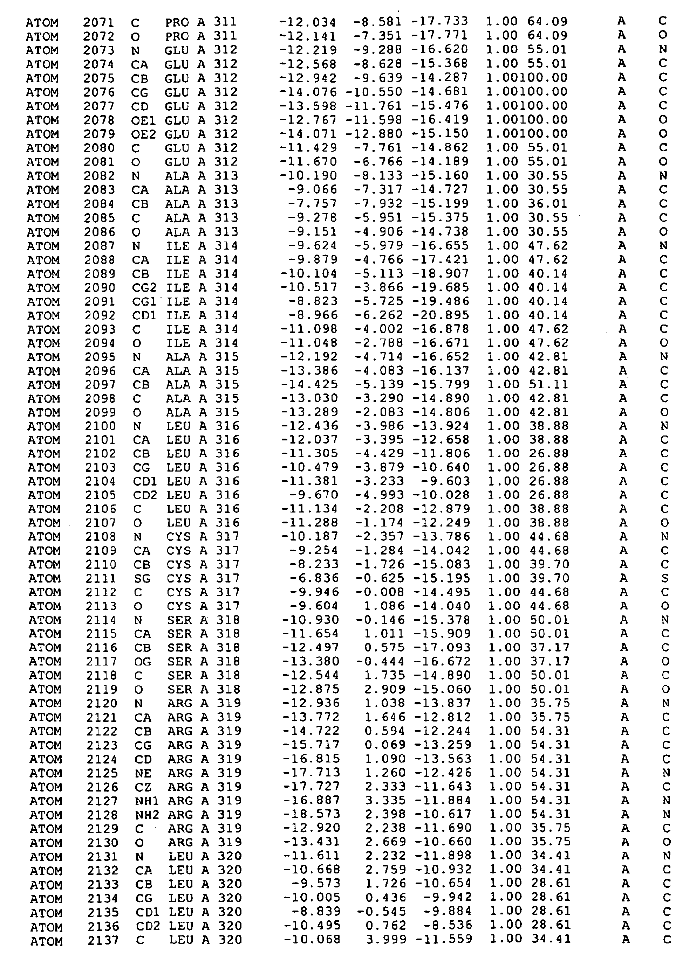

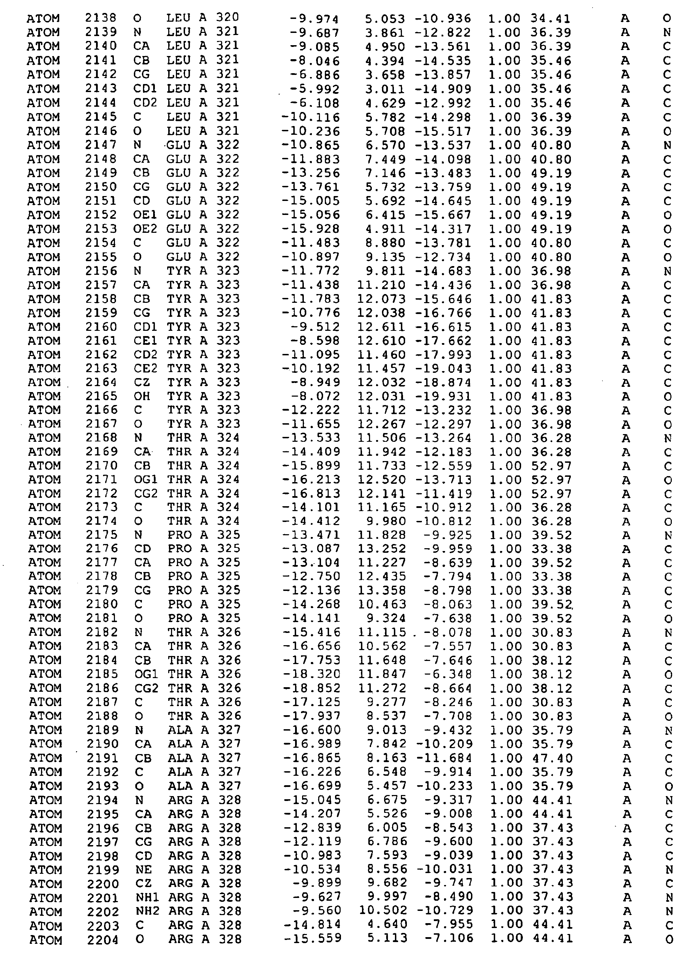

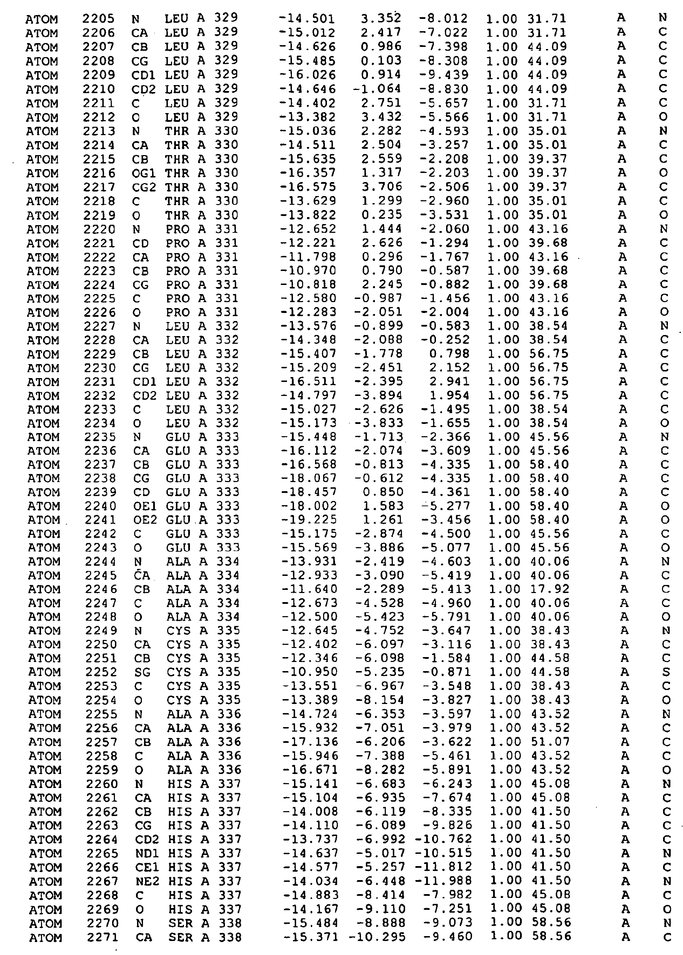

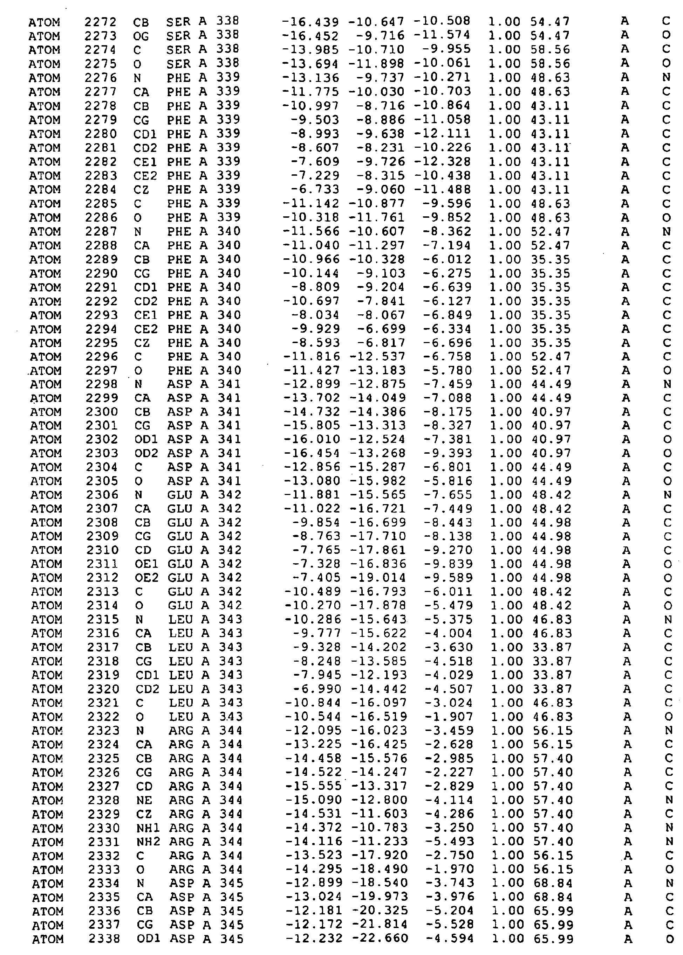

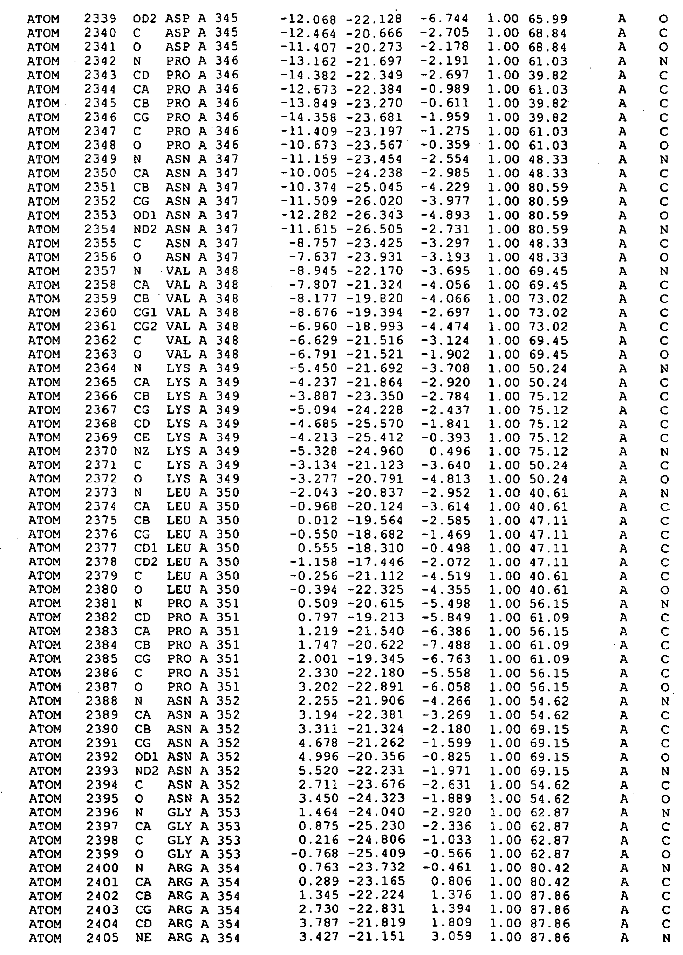

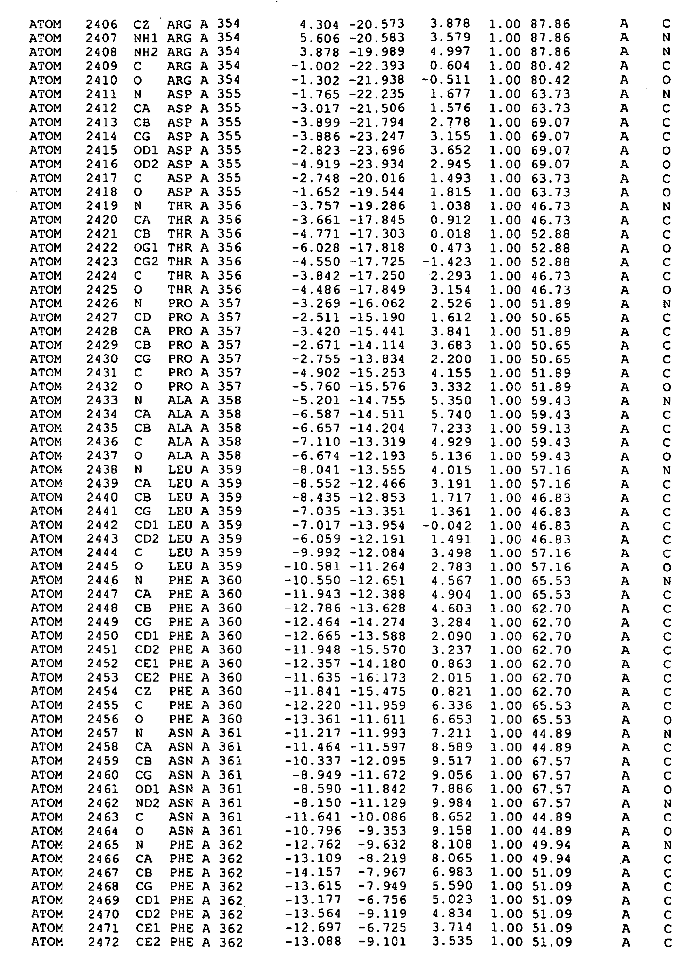

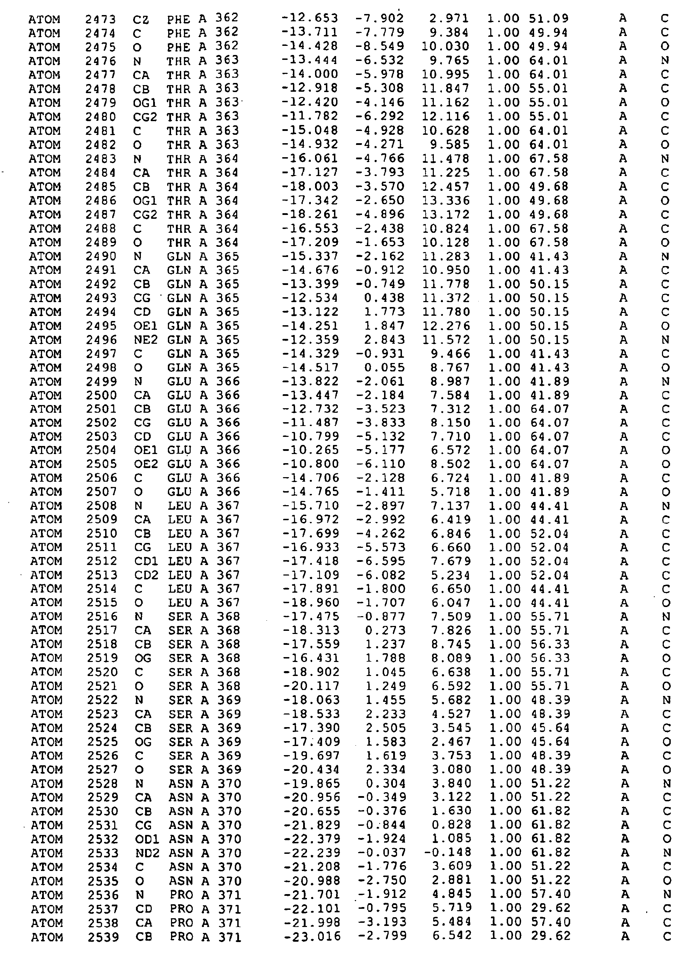

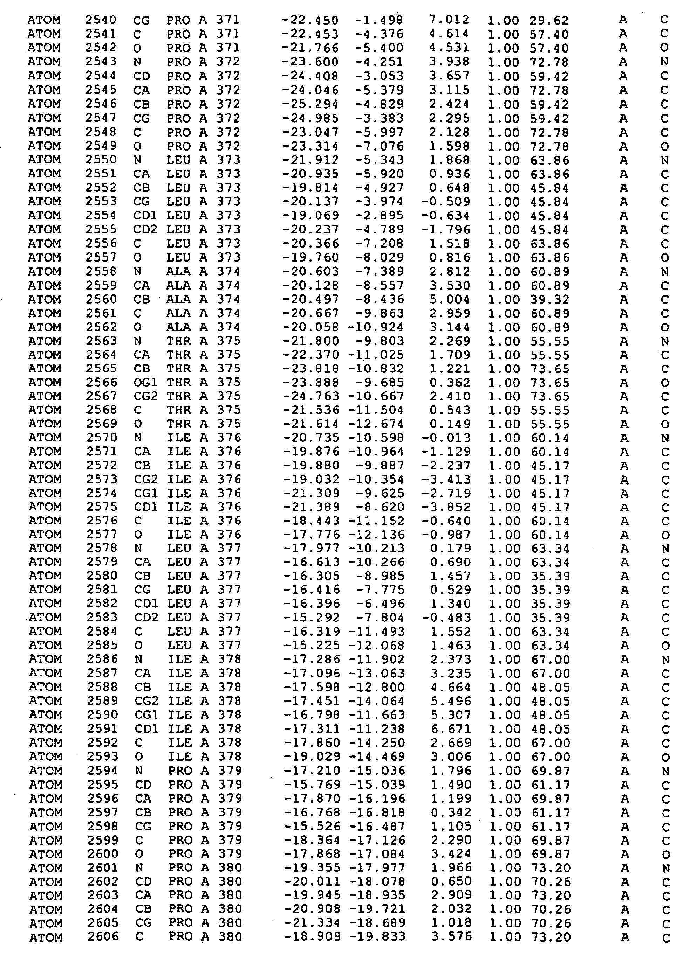

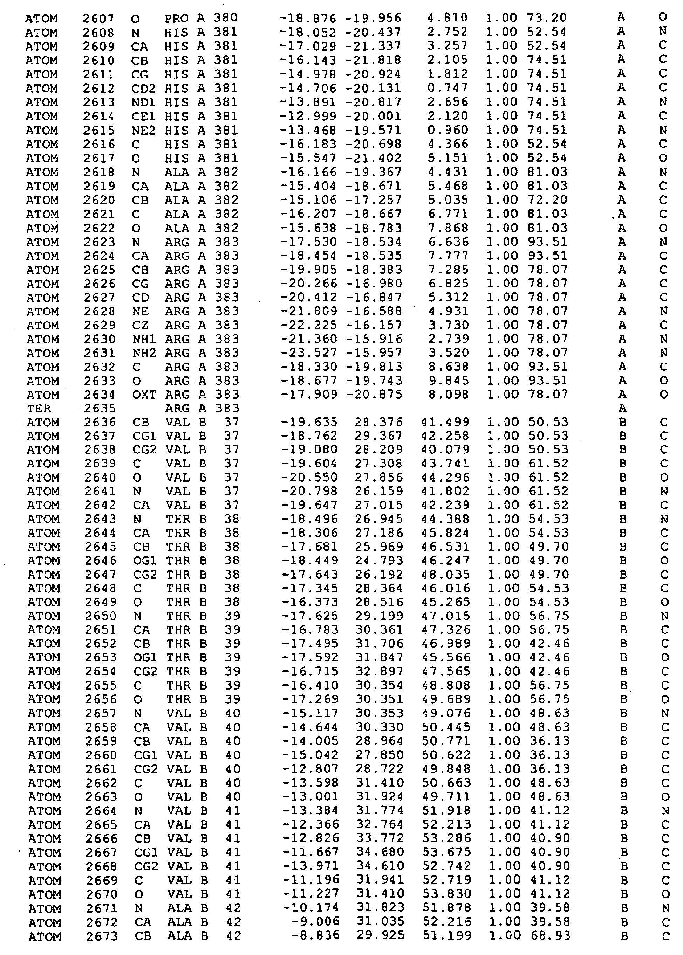

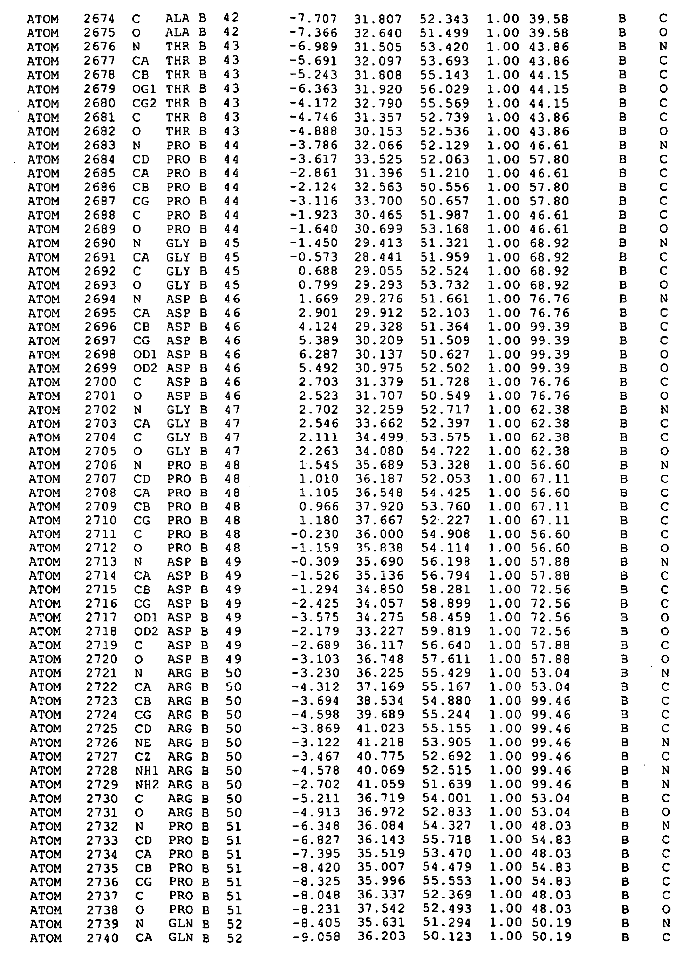

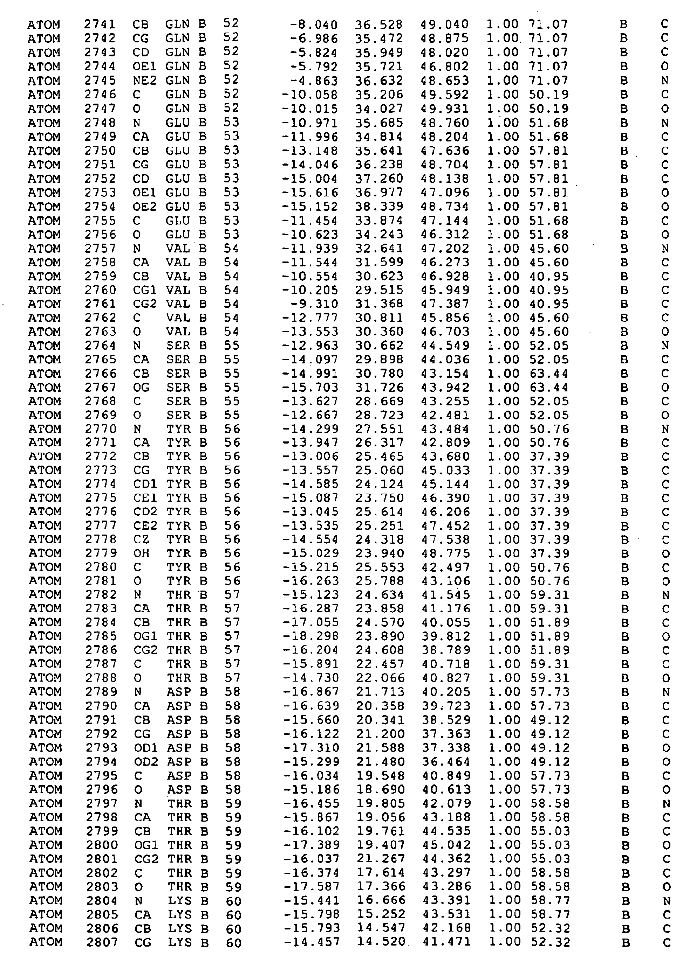

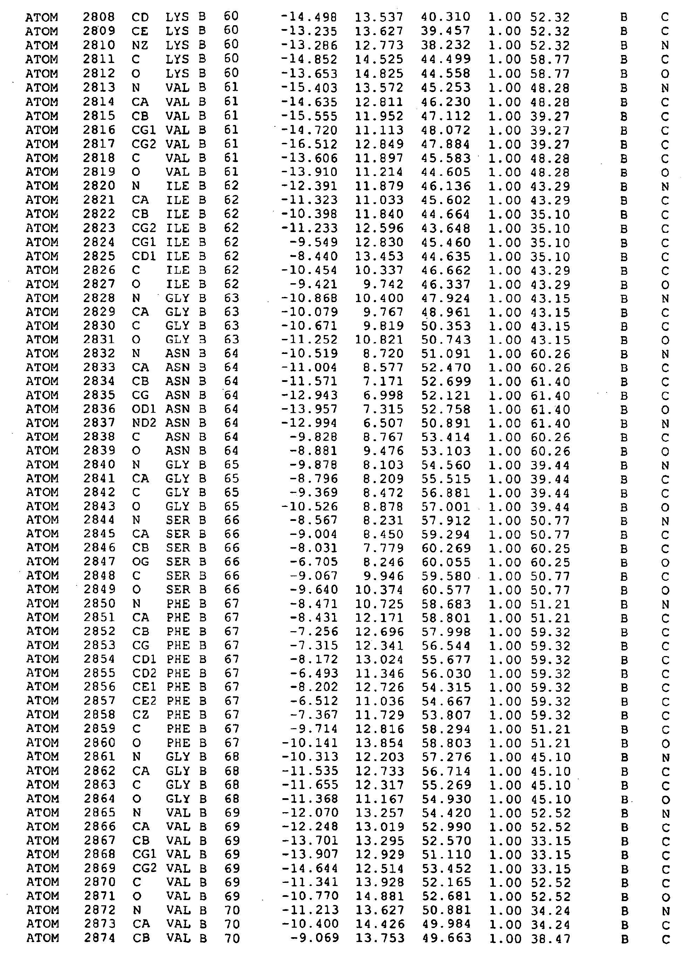

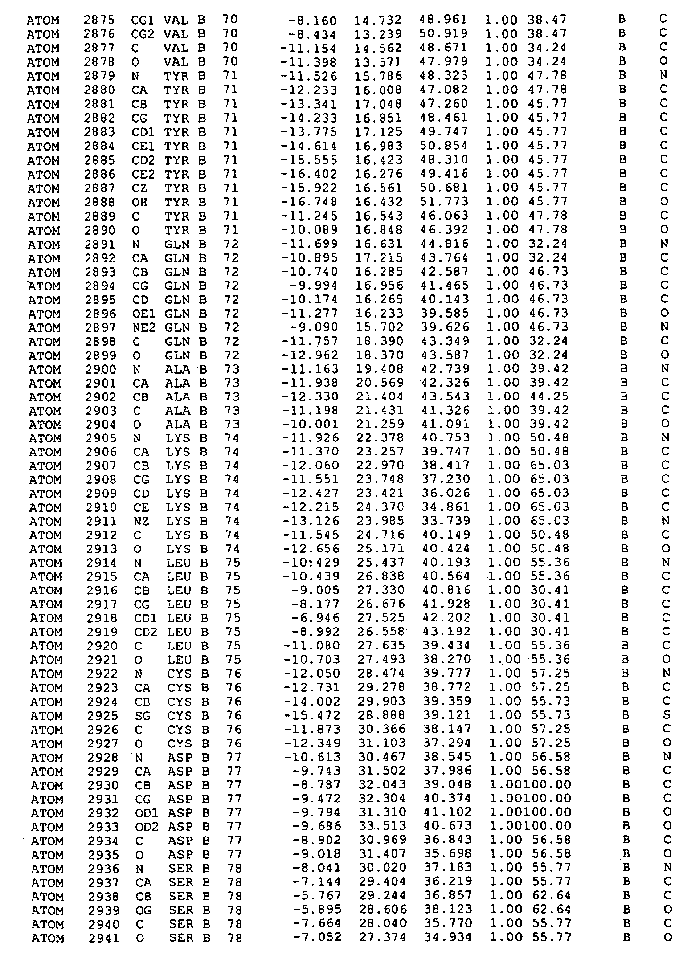

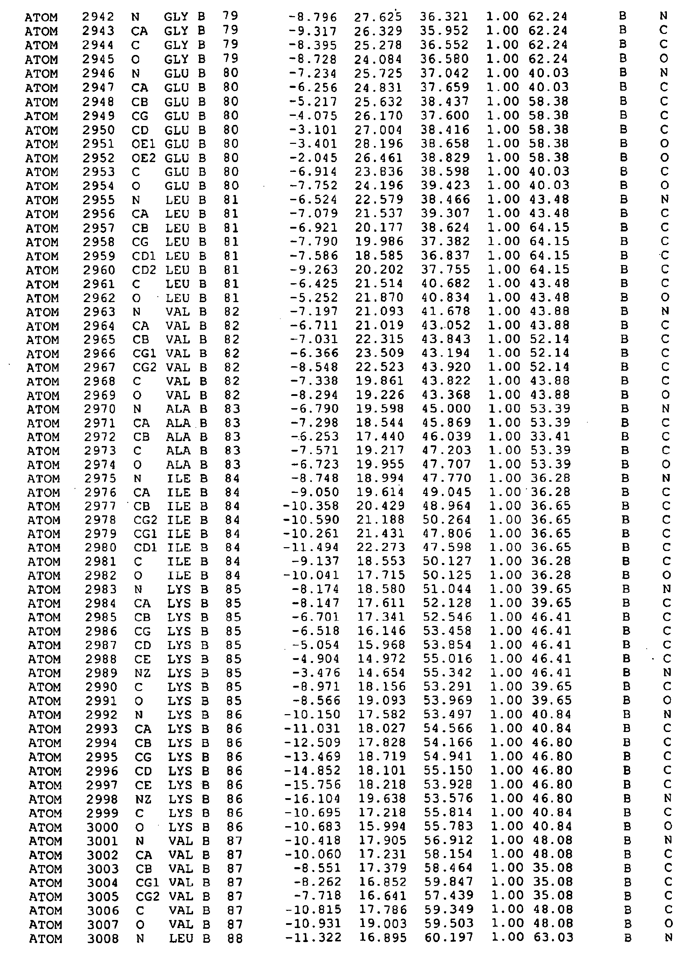

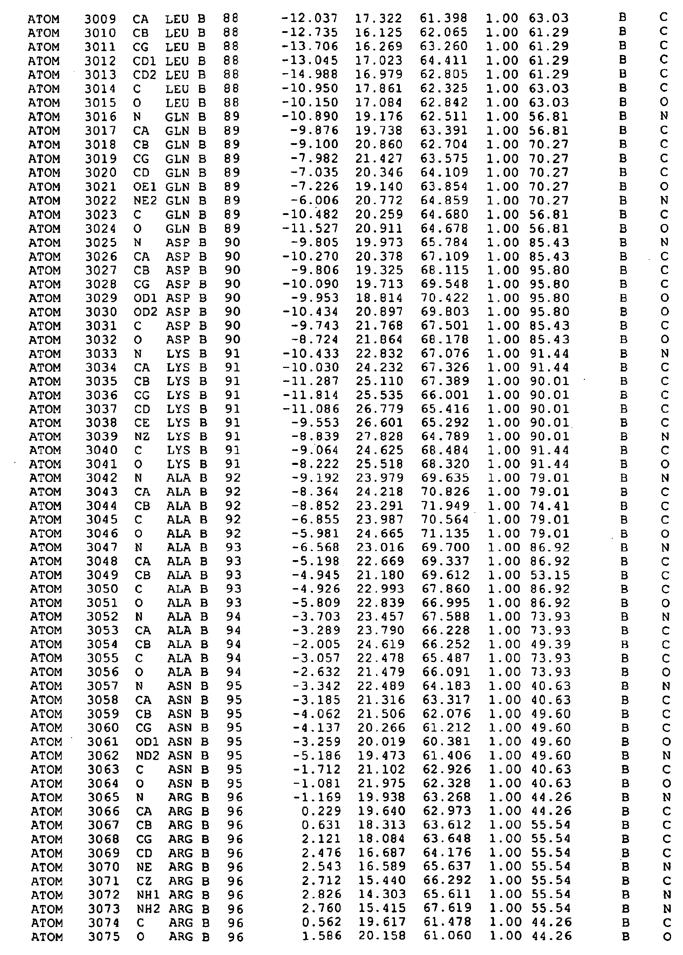

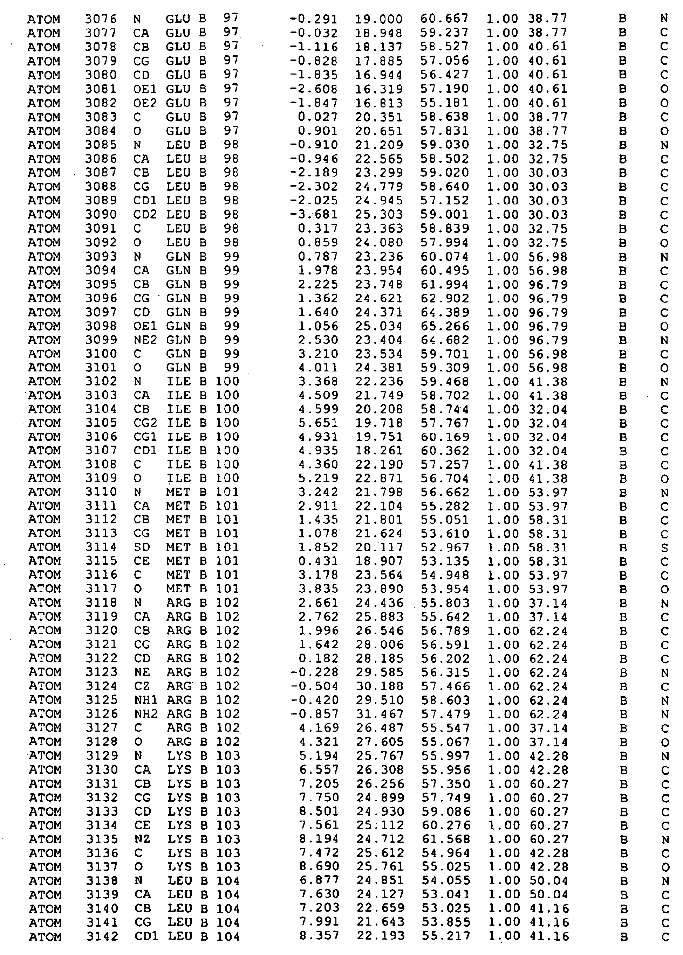

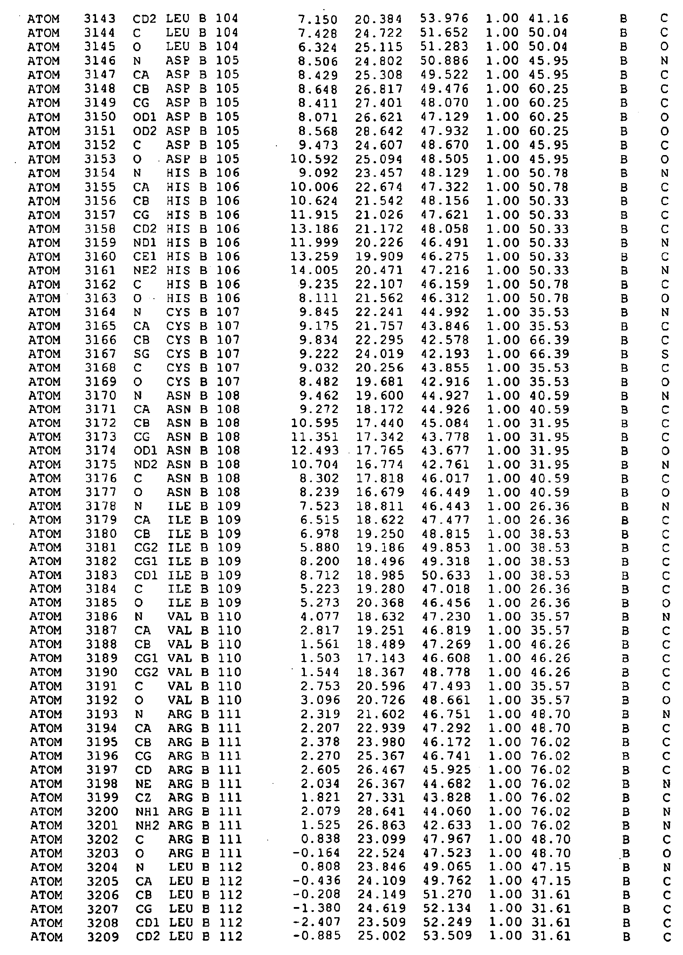

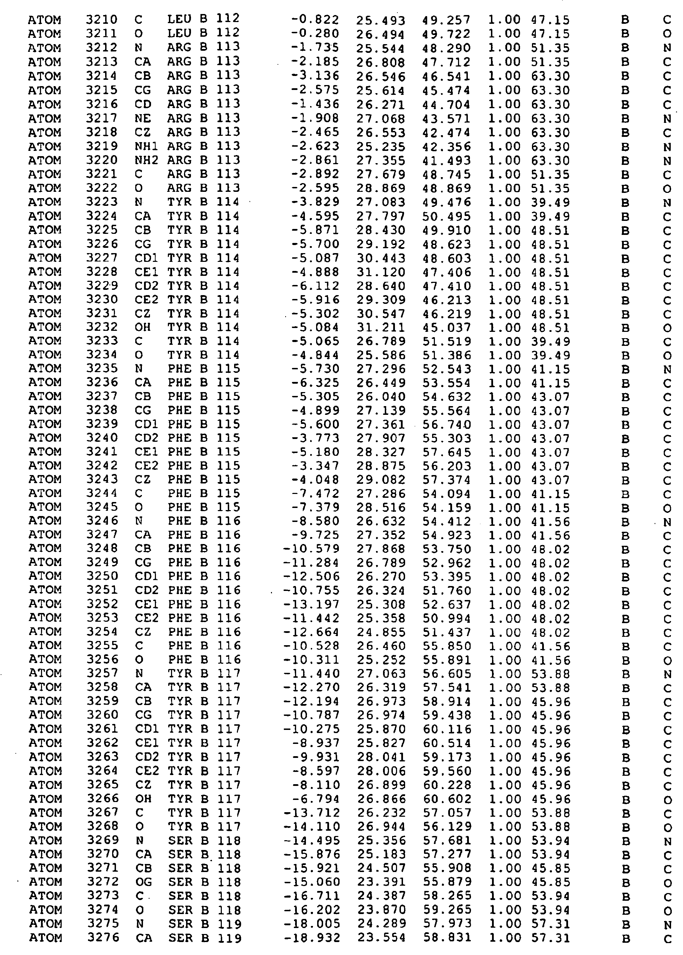

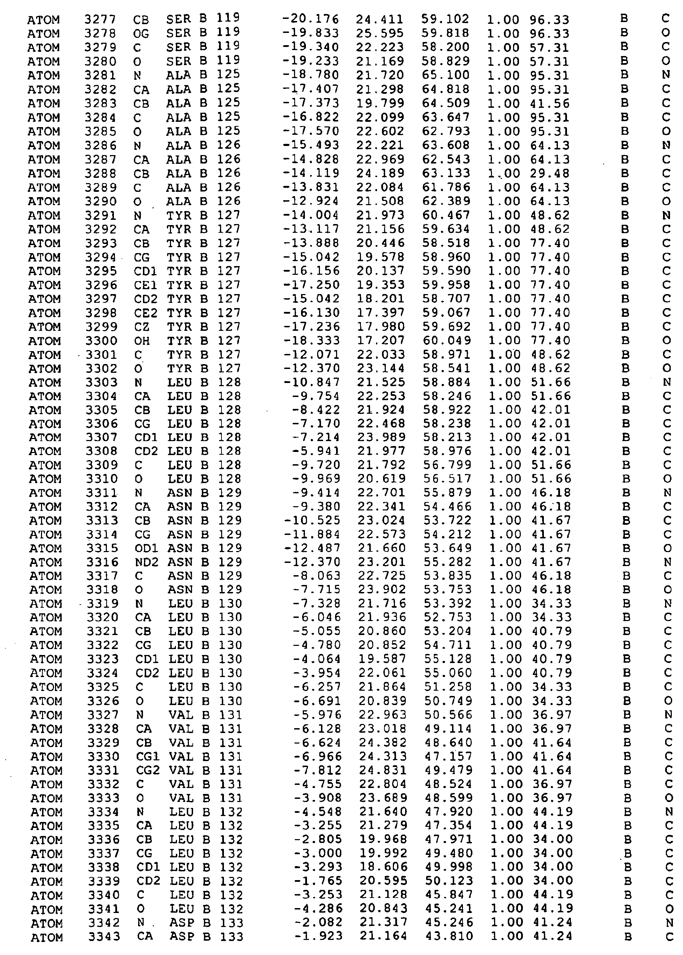

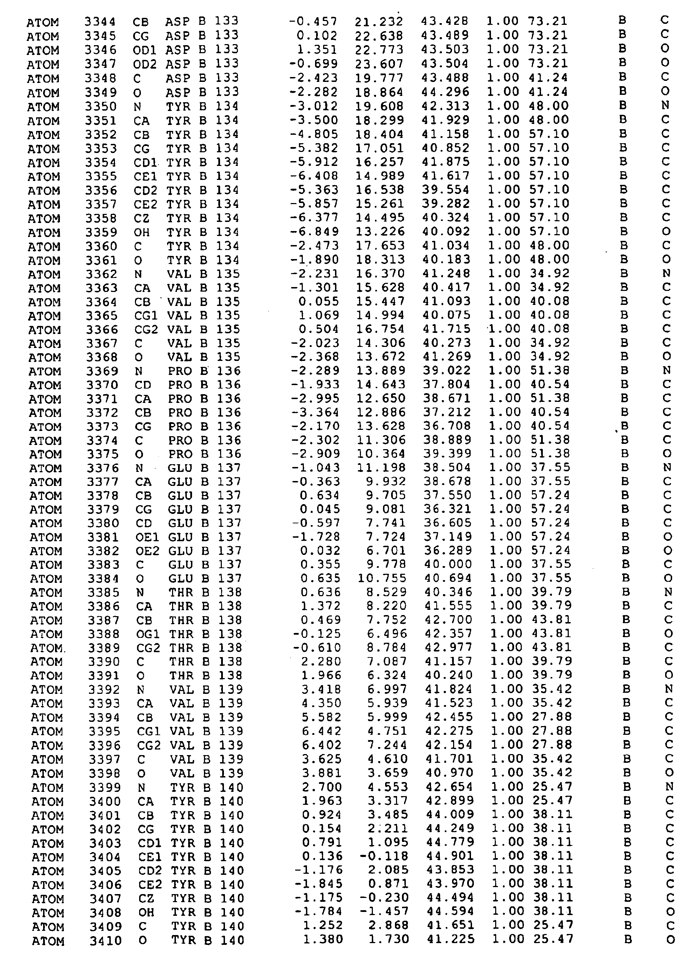

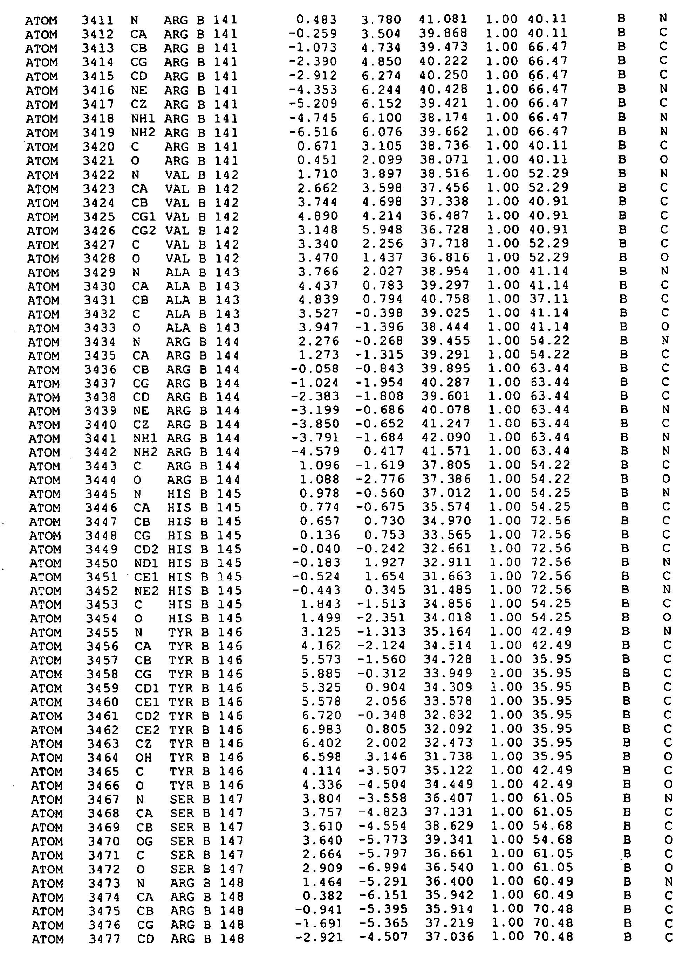

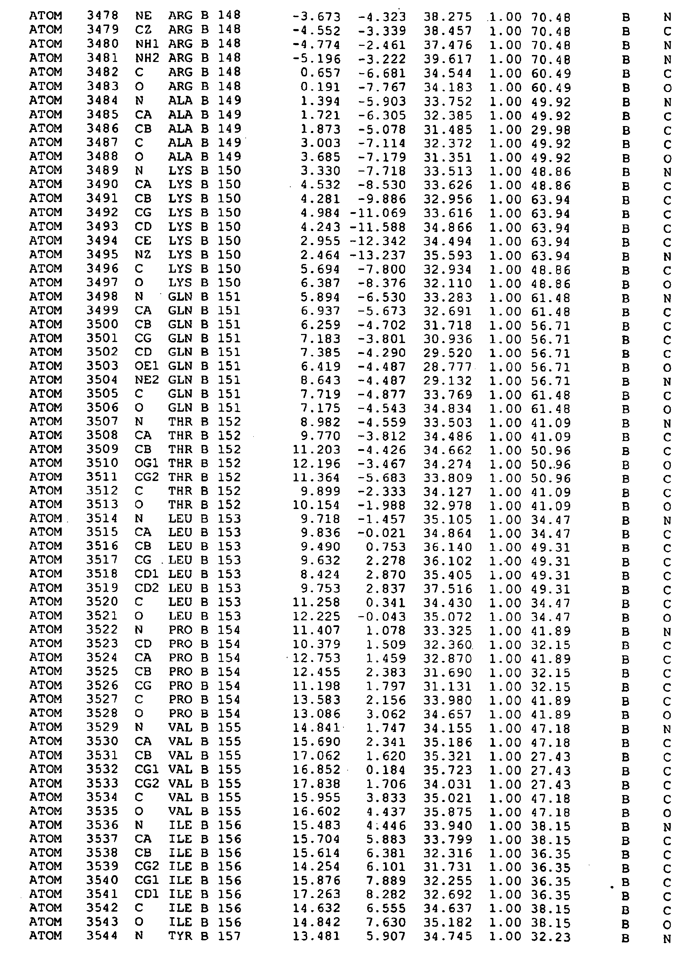

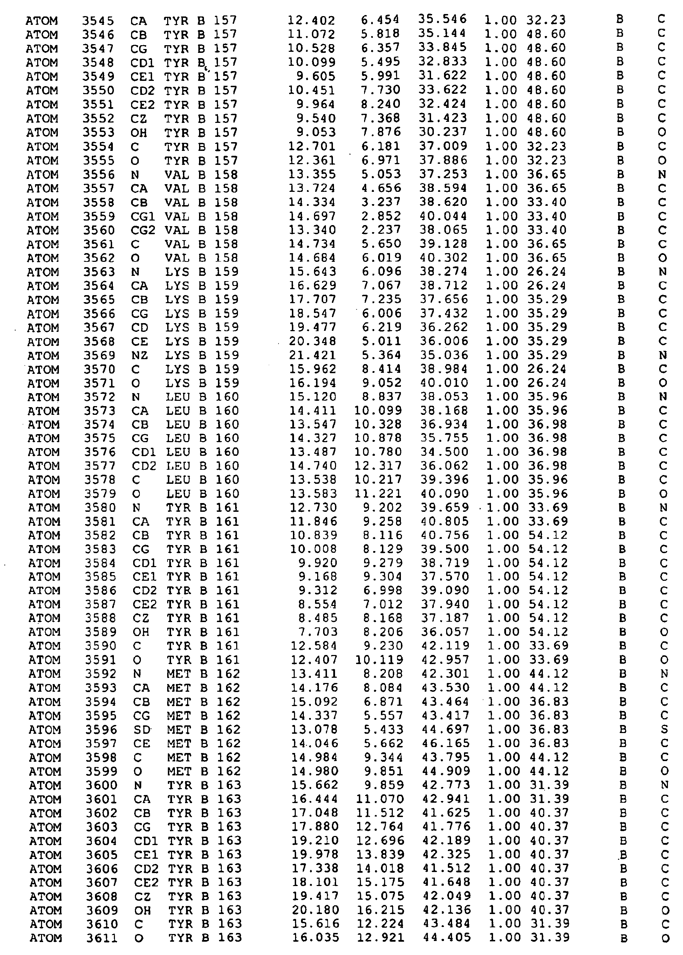

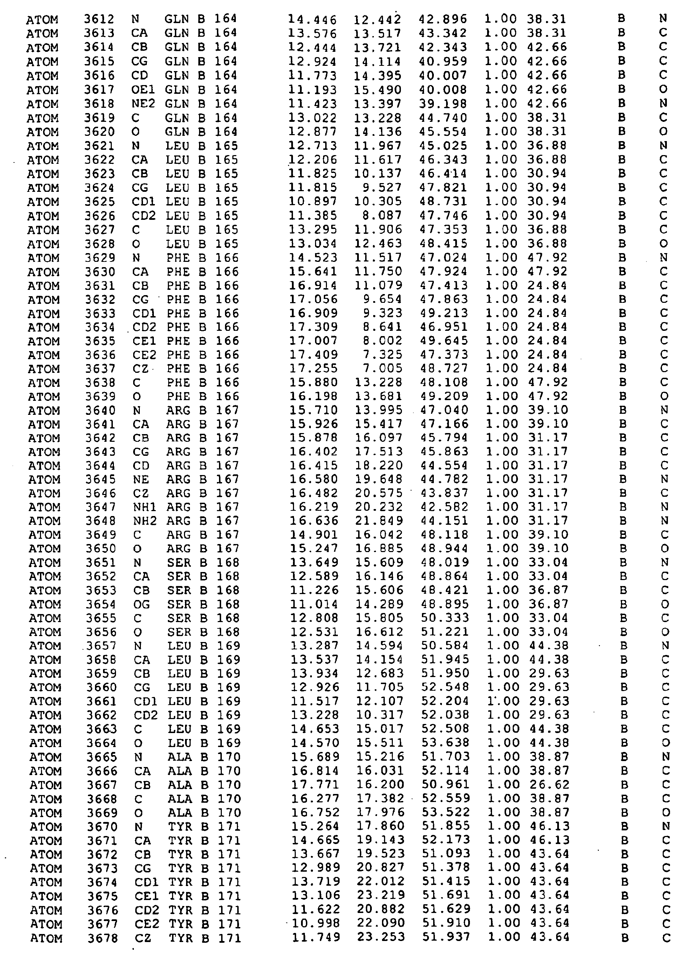

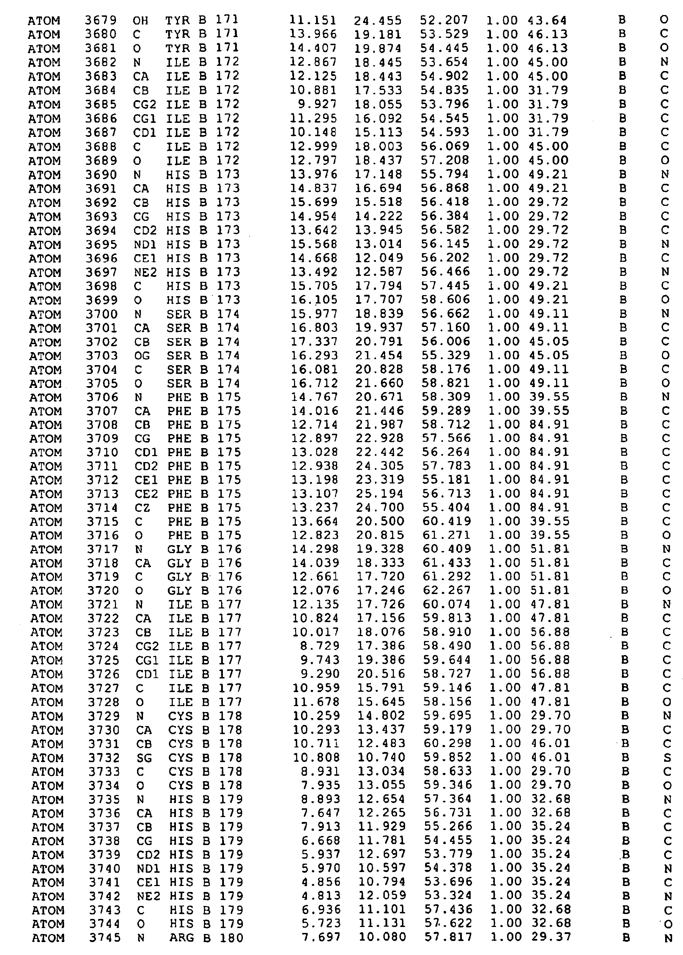

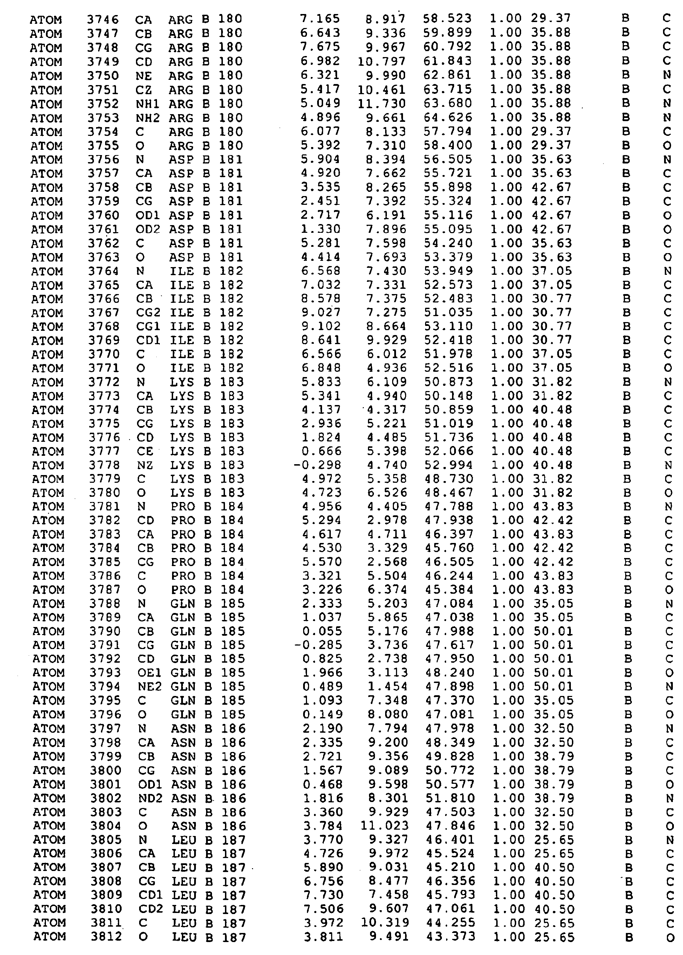

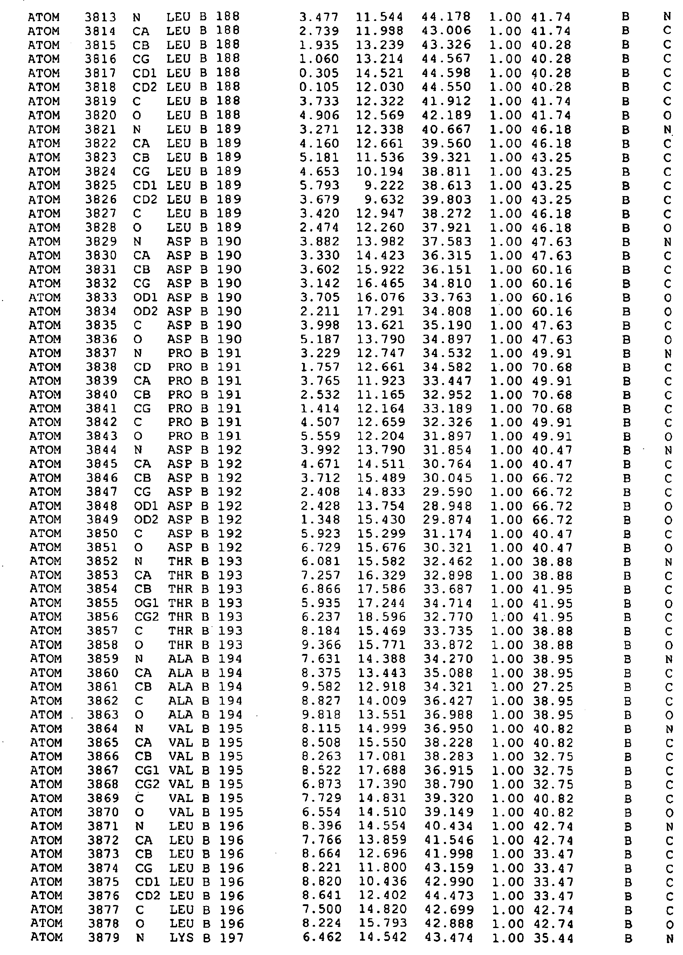

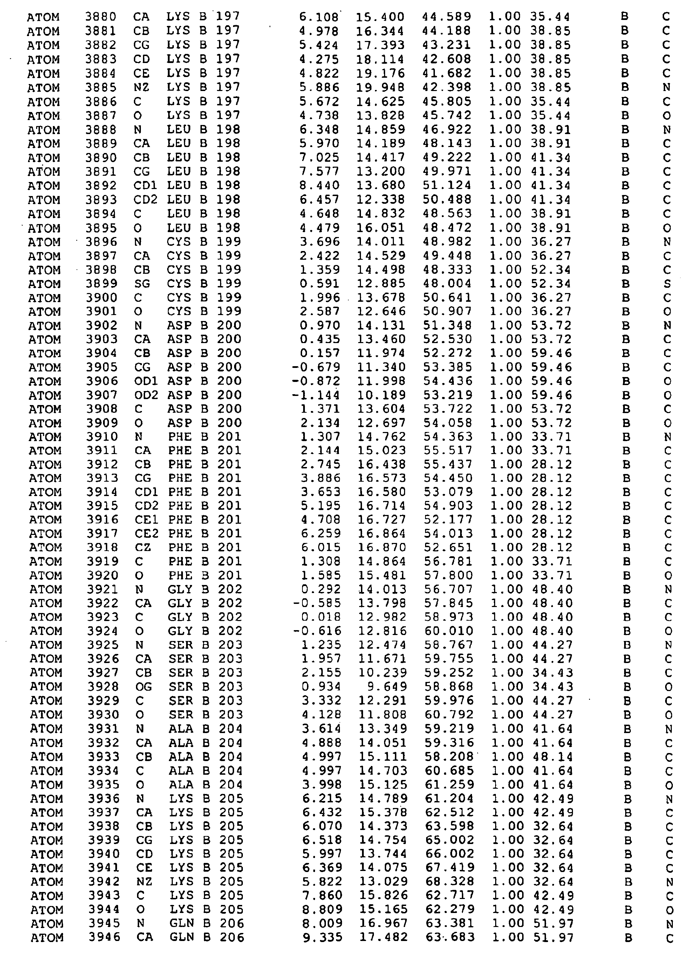

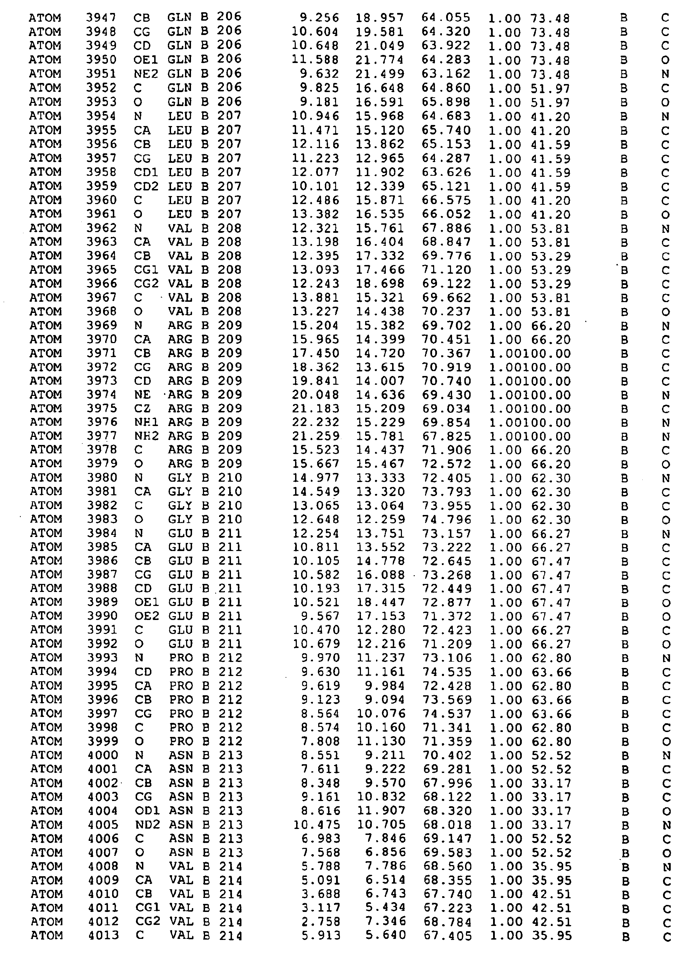

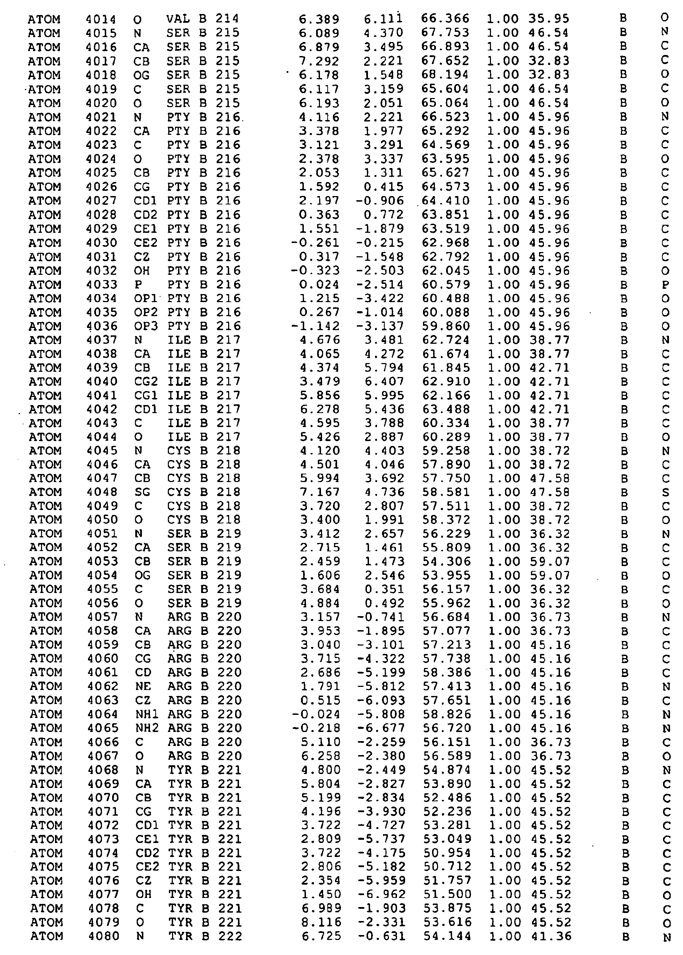

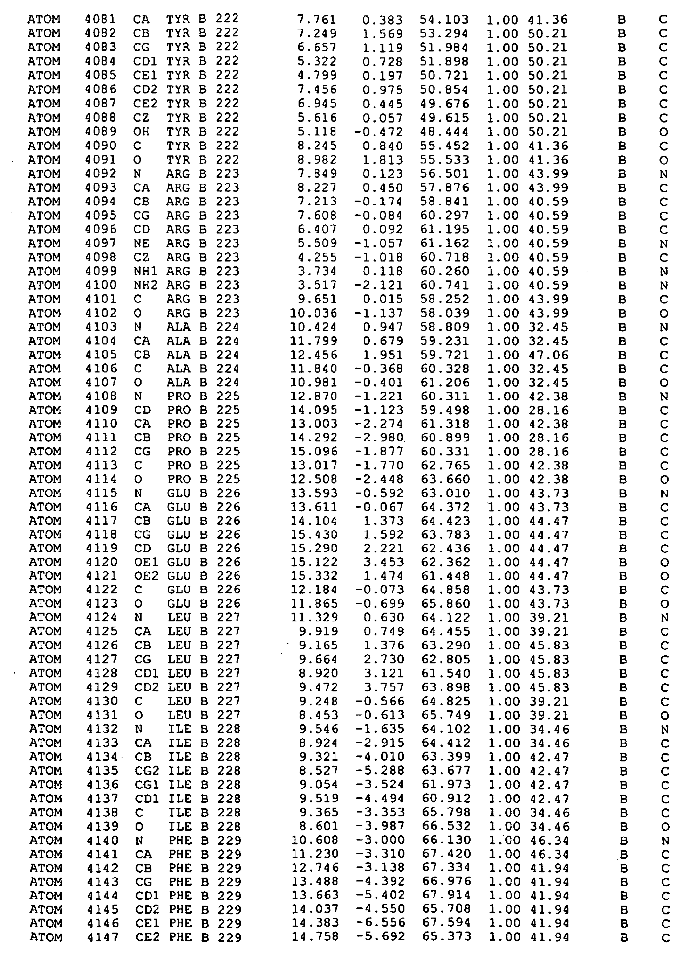

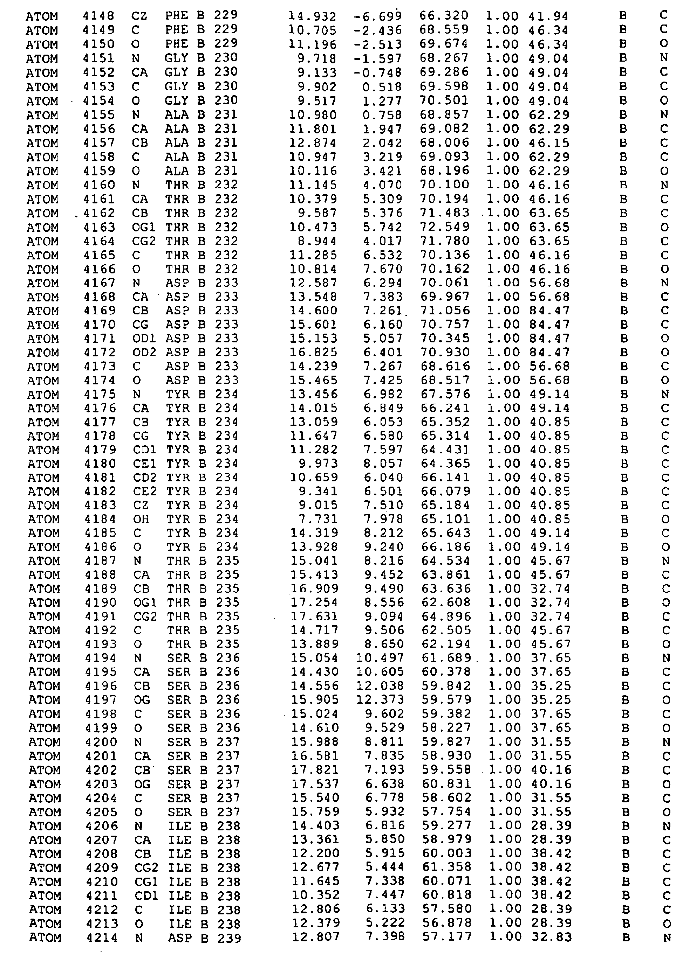

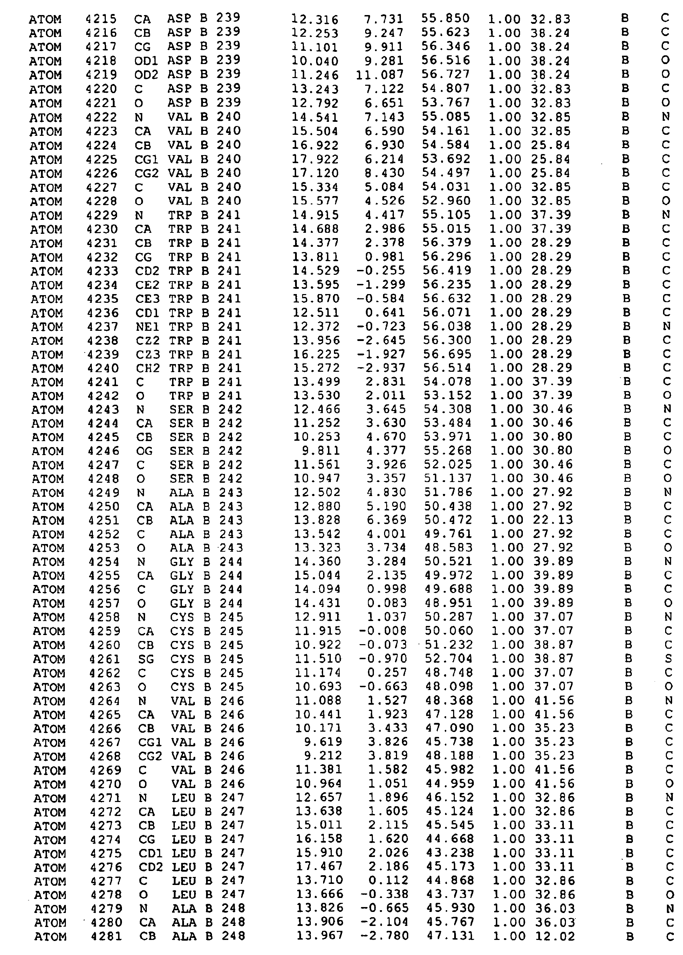

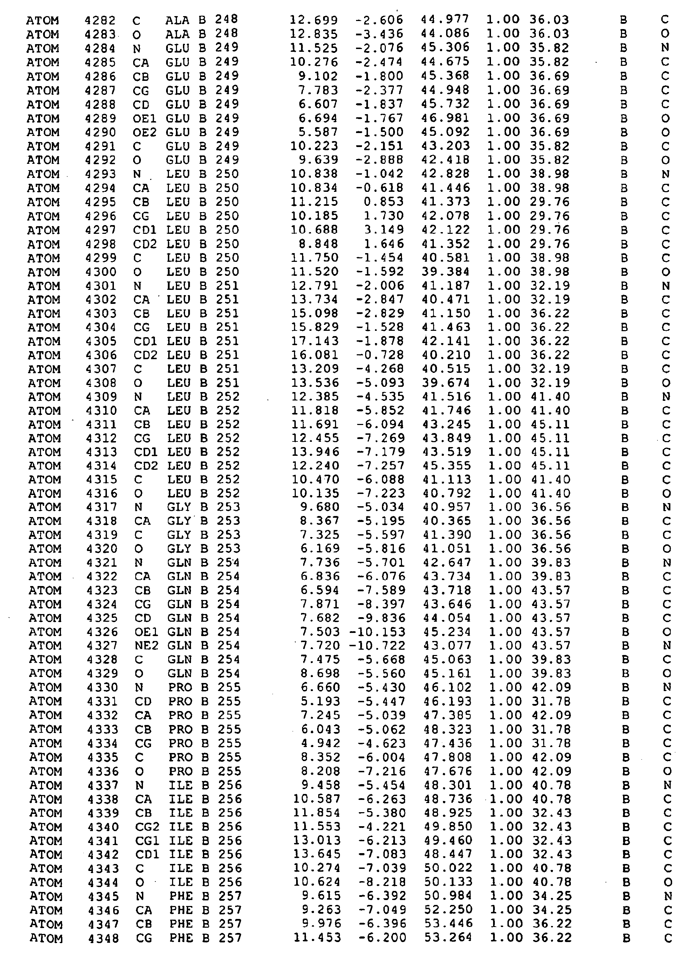

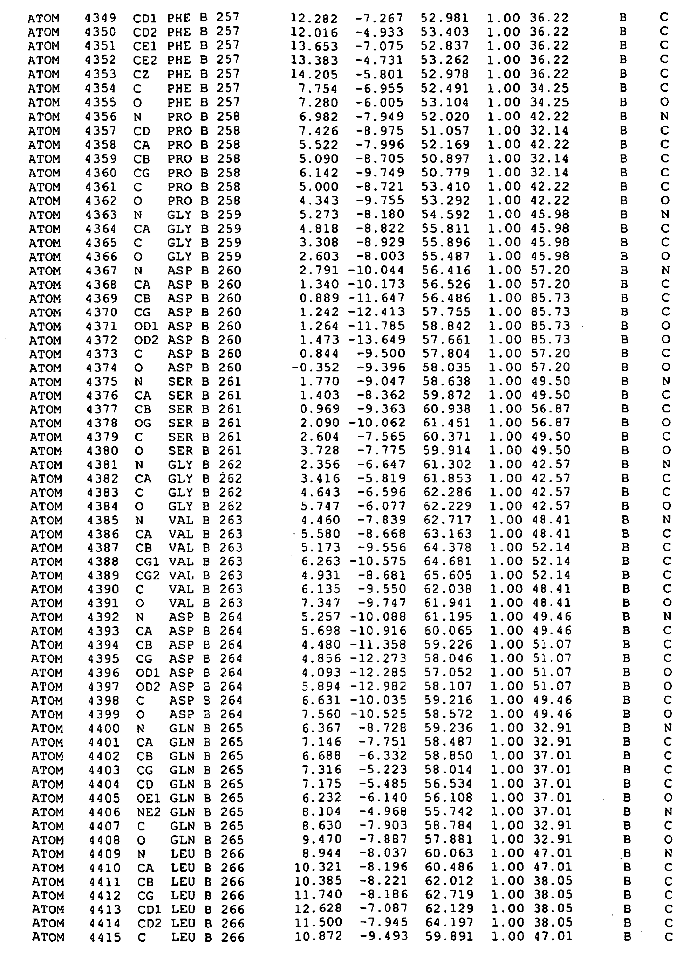

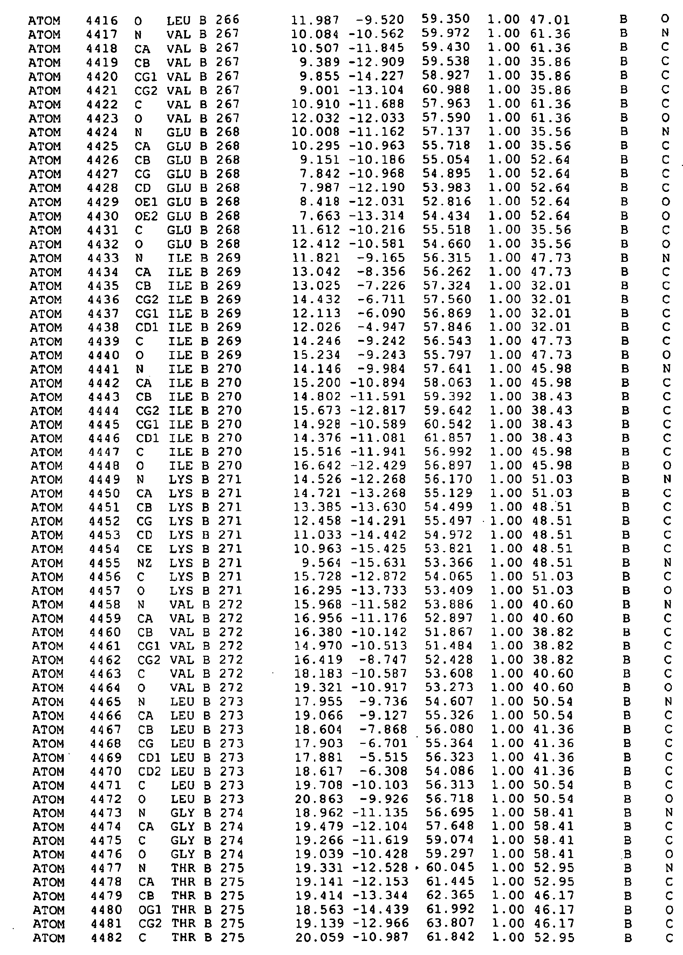

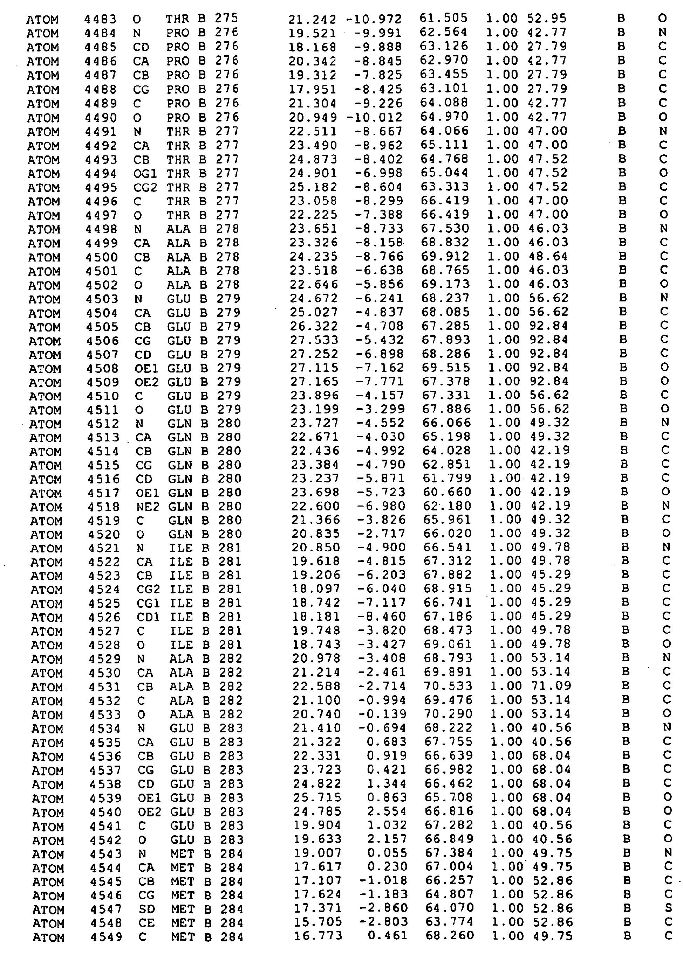

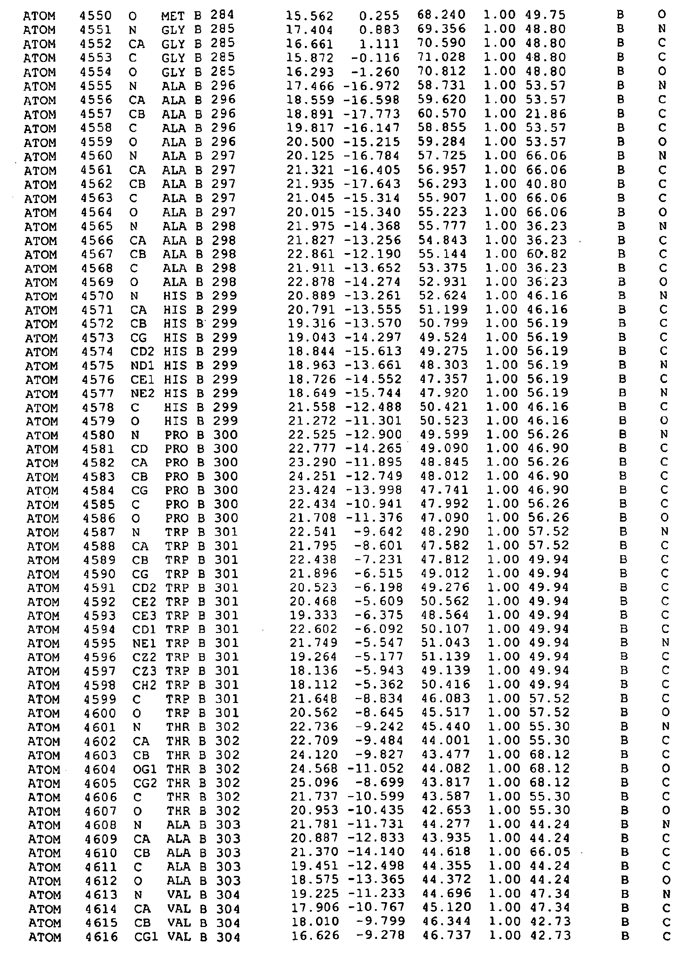

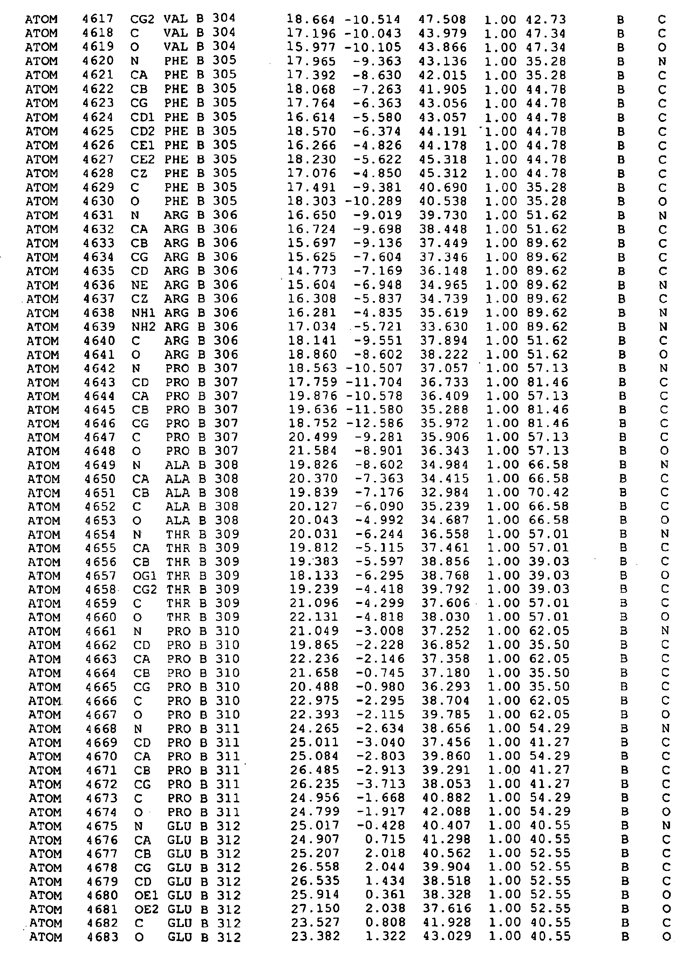

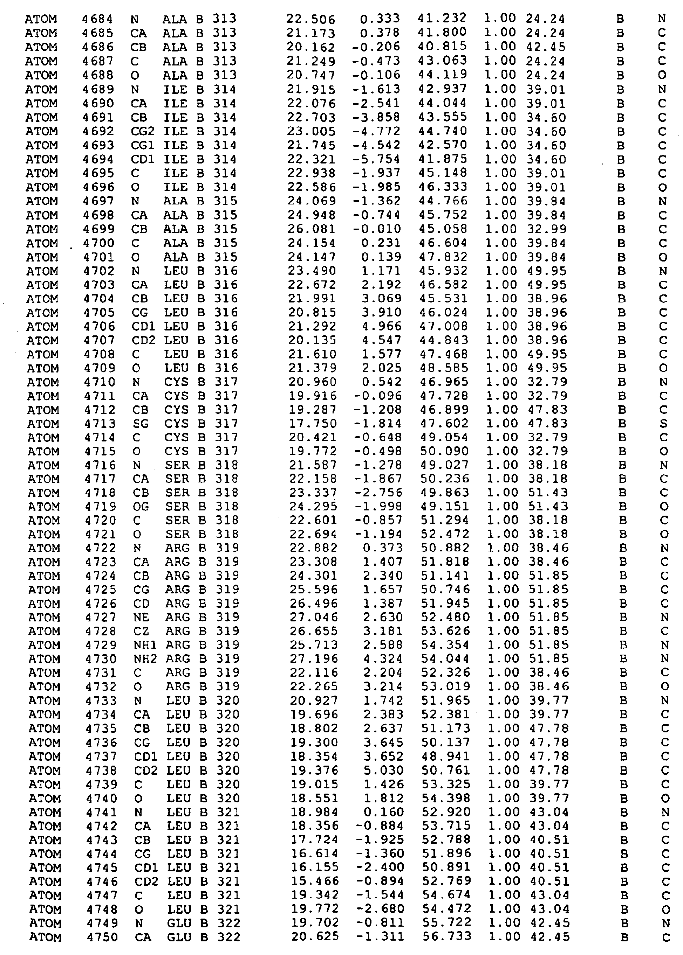

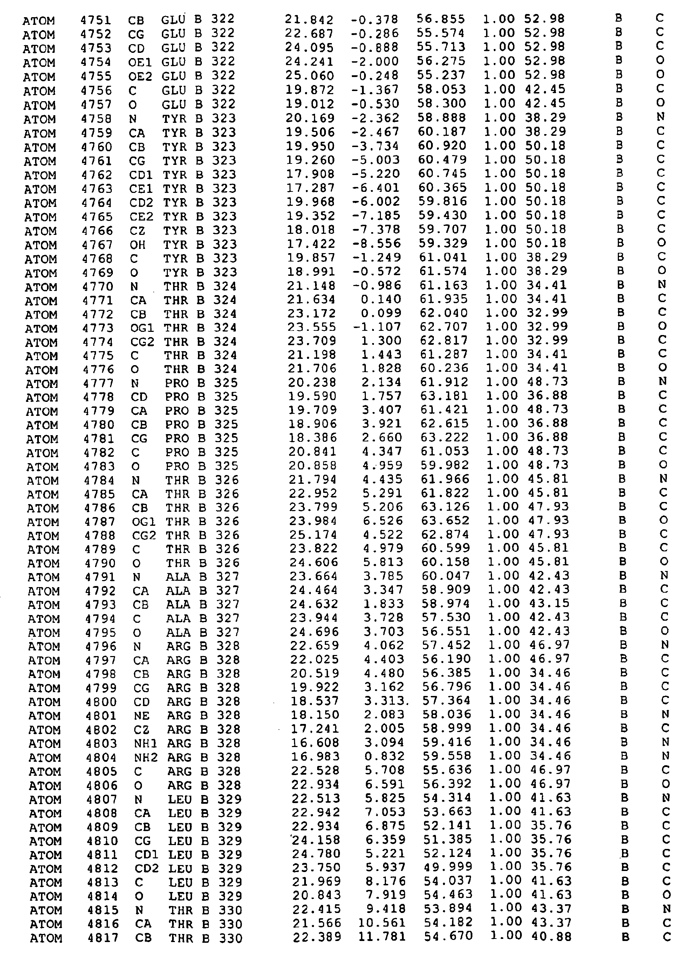

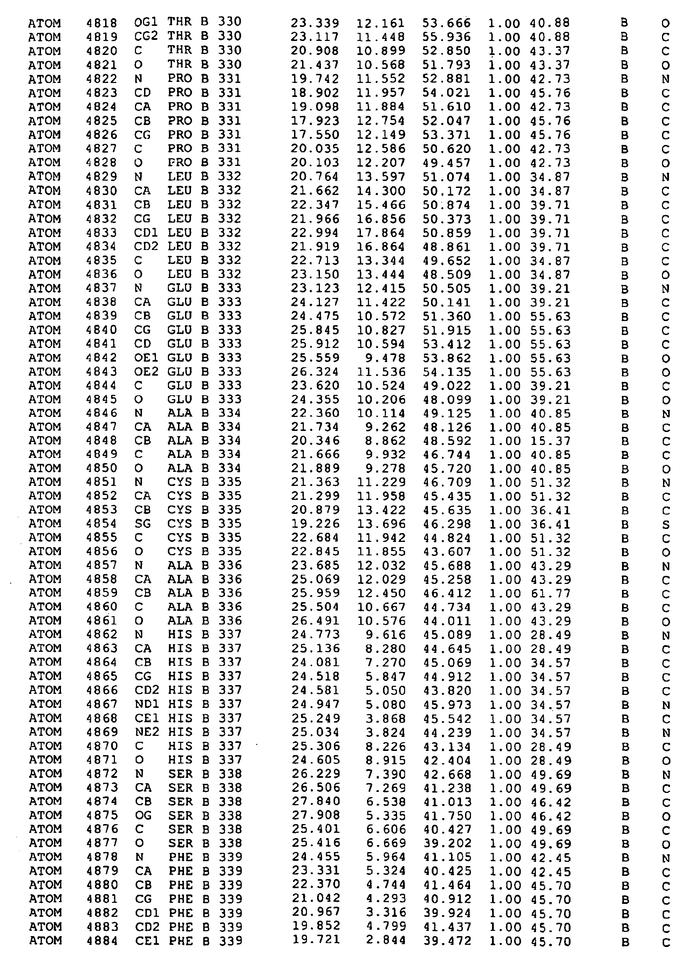

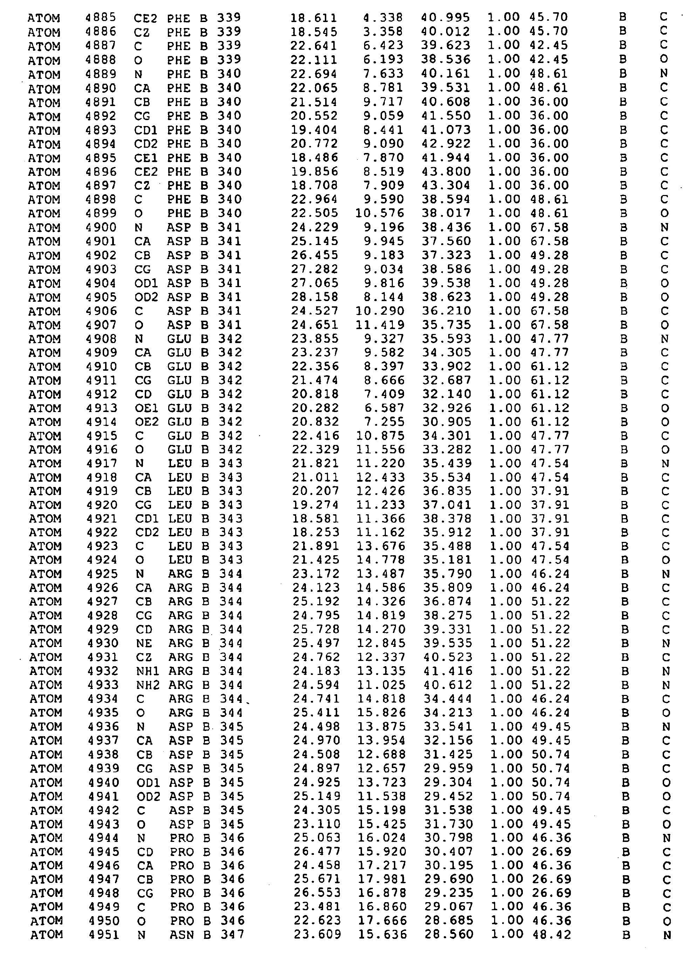

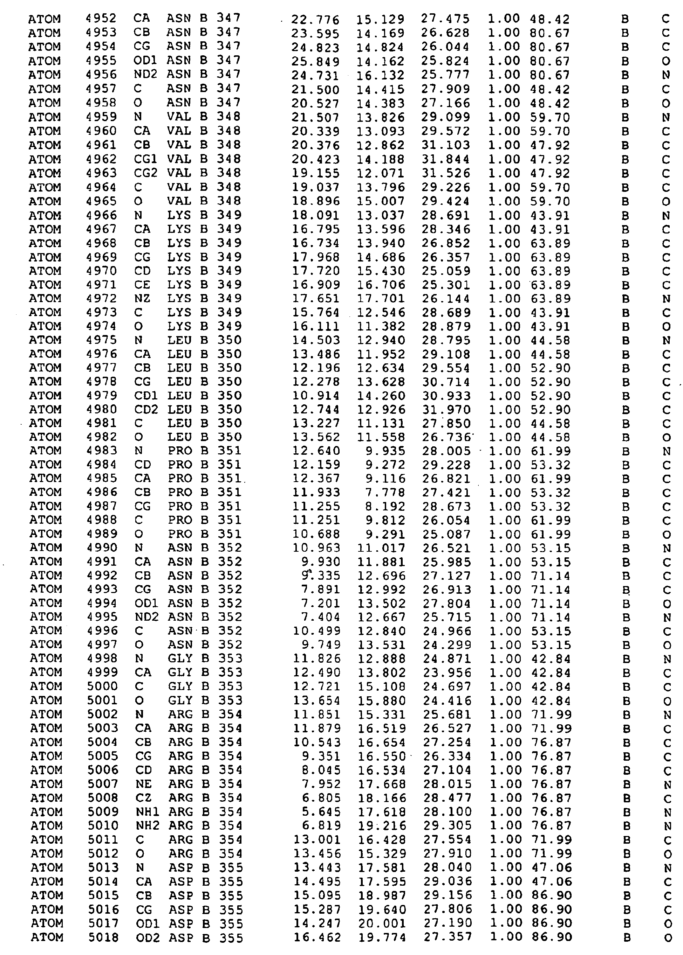

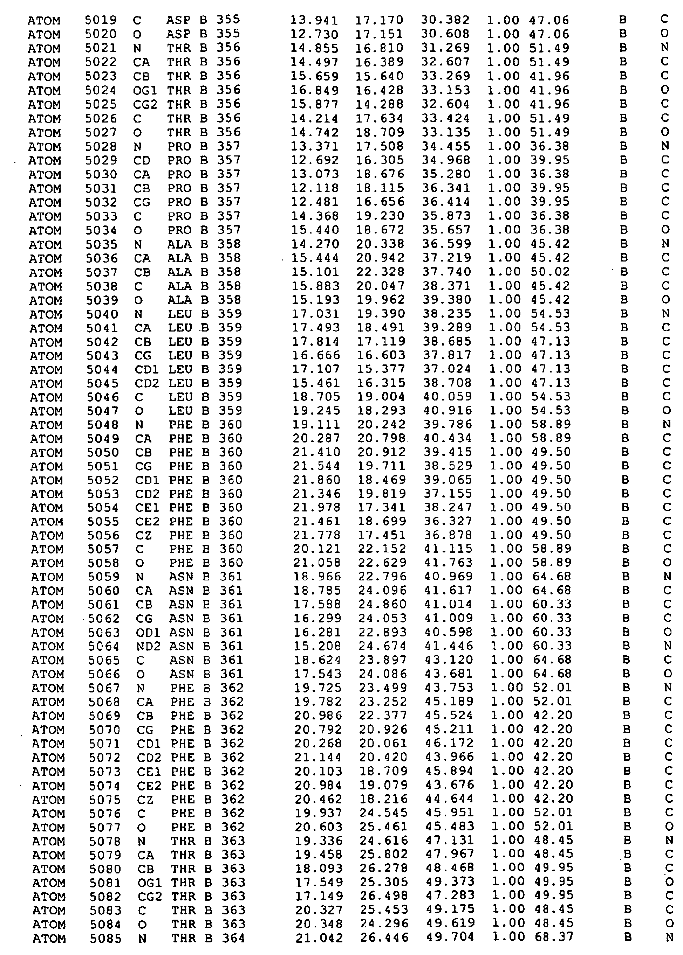

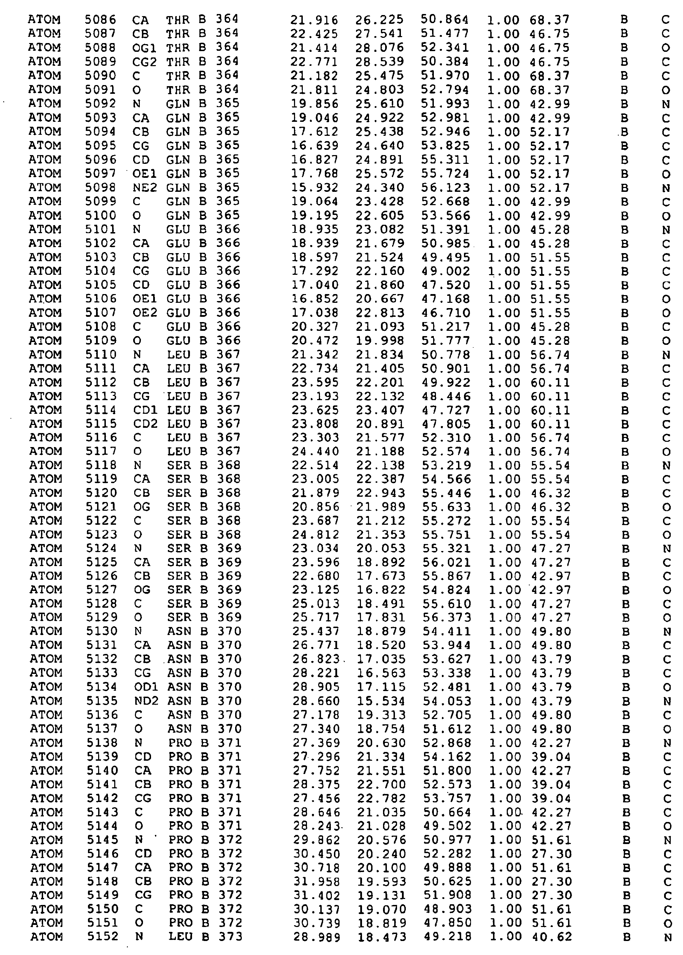

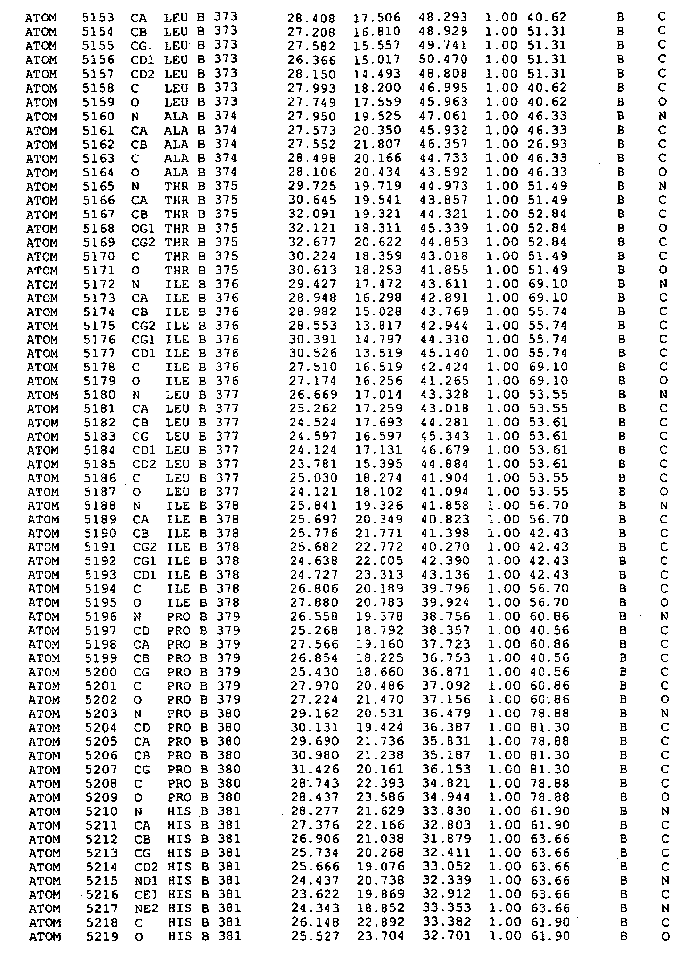

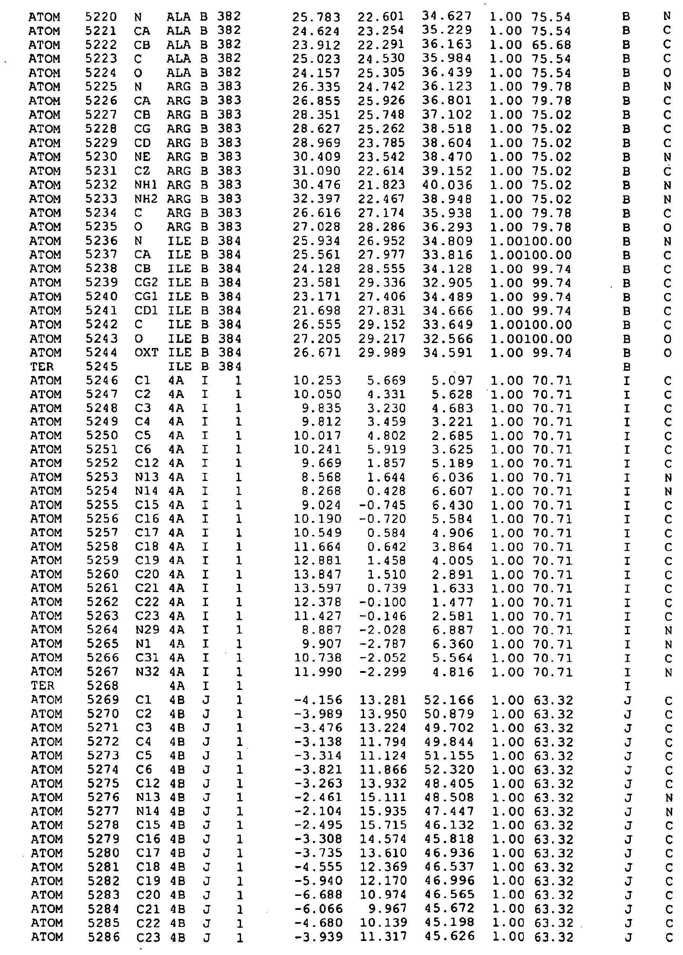

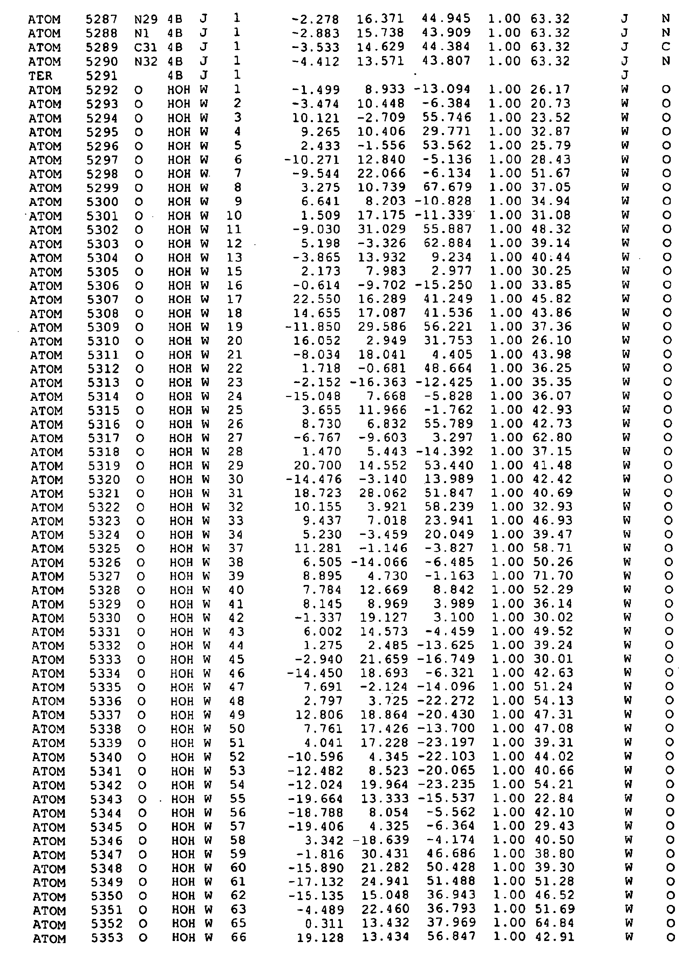

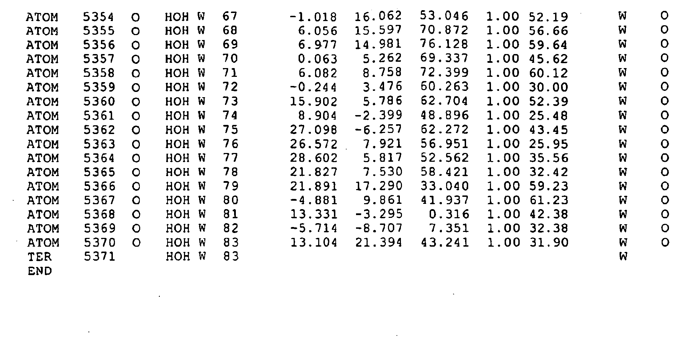

In the crystal structure of the phosphorylated GSK-3β-inhibitor1 complex, amino acid residues I62, G63, F67, V70, A83, K85, V110, L132, D133, Y134, V135, T138, N186, L188, C199, and D200 according to Figure 3 were within 5 Å of the inhibitor bound in the active site. These amino acids residues were identified using the program QUANTA (Molecular Simulations, Inc., San Diego, CA ⓒ1998), O (Jones et al., Acta Crystallogr. A47, pp. 110-119 (1991)) and RIBBONS (Carson, J. Appl. Crystallogr., 24, pp. 9589-961 (1991)). The programs allow the display and output of all residues within 5 Å from the inhibitor. In addition, amino acid residues V61, I62, G63, N64, G65, F67, V69, V70, A83, K85, K86, V87, E97, M101, V110, R111, L130, V131, L132, D133, Y134, V135, P136, E137, T138, R141, D181, K183, Q185, N186, L187, L188, L189, K197, L198, C199, D200, F201 and G202 according to Figure 3 were within 8 Å of the inhibitor bound in the active site. These amino acid residues were identified using the programs QUANTA, O and RIBBONS, *supra.*

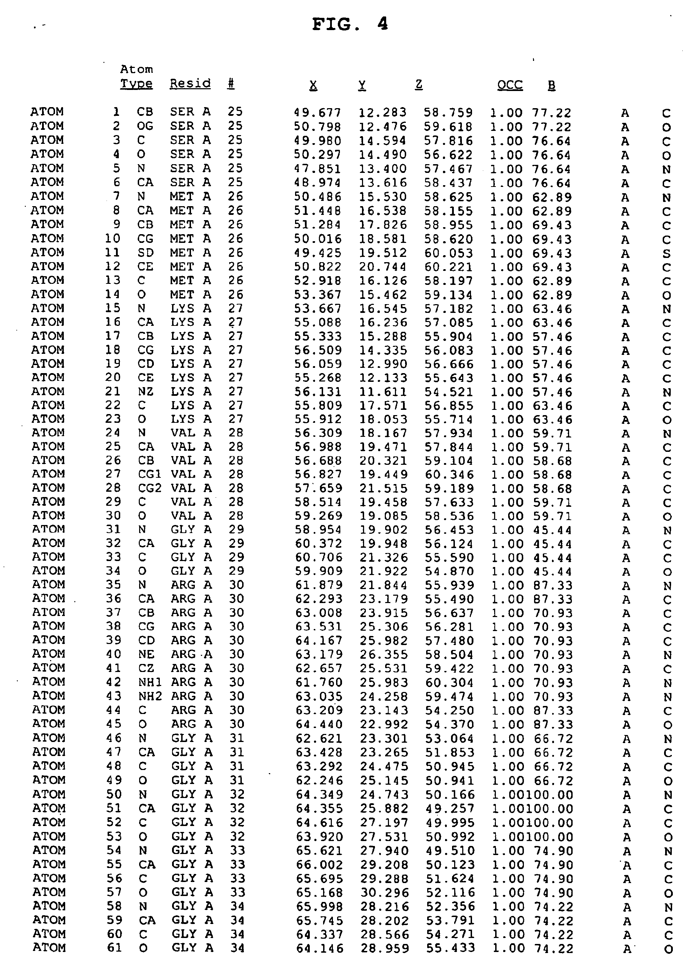

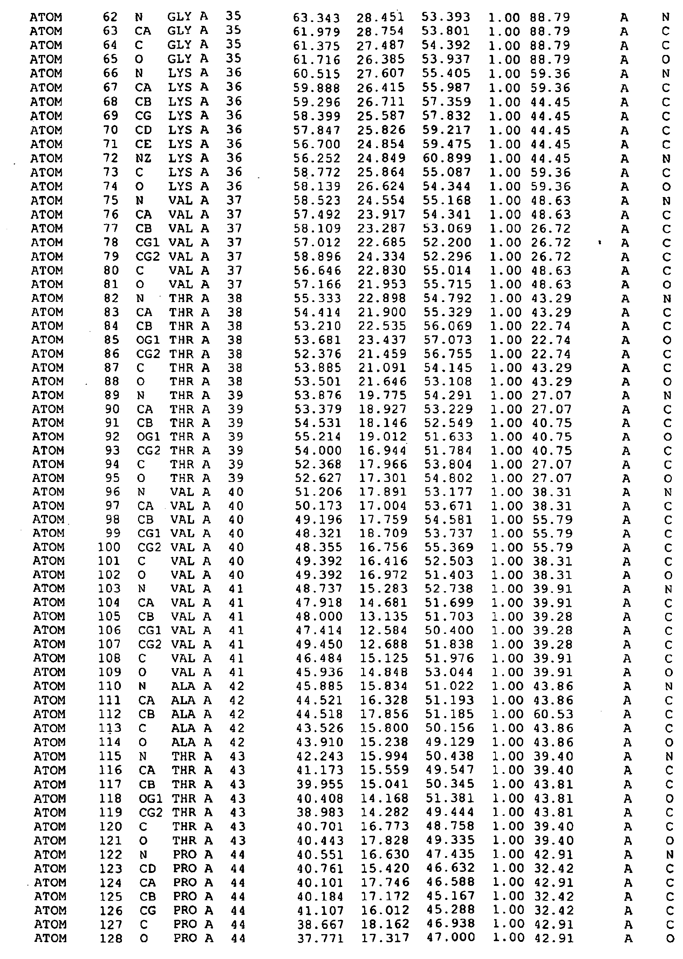

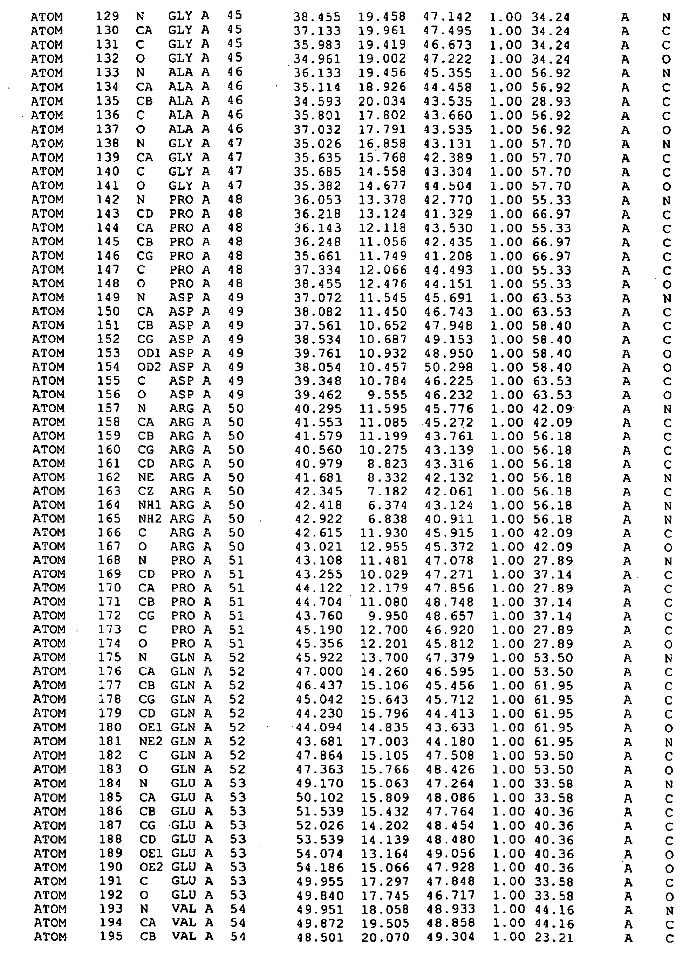

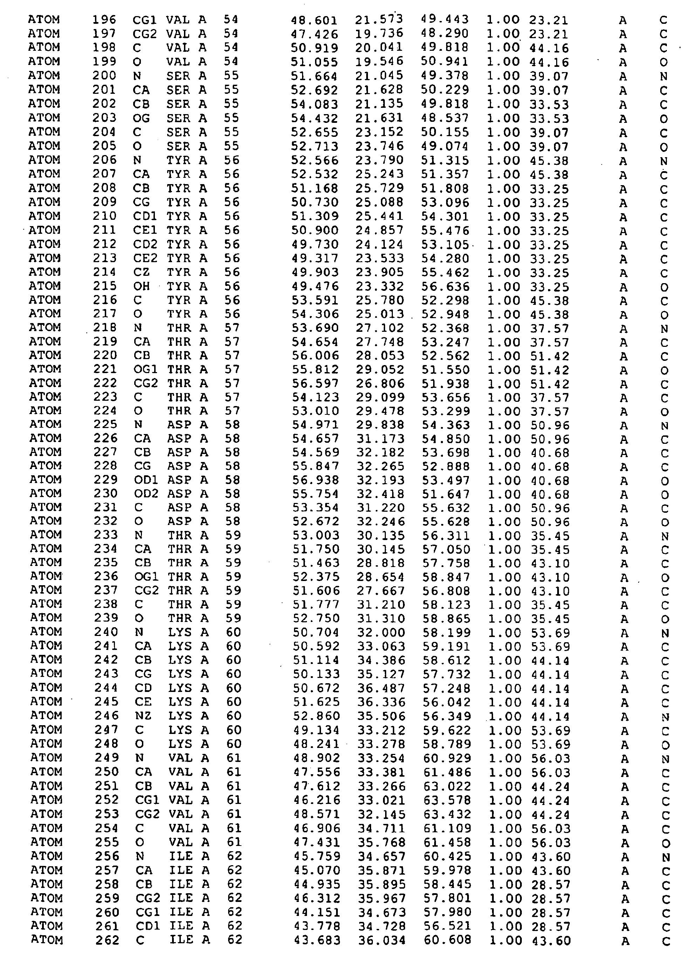

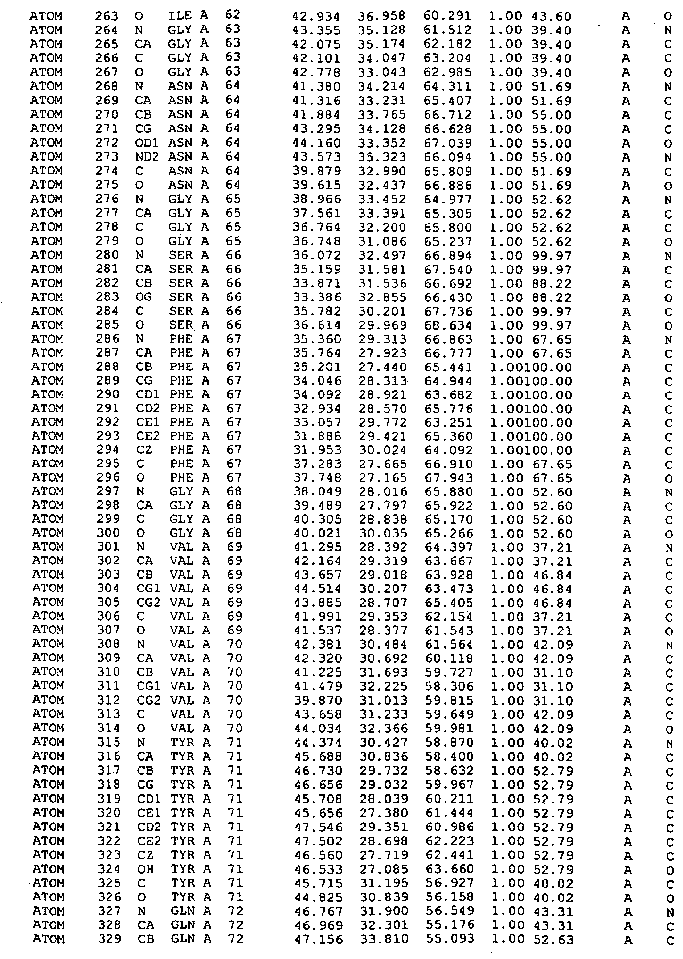

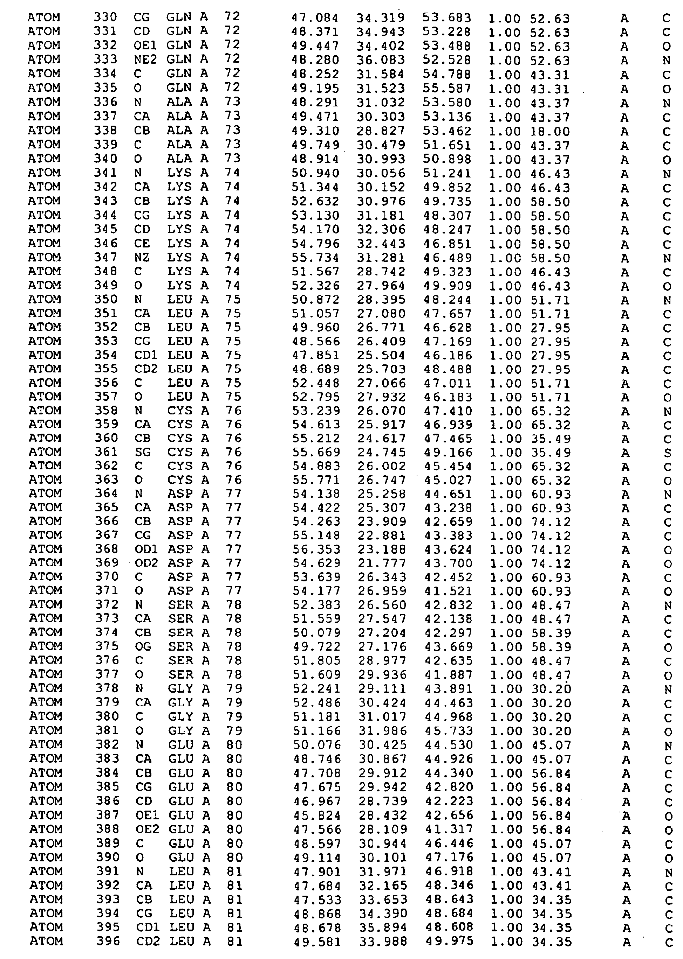

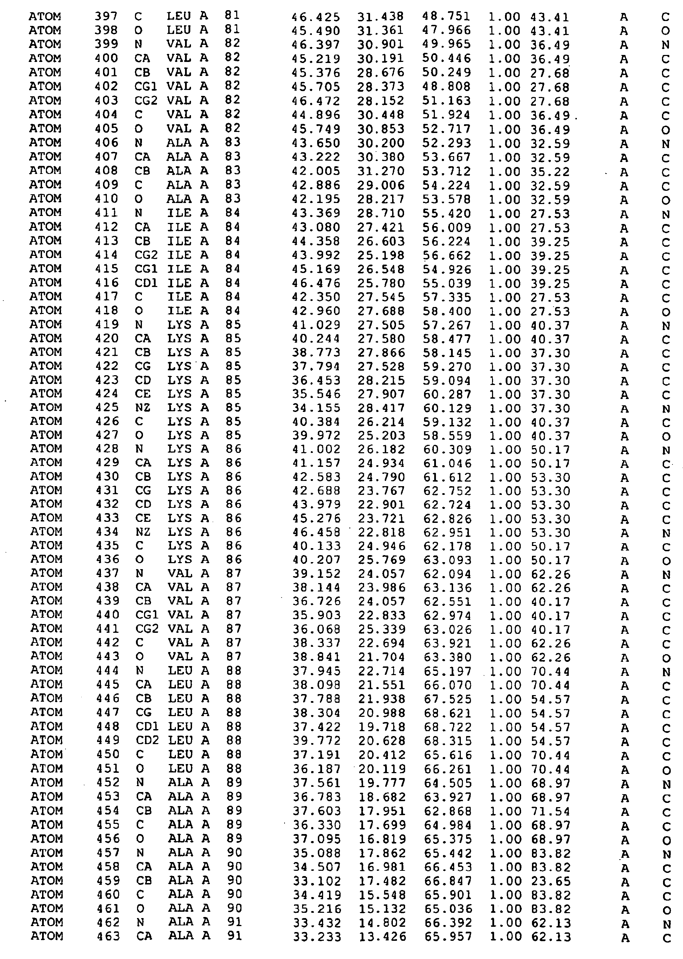

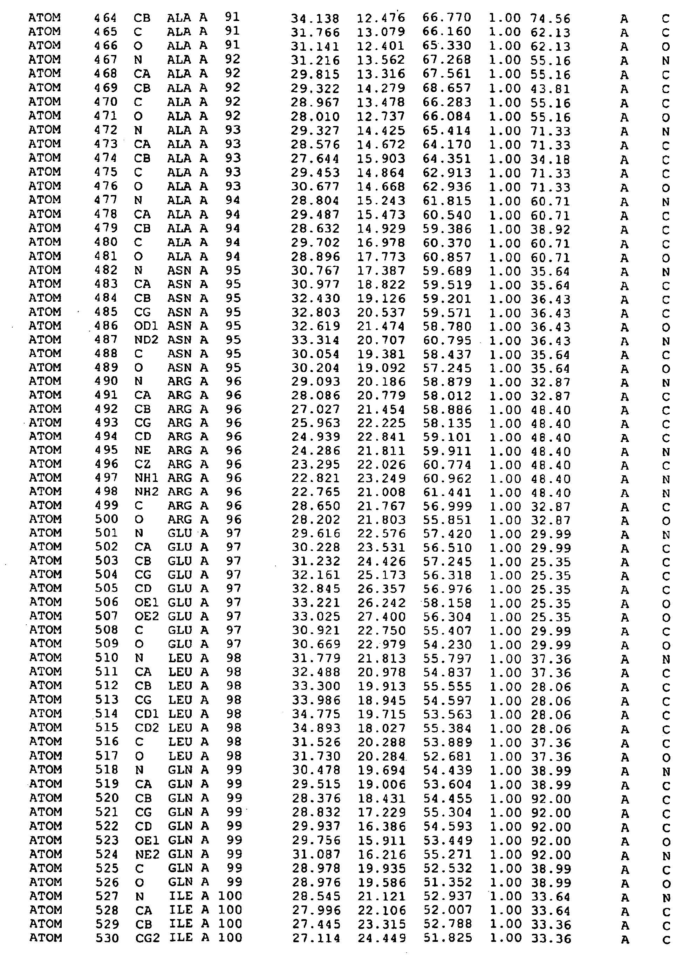

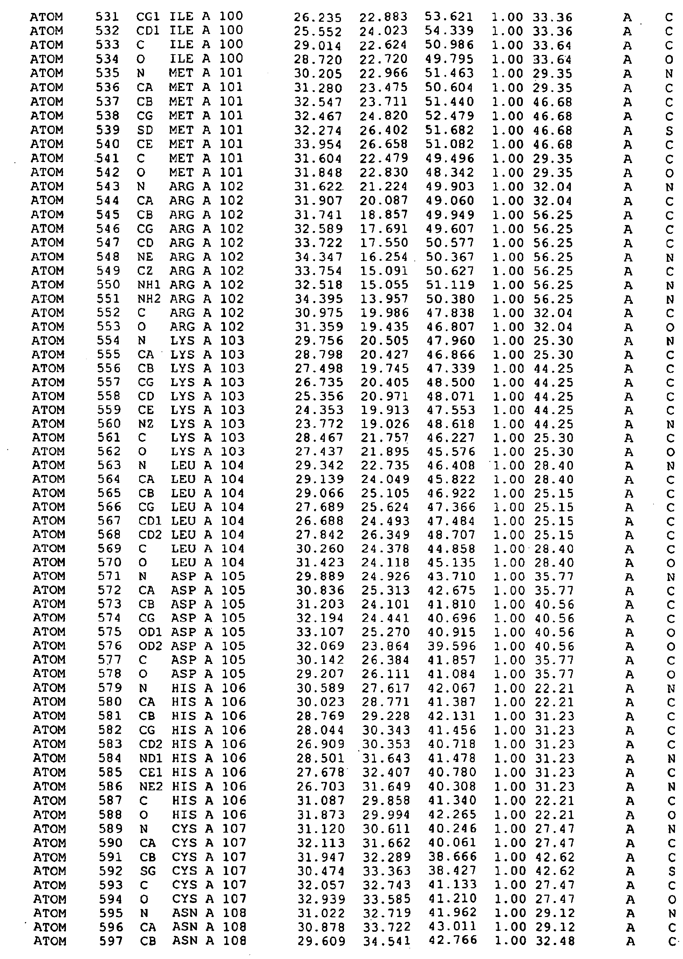

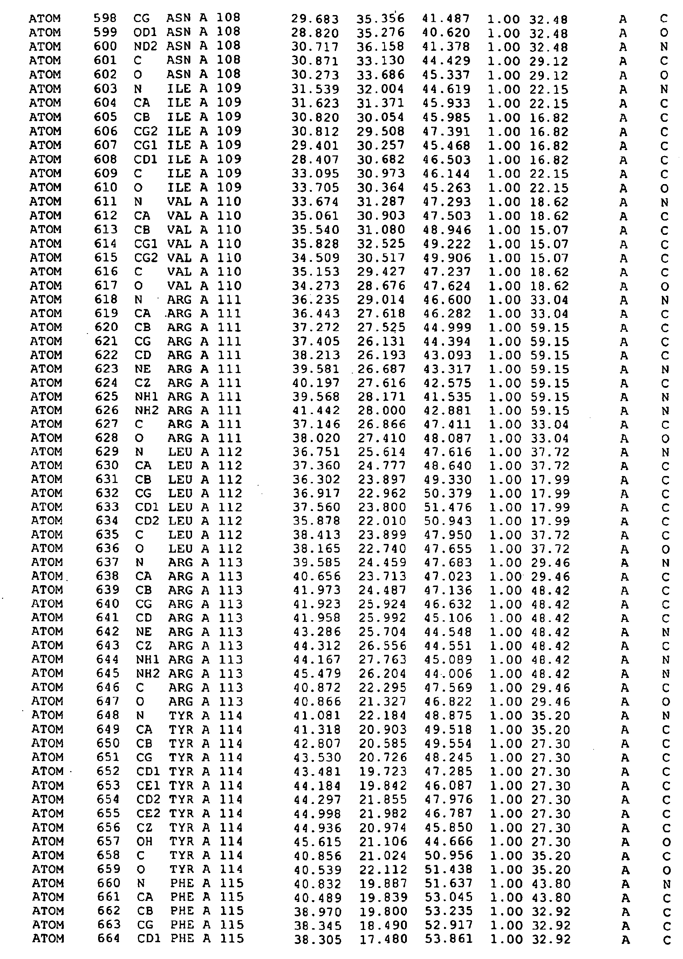

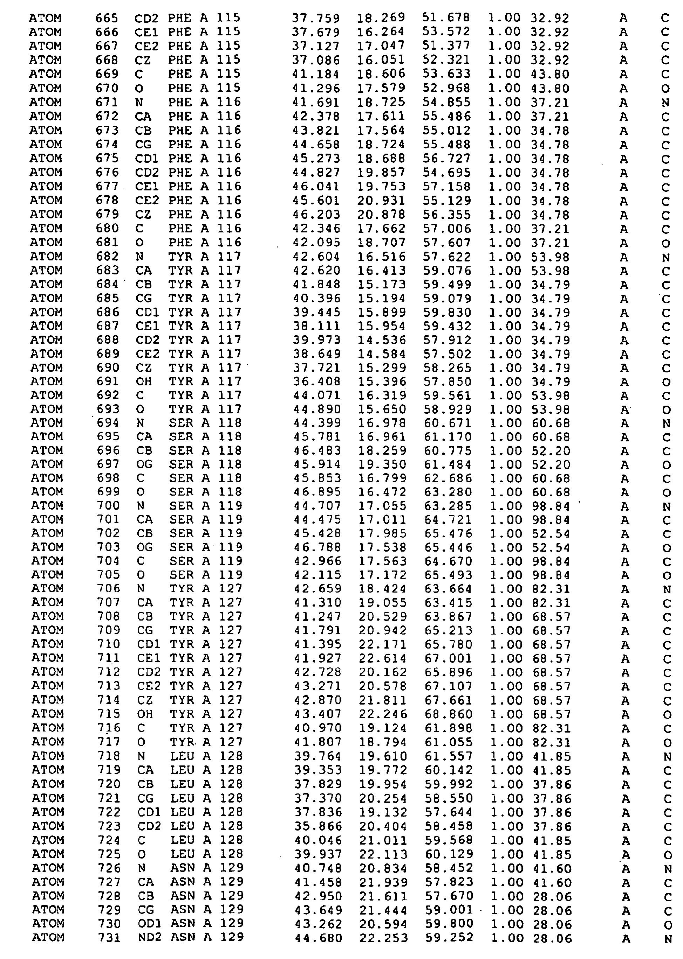

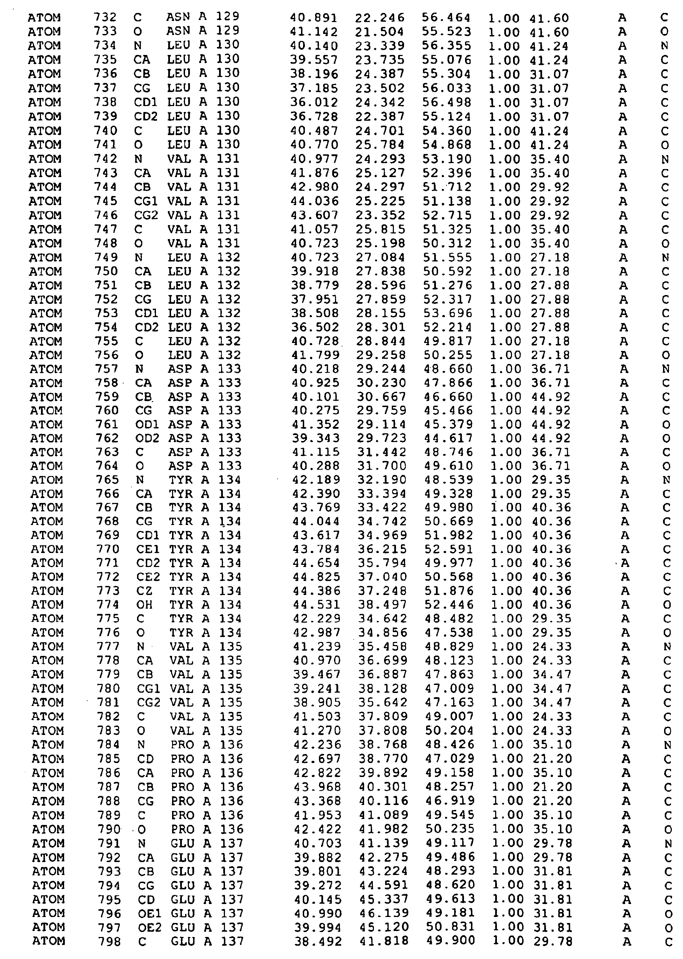

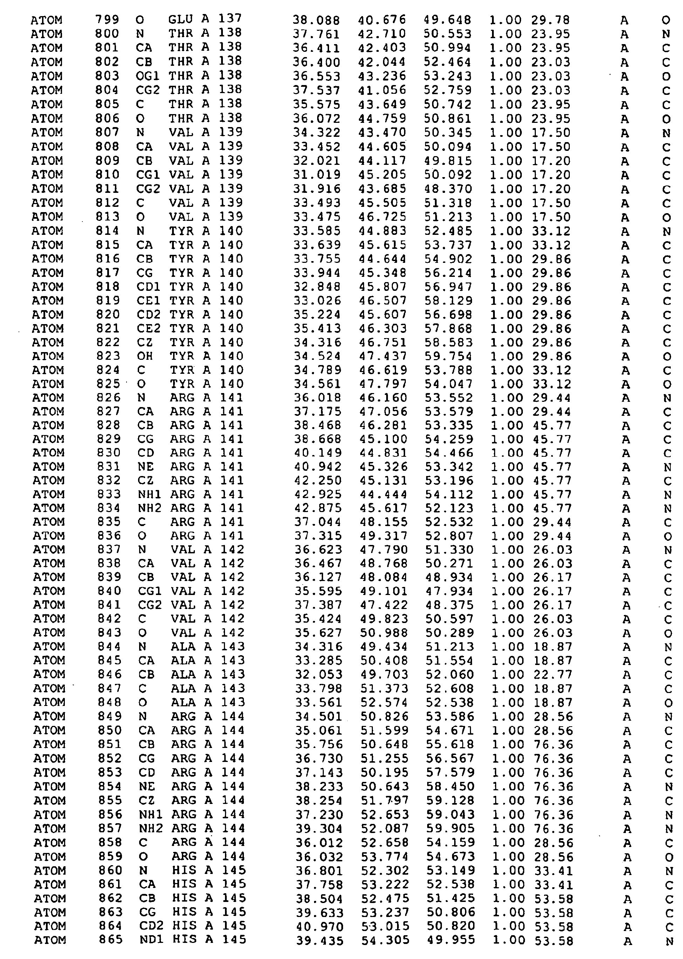

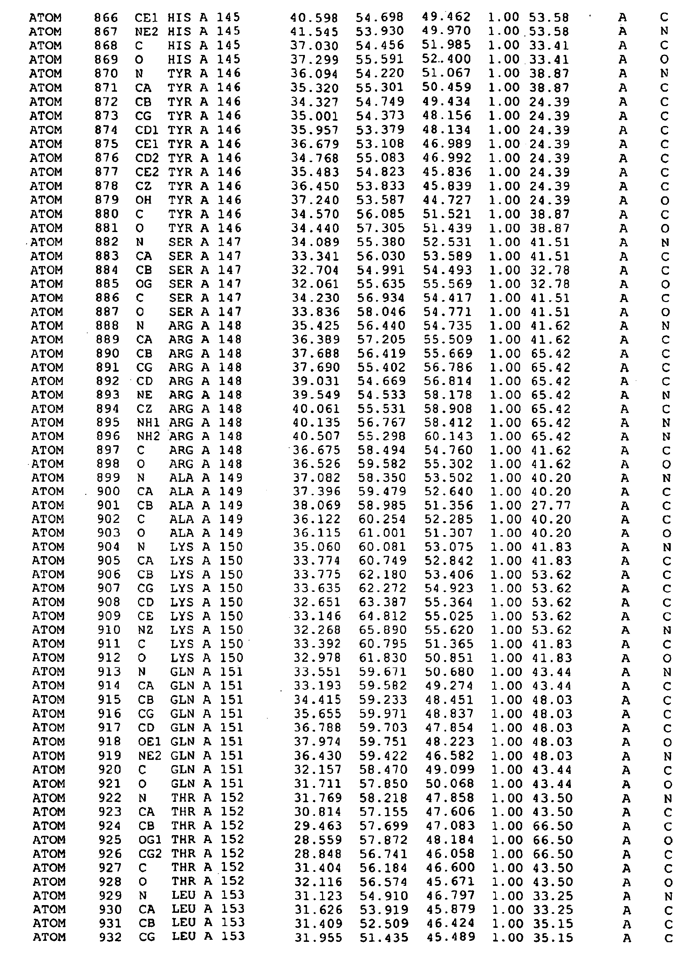

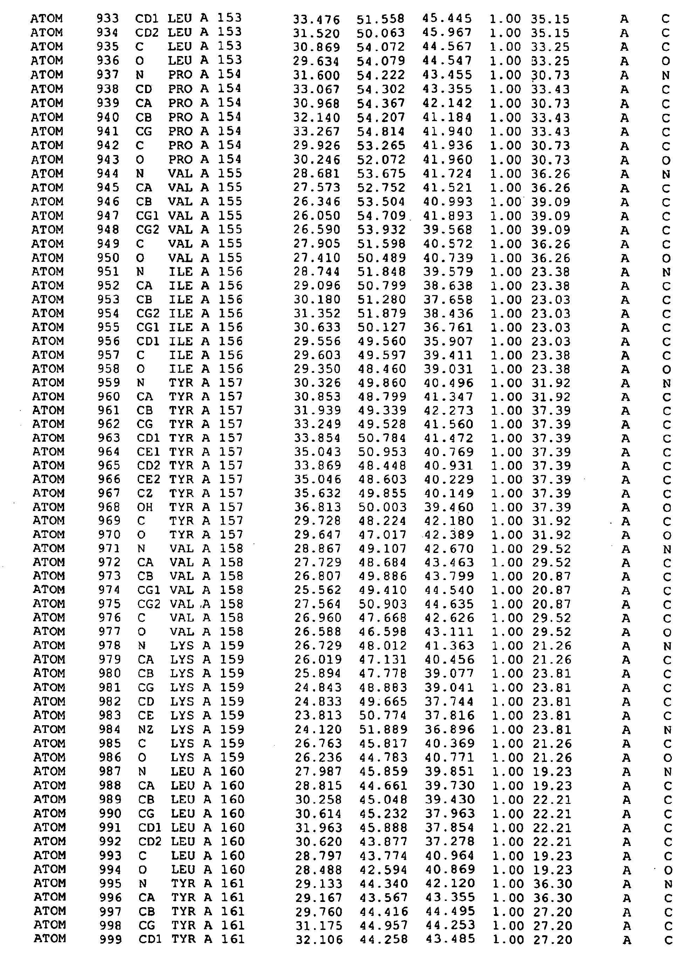

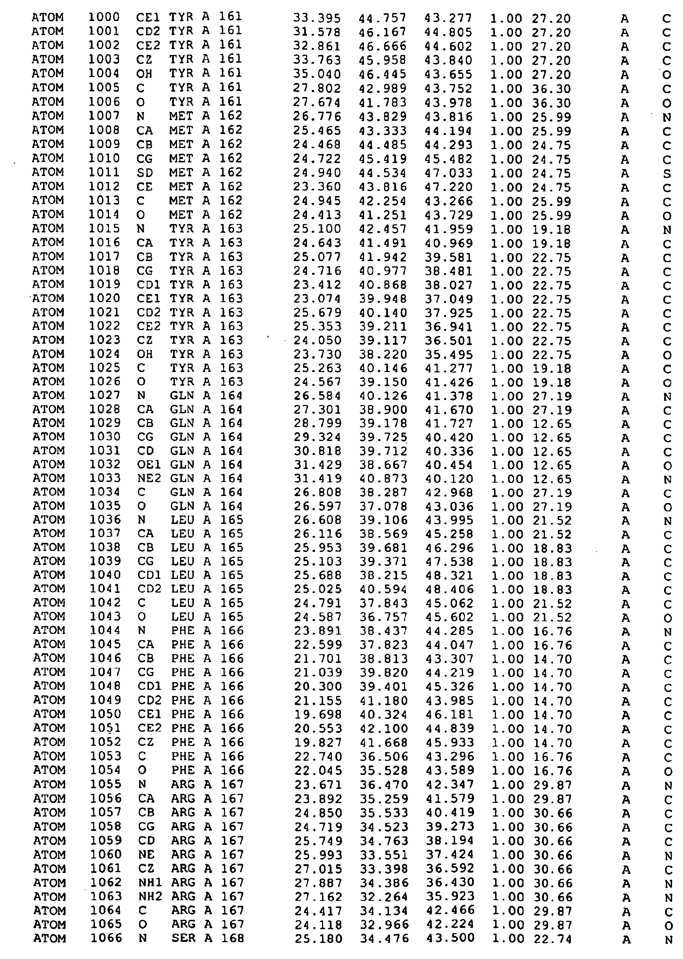

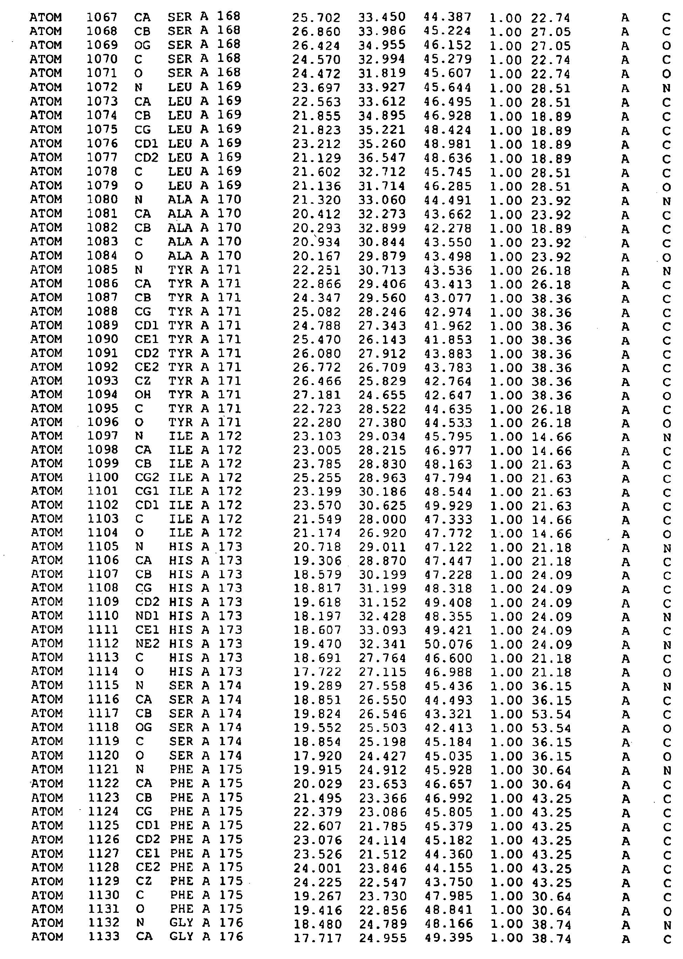

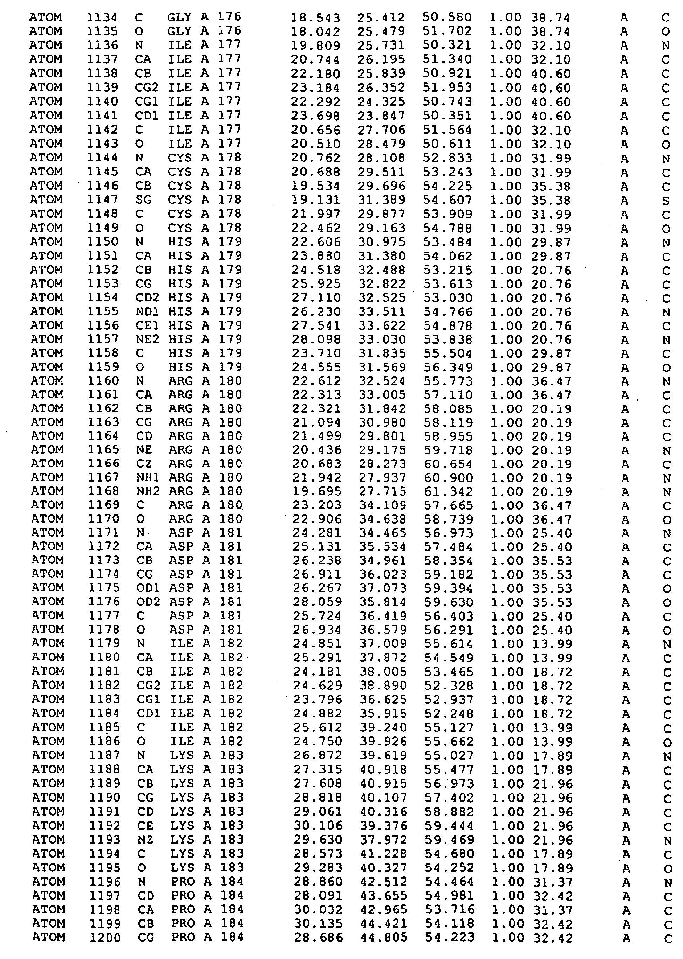

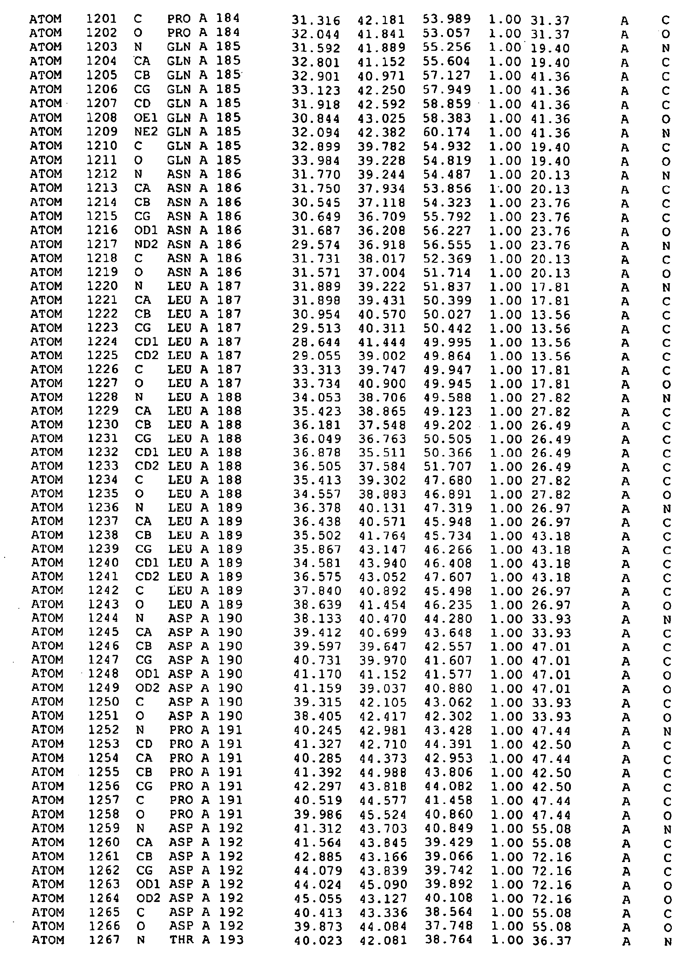

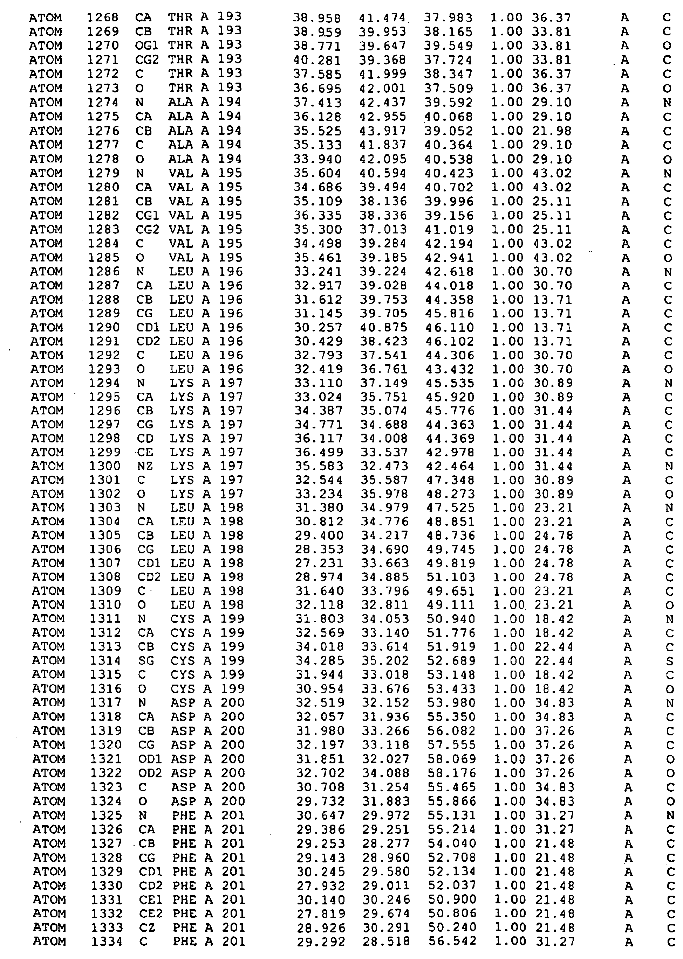

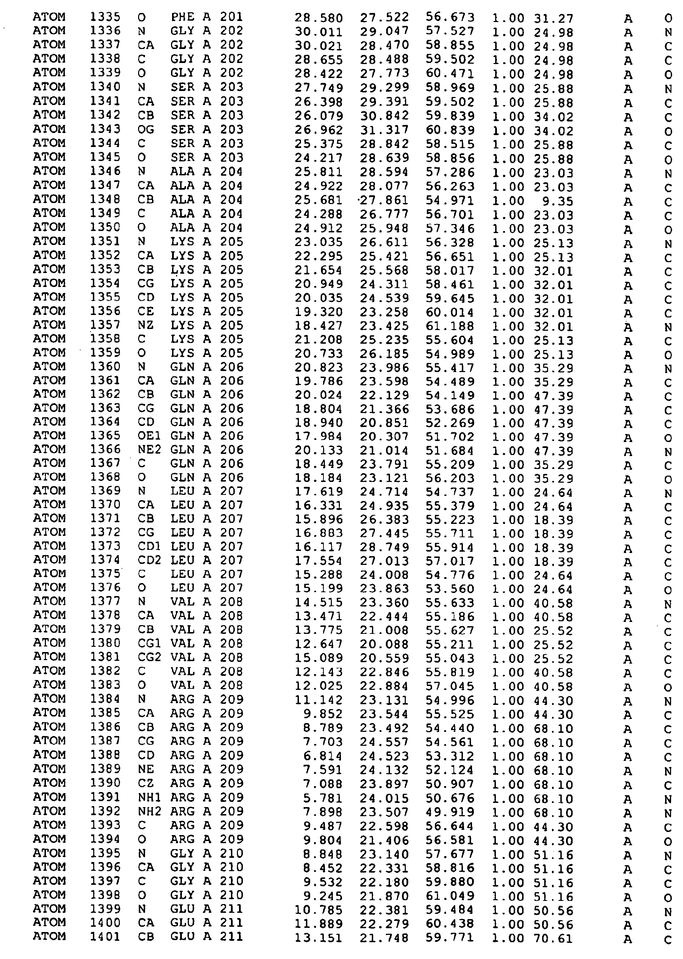

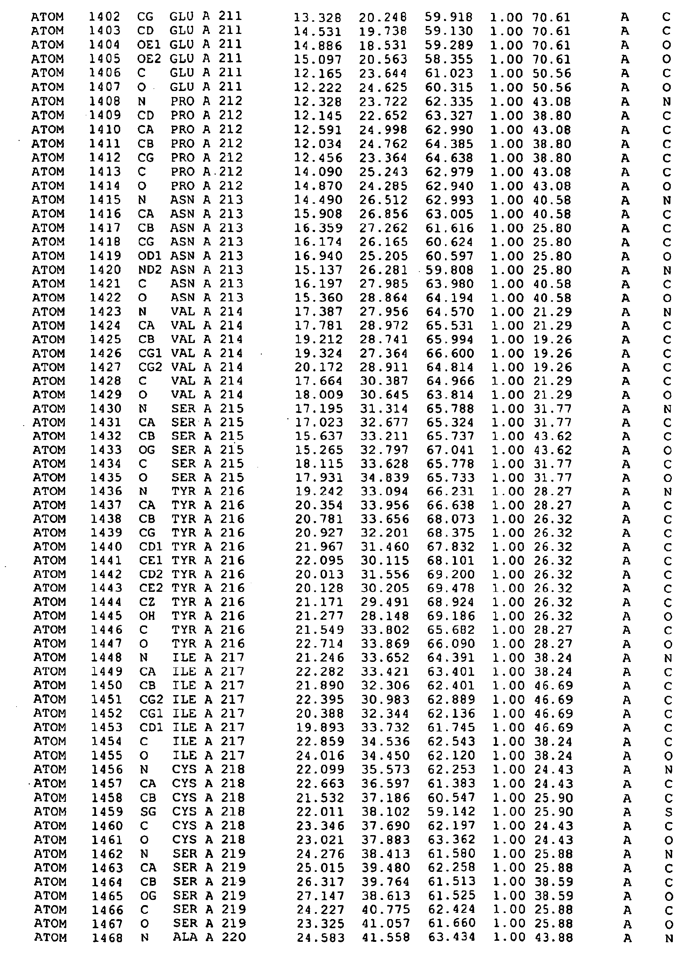

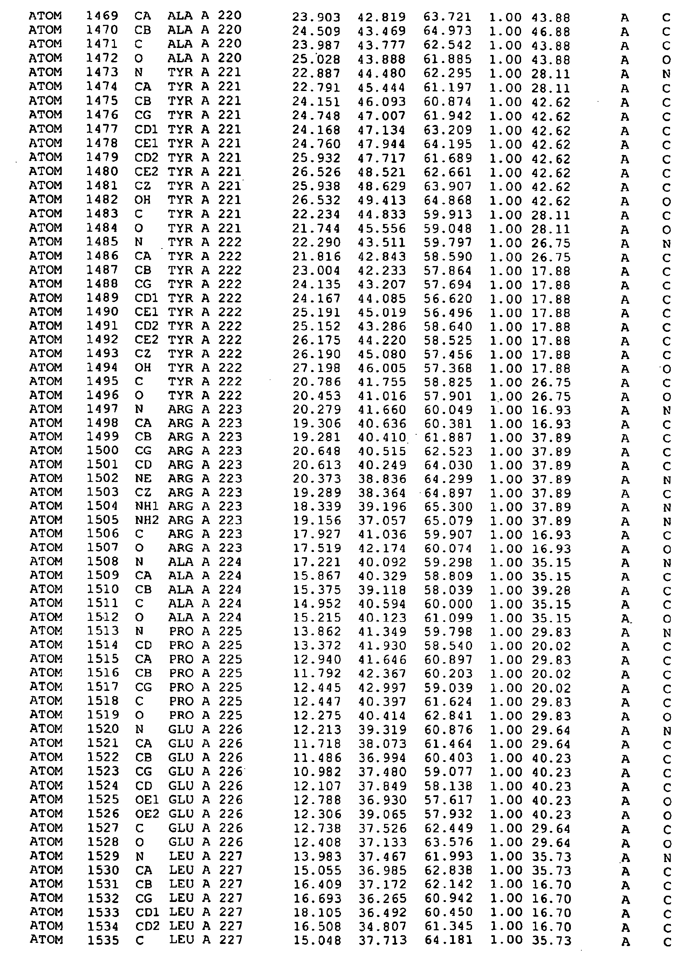

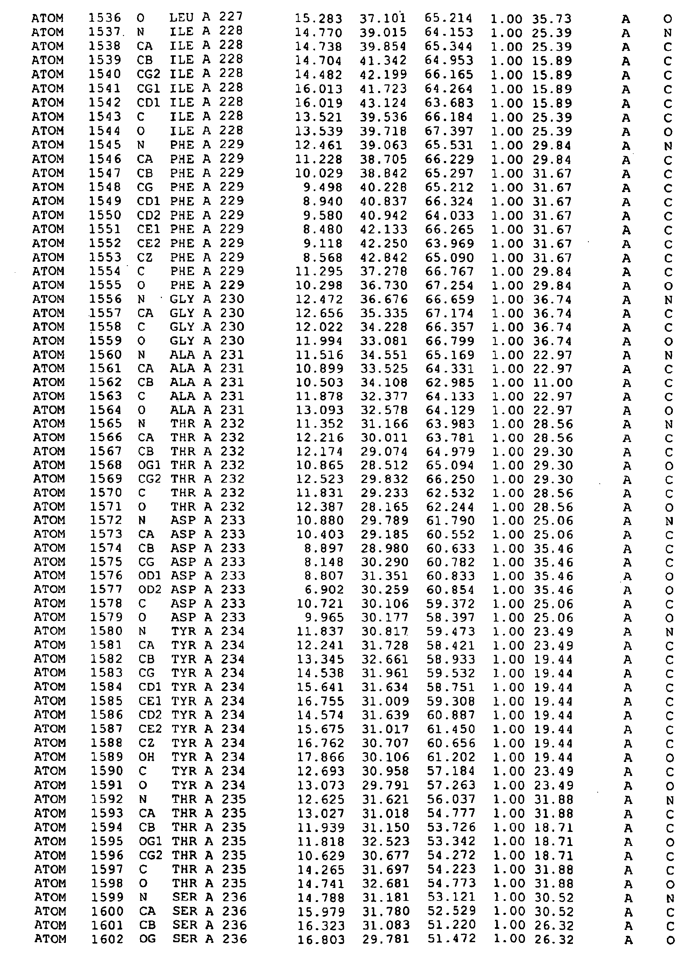

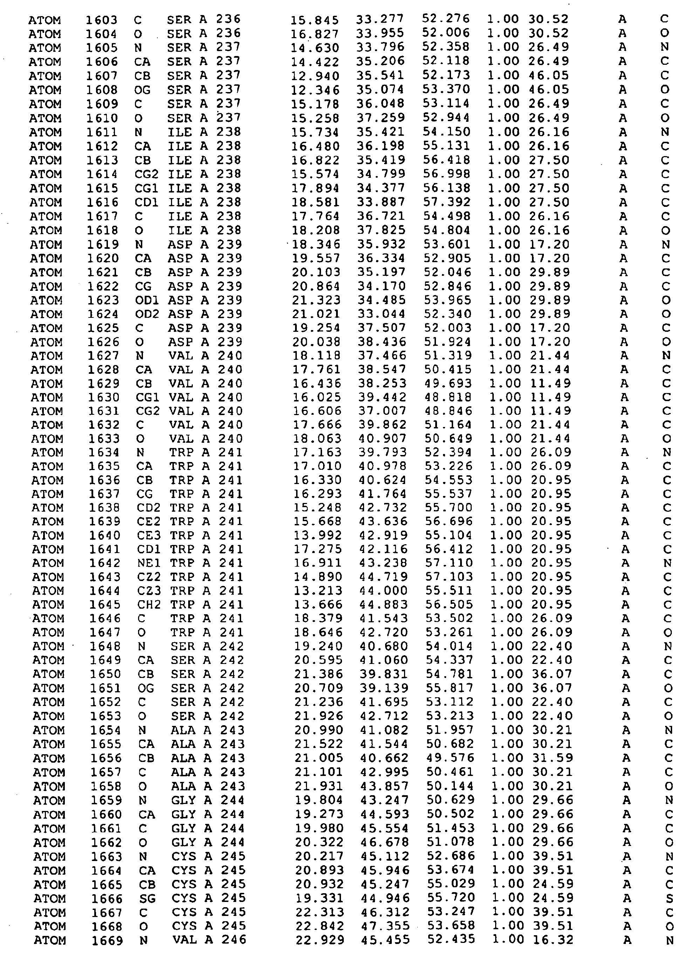

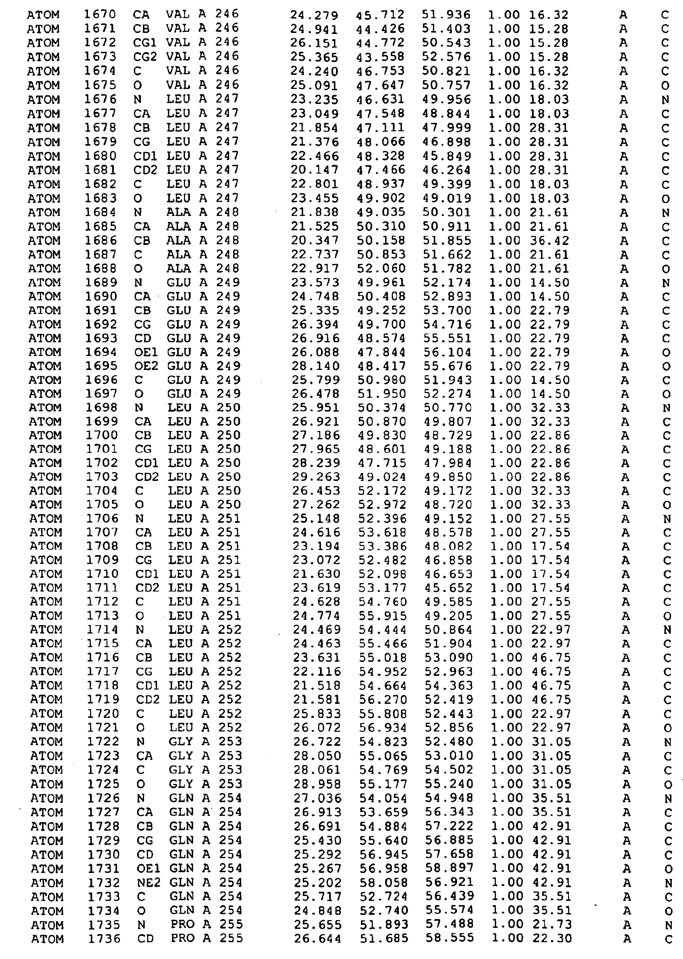

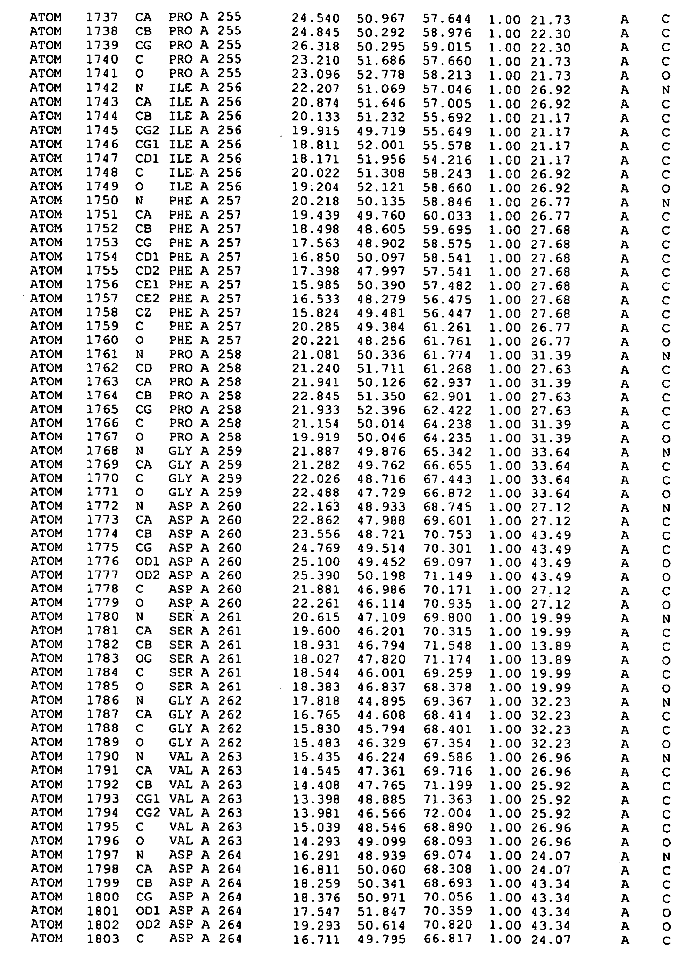

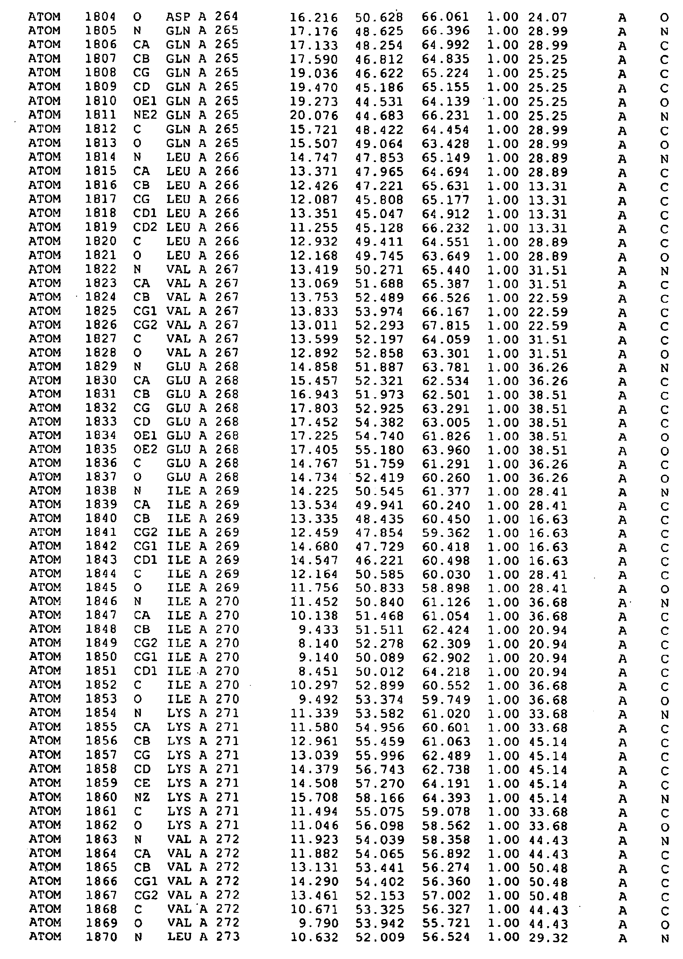

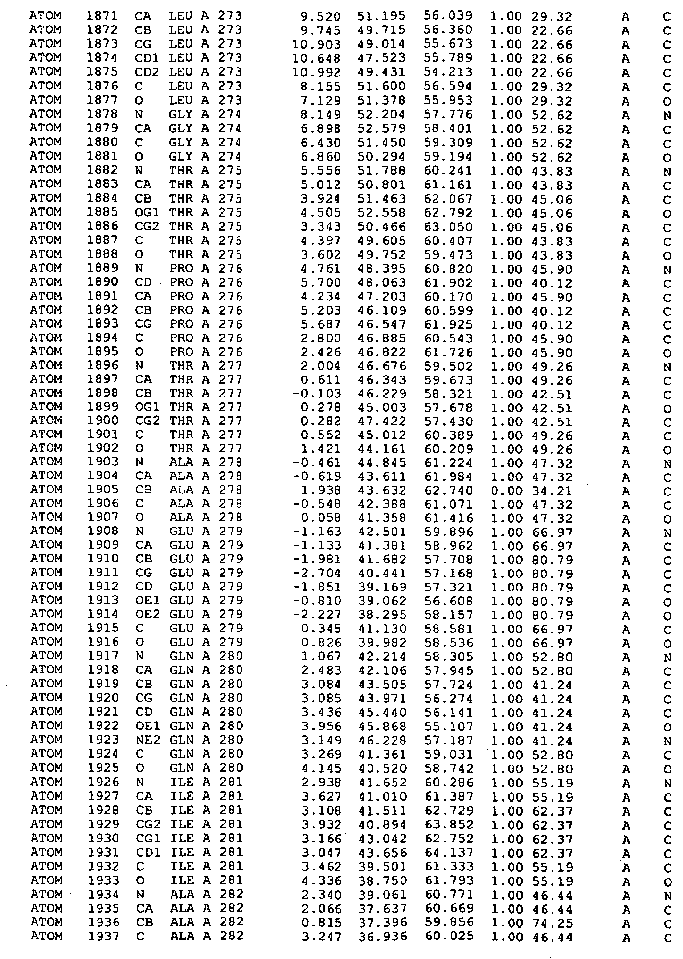

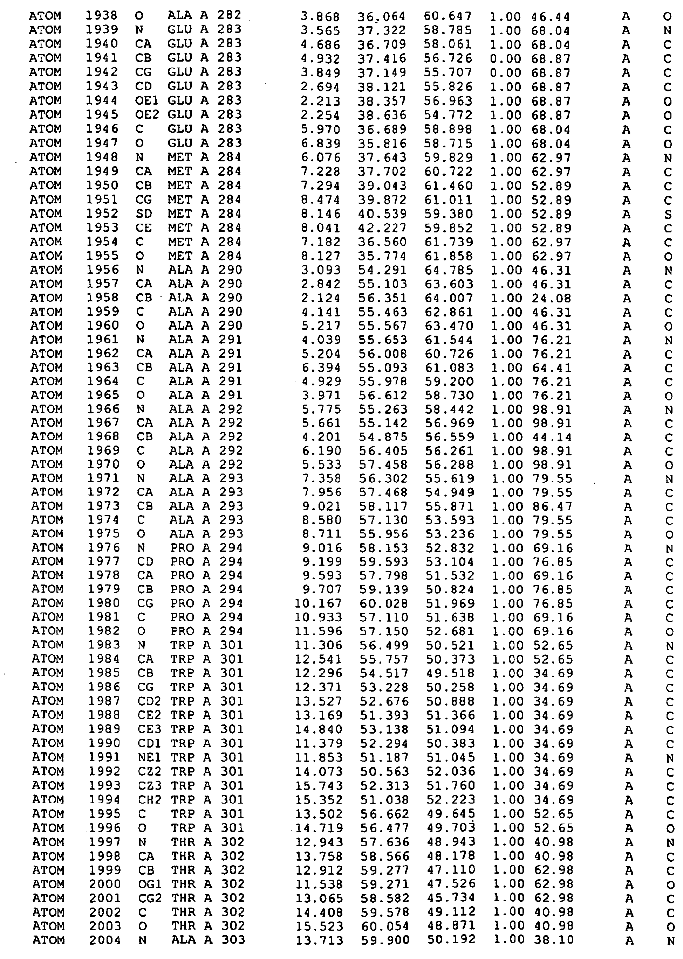

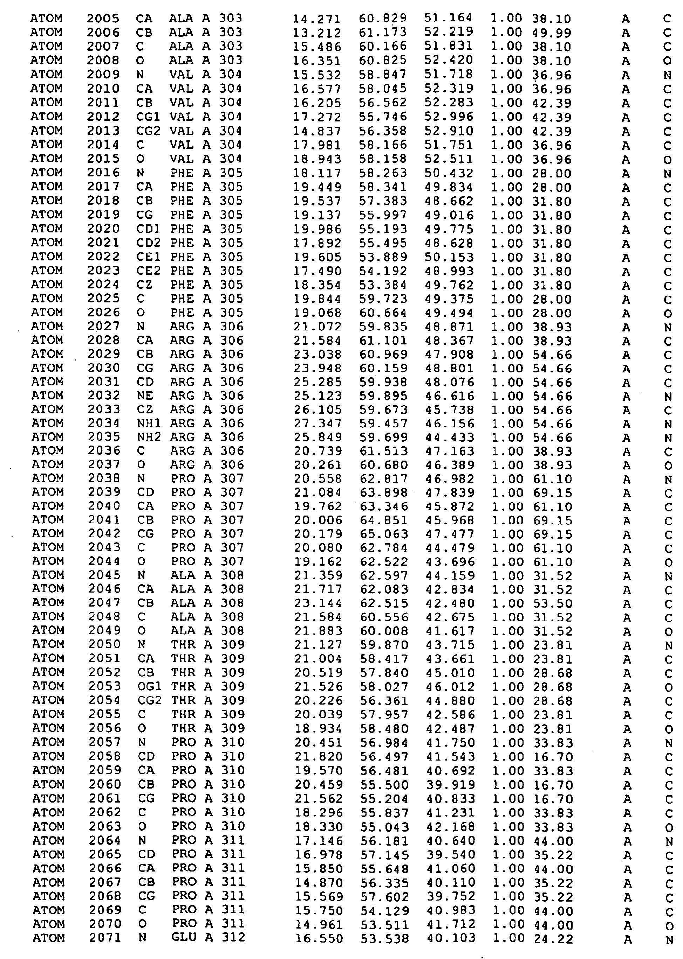

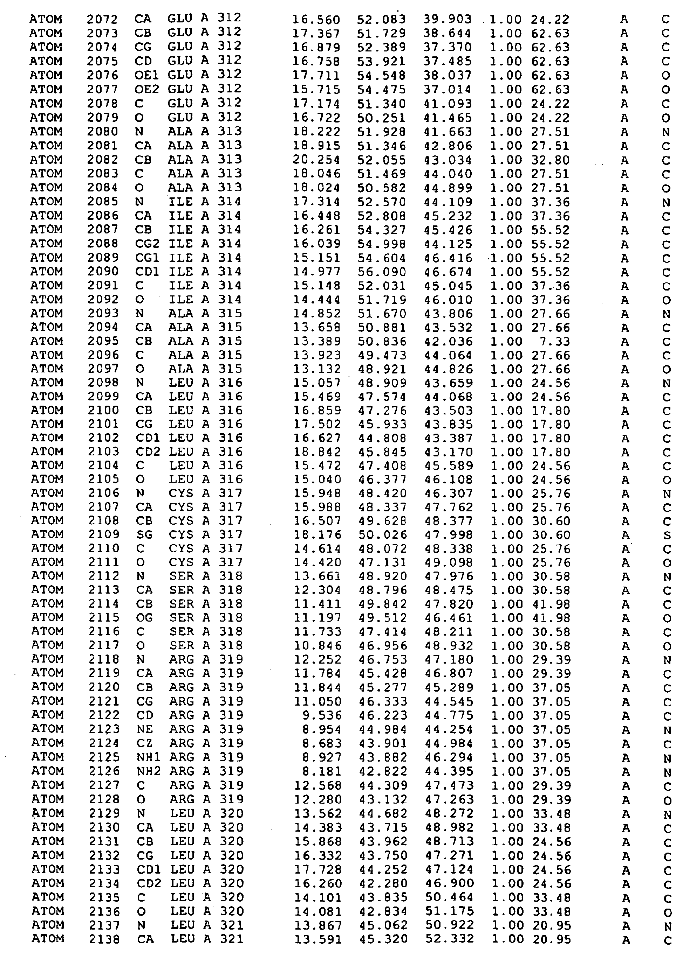

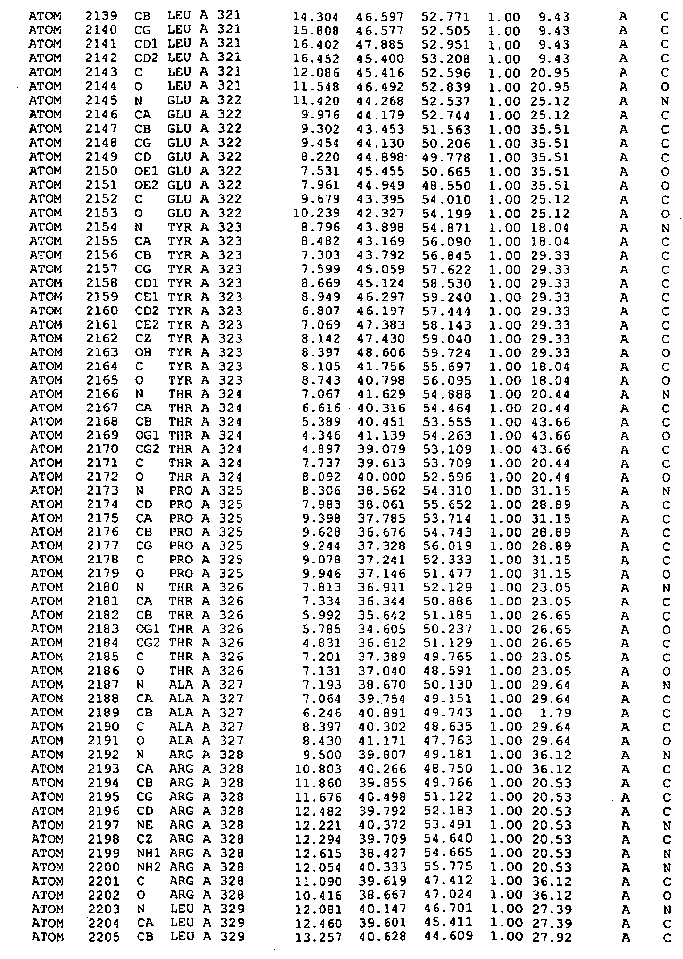

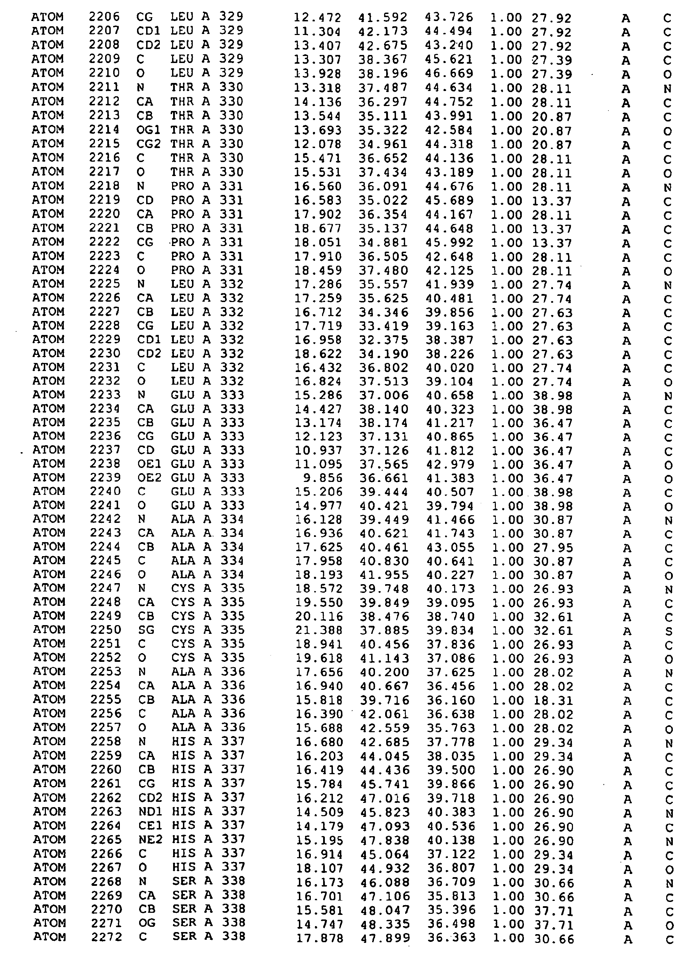

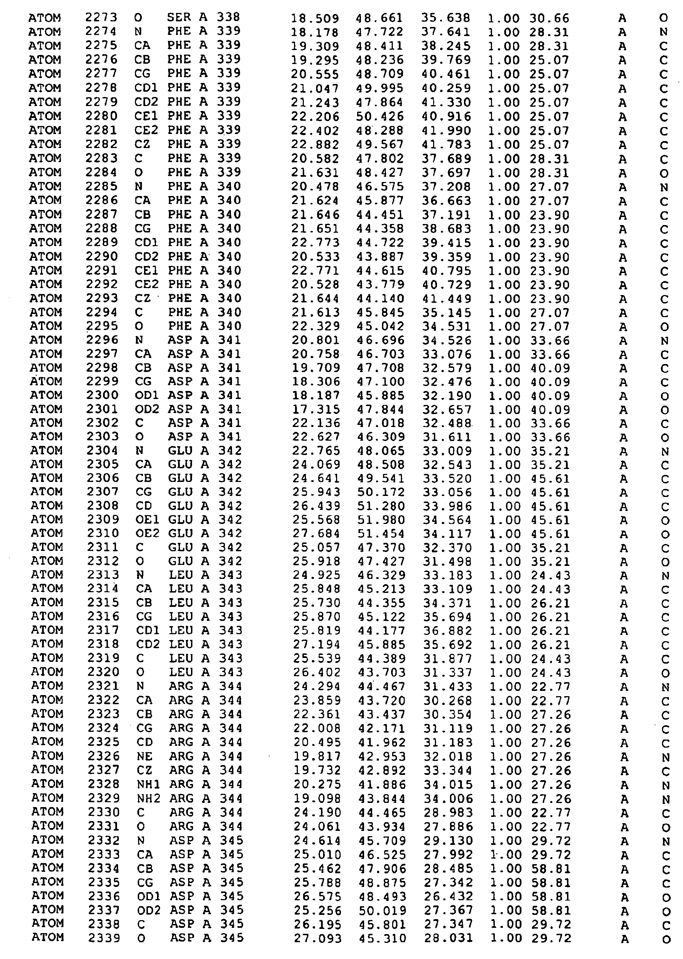

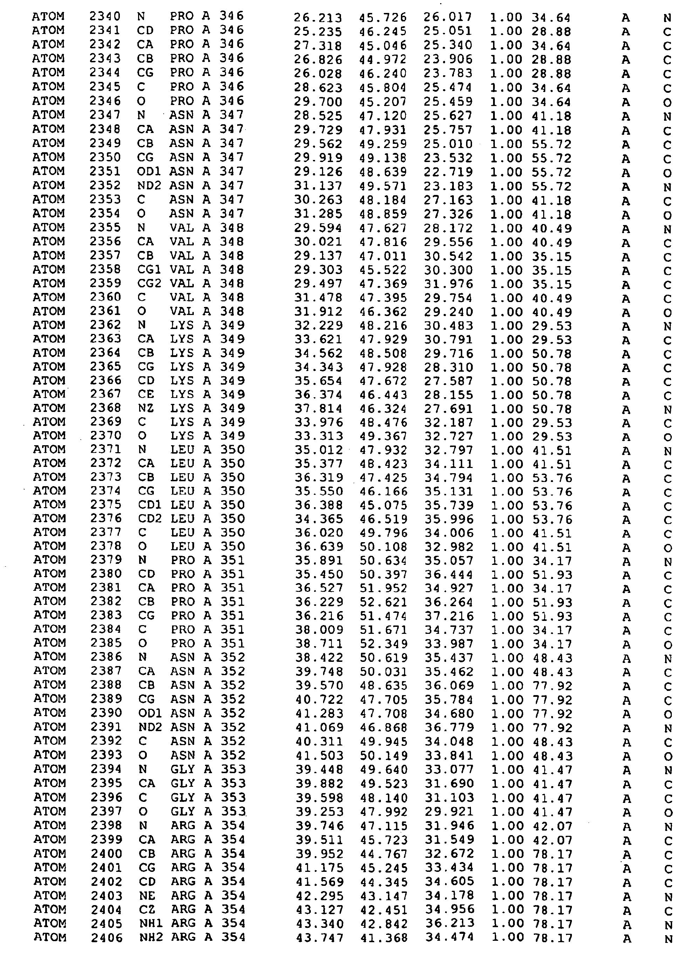

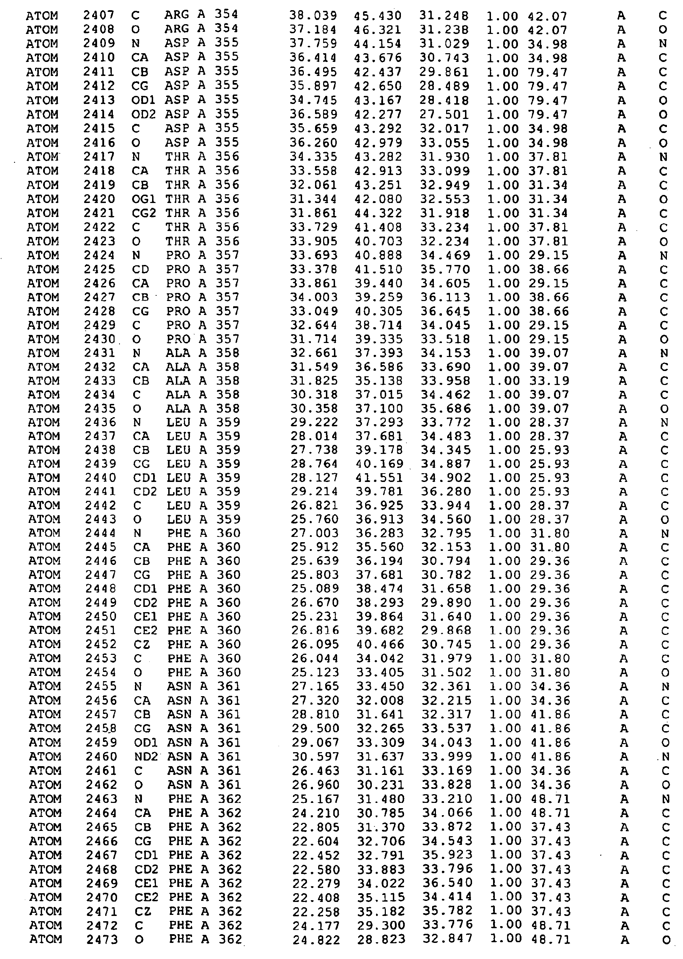

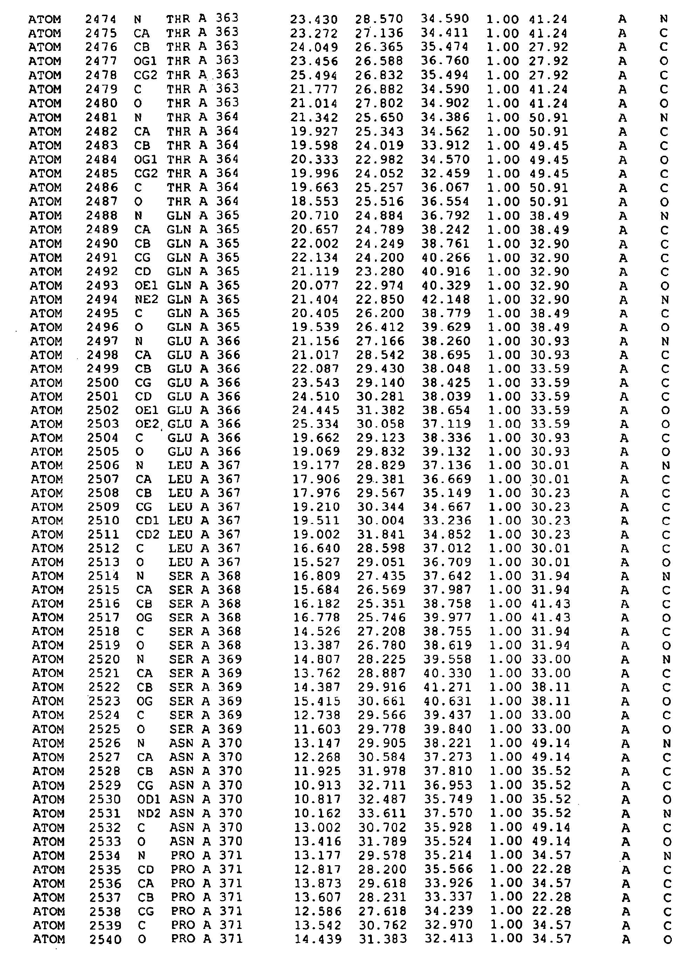

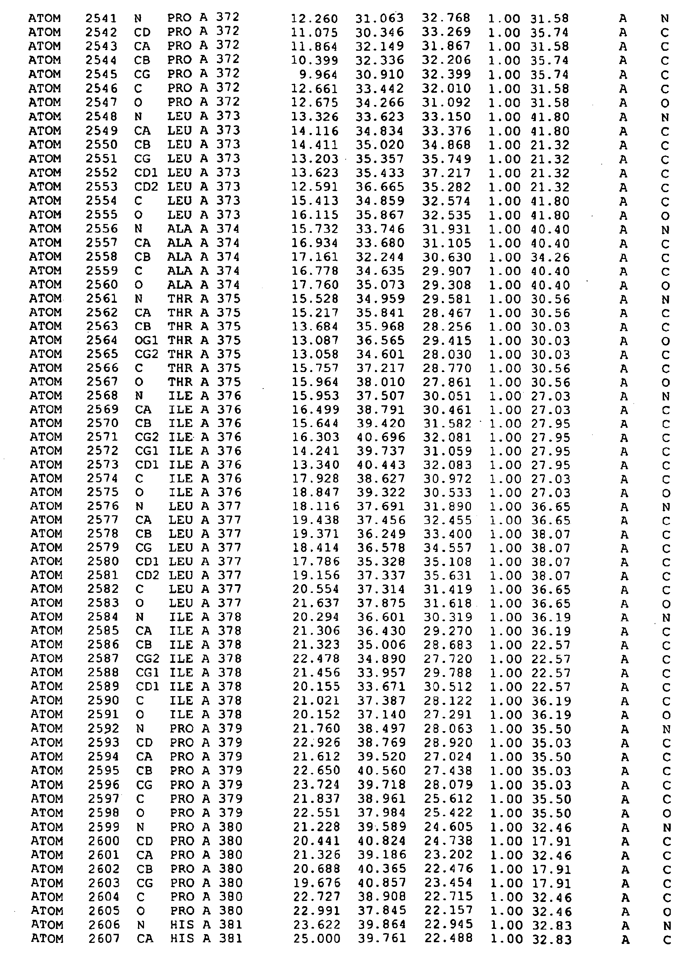

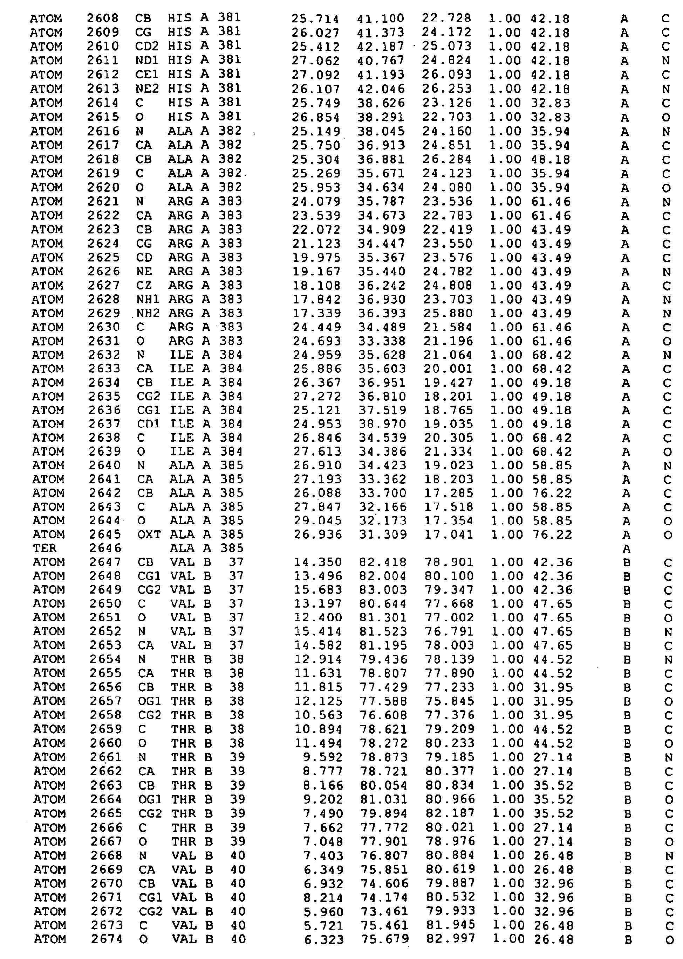

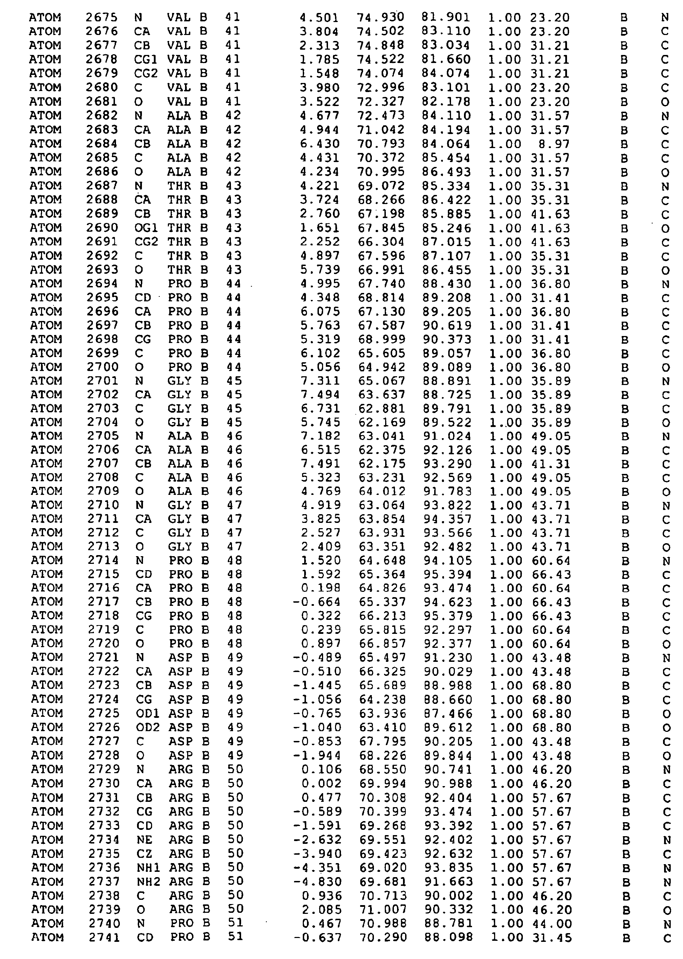

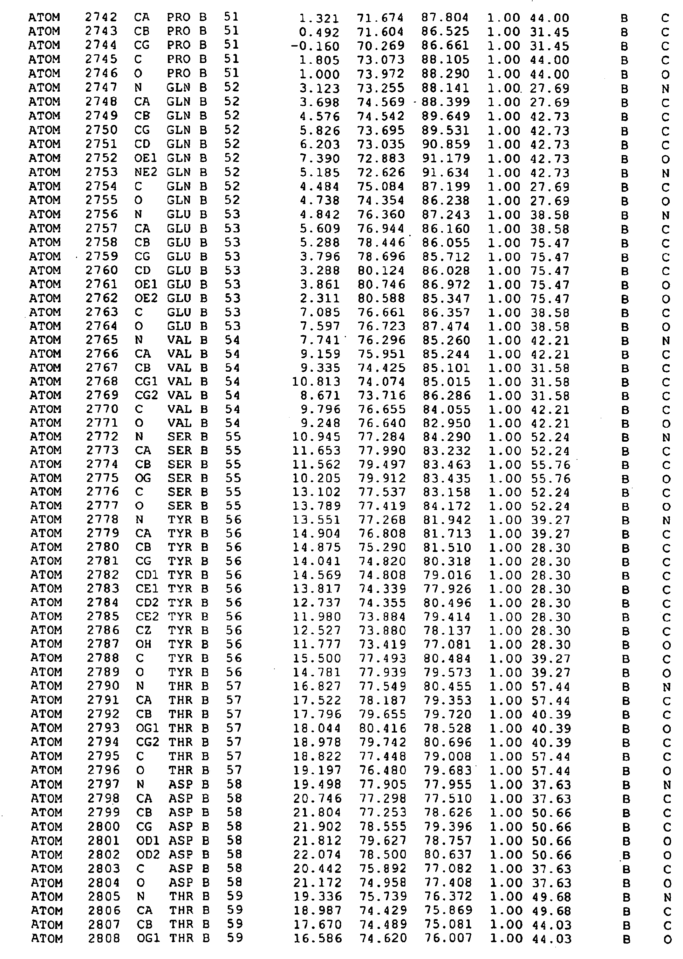

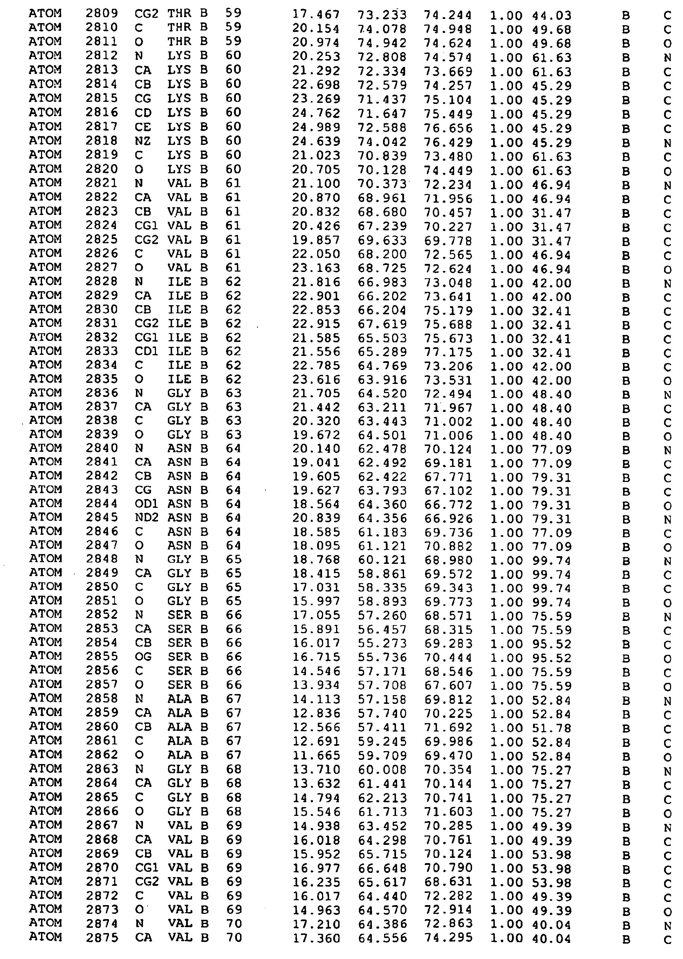

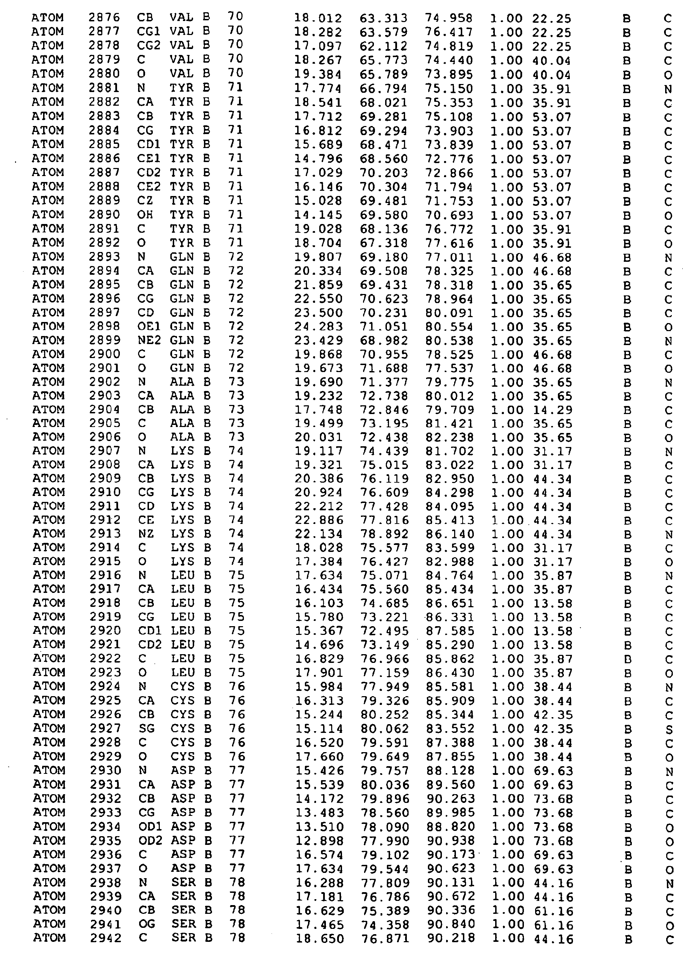

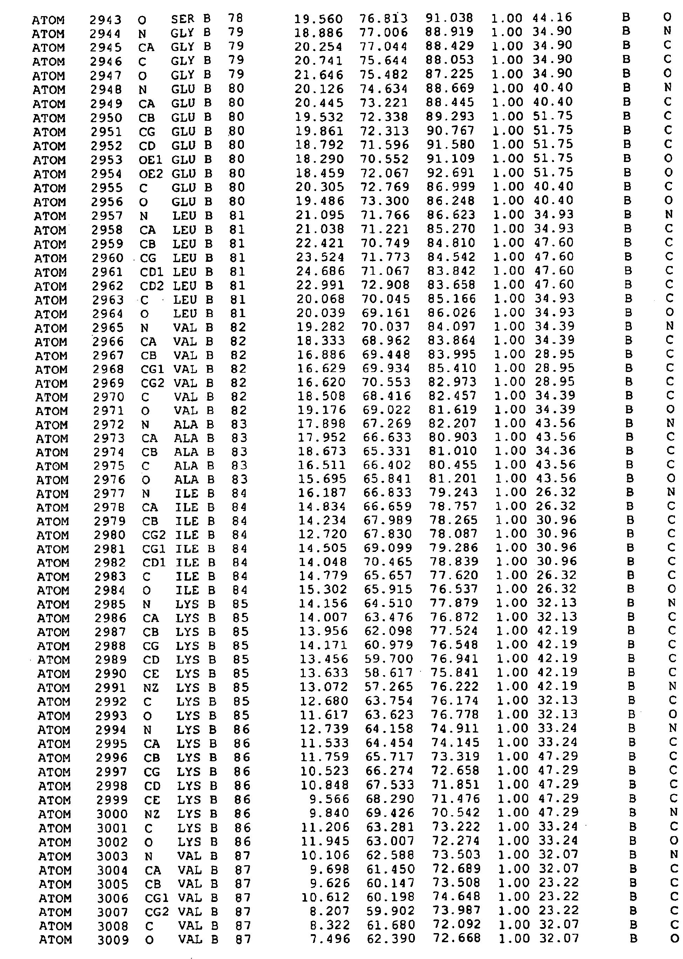

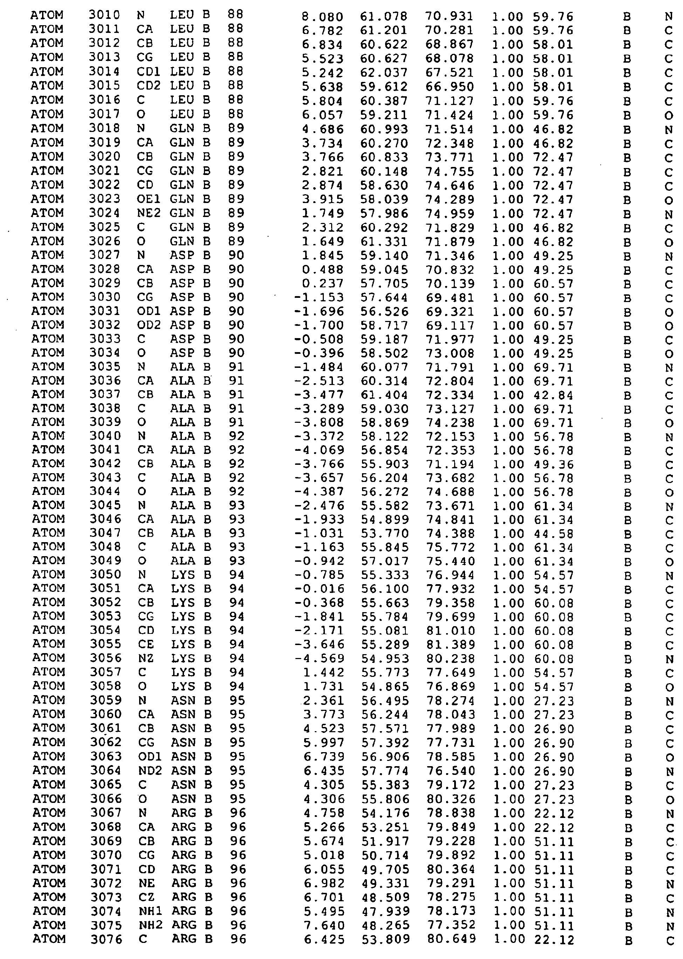

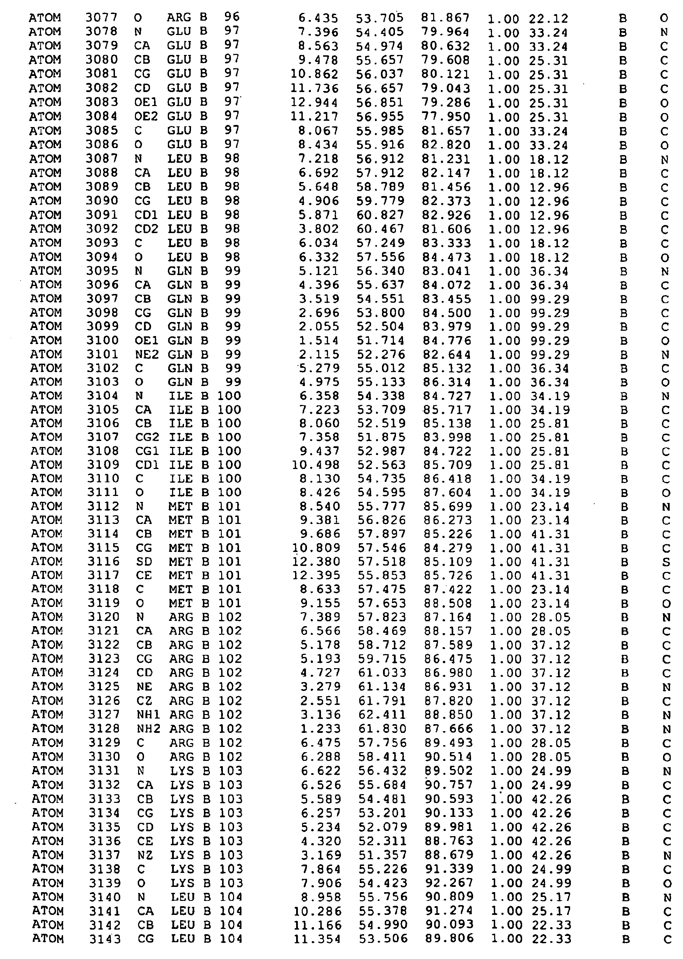

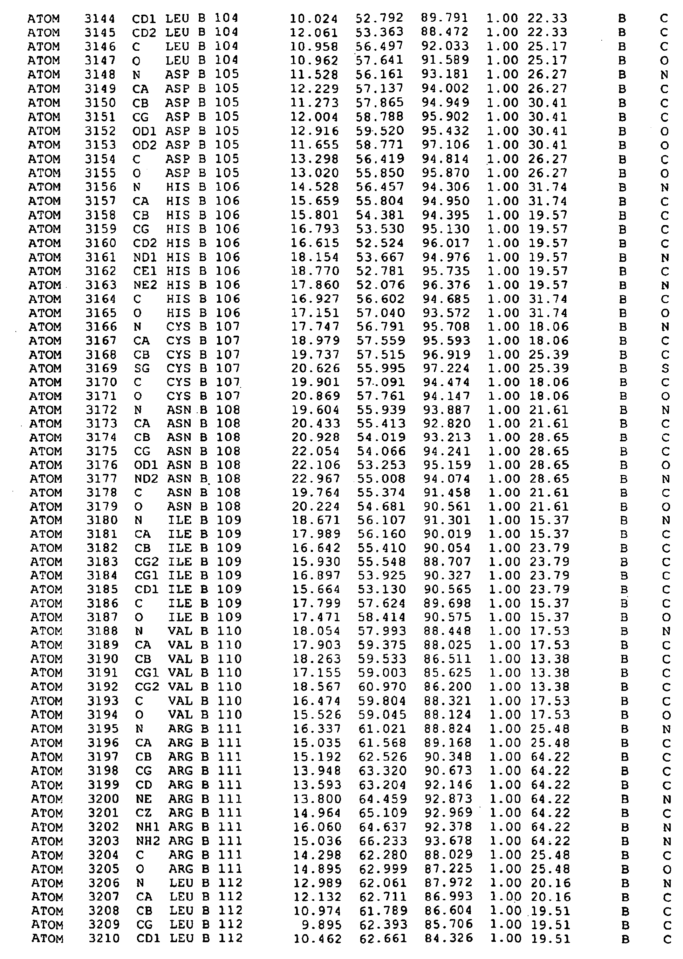

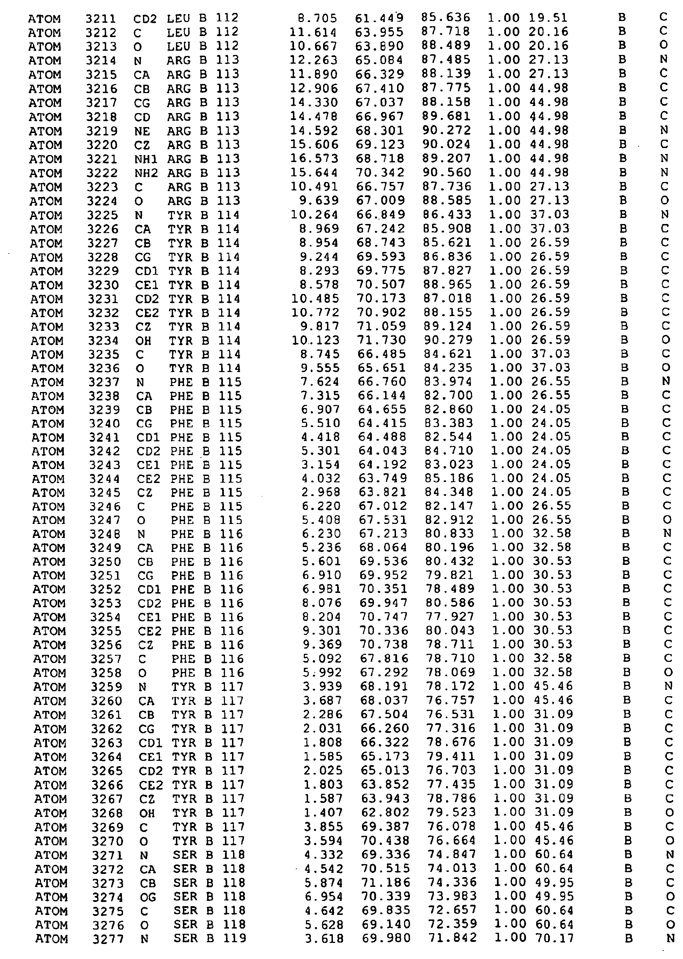

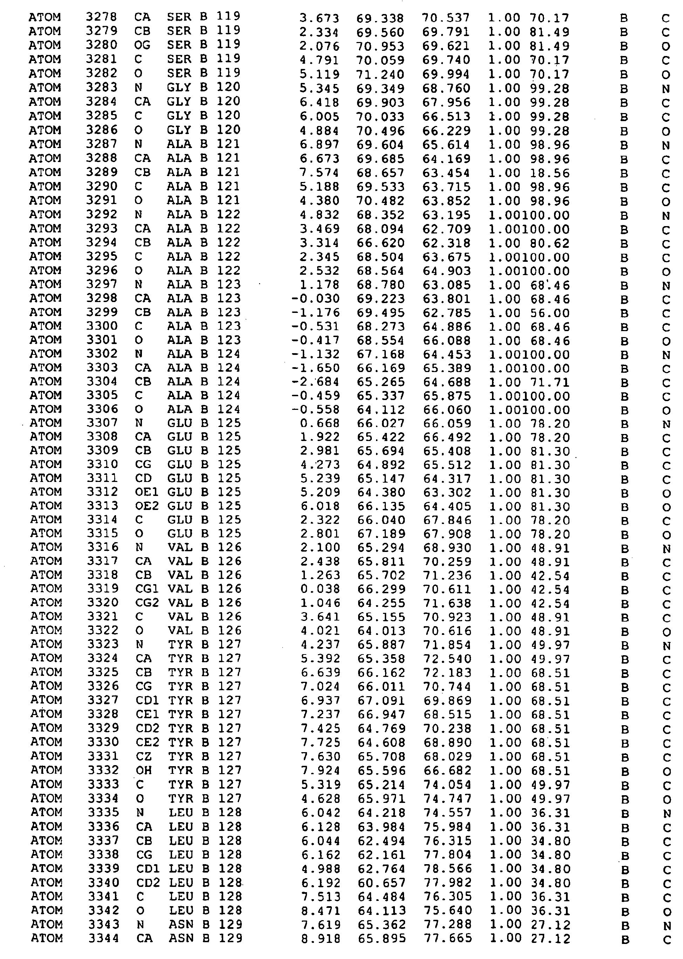

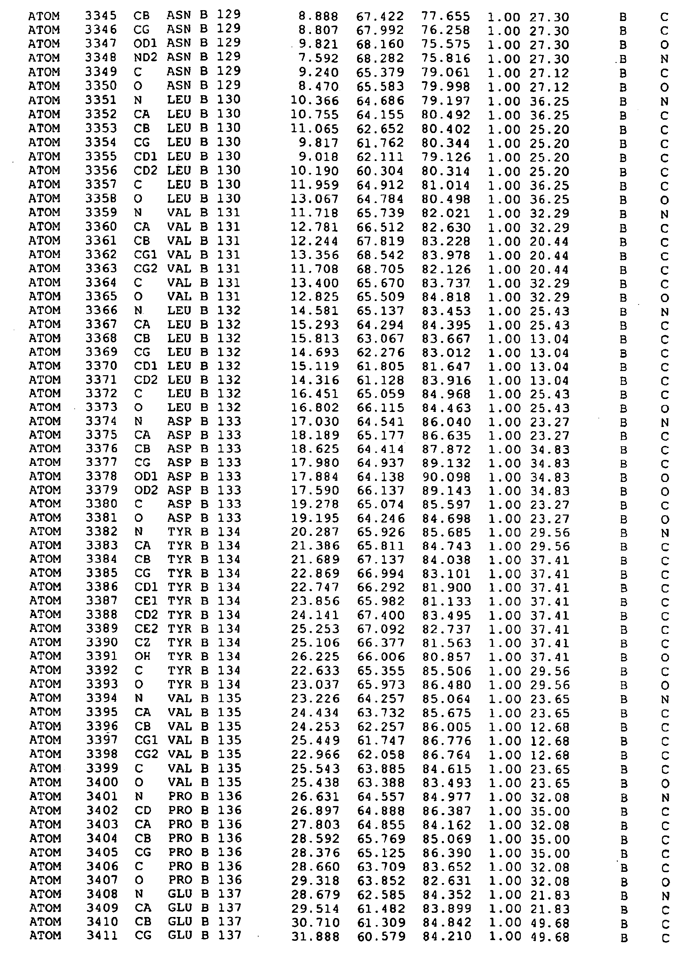

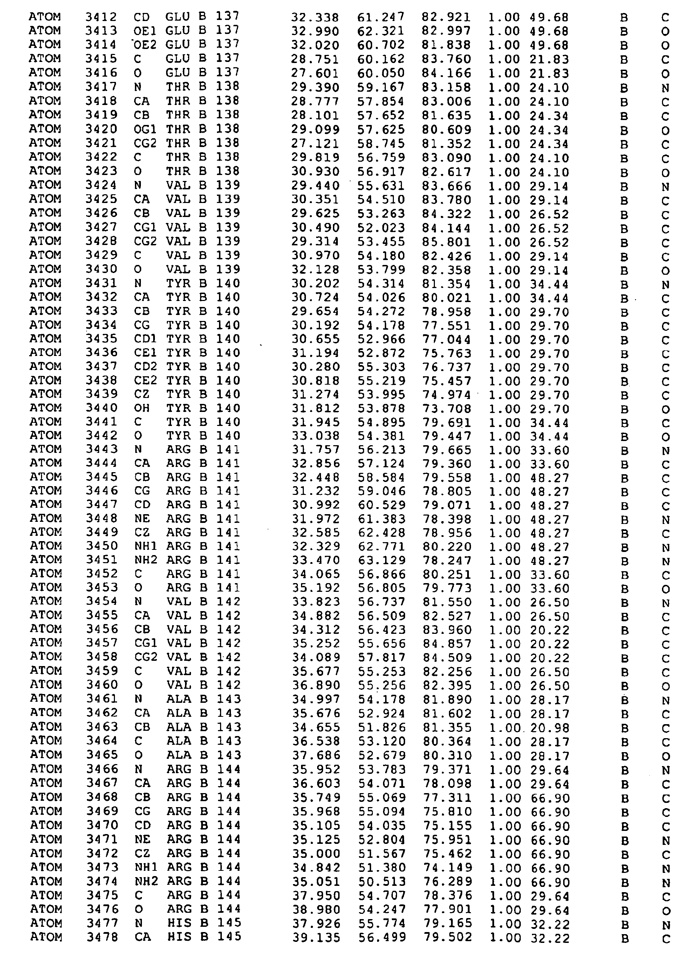

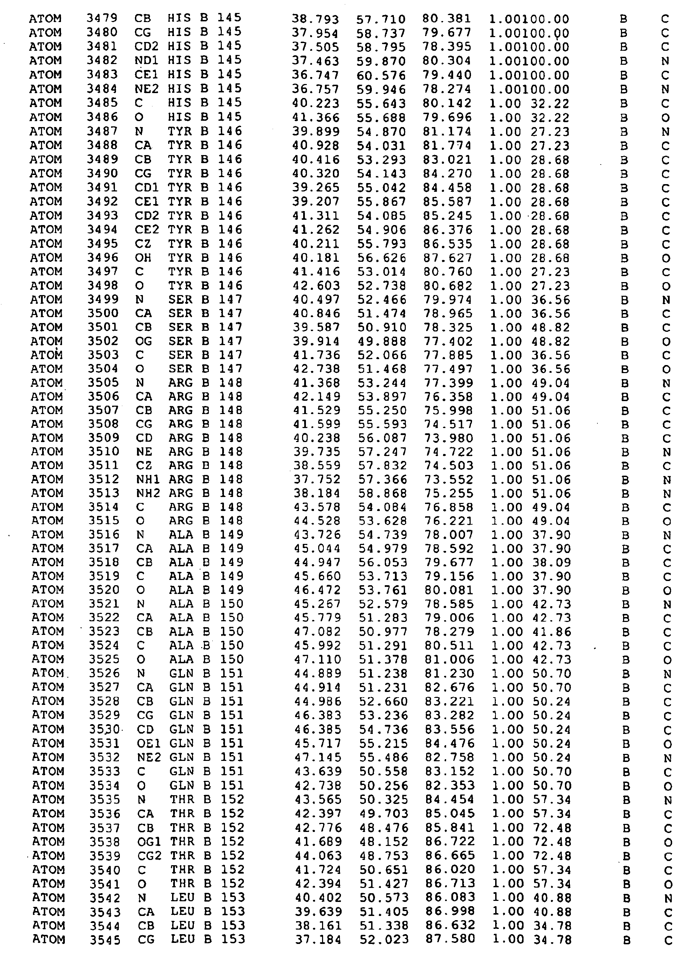

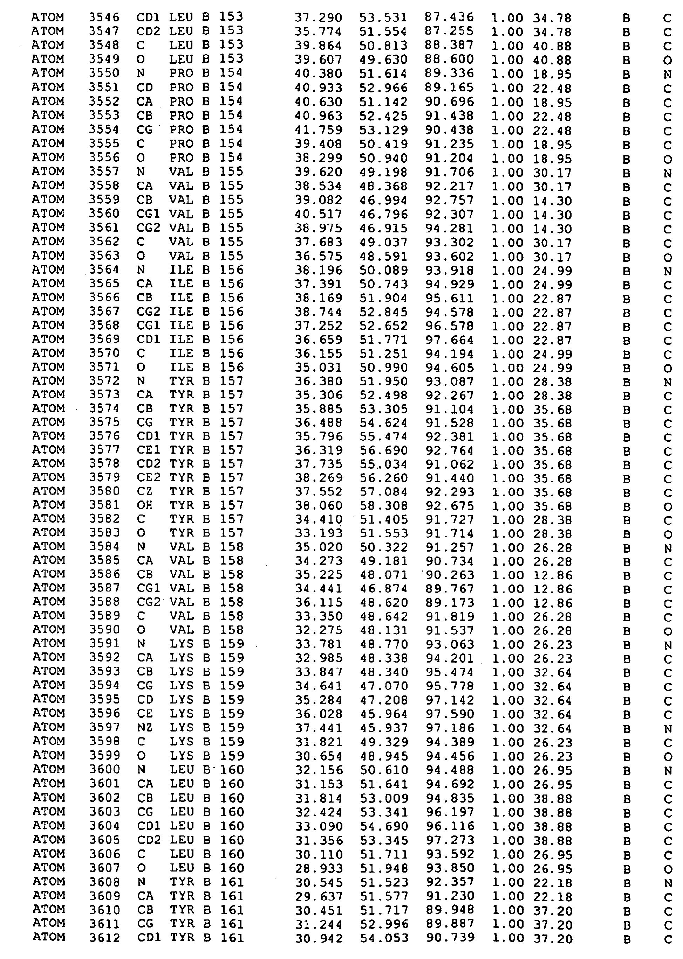

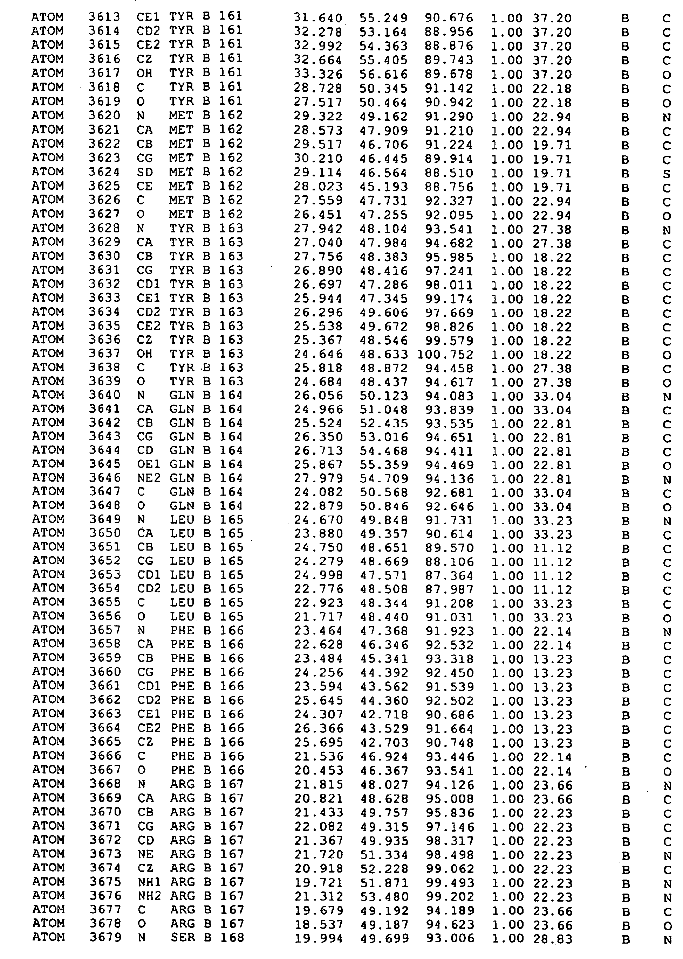

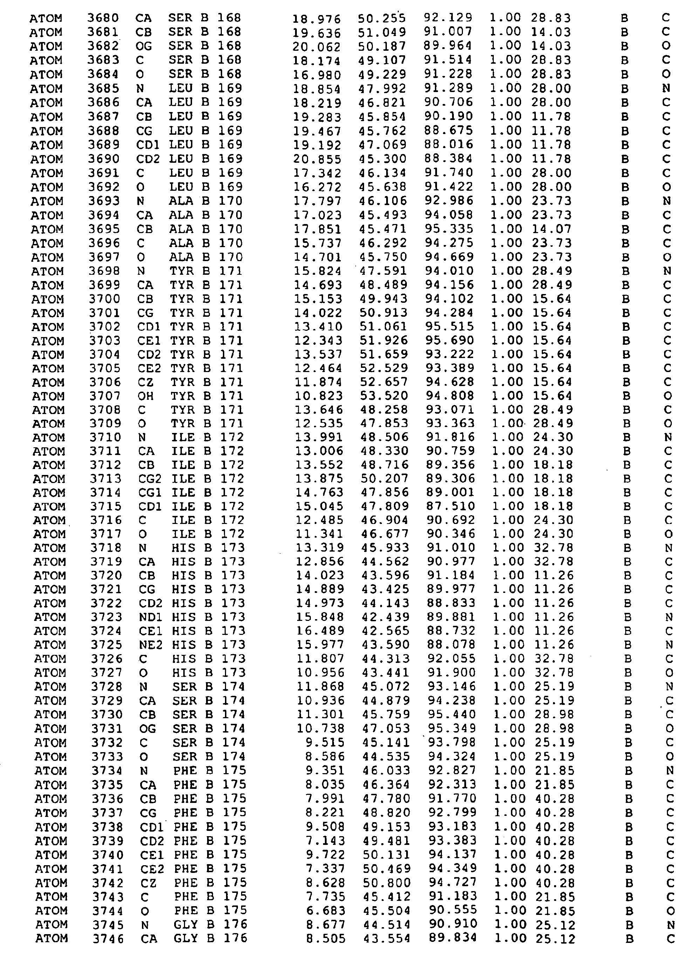

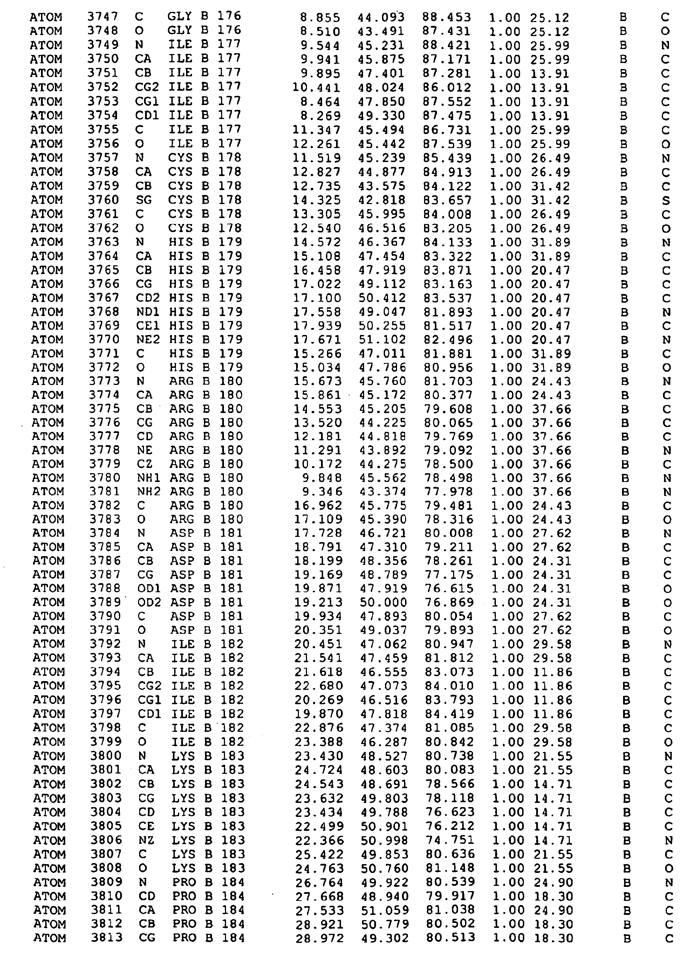

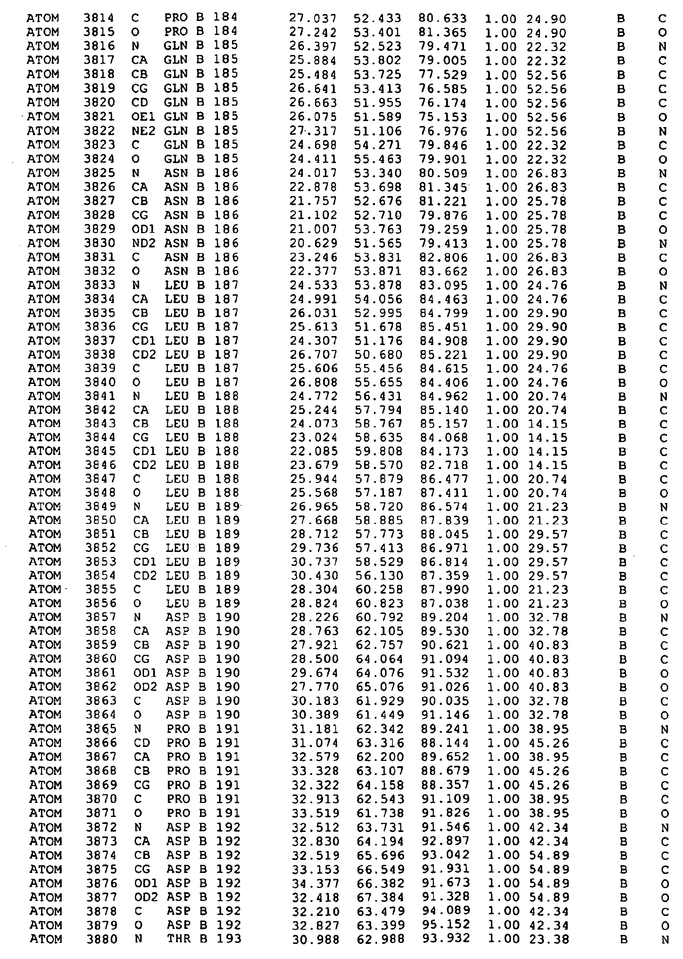

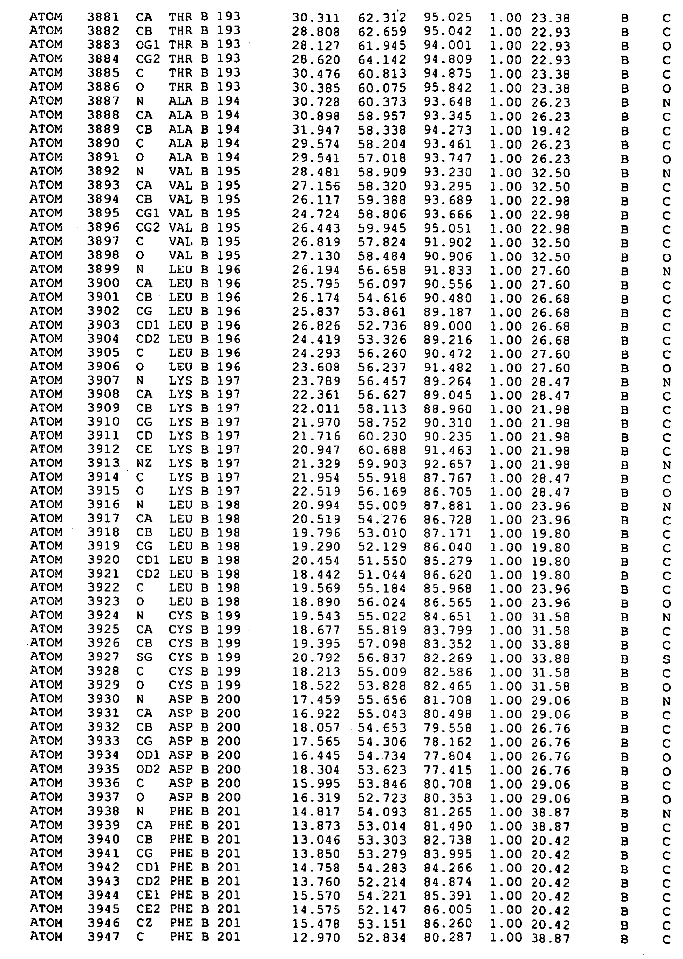

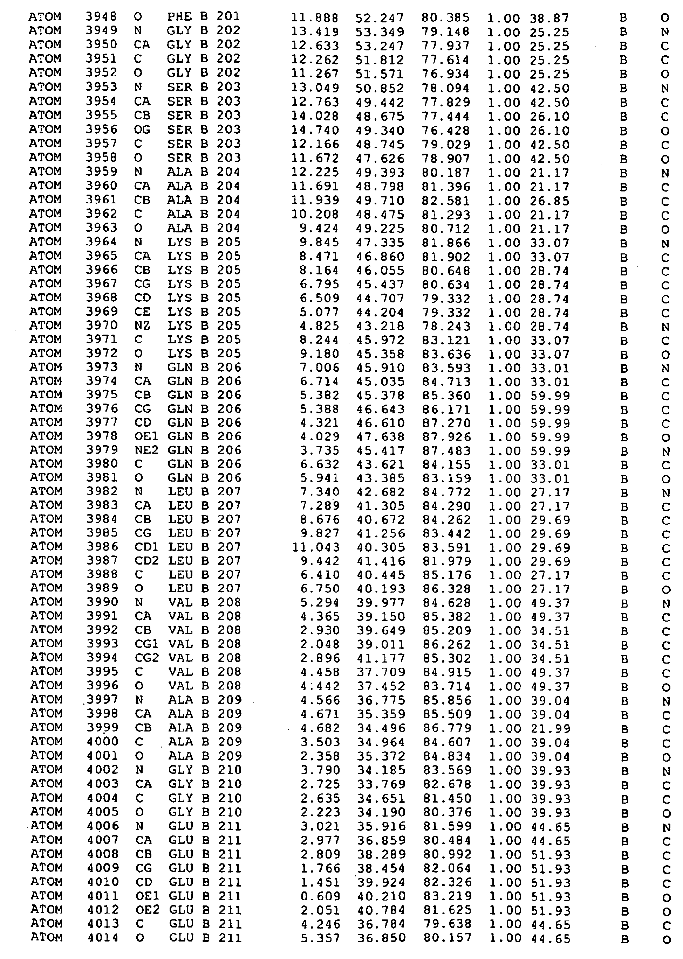

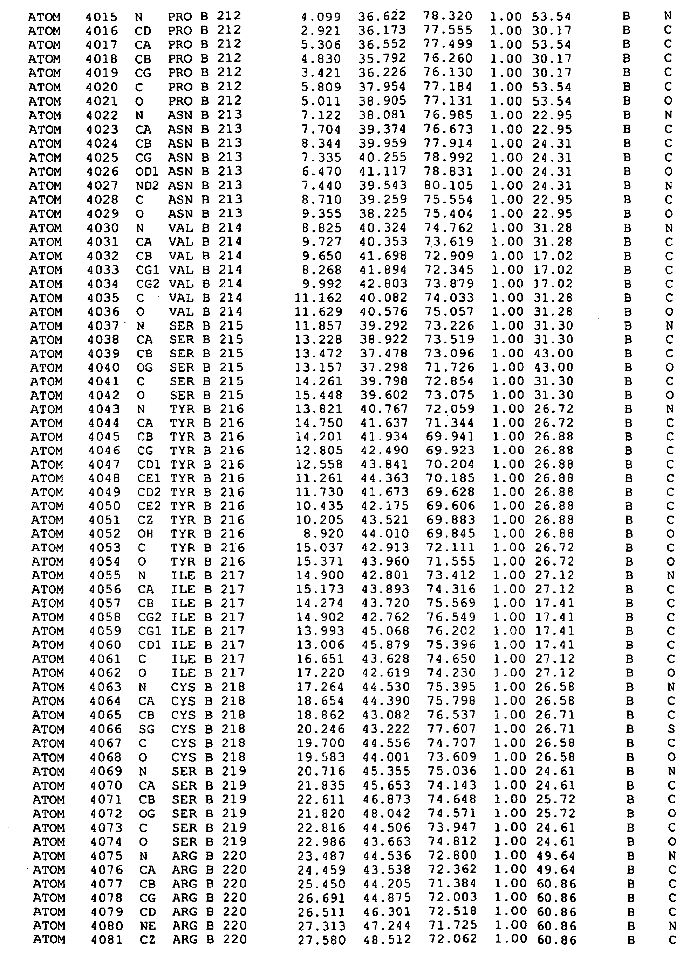

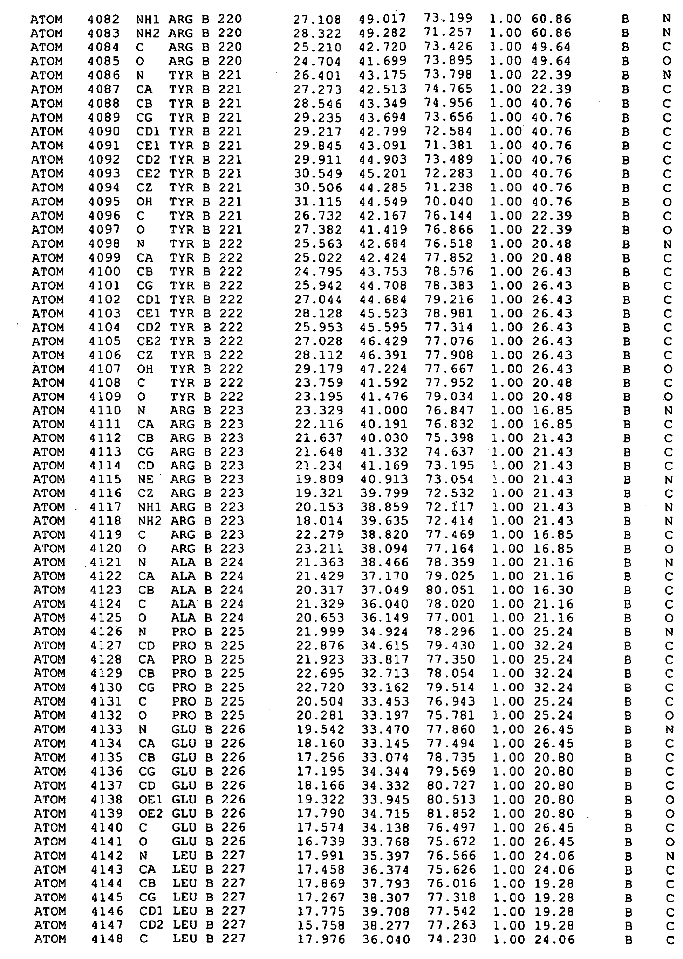

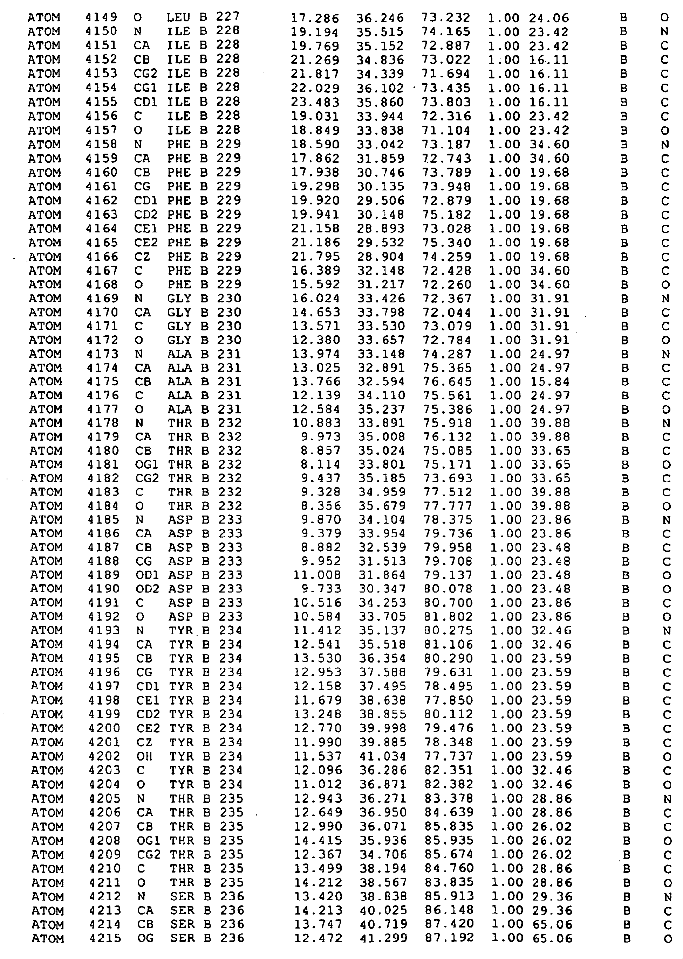

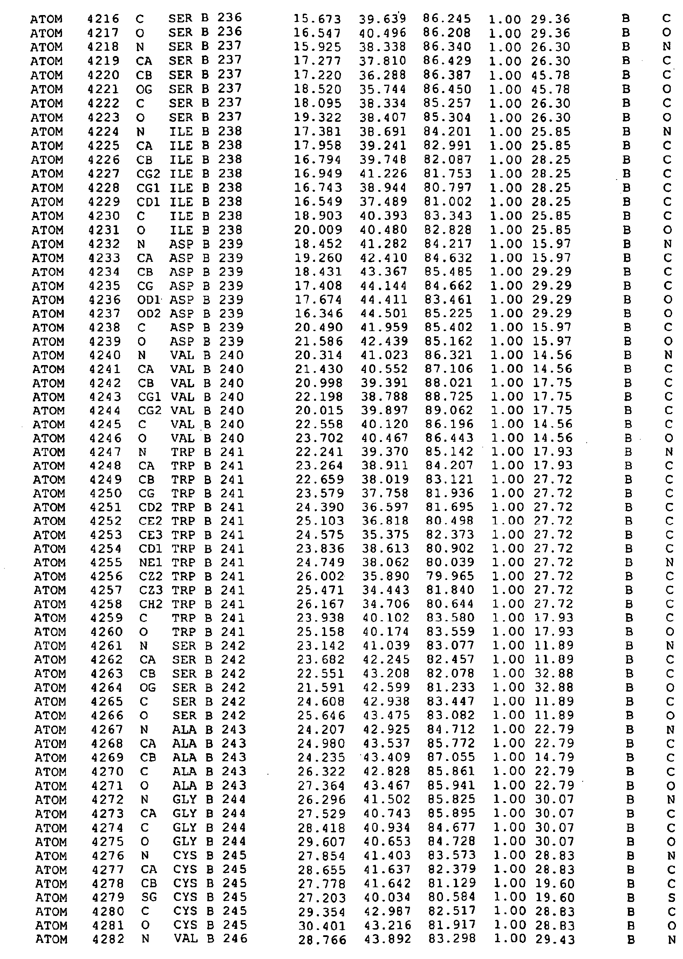

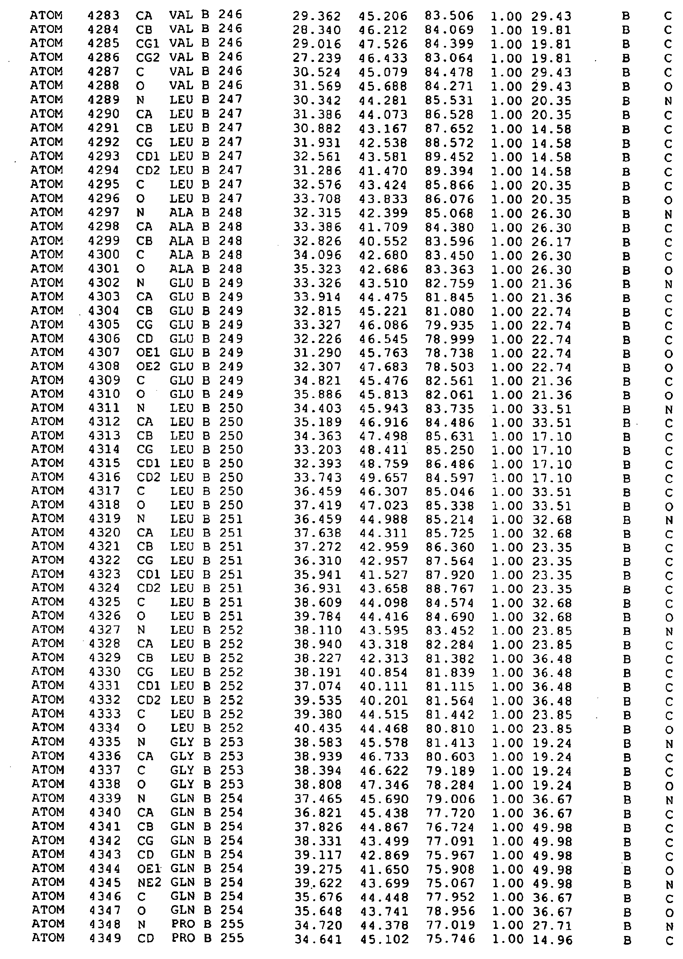

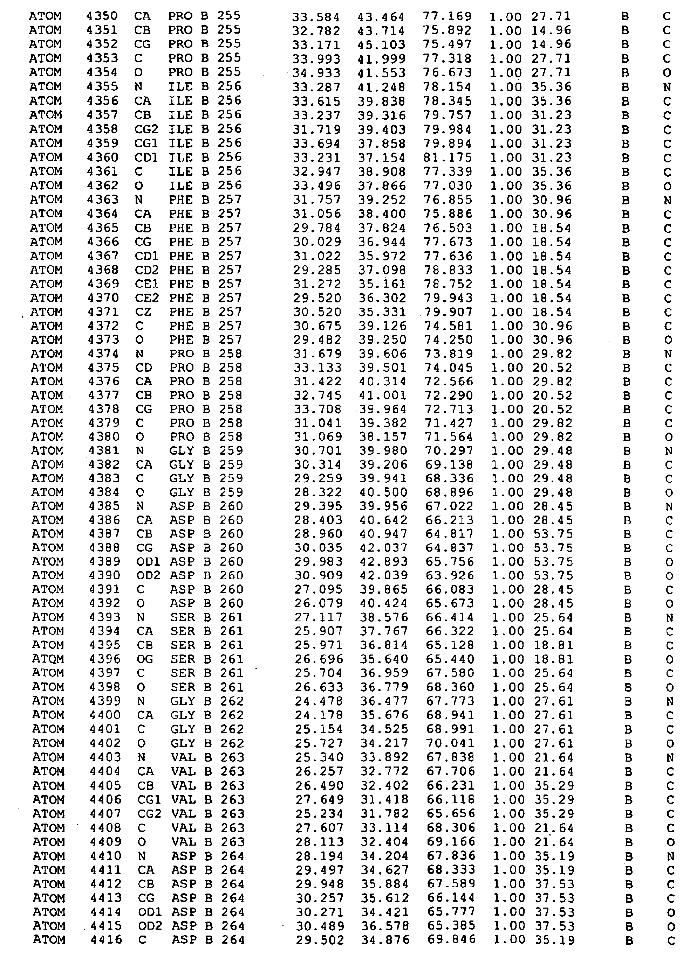

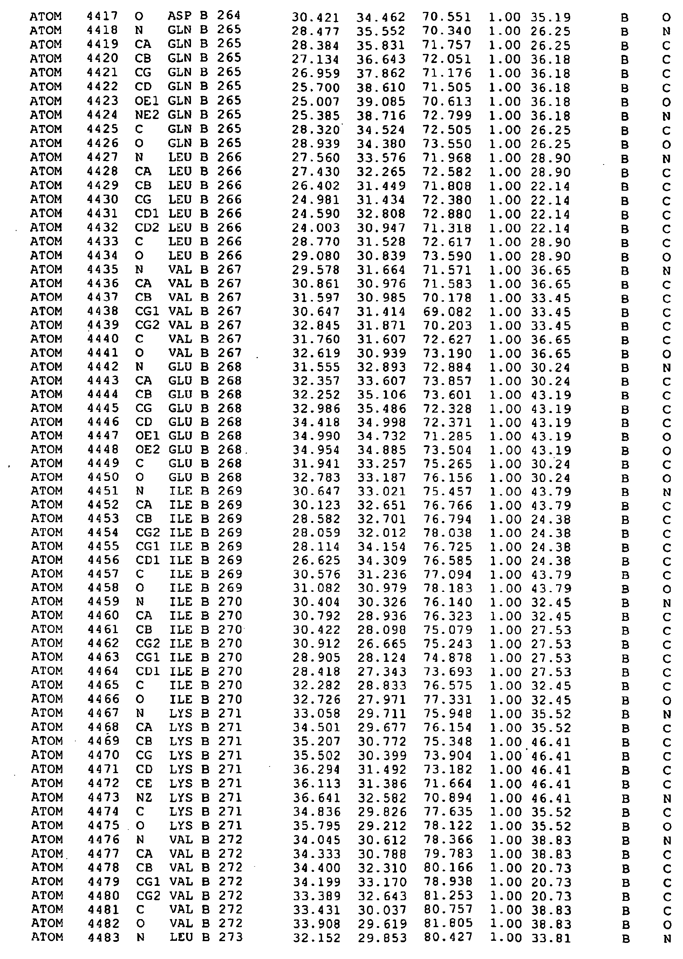

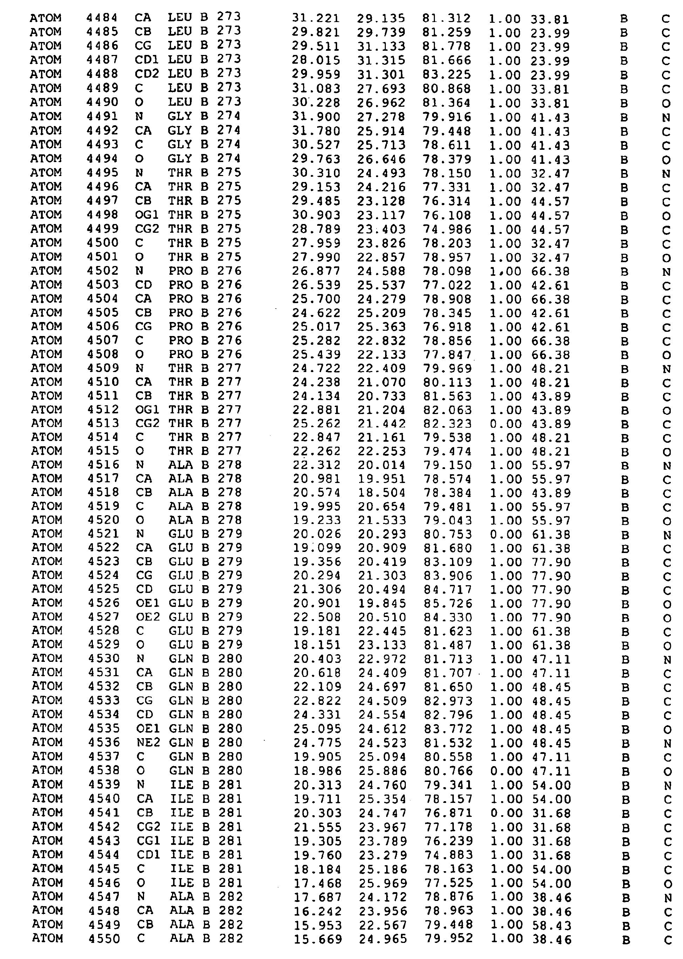

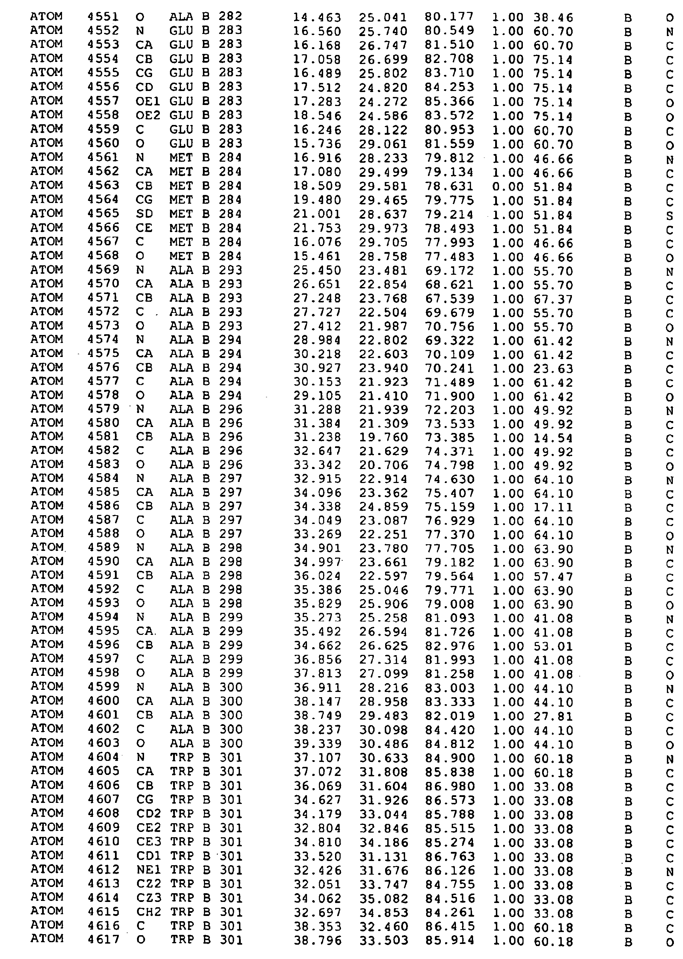

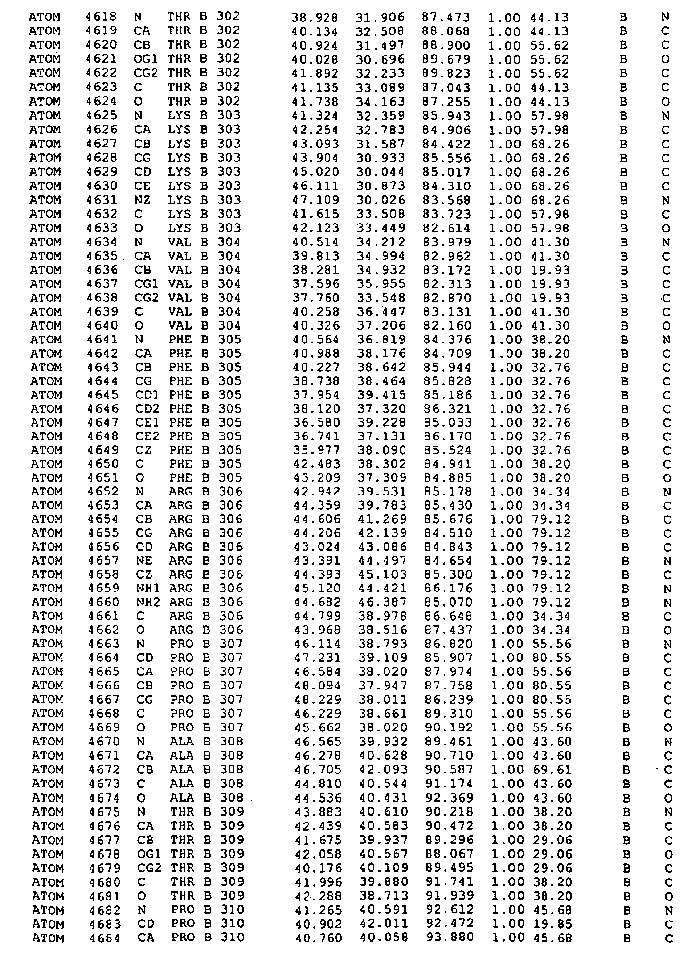

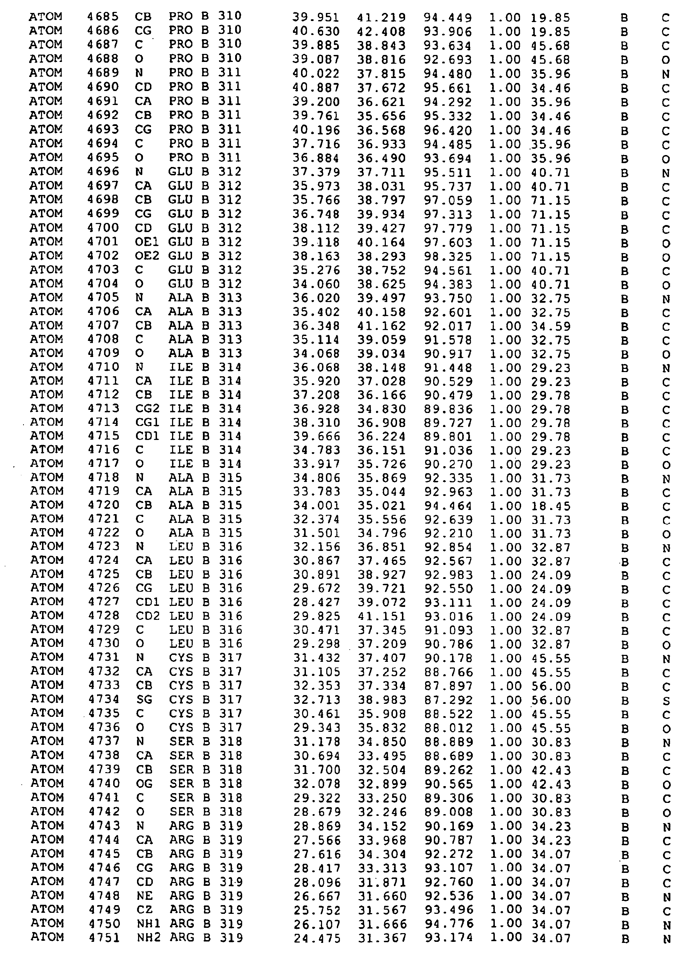

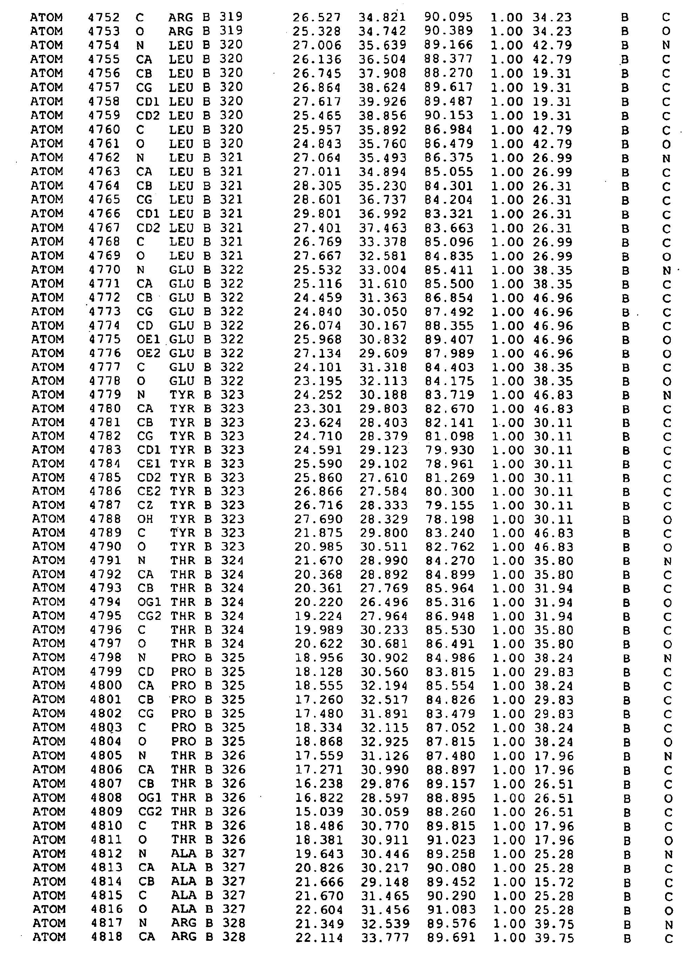

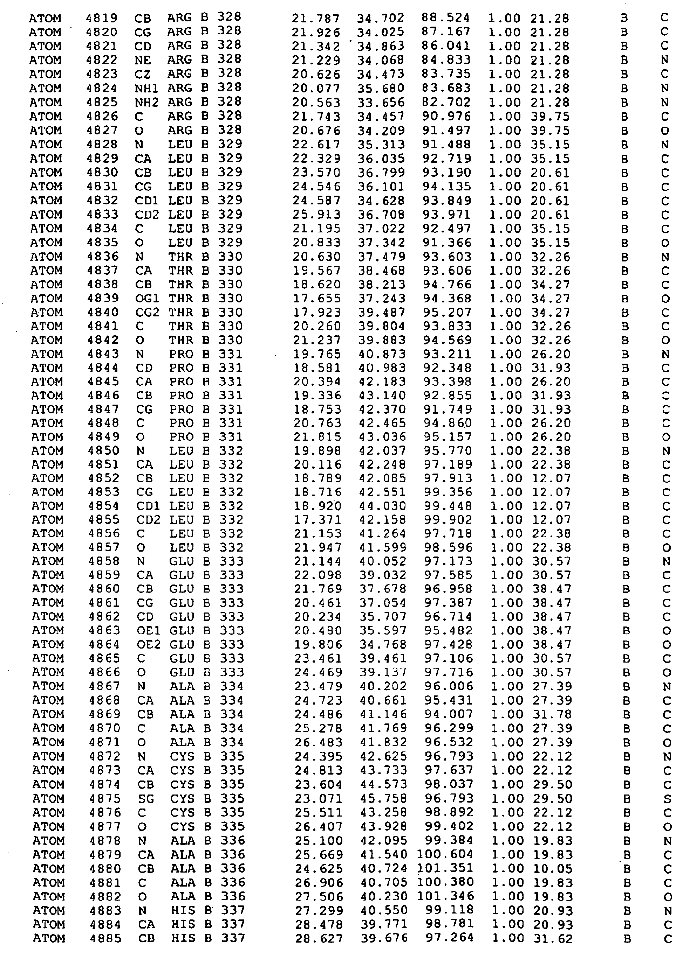

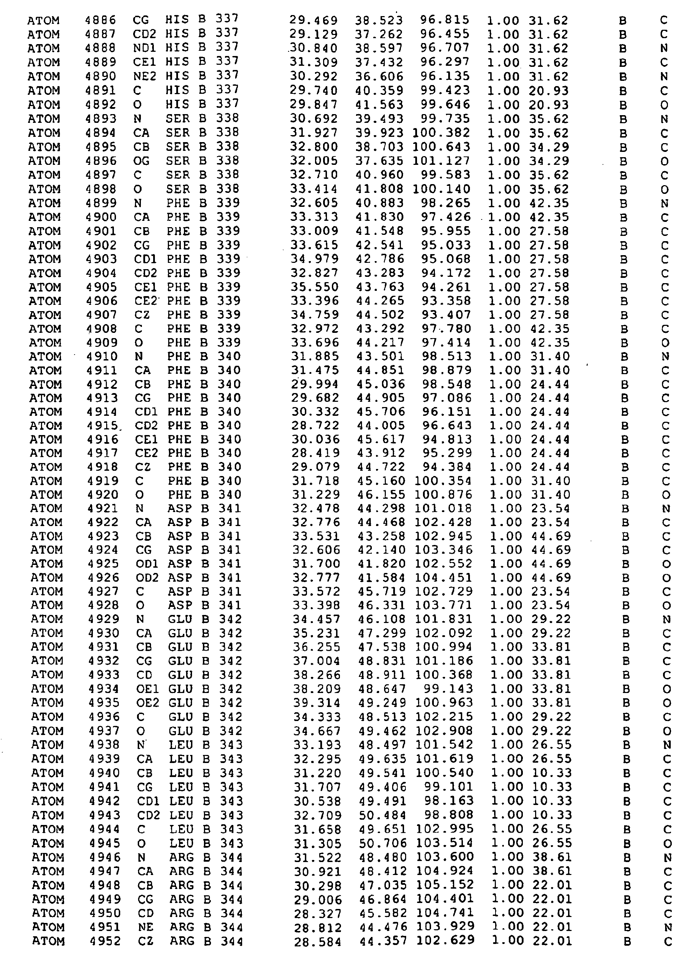

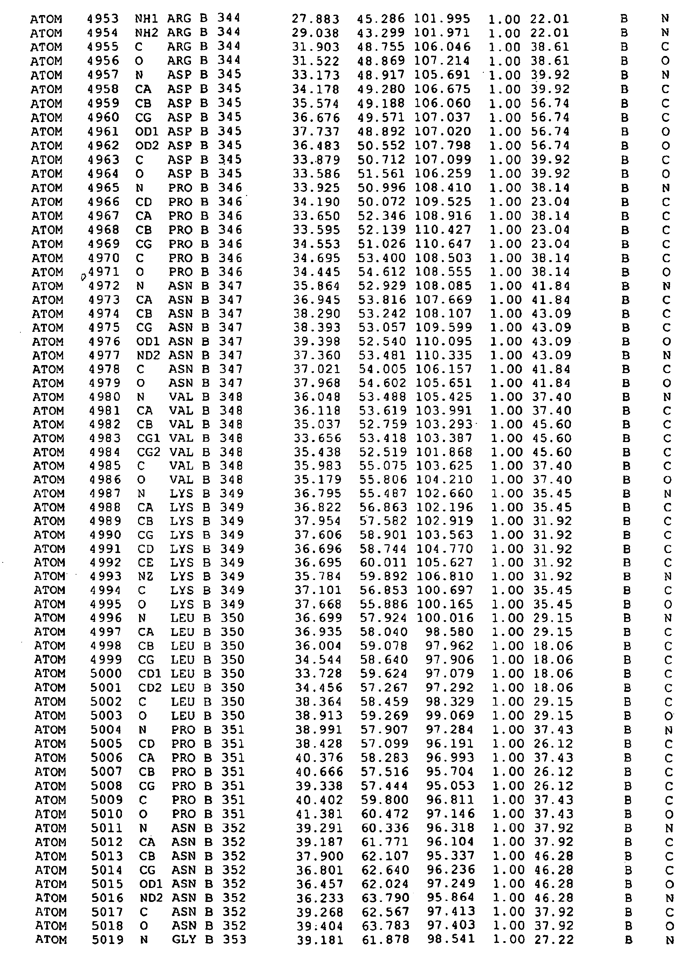

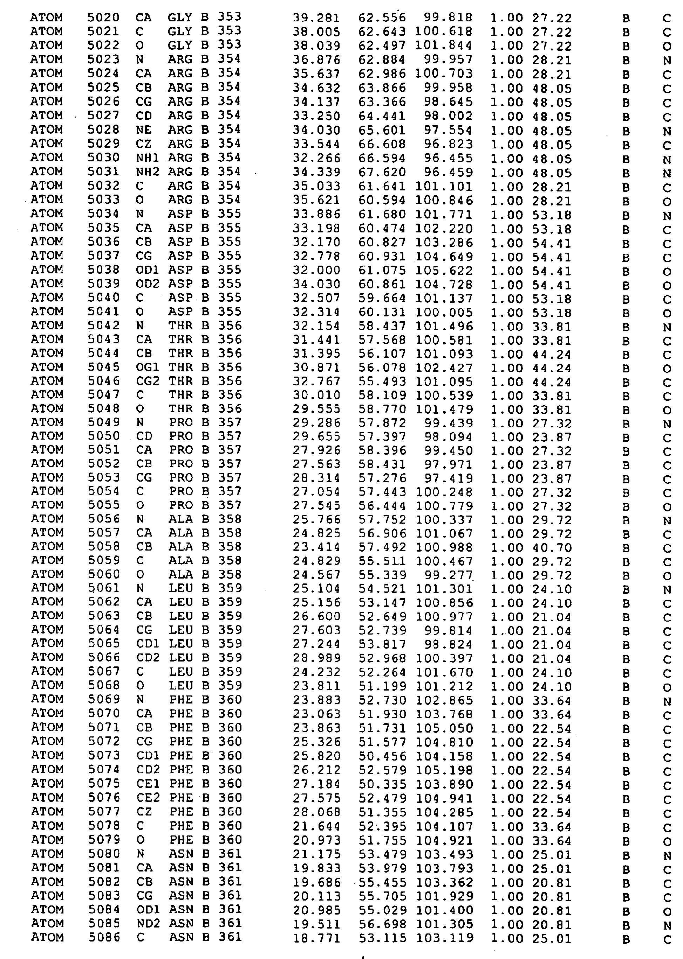

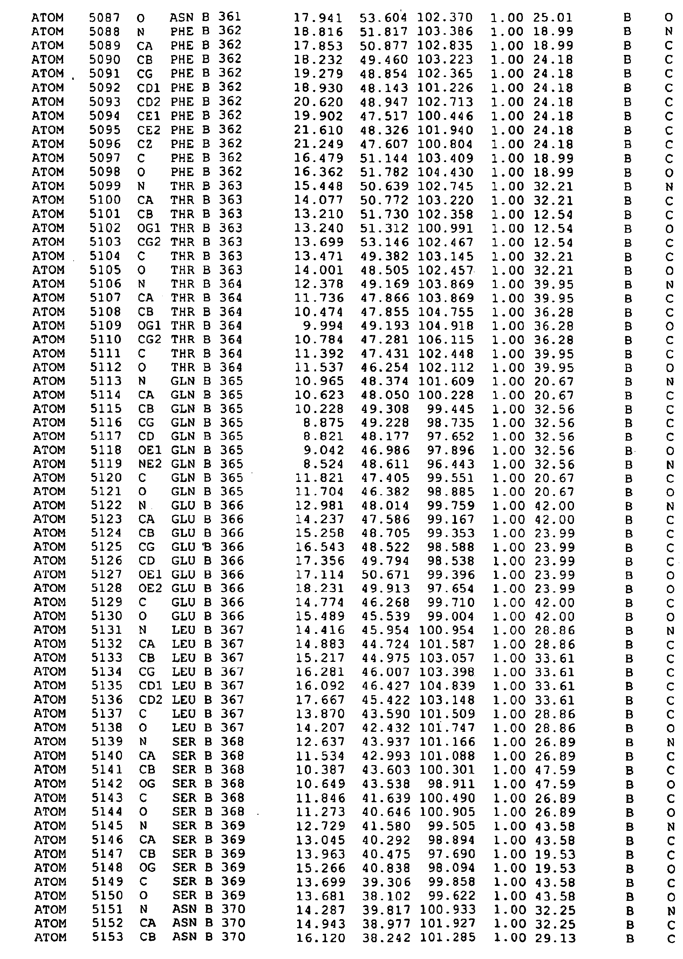

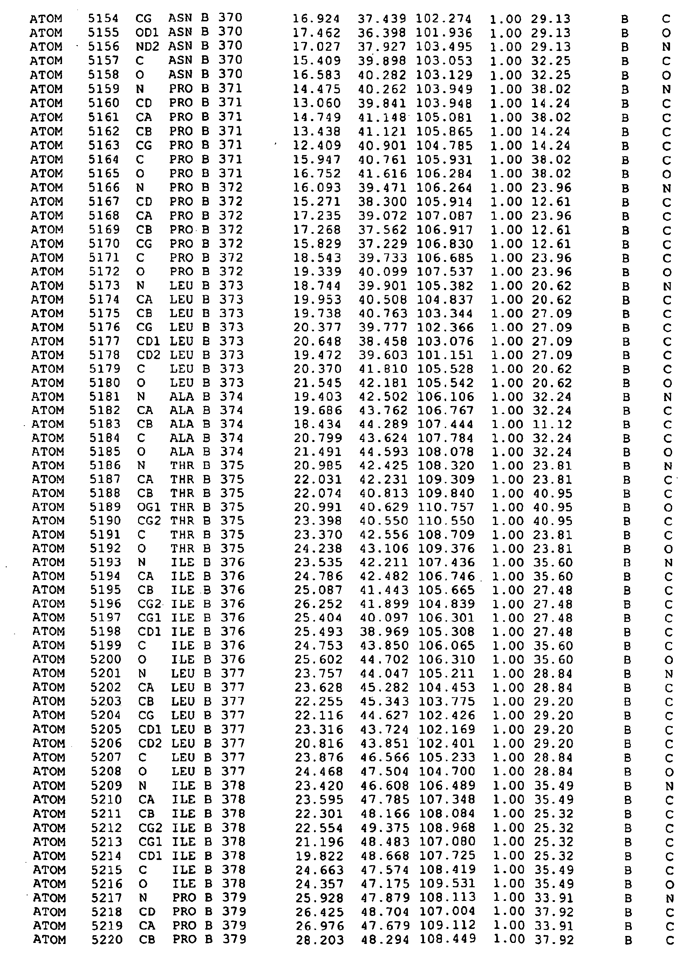

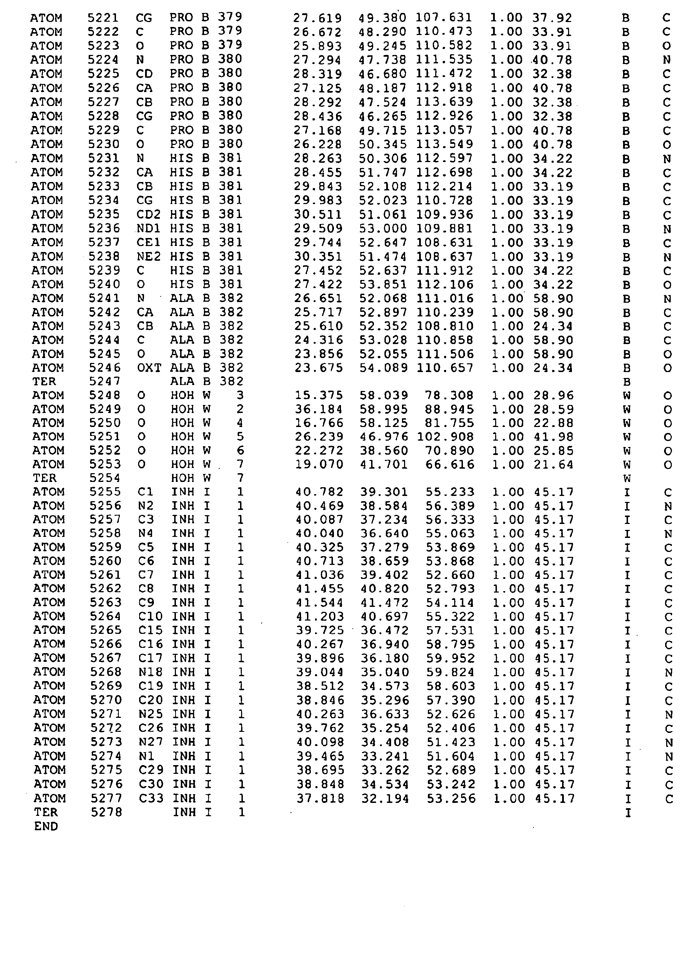

In the crystal structure of the unphosphorylated GSK-3β-inhibitor2 complex, amino acid residues I62, G63, V70, A83, V110, L132, D133, Y134, V135, P136, E137, T138, R141, L188 and C199 according to Figure 4 were within 5 Å of the inhibitor bound in the active site. These amino acid residues were identified using the programs QUANTA, O and RIBBONS. In addition, amino acid residues V61, I62, G63, N64, G65, G68, V69, V70, Y71, Q72, L81, V82, A83, I84, K85, E97, M101, V110, R111, L130, V131, L132, D133, Y134, V135, P136, E137, T138, V139, Y140, R141, Q185, N186, L187, L188, L189, K197, L198, C199, D200 and F201 according to Figure 4 were within 8 Å of the inhibitor bound in the active site. These amino acids residues were identified using the programs QUANTA, O and RIBBONS.

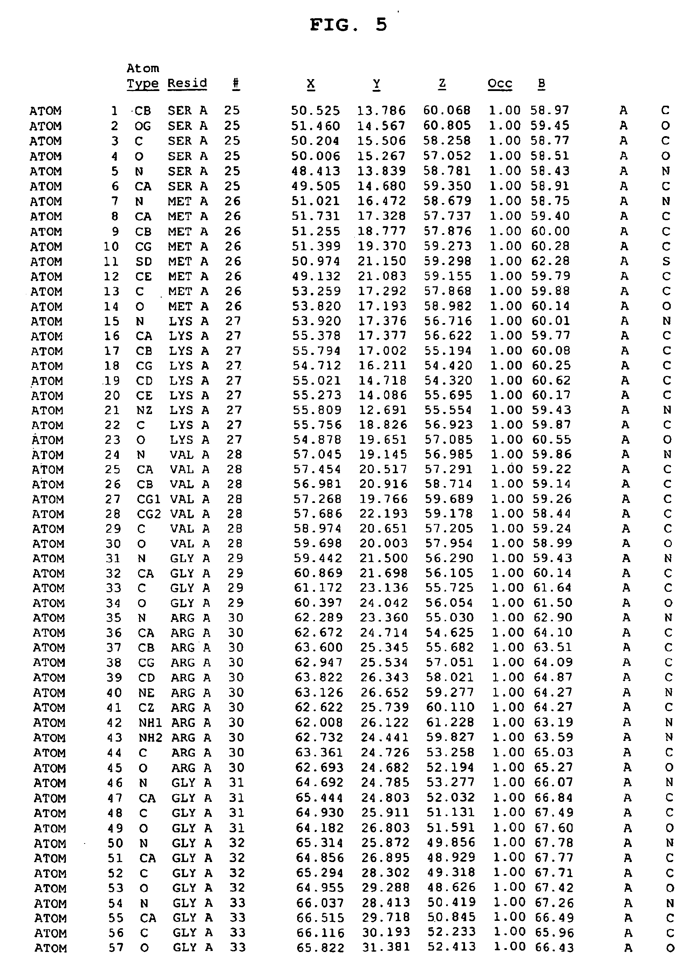

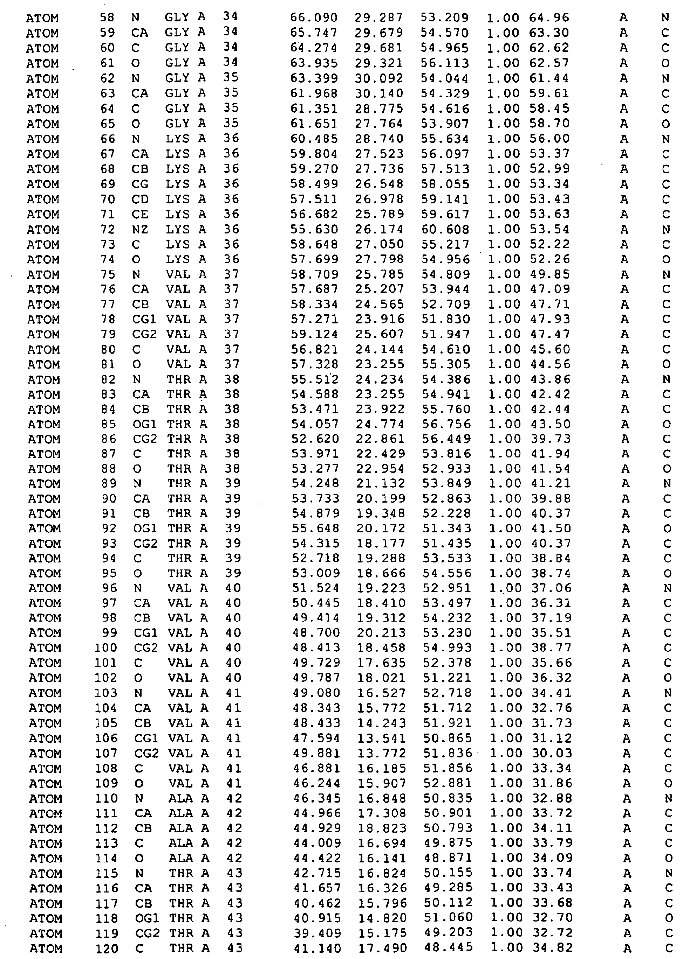

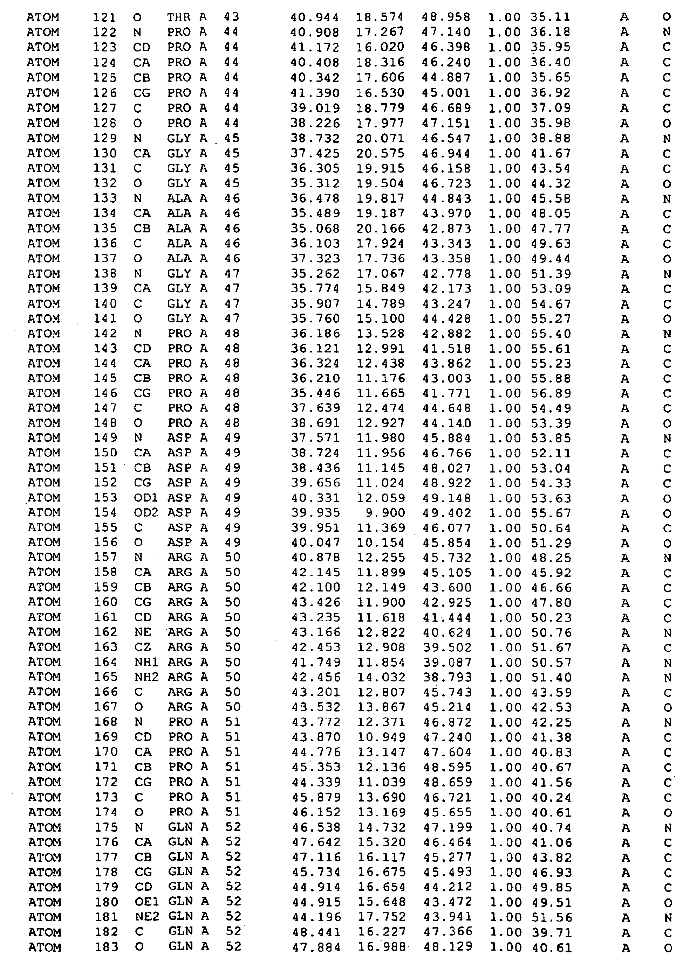

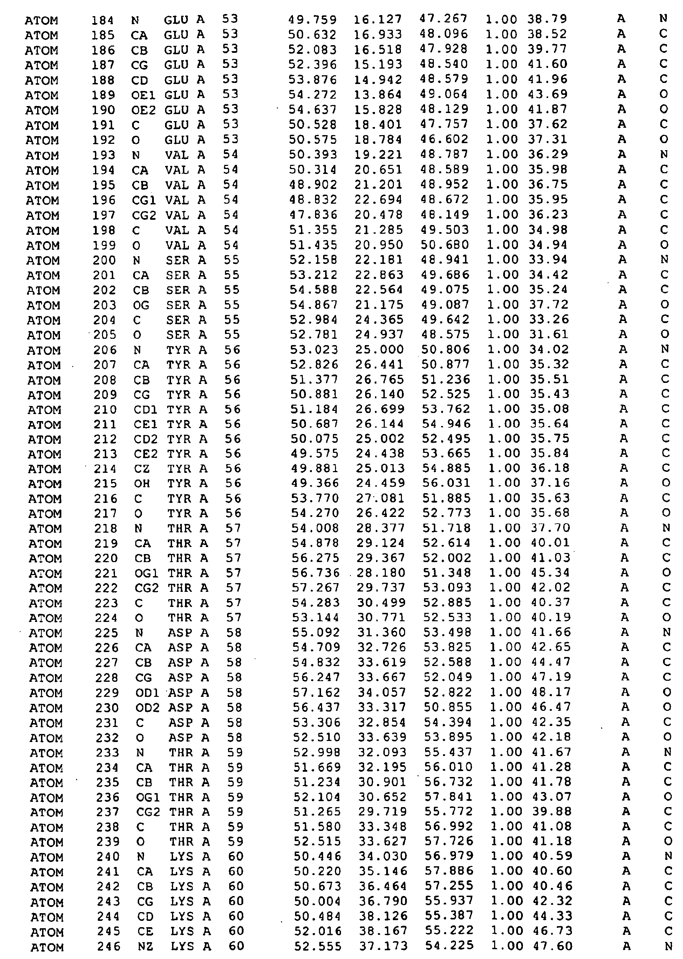

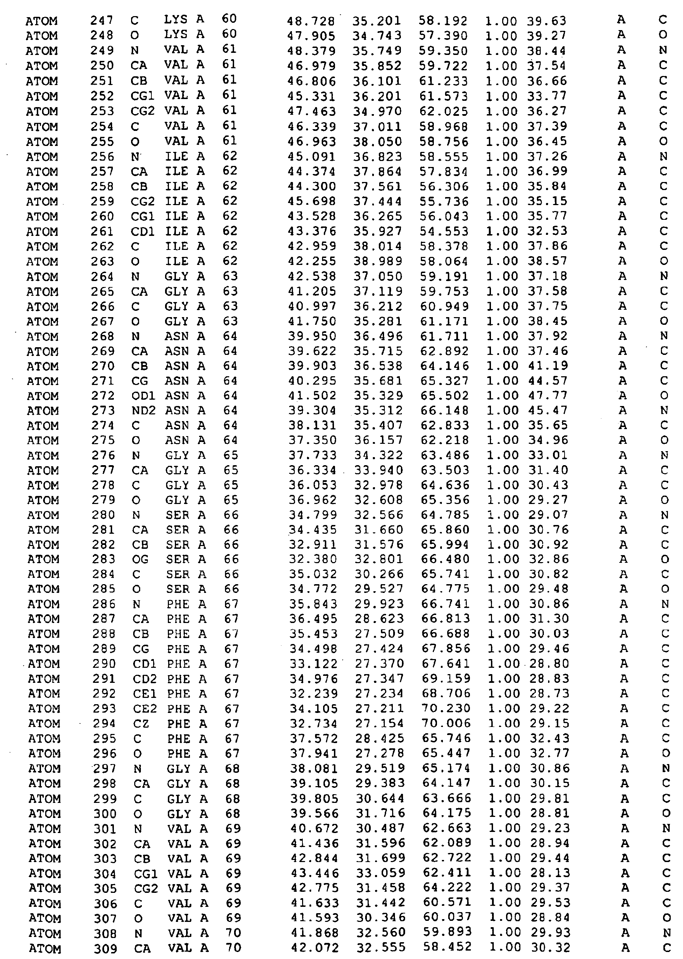

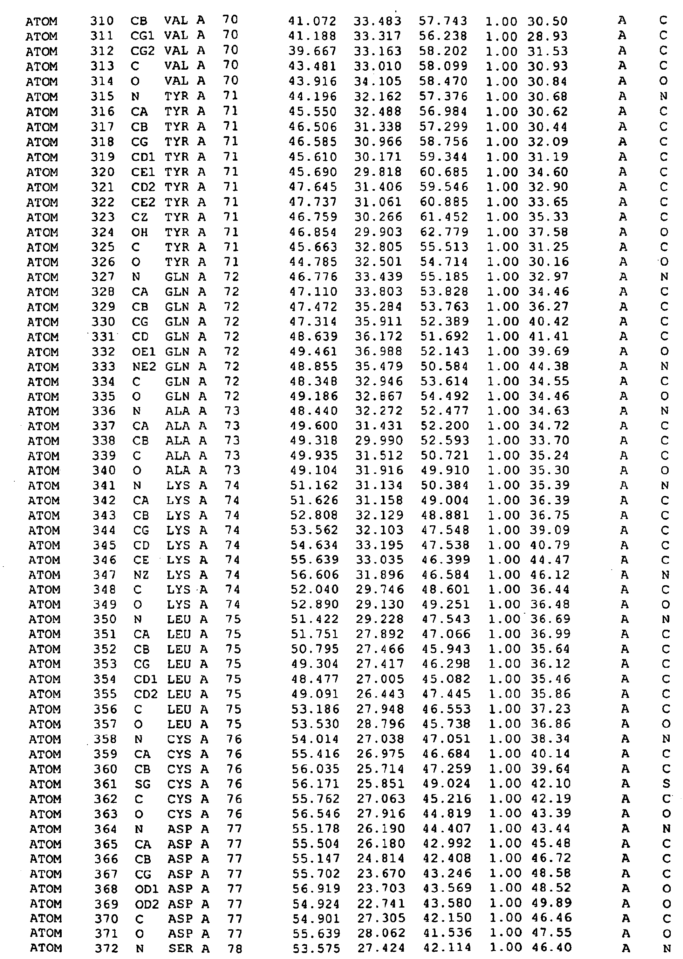

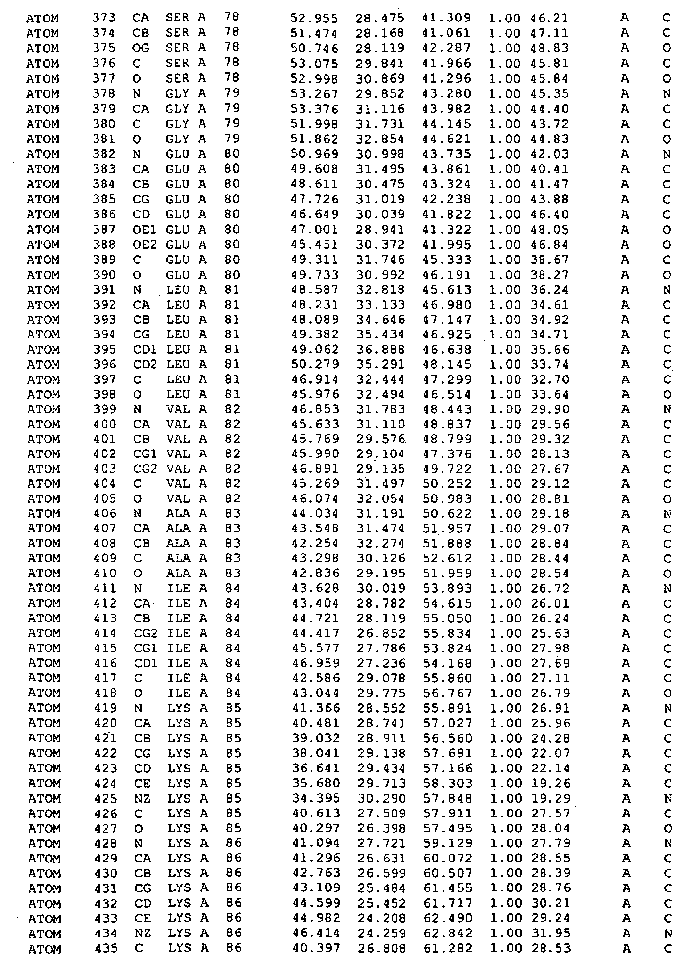

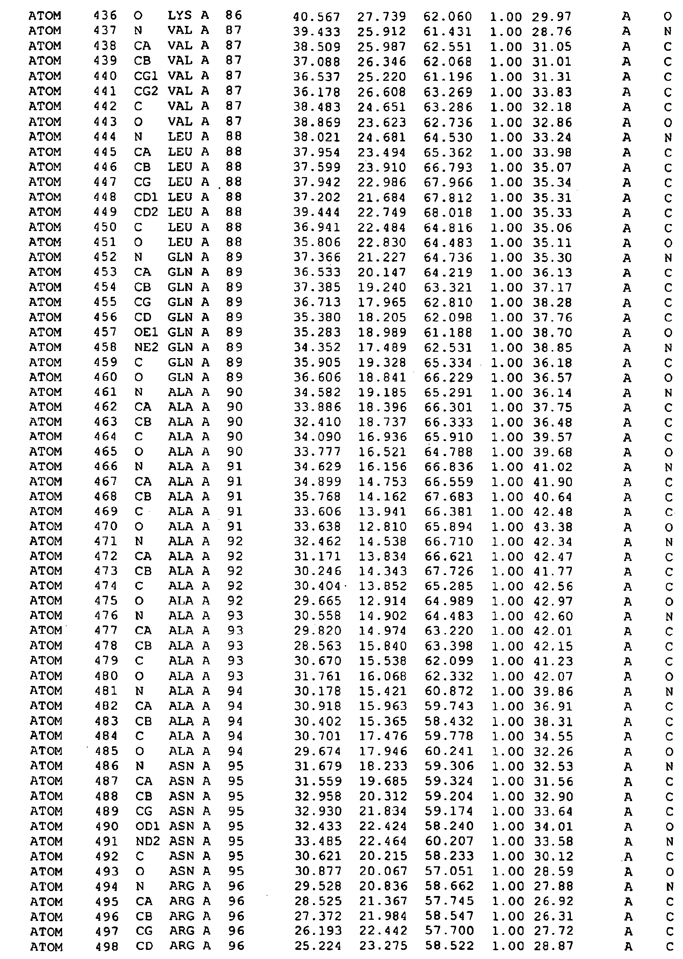

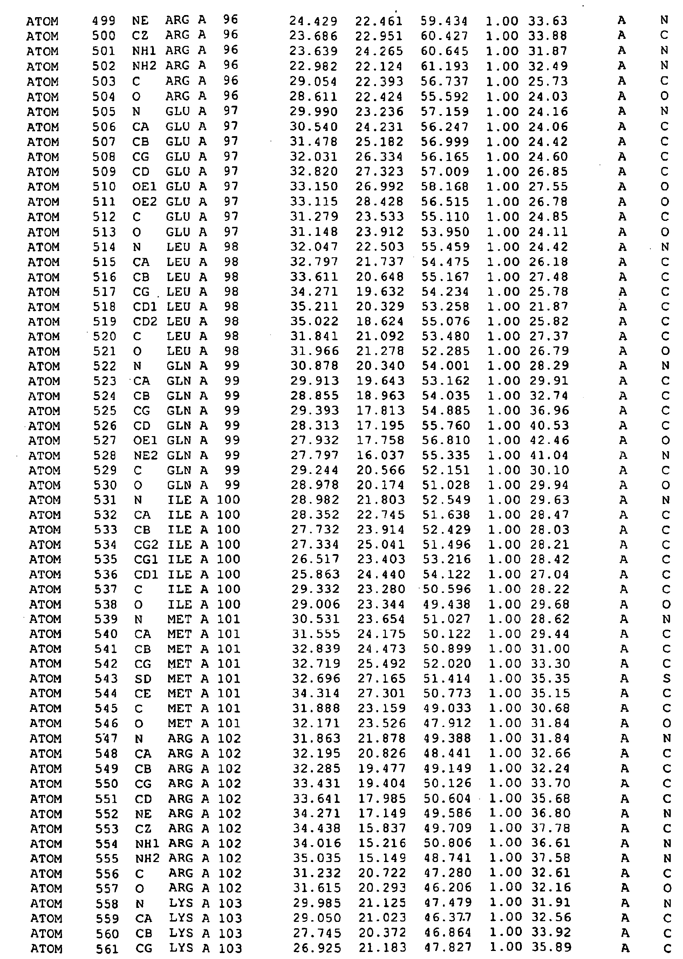

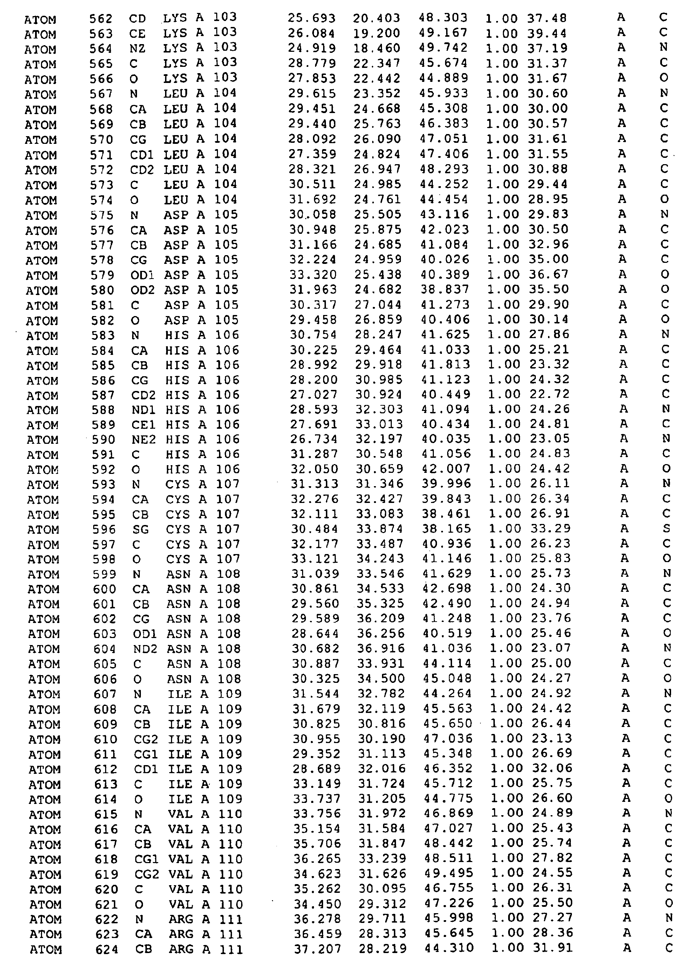

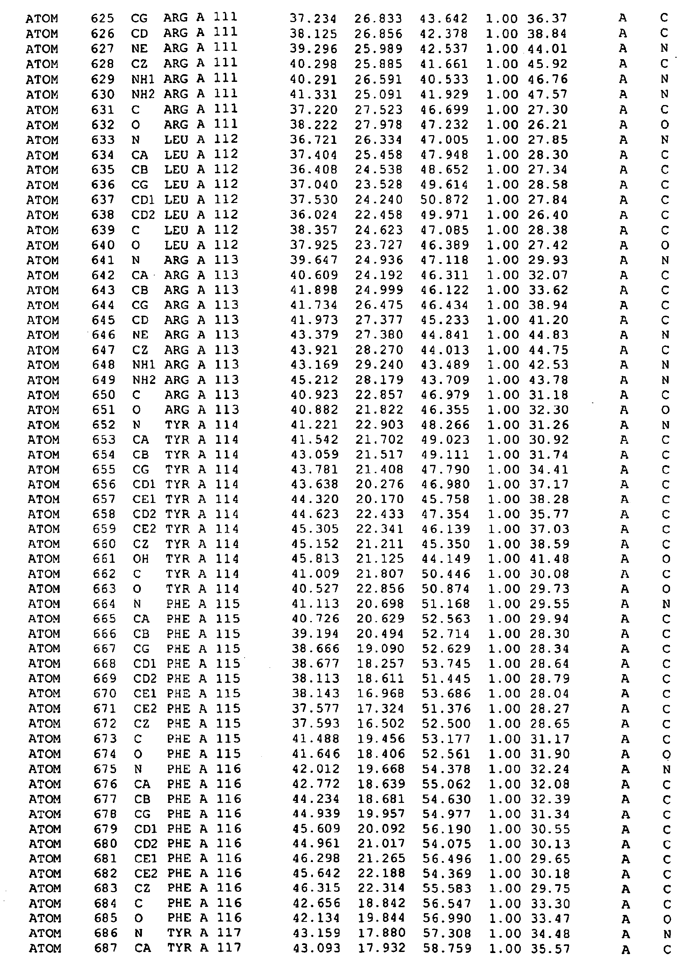

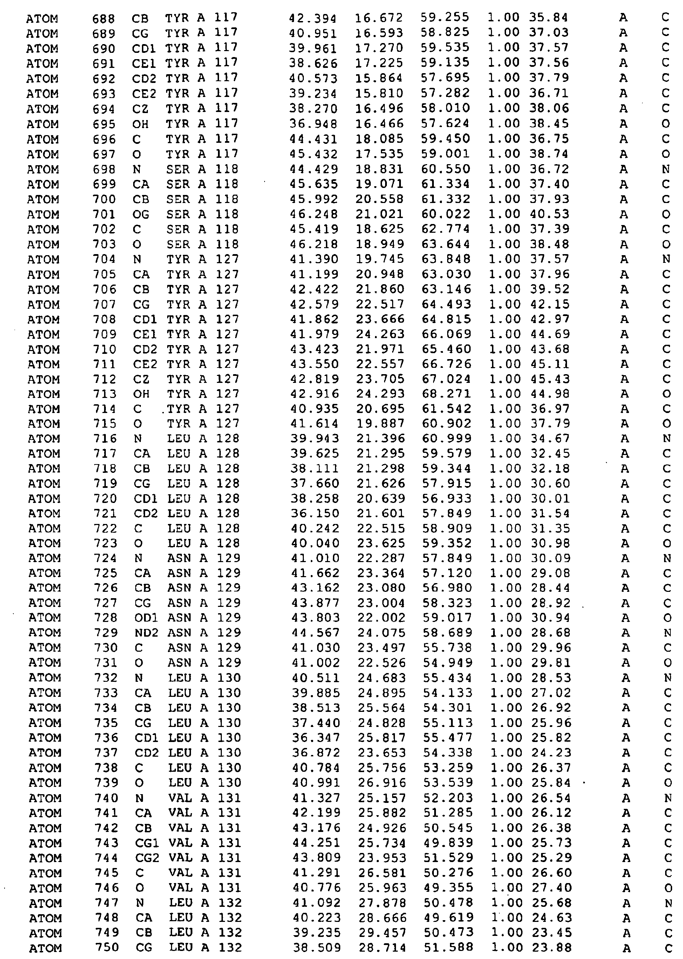

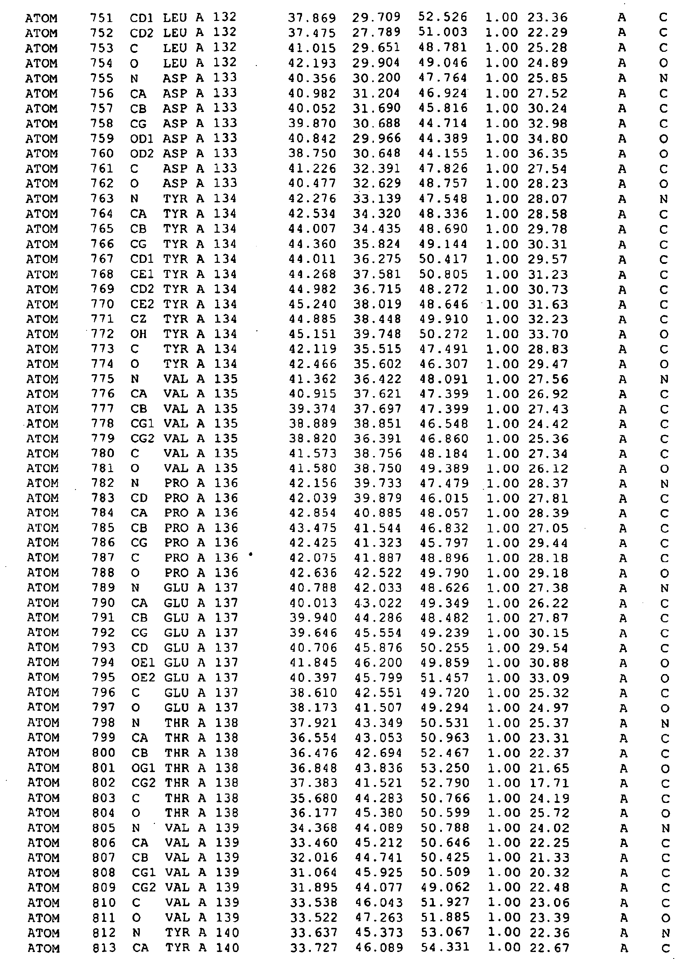

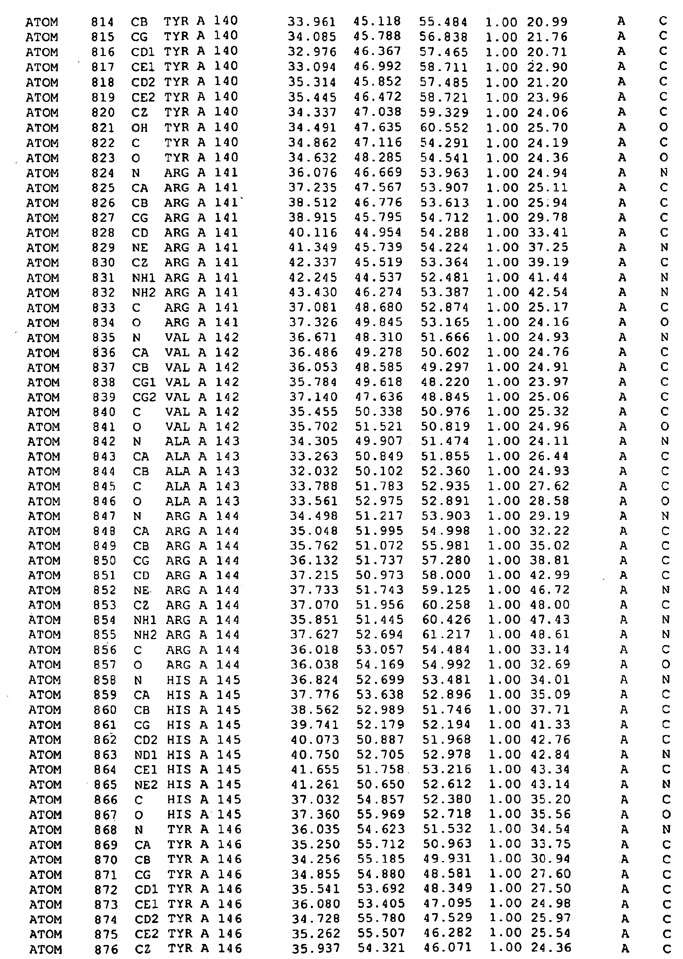

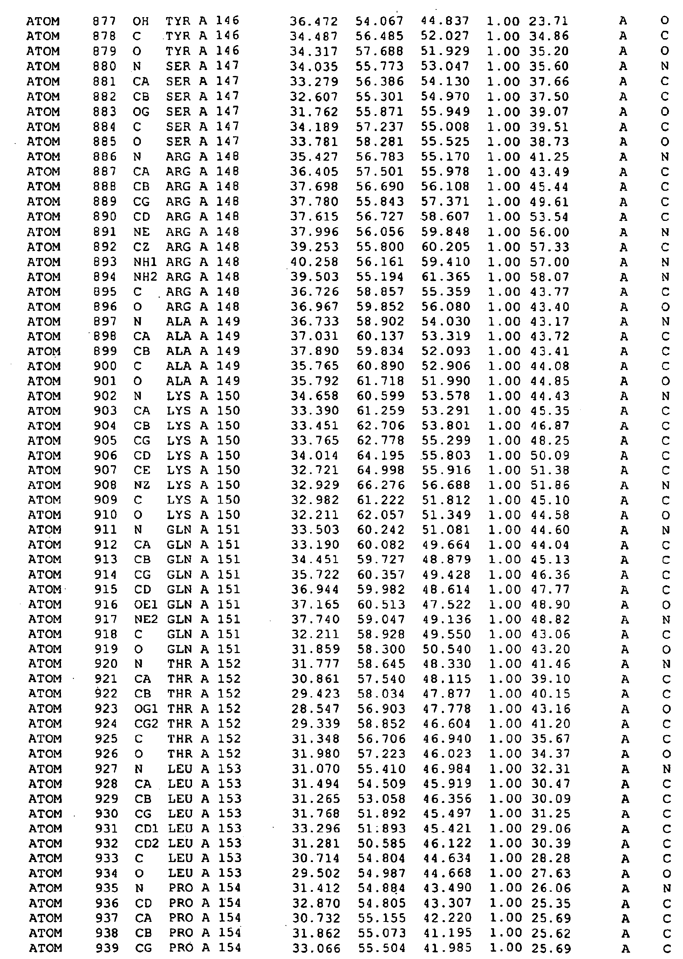

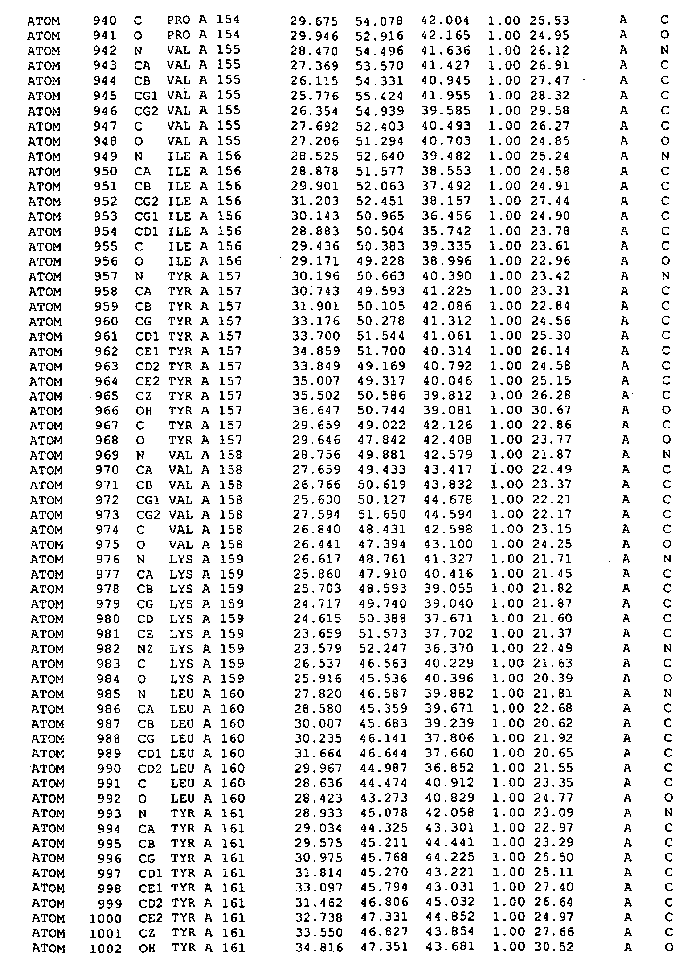

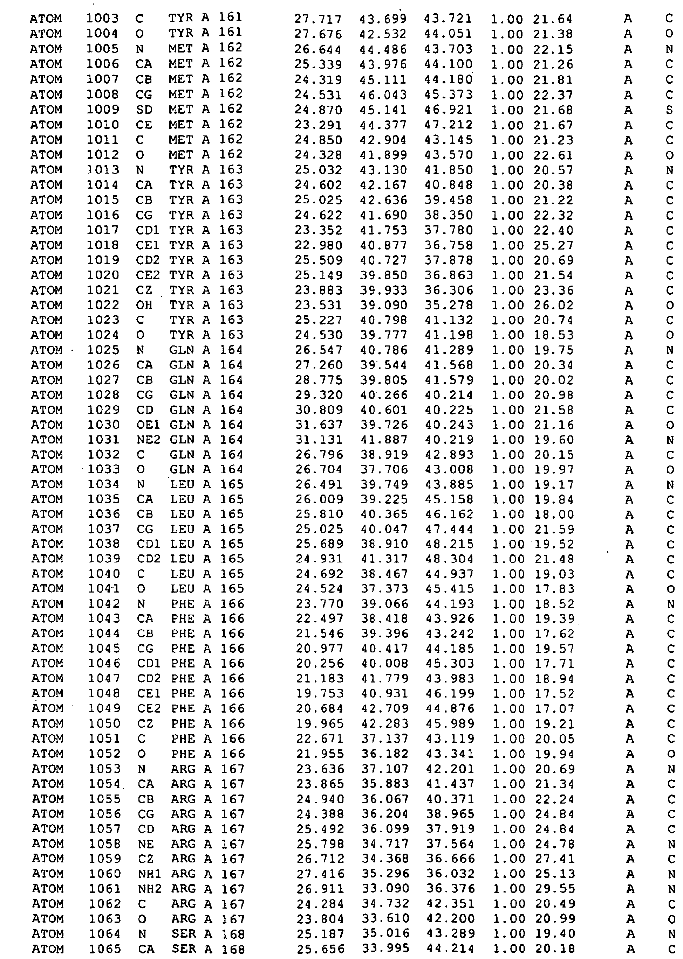

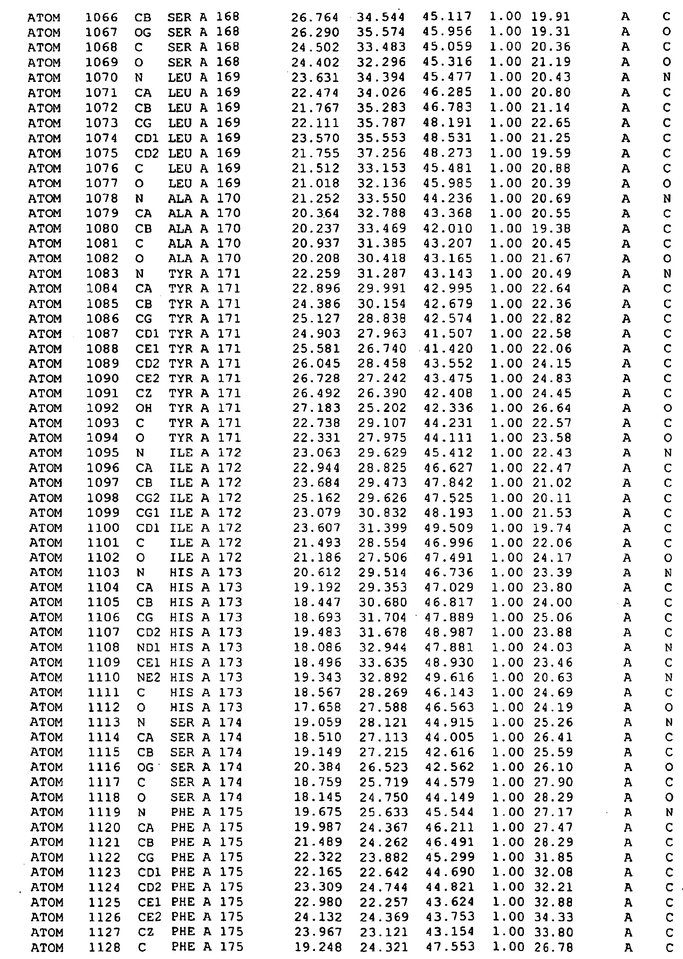

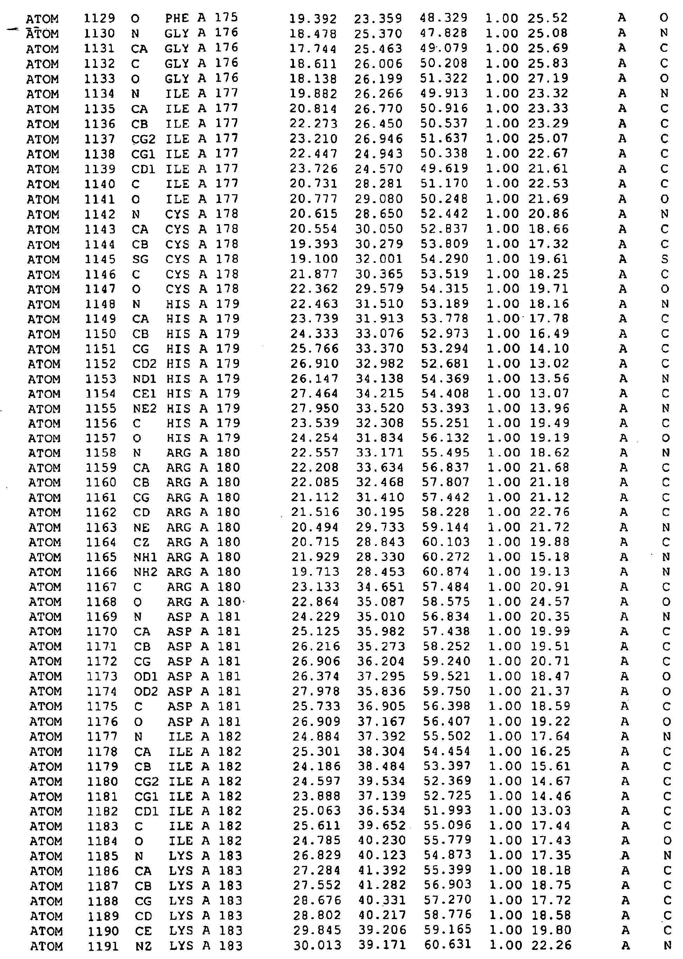

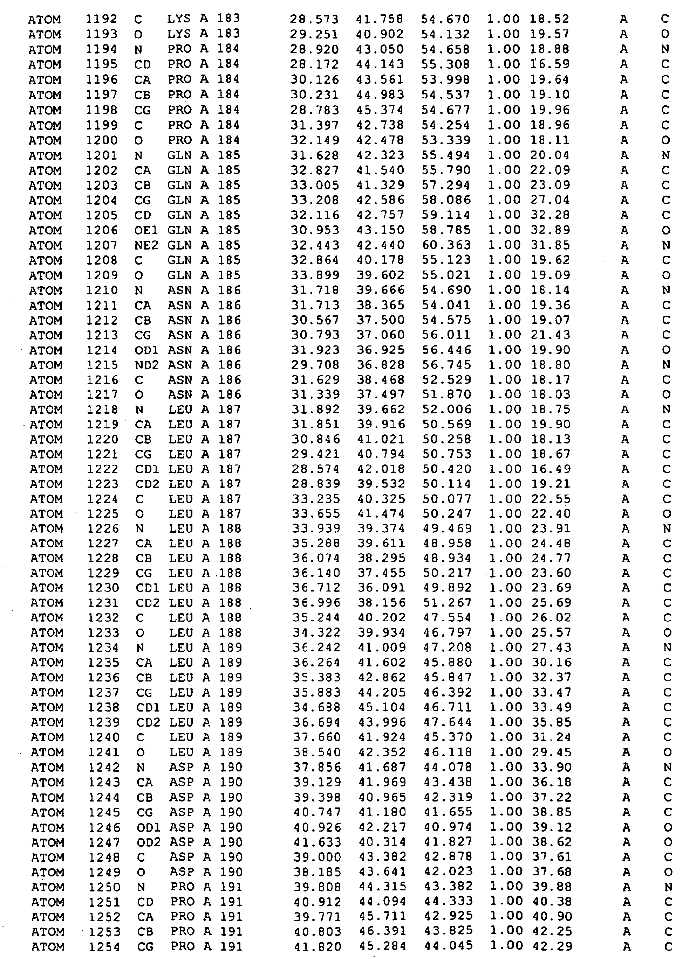

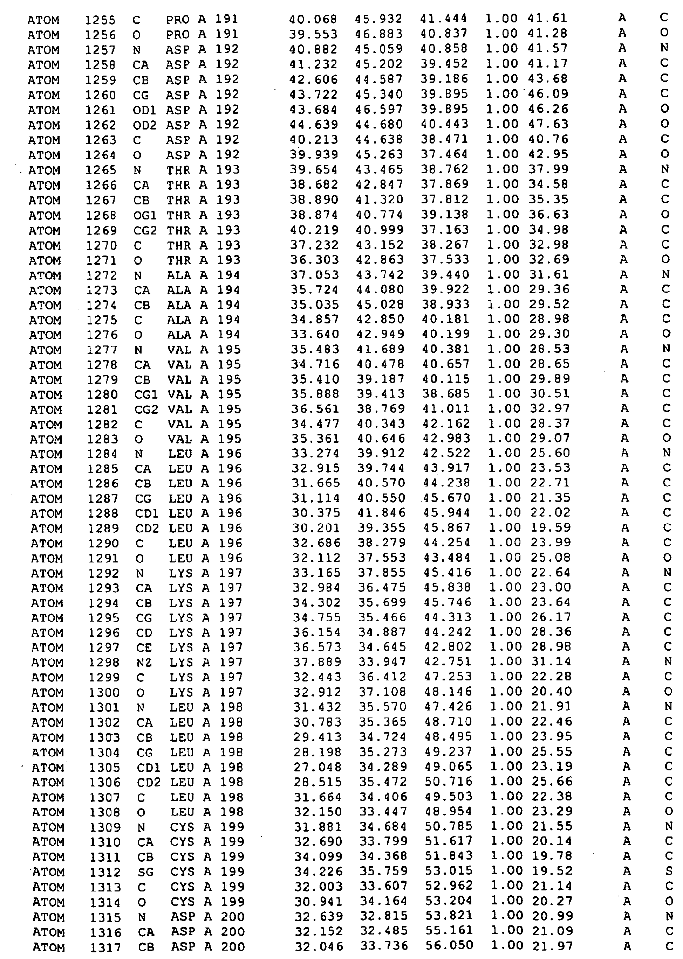

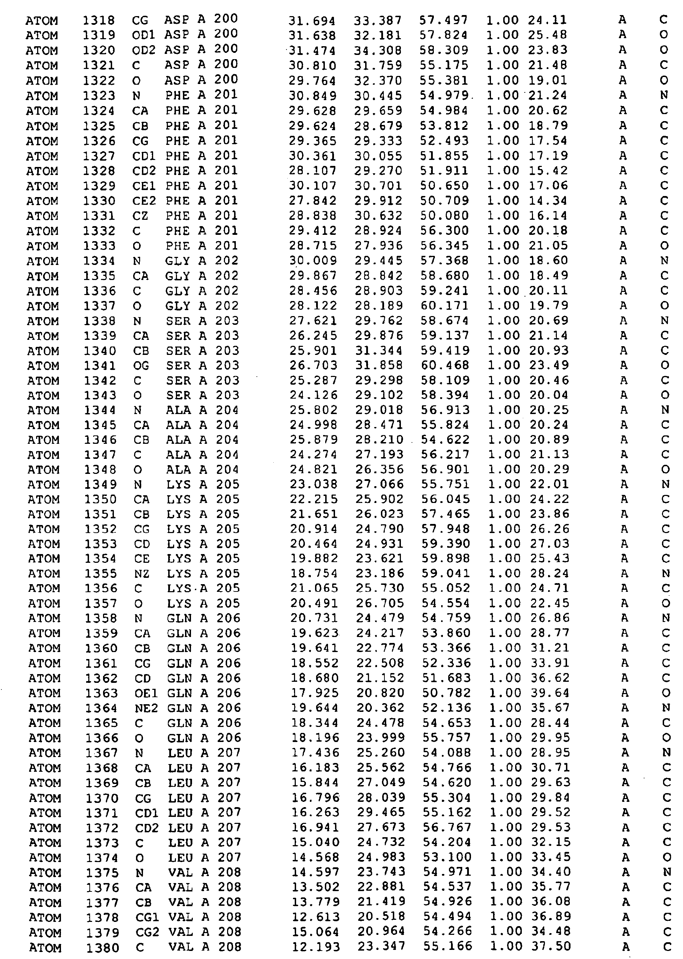

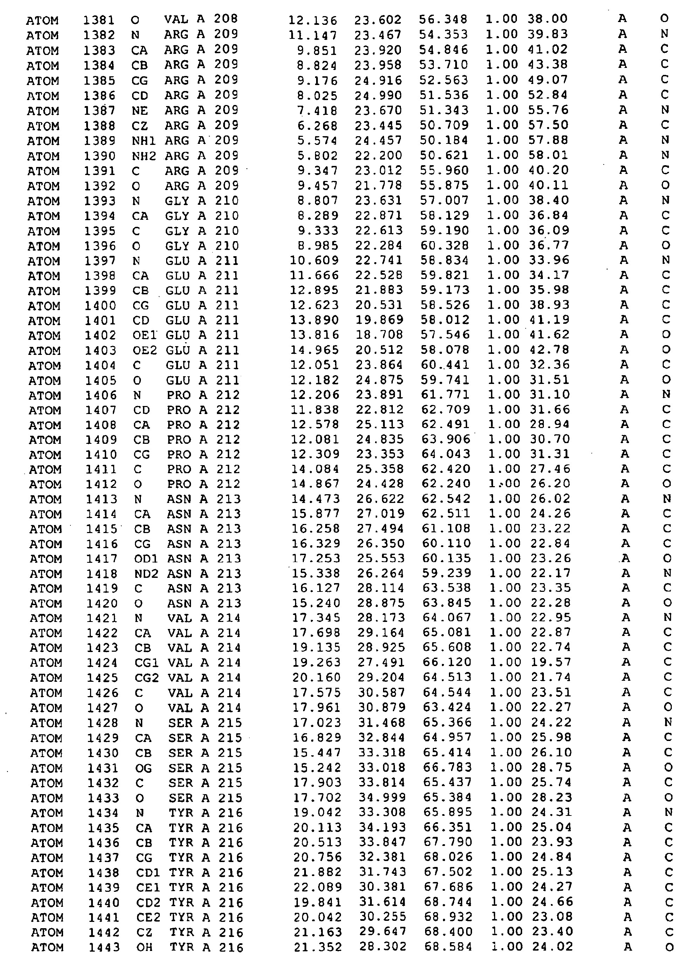

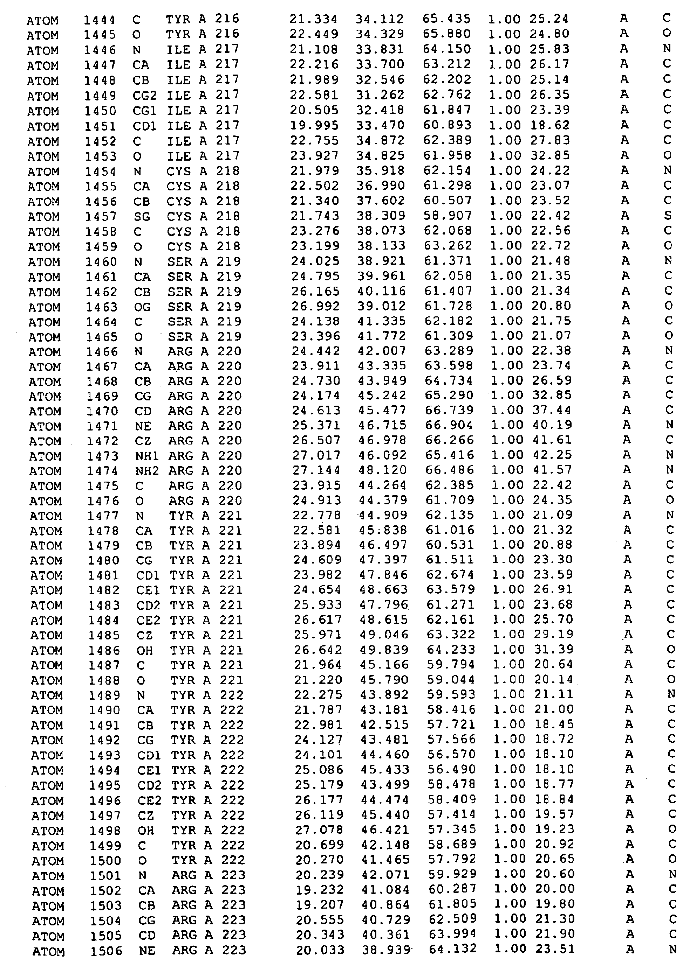

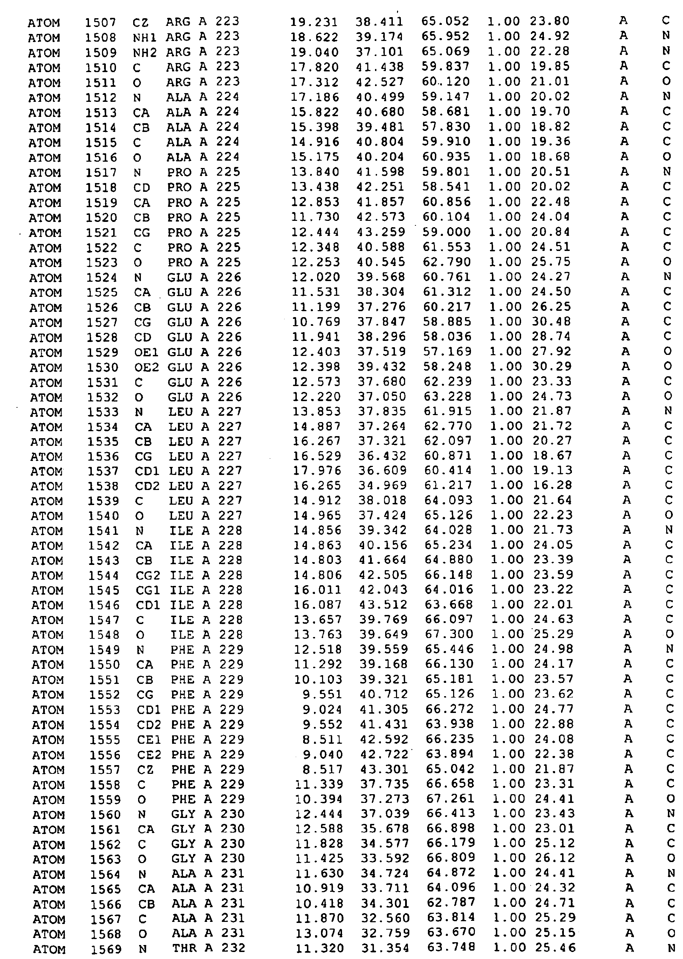

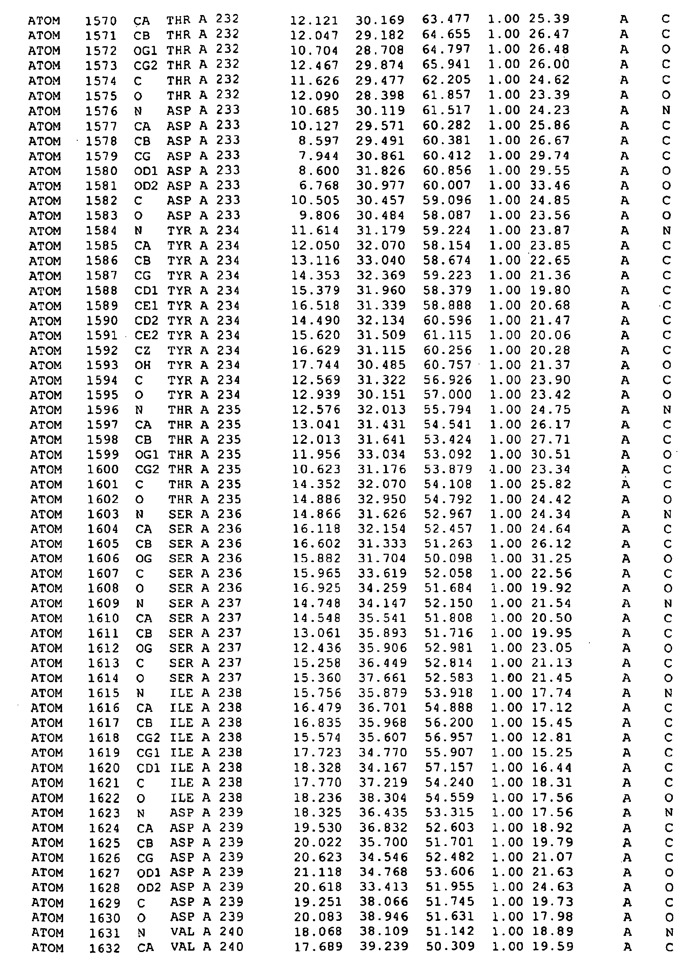

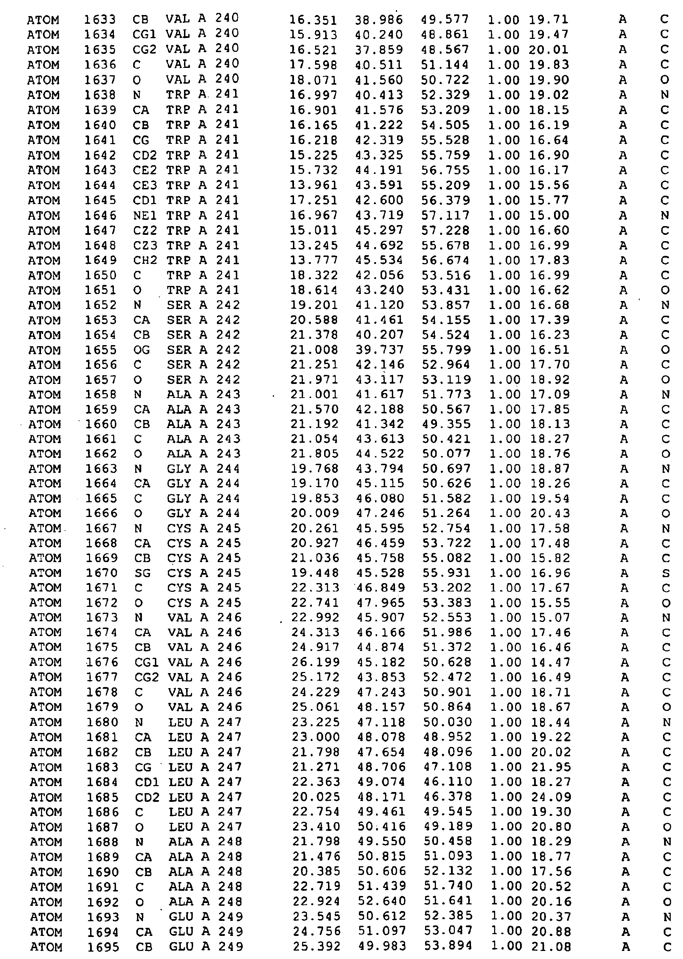

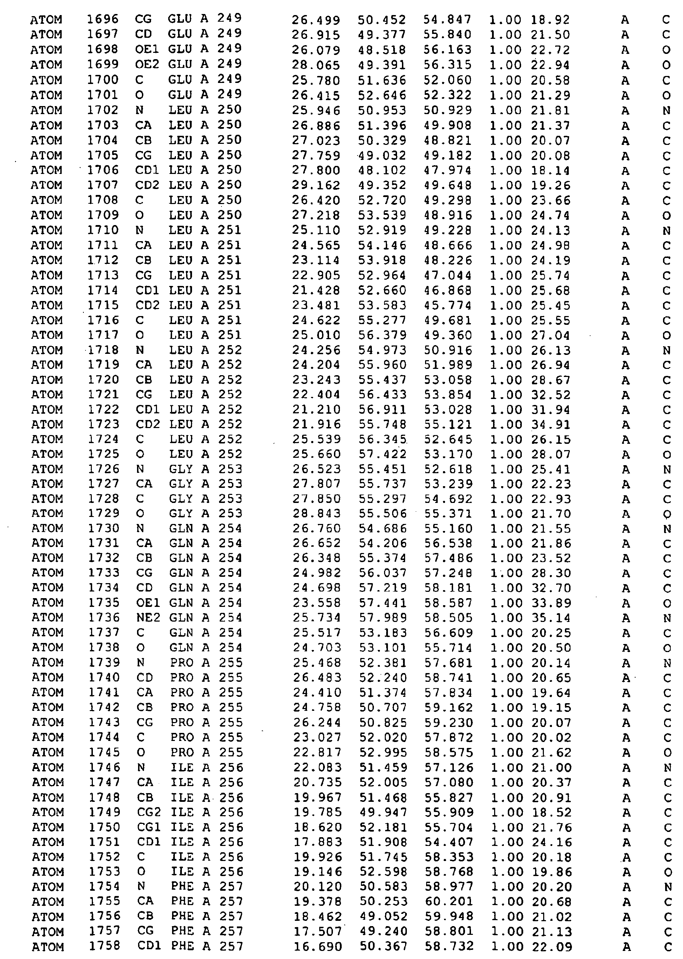

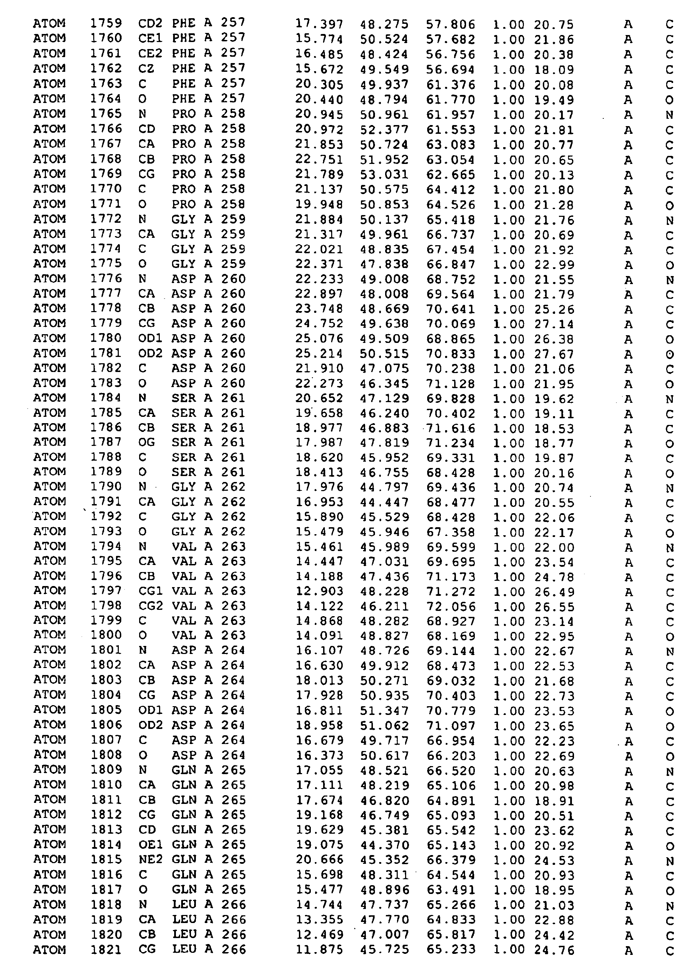

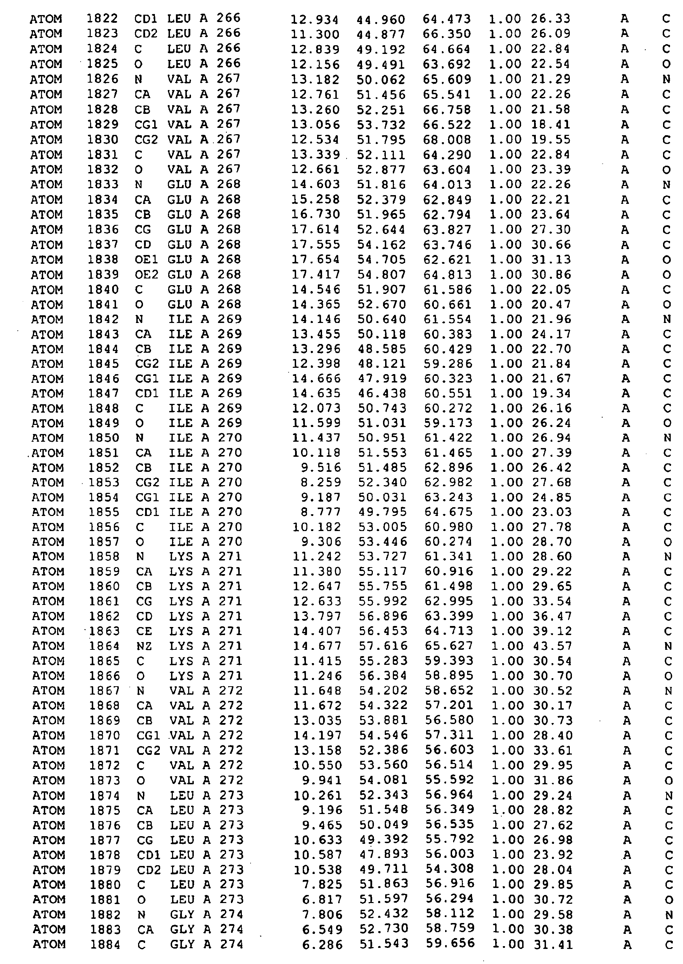

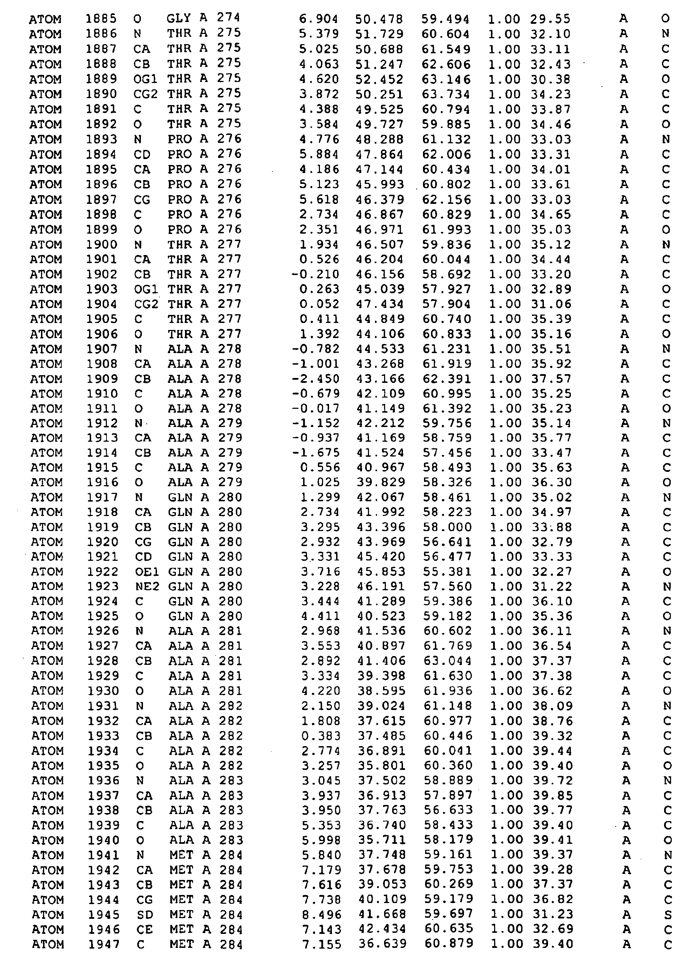

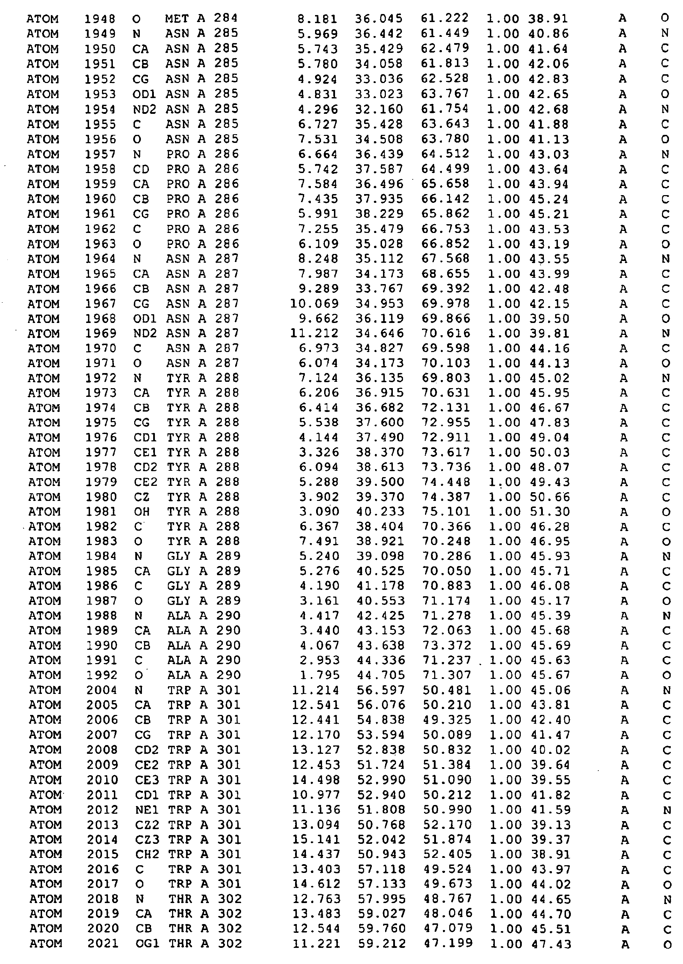

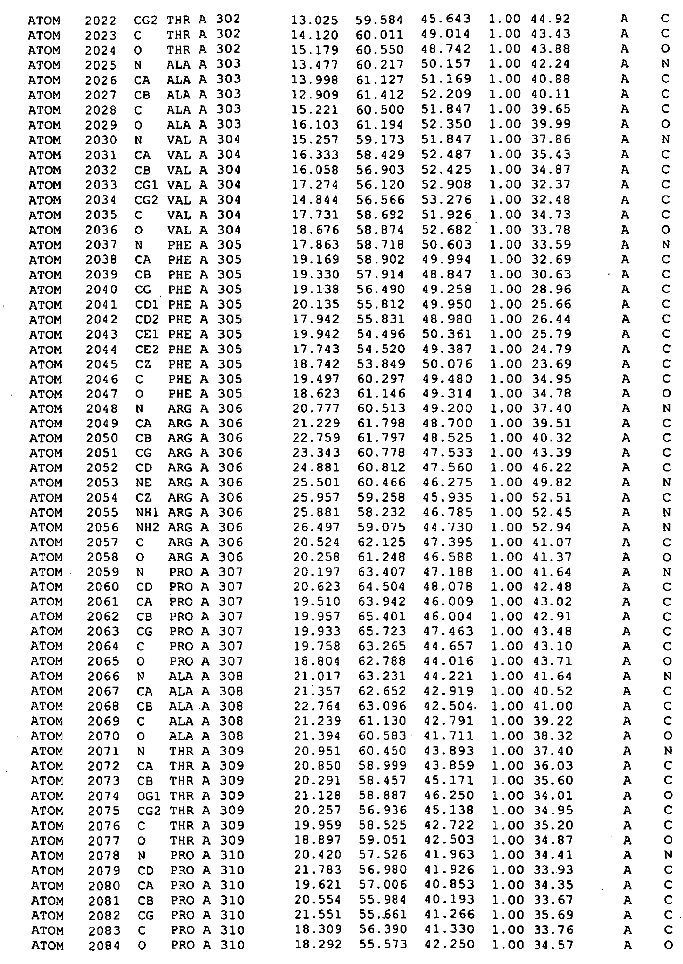

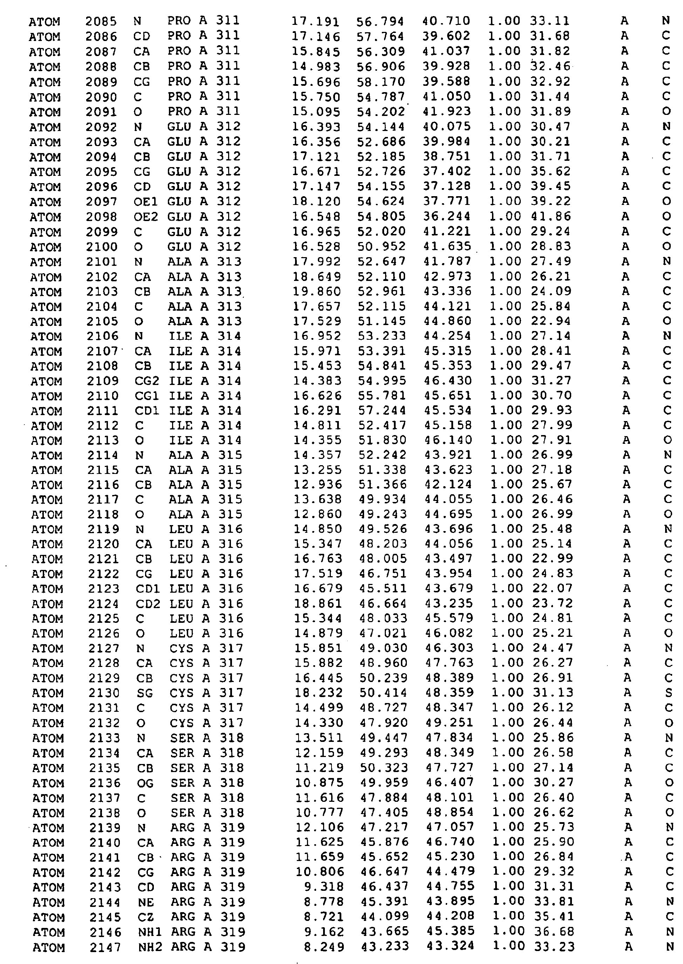

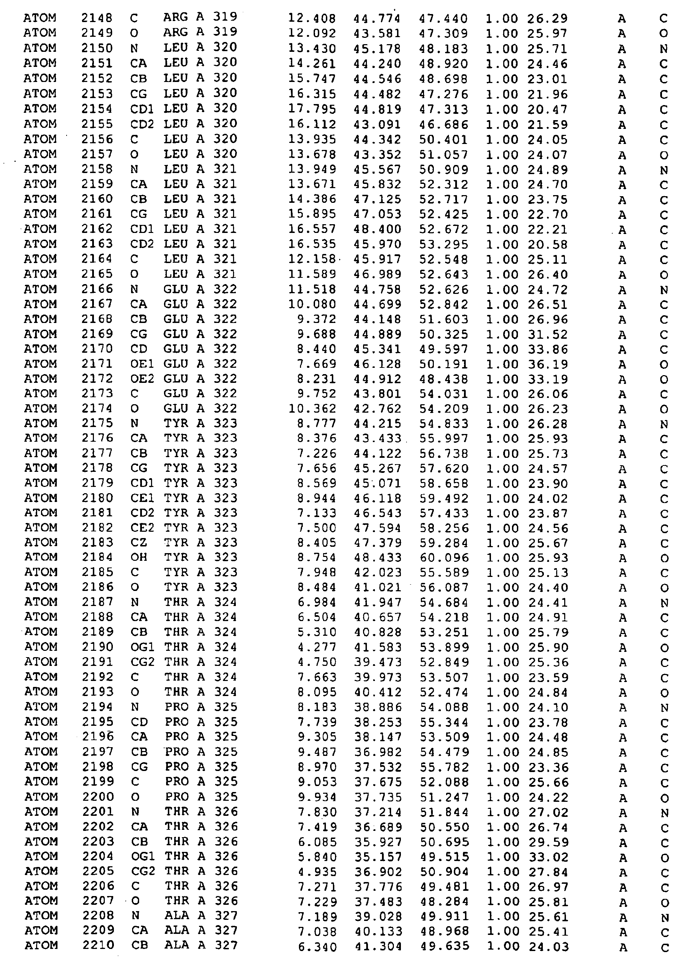

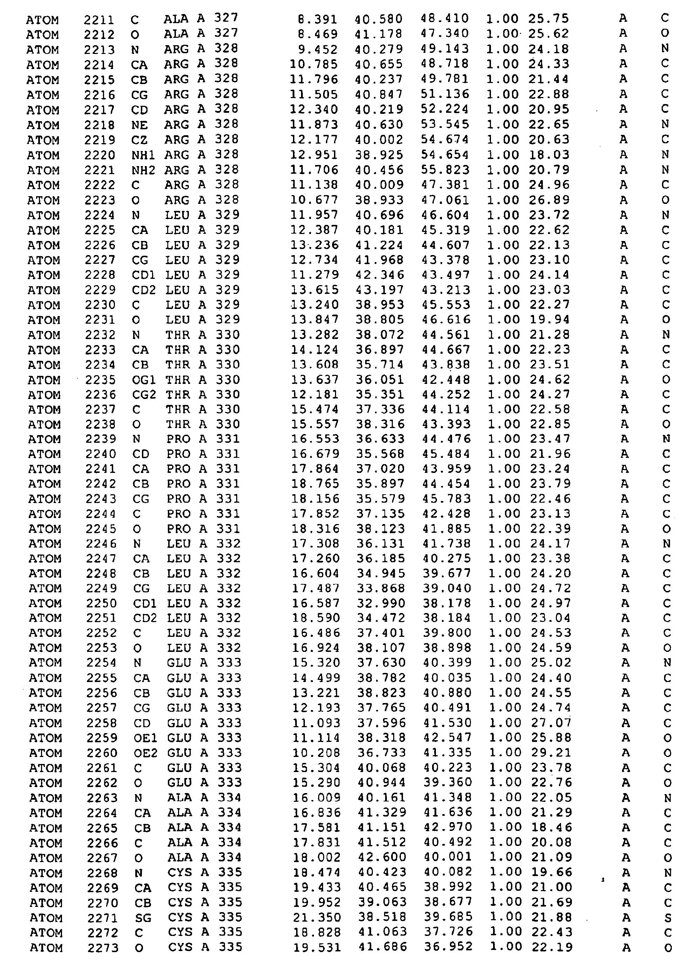

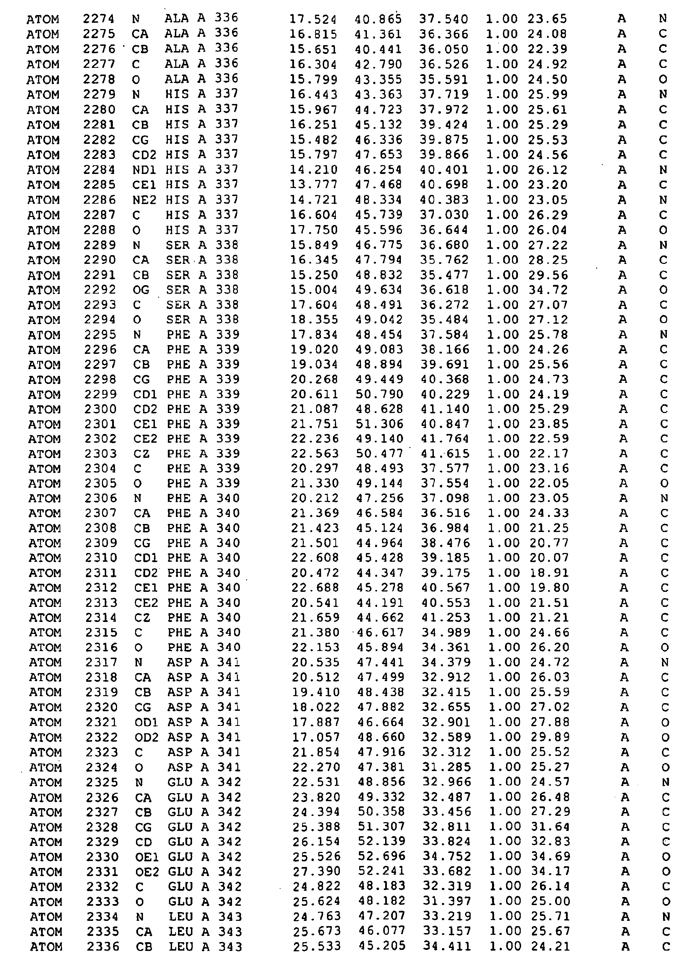

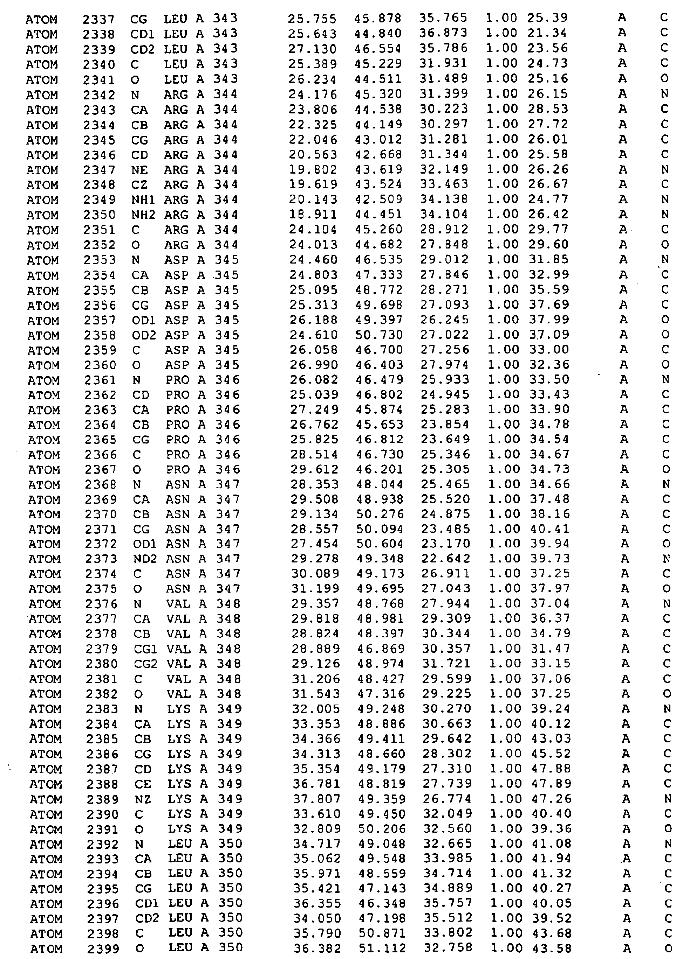

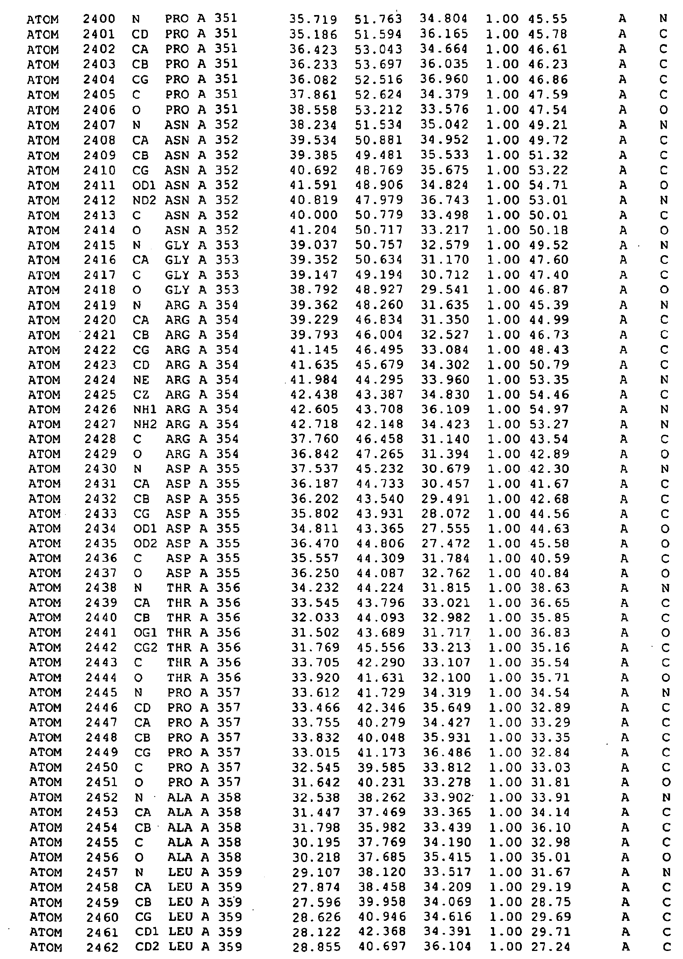

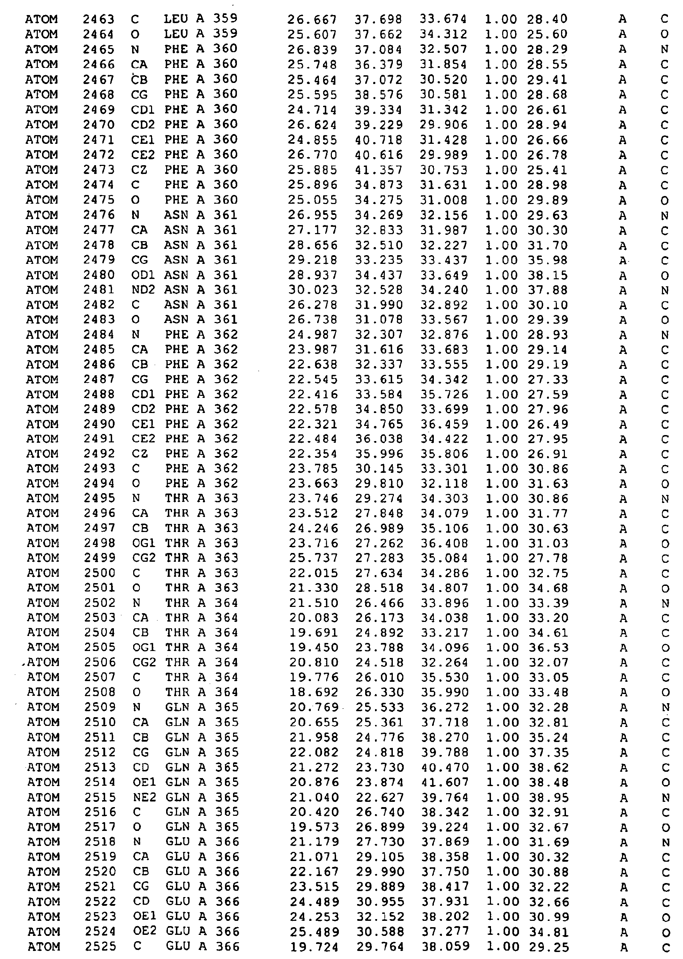

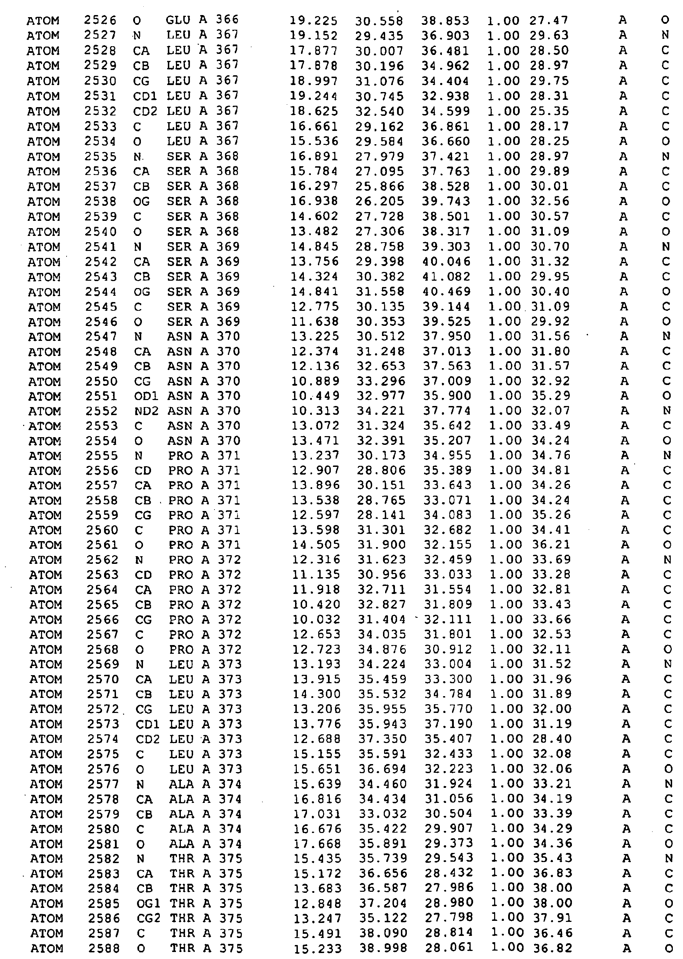

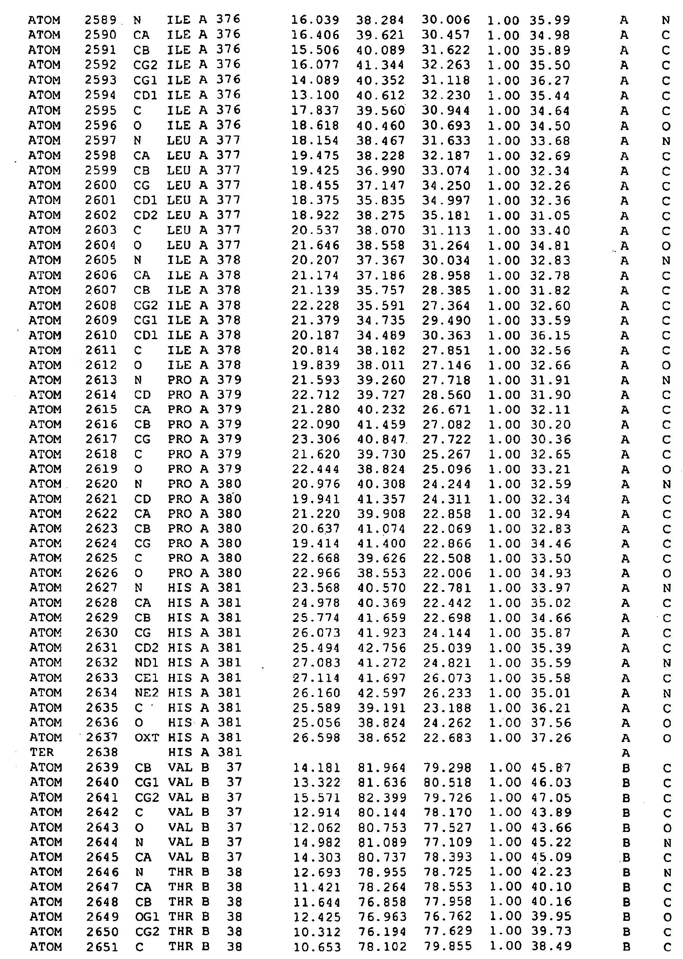

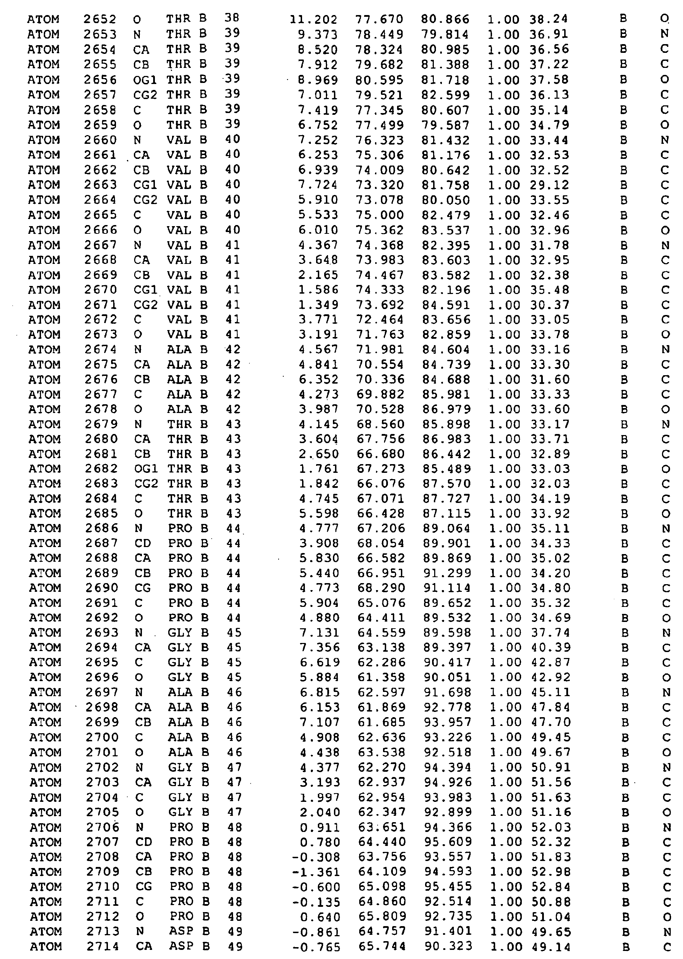

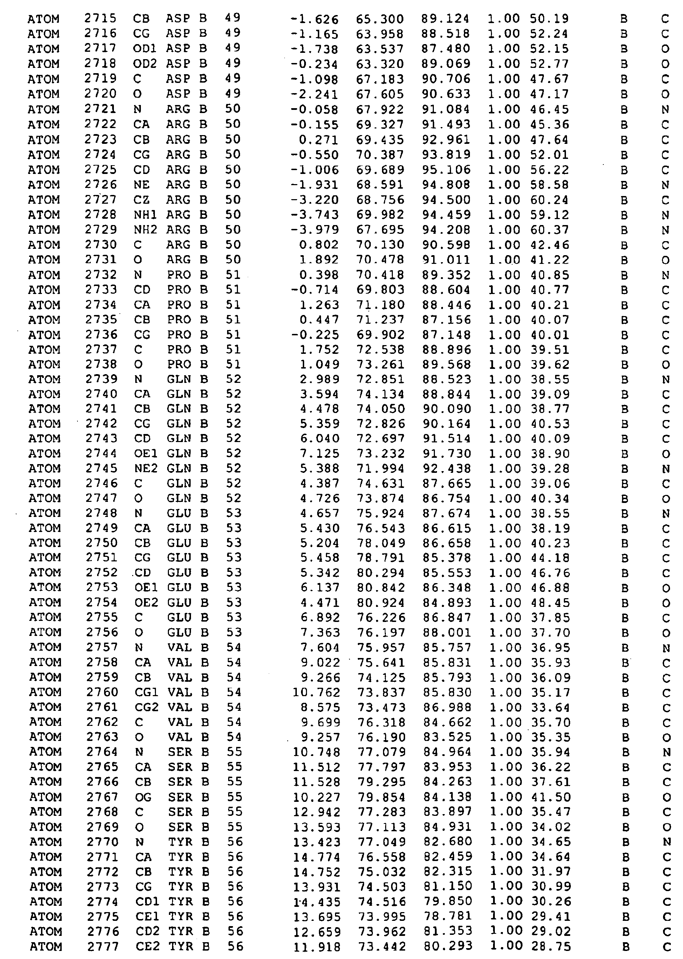

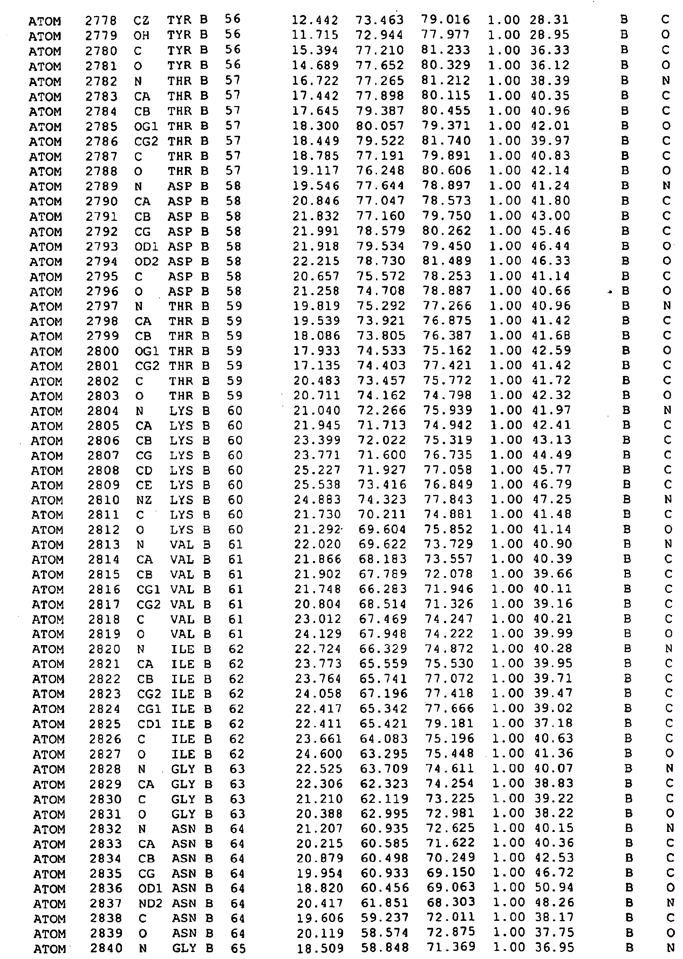

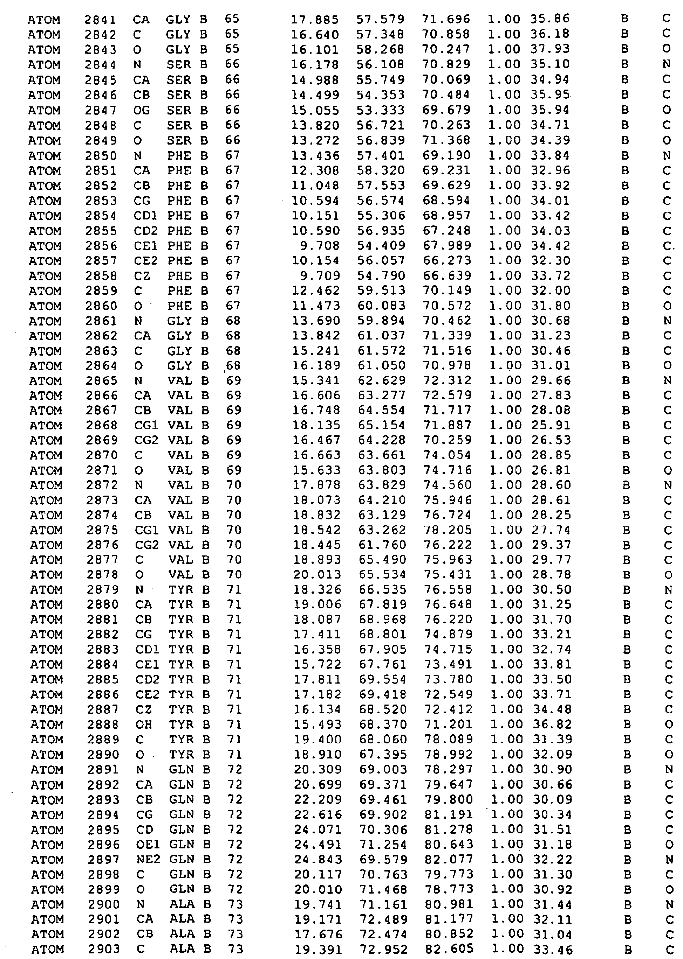

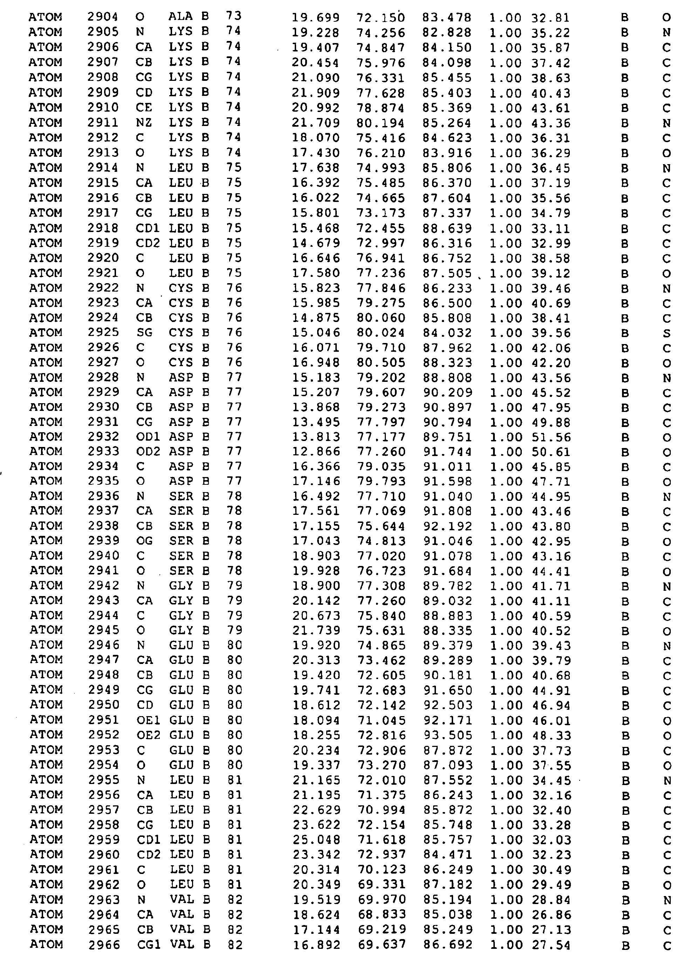

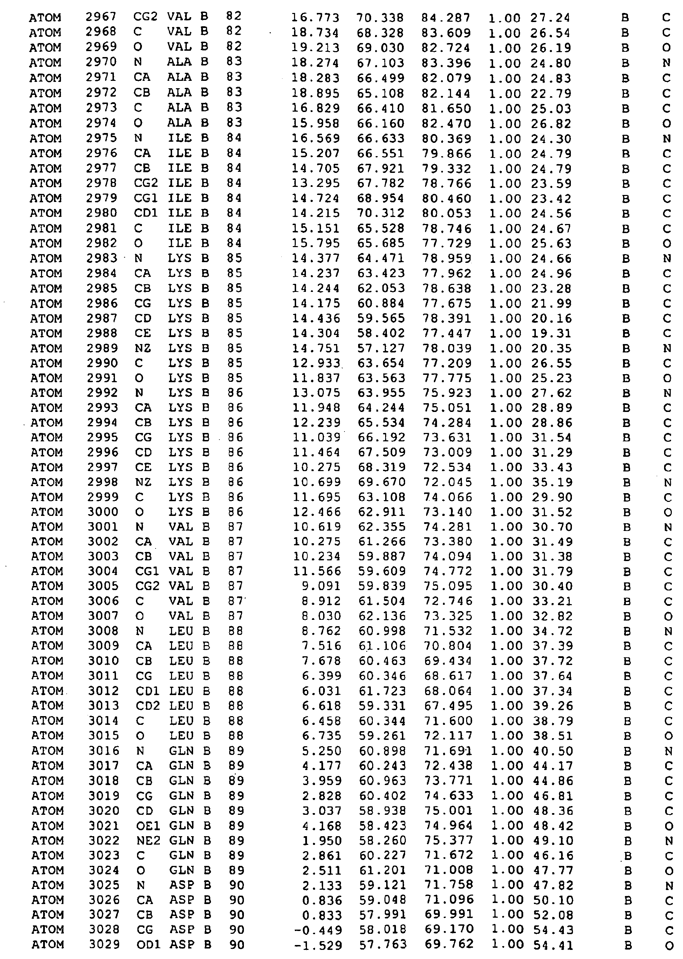

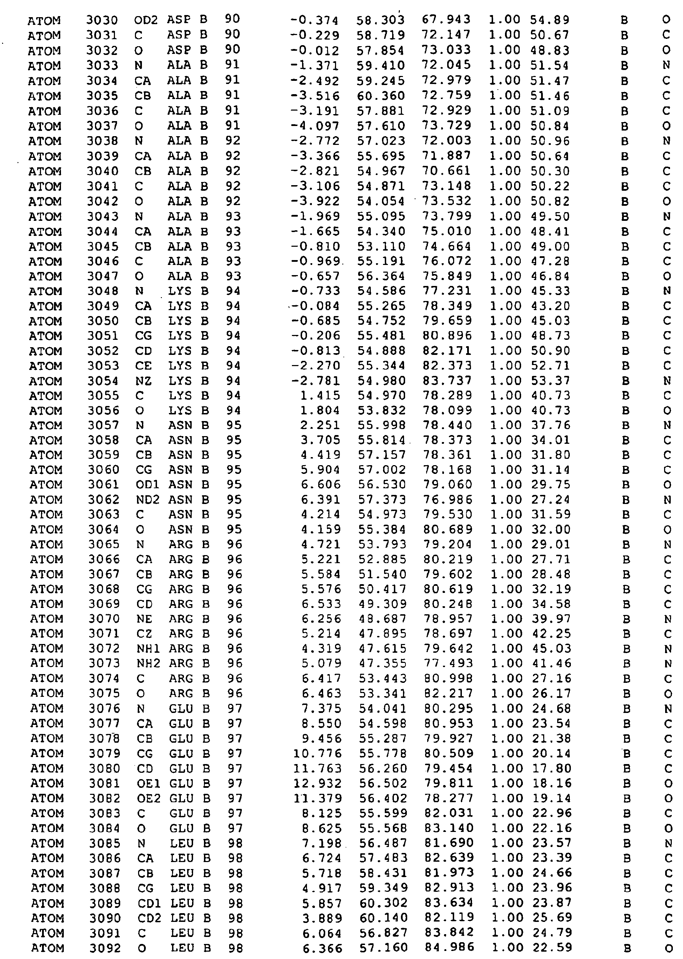

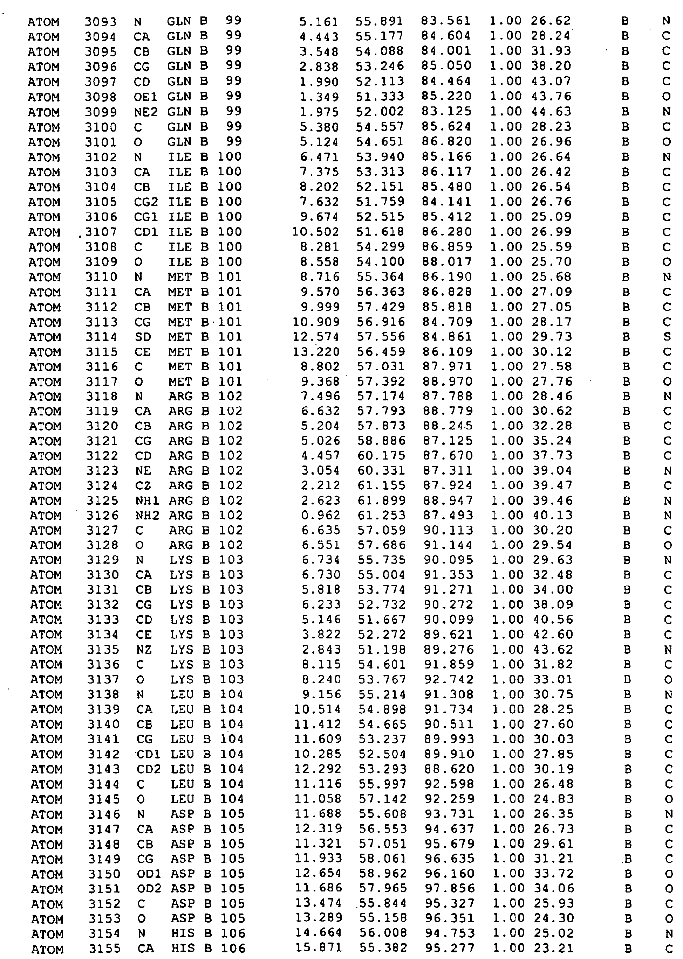

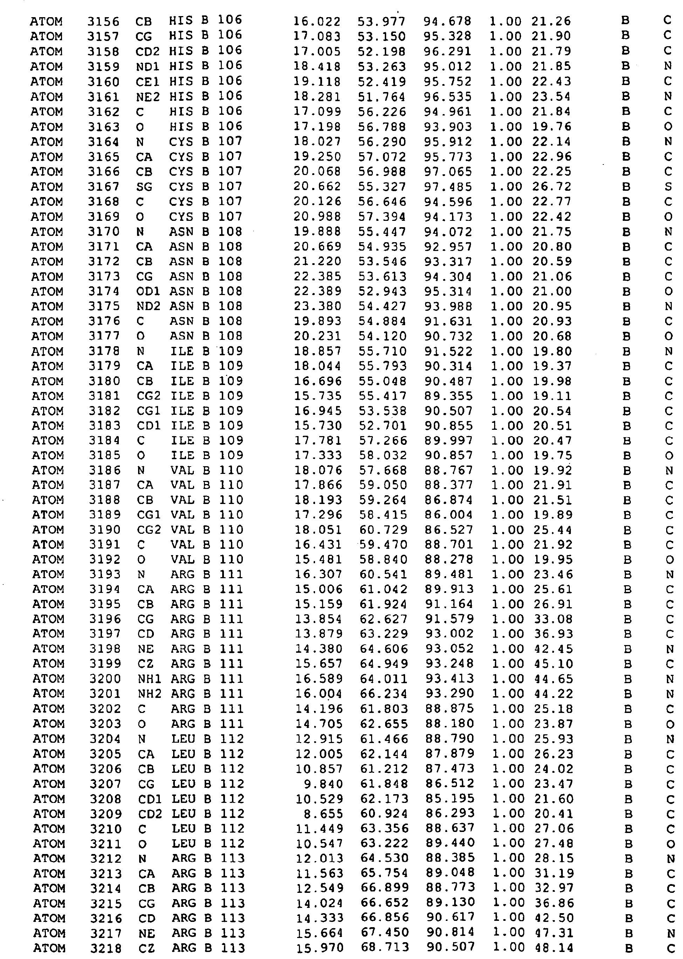

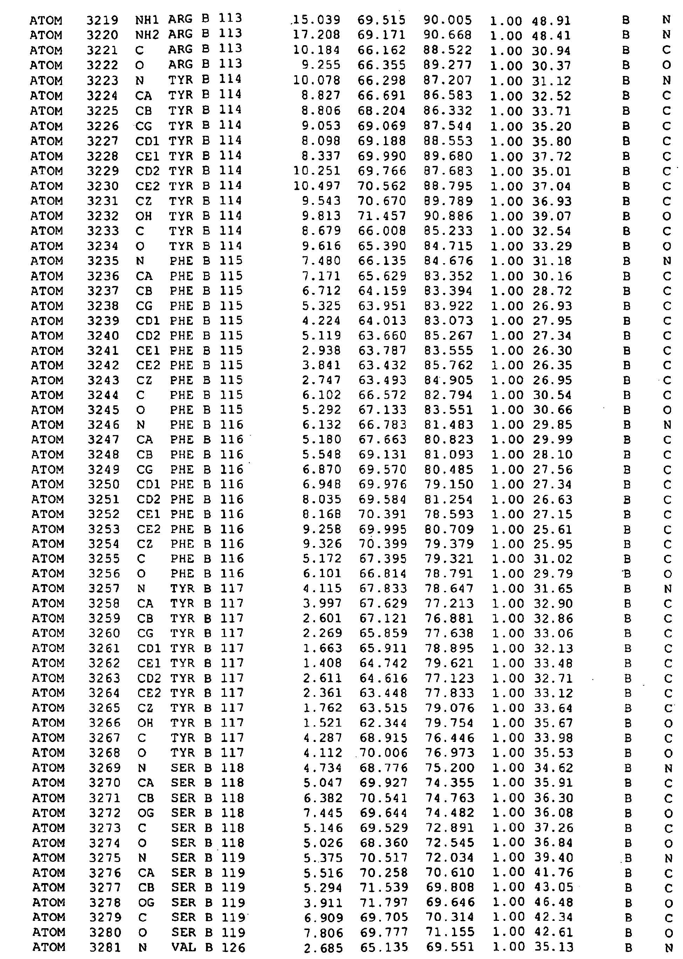

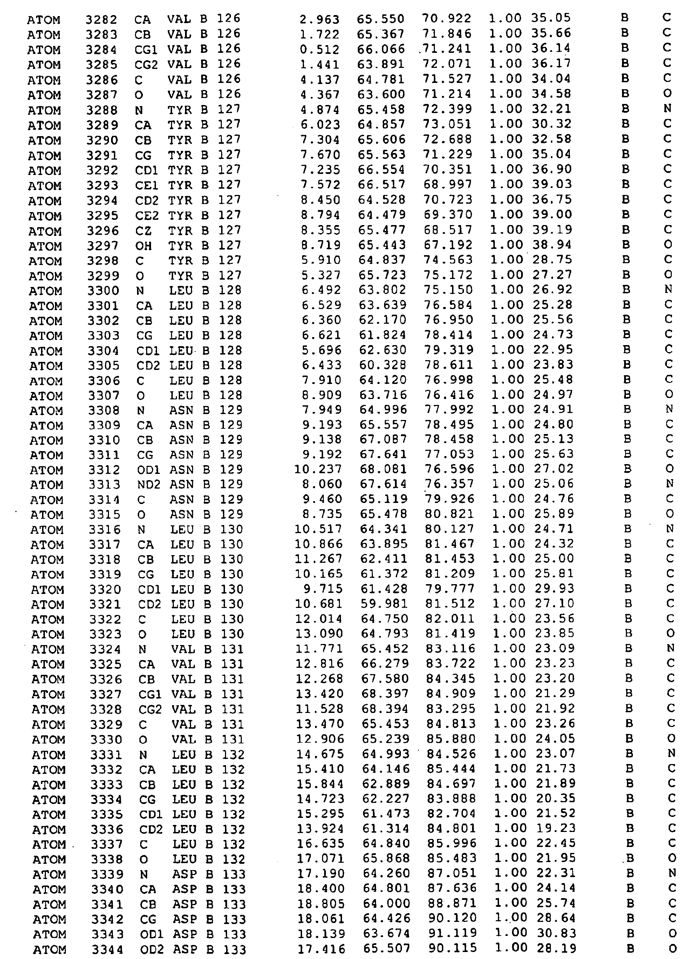

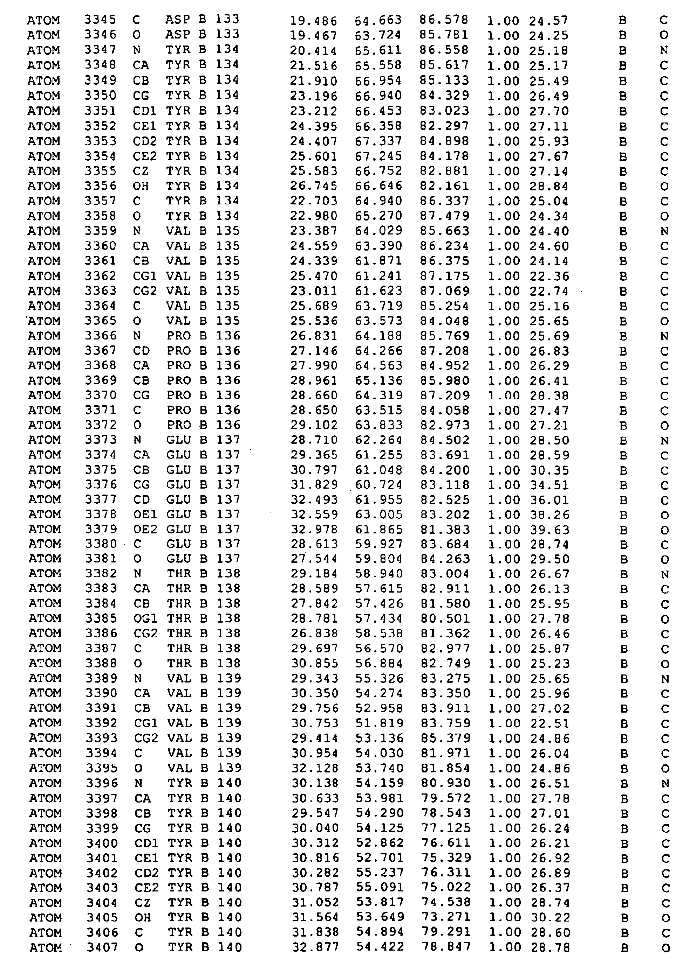

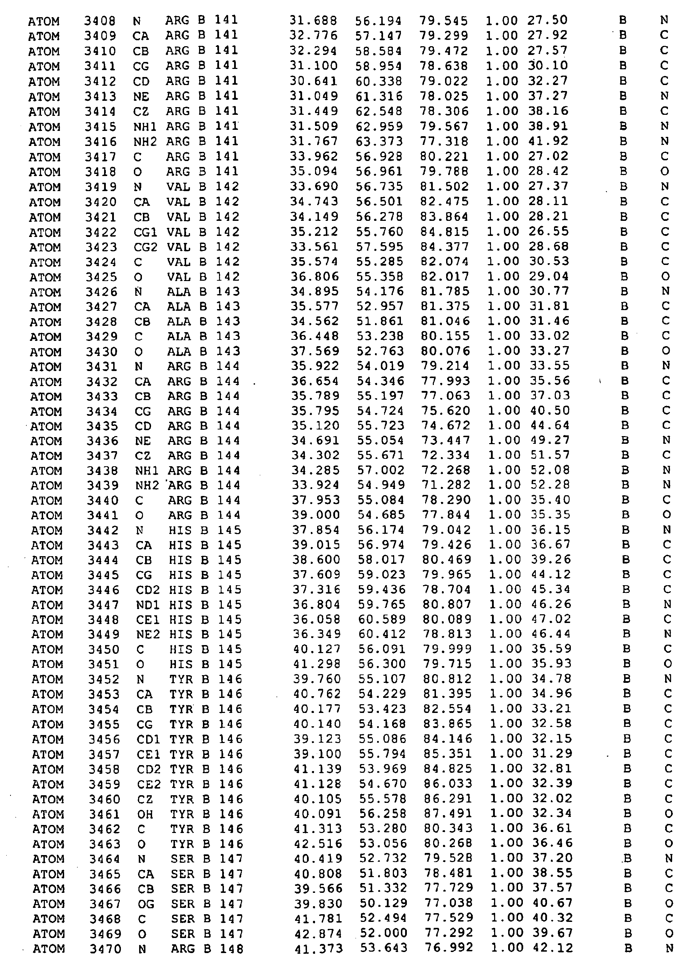

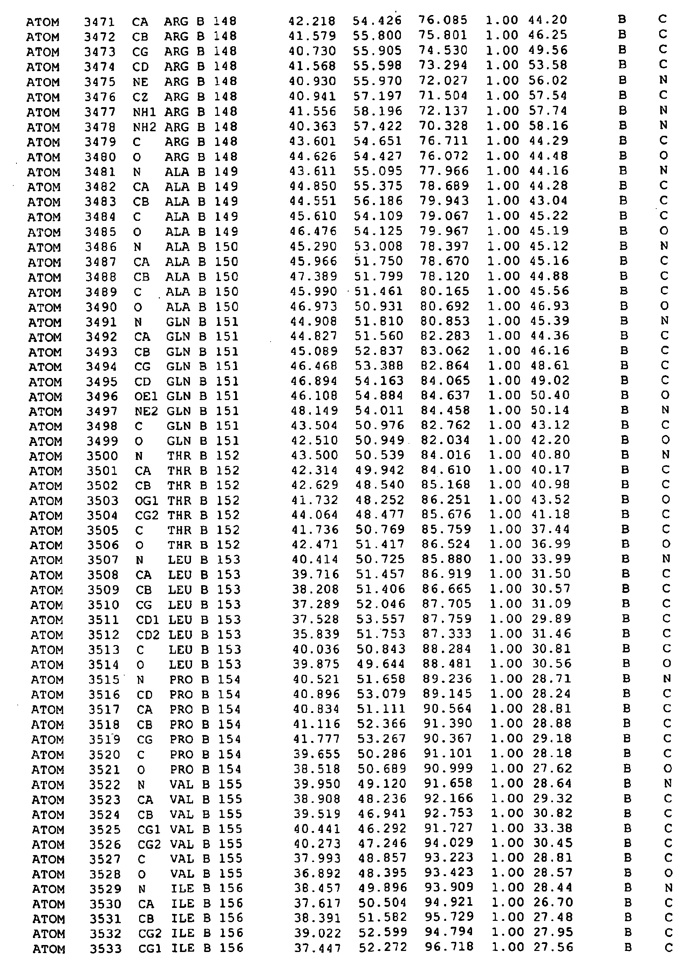

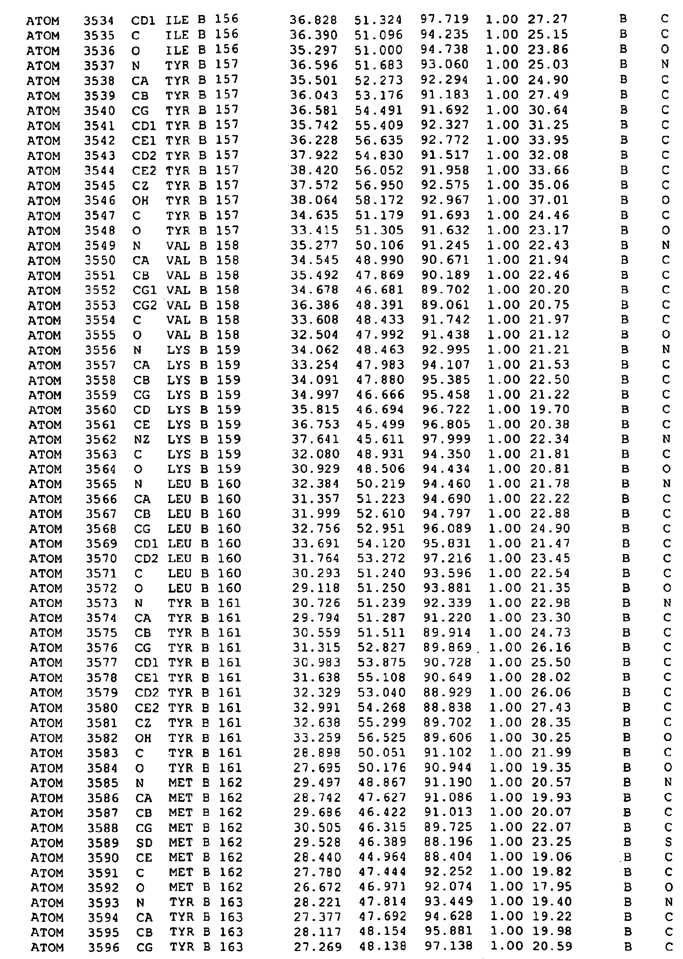

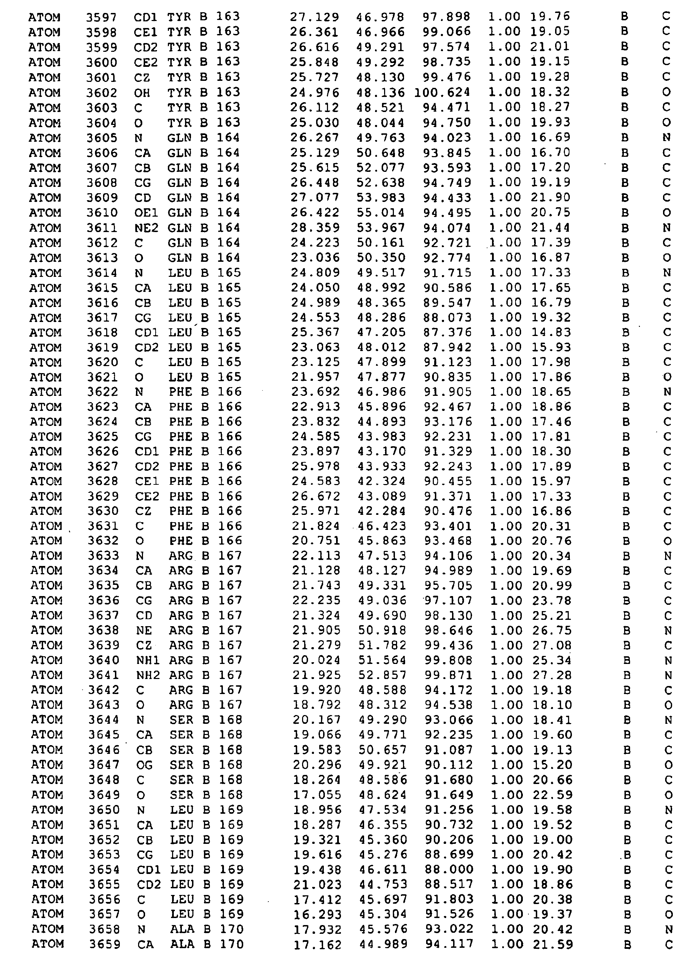

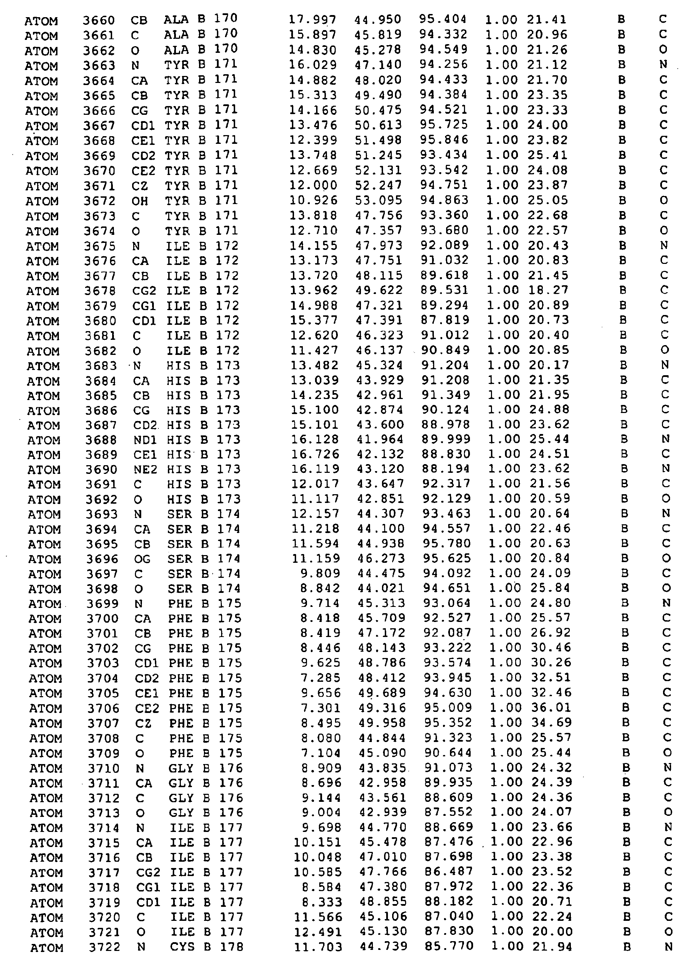

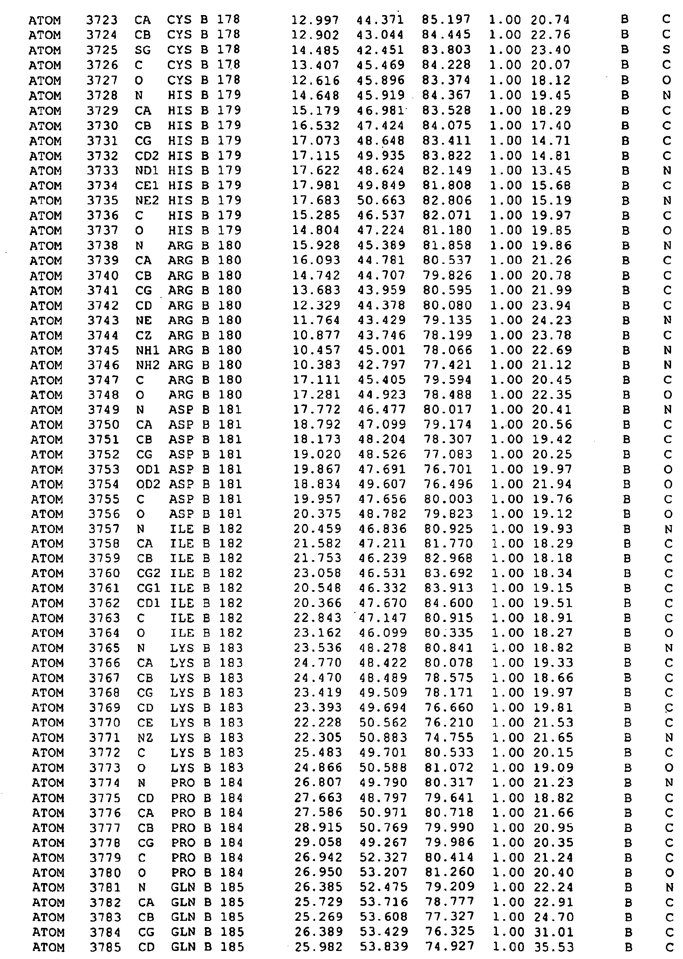

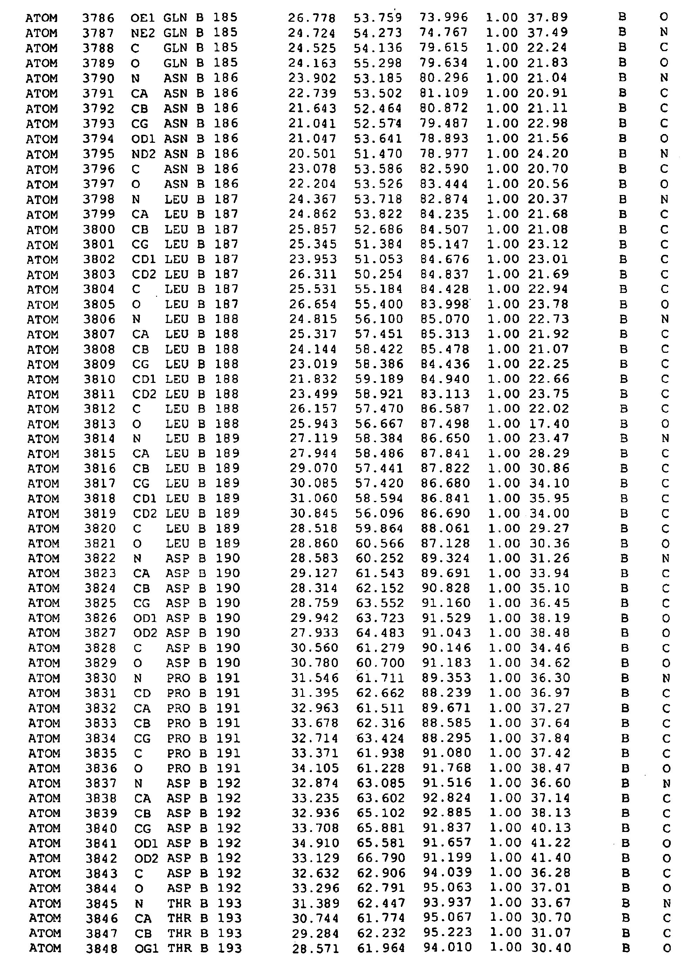

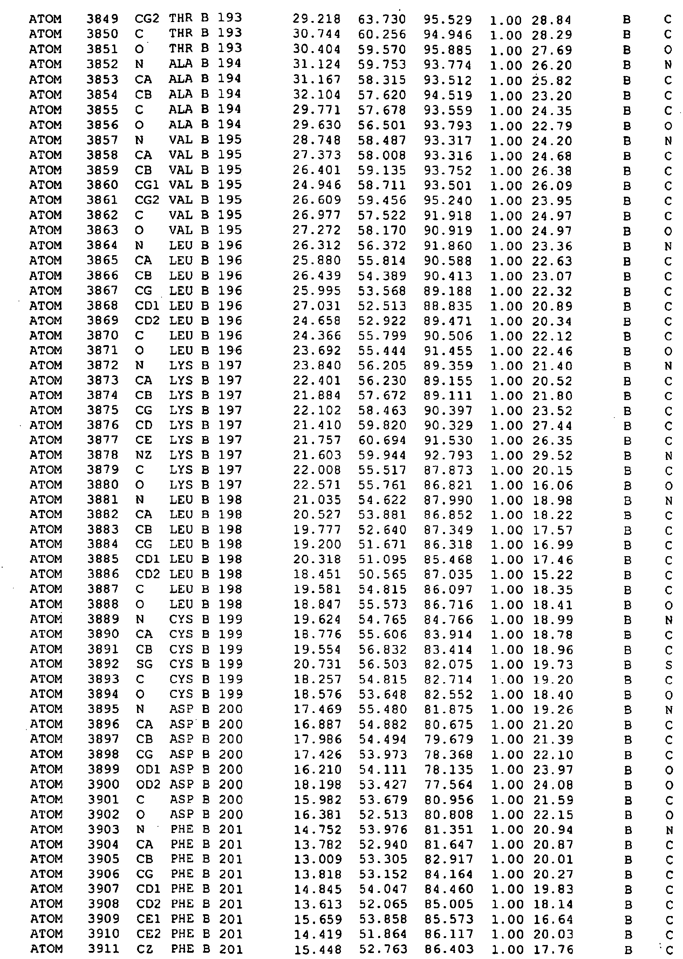

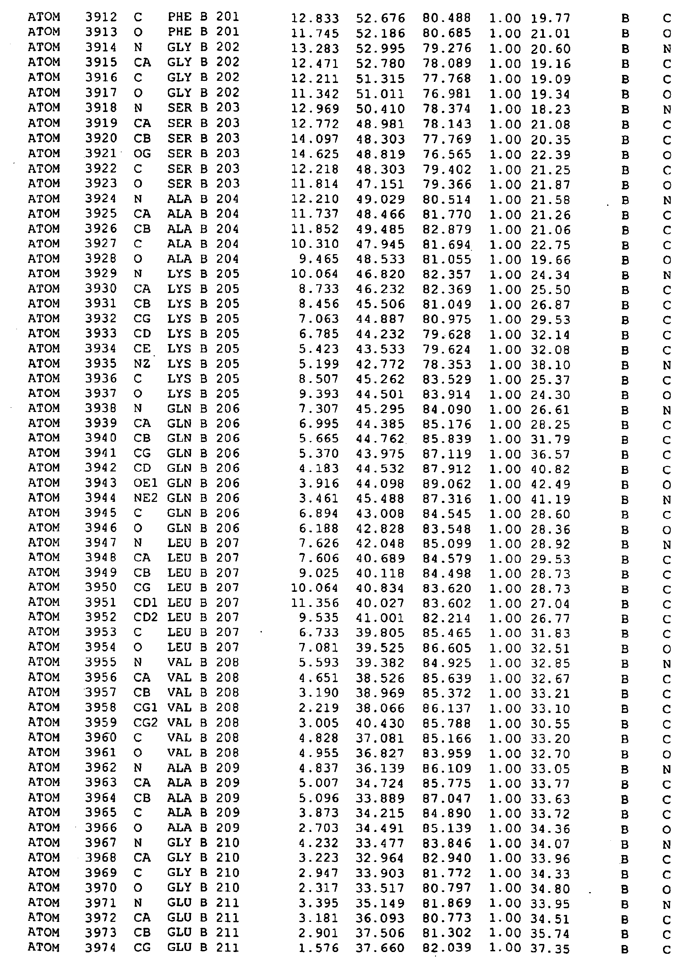

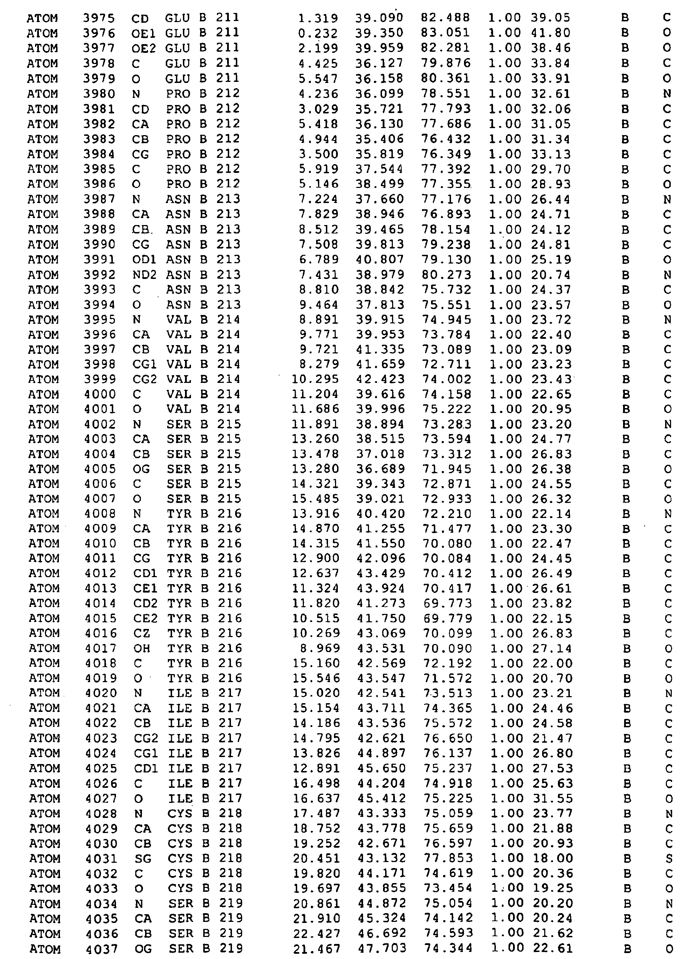

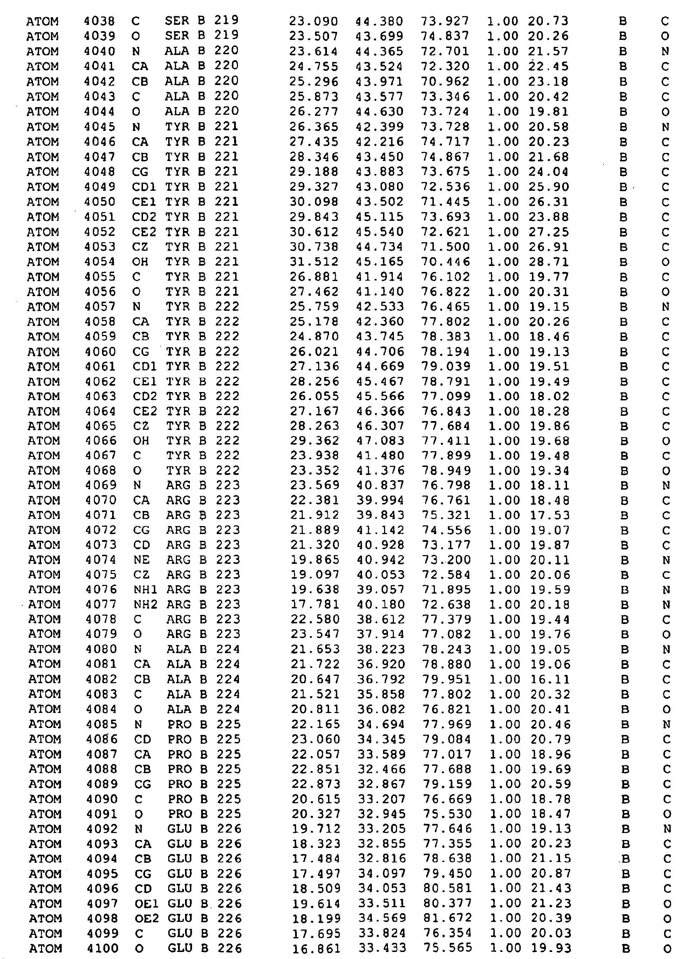

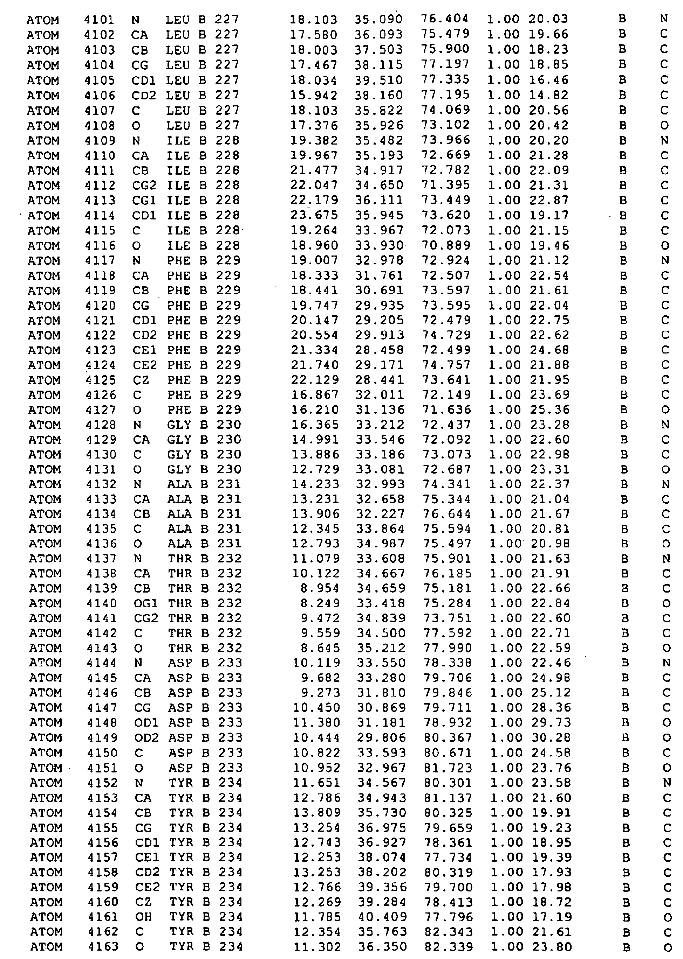

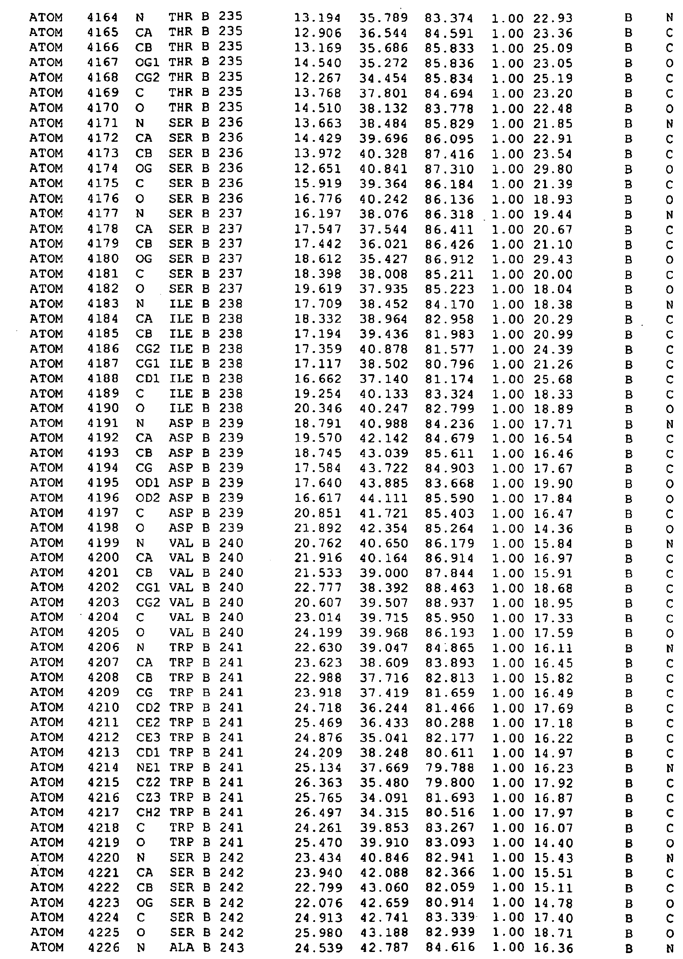

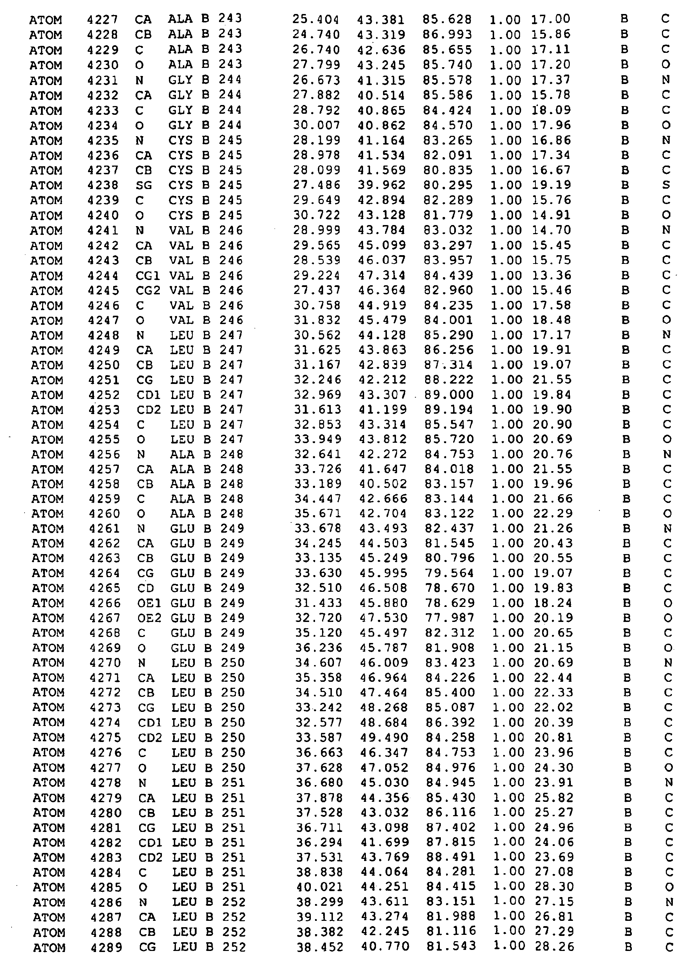

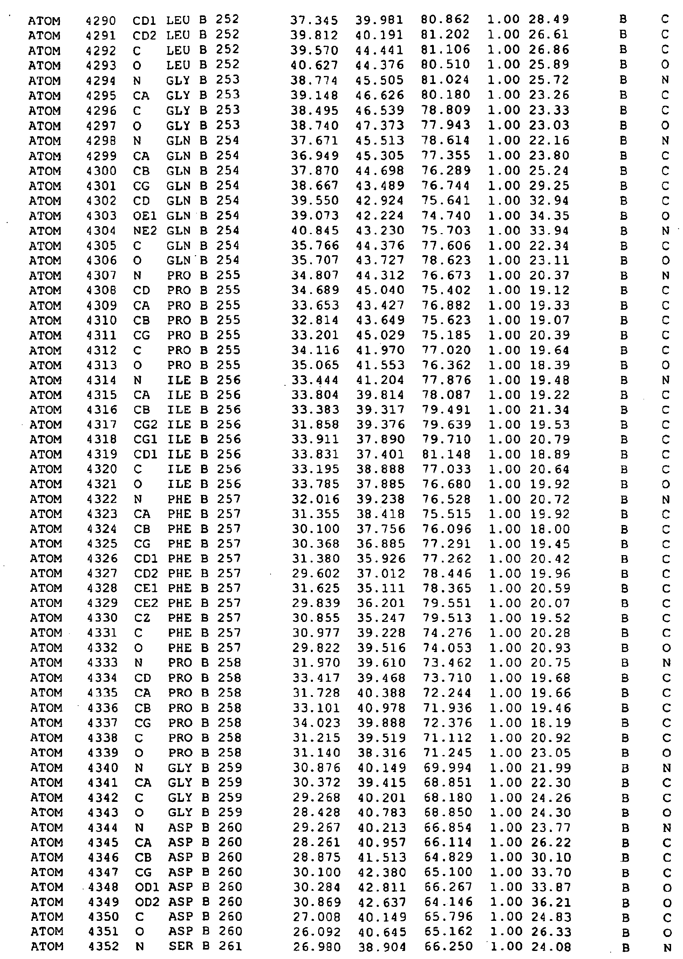

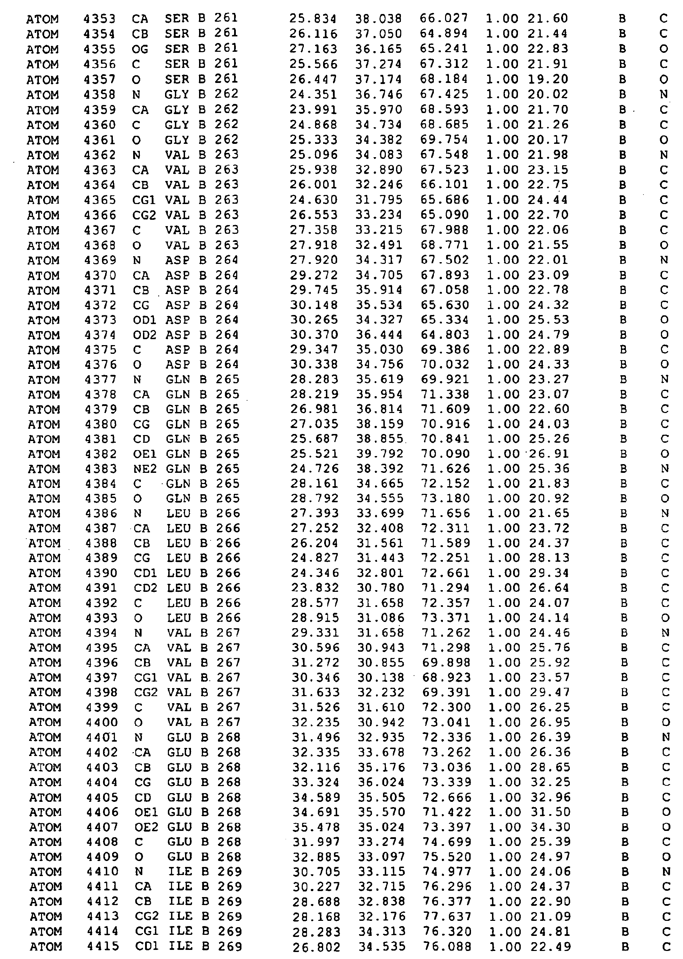

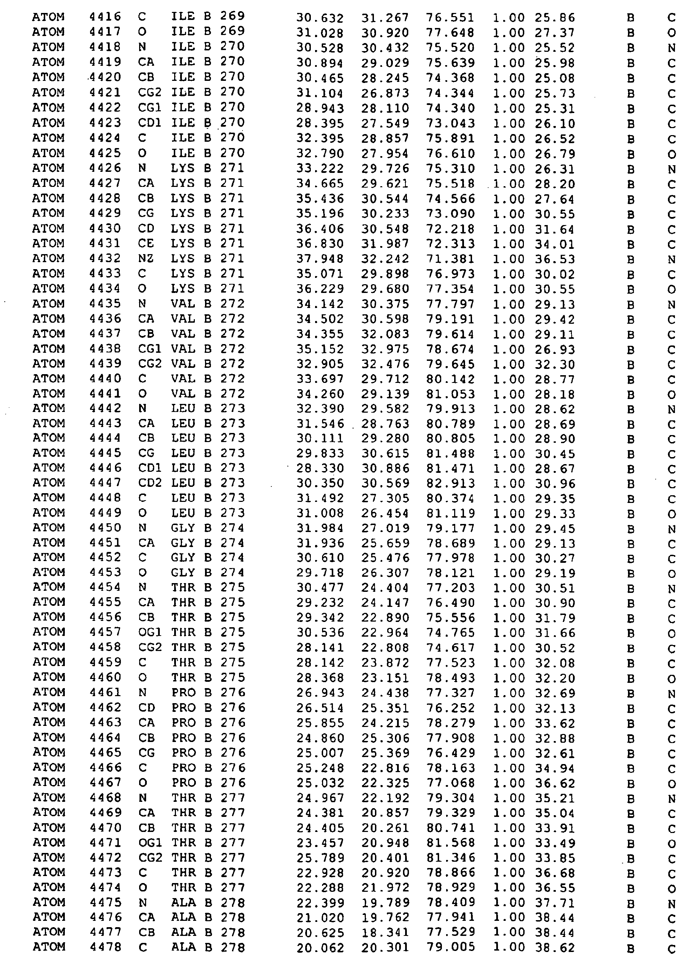

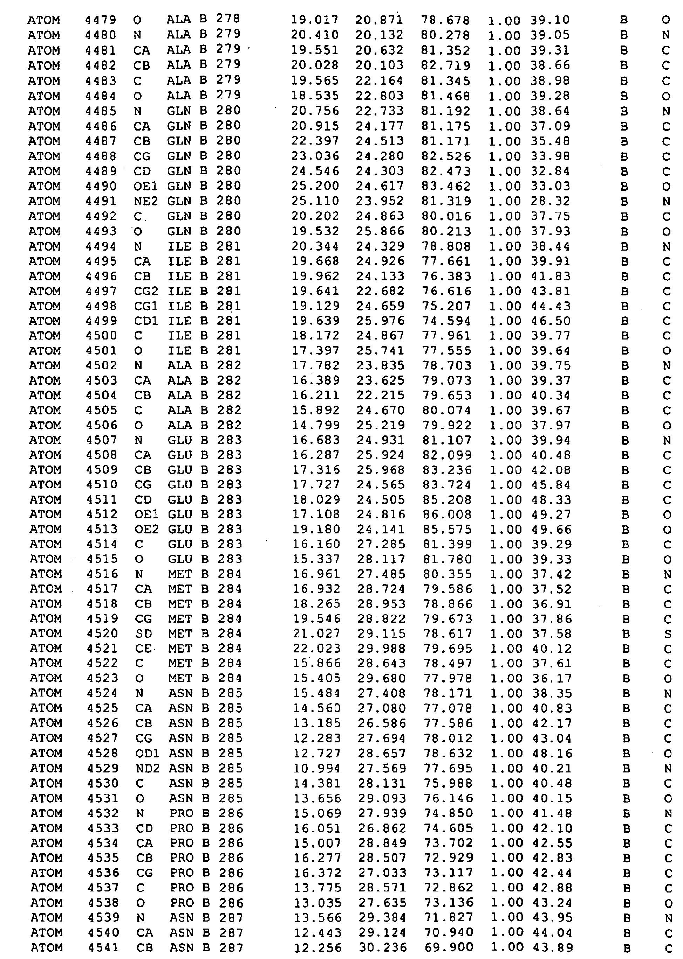

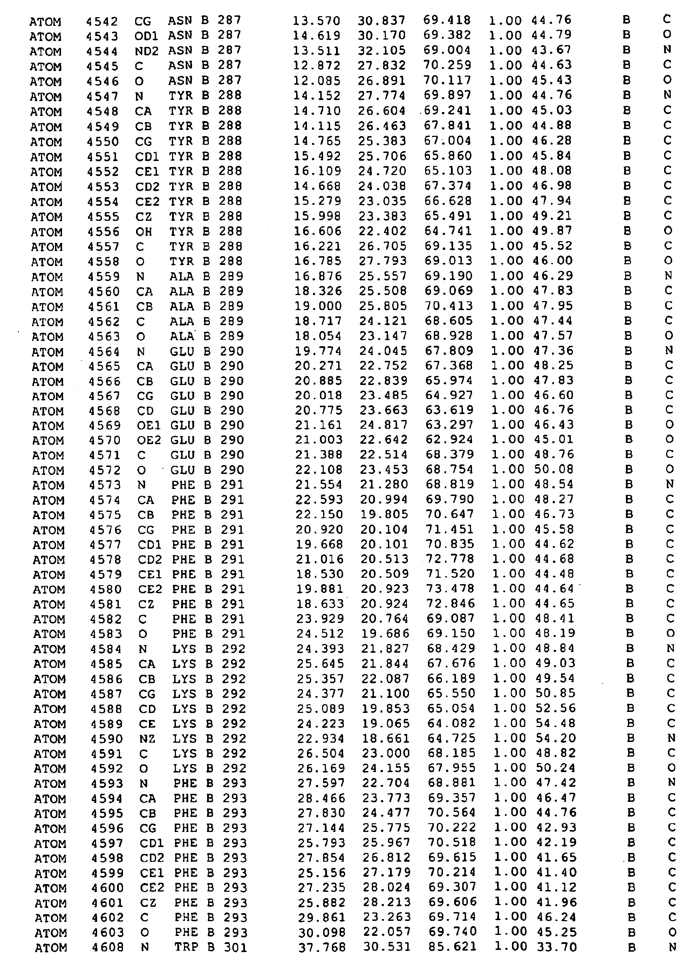

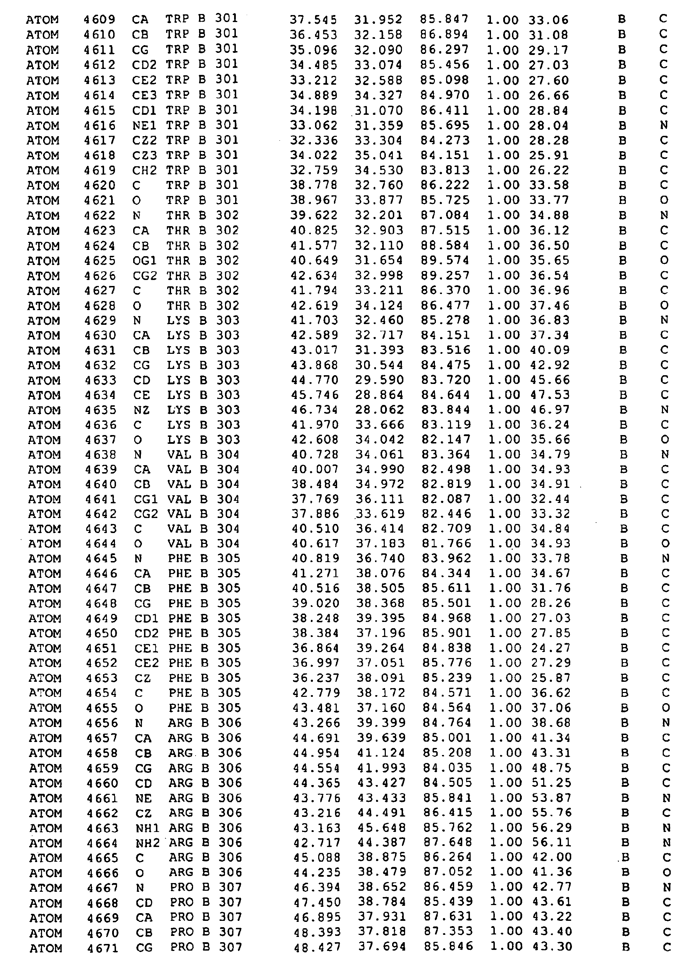

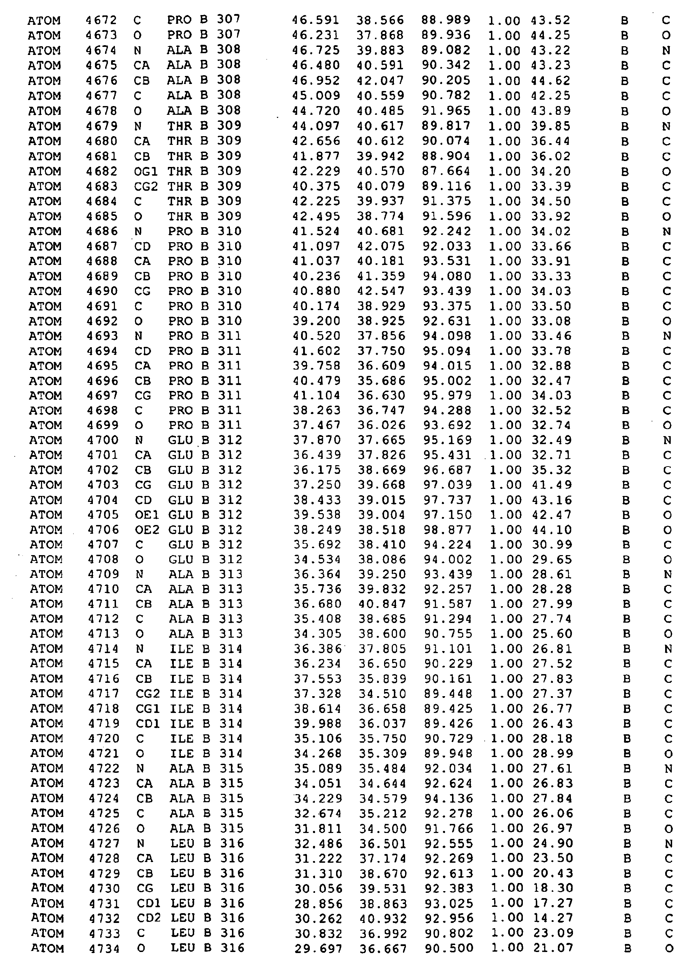

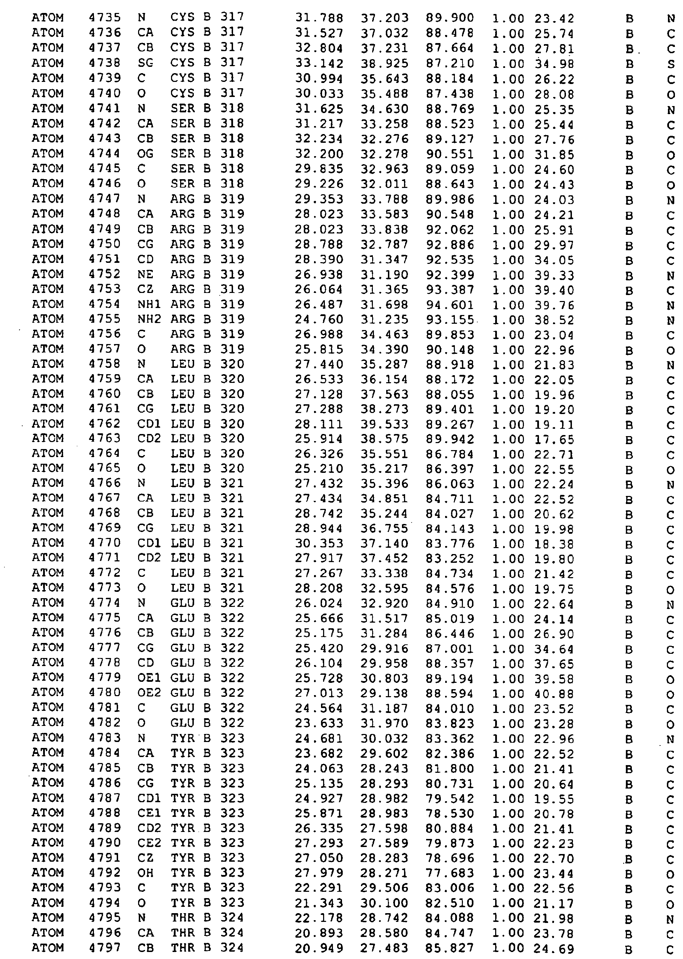

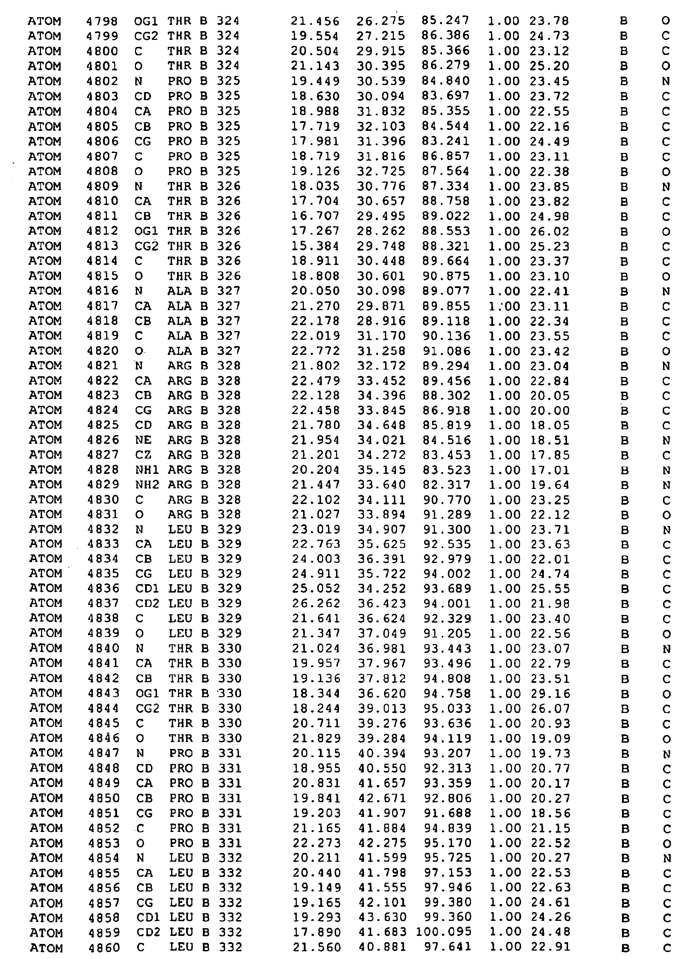

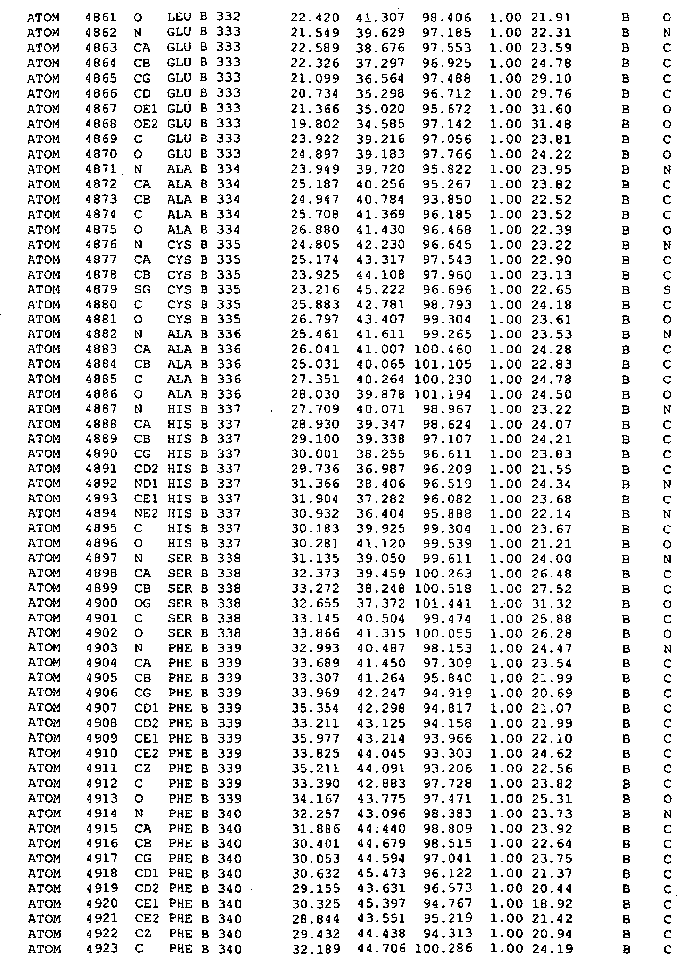

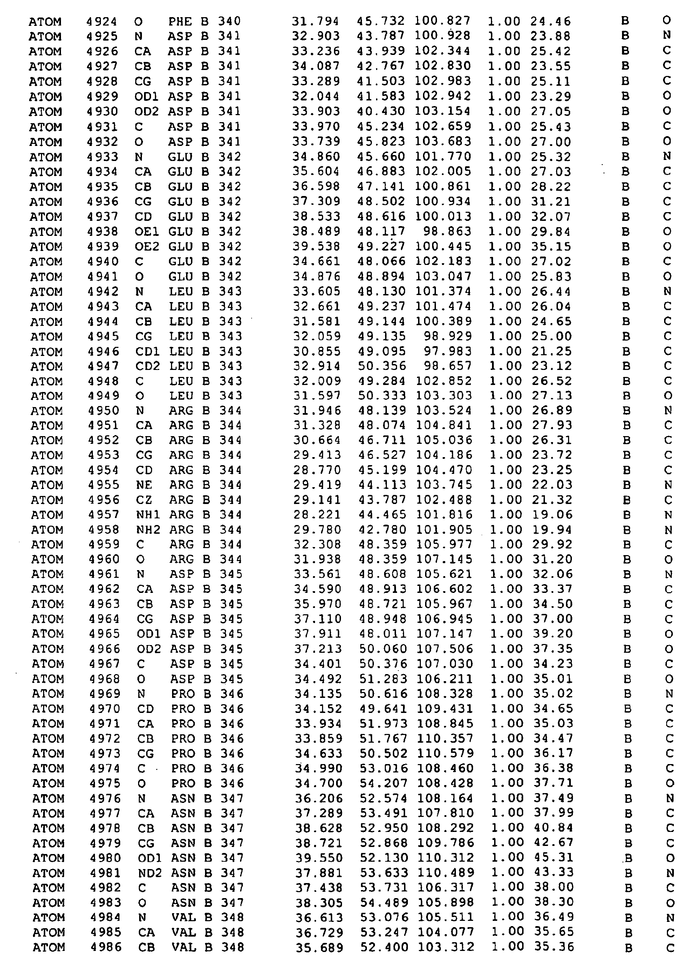

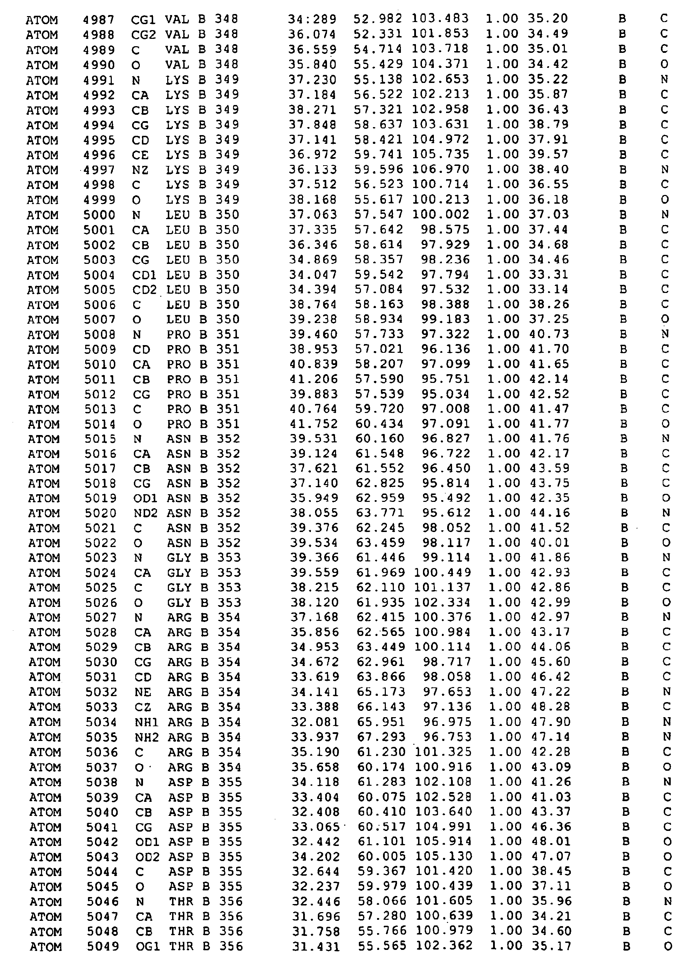

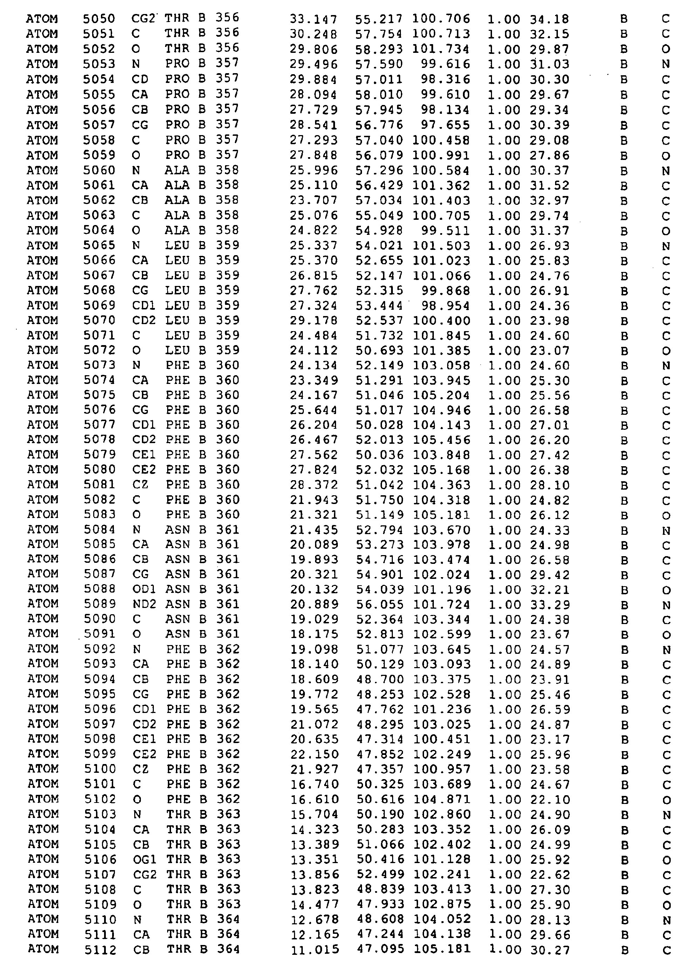

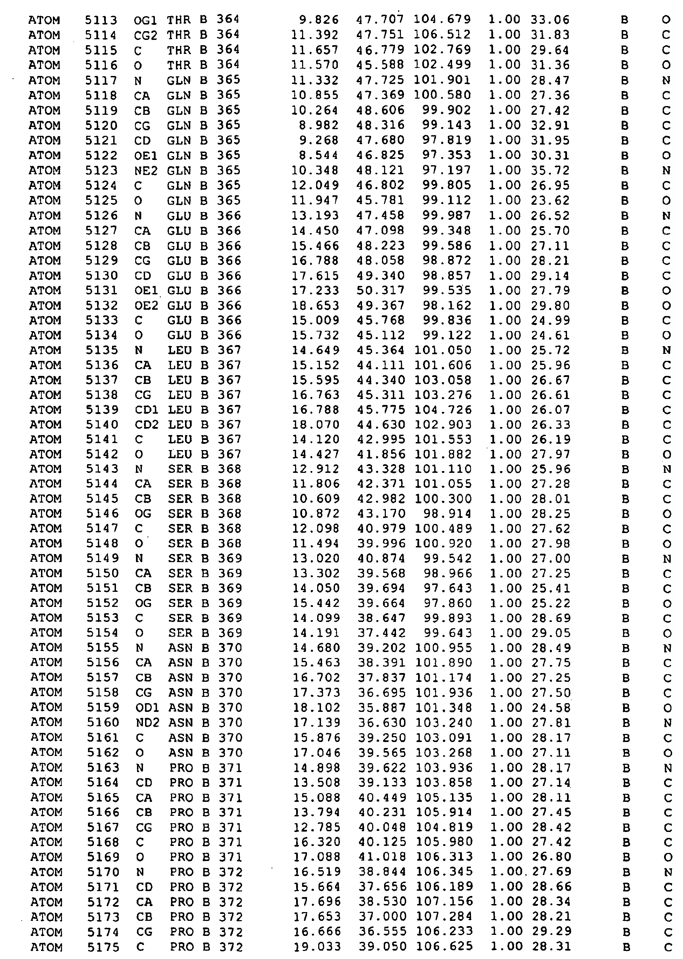

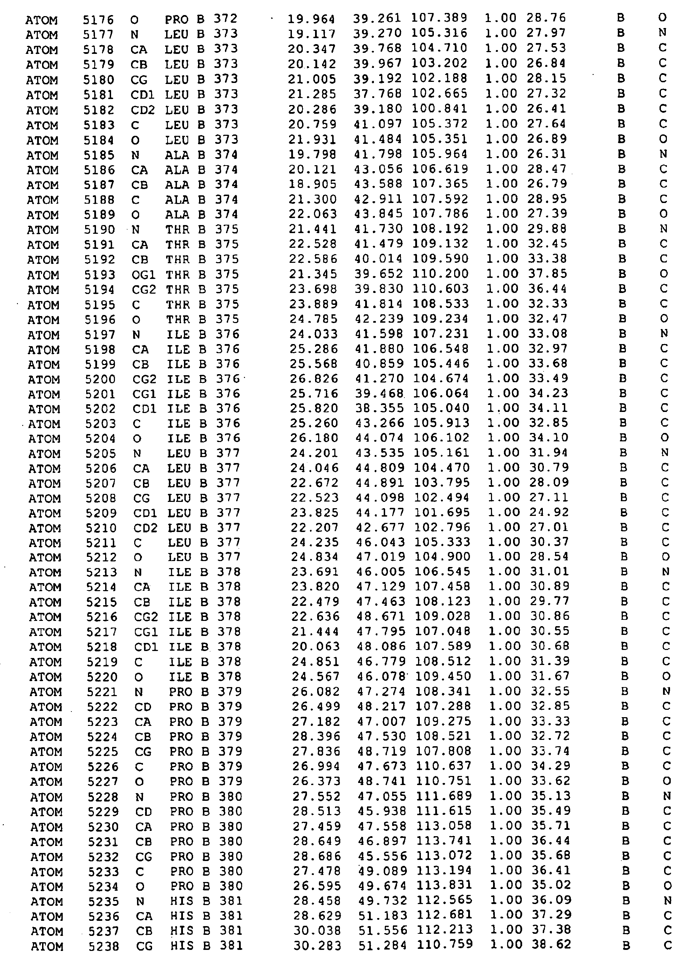

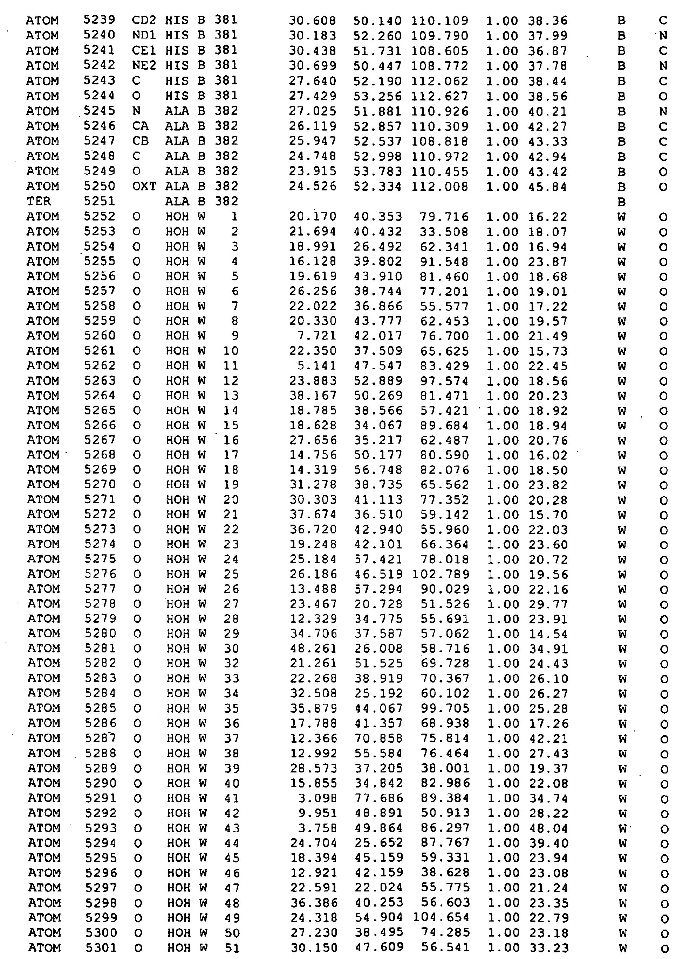

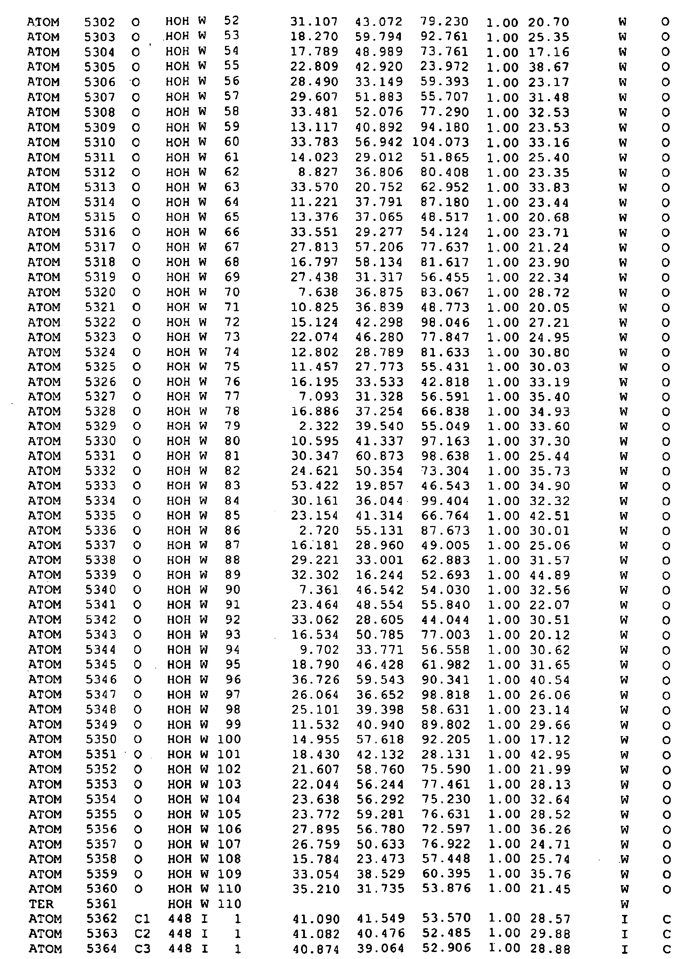

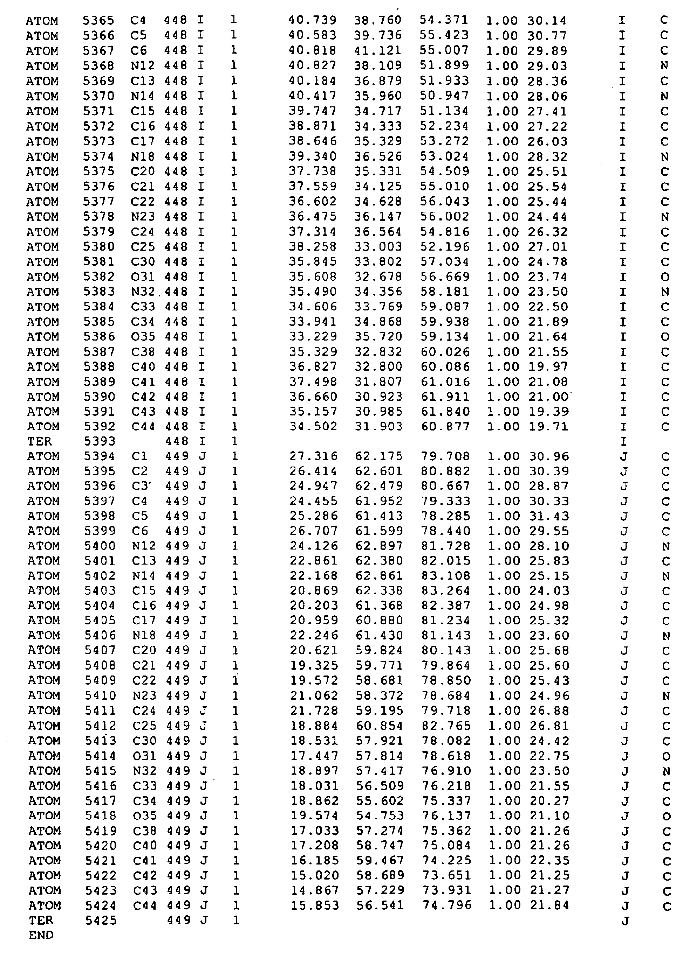

In the crystal structure of the unphosphorylated GSK-3β-inhibitor3 complex, amino acid residues I62, N64, G65, S66, F67, G68, V69, V70, A83, K85, K86, V87, E97, V110, L132, D133, Y134, V135, P136, E137, T138, R141, K183, Q185, N186, L188, C199 and D200 according to Figure 5 were within 5 Å of the inhibitor bound in the active site. These amino acid residues were identified using the programs QUANTA, O and RIBBONS. In addition, amino acid residues V61, I62, G63, N64, G65, S66, F67, G68, V69, V70, Y71, Q72, L81, V82, A83, I84, K85, K86, V87, L88, E97, M101, V110, R111, L130, V131, L132, D133, Y134, V135, P136, E137, T138, V139, Y140, R141, H179, D181, K183, Q185, N186, L187, L188, L189, K197, L198, C199, D200, F201, G202, S203 and S219 according to Figure 5 were within 8 Å of the inhibitor bound in the active site. These amino acid residues were identified using the programs QUANTA, O and RIBBON.

In the crystal structure of the unphosphorylated GSK-3β-inhibitor4 complex, amino acid residues I62, G63, N64, F67, V70, A83, V110, L132, D133, Y134, V135, P136, E137, T138, R141, Q185, N186, L188, C199 and D200 according to Figure 6 were within 5 Å of the inhibitor bound in the active site. These amino acid residues were identified using the programs QUANTA, O and RIBBONS. In addition, applicants have determined that amino acid residues V61, I62, G63, N64, G65, S66, F67, G68, V69, V70, Y71, Q72, L81, V82, A83, I84, K85, E97, M101, V110, R111, L130, V131, L132, D133, Y134, V135, P136, E137, T138, V139, Y140, R141, D181, K183, P184, Q185, N186, L187, L188, L189, K197, L198, C199, D200 and F201 according to Figure 6 were within 8 Å of the inhibitor bound in the active site. These amino acid residues were identified using the programs QUANTA, O and RIBBONS.

Using a multiple alignment program to compare the unphosphorylated GSK-3β structure and structures of other members of the protein kinase family, amino acid residues Y56, T59, K60, V61, V69, V70, Y71, Q72, A73, K74, L75, L81, V82, A83, I84, K85, K86, L98, M101, R102, L104, H106, C107, N108, I109, V110, R111, L112, R113, Y114, F115, F116, L128, N129, L130, V131, L132, D133, Y134, V135, P136, E137, T138, V139, Y140, R141, V142, P154, V155, I156, Y157, V158, K159, L160, Y161, M162, Y163, Q164, L165, F166, R167, S168, L169, A170, Y171, I172, H173, S174, F175, G176, I177, C178, H179, R180, D181, I182, K183, P184, Q185, N186, L187, L188, L189, D190, P191, A194, V195, L196, K197, L198, C199 and D200 according to Figure 1 were identified as the ATP-binding pocket (Gerstein et al., J. Mol. Biol., 251, pp. 161-175 (1995),). To perform this comparison, first, a sequence alignment between members of the protein kinase family was performed. Second, a putative core was constructed by superimposing a series of corresponding structures in the protein kinase family. Third, residues of high spatial variation were discarded, and the core alignment was iteratively refined. The amino acids that make up the final core structure have low structural variance and have similar local and global conformation relative to the corresponding residues in the protein family.

In the crystal structure of the phosphorylated GSK-3β-ADP-peptide complex, amino acids residues G65, S66, F67, D90, K91, R92, F93, K94, R96, R180, D181, K183, G202, S203, K205, P212, N213, V214, Y216, I217, C218, S219, R223, Y234 according to Figure 7 were within 5 Å of the peptide bound in the substrate binding groove. These amino acid residues were identified using the programs QUANTA, O and RIBBONS, *supra.* Amino acid residues D90, K91, R92, F93, K94 made backbone interactions with the peptide substrate. Amino acid residues R96, R180, K205, N213, Y234 bound to pS656 of the peptide substrate. Amino acid residues R96, R180 and K205 formed a positively charged binding pocket surrounding the pS656. Amino acid residue S66 bound to the backbone of amino acid residue S652 from the peptide substrate. Amino acid residues F67, F93 form an aromatic binding pocket surrounding the peptide substrate amino acid residue H650.

In the crystal structure of the phosphorylated GSK-3β-ADP-peptide complex, amino acid residues N64, G65, S66, F67, G68, V87, L88, D90, K91, R92, F93, K94, N95, R96, E97, R180, D181, 1182, K183 Q185, N186, D200, F201, G202, S203, A204, K205, Q206, L207, E211, P212, N213, V214, S215, Y216, I217, C218, S219, R220, Y221, Y222, R223, L227, T232 and Y234 according to Figure 5 were within 8 Å of the peptide bound in the substrate binding groove. These amino acid residues were identified using the programs QUANTA, O and RIBBONS, supra. Amino acid residues Y216, I217, C218, S219, R220 and R223 form a binding pocket that accommodates the proline side chain of the peptide substrate.

In the GSK-3β-ADP-peptide electron density map, the side chains of residues F67, K91, and R92 in the substrate binding pocket could not be located. Alanine and glycine residues were used to build the structure model at these positions. The structure coordinates of amino acid residues F67, K91 and R92 refer to the structure coordinates of amino acid residues A67, A91 and G92 in Figure 7, respectively. In Figures 1-7, where alanine or glycine residues were built in the model as a result of missing side chains in the electron density map, the same applies to those residues.

The inhibitor-binding pocket may comprise GSK-3β amino acid residues K85, M101, V110, L130 and L132 according to any one of Figures 1-7. The inhibitor-binding pocket may comprise GSK-3β amino acid residues I62, V135, P136, T138 and L188 according to any one of Figures 1-7. The inhibitor-binding pocket may comprise GSK-3β amino acid residues V70, V110, L188 and C199 according to any one of Figures 1-7. The inhibitor-binding pocket may comprise GSK-3β amino acids F67, V70, Q185 and C199 according to any one of Figures 1-7.

The inhibitor-binding pocket may comprise amino acid residues V61, I62, G63, N64, G65, S66, F67, G68, V69, V70, Y71, Q72, L81, V82, A83, I84, K85, K86, V87, L88, E97, M101, V110, R111, L112, L130, V131, L132, D133, Y134, V135, P136, E137, T138, V139, Y140, R141, H179, D181, K183, P184, Q185, N186, L187, L188, L189, K197, L198, C199, D200, F201, G202, S203 and S219 according to any one of Figures 1-7.

The ATP-binding pocket may comprise amino acid residues Y56, T59, K60, V61, V69, V70, Y71, Q72, A73, K74, L75, L81, V82, A83, I84, K85, K86, L98, M101, R102, L104, H106, C107, N108, I109, V110, R111, L112, R113, Y114, F115, F116, L128, N129, L130, V131, L132, D133, Y134, V135, P136, E137, T138, V139, Y140, R141, V142, P154, V155, I156, Y157, V158, K159, L160, Y161, M162, Y163, Q164, L165, F166, R167, S168, L169, A170, Y171, I172, H173, S174, F175, G176, I177, C178, H179, R180, D181, I182, K183, P184, Q185, N186, L187, L188, L189, D190, P191, A194, V195, L196, K197, L198, C199 and D200 according to any one of Figures 1-7.

The substrate binding pocket may comprise amino acid residues S66, F67 and F93 according to any one of Figures 1-7. The substrate binding pocket may comprise amino acid residues G65, S66, F67 and F93 according to any one of Figures 1-7.

The substrate binding pocket may comprise amino acid residues D90, K91, R92, F93, K94 according to any one of Figures 1-7.

The substrate binding pocket may comprise amino acid residues R96, R180, K205, N213 and Y234 according to any one of Figures 1-7. The substrate binding pocket may comprise amino acid residues R96, R180 and K205 according to any one of Figures 1-7.

The substrate binding pocket may comprise amino acid residues Y216, I217, C218, S219, R220 and R223 according to any one of Figures 1-7.

The substrate binding pocket may comprise amino acid residues G65, S66, F67, D90, K91, R92, F93, K94, R96, R180, D181, K183, G202, S203, K205, P212, N213, V214, Y216, I217, C218, S219, R223 and Y234 according to any one of Figures 1-7.

The substrate binding pocket may comprise amino acid residues N64, G65, S66, F67, G68, V87, L88, D90, K91, R92, F93, K94, N95, R96, E97, R180, D181, I182, K183 Q185, N186, D200, F201, G202, S203, A204, K205, Q206, L207, E211, P212, N213, V214, S215, Y216, I217, C218, S219, R220, Y221, Y222, R223, L227, T232 and Y234 according to any one of Figures 1-7.

Thus, the binding pockets disclosed in the application are defined by the structure coordinates of the above amino acids, as set forth in Figures 1-7.

It will be readily apparent to those of skill in the art that the numbering of amino acid residues in other homologues of GSK-3β may be different than that set forth for GSK-3β. Corresponding amino acid residues in homologues of GSK-3β are easily identified by visual inspection of the amino acid sequences or by using commercially available homology software programs.

Those of skill in the art understand that a set of structure coordinates for an enzyme or an enzyme-complex or a portion thereof, is a relative set of points that define a shape in three dimensions. Thus, it is possible that an entirely different set of coordinates could define a similar or identical shape. Moreover, slight variations in the individual coordinates will have little effect on overall shape. In terms of binding pockets, these variations would not be expected to significantly alter the nature of ligands that could associate with those pockets.

The variations in coordinates discussed above may be generated because of mathematical manipulations of the GSK-3β structure coordinates. For example, the structure coordinates set forth in any one of Figures 1-7 could be manipulated by crystallographic permutations of the structure coordinates, fractionalization of the structure coordinates, integer additions or subtractions to sets of the structure coordinates, inversion of the structure coordinates or any combination of the above.

Alternatively, modifications in the crystal structure due to mutations, additions, substitutions, and/or deletions of amino acids, or other changes in any of the components that make up the crystal could also account for variations in structure coordinates. If such variations are within a certain root mean square deviation as compared to the original coordinates, the resulting three-dimensional shape is considered encompassed by this invention. Thus, for example, a ligand that bound to the binding pocket of GSK-3β would also be expected to bind to another binding pocket whose structure coordinates defined a shape that fell within the acceptable root mean square deviation.

Various computational analyses may be necessary to determine whether a molecule or the binding pocket or portion thereof is sufficiently similar to the GSK-3β binding pockets described above. Such analyses may be carried out using well known software applications, such as the Molecular Similarity application of QUANTA (Molecular Simulations Inc., San Diego, CA ⓒ 1998), CCP4 (Acta Crystallogr., D50, 760-763 (1994)) or ProFit (A. C.R. Martin, ProFit version 1.8, http//www.bioinfo.org.uk/software).

The Molecular Similarity software application permits comparisons between different structures, different conformations of the same structure, and different parts of the same structure. The procedure used in Molecular Similarity to compare structures is divided into four steps: 1) load the structures to be compared; 2) define the atom equivalences in these structures; 3) perform a fitting operation; and 4) analyze the results.

Each structure in the comparison is identified by a name. One structure is identified as the target (i.e., the fixed structure); all remaining structures are working structures (i.e., moving structures). Since atom equivalency within QUANTA is defined by user input, for the purpose of this invention we will define equivalent atoms as protein backbone atoms N, C, O and Cα for all corresponding amino acids between the two structures being compared.

The corresponding amino acids may be identified by sequence alignment programs such as the "bestfit" program available from the Genetics Computer Group which uses the local homology algorithm described by Smith and Waterman in Advances in Applied Mathematics 2, 482 (1981). The identification of equivalent residues can also be assisted by secondary structure alignment, for example, aligning the α-helices, β-sheets or hinge region (residues 126-135 in GSK-3β) in the structure. For programs that calculate RMSD values of the backbone atoms, an RMSD cutoff value can be used to exclude pairs of equivalent atoms with extreme individual RMSD values, or in situations where the equivalent atom can not be found in the corresponding structure.

When a rigid fitting method is used, the working structure is translated and rotated to obtain an optimum fit with the target structure. The fitting operation uses an algorithm that computes the optimum translation and rotation to be applied to the moving structure, such that the root mean square difference of the fit over the specified pairs of equivalent atom is an absolute minimum. This number, given in angstroms, is reported by QUANTA.

The RMSD values of the inhibitor and substrate-binding pockets between the GSK-3β-ADP-peptide structure (Figure 7) and other GSK-3β structures (Figures 1-6) are illustrated in Tables 3-9. The RMSD values were calculated by the program LSQKAB in CCP4, supra. Backbone atoms (C, O, N and Cα) of all residues in the binding pocket were used in the calculation of the RMSD.

**TABLE 3**

| Inhibitor-binding pocket (K85, M101, V110, L130 and L132) | |
|---|---|
| GSK-3β structures | RMSD between inhibitor binding pockets (Å) |
| unphosphorylated GSK-3β | 0.32 |
| phosphorylated GSK-3β | 0.34 |
| GSK-3β inhibitor1 complex | 0.32 |
| GSK-3β inhibitor2 complex | 0.39 |
| GSK-3β inhibitor3 complex | 0.27 |
| GSK-3β inhibitor4 complex | 0.27 |

**TABLE 4**

| Inhibitor-binding pocket (I62, V135, P136, T138 and L188) | |
|---|---|
| GSK-3β structures | RMSD between inhibitor binding pockets (Å) |
| unphosphorylated GSK-3β | 1.16 |
| phosphorylated GSK-3β | 1.02 |
| GSK-3β inhibitor1 complex | 0.77 |
| GSK-3β inhibitor2 complex | 0.95 |
| GSK-3β inhibitor3 complex | 0.23 |
| GSK-3β inhibitor4 complex | 0.31 |

**TABLE 5**

| Inhibitor-binding pocket (F67, V70, Q185, C199) | |
|---|---|
| GSK-3β structures | RMSD between inhibitor binding pockets (Å) |
| unphosphorylated GSK-3β | 1.83 |
| phosphorylated GSK-3β | 1.89 |
| GSK-3β inhibitor1 complex | 1.67 |
| GSK-3β inhibitor2 complex | 2.23 |
| GSK-3β inhibitor3 complex | 1.71 |
| GSK-3β inhibitor4 complex | 0.80 |

**TABLE 6**

| Inhibitor-binding pocket (V70, V110, L188 and C199) | |
|---|---|
| GSK-3β structures | RMSD between inhibitor binding pockets (Å) |
| unphosphorylated GSK-3β | 0.80 |
| phosphorylated GSK-3β | 0.88 |
| GSK-3β inhibitor1 complex | 0.81 |
| GSK-3β inhibitor2 complex | 0.92 |
| GSK-3β inhibitor3 complex | 0.38 |
| GSK-3β inhibitor4 complex | 0.30 |

**TABLE 7**

| Substrate-binding pocket (G65, S66, F67 and F93) | |
|---|---|
| GSK-3β structures | RMSD between inhibitor binding pockets (Å) |
| unphosphorylated GSK-3β | 1.93 |
| phosphorylated GSK-3β | 1.32 |
| GSK-3β inhibitor1 complex | 1.32 |
| GSK-3β inhibitor2 complex | 1.74 |
| GSK-3β inhibitor3 complex | 1.43 |
| GSK-3β inhibitor4 complex | 2.09 |

**TABLE 8**

| Substrate-binding pocket (R96, R180, K205, N213 and Y234) | |
|---|---|
| GSK-3β structures | RMSD between inhibitor binding pockets (Å) |
| unphosphorylated GSK-3β | 0.67 |
| phosphorylated GSK-3β | 0.59 |
| GSK-3β inhibitor1 complex | 0.64 |
| GSK-3β inhibitor2 complex | 0.71 |
| GSK-3β inhibitor3 complex | 0.71 |
| GSK-3β inhibitor4 complex | 0.65 |

**TABLE 9**

| Substrate-binding pocket (Y216, 1217, C218, S219, R220 and R223) | |
|---|---|
| GSK-30 structures | RMSD between inhibitor binding pockets (Å) |
| unphosphorylated GSK-3β | 1.07 |
| phosphorylated GSK-3β | 0.36 |
| GSK-3β inhibitor1 complex | 0.46 |
| GSK-3β inhibitor2 complex | 1.35 |
| GSK-3β inhibitor3 complex | 1.39 |
| GSK-3β inhibitor4 complex | 1.21 |

The RMSD values of the overall structure between the GSK-3β-inhibitor2 structure (Figure 4) and other GSK-3β structures (Figures 1-3, and 5-7) are illustrated in Table 10. The RMSD values were calculated by the program LSQKAB in CCP4, supra. Backbone atoms (C, O, N and Cα) of all residues in the overall structure according to Figures 1-7 were used in the calculation of the RMSD.

**TABLE 10**

| GSK-3β structures | RMSD of overall structures (Å) |
|---|---|
| unphosphorylated GSK-3β | 0.52 |
| phosphorylated GSK-3β | 1.27 |
| GSK-3β-ADP-peptide complex | 1.94 |
| GSK-3β inhibitor1 complex | 0.79 |
| GSK-3β inhibitor3 complex | 1.77 |
| GSK-3β inhibitor4 complex | 0.90 |

The application discloses a molecule or molecular complex comprising a binding pocket defined by structure coordinates of amino acid residues which are identical to GSK-3β amino acid residues K85, M101, V110, L130 and L132 according to any one of Figures 1-7, wherein the root mean square deviation of the backbone atoms between said amino acid residues and said GSK-3β amino acid residues is not greater than about 3.0 Å. The RMSD may be not greater than about 1.0 Å. Preferred the RMSD may be not greater than about 0.5 Å.

More preferred the root mean square deviation of the backbone atoms between said amino acid residues and said GSK-3β amino acid residues according to Figure 7 may be not greater than about 0.5 Å. The RMSD may be not greater than about 0.2 Å.

The root mean square deviation of the backbone atoms between said amino acid residues and said GSK-3β amino acid residues according to any one of Figures 1-7 may be not greater than about 0.3 Å, and wherein at least one of said amino acid residues is not identical to the GSK-3β amino acid residue to which it corresponds. The RMSD may be not greater than about 0.2 Å.

The application discloses a molecule or molecular complex comprising a binding pocket defined by structure coordinates of amino acid residues which are identical to GSK-3β amino acid residues 162, V135, P136, T138 and L188 according to any one of Figures 1-7, wherein the root mean square deviation of the backbone atoms between said amino acid residues and said GSK-3β amino acid residues is not greater than about 3.0 Å. The RMSD may be not greater than about 1.5 Å. Preferred the RMSD may be not greater than about 1.0 Å.

More preferred the root mean square deviation of the backbone atoms between said amino acid residues and said GSK-3β amino acid residues according to Figure 7 may be not greater than about 0.8 Å. The RMSD may be not greater than about 0.5 Å. The RMSD may be not greater than about 0.3 Å.

The root mean square deviation of the backbone atoms between said amino acid residues and said GSK-3β amino acid residues according to any one of Figures 1-7 may be not greater than about 0.2 Å, and wherein at least one of said amino acid residues is not identical to the GSK-3β amino acid residue to which it corresponds.

The application discloses a molecule or molecular complex comprising a binding pocket defined by structure coordinates of amino acid residues which are identical to GSK-3β amino acid residues V70, V110, L188 and C199 according to any one of Figures 1-7, wherein the root mean square deviation of the backbone atoms between said amino acid residues and said GSK-3β amino acid residues is not greater than about 3.0 Å. The RMSD may be not greater than about 1.5 Å. Preferred the RMSD may be not greater than about 1.0 Å.

More preferred, the root mean square deviation of the backbone atoms between said amino acid residues and said GSK-3β amino acid residues according to Figure 7 may be not greater than about 0.6 Å. The RMSD may be not greater than about 0.4 Å. The RMSD may be not greater than about 0.2 Å.

The root mean square deviation of the backbone atoms between said amino acid residues and said GSK-3β amino acid residues according to any one of Figures 1-7 may be not greater than about 0.2 Å, and wherein at least one of said amino acids is not identical to the GSK-3β amino acid to which it corresponds.

The application discloses a molecule or molecular complex comprising a binding pocket defined by structure coordinates of amino acid residues which are identical to GSK-3β amino acid residues V70, F67, Q185 and C199 according to any one of Figures 1-7, wherein the root mean square deviation of the backbone atoms between said amino acid residues and said GSK-3β amino acid residues is not greater than about 3.0 Å. Preferred the RMSD may be not greater than about 2.5 Å.

More preferred the root mean square deviation of the backbone atoms between said amino acid residues and said GSK-3β amino acid residues according to Figure 7 may be not greater than about 1.6 Å. The RMSD may be not greater than about 1.1 Å. The RMSD may be not greater than about 0.6 Å. The RMSD may be not greater than about 0.2 Å.

The root mean square deviation of the backbone atoms between said amino acid residues and said GSK-3β amino acid residue according to any one of Figures 1-7 may be not greater than about 0.3 Å, and wherein at least one of said amino acid residues is not identical to the GSK-3β amino acid residues to which it corresponds. The RMSD may be not greater than about 0.2 Å.

The application discloses a molecule or molecular complex comprising part of a binding pocket, said binding pocket defined by structure coordinates of amino acid residues which correspond to GSK-3β amino acid residues Y56, T59, K60, V61, I62, G63, N64, G65, S66, F67, G68, V69, V70, Y71, Q72, A73, K74, L75, L81, V82, A83, I84, K85, K86, V87, L88, E97, L98, M101, R102, L104, H106, C107, N108, I109, V110, R111, L112, R113, Y114, F115, F116, L128, N129, L130, V131, L132, D133, Y134, V135, P136, E137, T138, V139, Y140, R141, V142, R144, P154, V155, I156, Y157, V158, K159, L160, Y161, M162, Y163, Q164, L165, F166, R167, S168, L169, A170, Y171, I172, H173, S174, F175, G176, I177, C178, H179, R180, D181, I182, K183, P184, Q185, N186, L187, L188, L189, D190, P191, A194, V195, L196, K197, L198, C199, D200, F201, G202, S203 and S219 according to any one of Figures 1-7, wherein the root mean square deviation of the backbone atoms between said amino acid residues and said GSK-3β amino acid residues is not greater than about 0.2 Å. Said amino acid residues may be identical to said GSK-3β amino acid residues.

The application also discloses a molecule or molecular complex comprising a binding pocket defined by structure coordinates of amino acid residues which are identical to GSK-3β amino acid residues G65, S66, F67 and F93 according to any one of Figures 1-7, wherein the root mean square deviation of the backbone atoms between said amino acid residues and said GSK-3β amino acid residues is not greater than about 3.0 Å. The RMSD may be not greater than about 2.5 Å. Preferred the RMSD may be not greater than about 2.0 Å.

More preferred the root mean square deviation of the backbone atoms between said amino acid residues and said GSK-3β amino acid residues according to Figure 7 may be not greater than about 1.5 Å. The RMSD may be not greater than about 1.1 Å. The RMSD may be not greater than about 0.7 Å. The RMSD may be not greater than about 0.5 Å.

The root mean square deviation of the backbone atoms between said amino acid residues and said GSK-3β amino acid residues according to any one of Figures 1-7 may be not greater than about 1.1 Å and wherein at least one of said amino acid residues is not identical to the GSK-3β amino acid residues to which it corresponds. The RMSD may be not greater than about 0.8 Å. The RMSD may be not greater than about 0.4 Å. The RMSD may be not greater than about 0.2 Å.

The application discloses a molecule or molecular complex comprising a binding pocket defined by structure coordinates of amino acid residues which correspond to GSK-3β amino acids R96, R180, K205, N213 and Y234 according to any one of Figures 1-7, wherein the root mean square deviation of the backbone atoms between said amino acid residues and said GSK-3β amino acid residues is not greater than about 3.0 Å, wherein said binding pocket comprises an amino acid residue asparagine corresponding to said GSK-3β amino acid residue N213. Said amino acid residues may be identical to said GSK-3β amino acids. The RMSD may be not greater than about 1.5 Å. Preferred the RMSD may be not greater than about 1.0 Å.

More preferred the root mean square deviation of backbone atoms between said amino acid residues and said GSK-3β amino acid residues according to Figure 7 may be not greater than about 0.4 Å. The RMSD may be not greater than about 0.2 Å.

The root mean square deviation of the backbone atoms between said amino acid residues and said GSK-3β amino acid residues according to any one of Figures 1-7 may be not greater than about 3.0 Å, and wherein at least one of said amino acid residues is not identical to the GSK-3β amino acid residues to which it corresponds. The RMSD may be not greater than about 1.2 Å. The RMSD may be not greater than about 0.7 Å. The RMSD may be not greater than about 0.3 Å.

The application discloses a molecule or molecular complex comprising a binding pocket defined by structure coordinates of amino acid residues which correspond to GSK-3β amino acid residues Y216, I217, C218, S219, R220 and R223 according to any one of Figures 1-7, wherein the root mean square deviation of the backbone atoms between said amino acid residues and said GSK-3β amino acid residues is not greater than about 3.0 Å, wherein said binding pocket comprises a cysteine amino acid residue corresponding to said GSK-3β amino acid residue C218. Said amino acid residues may be identical to said GSK-3β amino acids. The RMSD may be not greater than about 2.0 Å. Preferred the RMSD may be not greater than about 1.5 Å.

More preferred , the root mean square deviation of backbone atoms between said amino acid residues and said GSK-3β amino acid residues according to Figure 5 may be not greater than about 1.1 Å. The RMSD may be not greater than about 0.5 Å. The RMSD may be not greater than about 0.2 Å.

The root mean square deviation of the backbone atoms between said amino acid residues and said GSK-3β amino acid residues according to any one of Figures 1-7 may be not greater than about 1.1 Å, and wherein at least one of said amino acid residues is not identical to the GSK-3β amino acid to which it corresponds. The RMSD may be not greater than about 0.8 Å. The RMSD may be not greater than about 0.5 Å. The RMSD may be not greater than about 0.2 Å.

The application discloses a molecule or molecular complex comprising part of a binding pocket, said binding pocket defined by structure coordinates of amino acid residues which correspond to GSK-3β amino acid residues N64, G65, S66, F67, G68, V87, L88, D90, K91, R92, F93, K94, N95, R96, E97, R180, D181, I182**,** K183 Q185, N186, D200, F201, G202, S203, A204, K205, Q206, L207, E211, P212, N213, V214, S215, Y216, I219, C218, S219, R220, Y221, Y222, R223, L227, T232 and Y234 according to any one of Figures 1-7, wherein the root mean square deviation of the backbone atoms between said amino acid residues and said GSK-3β amino acid residues is not greater than about 1.0 Å. The RMSD may be greater than about 0.7 Å. The RMSD may be not greater than about 0.5 A. The may be not greater than about 0.2 Å. Said amino acid residues may be identical to said GSK-3β amino acid residues.

The application discloses a molecule or molecular complex comprising a GSK-3β protein defined by structure coordinates of the amino acid residues which correspond to GSK-3β amino acid residues according to any one of Figures 1-7, wherein the root mean square deviation of the backbone atoms between said amino acid residues and said GSK-3β amino acid residues is not greater than about 2.0 Å. The RMSD may be not greater than about 1.7 Å. The RMSD may be not greater than about 1.1 Å. The RMSD may be not greater than about 0.7 Å. Said amino acid residues may be identical to said GSK-3β amino acid residues.

The application discloses a molecule or molecular complex comprising a protein kinase comprising a glutamine or glutamic acid residue that corresponds to Gln185 of GSK-3β protein, wherein the χ1 angle is in the range of 123° to 180°, and the χ2 angle is in the range of -174° to -180° and 106° to 180°. The χ1 angle may be in the range of -100° to -180° and the χ2 angle may be in the range of -151° to -180° and 126° to 180°.

The above molecules or molecular complexes may be GSK-3β proteins or a GSK-3β homologues. The above molecules or molecular complexes may be in crystalline form.

### Rational Drug Design

The GSK-3β X-ray coordinate data, when used in conjunction with a computer programmed with software to generate those coordinates into the three-dimensional structure of GSK-3β may be used for a variety of purposes, such as drug discovery. The coordinate data themselves may also be used directly to perform computer modelling and fitting operations.

For example, the structure encoded by the data may be computationally evaluated for its ability to associate with chemical entities. Chemical entities that associate with GSK-3β may inhibit GSK-3β and its homologues, and are potential drug candidates.
Alternatively, the structure encoded by the data may be displayed in a graphical three-dimensional representation on a computer screen. This allows visual inspection of the structure, as well as visual inspection of the structure's association with chemical entities.

Thus, a method for evaluating the potential of a chemical entity to associate with a molecule or molecular complex as described above is disclosed.

This method comprises the steps of: a) employing computational means to perform a fitting operation between the chemical entity and a binding pocket of the molecule or molecular complex; b) analyzing the results of said fitting operation to quantify the association between the chemical entity and the binding pocket; and optionally c) outputting said quantified association to a suitable output hardware, such as a CRT display terminal, a printer or a disk drive, as described previously. The method may further comprise the step of generating a three-dimensional graphical representation of the molecule or molecular complex or binding pocket thereof prior to step a).

Alternatively, the structure coordinates of the above binding pockets can be utilized in a method for identifying an agonist or antagonist of a molecule comprising any of the above binding pockets. This method comprises the steps of:
a) using the three-dimensional structure of said molecule or molecular complex to design or select a chemical entity;
b) contacting said chemical entity with the molecule or molecular complex and monitoring the activity of the molecule or molecular complex; and
c) classifying said chemical entity as an agonist or antagonist based on the effect of said chemical entity on the activity of the molecule or molecular complex.

Step a) may be using the three-dimensional structure of the binding pocket of the molecule or molecular complex. The three-dimensional structure may be displayed as a graphical representation.

The application permits the use of molecular design techniques to identify, select and design chemical entities, including inhibitory compounds, capable of binding to the above binding pockets.

The elucidation of binding pockets on GSK-3β provides the necessary information for designing new chemical entities and compounds that may interact with GSK-3β substrate or ATP-binding pockets, in whole or in part. Due to the homology in the kinase core of GSK-3β and GSK-3α, compounds that inhibit GSK-3β are also expected to inhibit GSK-3α, especially those compounds that bind the ATP-binding pocket.

Throughout this section, discussions about the ability of an entity to bind to, associate with or inhibit the above binding pockets refers to features of the entity alone. Assays to determine if a compound binds to GSK-3β are well known in the art and are exemplified below.

The design of compounds that bind to or inhibit the above binding pockets generally involves consideration of two factors. First, the entity must be capable of physically and structurally associating with parts or all of the above binding pockets. Non-covalent molecular interactions important in this association include hydrogen bonding, van der Waals interactions, hydrophobic interactions and electrostatic interactions.

Second, the entity must be able to assume a conformation that allows it to associate with the above binding pockets directly. Although certain portions of the entity will not directly participate in these associations, those portions of the entity may still influence the overall conformation of the molecule. This, in turn, may have a significant impact on potency. Such conformational requirements include the overall three-dimensional structure and orientation of the chemical entity in relation to all or a portion of the binding pocket, or the spacing between functional groups of an entity comprising several chemical entities that directly interact with the above binding pockets.

The potential inhibitory or binding effect of a chemical entity on the above binding pockets may be analyzed prior to its actual synthesis and testing by the use of computer modeling techniques. If the theoretical structure of the given entity suggests insufficient interaction and association between it and the above binding pockets, testing of the entity is obviated. However, if computer modeling indicates a strong interaction, the molecule may then be synthesized and tested for its ability to bind to the above binding pocket. This may be achieved by testing the ability of the molecule to inhibit GSK-3β using the assays described in Example 18. In this manner, synthesis of inoperative compounds may be avoided.

A potential inhibitor of the above binding pockets may be computationally evaluated by means of a series of steps in which chemical entities or fragments are screened and selected for their ability to associate with the above binding pockets.

One skilled in the art may use one of several methods to screen chemical entities or fragments for their ability to associate with the above binding pockets. This process may begin by visual inspection of, for example, any of the above binding pockets on the computer screen based on the GSK-3β structure coordinates in any one of Figures 1-7 or other coordinates which define a similar shape generated from a machine-readable storage medium. Selected fragments or chemical entities may then be positioned in a variety of orientations, or docked, within that binding pocket as defined supra. Docking may be accomplished using software such as QUANTA and Sybyl, followed by energy minimization and molecular dynamics with standard molecular mechanics force fields, such as CHARMM and AMBER.

Specialized computer programs may also assist in the process of selecting fragments or chemical entities. These include:
1. GRID (P. J. Goodford, "A Computational Procedure for Determining Energetically Favorable Binding Sites on Biologically Important Macromolecules", J. Med. Chem., 28, pp. 849-857 (1985)). GRID is available from Oxford University, Oxford, UK.
2. MCSS (A. Miranker et al., "Functionality Maps of Binding Sites: A Multiple Copy Simultaneous Search Method." Proteins: Structure, Function and Genetics, 11, pp. 29-34 (1991)). MCSS is available from Molecular Simulations, San Diego, CA.
3. AUTODOCK (D. S. Goodsell et al., "Automated Docking of Substrates to Proteins by Simulated Annealing", Proteins: Structure, Function, and Genetics, 8, pp. 195-202 (1990)). AUTODOCK is available from Scripps Research Institute, La Jolla, CA.
4. DOCK (I. D. Kuntz et al., "A Geometric Approach to Macromolecule-Ligand Interactions", J. Mol. Biol., 161, pp. 269-288 (1982)). DOCK is available from University of California, San Francisco, CA.

Once suitable chemical entities or fragments have been selected, they can be assembled into a single compound or complex. Assembly may be preceded by visual inspection of the relationship of the fragments to each other on the three-dimensional image displayed on a computer screen in relation to the structure coordinates of GSK-3β. This would be followed by manual model building using software such as QUANTA or Sybyl (Tripos Associates, St. Louis, MO).

Useful programs to aid one of skill in the art in connecting the individual chemical entities or fragments include:
1. CAVEAT (P. A. Bartlett et al., "CAVEAT: A Program to Facilitate the Structure-Derived Design of Biologically Active Molecules", in "Molecular Recognition in Chemical and Biological Problems", Special Pub., Royal Chem. Soc., 78, pp. 182-196 (1989); G. Lauri and P. A. Bartlett, "CAVEAT: a Program to Facilitate the Design of Organic Molecules", J. Comput. Aided Mol. Des., 8, pp. 51-66 (1994)). CAVEAT is available from the University of California, Berkeley, CA.
2. 3D Database systems such as ISIS (MDL Information Systems, San Leandro, CA). This area is reviewed in Y. C. Martin, "3D Database Searching in Drug Design", J. Med. Chem., 35, pp. 2145-2154 (1992).
3. HOOK (M. B. Eisen et al., "HOOK: A Program for Finding Novel Molecular Architectures that Satisfy the Chemical and Steric Requirements of a Macromolecule Binding Site", Proteins: Struct., Funct., Genet., 19, pp. 199-221 (1994). HOOK is available from Molecular Simulations, San Diego, CA.

Instead of proceeding to build an inhibitor of any of the above binding pockets in a step-wise fashion, one fragment or chemical entity at a time as described above, inhibitory or other GSK-3β binding compounds may be designed as a whole or "de novo" using either an empty binding pocket or optionally including some portion(s) of a known inhibitor(s). There are many de novo ligand design methods including:
1. LUDI (H.-J. Bohm, "The Computer Program LUDI: A New Method for the De Novo Design of Enzyme Inhibitors", J. Comp. Aid. Molec. Design, 6, pp. 61-78 (1992)). LUDI is available from Molecular Simulations Incorporated, San Diego, CA.
2. LEGEND (Y. Nishibata et al., Tetrahedron, 47, p. 8985 (1991)). LEGEND is available from Molecular Simulations Incorporated, San Diego, CA.
3. LeapFrog (available from Tripos Associates, St. Louis, MO).
4. SPROUT (V. Gillet et al., "SPROUT: A Program for Structure Generation)", J. Comput. Aided Mol. Design, 7, pp. 127-153 (1993)). SPROUT is available from the University of Leeds, UK.

Other molecular modeling techniques may also be employed in accordance with this invention (see, e.g., N. C. Cohen et al., "Molecular Modeling Software and Methods for Medicinal Chemistry, J. Med. Chem., 33, pp. 883-894 (1990); see also, M. A. Navia and M. A. Murcko, "The Use of Structural Information in Drug Design", Current Opinions in Structural Biology, 2, pp. 202-210 (1992); L. M. Balbes et al., "A Perspective of Modern Methods in Computer-Aided Drug Design", in Reviews in Computational Chemistry, Vol. 5, K. B. Lipkowitz and D. B. Boyd, Eds., VCH, New York, pp. 337-380 (1994); see also, W. C. Guida, "Software For Structure-Based Drug Design", Curr. Opin. Struct. Biology, 4, pp. 777-781 (1994)).

Once a compound has been designed or selected by the above methods, the efficiency with which that entity may bind to any of the above binding pockets may be tested and optimized by computational evaluation. For example, an effective binding pocket inhibitor must preferably demonstrate a relatively small difference in energy between its bound and free states (i.e., a small deformation energy of binding). Thus, the most efficient binding pocket inhibitors should preferably be designed with a deformation energy of binding of not greater than about 10 kcal/mole, more preferably, not greater than 7 kcal/mole. Binding pocket inhibitors may interact with the binding pocket in more than one conformation that is similar in overall binding energy. In those cases, the deformation energy of binding is taken to be the difference between the energy of the free entity and the average energy of the conformations observed when the inhibitor binds to the protein.

An entity designed or selected as binding to any one of the above binding pockets may be further computationally optimized so that in its bound state it would preferably lack repulsive electrostatic interaction with the target enzyme and with the surrounding water molecules. Such non-complementary electrostatic interactions include repulsive charge-charge, dipole-dipole and charge-dipole interactions.

Specific computer software is available in the art to evaluate compound deformation energy and electrostatic interactions. Examples of programs designed for such uses include: Gaussian 94, revision C (M. J. Frisch, Gaussian, Inc., Pittsburgh, PA ©1995); AMBER, version 4.1 (P. A. Kollman, University of California at San Francisco, ©1995); QUANTA/CHARMM (Molecular Simulations, Inc., San Diego, CA ©1998); Insight II/Discover (Molecular Simulations, Inc., San Diego, CA ©1998): DelPhi (Molecular Simulations, Inc., San Diego, CA ©1998): and AMSOL (Quantum Chemistry Program Exchange, Indiana University). These programs may be implemented, for instance, using a Silicon Graphics workstation such as an Indigo2 with "IMPACT" graphics. Other hardware systems and software packages will be known to those skilled in the art.

Another approach is the computational screening of small molecule databases for chemical entities or compounds that can bind in whole, or in part, to any of the above binding pockets. In this screening, the quality of fit of such entities to the binding pocket may be judged either by shape complementarity or by estimated interaction energy (E. C. Meng et al., J. Compo Chem., 13, pp. 505-524 (1992)).

Although the phosphorylated and unphosphorylated forms of GSK-3β have similar binding pockets, the subtle differences in water molecules, ions, position of the Y216 residue near the binding pockets as well as deviations in the overall structure may render slightly different results in the calculation of binding energies for inhibitors. By comparing the binding energies of inhibitors to the phosphorylated and unphosphorylated form, one may select inhibitors that are more suitable for one form than the other. Furthermore, the identification of inhibitors for both forms would allow the options of inhibiting GSK-3β prior to or after phosphorylation by upstream kinases *in vivo.*

Another particularly useful drug design technique is iterative drug design. Iterative drug design is a method for optimizing associations between a protein and a compound by determining and evaluating the three-dimensional structures of successive sets of protein/compound complexes.

In iterative drug design, crystals of a series of protein or protein complexes are obtained and then the three-dimensional structures of each crystal is solved. Such an approach provides insight into the association between the proteins and compounds of each complex. This is accomplished by selecting compounds with inhibitory activity, obtaining crystals of this new protein/compound complex, solving the three-dimensional structure of the complex, and comparing the associations between the new protein/compound complex and previously solved protein/compound complexes. By observing how changes in the compound affect the protein/compound associations, these associations may be optimized.

In some cases, iterative drug design is carried out by forming successive protein-compound complexes and then crystallizing each new complex. Alternatively, a pre-formed protein crystal is soaked in the presence of an inhibitor, thereby forming a protein/compound complex and obviating the need to crystallize each individual protein/compound complex. The phosphorylated crystals disclosed may be soaked in the presence of a compound or compounds, to provide other crystal complexes.

### Structure Determination of Other Molecules

The structure coordinates set forth in any one of Figures 1-7 can also be used to aid in obtaining structural information about another crystallized molecule or molecular complex. This may be achieved by any of a number of well-known techniques, including molecular replacement.

For example, a Fourier transform of at least a portion of the structure coordinates set forth in any one of Figures 1-7 may be used to determine at least a portion of the structure coordinates of GSK-3β homologues, and other sufficiently homologous kinases such as CDK2. The molecule may be a GSK-3β homologue. The molecular complex may be selected from the group consisting of a GSK-3β protein complex and a GSK-3β homologue complex.

The application discloses a method of utilizing molecular replacement to obtain structural information about a molecule or molecular complex whose structure is unknown comprising the steps of:
a) crystallizing said molecule or molecular complex of unknown structure;
b) generating an X-ray diffraction pattern from said crystallized molecule or molecular complex; and
c) applying at least a portion of the GSK-3β structure coordinates set forth in any one of Figures 1-7 to the X-ray diffraction pattern to generate a three-dimensional electron density map of the molecule or molecular complex whose structure is unknown.

By using molecular replacement, all or part of the structure coordinates of the GSK-3β as disclosed in the application (and set forth in any one of Figures 1-7) can be used to determine the structure of a crystallized molecule or molecular complex whose structure is unknown more quickly and efficiently than attempting to determine such information *ab initio*.

Molecular replacement provides an accurate estimation of the phases for an unknown structure. Phases are a factor in equations used to solve crystal structures that can not be determined directly. Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory estimate of the phases for the unknown structure.

Thus, this method involves generating a preliminary model of a molecule or molecular complex whose structure coordinates are unknown, by orienting and positioning the relevant portion of the GSK-3β according to any one of Figures 1-7 within the unit cell of the crystal of the unknown molecule or molecular complex so as best to account for the observed X-ray diffraction pattern of the crystal of the molecule or molecular complex whose structure is unknown. Phases can then be calculated from this model and combined with the observed X-ray diffraction pattern amplitudes to generate an electron density map of the structure whose coordinates are unknown. This, in turn, can be subjected to any well-known model building and structure refinement techniques to provide a final, accurate structure of the unknown crystallized molecule or molecular complex (E. Lattman, "Use of the Rotation and Translation Functions", in Meth. Enzymol., 115, pp. 55-77 (1985); M. G. Rossmann, ed., the Molecular Replacement Method", Int. Sci. Rev. Ser., No. 13, Gordon & Breach, New York (1972)).

The structure of any portion of any crystallized molecule or molecular complex that is sufficiently homologous to any portion of the GSK-3β can be resolved by this method.

The method of molecular replacement may be utilized to obtain structural information about a GSK-3 homologue. The structure coordinates of GSK-3β as disclosed in the application are particularly useful in solving the structure of GSK-3β complexes that are bound by ligands, substrates and inhibitors.

Furthermore, the structure coordinates of GSK-3β as disclosed in the application are useful in solving the structure of GSK-β proteins that have amino acid substitutions, additions and/or deletions (referred to collectively as "GSK-3β mutants", as compared to naturally occurring GSK-3β. These GSK-3β mutants may optionally be crystallized in co-complex with a chemical entity, such as a non-hydrolyzable ATP analogue, a suicide substrate or a inhibitor. The crystal structures of a series of such complexes may then be solved by molecular replacement and compared with that of wild-type GSK-3β**.** Potential sites for modification within the various binding pockets of the enzyme may thus be identified. This information provides an additional tool for determining the most efficient binding interactions, for example, increased hydrophobic interactions, between GSK-3β and a chemical entity or compound.

The structure coordinates are also particularly useful to solving the structure of crystals of GSK-3β or GSK-3β homologues co-complexed with a variety of chemical entities. This approach enables the determination of the optimal sites for interaction between chemical entities, including candidate GSK-3β inhibitors and GSK-3β. For example, high resolution X-ray diffraction data collected from crystals exposed to different types of solvent allows the determination of where each type of solvent molecule resides. Small molecules that bind tightly to those sites can then be designed and synthesized and tested for their GSK-3β inhibition activity.

All of the complexes referred to above may be studied using well-known X-ray diffraction techniques and may be refined versus 1.5-3.4 Å resolution X-ray data to an R value of about 0.30 or less using computer software, such as X-PLOR (Yale University, ©1992, distributed by Molecular Simulations, Inc.; see, e.g., Blundell & Johnson, *supra*; Meth. Enzymol., vol. 114 & 115, H. W. Wyckoff et al., eds., Academic Press (1985)). This information may thus be used to optimize known GSK-3β inhibitors, and more importantly, to design new GSK-3β inhibitors.

In order that this invention be more fully understood, the following examples are set forth. These examples are for the purpose of illustration only and are not to be construed as limiting the scope of the invention in any way.

### Reference Example 1

### 3-Amino-4,5-diphenyl-1H-pyrazolo[3,4-c]pyridazine (Compound 1)

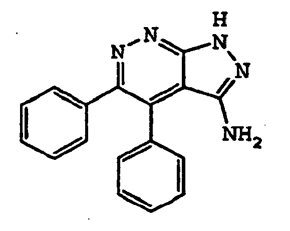

The appropriate diaryl keto hydrazone (see Scheme I, formula 1, wherein X is H and Y is H; 0.85 mmol) and ethyl cyanoacetate (0.9 mmol) were added to 3 mL ethanol. Sodium ethoxide (0.9 mmol) in THF was subsequently added and the mixture refluxed for 6 hours. After cooling, the solvent was removed under vacuum and the residue taken up in 10 mL dichloromethane. It was then washed with 0.1 M HCl, water and dried with sodium sulfate. After filtering, the solvent was removed under vacuum and the product, 4-cyano-5,6-diaryl 2(1H) pyridazinone (Scheme I, formula 2, wherein X is H and Y is H) was purified by chromatography on silica gel (5:95 methanol/dichloromethane).

Purified 4-cyano-5,6-diaryl 2(1H) pyridazinone (100 mg) was added to 2 mL POCl₃ and heated to 100°C for 5-6 hours. After cooling, the reaction mixture was poured onto 10 mL ice and stirred for one hour. The resulting 3-chloro-4-cyano-5,6-diaryl pyridazine (Scheme I, formula 3, wherein X is H and Y is H) was filtered off, washed with water, air dried and used in the next step without further purification.

Crude 3-chloro-4-cyano-5,6-diaryl pyridazine was refluxed with 2 equivalents of anhydrous hydrazine in ethanol for several hours. Upon cooling, the product would sometimes precipitate out, in which case the pure title compound was obtained by recrystallizing from ethanol. Otherwise the title reference compound was purified by chromatography on silica gel (5:95 methanoldichloromethane): MS (ES+) m/e: 288.01 (M+H); analytical HPLC (C₁₈ column) 2.96 minutes.

### Example 2

### 3-Amino-4(4-chlorophenyl)-5-phenyl-1H-pyrazolo[3,4-c]pyridazine (Compound 2)

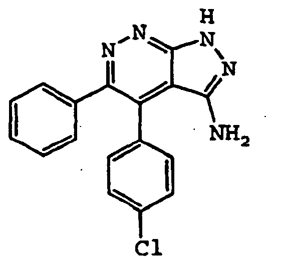

The title compound was obtained according to synthetic procedures described in Ref. EXAMPLE 1 using the appropriate diaryl keto hydrazone (X is H and Y is p-C1): MS (ES+) m/e: 321.89 (M+H); analytical HPLC (C₁₈ column) 3.33 minutes.

### Example 3

### 3-Amino-4,5-bis(4-fluorophenyl)-1H-pyrazolo[3,4-c] pyridazine (Compound 3)

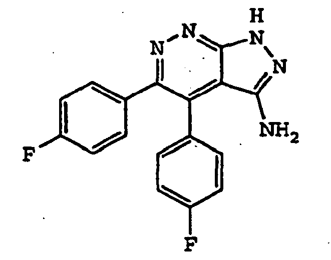

The title compound was obtained according to synthetic procedures described in Ref. EXAMPLE 1 using the appropriate diaryl keto hydrazone (X is p-F and Y is p-F): MS (138+) m/e: 324.1 (M+H); analytical HPLC (C₁₈ column) 3.26 minutes.

### Example 4

### 3-Amino-4-phenyl-5-(4-fluorophenyl)-1H-pyrazolo[3,4-c] pyridazine (Compound 4).

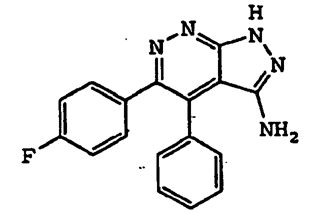

The title compound was obtained according to synthetic procedures described in Ref. EXAMPLE 1 using the appropriate diaryl keto hydrazone (X is p-F and Y is H): MS (ES+) m/e: 306.1 (M+H) ; analytical HPLC (C₁₈ column 3.08 minutes.

### Example 5

### 3-Amino-4-(4-fluorophenyl)-5-phenyl-1H-pyrazolo[3,4-c] pyridazine (Compound 5)

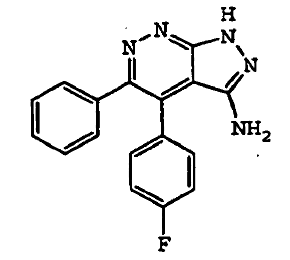

The title compound was obtained according to synthetic procedures described in Ref. EXAMPLE 1 using the appropriate diaryl keto hydrazone (X is H and Y is p-F): MS (ES+) m/e 306.1 (M+H); analytical HPLC (C₁₈ column) 3.02 minutes.

### Example 6

### 3-Amino-4-(3-fluorophenyl)-5-phenyl-1H-pyrazolo[3,4-c] pyridazine (Compound 6)

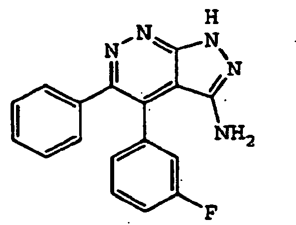

The title compound was obtained according to synthetic procedures described in Ref. EXAMPLE 1 using the appropriate diaryl keto hydrazone (X is H and Y is m-F): MS (ES+) m/e: 306.1 (M+H); analytical HPLC (C₁₈ column) 2.94 minutes.

### Example 7

### 3-Amino-4-phenyl-5-(4-pyridyl)-1H-pyrazolo[3,4-c] pyridazine (Compound 7)

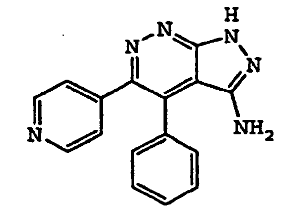

The title compound was obtained according to synthetic procedures described in Ref. EXAMPLE 1 using the appropriate heteroaryl aryl keto hydrazone: MS (ES+) m/e: 289.1 (M+H); analytical HPLC (C₁₈ column) 1.77 minutes.

### Example 8

### 3-Amino-4-phenyl-5-(3-fluorophenyl)-1H-pyrazolo[3,4-c] pyridazine (Compound 8)

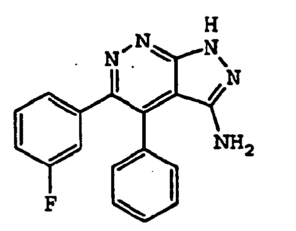

The title compound was obtained according to synthetic procedures described in Ref. EXAMPLE 1 using the appropriate diaryl keto hydrazone (X is m-F and Y is H): MS (ES+) m/e: 306.1 (M+H); analytical HPLC (C₁₈ column) 3.12 minutes.

### Example 9

### N-(4,5-Diphenyl-1H-pyrazolo[3,4-c]pyridazin-3-yl)-acetamide (Compound 9)

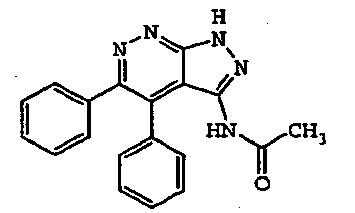

The title compound was obtained according to synthetic procedures described in Ref. EXAMPLE 1 using the appropriate diaryl keto hydrazone (X is H and.Y is H), followed by amidation with CH₃CO₂H: MS (ES+) m/e: 330.1 (M+H) ; analytical HPLC (C₁₈ column) 2.65 minutes.

### Example 10

### N-(4,5-Diphenyl-1H-pyrazolo[3,4-c]pyridazin-3-yl)-benzamide (Compound 10)

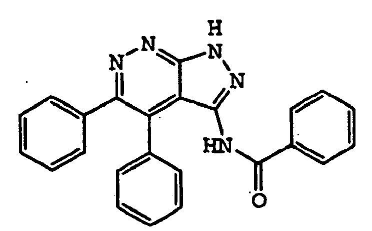

The title compound was obtained according to synthetic procedures described in Ref. EXAMPLE 1 using the appropriate diaryl keto hydrazone (X is H and Y is H), followed by amidation with benzoic acid: MS (ES+) m/e: 414.2 (M+Na+); analytical HPLC (C₁₈ column) 2.90 minutes.

### Example 11

### 3-Amino-4-phenyl-5-(4-methyl-phenyl)-1H-pyrazolo[3,4-c] pyridazine (Compound 11)

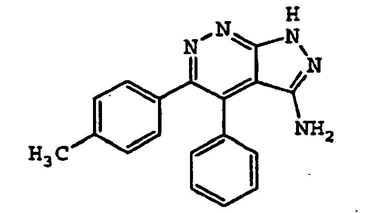

The title compound was obtained according to synthetic procedures described in Ref. EXAMPLE 1 using the appropriate diaryl keto hydrazone (X is p-CH₃ and Y is H): MS (ES+) m/e: 302.1 (M+H); analytical HPLC (C₁₈ column) 3.07 minutes.

### Example 12

### 3-Amino-4-phenyl-5-(2-methyl-phenyl)-1H-pyrazolo[3,4-c] pyridazine (Compound 12)

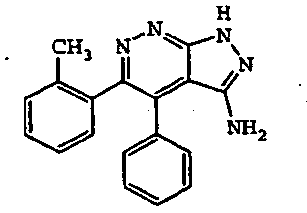

The title compound was obtained according to synthetic procedures described in Ref. EXAMPLE 1 using the appropriate diaryl keto hydrazone (X is o-CH₃ and Y is H): MS (ES+) m/e: 302.1 (M+H); analytical HPLC (C₁₈ column) 2.94 minutes.

### Example 13

### 3-Amino-4-phenyl-5-(3-methyl-phenyl)-1H-pyrazolo[3,4-c] pyridazine (Compound 13)

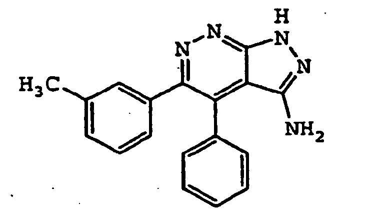

The title compound was obtained according to synthetic procedures described in Ref. EXAMPLE 1 using the appropriate diaryl keto hydrazone (X is m-CH₃ and Y is H): MS (ES+) m/e: 302.1 (M+H): analytical HPLC (C₁₈ column) 3.09 minutes.

### Example 14

### 3-Amino-4-phenyl-5-(2-chloro-phenyl)-1H-pyrazolo[3,4-c] pyridazine (Compound 14)

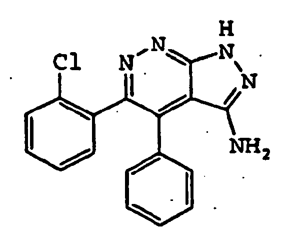

The title compound was obtained according to synthetic procedures described in Ref. EXAMPLE 1 using the appropriate diaryl keto hydrazone (X is o-C1 and Y is H): MS (ES+) m/e: 322.1 (M+H); analytical HPLC (C₁₈ column) 3.48 minutes.

### Example 15

### 3-Amino-4-phenyl-5-(2-fluorophenyl)-1H-pyrazolo[3,4-c] pyridazine (Compound 15)

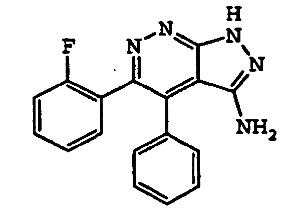

The title compound was obtained according to synthetic procedures described in Ref. EXAMPLE 1 using the appropriate diaryl keto hydrazone (X is o-F and Y is H): MS (ES+) m/e: 306.1 (M+H) ; analytical HPLC (C₁₈ column) 2.97 minutes.

### Example 16

### 3-Amino-4-phenyl-5- (4-chloro-phenyl) -1H-pyrazolo[3,4-c] pyridazine (Compound 16)

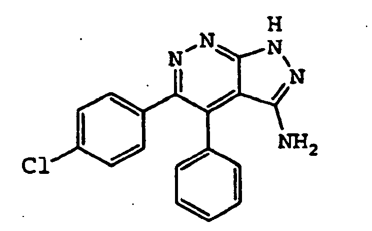

The title compound was obtained according to synthetic procedures described in EXAMPLE 1 using the appropriate diaryl keto hydrazone (X is p-Cl and Y is H): MS (ES+) m/e: 322 (M+H); analytical HPLC (C₁₈ column) 4.06 minutes.

### Reference Example 17

### 4-Phenyl-1H-pyrazolo[3,4-c]pyridazin-3-ylamine(Ref. Compound 32)

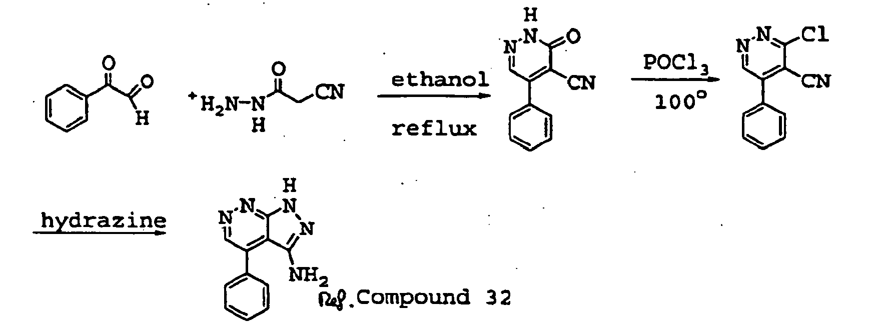

### Step A: 3-Oxo-5-phenyl-2,3-dihydro-pyridazine-4-carbonitrile

Phenyl glyoxal (1.0 g, 7.46 mmol) and cyanoacetohydrazide (740 mg, 7.46 mmol) were heated to reflux in 10 mL ethanol for 16 hours. After cooling, the solvent was evaporated and the crude brown mixture was purified by silica chromatography (1:9 methanol/dichloromethane). The still impure product was further recrystallized from methanol affording 70 mg pure product.

### Step B: 3-Chloro-5-phenyl-pyridazine-4-carbonitrile

3-Oxo-5-phenyl-2,3-dihydro-pyridazine-4-carbonitrile (70 mg) was suspended in 1 mL phosphorous oxychloride and heated to 100°C for 6 hours. The mixture was then cooled and poured onto ice. The resulting brown solid product was filtered and air dried and used in the next step without further purification.

### Step C: 4-Phenyl-1H-pyrazolo[3,4-c]pyridazin-3-ylamine

3-Chloro-5-phenyl-pyridazine-4-carbonitrile obtained from Step B was suspended in 1 mL ethanol with 23 µL hydrazine and the mixture was refluxed for several hours. The solvent was then evaporated and the title reference product was purified by silica gel chromatography (1:9 methanol/dichloromethane): MS (ES+) 212 (M+H); HPLC 1.121 minutes.

### Examples 18

### Kᵢ Determination for the Inhibition of GSK-3

Compounds were screened for their ability to inhibit GSK-3β (amino acids 1-420) activity using a standard coupled enzyme system (Fox et al. (1998) Protein Sci. 7, 2249). Reactions were carried out in a solution containing 100 mM HEPES (pH 7.5), 10 mM MgCl₂, 25 mM NaCl, 300 µM NADH, 1 mM DTT and 1.5% DMSO. Final substrate concentrations in the assay were 20 µM ATP (Sigma Chemicals, St Louis, MO) and 300 µM peptide (HSSPHQS (PO₃H₂) EDEEE, American Peptide, Sunnyvale, CA). Reactions were carried out at 30 °C and 20 nM GSK-3β. Final concentrations of the components of the coupled enzyme system were 2.5 mM phosphoenolpyruvate, 300 µM NADH, 30 µg/ml pyruvate kinase and 10 pg/ml lactate dehydrogenase.

An assay stock buffer solution was prepared containing all of the reagents listed above with the exception of ATP and the test compound of interest. The assay stock buffer solution (175 µl) was incubated in a 96 well plate with 5 µl of the test compound of interest at final concentrations spanning 0.002 µM to 30 µM at 30 °C for 10 min. Typically, a 12 point titration was conducted by preparing serial dilutions (from 10 mM compound stocks) with DMSO of the test compounds in daughter plates. The reaction was initiated by the addition of 20 µl of ATP (final concentration 20 µM). Reaction rates were obtained using a Molecular Devices Spectramax plate reader (Sunnyvale, CA) over 10 min at 30 °C. The Kᵢ values were determined from the rate data as a function of inhibitor concentration.

The GSK-3 inhibitory activity of certain compounds of this invention are shown in Table 11. For GSK-3 Kᵢ values, "+++" represents ≤ 0.1 µM, "++" represents between 0.1 and 10 µM, and "+" represents ≥ 10 µM.

**TABLE 11 Inhibitory Activity**

| Compound No. | Kᵢ |
|---|---|
| Ref. 1 | +++ |
| 2 | + |
| 3 | ++ |
| 4 | + |
| 5 | +++ |
| 6 | +++ |
| 7 | + |
| 8 | +++ |
| 9 | ++ |
| 10 | ++ |
| 11 | +++ |
| 12 | ++ |
| 13 | ++ |
| 14 | +++ |
| 15 | +++ |
| 16 | ++ |
| Ref. 32 | ++ |

### Reference Example 19

### Expression and Purification of GSK-3β

Full length human GSK-3β (1-420) (SEQ ID NO:1) with an N-terminal hexa-histidine tag and a thrombin cleavage site was overexpressed in a baculo virus expression system. GSK-3β was purified using Talon metal affinity chromatography (Clontech, Palo Alto, CA) followed by size-exclusion on a Superdex 200 column (Pharmacia, Uppsala, Sweden). The hexa-histidine tag was then removed by incubation with thrombin (Calbiochem, La Jolla, CA). In addition to the authentic thrombin site, a second cleavage product was identified 10 amino acids downstream at Threonine 7. Incubation overnight at 4°C with 12 U mg⁻¹ thrombin produced more than 90% GSK-3β (7-420), which was used for crystallographic studies. The reaction was quenched with PMSF and thrombin was removed with benzamidine sepharose (Pharmacia, Uppsala, Sweden). To separate unphosphorylated GSK-3β (7-420) from the phosphorylated species and GSK-3β cleaved at the authentic thrombin cleavage site, the protein was applied to a MonoS 10/10 column (Pharmacia, Uppsala, Sweden) equilibrated in 25 mM HEPES, pH 7.2, 10% Glycerol (v/v), 2 mM DTT. The protein was eluted with a linear gradient from 0 to 300 mM NaCl in 30 column volumes. Unphosphorylated GSK-3β (7-420) eluted at 150 mM NaCl. Phosphorylated GSK-3β (7-420) eluted at around 200 mM NaCl The protein was dialyzed against 25 mM Tris pH 8.0 containing 200 mM NaCl and 2 mM DTT at 4 °C, concentrated to 15 mg ml⁻¹. and centrifuged at 100,000 x *g* prior to crystallization. All protein molecular weights were confirmed by electrospray mass spectrometry. Phosphorylation on Tyr 216 was confirmed by tryptic digest and MALDI-TOF spectrometry.

### Reference Example 20

### Crystallization of GSK-3β

Crystallization of GSK-3β was carried out using the hanging drop vapor diffusion technique. The unphosphorylated GSK-3β formed diamond shape crystals over a reservoir containing 15% PEG 3350, 50 mM Na/KP04 pH 4.1, 10 mM DTT. The crystallization droplet contained 1 µl of 15 mg ml⁻¹ protein solution and 1 µl of reservoir solution. Crystals formed in less than 1 hour and were harvested in a reservoir solution after 12 hrs.

The phosphorylated form (Tyrosine 216) of GSK-3β formed plate-like crystals over a reservoir containing Solution A (7-10% PEG 3350, 100 mM Tris HCl pH 7.5, 5% Dimethylsulphoxide(DMSO)). The component DMSO was important for the crystallaization of phosphorylated GSK-3β. The crystallization droplet contained 1 µl of protein (16 mg/mL) and 1 µl of reservoir solution. The crystals formed overnight and were harvested in Solution A after a few days.

In order to obtain crystals of the ADP-peptide-GSK-3β complex, 0.3 mM protein was mixed with 1.4 mM glycogen synthase peptide (residues 650 to 661), 2 mM ADP and 2 mM MgCl. The mixture was incubated on ice for two hours. Small rod shaped crystals of the complex formed over a reservoir containing 10-15% PEG 3350 and 50 mM ammonium fluoride. Crystals large enough for data collection were obtained after repeated cycles of micro seeding.

Once the above crystals were harvested in reservoir solution, they were transferred to reservoir solutions containing increasing concentrations of glycerol, starting with 2% and increasing to 5, 10, 15, 20, 25 and 30%. After soaking the crystals in 30% glycerol for less than 5 minutes, the crystals were scooped up with a cryo-loop, frozen in liquid nitrogen and stored for data collection.

### Reference Example 21

### Formation of GSK-3β-inhibitor Complex Crystals

Phosphorylated GSK-3β-inhibitor1 complex crystals were formed by soaking phosphorylated GSK-3β crystals in Solution A that also contained 500 µM of ref. inhibitor 4,5-Diphenyl-1H-pyrazolo[3,4-c]pyridazin-3-ylamine.

Crystals of unphosphorylated GSK-3β-inhibitor2-4 complexes were formed by co-crystallizing the protein with the inhibitors. The inhibitor was added to the concentrated GSK-3β protein solution right before setting up the crystallization drop. Alternatively, inhibitor could be added to a diluted protein solution, and the mixture concentrated to the required concentration. The unphosphorylated GSK-3β protein co-crystallized with inhibitors(5-Methyl-2H-pyrazol-3-yl)-(2-pyridin-4-yl-quinazolin-4-yl)-amine, 4-(5-Methyl-2-phenylamino-pyrimidin-4-yl)-1H-pyrrole-2-carboxylic acid (2-hydroxy-1-phenyl-ethyl)-amide), and (1H-Indazol-3-yl)-[2-(2-trifluoromethyl-phenyl)-quinazolin-4-yl]-amine) over a reservoir solution containing 15-20% PEG 3350, 0.1-1 M of KF, potassium formate or ammonium formate. The crystallization droplet contained 1 µl of 10-20 mg/ml protein solution containing the inhibitor and 1 µl of reservoir solution.

Once the crystals of both forms were harvested, they were transferred to reservoir solutions containing increasing concentrations of glycerol, starting with 5% and increasing to 10, 15, 20, 25 and 30%. After soaking the crystals in 30% glycerol for less than 5 minutes, the crystals were scooped up with a cryo-loop, frozen in liquid nitrogen and stored for data collection.

### Reference Example 22

### X-Ray Data Collection and Structure Determination

X-ray diffraction data for the unphosphorylated GSK-3β, phosphorylated GSK-3β, phosphorylated GSK-3β-inhibitor complex, unphosphorylated GSK-3β-inhibitor complexes, phosphorylated GS-K-3β-ADP-peptide complex structures were collected on a Raxis 4 image plate, with mirror-focused CuKα X-rays generated by a rotating-anode source. X-ray data used to refine the unphosphorylated GSK-3β structure was collected at beam line 5.0.2 of the Advanced Light Source Lawrence Berkeley Laboratory, Berkeley, California. Data collected on the Raxis 4 image plate was processed with DENZO and SCALEPACK (Otwinowski et al., Methods Enzymol., 180, 51-.62 (1989)) Data collected at ALS were processed with the program MOSFLM and the data was scaled using SCALA (Collaborative Computational Project, N., Acta Cryst., D50, pp. 760-763 (1994)).

The data statistics of the unphosphorylated form are summarized in Table 12. The spacegroup of the unphosphorylated crystals was P2₁2₁2₁, with unit cell dimensions a= 83 b= 86 c= 178 Å, α = β = γ = 90°. The starting phases for unphosphorylated GSK-3β were obtained by molecular replacement using coordinates of CDK2 (Protein Data Bank Accession number 1AQ1) (Lawrie, A.M., et al., Nat. Struct. Biol., 4, pp. 796-801 (1997)) as a search model in the program AMORE (Navaza, J. Acta. Cryst., 50, pp. 157-163 (1994)). The asymmetric unit contained two molecules. Multiple rounds of rebuilding with QUANTA and refinement with CNX resulted in a final model that included amino acid residues 25 to 384 for molecule A and residues 37 to 382 for molecule B, 4 phosphate ions and 46 water molecules. The α carbons in A and B chains have a root-mean-squared deviation after superposition of 0.48 Å. The refined model has a crystallographic R-factor of 23.7% and R-free of 27.4%. The coordinates of the structure have been deposited with the Protein Databank (accession code 1I09).

The data statistics of the phosphorylated form are summarized in Table 13. The spacegroup of the crystals was P1, with unit cell dimensions a= 64 Å b=67.2 Å c= 67.4 Å α= 100.1° β= 103.5° γ= 90° or a= 64 Å b=67 Å c= 67 Å α= 80° β= 77° γ= 89.8°. The dimensions of the unit cell varied 1-2% from crystal to crystal. The starting phases for phosphorylated GSK-3β were obtained by molecular replacement using coordinates of the unphosphorylated form as a search model in the program AMORE ENRfu, *supra*. The asymmetric unit contained two molecules. Multiple rounds of rebuilding with QUANTA and refinement with CNX resulted in a final model that included residues 37 to 383 for molecule A and residues 37 to 383 for molecule B and 83 water molecules. All data was included and NCS restraint was applied through out the refinement. The final step of refinement was an individual B-factor refinement. The refined model has a crystallographic R-factor of 23.8% and R-free of 27.6%.

The spacegroup of the unphosphorylated GSK-3β-inhibitor2-4 complex crystals was P2₁2₁2₁, with unit cell dimensions a= 83 b= 86 c= 178 Å, α = β = γ = 90°. The starting phases for unphosphorylated GSK-3β-inhibitor complexes were obtained by molecular replacement using coordinates of the unphosphorylated form as a search model in the program AMORE ENRfu, *supra*. The asymmetric unit contained two molecules. Multiple rounds of rebuilding with QUANTA and refinement with CNX were performed.

The data statistics of the unphosphorylated GSK-3β-inhibitor2 complex are summarized in Table 14. The structure was refined to 2.9 Å, and the R-factor was 24.1% and R-free was 28%. The final model included amino acid residues 25 to 385 for molecule A, residues 37 to 382 for molecule B, inhibitor2 and 6 water molecules.

For the unphosphorylated GSK-3β-inhibitor3 complex, the structure was refined to 2.3 Å, and the R-factor was 25.3% and R-free was 28.6%. For molecule A, residues 25 to 381 were included in the final model. For molecule B, amino acid residues 37 to 382 were included in the final model.

For the unphosphorylated GSK-3β-inhibitor4 complex, the structure was refined to 2.8 Å, and the R-factor was 24.1% and R-free was 28.3%. For molecule A, residues 36 to 381 were included in the final model. For molecule B, amino acid residues 37 to 382 were included in the final model.

The data statistics of the phosphorylated GSK-3β-inhibitor1 complex are summarized in Table 15. The spacegroup of the crystals was P1, with unit cell dimensions a= 64 Å b=67 Å c= 67 Å α= 100° β= 103° γ= 89.8° or a= 64 Å b=67 Å c= 67 Å α= 80° β= 77° γ= 89.8°. The starting phases for phosphorylated GSK-3β-inhibitor complex were obtained by molecular replacement using coordinates of the phosphorylated form as a search model in the program AMORE ENRfu, supra. The asymmetric unit contained two molecules. Multiple rounds of rebuilding with QUANTA and refinement with CNX resulted in a final model that included residues 37 to 383 for molecule A and residues 37 to 384 for molecule B, the inihibitors bound to molecule A and B, and 83 water molecules. The structure was refined to 2.8 Å, and had an R-factor of 22.6% and R-free of 26.9%.

The data statistics of the phosphorylated GSK-3β-ADP-peptide complex are summarized in Table 16. The spacegroup of the crystals was P2₁2₁2₁, with unit cell dimensions a= 75.16 Å b= 107.93 Å c= 121.2 Å α= 90° β= 90° γ= 90°. The starting phases for phosphorylated GSK-3β were obtained by molecular replacement using coordinates of the unphosphorylated form as a search model in the program AMORE ENRfu, *supra.* The asymmetric unit contained two molecules. The glycine rich loop was well ordered and could be built in the model. Multiple rounds of rebuilding with QUANTA and refinement with CNX was performed. All data was included and NCS restraint was applied through out the refinement. The final step of refinement was a grouped B-factor refinement. The refined model has a crystallographic R-factor of 23.5% and R-free of 27.2%.

The Ramachandran plot of phosphorylated GSK-3β in complex with ADP and glycogen synthase peptide showed that 80.1% of the residues were in most favored regions and 19.6% in additionally allowed regions. The Ramachandran plot of apo-phosphorylated GSK-3β showed that 85.3% of the amino acid residues were in most favored regions and 14.3% in additionally allowed regions. Two amino acid residues (Cys 218 in molecule A and B) in both crystal structures were in disallowed regions.

In the above models, disordered residues were not included in the model. Alanine or glycine residues were used in the model if the side chains of certain residues could not be located in the electron density.

### Reference Example 23

### Overall Structure of Unphosphorylated GSK-3β

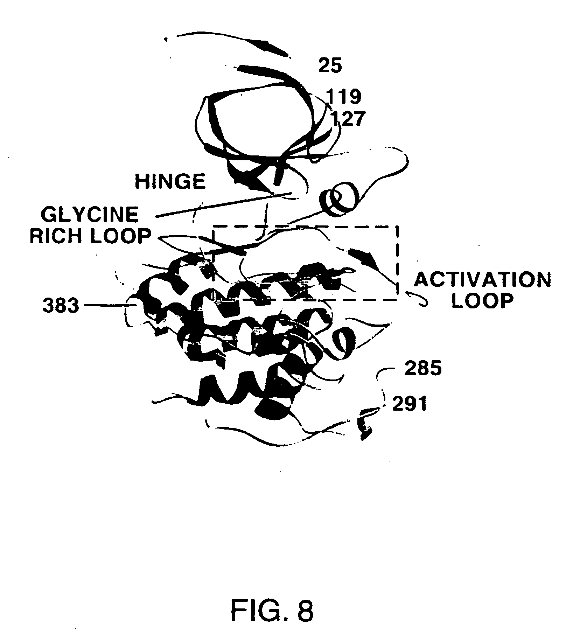

GSK-3β has the typical 2 domain kinase fold (Hanks, S.K., et al., Science, 241, pp. 42-52 (1988) ; Hanks, S.K. and A.M. Quinn, Methods Enzymol., 200, pp. 38-62 (1991)) with a β-strand domain (amino acid residues 25-138) at the N-terminal end and an α-helical domain at the C-terminal end (amino acid residues 139-343) (Fig. 8). The active site is at the interface of the α-helical and β-strand domain, and is bordered by the glycine rich loop and the hinge. The activation loop runs along the surface of the substrate-binding groove. The C-terminal 39 amino acid residues (amino acid residues 344-382) are outside the core kinase fold and form a small domain that packs against the α-helical domain. The β-strand domain consists of seven anti-parallel β-strands. Strands 2 to 6 form a β-barrel that is interrupted between strand 4 and 5 by a short helix (amino acid residues 96-102) which packs against the β-barrel. This helix is conserved in all kinases, and two of its residues play key roles in the catalytic activity of the enzyme. Arginine 96 is involved in the alignment of the two domains. Glutamate 97 is positioned in the active site and forms a salt bridge with lysine 85, a key residue in catalysis.

### Phosphorylation of Peptides by GSK-3β

Before a serine/threonine kinase can phosphorylate a substrate, its β-strand and α-helical domains must be aligned into a catalytically active conformation. Most kinases use one or two phosphorylated amino acid residues on the activation loop for this purpose. Polar amino acid residues, typically arginines and lysines, from the β-strand and α-helical domains, bind the phosphate group of the phosphorylated amino acid residue on the activation loop, which leads to proper alignment of the two domains. The second phosphorylated amino acid residue (if present, for example Tyr216 in GSK-3β) opens the substrate-binding groove and allows the substrate to bind.

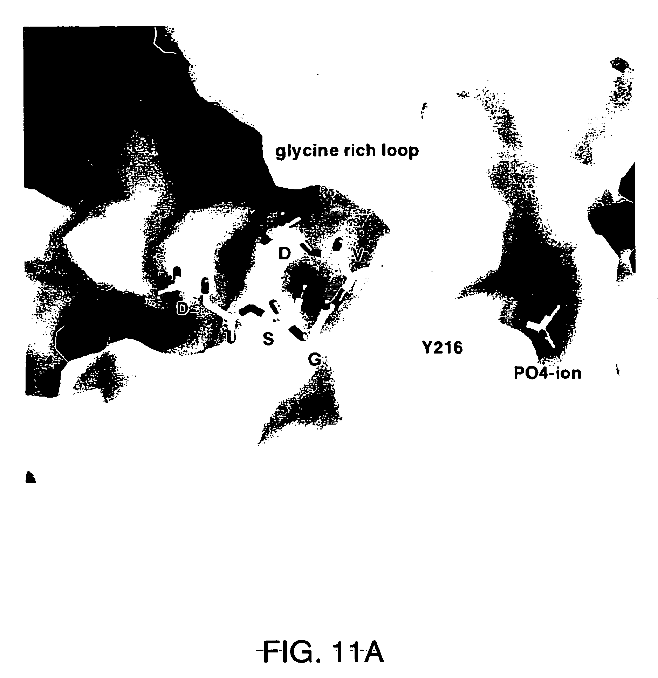

Comparison of the GSK-3β with other kinases such as CDK2, p38γ and ERK2 revealed that the structure of apo-GSK-3β resembles closely the substrate-bound, activated form of a kinase. The activation loop (amino acid residues 200 to 226) in the GSK-3β structure is well ordered, and is positioned against the α-helical domain. This orientation opens the peptide substrate binding groove (Fig. 11), and mimics the position of the activation loop of the activated substrate bound CDK2 (Fig. 10) but not apo-CDK2 (Protein Data Bank Accession number 1HCL) (Schulze-Gahmen, U., et al., Proteins, 22, pp. 378-91 (1995)). Comparison of the activation loops of GSK-3β, P38γ (Protein Data Bank Accession number 1CM8) (Bellon, S., et al., Structure Fold Des, 7, pp. 1057-65 (1999)), substrate-bound activated CDK2 (Protein Data Bank Acession number 1QMZ) (Brown, N.R., et al., Nat. Cell. Biol., 1, pp. 438-443 (1999)), and ERK2 (Protein Data Bank Accession number 2ERK) (Canagarajah, B.J., et al., Cell, 90, pp. 859-69 (1997)) shows that the backbone and side chain atoms of important residues align (Fig. 10)., R96; R180 and K205 of GSK-3β (Fig. 10A) superimpose well with R73, R152 and R176 of phosphorylated P38γ, respectively (Fig. 10C). These amino acid residues also superimpose well with the corresponding residues in phosphorylated ERK2 and substrate bound activated CDK2. In p38γ, CDK2 and ERK2, these residues point to the phosphate group from the phosphorylated threonine on the activation loop, the residue that is important for aligning the N-terminal and C-terminal domains. In the GSK-3β structure, R96, R180 and K205 point to a PO₄⁻ ion that is located in the same position as the phosphate group of the phosphorylated threonine in CDK2, ERK2 and p38γ.

The superposition of the GSK-3β and p38γ activation loops shows that the GSK-3β phosphorylation site, Y216, is located in a similar position as the phospho-tyrosine (amino acid residue 185) of p38γ. The phospho-tyrosine of p38γ acts as a gatekeeper for the substrate-binding groove. When it is phosphorylated, its side chain moves out of the groove allowing substrate peptides to bind. The side chain of Y216 of GSK-3β is also positioned to block access to the substrate-binding groove.

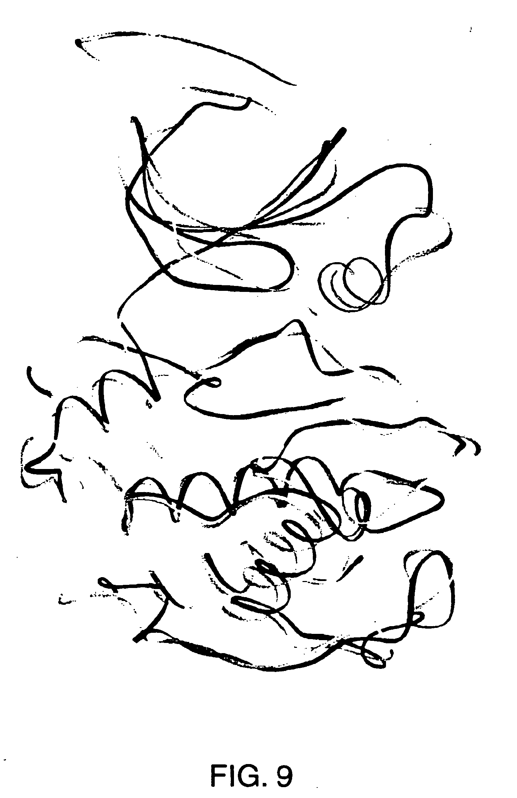

The sequence of GSK-3β is 25% identical and 41% similar to the sequence of CDK2. The structure of GSK-3β presented here superimposes well with the structure of activated, substrate-bound CDK2 (Fig. 9). Because of the similarity in sequence and fold, we can use the structure of activated, substrate-bound CDK2 as a model for the substrate binding of GSK-3β.

### Primed Phosphorylation

The GSK-3β substrate-binding groove is partially occupied by a loop from a neighboring GSK-3β molecule in the crystal (Fig. 11A). The loop is positioned in front of the active site. Superposition of the α-helical domains of activated substrate-bound CDK2 and GSK-3β shows that four residues in the loop, DSGV (amino acid residues 260 to 263), are in a very similar position as the peptide in activated substrate-bound CDK2. S261 of the loop in GSK-3β occupies the same position as the target serine in peptide-bound CDK2 (compare position S261 in Fig. 11A with S* in Fig. 11B). The CDK2-bound peptide has an extended conformation, while loop 260-264 in the GSK-3β adopts a turn and occupies a small portion of the substrate-binding groove. It is likely that the natural substrate for GSK-3β also has an extended conformation, similar to the peptide bound to CDK2. If we use the CDK2-bound peptide as a model for a GSK-3β substrate, it becomes clear why GSK-3β prefers a phosphorylated serine or threonine at the P+4 position. The phosphate group at the P+4 position will occupy the same position as the phosphate ion near the activation loop of our structure, contacting R96, R180 and K205 (Fig. 11). This means that while CDK2, p38γ and ERK2 use a phospho-threonine on the activation loop to align the β-strand and α-helical domains, GSK-3β uses the phosphorylated serine at the P+4 position of the substrate to align the two domains for optimal catalytic activity.

### Reference Example 24

### Active Site of GSK-3β-inhibitor Complexes

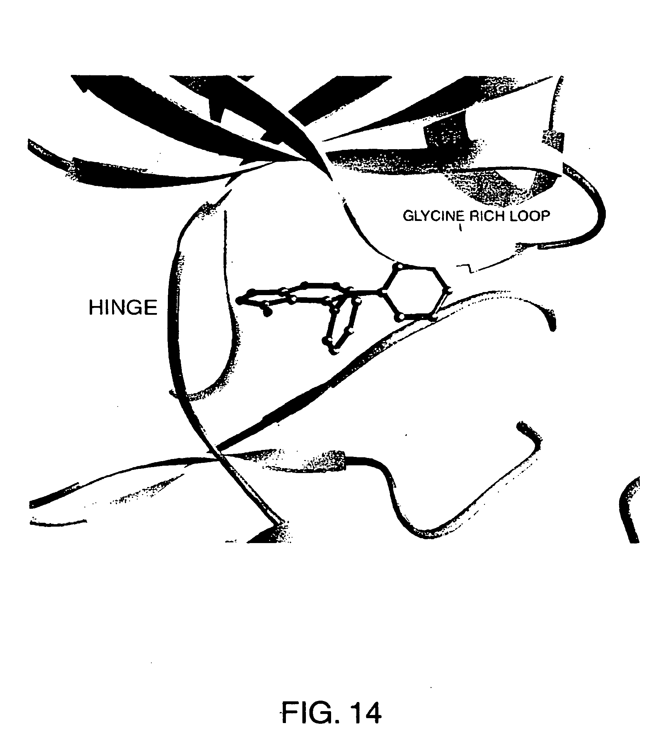

Ref. Inhibitor1 4,5-Diphenyl-1H-pyrazolo[3,4-clpyridazin-3-ylamine is bound in the deep cleft of the active site in the phosphorylated GSK-3β structure (Figure 14). Ref Inhibitor1 forms two hydrogen bonds with the hinge portion of the active site. The 1H pyrazole nitrogen shares a proton with the D133 backbone carbonyl. The other pyrazole nitrogen (position 2) accepts a proton from the V135 backbone nitrogen. The side chains of L132 and K85 are positioned inside the active site. K85 is a catalytically important residue and forms a salt bridge with E97 (not shown).

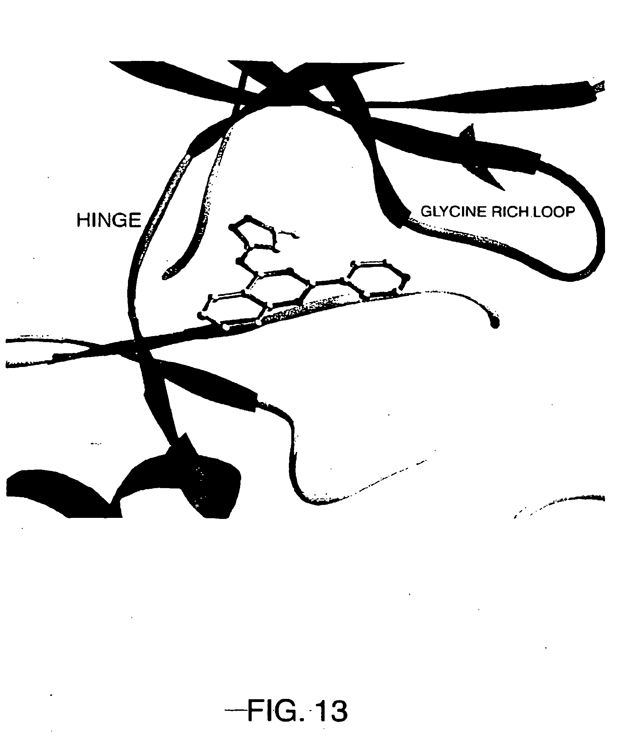

Inhibitor2 (5-Methyl-2*H*-pyrazol-3-yl)-(2-pyridin-4-yl-quinazolin-4-yl)-amine is bound in the active site in the unphosphorylated GSK-3β structure (Figure 13). The inhibitor2 forms four H-bonds with the hinge backbone. Two hydrogen bonds come from the pyrazole ring. The nitrogen in position one donates a hydrogen to the backbone carbonyl of Asp 133. The nitrogen in position 2 accepts a hydrogen from the Val 135 amide nitrogen. The backbone carbonyl of Val 135 is within hydrogen bonding range of hydrogen donating groups on the inhibitor2. It contacts the linker nitrogen and the quinazoline carbon at position 8.

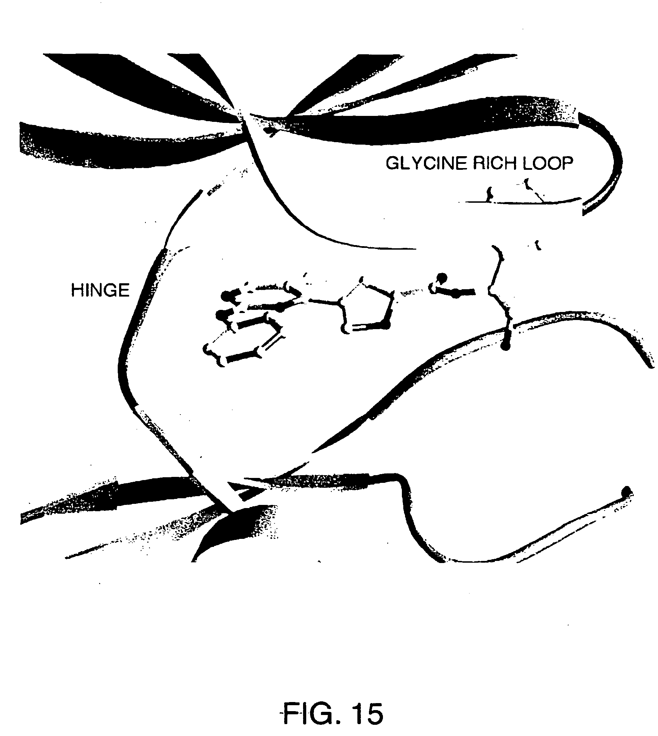

Inhibitor3 4-(5-Methyl-2-phenylamino-pyrimidin-4-yl)-1H-pyrrole-2-carboxylic acid (2-hydroxy-1-phenylethyl)-amide) is bound in the active site in the unphosphorylated GSK-3β structure (Figure 15). Inhibitor3 is a potent inhibitor of GSK-3β with a Ki of 4 nM. Inhibitor3 forms 5 hydrogen bonds with the GSK-3β protein. The three amino-pyrimidine hydrogen bonds contact the hinge backbone. The carbonyl forms a hydrogen bond with the side chain of the catalytic lysine (K85) and the hydroxyl forms a hydrogen bond with the side chain of asparagine 186. Asn186 is a conserved residue in GSK-3β and ERK2. The 5-methyl group of the amino-pyrimidine ring points toward L132 and V110 in GSK-3β. There is limited space between these two residues suggesting that a small substituent is allowed in this position.

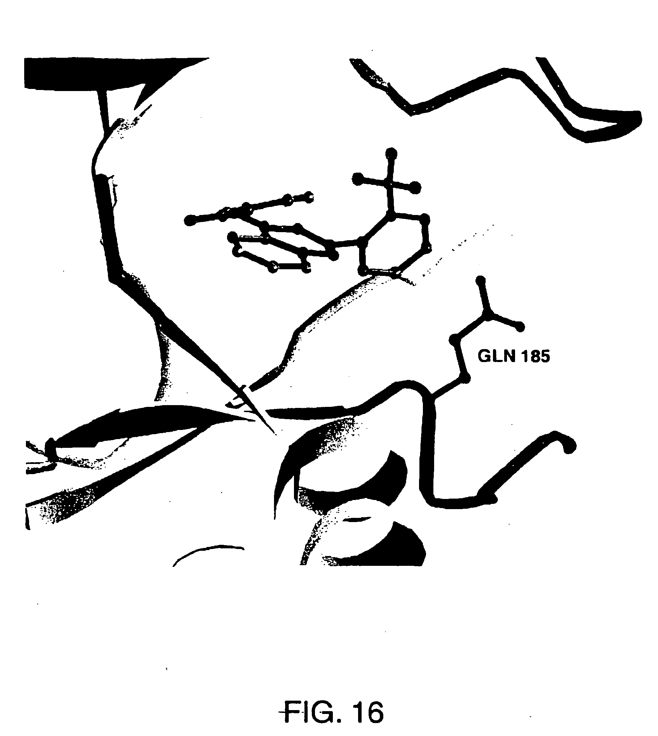

Inhibitor4 (1*H*-Indazol-3-yl)-[2-(2-trifluoromethyl-phenyl)-quinazolin-4-yl]-amine) is bound in the active site in the unphosphorylated GSK-3β structure (Figure 16). The indazole ring of inhibitor4 forms two hydrogen bonds with the hinge backbone. The nitrogen in position one donates a hydrogen to the backbone carbonyl of Asp 133. The nitrogen in position 2 accepts a hydrogen from the Val 135 amide nitrogen. The backbone carbonyl of Val 135 is within hydrogen bonding range of hydrogen donating groups from inhibitor4. It contacts the linker nitrogen and the quinazoline carbon at position 8. The trifluoromethyl phenyl ring is almost perpendicular to the quinazoline ring with the ortho trifluoromethyl substituent pointing to the glycine rich loop. The side chain of glutamine 185 packs against the trifluoromethyl phenyl ring and points to the glycine rich loop.

### Reference Example 25

### Overall Structure of apo-Phosphorylated GSK-3β and Substrate-bound Phosphorylated GSK-3β

The apo-phosphorylated GSK-3β and substrate-bound phosphorylated GSK-3β structures have the typical two-domain kinase fold (residues 37 to 343) (Hanks, S.K. and Quinn, A.M.; Methods Enzymol., 200, pp. 38-62 (1991); Hanks, S.K. and Hunter, T., FASEB J., 9, pp. 576-96 (1995)). The N-terminal β-strand domain (amino acid residues 37 to 138) forms a β-barrel consisting of seven β-strands (Figure 17). The C-terminal α-helical domain contains amino acid residues 139 to 343. The C-terminal 40 amino acid residues (residues 344 to 383) are not part of the kinase fold, but pack against the α-helical domain.

The active site and the substrate-binding groove are located at the interface of the β-strand and α-helical domain. The active site is bordered by the hinge and the glycine-rich loop and contains an ADP-molecule. The substrate-binding groove contains a 12 amino acid residue phosphorylated peptide derived from the sequence in glycogen synthase recognized by GSK-3β (650 HSSPHQpSEDEEE 661). The peptide is positioned between the activation loop (amino acid residues 200 to 226) and the β-strand domain (Figure 17).

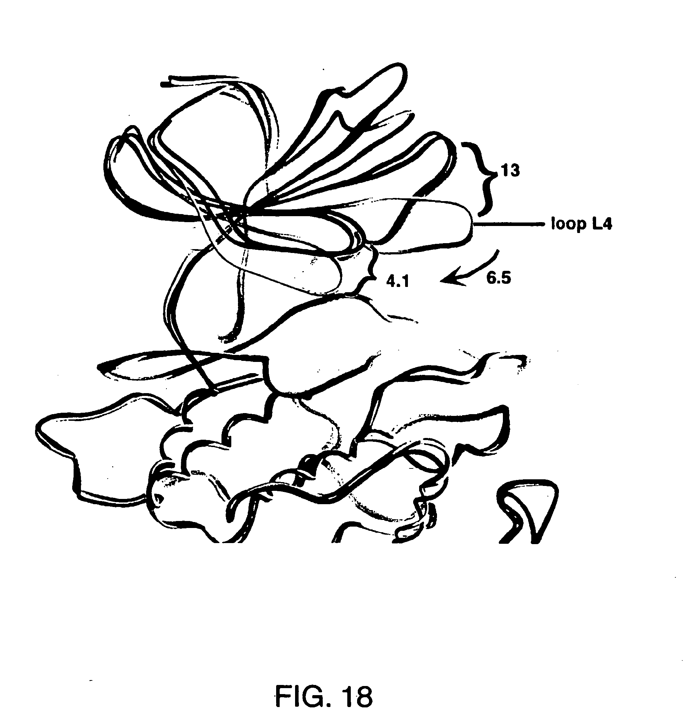

Comparison between the structures of unphosphorylated, phosphorylated apo-GSK-3β and phosphorylated peptide-bound GSK-3β reveal local differences induced by the presence of the substrates (Figure 18). Superposition of the protein backbone of unphosphorylated and phosphorylated apo-GSK-3β resulted in a mean displacement of 0.7 Å and a maximum displacement of 3.7 Å of the Ile 217 backbone carbonyl. In the phosphorylated peptide-bound GSK-3β structure, the phosphorylated side chain of Tyr 216 rotated out of the substrate-binding groove and induced a 180° flip of the Ile 217 backbone carbonyl. As a consequence of this adjustment, the torsion angles of Cys 218 appeared in disallowed regions of the Ramachandran plot. In the phosphorylated apo-GSK-3β structure, the side chain of Y216 also flips out of the substrate binding groove (Fig. 10). The N-terminal domain of the phosphorylated peptide-bound GSK-3β rotated by 6.5°, with the glycine rich loop covering the ADP molecule. The reorganization of the glycine rich loop resulted in 4.1 Å translation of the Ser 66 α-carbon. The loop connecting β-3 to α-C (L4, amino acid residues 87 to 95) migrated 13 Å (amino acid residue 92) towards the substrate-binding groove, and form contacts with the backbone atoms of the glycogen synthase peptide. The αG helix (amino acid residues 262 to 273) rotated towards phosphorylated Tyr 216. The rotation of the β-strand domain and the adjustments of the glycine rich loop and L4 were induced by the presence of ADP and the substrate peptide since the β-strand domain of apo-phosphorylated GSK-3β were not rotated in comparison to unphosphorylated GSK-3β.

The loop connecting α1L14 with Trp 301 (amino acid residues 284 to 300) was poorly ordered in the apo-and ADP, peptide-bound structures. This loop is part of the Frat1 binding site (Bax, B. et al., Structure(Camb), 9, pp. 1143-52 (2001)) and covers a hydrophobic groove between αG and amino acid residues 288-294. The loop is separate from the substrate-binding groove and does not seem to influence peptide phosphorylation even when Frat1 is bound to GSK-3β (Thomas, G.M. et al., FEBS Lett., 458, pp. 247-51 (1999)).

### Reference Example 26

### The Active site of the GSK-3β-ADP-peptide Complex

The active site is located at the interface of the β-strand and α-helical domain and is enclosed by the glycine rich loop and the hinge. The glycine rich loop is formed by two anti-parallel β-strands. The conformation of the glycine rich loop adjusts to the ligand that occupies the active site. The absence of the γ-phosphate in the ADP molecule allows the glycine rich loop to descend closer to the α-helical domain. A similar adjustment of the glycine rich loop was observed in the PKA-ADP complex (Protein Data Bank Accession number 1JBP) (Madhusudan, Trafny, E.A. et al., Protein Sci, 3, pp. 176-87 (1994)).

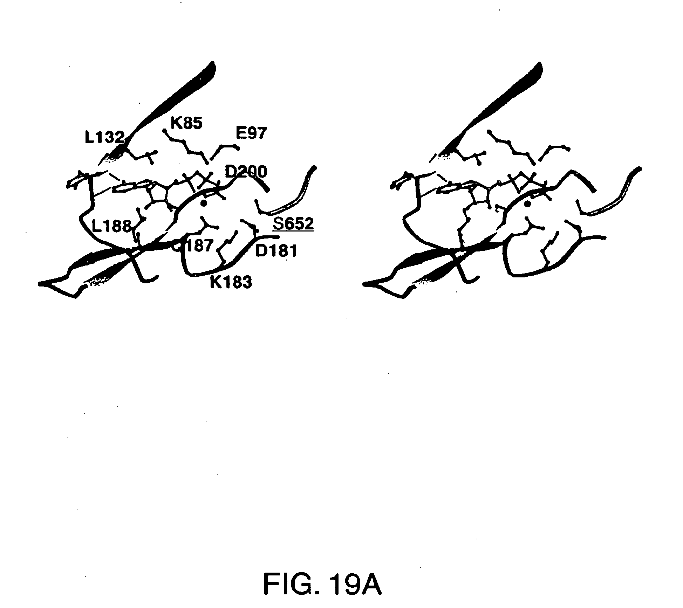

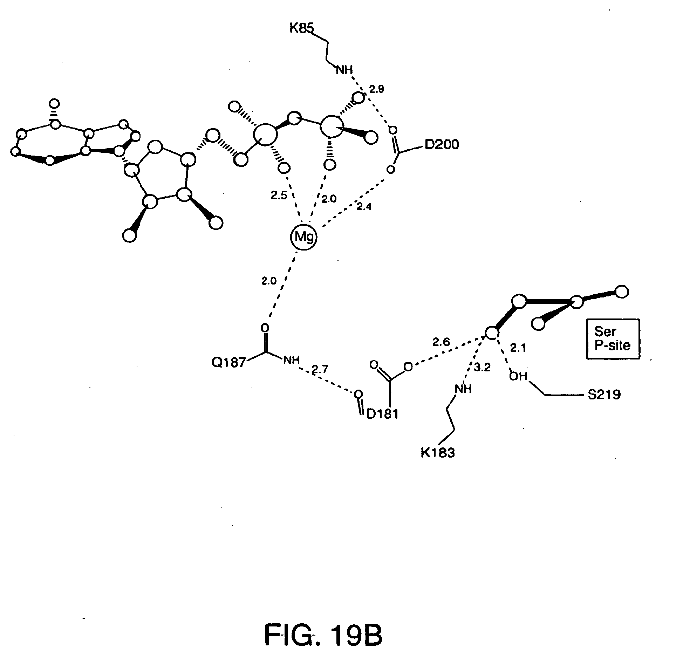

The entire β-strand domain rotated 6.5° as a result of the ADP molecule being bound in the active site. The adenine moiety of ADP is surrounded by hydrophobic amino acid residues from the glycine rich loop (Ile 62, Val 70), from the hinge (Tyr 134) and from the bottom of the active site (Leu 188, Cys 199) (Figure 19A). Two hydrogen bonds between the base and the hinge backbone were observed. The amino group on position 6 forms a hydrogen bond with the backbone carbonyl of Asp 133, and the N1 nitrogen accepts a hydrogen from Val135 amide nitrogen. The 3' hydroxyl from the ADP ribose donates a hydrogen to the backbone carbonyl of Gln 185. The active site residues that play a role in the serine phosphorylation reaction form a web of hydrogen bonds around the ADP phosphates and the glycogen synthase target serine (Figure 19B). The two ADP phosphates form a complex with one Mg ion, which in turn contacts the side chains of Asp 200 and Gln 185. Asp 200 is part of the Asp Phe Gly motif and is the first amino acid residue of the activation loop. The Asp 200 homologue in other kinase-AMPPNP complexes binds a second Mg ion (or Mn ion) that is positioned between the β- and γ- phosphate (Bellon, S. et al., Structure Fold Des., 7, pp. 1057-65 (1999); Xie, X. et al., Structure, 6, pp. 983-91 (1998); Bossemeyer, D. et al., EMBO J, 12, pp. 849-59 (1993)). However, a second Mg ion could not be located in our electron density maps. On the other hand, the structure of the PKA-ADP complex (Protein Data Bank Accession number 1JBP) (Madhusudan, Trafny, E.A. et al., Protein Sci, 3, pp. 176-87 (1994)) does not have any Mg ion associated with the phosphate groups.

Lys 85 is positioned between Glu97 and the α-and β- phosphates and likely facilitates the transfer of the γ-phosphate from ATP to the substrate serine. Asp 181 is also a conserved residue in kinases and its side chain is within hydrogen bonding range of the peptide target serine (Ser 652). The backbone carbonyl of Asp 181 forms a hydrogen bond with the Gln 185 side chain. Asp 181 prepares the serine hydroxyl for the nucleophilic attack on the γ- phosphate (Adams, J.A., Chem. Rev., 101, pp. 2271-2290 (2001)). Lys 183 makes a hydrogen bond with the peptide target serine. Lys 183 is positioned between the phosphate groups and the target serine and is involved in the phosphate transfer. When the γ-phosphate is present in the kinase active site, the Lys 183 is likely to contact the terminal phosphate group.

### Reference Example 27

### The Substrate-binding Groove of the GSK-3β-ADP-peptide Complex

GSK-3β has one phosphorylation site in the activation loop, Y216, which acts as a gate for the substrate-binding groove. In the crystal structure of unphosphorylated GSK-3β, the unphosphorylated tyrosyl side chain occupied the substrate-binding groove. A comparison between the unphosphorylated and phosphorylated peptide-bound GSK-3β structures showed that there would be space to accommodate the glycogen synthase peptide even if the unphosphorylated tyrosine remains in the substrate-binding groove. The P+2 residue of the peptide (His 654) is the closest amino acid residue to Tyr 216. Its imidazole ring is part of a cluster of aromatic residues formed by GSK-3β residues Phe 67, Phe 93 and the peptide substrate amino acid residue His 650.

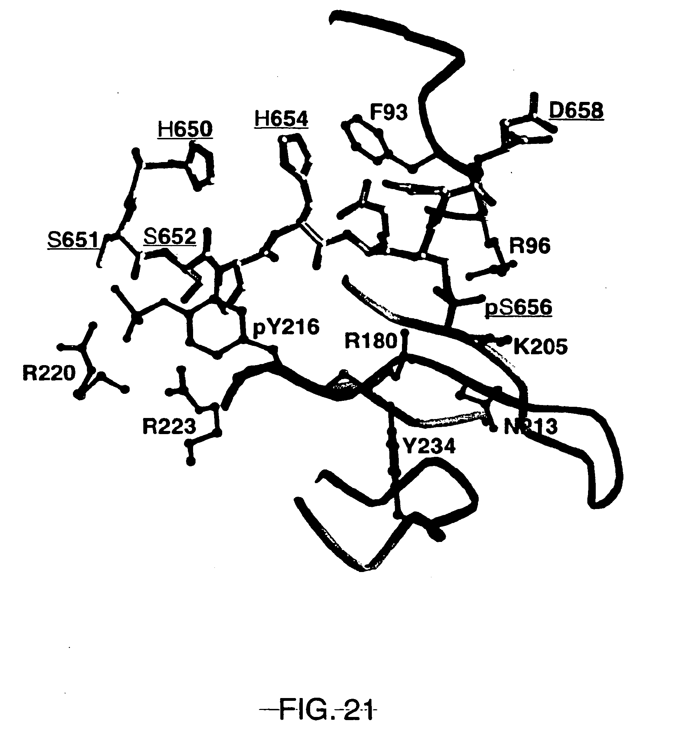

The phosphorylated Tyr216 side chain has moved out of the substrate-binding groove in the GSK-3β-ADP-peptide complex structure (Figure 21). The phosphophenol group of Tyr 216 is bound to the side chains of Arg 220 and Arg 223, which results in neutralization of the negative charge of the phosphate group and a 180° flip of the Ile 217 backbone carbonyl. The phospho-phenol group also caused a slight adjustment of the αG helix (residues 262-272), which results in the distance between the phosphate oxygen atoms and amide nitrogen of G262 (which is the closest H-bond donor) outside the H-bonding range (4.4 Å).

Glycogen synthase is a major substrate of GSK-3β with multiple phosphorylation sites. The canonical phosphorylation site for GSK-3β is SXXXpS. The P+4 serine (or threonine) is first phosphorylated by a different kinase, which is called primed phosphorylation. In the case of glycogen synthase, GSK-3β phosphorylates four sites sequentially after it is phosphorylated by casein kinase-II. The co-crystallized peptide present in the substrate-binding groove is derived from glycogen synthase. The sequence (650-HSSPHQ(pS)EDEEE-661) contains the canonical GSK-3β phosphorylation motif and GSK-3β can phosphorylate Ser 652 under the appropriate conditions. Ten of the twelve residues (residues 650 to 659) had visible electron density and were built in the substrate-binding groove (Figure 21). The peptide has the shape of a large loop with residues 651 to 656 fitting in the substrate binding groove and residues 650, 657 to 659 exposed to solvent. The structure reveals why GSK-3β prefers a phosphorylated serine or threonine at the P+4 position of the glycogen synthase phosphorylation site. The phosphate group of Ser 656 occupies a positively surface charged pocket formed by residues Arg 96, Arg 180 and Lys 205. These amino acid residues are conserved in serine/threonine kinases and are responsible for the proper alignment of the β-strand and α-helical domains, the latter of which is required for optimal catalytic activity. Other serine/threonine kinases such as CDK2 (Schulze-Gahmen, U. et al., Proteins, 22, pp. 378-91 (1995)), ERK2 (Zhang, F. et al., Nature, 367, pp. 704-11 (1994); Canagarajah, B.J. et al., Cell, 90, pp. 859-69 (1997)) and p38γ (Bellon, S. et al., Structure Fold Des., 7, pp. 1057-65 (1999)) have a phosphorylated threonine in the activation loop for this purpose, but GSK-3 uses a phosphorylated residue from the substrate. The target serine (Ser 652) is positioned in front of the active site, 6.0 Å from the β-phosphate of the ADP molecule. The loop between residues Lys 85 and Arg 96 migrates more than 10 Å to facilitate the glycogen synthase peptide binding. Phe 93 forms a pi-stack with the peptide His 654. Ser 66, which is at the tip of the glycine rich loop, makes a hydrogen bond with the target serine backbone-nitrogen (Ser 652). The backbone carbonyl of pSer 656 forms a hydrogen bond with Lys 94.

The canonical phosphorylation motif for GSK-3β is SXXXpS. There is no sequence requirement for the three residues between the target serine and the phosphoserine. The P+1 residue in the glycogen synthase peptide used here is a proline. Although prolines are not uncommon as the P+1 residue in phosphorylation motifs, the GSK-3β phosphorylation does not require a proline. A sequence analysis of the phosphorylation motifs of the proteins that undergo primed phosphorylation shows that residues such as A, G, Q, E, S, T, R and V can replace the proline at the P+1 position. In the GSK-3β-ADP-peptide structure, the proline side chain fits in a pocket formed by the side chains of pY216, R220, R223 and the backbones of residues 217 to 219. The pocket is shallow, and the distance between the Cγ of Pro 654 and Arg 223 is 3.8 Å. This pocket can only accommodate small residues such as Ala, Ser or Val. Larger residues, such as Arg and Gln will have to point their side chains into the solvent. This might explain the absence of bulky hydrophobic residues at the P+1 position. This fact is reflected in the interactions between the glycogen synthase peptide and GSK-3β. Except for the phosphoserine side chain interactions with the basic residues, the other interactions are with the peptide backbone. The pi-stack interaction between His 654 and Phe 93 is probably specific for the phosphorylation of Ser 652, because there is no aromatic residue at the P+2 position in other motifs of glycogen synthase or eIF2b.

The crystal structure of the GSK-3β-ADP-peptide complex provides a detailed picture of the phosphorylation of the glycogen synthase peptide. Although the ADP molecule does not contain the γ-phosphate, the catalytic residues are located around the ADP molecule similar to the positions in other kinase nucleotide complexes. The phosphoserine of the peptide serves as an anchor for the peptide and is required for the proper alignment of the β-strand and α-helical domains of GSK-3β.

### Biological Implications

Activation of the insulin-signaling pathway induces increased glucose uptake and conversion to glycogen. Patients with type II diabetes have decreased sensitivity towards insulin, which reduces glycogen synthesis and increased blood glucose levels. The conversion of glucose into glycogen by glycogen synthase is the rate limiting step in glycogen synthesis and the phosphorylation status of glycogen synthase determines the catalytic rate. Glycogen synthase has at least nine phosphorylation sites and the more it is phosphorylated the lower its catalytic activity. GSK-3β is one of the kinases that phosphorylates and inhibits glycogen synthase. It phosphorylates sequentially multiple sites at the C-terminal end of glycogen synthase after Casein Kinase II phosphorylates glycogen synthase. The canonical sequence recognized by GSK-3β is SXXXpS, which is four times present in glycogen synthase. GSK-3β is a potential therapeutic target for type 2 diabetes because its inhibition leads to increased glycogen synthesis and decreased blood glucose levels.

The crystal structures of apo-phosphorylated GSK-3β and GSK-3β in complex with ADP and glycogen synthase peptide provide insight on how GSK-3β phosphorylates its substrates. The ADP molecule occupies the active site and the glycogen synthase peptide occupies the substrate-binding groove. The phosphorylated serine at the P+4 position of the glycogen synthase peptide binds three well-conserved basic residues, which results in optimal alignment of the β-strand and α-helical domains of the GSK-3β kinase core. Other interactions between GSK-3β and the glycogen synthase peptide involve mostly backbone atoms of the peptide, which might explain the tolerance for different residues at position P+1, P+2 and P+3. The present invention will be helpful in understanding the role of GSK-3β in the insulin-signaling pathway and development of potential new anti-diabetic therapies.

### Reference Example 28

### The Use of GSK-3β Coordinates for Inhibitor Design

The coordinates of any one of Figures 1-7 are used to design compounds, including inhibitory compounds, that associate with GSK-3β or GSK-3β homologues. This process may be aided by using a computer comprising a machine-readable data storage medium encoded with a set of machine-executable instructions, wherein the recorded instructions are capable of displaying a three-dimensional representation of the GSK-3β, the GSK-3β homologues or portions thereof. The graphical representation is used according to the methods described herein to design compounds. Such compounds associate with the GSK-3β or GSK-3β homologue at the active site or substrate binding pocket.

While we have described a number of embodiments of this invention, it is apparent that our basic constructions may be altered to provide other embodiments which utilize the products, processes and methods of this invention. Therefore, it will be appreciated that the scope of this invention is to be defined by the appended claims, rather than by the specific embodiments which have been presented by way of example.

**TABLE 12**

| Summary of data collection | | |
|---|---|---|
| Source | R-Axis IV | ALS 5.0.2 |
| Wavelength (Å) | 1.54 | 1.1 |
| Resolution (Å) | 3.0 | 2.7 |
| No. of Reflections (measured/unique) | 303687/26039 | 251279/34993 |
| Completeness (%) (overall/outer shell) | 96.7/89.3 | 98.9/99.8 |
| R_{merge} (%) ¹ (overall/outer shell) | 0.064/0.30 | 0.070/0.32 |

| Structure refinement | | |
|---|---|---|
| Resolution (Å) | 48.3-2.7 | |
| No. of reflections | 34747 | |
| R factor | 23.7 | |
| Free R factor † | 27.4 | |
| Rms deviations | | |
| | | |
| Bond lengths | 0.01 | |
| Bond angles | 1.5° | |

| | | |
|---|---|---|
| ¹ R_{merge} = 100 × ∑ₕ∑ᵢ \|Iₕᵢ - <Iₕ> \|/∑ₕ∑ᵢIₕᵢ. ^{†} The Free R factor was calculated with 9.1% of the data. | | |

**TABLE 13**

| Summary of data collection | |
|---|---|
| Source | R-Axis IV |
| Wavelength (Å) | 1.54 |
| Resolution (Å) | 2.5 |
| No. of Reflections (measured/unique) | 32357/3447 |
| Completeness (%) (overall/outer shell) | 87.0/61.8 |
| I/δ(I) (overall/outer shell) | 10.4/2.3 |
| R_{merge}(%) shell) (overall/outer shell) | 0.064/0.30 |

| Structure refinement | |
|---|---|
| Resolution (Å) | 42.8-2.5 |
| No. of reflections | 32357 |
| R factor | 23.8 |
| Free R factor¹ | 27.6 |
| Rms deviations | |
| | |
| Bond lengths | 0.008 |
| Bond angles | 1.5° |
| Bfactor (average)² | 45 Å |

| | |
|---|---|
| ¹ The Free R factor was calculated with 10% of the data. ² The B-factor of the data (Wilson plot) was 26.4 Å². | |

**TABLE 14**

| Summary of data collection | |
|---|---|
| Source | R-Axis IV |
| Wavelength (Å) | 1.54 |
| Resolution (Å) | 2.9 |
| No. of Reflections (measured/unique) | 338559/26836 |
| Completeness (%) (overall/outer shell) | 93.1/85.1 |
| I/δ(I) (overall/outer shell) | 10.2/2.7 |
| R_{merge} (%) (overall/outer shell) | 0.072/0.29 |

| Structure refinement | |
|---|---|
| Resolution (Å) | 25.7-2.9 |
| No. of reflections | 26796 |
| R factor | 24.0 |
| Free R factor ¹ | 27.9 |
| Rms deviations | |
| Bond lengths | 0.011 |
| Bond angles | 1.8° |
| Bfactor (average)² | 39.3 Å |

| | |
|---|---|
| ¹ The Free R factor was calculated with 9.4% of the data. ² The B-factor of the data (Wilson plot) was 33 Å². | |

**TABLE 15**

| Summary of data collection | |
|---|---|
| Source | R-Axis IV |
| Wavelength (Å) | 1.54 |
| Resolution (Å) | 2.8 |
| No. of Reflections (measured/unique) | 71493/23161 |
| Completeness (%) (overall/outer shell) | 88.3/90.3 |
| I/δ(I) (overall/outer shell) | 14.7/2.2 |
| R_{merge}(%) (overall/outer shell) | 0.046/0.33 |

| Structure refinement | |
|---|---|
| Resolution (Å) | 32.2-2.8 |
| No. of reflections | 23148 |
| R factor | 22.5 |
| Free R factor ¹ | 26.8 |
| Rms deviations | |
| | |
| Bond lengths | 0.013 |
| Bond angles | 1.6° |
| Bfactor (average)² | 51.1 Å |

| | |
|---|---|
| ¹ The Free R factor was calculated with 9.4% of the data. ² The B-factor of the data (Wilson plot) was 91.3 Å². | |

**TABLE 16**

| Summary of data collection | |
|---|---|
| Source | R-Axis IV |
| Wavelength (Å) | 1.54 |
| Resolution (Å) | 2.8 |
| No. of Reflections (measured/unique) | 311458/24709 |
| Completeness (%) (overall/outer shell) | 99.4/99.8 |
| I/δ(I) (overall/outer shell) | 18.4/3.6 |
| R_{merge}(%) (overall/outer shell) | 0.075/0.33 |

| Structure refinement | |
|---|---|
| Resolution (Å) | 49.3-2.8 |
| No. of reflections | 24288 |
| R factor | 23.5 |
| Free R factor¹ | 27.2 |
| Rms deviations | |
| | |
| Bond lengths | 0.013 |
| Bond angles | 1.7° |

| | |
|---|---|
| ¹ The Free R factor was calculated with 9.4% of the data. | |

## Claims

1. A compound of formula I: 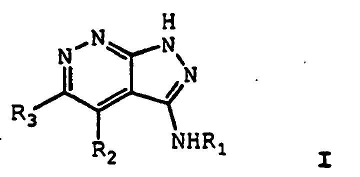 or a pharmaceutically acceptable salt thereof, wherein:
R₁ is selected from H; aliphatic selected from linear or branched: C₁-C₁₂ alkyl, C₂-C₁₂ alkenyl and C₂-C₁₂ alkynyl; RC(O) -; RS(O)ₙ-; ROC (O) : C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl , tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; wherein said aliphatic, carbocyclyl, heterocyclyl, aryl, aralkyl, heteroaryl, or heteroaralkyl is optionally substituted with halogen, CF₃, -R', -OR', -OH, -SH, -SR', acyloxy, -NO₂, -CN, -NH₂, -NHR', -N(R')₂, -NHCOR' , - NHCONH₂, -NHCONHR' , -NHCON(R')₂, -NRCOR' , -NHCO₂H, - NHCO₂R', -CO₂R', -CO₂H, -COR' , -CONH₂, -CONHR' , -CON(R')2, - S (O)₂H, -S(O)₂R', -SO₂NH₂, -S(O)H, -S(O)R', -SO₂NHR',-SO₂N(R')₂, -NHS(O)₂H, or -NHS(O)₂R' ; and wherein said aliphatic, carbocyclyl or heterocyclyl may be further optionally substituted with =O, =S, =NNHR', =NN(R')₂, =NOR', =NNHCOR', =NNHCO₂R', =NNHSO₂R', =N-CN, or =NR';
R₂ and R₃ are each independently selected from H; aliphatic selected from linear or branched: C₁-C₁₂ alkyl, C₂-C₁₂ alkenyl and C₂-C₁₂ alkynyl; C₃-C₁₂ carbocyclyl, heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane; benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl 2-naphthyl, 1-anthracyl and 2-anthracyl, tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; - N(R)₂; -NRCOR; -NRCO₂R; -NRSO₂R; -S(O)ₙR; -SO₂N(R)₂ ; -SR; - OR; -CF₃; halo; -NO₂ ; -CN; -C(O)R; -CO₂R; -OC(O)R; - CON(R)₂ ; or -OC(O)N(R)₂ ; wherein said aliphatic, carbocyclyl, heterocyclyl, aryl, aralkyl, heteroaryl, or heteroaralkyl is optionally substituted with halogen, CF₃, -R' , -OR', -OH, -SH, -SR', acyloxy, -NO₂, -CN, -NH₂, NHR', -N(R')₂, -NHCOR', -NHCONH₂, -NHCONHR', -NHCON(R')₂, -NRCOR', -NHCO₂H, -NHCO₂R', -CO₂R' , -CO₂H, -COR', - CONH₂, -CONHR' , -CON(R')₂, -S(O)₂H, -S(O)₂R', -SO₂NH₂, - S(O)H, -S(O)R', -SO₂NHR' , -SO₂N(R')₂, -NHS(O)₂H, or - NHS(O)₂R'; and wherein said aliphatic, carbocyclyl or heterocyclyl may be further optionally substituted with =O, =S, =NNHR', =NN(R')₂, =N-OR', =NNHCOR', =NNHCO₂R', =NNHSO₂R', =N-CN, or =NR'; or wherein R₂ and R₃ taken together with the intervening atoms optionally form a five- to nine-membered ring that is fused to the pyridazinyl ring of formula I, said fused ring having 0-2 heteroatoms;
R is selected from H; aliphatic selected from linear or branched: C₁-C₁₂ alkyl, C₂-C₁₂ alkenyl and C₂-C₁₂ alkynyl; C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl, tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl; 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; wherein each member of R except H is optionally substituted with halogen, CF₃, -R' , -OR', -OH, -SH, -SR' , acyloxy, -NO₂, -CN, -NH₂, -NHR' , -N(R')₂, -NHCOR', - NHCONH₂, -NHCONHR' , -NHCON(R')₂, -NRCOR' , -NHCO₂H, NHCO₂R', -CO₂R', -CO₂H, -COR' , -CONH₂, -CONHR' , -CON(R')₂, - S(O)₂H, -S(O)₂R', -SO₂NH₂, -S(O)H, -S(O)R', -SO₂NHR', - SO₂N(R')₂, -NHS(O)₂H_{,} or -NHS(O)₂R' ; and wherein said aliphatic, carbocyclyl or heterocyclyl may be further optionally substituted with =O, =S, =NNHR', =NN(R')₂, =NOR', =NNHCOR', =NNHCO₂R' =NNHSO₂R', =N-CN, or =NR'; and
n is 1 or 2;
R' is independently selected from H; aliphatic selected from linear or branched: C₁-C₁₂ alkyl C₂-C₁₂ alkenyl and C₂-C₁₂ alkynyl; C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophehyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, , azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl, tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; and each R' is optionally substituted with halogen, nitro, cyano, amino, -NH-(unsubstituted aliphatic), -N-(unsubstituted aliphatic)₂, carboxy, carbamoyl, hydroxy, - O-(unsubstituted aliphatic), -SH, -S-(unsubstituted aliphatic), CF₃, -SO₂NH₂, unsubstituted aliphatic, unsubstituted carbocyclyl, unsubstituted heterocyclyl, unsubstituted aryl, unsubstituted aralkyl, unsubstituted heteroaryl, or unsubstituted heteroaralkyl;
provided that when R₁ is H, R₂ and R₃ are not both unsubstituted phenyl; and when R₁ is H, R₂ and R₃ are other than H, halogen, or an unsubstituted alkyl.

2. The compound according to claim 1, wherein R₁ is H, RC(O)-, aralkyl, aralkenyl, or aralkynyl.

3. The compound according to claim 2, wherein R is aliphatic selected from linear or branched: C₁-C₁₂ alkyl, C₂-C₁₂ alkenyl and C₂-C₁₂ alkynyl or aryl selected from phenyl, 1-naphthyl, 2-naphthyl, 1-anthracyl and 2-anthracyl , tetrahydronaphthyl.

4. The compound according to claim 2, wherein R₁ is H, CH₃C(O)-, PhC(O)-, or PhCH₂-.

5. The compound according to claim 1, wherein R₂ and R₃ are independently H: aryl selected from phenyl, 1-naphthyl, 2-naphthyl, l-anthracyl and 2-anthracyl , tetrahydronaphthyl; C₃-C₁₂ carbocyclyl; heterocyclyl, selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; or heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl.

6. The compound according to claim 5, wherein R₂ and R₃ are independently H, phenyl, naphthyl, pyridyl, thienyl, furanyl, pyrimidinyl, benzodioxolyl, or cyclohexyl, any of which except H is optionally substituted with halogen, CF₃, -R' , -OR', -OH, -SH, -SR' acyloxy, -NO₂, -CN, -NH₂, -NHR' , -N(R')₂, -NHCOR', - NHCONH₂, -NHCONHR' , -NHCON(R')₂, -NRCOR', -NHCO₂H, - NHCO₂R', -CO₂R', -CO₂H, -COR', -CONH₂, -CONHR', -CON(R')₂. -S(O)₂H, -S(O)2R', -SO₂NH₂, -S(O)H, -S(O)R',-SO₂NHR', -SO₂N(R')₂, -NHS(O)₂H, or -NHS(O)₂R' ; and wherein said aliphatic, carbocyclyl or heterocyclyl may be further optionally substituted with =O, =S, =NNHR', =NN(R')₂, =N-OR', =NNHCOR', =NNHCO₂R', =NNHSO₂R =N-CN, or =NR'.

7. The compound according to claim 6, wherein the substituents are selected from halo, alkyl, -CN, -NO₂, -SO₂NH₂, -SO₂NH-(alkyl), -SO₂N(alkyl)₂, -O-alkyl, -NH₂, -N-alkyl, -N-(alkyl)₂, -CONH₂, -CONH(alkyl-), -CONH(alkyl)₂, - O-phenyl, or -S-alkyl.

8. The compound according to claim 1 having formula Ia: 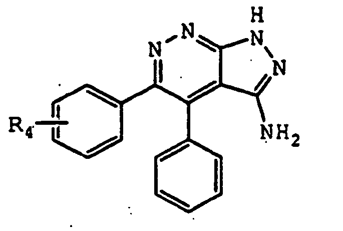 wherein R₄ is halo.

9. The compound according to claim 8, wherein R₄ is F.

10. The compound according to claim 1, wherein R₁ is H, R₂ and R₃ are independently phenyl optionally substituted with CF₃, -R', -OR', -OH, -SH, - SR', acyloxy, -NO₂, -CN, -NH₂, -NHR', -N(R')₂, -NHCOR' , - NHCONH₂, -NHCONHR', -NHCON(R')₂, -NRCOR', -NHCO₂H, - NHCO₂R', -CO₂R', -CO₂H, -COR', -CONH₂, -CONHR' , -CON(R')₂, - S(O)₂H, -S(O)₂R', -SO₂NH₂, -S(O)H, -S(O)R', -SO2NHR', - SO₂N(R')₂, -NHS(O)₂H, or -NHS(O)₂R';
provided that R₂ and R₃ are not both unsubstituted phenyl.

11. A. compound of claim 1 selected from the group consisting of:
3-amino-4(4-chlorophenyl)-5-phenyl-1H-pyrazolo[3,4-c]pyridazine (Compound 2);
3-amino-4,5-bis(4-fluorophenyl)-1H- pyrazolo[3,4-c] pyridazine (Compound 3);
3-amino-4-phenyl-5-(4-fluorophenyl)-1H-pyrazolo[3,4-c] pyridazine (Compound 4);
3-amino-4-(4-fluorophenyl)-5-phenyl-1H-pyrazolo[3,4-c] pyridazine (Compound 5);
3-amino-4-(3-fluorophenyl)-5-phenyl-1H-pyrazolo[3,4-c] pyridazine (Compound 6);
3-amino-4-phenyl-5-(4-pyridyl)-1H-pyrazolo[3,4-c] pyridazine (Compound 7);
3-amino-4-phenyl-5-(3-fluorophenyl)-1H-pyrazolo[3,4-c] pyridazine (Compound 8);
N-(4,5-diphenyl-1H-pyrazolo[3,4-c] pyridazin-3-yl)-acetamide (Compound 9);
N-(4,5-diphenyl-1H-pyrazolo[3,4-c] pyridazin-3-yl)-benzamide (Compound 10);
3-amino-4-phenyl-5-(4-methyl-phenyl)-1H-pyrazolo[3,9-c] pyridazine (Compound 11);
3-amino-4-phenyl-5-(2-methyl-phenyl)-1H-pyrazolo[3,4-c] pyridazine (Compound 12);
3-amino-4-phenyl-5-(3-methyl-phenyl)-1H-pyrazolo[3,4-c] pyridazine (Compound 13);
3-amino-4-phenyl-5-(2-chloro-phenyl)-1H-pyrazolo[3,4-c] pyridazine (Compound 14);
3-amino-4-phenyl-5-(2-fluorophenyl)-1H-pyrazolo[3,4-c] pyridazine (Compound 15);
3-amino-4-phenyl-5-(4-chloro-phenyl)-1H-pyrazolo[3,4-c] pyridazine (Compound 16);
3-amino-4(2-cyanophenyl)-5-phenyl-1H-pyrazolo[3,4-c]pyridazine (Compound 17);
3-amino-4(3-cyanophenyl)-5-phenyl-1H-pyrazolo[3,4-c]pyridazine (Compound 18);
3-amino-4(4-cyanophenyl)-5-phenyl-1H-pyrazolo[3,4-c]pyridazine (Compound 19);
3-amino-4-phenyl-5-(2-cyanophenyl)-1H-pyrazolo[-3,4-c] pyridazine (Compound 20);
3-amino-4-phenyl-5-(3-cyanophenyl)-1H-pyrazolo[3,4-c] pyridazine (Compound 21);
3-amino-4-phenyl-5-(4-cyanophenyl)-1H-pyrazolo[3,4-c] pyridazine (Compound 22);
3-amino-4-phenyl-5-(2-pyridyl)-1H-pyrazolo[3,4-c] pyridazine (Compound 23);
13-amino-4-phenyl-5-(3-pyridyl)-1H-pyrazolo[3,4-c] pyridazine (Compound 24);
N-(4,5-diphenyl-1H-pyrazolo[3,4-c] pyridazin-3-yl)-benzylamine (Compound 25);
3-amino-4(2-pyridyl)-5-phenyl-1H-pyrazolo[3,4-c] pyridazine (Compound 26);
3-amino-4(3-pyridyl)-S-phenyl-1H-pyrazolo[3,4-c] pyridazine (Compound 27);
3-amino-4(4-pyridyl)-5-phenyl-1H-pyrazolo[3,4-c] pyridazine (Compound 28);
3-amino-9-phenyl-5-(2-thienyl)-1H-pyrazolo[3,4-c] pyridazine (Compound 29);
3-amino-4(2-furanyl)-5-phenyl-1H-pyrazolo[3,4-c] pyridazine (Compound 30);
3-arnino-4-phenyl-5-naphthyl-1H-pyrazolo[3,4-c]pyridazine (Compound 31);
5-phenyl-4-o-tolyl-1H-pyrazolo[3,4-c] pyridazin-3-ylamine (Compound 35);
5-phenyl-4-m-tolyl-1H-pyrazolo[3,4-c] pyridazin-3-ylamine (Compound 36);
5-phenyl-4-p-tolyl-1H-pyrazolo[3,4-c] pyridazin-3-ylamine (Compound 37);
4-(4-nitro-phenyl)-5-phenyl-1H-pyrazolo[3,4-c]pyridazin-3-ylamine (Compound 38);
4-(3-amino-5-phenyl-1H-pyrazolo[3,4-c] pyridazin-4-yl)-benzenesulfonamide (Compound 39):
4-(3-fluoro-4-methyl-phenyl)-5-phenyl-1H-pyrazolo[3,4-c]pyridazin-3-ylamine (Compound 40);
4-(3-amino-4-phenyl-1H-pyrazolo[3,4-c] pyridazin-5-yl)-benzamide (Compound 41);
5-(2-amino-phenyl)-4-phenyl-1H-pyrazolo[3,4-c]pyridazin-3-ylamine (Compound 42);
9-(4-ethyl-phenyl)-5-phenyl-1H-pyrazolo[3,4-c]pyridazin-3-ylamine (Compound 43);
4-(2, 6-difluoro-phenyl)-5-phenyl-1H-pyrazolo[3,4-c]pyridazin-3-ylamine (Compound 44);
4-(3,4-dimethoxy-phenyl)-5-phenyl-1H-pyrazolo[3,4-c]pyridazin-3-ylamine (Compound 45);
4-(3-fluoro-phenyl)-5-phenyl-1H-pyrazolo[3,4-c]pyridazin-3-ylamine (Compound 46);
4-(3-nitro-phenyl)-5-phenyl-1H-pyrazolo[3,4-c]pyridazin-3-ylamine (Compound 47);
S-(2-chloro-phenyl)-4-(2,6-difluorophenyl)-1H-pyrazolo[3,4-c]pyridazin-3-ylamine (Compound 48);
5-(2-chloro-phenyl)-4-(3,4-dimethoxy-phenyl)-1H-pyrazolo[3,4-c]pyridazin-3-ylamine (Compound 49) :
5-(2-chloro-phenyl)-4-(3-fluoro-phenyl)-1H-pyrazolo[3,4-c]pyridazin-3-ylamine (Compound 50);
5-(2-chloro-phenyl)-4-(4-dimethylamino-phenyl)-1H-pyrazolo[3,4-c]pyridazin-3-ylamine (Compound 51);
4-[3-amino-5-(2-chloro-phenyl)-1H-pyrazolo[3,4-c]pyridazin-4-yl]-benzonitrile (Compound 52);
5-(2-chloro-phenyl)-4-pyridin-4-yl-1H-pyrazolo[3.4-c]pyridazin-3-ylamine (Compound 53);
5-(2-chloro-phenyl)-4-(3-nitro-phenyl)-1H-pyrazolo[3,4-c]pyridazin-3-ylamine (Compound 54);
5-(2-chloro-phenyl)-4-pyridin-3-yl-1H-pyrazolo[3,4-c]pyridazin-3-ylamine (Compound 55);
4-(2,6-difluoro-phenyl)-5-(3-methoxy-phenyl)-1H-pyrazolo[3,4-c]pyridazin-3-ylamine (Compoud 56) ;
4-(3,4-dimethoxy-phenyl)-5-(3-methoxy-phenyl)-1H-pyrazolo[3,4-c]pyridazin-3-ylamine (Compound 57);
4-(3-fluoro-phenyl)-5-(3-methoxy-phenyl)-1H-pyrazolo[3,4-c]pyridazin-3-ylamine (Compound 58);
4-(4-dimethylamino-phenyl)-5-(3-methoxy-phenyl)-1H-pyrazolo[3,4-c]pyridazin-3-ylamine (Compound 59);
4-[3-amino-5-(3-methoxy-phenyl)-1H-pyrazolo[3,4-c]pyridazin-4-yl]-benzonitrile (Compound 60);
5-(3-methoxy-phenyl)-4-pyridin-4-yl-1H-pyrazolo[3,4-c]pyridazin-3-ylamine (Compound 61);
5-(3-methoxy-phenyl)-4-(3-nitro-phenyl)-1H-pyrazolo[3,4-c]pyridazin-3-ylamine (Compound 62);
5-(3-methoxy-phenyl)-4-pyridin-3-yl-1H-pyrazolo[3,4-c]pyridazin-3-ylamine (Compound 63);
4-(2,6-difluoro-phenyl)-5-p-tolyl-1H-pyrazolo[3,4-c]pyridazin-3-ylamine (Compound 64);
4-(3,4-dimethoxy-phenyl)-5-p-tolyl-1H-pyrazolo[3,9-c]pyridazin-3-ylamine (Compound 65);
4-(3-fluoro-phenyl)-5-p-tolyl-1H-pyrazolo[3,4-c]pyridazin-3-ylamine (Compound 66);
9-(4-dimethylamino-phenyl)-5-p-tolyl-1H-pyrazolo[3,4-c]pyridazin-3-ylamine (Compound 67);
4-(3-amino-S-p-tolyl-1H-pyrazolo[3,4-c]pyridazin-4-yl)-benzonitrile (Compound 68);
4-pyridin-4-yl-5-p-tolyl-1H-pyrazolo[3,4-c]pyridazin-3-ylamine (Compound 69);
4-(3-nitro-phenyl)-5-p-tolyl-1H-pyrazolo[3,4-c]pyridazin-3-ylamine (Compound 70);
4-pyridin-3-yl-5-p-tolyl-1H-pyrazolo[3,4-c]pyridazin-3-ylamine (Compound 71);
3-(3-amino-5-benzo[1,3]dioxol-5-yl-1H-pyrazolo^{.}[3,4-c]pyridazin-4-yl)-benzonitrile (Compound 72);
3-[3-amino-5-(3-chloro-phenyl)-1H-pyrazolo[3,4-c]pyridazin-4-yl]-benzonitrile (Compound 73);
9-[3-amino-4-(3-cyano-phenyl)-1H-pyrazolo[3,4-c]pyridazin-5-yl]-benzamide (Compound 74);
3-[3-amino-5-(3-nitro-phenyl)-1H-pyrazolo[3,4-c]pyridazin-9-yl]-benzonitrile (Compound 75);
3-[3-amino-4-(3-cyano-phenyl)-1H-pyrazolo[3,4-c]pyridazin-5-yl]-benzamide (Compound 76);
3-(3-amino-5-p-tolyl-1H-pyrazolo[3,4-c]pyridazin-4-yl)-benzonitrile (Compound 77);
3-[3-amino-5-(3-phenoxy-phenyl)-1H-pyrazolo[3,4-c]pyridazin-4-yl]-benzonitrile (Compound 78);
4-[3-amino-4-(3-methoxy-phenyl)-1H-pyrazolo[3,4-c]pyridazin-5-yl]-benzonitrile (Compound . 79);
5-benzo[1,3]dioxol-5-yl-4-(4-chlorophenyl)-1H-pyrazolo[3,4-c]pyridazin-3-ylamine. (Compound 80);
4-(4-chloro-phenyl)-5-m-tolyl-1H-pyrazolo[3,4-c]pyridazin-3-ylamine (Compound 81);
4-[3-amino-4-(4-chloro-phenyl)-1H-pyrazolo[3,4-c]pyridazin-5-yl]-benzamide (Compound 82);
4-(4-chloro-phenyl)-5-(3-nitro-phenyl)-1H-pyrazolo[3,4-c]pyridazin-3-ylamine (Compound 83);
3-[3-amino-4-(4-chloro-phenyl)₋1H-pyrazolo[3,4-c]pyridazin-5-yl]-benzamide (Compound 84);
4-(4-chloro-phenyl)-5-(3-phenoxy-phenyl)-1H-pyra.zolo[3,4-c]pyridazin-3-ylamine (Compound 85);
4-[3-amino-4-(4-chloro-phenyl)-1H-pyrazolo[3,4-c]pyridazin-5-yl]-benzonitrile (Compound 86);
5-benzo[1,3]dioxol-5-yl-4-o-tolyl-1H-pyrazolo[3,4-c]pyridazin-3-ylamine (Compound 87);
5-(3-chloro-phenyl)-4-o-tolyl-1H-pyrazolo[3,4-c]pyridazin-3-ylamine (Compound 88);
4-(3-amino-4-o-tolyl-1H-pyrazolo[3,4-c] pyridazin-5-yl)-benzamide (Compound 89);
5-(3-nitro-phenyl)-4-o-tolyl-1H-pyrazolo[3,4-c]pyridazin-3-ylamine (Compound 90);
3-(3-amino-4-o-tolyl-1H-pyrazolo[3,4-c]pyridazin-5-yl)-benzamide (Compound 91);
4-o-tolyl-5-p-tolyl-1H-pyrazolo[3,4-c]pyridazin-3-ylamine (Compound 92);
5-(3-phenoxy-phenyl)-4-o-tolyl-1H-pyrazolo[3,4-c].pyridazin-3-ylamine (Compound 93);
4-(3-amino-4-o-tolyl-1H-pyrazolo[3,4-c]pyridazin-5-yl)-benzonitrile (Compound 94.);
5-benzo[1,3]dioxol-5-yl-4-phenyl-1H-pyrazolo[3,4-c]pyridazin-3-ylamine (Compound 95);
5-(3-chloro-phenyl)-4-phenyl-1H-pyrazolo[3,4-c]pyridazin-3-ylamine (Compound 96);
S-(3-nitro-phenyl)-4-phenyl-1H-pyrazolo[3,9-c]pyridazin-3-ylamine (Compound 97):
3-(3-amino-4-phenyl-1H-pyrazolo[3,4-c]pyridazin-5-yl)-benzamide (Compound 98);
5-(4-tert-butyl-phenyl)-4-phenyl-1H-pyrazolo[3,4-c]pyridazin-3-ylamine (Compound 99);
5-(3-phenoxy-phenyl)-4-phenyl-1H-pyrazolo[3,4-c]pyridazin-3-ylamine (Compound 100);
4-cyclohexyl-5-phenyl₋1H-pyrazolo[3,4-c]pyridazin-3-ylamine (Compound 101); and
4-(2-Methylsulfanyl-pyrimidin-4-yl)-5-phenyl-1H-pyrazolo[3,4-c]pyridazin-3-ylamine (Compound 102).

12. The compound according to claim 11, wherein the compound is:
3-amino-4-phenyl-5-(3-fluorophenyl)-1H-pyrazolo[3,4-c] pyridazine (Compound 8).

13. A pharmaceutical composition comprising a compound according to any one of claims 1-12 and a pharmaceutically acceptable carrier, adjuvant, or vehicle.

14. Use of:
(a) a compound of formula I: 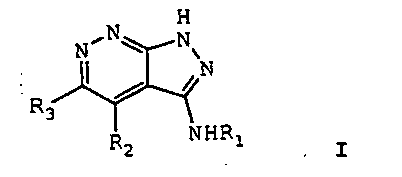 or a pharmaceutically acceptable salt thereof, wherein:
R₁ is selected from H; aliphatic selected from linear or branched: C₁-C₁₂ alkyl, C₂-C₁₂ alkenyl and C₂-C₁₂ alkynyl; RC (O) ; RS(O)ₙ ; ROC(O); C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl, tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; wherein said aliphatic, carbocyclyl, heterocyclyl, aryl, aralkyl, heteroaryl, or heteroaralkyl is optionally substituted with halogen, CF₃, -R', -OR', -OH, -SH, -SR' , acyloxy, -NO₂, -CN, -NH₂, -NHR' , -N(R')₂, -NHCOR', - NHCONH₂, -NHCONHR' , -NHCON(R')₂, -NRCOR', -NHCO₂H, - NHCO₂R', -CO₂R', -CO₂H, -COR', -CONH₂, -CONHR', -CON(R')₂,-S(O)₂H, -S(O)₂R', -SO₂NH₂ -S(O)H, -S(O)R' , -SO₂NHR', - SO₂N(R')₂, -NHS(O)₂H, or -NHS(O)₂R'; and wherein said aliphatic, carbocyclyl or heterocyclyl may be further optionally substituted with =O, =S, =NNHR', =NN(R')₂, =NOR', =NNHCOR', =NNHCO₂R', =NNHSO₂R', =N-CN, or =NR';
R₂ and R₃ are each independently selected from H; aliphatic selected from linear or branched: C₁-C₁₂ alkyl, C₂-C₁₂ alkenyl and C₂-C₁₂ alkynyl: C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; - N(R)₂; .-NRCOR; -NRCO₂R; -NRSO₂R; -S(O)ₙR; -SO₂N(R)₂; -SR; - OR; -CF₃: halo; -NO₂; -CN; -C (O) R; -CO₂R -OC (O) R; - CON(R)₂; or -OC(O)N(R)₂; wherein said aliphatic, carbocyclyl, heterocyclyl, aryl, aralkyl, heteroaryl, or heteroaralkyl is optionally substituted with halogen, CF₃, -R', -OR', -OH, -SH, -SR', acyloxy, -NO₂, -CN, -NH₂, - NHR', -N(R')₂, -NHCOR', -NHCONH₂, -NHCONHR', -NHCON(R')₂, -NRCOR', -NHCO₂H -NHCO₂R', -CO₂R' , -CO₂H, -COR', -CONH₂, - CONHR' , -CON(R')₂, -S(O)₂H, -S(O)₂R', -SO₂NH₂, -S(O)H, - S(O)R', -SO₂NHR', -SO₂N(R')₂, -NHS(O)₂H, or -NHS(O)₂R'; and wherein, said aliphatic, carbocyclyl or heterocyclyl may be further optionally substituted with =O, =S, =NNHR', =NN(R')₂, =N-OR', =NNHCOR', =NNHCO₂R' =NNHSO₂R', =N-CN, or =NR'; or wherein R₂ and R₃ taken together with the intervening atoms optionally form a five- to nine-membered ring that is fused to the pyridazinyl ring of formula I, said fused ring having 0-2 heteroatoms;
R is selected from H; aliphatic selected from linear or branched: C₁-C₁₂ alkyl, C₂-C₁₂ alkenyl and C₂-C₁₂ alkynyl: C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazblidinyl, pyrazolinyl, pyrazolidinyl, pyranyl, dioxanyl, dithianyl, trithianyl, quinuclidinyl, oxepanyl, and thiepanyl), aryl (phenyl, 1-naphthyl, 2-naphthyl, 1-anthracyl and 2-anthracyl; aryl selected from phenyl, 1-naphthyl, 2-naphthyl, 1-anthracyl and 2-anthracyl, tetrahydronaphthyl; aralkyl; aralkenyl; aralkynyl; heteroaryl; selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; heteroaralkynyl; wherein each member of R except H is optionally substituted with halogen, CF₃, -R', -OR', -OH, -SH, -SR' , acyloxy, -NO₂, - CN, -NH₂, -NHR', -N(R')₂, -NHCOR', -NHCONH₂, -NHCONHR' , -NHCON(R')₂, -NRCOR', -NHCO₂H, -NHCOzR' , -CO₂R -CO₂H, - COR', -CONH₂, -CONHR' , -CON(R')_{2,} -S(O)₂H, -S(O)₂R', -SO₂NH₂, -S(O)H, -S(O)R', -SO₂NHR', -SO₂N(R')₂, -NHS(O)₂H, or -NHS(O)₂R'; and wherein said aliphatic, carbocyclyl or heterocyclyl may be further optionally substituted with =O, =S, =NNHR', =NN(R')₂, =N-OR', =NNHCOR', =NNHCO₂R', =NNHSO₂R', =N-CN, or =NR';
R' is independently selected from H; aliphatic selected from linear or branched: C₁-C₁₂ alkyl, C₂-C₁₂ alkenyl and C₂-C₁₂ alkynyl; C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl, tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, .3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; and each R' is optionally substituted with halogen, nitro, cyano, amino, -NH-(unsubstituted aliphatic), -N-(unsubstituted aliphatic)₂, carboxy, carbamoyl, hydroxy, - O-(unsubstituted aliphatic), -SH, -S-(unsubstituted aliphatic), CF₃, -SO₂NH₂, unsubstituted aliphatic, unsubstituted carbocyclyl, unsubstituted heterocyclyl, unsubstituted aryl, unsubstituted aralkyl, unsubstituted heteroaryl, or unsubstituted heteroaralkyl; and
n is 1 or 2; or
(b) a pharmaceutical composition comprising said compound and a pharmaceutically acceptable carrier, adjuvant, or vehicle;
for the preparation of a medicament for inhibiting GSK-3 activity in a patient.

15. A compound of formula I: 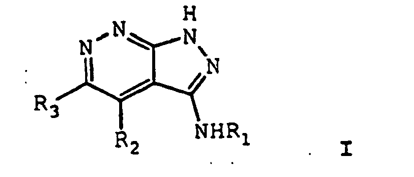 or a pharmaceutically acceptable salt thereof, wherein
R₁ is selected from H; aliphatic selected from linear or branched: C₁-C₁₂ alkyl, C₂-C₁₂ alkenyl and C₂-C₁₂ alkynyl; RC(O); RS(O)ₙ; ROC(O); C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl, tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaral.kyl; heteroaralkenyl; or heteroaralkynyl; wherein said aliphatic, carbocyclyl, heterocyclyl, aryl, aralkyl, heteroaryl, or heteroaralkyl is optionally substituted with halogen, CF₃, -R' , -OR', -OH, -SH, -SR', acyloxy, -NO₂, -CN', -NH₂, -NHR' , -N(R')₂, -NHCOR', - NHCONH₂, -NHCONHR' , -NHCON(R')₂, -NRCOR' , -NHCO₂H, NHCO₂R', -CO₂R', -CO₂H -COR', -CONH₂, -CONHR' , -CON(R')₂,-S(O)₂H, -S(O)₂R', -SO₂NH₂, -S(O)H, -S(O)R', -SO₂NHR', SO₂N(R')₂, -NHS (O) ₂H, or -NHS(O)₂R'; and wherein said aliphatic, carbocyclyl or heterocyclyl may be further optionally substituted with =O, =S, =NNHR', =NN(R')₂, =N-OR', =NNHCOR', =NNHCO₂R', =NNHSO₂R', =N-CN, or =NR';
R₂ and R₃ are each independently selected from H; aliphatic selected from linear or branched: C₁-C₁₂ alkyl, C₂-C₁₂ alkenyl and C₂-C₁₂ alkynyl C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl , tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; - N (R) ₂; -NRCOR; -NRCO₂R; -NRSO₂R; -S(O)ₙR ; -SO₂N (R) ₂; -SR; - OR; -CF₃; halo; -NO₂; -CN; -C(O)R; -CO₂R; -OC(O)R;-CON (R) ₂; or -OC(O)N(R)₂; wherein said aliphatic, carbocyclyl, heterocyclyl, aryl, aralkyl, heteroaryl, or heteroaralkyl is optionally substituted with halogen, CF₃, -R' , -OR' , -OH, -SH, -SR', acyloxy, -NO₂, -CN, -NH₂, - NH', -N(R')₂, =NHCOR', -NHCONH₂, -NHCONHR', -NHCON(R')₂, -NRCOR', -NHCO₂H, -NHCO₂R', -CO₂R', -CO₂H, -COR', -CONH₂, - CONHR', -CON(R')_{2,} -S(O)₂H, -S(O)₂R', -SO₂NH₂, -S (O) H, - S(O)R', -SO₂NHR', -SO₂N(R')₂, -NHS(O)₂H, or -NHS(O)₂R'; and wherein said aliphatic, carbocyclyl or heterocyclyl may be further optionally substituted with =O, =S, =NNHR', =NN(R')₂, =N-OR', =NNHCOR', =NNHCO₂R', =NNHSO₂R', =N-CN, or =NR'; or wherein R₂ and R₃ taken together with the intervening atoms optionally form a five- to nine-membered ring that is fused to the pyridazinyl ring of formula I, said fused ring having 0-2 heteroatoms;
R is selected from H; aliphatic selected from linear or branched: C₁-C₁₂ alkyl, C₂-C₁₂ alkenyl and C₂-C₁₂ alkynyl; C₁-C₁₂ carbocyclyl ; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl), aryl (phenyl, 1-naphthyl, 2-naphthyl, 1-anthracyl and 2-anthracyl; aryl selected from phenyl, 1-naphthyl, 2-naphthyl, 1-anthracyl and 2-anthracyl, tetrahydronaphthyl; aralkyl; aralkenyl; aralkynyl; heteroaryl; selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heterbaralkenyl; heteroaralkynyl; wherein each member of R except H is optionally substituted with halogen, CF₃, -R', -OR', -OH, -SH, -SR', acyloxy, -NO₂, CN, -NH₂, -NHR' , -N(R')₂, -NHCOR', -NHCONH₂, -NHCONHR' , -NHCON(R')₂, -NRCOR', -NHCO₂H, -NHCO₂R', -CO₂R', -CO₂H, - COR', -CONH₂, -CONHR' , -CON(R')₂, -S(O)₂H, -S(O)₂R' , -SO₂NH₂, -S(O)H, -S(O)R', -SO₂NHR', -SO₂N(R')₂, -NHS(O)₂H, or -NHS(O)₂R'; and wherein said aliphatic, carbocyclyl or heterocyclyl may be further optionally substituted with =O, =S, =NNHR', =NN(R')₂, =N-OR', =NNHCOR', =NNHCO₂R' =NNHSO₂R', =N-CN, or =NR';
R' is independently selected from H; aliphatic selected from linear or branched: C₁-C₁₂ alkyl, C₂-C₁₂ alkenyl and C₂-C₁₂ alkynyl; C₃-C₁₂ carbocyclyl; heterocyclyl selected from 3-1H-benzimidazol-2-one, 3-1H-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxane, benzotriazol-1-yl, benzopyrrolidine, benzopiperidine, benzoxolane, benzothiolane, benzothiane, aziranyl, oxiranyl, azetidinyl, pyrrolinyl, dioxolanyl, imidazolinyl, imidazolidinyl, pyrazolinyl, pyrazolidinyl, pyranyl, dioxanyl, dithianyl, trithianyl, quinuclidinyl, oxepanyl, and thiepanyl; aryl selected from phenyl, 1-naphthyl, 2-naphthyl, 1-anthracyl and 2-anthracyl, tetrahydronaphthyl; aralkyl; aralkenyl; aralkynyl; heteroaryl selected from benzimidazolyl, benzothienyl, benzofuranyl, indolyl, quinolinyl, benzothiazolyl, benzoxazolyl, benzimidazolyl, isoquinolinyl, isoindolyl, acridinyl, benzoisoxazolyl, 2-furanyl, 3-furanyl, N-imidazolyl, 2-imidazolyl, 4-imidazolyl, .5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4-thiazolyl, 5-thiazolyl, 5-tetrazolyl, 2-triazolyl, 5-triazolyl, 2-thienyl, and 3-thienyl; heteroaralkyl; heteroaralkenyl; or heteroaralkynyl; and each R' is optionally substituted with halogen, nitro, cyano, amino, -NH-(unsubstituted aliphatic), -N-(unsubstituted aliphatic)₂, carboxy, carbamoyl, hydroxy, 0-(unsubstituted aliphatic), -SH, -S-(unsubstituted aliphatic) CF₃, -SO₂NH₂, unsubstituted aliphatic, unsubstituted carbocyclyl, unsubstituted heterocyclyl, unsubstituted aryl, unsubstituted aralkyl, unsubstituted heteroaryl, or unsubstituted heteroaralkyl; and
n is 1 or 2; or
or a pharmaceutical composition comprising said compound and a pharmaceutically acceptable carrier, adjuvant, or vehicle;
for use in inhibiting GSK-3 activity in a patient.

16. Use of claim 14 for enhancing glycogen synthesis or lowering blood levels of glucose in a patient in need thereof.

17. The use according to claim 16 wherein the patient is treated for diabetes.

18. Use of claim 14 for inhibiting the production of hyperphosphorylated Tau protein in a patient in need thereof.

19. The use according to claim 18, wherein the patient is treated for Alzheimer's disease.

20. Use of claim 14 for inhibiting the phosphorylation of β-catenin in a patient in need thereof.

21. The use according to claim 20, wherein the patient is treated for schizophrenia.

22. The use according to any one of claims 14, 16, 18, and 20, wherein said compound or composition is administered with an additional therapeutic agent.

23. Compound or composition of claim 15 for use in enhancing glycogen synthesis or lowering blood levels of glucose in a patient in need thereof.

24. The compound or composition according to claim 23, wherein the patient is treated for diabetes.

25. Compound or composition of claim 15 for use in inhibiting the production of hyperphosphorylated Tau protein in a patient in need thereof.

26. The compound or composition according to claim 25, wherein the patient is treated for Alzheimer's disease.

27. Compound or composition of claim 15 for use in inhibiting the phosphorylation of β-catenin in a patient in need thereof.

28. The compound or composition according to claim 27, wherein the patient is treated for schizophrenia.

29. The compound or composition according to any one of claims 15, 23, 25, and 27, wherein said compound or composition is administered with an additional therapeutic agent.

30. An in vitro method for inhibiting GSK-3 activity in a biological sample, comprising the step of contacting the biological sample with a compound as defined in claim 14 or a composition comprising said compound and a carrier, adjuvant, or vehicle.

## Patentansprüche

1. Verbindung der Formel I: 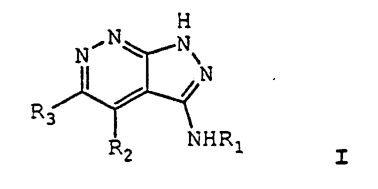 oder ein pharmazeutisch verträgliches Salz davon, wobei:
R₁ ausgewählt ist aus H; einem Aliphat ausgewählt aus linear oder verzweigt: C₁-C₁₂-Alkyl, C₂-C₁₂-Alkenyl und C₂-C₁₂-Alkinyl; RC(O)-; RS(O)ₙ-; ROC(O); C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl;
wobei das Aliphat, Carbocyclyl, Heterocyclyl, Aryl, Aralkyl, Heteroaryl oder Heteroaralkyl gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann;
R₂ und R₃ jeweils unabhängig voneinander ausgewählt sind aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzothiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl; -N(R)₂; -NRCOR; -NRCO₂R; -NRSO₂R; -S(O)ₙR; -SO₂N(R)₂; -SR; -OR; -CF₃; Halo; -NO₂; -CN; -C(O)R; -CO₂R; -OC(O)R; -CON(R)₂; oder -OC(O)N(R)₂; wobei das Aliphat, Carbocyclyl, Heterocyclyl, Aryl, Aralkyl, Heteroaryl oder Heteroaralkyl gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann; oder wobei R₂ und R₃ zusammen mit den dazwischenliegenden Atomen gegebenenfalls einen fünf- bis neungliedrigen Ring bilden, welcher an den Pyridazinylring der Formel I kondensiert ist, wobei der kondensierte Ring 0 bis 2 Heteroatome aufweist;
R ausgewählt ist aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl;
wobei jeder Bestandteil von R außer H gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann; und
n gleich 1 oder 2 ist;
R' unabhängig ausgewählt ist aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl; und jeder Rest R' gegebenenfalls mit Halogen, Nitro, Cyano, Amino, -NH-(unsubstituiertem Aliphat), -N-(unsubstituiertem Aliphat)₂, Carboxy, Carbamoyl, Hydroxy, -O-(unsubstituiertem Aliphat), -SH, -S-(unsubstituiertem Aliphat), CF₃, -SO₂NH₂, unsubstituiertem Aliphat, unsubstituiertem Carbocyclyl, unsubstituiertem Heterocyclyl, unsubstituiertem Aryl, unsubstituiertem Aralkyl, unsubstituiertem Heteroaryl oder unsubstituiertem Heteroaralkyl substituiert ist; mit der Maßgabe, dass, wenn R₁ für H steht, R₂ und R₃ nicht beide unsubstituiertes Phenyl darstellen; und wenn R₁ für H steht, R₂ und R₃ nicht H, Halogen oder unsubstituiertes Alkyl darstellen.

2. Verbindung gemäß Anspruch 1, wobei R₁ für H, RC(O)-, Aralkyl, Aralkenyl oder Aralkinyl steht.

3. Verbindung gemäß Anspruch 2, wobei R ein Aliphat ist, ausgewählt aus linear oder verzweigt: C₁-C₁₂-Alkyl, C₂-C₁₂-Alkenyl und C₂-C₁₂-Alkinyl oder ein Aryl, ausgewählt aus Phenyl, 1-Naphthyl, 2-Naphthyl, 1-Anthracyl und 2-Anthracyl, Tetrahydronaphthyl.

4. Verbindung gemäß Anspruch 2, wobei R₁ für H, CH₃C(O)-, PhC(O)- oder PhCH₂-steht.

5. Verbindung gemäß Anspruch 1, wobei R₂ und R₃ unabhängig voneinander für H; Aryl ausgewählt aus Phenyl, 1-Naphthyl, 2-Naphthyl, 1-Anthracyl und 2-Anthracyl, Tetrahydronaphthyl, C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; oder Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl stehen.

6. Verbindung gemäß Anspruch 5, wobei R₂ und R₃ unabhängig voneinander für H, Phenyl, Naphthyl, Pyridyl, Thienyl, Furanyl, Pyrimidinyl, Benzodioxolyl oder Cyclohexyl stehen, wobei jedes davon außer H gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann.

7. Verbindung gemäß Anspruch 6, wobei die Substituenten ausgewählt sind aus Halo, Alkyl, -CN, -NO₂, -SO₂NH₂, -SO₂NH-(Alkyl), -SO₂N(Alkyl)₂, -O-Alkyl, -NH₂, -N-Alkyl, -N-(Alkyl)₂, -CONH₂, -CONH(Alkyl), -CONH(Alkyl)₂, -O-Phenyl oder -S-Alkyl.

8. Verbindung gemäß Anspruch 1 mit der Formel Ia: 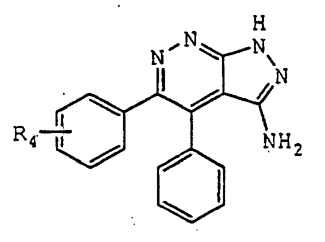 wobei R₄ für Halo steht.

9. Verbindung gemäß Anspruch 8, wobei R₄ für F steht.

10. Verbindung gemäß Anspruch 1, wobei R₁ für H steht, R₂ und R₃ unabhängig voneinander Phenyl darstellen, welches gegebenenfalls mit CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist;
mit der Maßgabe, dass R₂ und R₃ nicht beide unsubstituiertes Phenyl darstellen.

11. Verbindung gemäß Anspruch 1, ausgewählt aus:
3-Amino-4-(4-chlorphenyl)-5-phenyl-1H- pyrazolo[3,4-c]pyridazin (Verbindung 2);
3-Amino-4,5-bis(4-fluorphenyl)-1H-pyrazol[3,4-c]pyridazin (Verbindung 3);
3-Amino-4-phenyl-5-(4-fluorphenyl)-1H-pyrazolo[3,4-c]pyridazin (Verbindung 4);
3-Amino-4-(4-fluorphenyl)-5-phenyl-1H- pyrazolo[3,4-c]pyridazin (Verbindung 5);
3-Amino-4-(3-fluorphenyl)-5-phenyl-1H-pyrazolo[3,4-c]pyridazin (Verbindung 6);
3-Amino-4-phenyl-5-(4-pyridyl)-1H-pyrazolo[3,4-c]pyridazin (Verbindung 7);
3-Amino-4-phenyl-5-(3-fluorphenyl)-1H-pyrazolo[3,4-c]pyridazin (Verbindung 8);
N-(4,5-Diphenyl-1H-pyrazolo[3,4-c]pyridazin-3-yl)acetamid (Verbindung 9);
N-(4,5-Diphenyl-1H-pyrazolo[3,4-c]pyridazin-3-yl)benzamid (Verbindung 10);
3-Amino-4-phenyl-5-(4-methylphenyl)-1H-pyrazolo[3,4-c]pyridazin (Verbindung 11);
3-Amino-4-phenyl-5-(2-methylphenyl)-1H-pyrazolo[3,4-c]pyridazin (Verbindung 12);
3-Amino-4-phenyl-5-(3-methylphenyl)-1H-pyrazolo[3,4-c]pyridazin (Verbindung 13);
3-Amino-4-phenyl-5-(2-chlorphenyl)-1H-pyrazolo[3,4-c]pyridazin (Verbindung 14);
3-Amino-4-phenyl-5-(2-fluorphenyl)-1H-pyrazolo[3,4-c]pyridazin (Verbindung 15);
3-Amino-4-phenyl-5-(4-chlorphenyl)-1H-pyrazolo[3,4-c]pyridazin (Verbindung 16);
3-Amino-4-(2-cyanophenyl)-5-phenyl-1H-pyrazolo[3,4-c]pyridazin (Verbindung 17);
3-Amino-4-(3-cyanophenyl)-5-phenyl-1H -pyrazolo[3,4-c]pyridazin (Verbindung 18);
3-Amino-4-(4-cyanophenyl)-5-phenyl-1H-pyrazolo[3,4-c]pyridazin (Verbindung 19);
3-Amino-4-phenyl-5-(2-cyanophenyl)-1H-pyrazolo[3,4-c]pyridazin (Verbindung 20);
3-Amino-4-phenyl-5-(3-cyanophenyl)-1H-pyrazolo[3,4-c]pyridazin (Verbindung 21);
3-Amino-4-phenyl-5-(4-cyanophenyl)-1H-pyrazolo[3,4-c]pyridazin (Verbindung 22);
3-Amino-4-phenyl-5-(2-pyridyl)-1H-pyrazolo[3,4-c]pyridazin (Verbindung 23);
3-Amino-4-phenyl-5-(3-pyridyl)-1H-pyrazolo[3,4-c]pyridazin (Verbindung 24);
N-(4,5- Diphenyl-1H-pyrazolo[3,4-c]pyridazin-3-yl)benzylamin (Verbindung 25);
3-Amino-4-(2-pyridyl)-5-phenyl-1H-pyrazolo[3,4-c]pyridazin (Verbindung 26);
3-Amino-4-(3-pyridyl)-5-phenyl-1H-pyrazolo[3,4-c]pyridazin (Verbindung 27);
3-Amino-4-(4-pyridyl)-5-phenyl-1H-pyrazolo[3,4-c]pyridazin (Verbindung 28);
3-Amino-4-phenyl-5-(2-thienyl)-1H-pyrazolo[3,4-c]pyridazin (Verbindung 29);
3-Amino-4-(2-furanyl)-5-phenyl-1H-pyrazolo[3,4-c]pyridazin (Verbindung 30);
3-Amino-4-phenyl-5-naphthyl-1H-pyrazolo[3,4-c]pyridazin (Verbindung 31);
5- Phenyl-4-o-tolyl-1H-pyrazolo[3,4-c]pyridazin-3-yl-amin (Verbindung 35);
5-Phenyl-4-m-tolyl-1H-pyrazolo[3,4-c]pyridazin-3-yl-amin (Verbindung 36);
5-Phenyl-4-p-tolyl-1H-pyrazolo[3,4-c]pyridazin-3-yl-amin (Verbindung 37);
4-(4-Nitrophenyl)-5-phenyl-1H-pyrazolo[3,4-c]pyridazin-3-yl-amin (Verbindung 38);
4-(3-Amino-5-phenyl-1H-pyrazolo[3,4-c]pyridazin-4-yl)benzolsulfonamid (Verbindung 39);
4-(3-Fluor-4-methylphenyl)-5-phenyl-1H-pyrazolo[3,4-c]pyridazin-3-yl-amin (Verbindung 40);
4-(3-Amino-4-phenyl-1H-pyrazolo[3,4-c]pyridazin-5-yl)benzamid (Verbindung 41);
5-(2-Aminophenyl)-4-phenyl-1H-pyrazolo[3,4-c] pyridazin-3-yl-amin (Verbindung 42);
4-(4-Ethylphenyl)-5-phenyl-1H-pyrazolo[3,4-c]pyridazin-3-yl-amin (Verbindung 43);
4-(2,6-Difluorphenyl)-5-phenyl-1H-pyrazolo[3,4-c]pyridazin-3-yl-amin (Verbindung 44);
4-(3,4-Dimethoxyphenyl)-5-phenyl-1H-pyrazolo[3,4-c]pyridazin-3-yl-amin (Verbindung 45);
4-(3-Fluorphenyl)-5-phenyl-1H-pyrazolo[3,4-c]pyridazin-3-yl-amin (Verbindung 46);
4-(3-Nitrophenyl)-5-phenyl-1H-pyrazolo[3,4-c]pyridazin-3-yl-amin (Verbindung 47);
5-(2-Chlorphenyl)-4-(2,6-difluorphenyl)-1H-pyrazolo[3,4-c]pyridazin-3-yl-amin (Verbindung 48);
5-(2-Chlorphenyl)-4-(3,4-dimethoxyphenyl)-1H-pyrazolo[3,4-c]pyridazin-3-yl-amin (Verbindung 49);
5-(2-Chlorphenyl)-4-(3-fluorphenyl)-1H-pyrazolo[3,4-c]pyridazin-3-yl-amin (Verbindung 50);
5-(2-Chlorphenyl)-4-(4-dimethylaminophenyl)-1H-pyrazolo[3,4-c]pyridazin-3-yl-amin (Verbindung 51);
4-[3-Amino-5-(2-chlorphenyl)-1H-pyrazolo[3,4-c]pyridazin-4-yl]benzonitril (Verbindung 52);
5-(2-Chlorphenyl)-4-pyridin-4-yl-1H-pyrazolo[3,4-c]pyridazin-3-yl-amin (Verbindung 53);
5-(2-Chlorphenyl)-4-(3-nitrophenyl)-1H-pyrazolo[3,4-c]pyridazin-3-yl-amin (Verbindung 54);
5-(2-Chlorphenyl)-4-pyridin-3-yl-1H-pyrazolo[3,4-c]pyridazin-3-yl-amin (Verbindung 55);
4-(2,6-Difluorphenyl)-5-(3-methoxyphenyl)-1H-pyrazolo[3,4-c]pyridazin-3-yl-amin (Verbindung 56);
4-(3,4-dimethoxyphenyl)-5-(3-methoxyphenyl)-1H-pyrazolo[3,4-c]pyridazin-3-yl-amin (Verbindung 57);
4-(3-Fluorphenyl)-5-(3-methoxyphenyl)-1H-pyrazolo[3,4-c]pyridazin-3-yl-amin (Verbindung 58);
4-(4-Dimethylaminophenyl)-5-(3-methoxyphenyl)-1H-pyrazolo[3,4-c]pyridazin-3-yl-amin (Verbindung 59);
4-[3-Amino-5-(3-methoxyphenyl)-1H-pyrazolo[3,4-c]pyridazin-4-yl]benzonitril (Verbindung 60);
5-(3-Methoxyphenyl)-4-pyridin-4-yl-1H-pyrazolo[3,4-c]pyridazin-3-yl-amin (Verbindung 61);
5-(3-Methoxyphenyl)-4-(3-nitrophenyl)-1H-pyrazolo[3,4-c]pyridazin-3-yl-amin (Verbindung 62);
5-(3-Methoxyphenyl)-4-pyridin-3-yl-1H-pyrazolo[3,4-c]pyridazin-3-yl-amin (Verbindung 63);
4-(2,6-Difluorphenyl)-5-p-tolyl-1H-pyrazolo[3,4-c]pyridazin-3-yl-amin (Verbindung 64);
4-(3,4-Dimethoxyphenyl)-5-p-tolyl-1H-pyrazolo[3,4-c]pyridazin-3-yl-amin (Verbindung 65);
4-(3-Fluorphenyl)-5-p-tolyl-1H-pyrazolo[3,4-c]pyridazin-3-yl-amin (Verbindung 66);
4-(4-Dimethylaminophenyl)-5-p-tolyl-1H-pyrazolo[3,4-c]pyridazin-3-yl-amin (Verbindung 67);
4-(3-Amino-5-p-tolyl-1H-pyrazolo[3,4c]pyridazin-4-yl)benzonitril (Verbindung 68);
4-Pyridin-4-yl-5-p-tolyl-1H-pyrazolo[3,4c]pyridazin-3-yl-amin (Verbindung 69);
4-(3-Nitrophenyl)-5-p-tolyl-1H-pyrazolo[3,4-c]pyridazin-3-yl-amin (Verbindung 70);
4-Pyridin-3-yl-5-p-tolyl-1H-pyrazolo[3,4c]pyridazin-3-yl-amin (Verbindung 71);
3-(3-Amino-5-benzo[1,3]dioxol-5-yl-1H-pyrazolo[3,4-c]pyridazin-4-yl)benzonitril (Verbindung 72);
3-[3-Amino-5-(3-chlorphenyl)-1H-pyrazolo[3,4-c]pyridazin-4-yl]benzonitril (Verbindung 73);
4-[3-Amino-4-(3-cyanophenyl)-1H-pyrazolo[3,4-c]pyridazin-5-yl]benzamid (Verbindung 74);
3-[3-Amino-5-(3-nitrophenyl)-IH-pyrazolo[3,4-c]pyridazin-4-yl]benzonitril (Verbindung 75);
3-[3-Amino-4-(3-cyanophenyl)-1H-pyrazolo[3,4-c]pyridazin-5-yl]benzamid (Verbindung 76);
3-(3-Amino-5-p-tolyl-1H-pyrazolo[3,4c]pyridazin-4-yl)benzonitril (Verbindung 77);
3-[3-Amino-5-(3-phenoxyphenyl)-1H-pyrazolo[3,4-c]pyridazin-4-yl]benzonitril (Verbindung 78);
4-[3-Amino-4-(3-methoxyphenyl)-1H-pyrazolo[3,4-c]pyridazin-5-yl]benzonitril (Verbindung 79);
5-Benzo[1,3]dioxol-5-yl-4-(4-chlorphenyl)-1H-pyrazolo[3,4-c]pyridazin-3-yl-amin (Verbindung 80);
4-(4-Chlorphenyl)-5-m-tolyl-1H-pyrazolo[3,4-c]pyridazin-3-yl-amin (Verbindung 81);
4-[3-Amino-4-(4-chlorphenyl)-1H-pyrazolo[3,4-c]pyridazin-5-yl]benzamid (Verbindung 82);
4-(4-Chlorphenyl)-5-(3-nitrophenyl)-1H-pyrazolo[3,4-c]pyridazin-3-yl-amin (Verbindung 83);
3-[3-Amino-4-(4-chlorphenyl)-1H-pyrazolo[3,4-c]pyridazin-5-yl]benzamid (Verbindung 84);
4-(4-Chlorphenyl)-5-(3-phenoxyphenyl)-1H-pyrazolo[3,4-c]pyridazin-3-yl-amin (Verbindung 85);
4-[3-Amino-4-(4-chlorphenyl)-1H-pyrazolo[3,4-c]pyridazin-5-yl]benzonitril (Verbindung 86);
5-Benzo[1,3]dioxol-5-yl-4-o-tolyl-1H-pyrazolo[3,4-c]pyridazin-3-yl-amin (Verbindung 87);
5-(3-Chlorphenyl)-4-o-tolyl-1H-pyrazolo[3,4-c]pyridazin-3-yl-amin (Verbindung 88);
4-(3-Amino-4-o-tolyl-1H-pyrazolo[3,4-c]pyridazin-5-yl)benzamid (Verbindung 89);
5-(3-Nitrophenyl)-4-o-tolyl-1H-pyrazolo[3,4-c]pyridazin-3-yl-amin (Verbindung 90);
3-(3-Amino-4-o-tolyl-1H-pyrazolo[3,4c]pyridazin-5-yl)benzamid (Verbindung 91);
4-o-Tolyl-5-p-tolyl-1H-pyrazolo[3,4c]pyridazin-3-yl-amin (Verbindung 92);
5-(3-Phenoxyphenyl)-4-o-tolyl-1H-pyrazolo[3,4-c]pyridazin-3-yl-amin (Verbindung 93);
4-(3-Amino-4-o-tolyl-1H-pyrazolo[3,4-c]pyridazin-5-yl)benzonitril (Verbindung 94);
5-Benzo[1,3]dioxol-5-yl-4-phenyl-1H-pyrazolo[3,4-c]pyridazin-3-yl-amin (Verbindung 95);
5-(3-Chlorphenyl)-4-phenyl-1H-pyrazolo[3,4-c]pyridazin-3-yl-amin (Verbindung 96);
5-(3-Nitrophenyl)-4-phenyl-1H-pyrazolo[3,4-c]pyridazin-3-yl-amin (Verbindung 97);
3-(3-Amino-4-phenyl-1H-pyrazolo[3,4-c]pyridazin-5-yl)benzamid (Verbindung 98);
5-(4-tert-Butylphenyl)-4-phenyl-1H-pyrazolo[3,4-c]pyridazin-3-yl-amin (Verbindung 99);
5-(3-Phenoxyphenyl)-4-phenyl-1H-pyrazolo[3,4-c]pyridazin-3-yl-amin (Verbindung 100);
4-Cyclohexyl-5-phenyl-1H-pyrazolo[3,4-c]pyridazin-3-yl-amin (Verbindung 101); und
4-(2-Methylsulfanylpyrimidin-4-yl)-5-phenyl-1H-pyrazolo[3,4-c]pyridazin-3-yl-amin (Verbindung 102).

12. Verbindung gemäß Anspruch 11, wobei die Verbindung
3-Amino-4-phenyl-5-(3-fluorphenyl)-1H-pyrazolo[3,4-c]pyridazin (Verbindung 8) ist.

13. Arzneimittel, umfassend eine Verbindung gemäß einem der Ansprüche 1 bis 12 und einen pharmazeutisch verträglichen Träger, ein pharmazeutisch verträgliches Hilfsmittel oder Vehikel.

14. Verwendung von:
(a) einer Verbindung der Formel I: 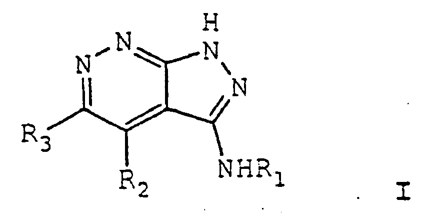 oder eines pharmazeutisch verträglichen Salzes davon, wobei:
R₁ ausgewählt ist aus H; einem Aliphat ausgewählt aus linear oder verzweigt: C₁-C₁₂-Alkyl, C₂-C₁₂-Alkenyl und C₂-C₁₂-Alkinyl; RC(O); RS(O)ₙ; ROC(O); C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl;
wobei das Aliphat, Carbocyclyl, Heterocyclyl, Aryl, Aralkyl, Heteroaryl oder Heteroaralkyl gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann;
R₂ und R₃ jeweils unabhängig voneinander ausgewählt sind aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzothiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl; -N(R)₂; -NRCOR; -NRCO₂R; -NRSO₂R; -S(O)ₙR; -SO₂N(R)₂; -SR; -OR; -CF₃; Halo; -NO₂; -CN; -C(O)R; -CO₂R; -OC(O)R; -CON(R)₂; oder -OC(O)N(R)₂; wobei das Aliphat, Carbocyclyl, Heterocyclyl, Aryl, Aralkyl, Heteroaryl oder Heteroaralkyl gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann; oder wobei R₂ und R₃ zusammen mit den dazwischen liegenden Atomen gegebenenfalls einen fünf- bis neungliedrigen Ring bilden, welcher an den Pyridazinylring der Formel I kondensiert ist, wobei der kondensierte Ring 0 bis 2 Heteroatome aufweist;
R ausgewählt ist aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pymolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; Heteroaralkinyl;
wobei jeder Bestandteil von R außer H gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann;
R' unabhängig ausgewählt ist aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl; und jeder Rest R' gegebenenfalls mit Halogen, Nitro, Cyano, Amino, -NH-(unsubstituiertem Aliphat), -N-(unsubstituiertem Aliphat)₂, Carboxy, Carbamoyl, Hydroxy, -O-(unsubstituiertem Aliphat), -SH, -S-(unsubstituiertem Aliphat), CF₃, -SO₂NH₂, unsubstituiertem Aliphat, unsubstituiertem Carbocyclyl, unsubstituiertem Heterocyclyl, unsubstituiertem Aryl, unsubstituiertem Aralkyl, unsubstituiertem Heteroaryl oder unsubstituiertem Heteroaralkyl substituiert ist; und
n gleich 1 oder 2 ist; oder
(b) einem Arzneimittel, umfassend diese Verbindung und einen pharmazeutisch verträglichen Träger, ein pharmazeutisch verträgliches Hilfsmittel oder Vehikel; für die Herstellung eines Medikaments zur Hemmung von GSK-3-Aktivität bei einem Patienten.

15. Verbindung der Formel I: 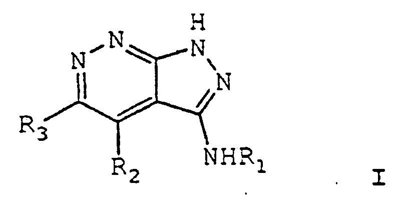 oder ein pharmazeutisch verträgliches Salz davon, wobei:
R₁ ausgewählt ist aus H; einem Aliphat ausgewählt aus linear oder verzweigt: C₁-C₁₂-Alkyl, C₂-C₁₂-Alkenyl und C₂-C₁₂-Alkinyl; RC(O); RS(O)ₙ; ROC(O); C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-tetrazolyl, 2-Triazolyl, 5-Triazolyl, 2-Thienyl und 3-Thienyl; Heteroaralkyl; Heteroaralkenyl; oder Heteroaralkinyl;
wobei das Aliphat, Carbocyclyl, Heterocyclyl, Aryl, Aralkyl, Heteroaryl oder Heteroaralkyl gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')_{2,} -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann;
R₂ und R₃ jeweils unabhängig voneinander ausgewählt sind aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzothiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl; -N(R)₂; -NRCOR; -NRCO₂R; -NRSO₂R; -S(O)ₙR; -SO₂N(R)₂; -SR; -OR; -CF₃; Halo; -NO₂; -CN; -C(O)R; -CO₂R; -OC(O)R; -CON(R)₂; oder -OC(O)N(R)₂; wobei das Aliphat, Carbocyclyl, Heterocyclyl, Aryl, Aralkyl, Heteroaryl oder Heteroaralkyl gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')_{2,} -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann; oder wobei R₂ und R₃ zusammen mit den dazwischen liegenden Atomen gegebenenfalls einen fünf- bis neungliedrigen Ring bilden, welcher an den Pyridazinylring der Formel I kondensiert ist, wobei der kondensierte Ring 0 bis 2 Heteroatome aufweist;
R ausgewählt ist aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; Heteroaralkinyl; wobei jeder Bestandteil von R außer H gegebenenfalls mit Halogen, CF₃, -R', -OR', -OH, -SH, -SR', Acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H oder -NHS(O)₂R' substituiert ist; und wobei das Aliphat, Carbocyclyl oder Heterocyclyl weiterhin gegebenenfalls mit =O, =S, =NNHR', =NN(R')₂, =N-OR', =NNHCOR', =NNHCO₂R', =NNHSO₂R', =N-CN oder =NR' substituiert sein kann;
R' unabhängig ausgewählt ist aus H; einem Aliphat ausgewählt aus linear oder verzweigt: C₁-C₁₂-Alkyl, C₂-C₁₂-Alkenyl und C₂-C₁₂-Alkinyl; C₃-C₁₂-Carbocyclyl; Heterocyclyl ausgewählt aus 3-1H-Benzimidazol-2-on, 3-1H-Alkylbenzimidazol-2-on, 2-Tetrahydrofuranyl, 3-Tetrahydrofuranyl, 2-Tetrahydrothiophenyl, 3-Tetrahydrothiophenyl, 2-Morpholino, 3-Morpholino, 4-Morpholino, 2-Thiomorpholino, 3-Thiomorpholino, 4-Thiomorpholino, 1-Pyrrolidinyl, 2-Pyrrolidinyl, 3-Pyrrolidinyl, 1-Piperazinyl, 2-Piperazinyl, 1-Piperidinyl, 2-Piperidinyl, 3-Piperidinyl, 4-Piperidinyl, 4-Thiazolidinyl, Diazolonyl, N-substituiertes Diazolonyl, 1-Phthalimidinyl, Benzoxan, Benzotriazol-1-yl, Benzpyrrolidin, Benzopiperidin, Benzoxolan, Benzthiolan, Benzothian, Aziranyl, Oxiranyl, Azetidinyl, Pyrrolinyl, Dioxolanyl, Imidazolinyl, Imidazolidinyl, Pyrazolinyl, Pyrazolidinyl, Pyranyl, Dioxanyl, Dithianyl, Trithianyl, Chinuclidinyl, Oxepanyl und Thiepanyl; Aryl ausgewählt aus Phenyl, 1-Naphthyl, 2-Naphthyl, 1-Anthracyl und 2-Anthracyl, Tetrahydronaphthyl; Aralkyl; Aralkenyl; Aralkinyl; Heteroaryl ausgewählt aus Benzimidazolyl, Benzothienyl, Benzofuranyl, Indolyl, Chinolinyl, Benzothiazolyl, Benzoxazolyl, Benzimidazolyl, Isochinolinyl, Isoindolyl, Acridinyl, Benzisoxazolyl, 2-Furanyl, 3-Furanyl, N-Imidazolyl, 2-Imidazolyl, 4-Imidazolyl, 5-Imidazolyl, 3-Isoxazolyl, 4-Isoxazolyl, 5-Isoxazolyl, 2-Oxadiazolyl, 5-Oxadiazolyl, 2-Oxazolyl, 4-Oxazolyl, 5-Oxazolyl, 2-Pyrrolyl, 3-Pyrrolyl, 2-Pyridyl, 3-Pyridyl, 4-Pyridyl, 2-Pyrimidyl, 4-Pyrimidyl, 5-Pyrimidyl, 3-Pyridazinyl, 2-Thiazolyl, 4-Thiazolyl, 5-Thiazolyl, 5-Tetrazolyl, 2-Triazolyl, 5-Triazolyl, 2-Thienyl und 3-Thienyl; Heteroaralkyl; Heteroaralkenyl; oder Heteroaralkinyl; und jeder Rest R' gegebenenfalls mit Halogen, Nitro, Cyano, Amino, -NH-(unsubstituiertem Aliphat), -N-(unsubstituiertem Aliphat)₂, Carboxy, Carbamoyl, Hydroxy, -O-(unsubstituiertem Aliphat), -SH, -S-(unsubstituiertem Aliphat), CF₃, -SO₂NH₂, unsubstituiertem Aliphat, unsubstituiertem Carbocyclyl, unsubstituiertem Heterocyclyl, unsubstituiertem Aryl, unsubstituiertem Aralkyl, unsubstituiertem Heteroaryl oder unsubstituiertem Heteroaralkyl substituiert ist; und
n gleich 1 oder 2 ist; oder
ein Arzneimittel, umfassend diese Verbindung und einen pharmazeutisch verträglichen Träger, ein pharmazeutisch verträgliches Hilfsmittel oder Vehikel;
zur Verwendung bei der Hemmung von GSK-3-Aktivität bei einem Patienten.

16. Verwendung gemäß Anspruch 14 zur Steigerung der Glycogensynthese oder zur Senkung des Glucosespiegels im Blut bei einem Patienten, der dieser Behandlung bedarf.

17. Verwendung gemäß Anspruch 16, wobei der Patient gegen Diabetes behandelt wird.

18. Verwendung gemäß Anspruch 14 zur Hemmung der Produktion von hyperphosphoryliertem Tau-Protein bei einem Patienten, der dieser Behandlung bedarf.

19. Verwendung gemäß Anspruch 18, wobei der Patient gegen die Alzheimer-Erkrankung behandelt wird.

20. Verwendung gemäß Anspruch 14 zur Hemmung der Phosphorylierung von β-Catenin bei einem Patienten, der dieser Behandlung bedarf.

21. Verwendung gemäß Anspruch 20, wobei der Patient gegen Schizophrenie behandelt wird.

22. Verwendung gemäß einem der Ansprüche 14, 16, 18 und 20, wobei die Verbindung oder Zusammensetzung mit einem zusätzlichen Therapeutikum verabreicht wird.

23. Verbindung oder Zusammensetzung gemäß Anspruch 15 zur Verwendung bei der Steigerung der Glycogensynthese oder der Senkung des Glucosespiegels im Blut bei einem Patienten, der dieser Behandlung bedarf.

24. Verbindung oder Zusammensetzung gemäß Anspruch 23, wobei der Patient gegen Diabetes behandelt wird.

25. Verbindung oder Zusammensetzung gemäß Anspruch 15 zur Verwendung bei der Hemmung der Produktion von hyperphosphoryliertem Tau-Protein bei einem Patienten, der dieser Behandlung bedarf.

26. Verbindung oder Zusammensetzung gemäß Anspruch 25, wobei der Patient gegen die Alzheimer-Erkrankung behandelt wird.

27. Verbindung oder Zusammensetzung gemäß Anspruch 15 zur Verwendung bei der Hemmung der Phosphorylierung von β-Catenin bei einem Patienten, der dieser Behandlung bedarf.

28. Verbindung oder Zusammensetzung gemäß Anspruch 27, wobei der Patient gegen Schizophrenie behandelt wird.

29. Verbindung oder Zusammensetzung gemäß einem der Ansprüche 15, 23, 25 und 27,
wobei die Verbindung oder Zusammensetzung mit einem zusätzlichen Therapeutikum verabreicht wird.

30. In vitro-Verfahren zur Hemmung von GSK-3-Aktivität in einer biologischen Probe, umfassend den Schritt des Inkontaktbringens der biologischen Probe mit einer Verbindung gemäß Anspruch 14 oder einer Zusammensetzung umfassend diese Verbindung und einen Träger, Hilfsmittel oder Vehikel.

## Revendications

1. Composé de la formule I: 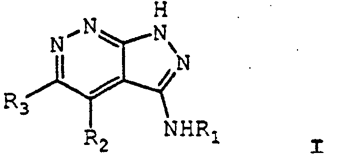 ou un sel pharmaceutiquement acceptable de celui-ci,
où:
R₁ est sélectionné parmi H; aliphatique sélectionné parmi linéaire ou branché: alkyle C₁-C₁₂, alcényle C₂-C₁₂ et alkynyle C₂-C₁₂; RC(O)-; RS(O)ₙ-; ROC(O); carbocyclyle C₃-C₁₂; hétérocyclyle sélectionné parmi 3-1H-benzimidazol-2-one, 3-1H- alkyl-benzimidazol-2-one, 2-tétrahydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyl, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphthyle, 2-naphthyle, 1-anthracyle et 2-anthracyle, tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle, et 3-thiényle; hétéroaralkyle; hétéroaralcényle; ou hétéroaralkynyle; où ledit aliphatique, carbocyclyle, hétérocyclyle, aryle, aralkyle, hétéroaryle ou hétéroaralkyle est facultativement substitué par halogène, CF₃, -R', -OR', -OH, -SH, -SR', acyloxy, -NO₂, -CN, -NH₂, -NHR', -N(R')₂, -NHCOR', -NHCONH₂, -NHCONHR', -NHCON(R')₂, -NRCOR', -NHCO₂H, - NHCO₂R', -CO₂R' , -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H ou -NHS(O)₂R'; et où ledit aliphatique, carbocyclyle ou hétérocyclyle peut en outre être facultativement substitué par =O, =S, =NNHR', =NN(R')₂, =N-OR', =NNHCOR', =NNHCO₂R', =NNHSO₂R', =N-CN ou =NR';
R₂ et R₃ sont chacun sélectionnés indépendamment parmi H; aliphatique sélectionné parmi linéaire ou branché:alkyle C₁-C₁₂, alcényle C₂-C₁₂ et alkynyle C₂-C₁₂; carbocyclyle C₃-C₁₂, hétérocyclyle sélectionné parmi 3-1H-benzimidazol-2-one, 3-1H-alkyl-benzimidazol-2-one, 2-tétrahydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphthyle, 2-naphthyle, 1-anthracyle et 2-anthracyle, tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle et 3-thiényle; hétéroaralkyle; hétéroaralcényle ou hétéroaralkynyle; -N(R)₂; -NRCOR; -NRCO₂R; -NRSO₂R; -S(O)ₙR; -SO₂N(R)₂; -SR; -OR; -CF₃; halo; -NO₂; -CN; -C (O) R; -CO₂R; -OC(O)R; -CON(R)₂; ou -OC(O)N(R)₂; où ledit aliphatique, carbocyclyle, hétérocyclyle, aryle, aralkyle, hétéroaryle ou hétéroaralkyle est facultativement substitué par halogène, CF₃, -R', -OR', -OH, -SH, -SR', acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR' , -CONH₂, -CONHR' , -CON(R')₂, -S(O)₂H, -S(O)₂R', -SO₂NH₂, S(O)H, -S(O)R', -SO₂NHR', -SO₂N(R')₂, -NHS(O)₂H ou -NHS(O)₂R'; et où ledit aliphatique, carbocyclyle ou hétérocyclyle peut en outre être facultativement substitué par =O, =S, =NNHR', =NN(R')₂, =N-OR', =NNHCOR', =NNHCO₂R', =NNHSO₂R', =N-CN ou =NR'; ou bien où R₂ and R₃ pris ensemble avec les atomes intervenants forment optionnellement un cycle de cinq à neuf membres qui est condensé au cycle de pyradazinyle de la formule I, ledit cycle condensé ayant 0-2 hétéroatomes;
R est sélectionné parmi H; aliphatique sélectionné parmi linéaire ou branché: alkyle C₁-C₁₂, alcényle C₂-C₁₂ et alkynyle C₂-C₁₂; carbocyclyle C₃-C₁₂; hétérocyclyle sélectionné parmi 3-1H-benzimidazol-2-one, 3-1H-alkyl-benzimidazol-2-one, 2-tétra-hydrofuranyle, 3-tétrahydro-furanyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle, et thiépanyle; aryle sélectionné parmi phényle, 1-naphthyle, 2-naphthyle, 1-anthracyle et 2-anthracyle, tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle; 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle et 3-thiényle; hétéroaralkyle; hétéroaralcényle ou hétéroaralkynyle; où chaque membre de R excepté H est facultativement substitué par halogène, CF₃, -R', -OR', -OH, -SH, -SR', acyloxy, -NO₂, -CN, -NH₂, -NHR', -N(R')₂, -NHCOR', -NHCONH₂, -NHCONHR', -NHCON(R')₂, -NRCOR', -NHCO₂H, - NHCO₂R', -CO₂R', -CO₂H, -COR', -CONH₂, -CONHR' , -CON(R')₂. - S(O)₂H, -S(O)₂R', -SO₂NH₂, -S(O)H, -S(O)R', -SO₂NHR', - SO₂N(R')₂, -NHS(O)₂H ou -NHS(O)₂R'; et où ledit aliphatique, carbocyclyle ou hétérocyclyle peut en outre être facultativement substitué par =O, =S, =NNHR',=NN(R')2, =N- OR', =NNHCOR', =NNHCO₂R', =NNHSO₂R', =N-CN ou =NR'; et
n est 1 ou 2;
R' est indépendamment sélectionné parmi H; aliphatique sélectionné parmi linéaire ou branché; alkyleC₁-C₁₂, alcényleC₂-C₁₂ et alkynyleC₂-C₁₂; carbocyclyle C₃-C₁₂; hétérocyclyle sélectionné parmi 3-1H-benzimidazol-2-one, 3-1H-alkyl-benzimidazol-2-one, 2-tétrahydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphtyle, 2-naphtyle, 1-anthracyle et 2-anthracyle, tétrahydronaphtyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tetrazolyle, 2-triazolyle, 5-triazolyle, 2-thiényle et 3-thiényle; hétéroaralkyle; hétéroaralcényle ou hétéroaralkynyle; et chaque R' est facultativement substitué par halogène, nitro, cyano, amino, -NH-(aliphatique non substitué), -N-(aliphatique non substitué)₂, carboxy, carbamoyle, hydroxy, -0-(aliphatique non substitué), -SH, -S-(aliphatique non substitué), CF₃, -SO₂NH₂, aliphatique non substitué, carbocyclyle non substitué, hétérocyclyle non substitué, aryle non substitué, aralkyle non substitué, hétéroaryle non substitué ou hétéroaralkyle non substitué;
à condition que lorsque R₁ est H, R₂ et R₃ ne soient pas tous les deux phényle non substitué; et lorsque R₁ est H, R₂ et R₃ sont autres que H, halogène ou un alkyle non substitué.

2. Composé selon la revendication 1, où R₁ est H, RC(O)-, aralkyle, aralcényle ou aralknyle.

3. Composé selon la revendication 2, où R est aliphatique sélectionné parmi linéaire ou branché; alkyle C₁-C₁₂ , alcényle C₂-C₁₂ et alkynyle C₂₋₁₂ ou aryle sélectionné parmi phényle, 1-naphtyle, 2-naphtyle, 1-anthracyle et 2-anthracyle, tétrahydronaphtyle.

4. Composé selon la revendication 2, où R₁ est H, CH₃C(O) -, PhC(O)- ou PhCH₂-.

5. Composé selon la revendication 1, où R₂ et R₃ sont indépendamment H: aryle sélectionné parmi phényle, 1-naphthyle, 2-naphthyle, 1-anthracyle et 2-anthracyl, tétrahydronaphthyle; carbocyclyleC₃-C₁₂; hétérocyclyle sélectionné parmi 3-1H-benzimidazol-2-one, 3-1H-alkyl-benzimidazol-2-one, 2-tétrahydrofuranyle, 3-tétrahydro-furanyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolinyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle, et thiépanyle; ou hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle et 3-thiényle.

6. Composé selon la revendication 5, où R₂ et R₃ sont indépendamment H, phényle, naphtyle, pyridyle, thiényle, furanyle, pyrimidinyle, benzodioxolyle, ou cyclohexyle, l'un quelconque de ceux-ci, excepté H, est facultativement substitué par halogène, CF₃, -R', -OR', -OH, -SH, -SR', acyloxy, -NO₂, -CN, -NH₂, -NHR', -N(R')₂, -NHCOR', - NHCONH₂, -NHCONHR', -NHCON(R')2_{,} -NRCOR', -NHCO₂H, - NHCO₂R', -CO₂R', -CO₂H, -COR', -CONH₂, -CONHR' , -CON(R')₂, -S(O)₂H, -S(O)₂R', -SO₂NH₂, -S(O)H, -S(O)R', -SO₂NHR' , -SO₂N(R')₂, -NHS(O)₂H, ou -NHS(O)₂R'; et où ledit aliphatique, carbocyclyle ou hétérocyclyle peut en outre être facultativement substitué par =O, =S, =NNHR', =NN(R')₂, =N-OR', =NNHCOR', =NNHCO₂R', =NNHSO₂R', =N-CN ou =NR'.

7. Composé selon la revendication 6, où les substituants sont sélectionnés parmi halo, alkyle, -CN, -NO₂, -SO₂NH₂, -SO₂NH-(alkyle), -SO₂N (alkyle)₂, -O-alkyle, -NH₂, -N-alkyle, -N-(alkyle)₂, -CONH₂, -CONH(alkyle), -CONH(alkyle)₂, -O-phényle ou -S-alkyle.

8. Composé selon la revendication 1, ayant la formule Ia: 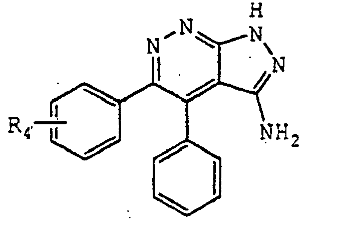 où R₄ est halo.

9. Composé selon la revendication 8, où R₄ is F.

10. Composé selon la revendication 1, où R₁ est H, R₂ et R₃ sont indépendamment phényle, facultativement substitué par CF₃, -R', -OR', -OH, -SH, -SR', acyloxy, -NO₂, -CN, -NH₂, -NHR', -N(R')₂, -NHCOR', -NHCONH₂, -NHCONHR', -NHCON(R')₂, -NRCOR', -NHCO₂H, - NHCO₂R', -CO₂R', -CO₂H, -COR' , -CONH₂, -CONHR' , -CON(R')₂, -S(O)₂H, -S(O)₂R', -SO₂NH₂, -S(O)H, -S(O)R', -SO₂NHR', -SO₂N(R')₂, -NHS(O)₂H ou -NHS(O)₂R';
à condition que R₂ et R₃ ne soient pas tous les deux phényle non substitué.

11. Composé selon la revendication 1, sélectionné dans le groupe consistant en:
3-amino-4(4-chlorophényle)-5-phényl-1H-pyrazolo[3,4-c]pyridazine (Composé 2);
3-amino-4,5-bis(4-fluorophényl)-1H-pyrazolo[3,4-c] pyridazine (Composé 3);
3-amino-4-phényl-5-(4-fluorophényl)-1H-pyrazolo[3,4-c]pyridazine (Composé 4);
3-amino-4-(4-fluorophényl)-5-phényl-1H-pyrazolo[3,4-c] pyridazine (Composé 5);
3-amino-4-(3-fluorophényl)-5-phényl-1H-pyrazolo[3,4-c] pyridazine (Composé 6);
3-amino-4-phényl-5-(4-pyridyl)-1H-pyrazolo[3,4-c] pyridazine (Composé 7);
3-amino-4-phényl-5-(3-fluorophényl)-1H-pyrazolo[3,4-c]pyridazine (Composé 8);
N-(4,5-diphényl-1H-pyrazolo[3,4-c]pyridazin-3-yl)-acetamide (Composé 9);
N-(4,5-diphényl-1H-pyrazolo[3,4-c]pyridazin-3-yl)-benzamide (Composé 10);
3-amino-4-phényl-5-(4-méthyl-phényl)-1H-pyrazolo[3,4-c]pyridazine (Composé 11);
3-amino-4-phényl-5-(2-méthyl-phényl)-1H-pyrazolo[3,4-c]pyridazine (Composé 12);
3-amino-4-phényl-5-(3-méthyl-phényl)-1H-pyrazolo[3,4-c]pyridazine (Composé 13);
3-amino-4-phényl-5-(2-chloro-phényl)-1H-pyrazolo[3,4-c] pyridazine (Composé 14);
3-amino-4-phényl-5-(2-fluorophényl)-1H-pyrazolo[3,4-c]pyridazine(Composé 15);
3-amino-4-phényl-5-(4-chloro-phényl)-1H-pyrazolo[3,4-c]pyridazine (Composé 16);
3-amino-4(2-cyanophényl)-5-phényl-1H-pyrazolo[3,4-c]pyridazine(Composé 17);
3-amino-4(3-cyanophényl)-5-phényl-1H-pyrazolo[3,4-c]pyridazine (Composé 18);
3-amino-4(4-cyanophényl)-5-phényk-1H-pyrazolo[3,4-c]pyridazine (Composé 19);
3-amino-4-phényl-5-(2-cyanophényl)-1H-pyrazolo[3,4-c]pyridazine (Composé 20);
3-amino-4-phényl-5-(3-cyanophényl)-1H-pyrazolo[3,4-c] pyridazine (Composé 21);
3-amino-4-phényl-5-(4-cyanophényl)-1H-pyrazolo[3,4-c] pyridazine (Composé 22);
3-amino-4-phényl-5-(2-pyridyl)-1H-pyrazolo[3,4-c] pyridazine (Composé 23);
3-amino-4-phényl-5-(3-pyridyl)-1H-pyrazolo[3,4-c] pyridazine (Composé 24);
N-(4,5-diphényl-1H-pyrazolo[3,4-c]pyridazin-3-yl)-benzylamine (Composé 25);
3-amino-4(2-pyridyl)-5-phényl-1H-pyrazolo[3,4-c] pyridazine (Composé 26);
3-amino-4(3-pyridyl)-5-phényl-1H-pyrazolo[3,4-c] pyridazine (Composé 27);
3-amino-4(4-pyridyl)-5-phényl-1H-pyrazolo[3,4-c] pyridazine (Composé 28);
3-amino-4-phényl-5-(2-thiényl)-1H-pyrazolo[3,4-c] pyridazine (Composé 29);
3-amino-4(2-furanyl)-5-phényl-1H-pyrazolo[3,4-c] pyridazine (Composé 30);
3-amino-4-phényl-5-naphthyl-1H-pyrazolo[3,4-c] pyridazine (Composé 31);
5-phényl-4-o-tolyl-1H-pyrazolo[3,4-c]pyridazin-3-ylamine (Composé 35);
5-phényl-4-m-tolyl-1H-pyrazolo[3,4-c]pyridazin-3-ylamine (Composé 36);
5-phényl-4-p-tolyl-1H-pyrazolo[3,4-c]pyridazin-3-ylamine (Composé 37);
4-(4-nitro-phényl)-5-phényl-1H-pyrazolo[3,4-c]pyridazin-3-ylamine (Composé 38);
4-(3-amino-5-phényl-1H-pyrazolo[3,4-c]pyridazin-4-yl)-benzènesulfonamide (Composé 39);
4-(3-fluoro-4-méthyl-phényl)-5-phényl-1H-pyrazolo[3,4-c]pyridazin-3-ylamine (Composé 40);
4-(3-amino-4-phényl-1H-pyrazolo[3,4-c]pyridazin-5-yl)-benzamide (Composé 41);
5-(2-amino-phényl)-4-phényl-1H-pyrazolo[3,4-c]pyridazin-3-ylamine (Composé 42);
4-(4-éthyl-phényl)-5-phényl-1H-pyrazolo[3,4-c]pyridazin-3-ylamine (Composé 43);
4-(2,6-difluoro-phényl-5-phényl-1H-pyrazolo[3,4-c]pyridazin-3-ylamine (Composé 44);
4-(3,4-diméthoxy-phényl)-5-phényl-1H-pyrazolo[3,4-c]pyridazin-3-ylamine (Composé 45);
4-(3-fluoro-phényl)-5-phényl-1H-pyrazolo[3,4-c]pyridazin-3-ylamine (Composé 46);
4-(3-nitro-phényl-5-phényl-1H-pyrazolo[3,4-c]pyridazin-3-ylamine (Composé 47);
5-(2-chloro-phényl)-4-(2,6-difluoro-phényl)-1H-pyrazolo[3,4-c]pyridazin-3-ylamine (Composé 48);
5-(2-chloro-phényl)-4-(3,4-diméthoxy-phényl)-1H-pyrazolo[3,4-c]pyridazin-3-ylamine (Composé 49);
5-(2-chloro-phényl)-4-(3-fluoro-phényl)-1H-pyrazolo[3,4-c]pyridazin-3-ylamine (Composé 50);
5-(2-chloro-phényl)-4-(4-diméthylamino-phényl)-1H-pyrazolo[3,4-c]pyridazin-3-ylamine (Composé 51);
4-[3-amino-5-(2-chloro-phényl)-1H-pyrazolo[3,4-c]pyridazin-4-yl]-benzonitrile (Composé 52);
5-(2-chloro-phényl)-4-pyridin-4-yl-1H-pyrazolo[3,4-c]pyridazin-3-ylamine (Composé 53);
5-(2-chloro-phényl)-4-(3-nitro-phényl)-1H-pyrazolo[3,4-c]pyridazin-3-ylamine (Composé 54);
5-(2-chloro-phényl)-4-pyridin-3-yl-1H-pyrazolo[3,4-c]pyridazin-3-ylamine (Composé 55);
4-(2,6-difluoro-phényl)-5-(3-méthoxy-phényl)-1H-pyrazolo[3,4-c]pyridazin-3-ylamine (Composé 56);
4-(3,4-diméthoxy-phényl)-5-(3-méthoxy-phényl)-1H-pyrazolo[3,4-c]pyridazin-3-ylamine (Composé 57);
4-(3-fluoro-phényl)-5-(3-méthoxy-phényl)-1H-pyrazolo[3,4-c]pyridazin-3-ylamine (Composé 58);
4-(4-diméthylamino-phényl)-5-(3-méthoxy-phényl)-1H-pyrazolo[3,4-c]pyridazin-3-ylamine (Composé 59);
4-[3-amino-5-(3-méthoxy-phényl)-1H-pyrazolo[3,4-c] pyridazin-4-yl]-benzonitrile (Composé 60);
5-(3-méthoxy-phényl)-4-pyridin-4-yl-1H-pyrazolo[3,4-c]pyridazin-3-ylamine (Composé 61);
5-(3-méthoxy-phényl)-4-(3-nitro-phényl)-1H-pyrazolo[3,4-c]pyridazin-3-ylamine (Composé 62);
5-(3-méthoxy-phényl)-4-pyridin-3-yl-1H-pyrazolo[3,4-c]pyridazin-3-ylamine (Composé 63);
4-(2,6-difluoro-phényl)-5-p-tolyl-1H-pyrazolo[3,4-c]pyridazin-3-ylamine (Composé 64);
4-(3,4-diméthoxy-phényl)-5-p-tolyl-1H-pyrazolo[3,4-c]pyridazin-3-ylamine (Composé 65);
4-(3-fluoro-phényl)-5-p-tolyl-1H-pyrazolo[3,4-c]pyridazin-3-ylamine (Composé 66);
4-(4-diméthylamino-phényl)-5-p-tolyl-1H-pyrazolo[3,4-c]pyridazin-3-ylamine (Composé 67);
4-(3-amino-5-p-tolyl-1H-pyrazolo[3,4-c]pyridazin-4-yl)-benzonitrile (Composé 68);
4-pyridin-4-yl-5-p-tolyl-1H-pyrazolo[3,4-c]pyridazin-3-ylamine (Composé 69);
4-(3-nitro-phényl)-5-p-tolyl-1H-pyrazolo[3,4-c]pyridazin-3-ylamine (Composé 70);
4-pyridin-3-yl-5-p-tolyl-1H-pyrazolo[3,4-c]pyridazin-3-ylamine (Composé 71);
3-(3-amino-5-benzo[1,3]dioxol-5-yl-1H-pyrazolo[3,4-c]pyridazin-4-yl)-benzonitrile (Composé 72);
3-[3-amino-5-(3-chloro-phényl)-1H-pyrazolo[3,4-c] pyridazin-4-yl]-benzonitrile (Composé 73);
4-[3-amino-4-(3-cyano-phényl)-1H-pyrazolo[3,4-c]pyridazin-5-yl]-benzamide (Composé 74);
3-[3-amino-5-(3-nitro-phényl)-1H-pyrazolo[3,4-c]pyridazin-4-yl]-benzonitrile (Composé 75);
3-[3-amino-4-(3-cyano-phényl)-1H-pyrazolo[3,4-c] pyridazin-5-yl]-benzamide (Composé 76);
3-(3-amino-5-p-tolyl-1H-pyrazolo[3,4-c]pyridazin-4-yl)-benzonitrile (Composé 77);
3-[3-amino-5-(3-phénoxy-phényl)-1H-pyrazolo[3,4-c] pyridazin-4-yl]-benzonitrile (Composé 78);
4-[3-amino-4-(3-méthoxy-phényl)-1H-pyrazolo[3,4-c] pyridazin-5-y1]-benzonitrile (Composé 79);
5-benzo[1,3]dioxol-5-yl-4-(4-chloro-phényl)-1H-pyrazolo[3,4-c]pyridazin-3-ylamine (Composé 80);
4-(4-chloro-phényl)-5-m-tolyl-1H-pyrazolo[3,4-c]pyridazin-3-ylamine (Composé 81);
4-[3-amino-4-(4-chloro-phényl)-1H-pyrazolo[3,4-c]pyridazin-5-yl]-benzamide (Composé 82);
4-(4-chloro-phényl)-5-(3-nitro-phényl)-1H-pyrazolo[3,4-c]pyridazin-3-ylamine (Composé 83);
3-[3-amino-4-(4-chloro-phényl)-1H-pyrazolo[3,4-c]pyridazin-5-yl]-benzamide (Composé 84);
4-(4-chloro-phényl)-5-(3-phénoxy-phényl)-1H-pyrazolo[3,4-c]pyridazin-3-ylamine (Composé 85);
4-[3-amino-4-(4-chloro-phényl)-1H-pyrazolo[3,4-c] pyridazin-5-yl)-benzonitrile (Composé 86);
5-benzo[1,3]dioxol-5-yl-4-o-tolyl-1H-pyrazolo[3,4-c] pyridazin-3-ylamine (Composé 87);
5-(3-chloro-phenyl)-4-o-tolyl-1H-pyrazolo[3,4-c] pyridazin-3-ylamine (Composé 88);
4-(3-amino-4-o-tolyl-1H-pyrazolo[3,4-c]pyridazin-5-yl)-benzamide (Composé 89);
5-(3-nitro-phényl)-4-o-tolyl-1H-pyrazolo[3,4-c] pyridazin-3-ylamine (Composé 90);
3-(3-amino-4-o-tolyl-1H-pyrazolo[3,4-c]pyridazin-5-yl)-benzamide (Composé 91);.
4-o-tolyl-5-p-tolyl-1H-pyrazolo[3,4-c]pyridazin-3-ylamine (Composé 92);
5-(3-phénoxy-phényl)-4-o-tolyl-1H-pyrazolo[3,4-c] pyridazin-3-ylamine (Composé 93);
4-(3-amino-4-o-tolyl-1H-pyrazolo[3,4-c]pyridazin-5-yl)-benzonitrile (Composé 94);
5-benzo[1,3]dioxol-5-yl-4-phényl-1H-pyrazolo [3,4-c]pyridazin-3-ylamine (Composé 95);
5-(3-chloro-phényl)-4-phényl-1H-pyrazolo[3,4-c] pyridazin-3-ylamine (Composé 96);
5-(3-nitro-phényl)-4-phényl-1H-pyrazolo[3,4-c] pyridazin-3-ylamine (Composé 97);
3-(3-amino-4-phényl-1H-pyrazolo[3,4-c]pyridazin-5-yl)-benzamide (Composé 98);
5-(4-tert-butyl-phényl)-4-phényl-1H-pyrazolo [3,4-c]pyridazin-3-ylamine (Composé 99);
5-(3-phénoxy-phényl)-4-phényl-1H-pyrazolo [3,4-c]pyridazin-3-ylamine -(Composé 100);
4-cyclohexyl-5-phényl-1H-pyrazolo[3,4-c] pyridazin-3-ylamine (Composé 101); et
4-(2-Méthylsulfanyl-pyrimidin-4-yl)-5-phényl-1H-pyrazolo[3,4-c]pyridazin-3-ylamine (Composé 102).

12. Composé selon la revendication 11, où le composé est:
3-amino-4-phényl-5-(3-fluorophényl)-1H-pyrazolo[3,4-c] pyridazine (Composé 8).

13. Composition pharmaceutique comprenant un composé selon l'une quelconque des revendications 1-12 et un support, adjuvant ou véhicule pharmaceutiquement acceptable.

14. Utilisation de:
(a) un composé de la formule I: 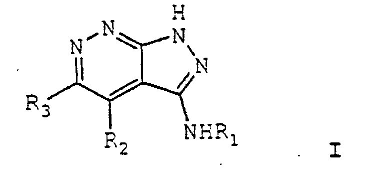 ou un sel pharmaceutiquement acceptable de celui-ci, où:
R₁ est sélectionné parmi H; aliphatique sélectionné parmi linéaire ou branché: alkyleC₁-C₁₂, alcényleC₂-C₁₂ et alkynyleC₂-C₁₂; RC(O) ; RS(O)ₙ; ROC (O) ; carbocyclyleC₃-C₁₂; hétérocyclyle sélectionné parmi 3-1H-benzimidazol-2-one, 3-1H-alkyl-benzimidazol-2-one, 2-tétrahydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué; 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphtyle, 2-naphthyle, 1-anthracyle et 2-anthracyle, tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tetrazolyle, 2-triazolyle, 5-triazolyle, 2-thiényle et 3-thiényle; hétéroaralkyle; hétéroalcényle ou hétéroalkynyle; où ledit aliphatique, carbocyclyle, hétérocyclyle, aryle, aralkyle, hétéroaryle ou hétéroaralkyle est facultativement substitué par halogène, CF₃, -R', -OR', -OH, -SH, -SR', acyloxy, -NO₂, -CN., -NH₂, -NHR', -N(R')₂, -NHCOR', -NHCONH₂, -NHCONHR', -NHCON(R')₂, -NRCOR', -NHCO₂H, - NHCO₂R', -CO₂R', -CO₂H, - COR', -CONH₂, -CONHR' , -CON(R')₂, -S(O)₂H, -S(O)₂R', -SO₂NH₂, -S(O)H, -S(O)R', -SO₂NHR', -SO₂N(R')₂, -NHS(O)₂H ou -NHS(O)₂R'; et où ledit aliphatique, carbocyclyle ou hétérocyclyle peut en outre être facultativement substitué par =O, =S, =NNHR', =NN(R')₂, =N-OR', =NNHCOR', =NNHCO₂R', =NNHSO₂R', =N-CN ou =NR';
R₂ et R₃ sont chacun indépendamment sélectionné parmi H; aliphatique sélectionné parmi linéaire ou branché; alkyle C₁-C₁₂, alcényle C₂₋₁₂ et alkynyle C₂-C₁₂; carbocyclyleC₂-C₁₂; hétérocyclyle sélectionné parmi 3-1H-benzimidazol-2-one, 3-1H-alkyl-benzimidazol-2-one, 2-tétra-hydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphthyle, 2-naphthyle, 1-anthracyle et 2-anthracyle, tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle et 3-thiényle; hétéroaralkyle; hétéroaralcényle ou hétéroaralkynyle; -N(R)₂; -NRCOR; -NRCO₂R; -NRSO₂R; -S(O)ₙR; -SO₂N(R)₂; -SR; -OR; -CF₂; halo; -NO₂, -CN, -C(O)R; -CO₂R; - OC(O)R; -CON(R)₂ ou -OC(O)N(R)₂; où ledit aliphatique, carbocyclyle, hétérocyclyle, aryle, aralkyle, hétéroaryle ou hétéroaralkyle est facultativement substitué par halogène, CF₃, -R', -OR', -OH, -SH, -SR', acyloxy, -NO₂, -CN, -NH₂, -NHR', -N(R')₂, -NHCOR', -NHCONH₂, -NHCONHR', - NHCON(R')z, -NRCOR', -NHCO₂H, -NHCO₂R', -CO₂R', -CO₂H, -COR', -CONH₂, - CONHR' , -CON(R')₂, -S(O)₂H, -S(O)₂R', -SO₂NH₂, -S(O)H, -S(O)R', -SO₂NHR', -SO₂N(R')₂, -NHS(O)₂H ou -NHS(O)₂R'; et où ledit aliphatique, carbocyclyle ou hétérocyclyle peut en outre être facultativement substitute par =0, =S, =NNHR', =NN(R')₂, =N-OR', =NNHCOR', =NNHCO₂R', =NNHSO₂R', =N-CN ou =NR' ; où R₂ et R₃ pris ensemble avec les atomes intervenants forment faculativement un cycle de cinq à neuf membres qui est condensé au cycle de pyridazinyle de la formule I, ledit cycle condensé ayant 0-2 hétéroatomes;
R est sélectionné parmi H; aliphatique sélectionné parmi linéaire ou branché: alkyleC₁₋₁₂, alcényleC₂₋₁₂ et C₂₋C₁₂ alkynyle C₂-C₁₂; carbocyclyle C₃-C₁₂; hétérocyclyle sélectionné parmi 3-1H-benzimidazol-2-one, 3-1H-alkyl-benzimidazol-2-one, 2-tétrahydrofuranyle, 3-tétrahydro-furanyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle, aryle(phényle, 1-naphthyle, 2-naphthyle, 1-anthracyle et 2-anthracyle; aryle sélectionné parmi phényle, 1-naphtyle, 2-naphtyle, 1-anthracyle et 2-anthracyle, tétrahydronaphthyle; aralkyle; aralcényle; aralkynyle; heteroaryle; sélectionné parmi benzimidazolyle, benzothiényle, benzofuranyle, indolyle, quinolinyle, benzoisoxazolyle, benzoxazolyle, benzimidazolyle, isoquinolinyle, isoindolyle, acridinyle benzothiooxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tetrazolyle, ,2-triazolyle, 5-triazolyle, 2-thiényle et 3-thiényle; hétéroaralkyle; hétéroaralcényle; hétéroaralkynyle; où chaque membre de R, excepté H, est facultativement substitué par halogène, CF₃, -R', -OR', -OH, ' -SH, -SR', acyloxy, -NO₂, - CN, -NH₂, -NHR', -N(R')₂, -NHCOR', -NHCONH₂, -NHCONHR', -NHCON (R')₂, -NRCOR' -NHCO₂H, -NHCO₂R' -CO₂R' -CO₂H, -COR', -CONH₂, -CONHR', -CON (R') ₂, -S(O)₂H, -S(O)₂R', -SO₂NH₂, -S (O) H, -S(O)R', -SO₂NHR' , -SO₂N(R')₂, -NHS(O)₂H ou -NHS(O)₂R' ; et où ledit aliphatique, carbocyclyle ou hétérocyclyle peut en outre être facultativement substitué par =O, =S, =NNHR', =NN(R')₂, =N-OR', =NNHCOR', =NNHCO₂R', =NNHSO₂R', =N-CN ou =NR';
R' est indépendamment sélectionné parmi H; aliphatique sélectionné parmi linéaire ou branché; alkyle C₁-C₁₂, alcényle C₂-C₁₂ et alkynyleC₂-C₁₂; carbocyclyle C₃-C₁₂; hétérocyclyle sélectionné parmi 3-1H-benzimidazol-2-one, 3-1H-alkyl-benzimidazol-2-one, 2-tétrahydrofuranyle, 3-tétrahydro-furanyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphthyle, 2-naphthyle, 1-anthracyle et 2-anthracyle, tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-iscxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle et 3-thiényle; hétéroaralkyle; hétéroaralcényle; ou hétéroaralkynyle; et chaque R' est facultativement substitué par halogène, nitro, cyano, amino, -NH-(aliphatique non substitué, -NH-(aliphatique non substitué), -N-(aliphatique non substitué)₂, carboxy, carbamoyle, hydroxy, -O-(aliphatique non substitué), -SH, - S-(aliphatique non substitué), CF₃, -SO₂NH₂, aliphatique non substitué, carbocyclyle non substitué, hétérocyclyle non substitué, aryle non substitué, aralkyle non substitué, hétéroaryle non substitué ou hétéroaralkyle non substitué; et
n est 1 ou 2; ou
(b) une composition pharmaceutique comprenant ledit compose et un support, adjuvant ou véhicule pharmaceutique acceptable;
pour la préparation d'un médicament pour inhiber l'activité de la GSK-3 dans un patient.

15. Composé de la formule I: 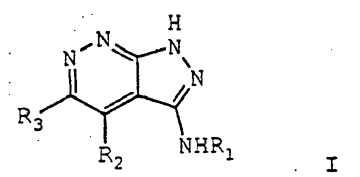 ou sel pharmaceutiquement acceptable de celui-ci, où:
R₁ est sélectionné parmi H; aliphatique sélectionné parmi linéaire ou branché: alkyleC₁-C₁₂, alcényleC₂-C₁₂ et alkynyleC₂-C₁₂; RC(O); RS(O)ₙ; ROC(O) ; carbocyclyleC₃-C₁₂; hétérocyclyle sélectionné parmi 3-1H-benzimidazol-2-one, 3-1H-alkyl-benzimidazol-2-one, 2-tétrahydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué; 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphtyle, 2-naphtyle, 1-anthracyle et 2-anthracyle; tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyl, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle et 3-thiényle; hétéroaralkyle; hétéroaralcényle; ou hétéroaralkynyle; où ledit aliphatique, carbocyclyle, hétérocyclyle, aryle, aralkyle, hétéroaryle ou hétéroaralkyle est facultativement substitué par halogène, CF₃, -R', -OR', -OH, -SH, -SR', acyloxy, -NO₂, -CN, -NH₂, -NHR', -N(R')₂, -NHCOR', -NHCONH₂, -NHCONHR', - NHCON(R')₂, -NRCOR', -NHCO₂H, - NHCO₂R', -CO₂R', -CO₂H, - COR', -CONH₂, -CONHR' , -CON(R')₂, - S(O)₂H, -S(O)₂R', -SO₂NH₂, -S(O)H, -S (O) R', -SO₂NHR', - SO₂N(R')₂, -NHS(O)₂H ou - NHS(O)₂R'; et où ledit aliphatique, carbocyclyle ou hétérocyclyle peut être facultativement substitué par =O, =S, =NNHR', =NN(R')₂, =NNHCOR', =NNHCO₂R', =NNHSO₂R', =N-CN ou =NR';
R₂ et R₃ sont chacun indépendamment sélectionné parmi H; aliphatique sélectionné parmi linéaire ou branché: alkyle C₁-C₁₂, alcényleC₂-C₁₂ et alkynyleC₂-C₁₂; carbocyclyle C₃-C₁₂; hétérocyclyle sélectionné parmi 3-1H-benzimidazol-2-one, 3-1H-alkyl-benzimidazol-2-one, 2-tétrahydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phtalimidinyle, benzoxane, benzotriazol-1-yle, benzopyrrolidine, benzopiperidine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphthyle, 2-naphthyle, 1-anthracyle et 2-anthracyl tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle et 3-thiényle; hétéroaralkyle; hétéroaralcényle ou hétéroaralkynyle; -N(R)₂; .-NRCOR; -NRCO₂R; -NRSO₂R; -S(O)R; -SO₂N(R)₂; -SR; -OR; . -CF₃; halo; -NO₂, -CN; -C(O)R; -CO₂R; -OC(O)R; -CON(R)₂ ou -OC(O)N(R)₂; où ledit aliphatique, carbocyclyle, hétérocyclyle, aryle, aralkyle, hétéroaryle ou hétéroaralkyle est facultativement substitué par halogène, CF₃, -R', -OR', -OH, -SH, -SR', acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR' , -CONH₂, -CONHR', -CON(R')₂, -S(O)₂H, -S(O)₂R', -SO₂NH₂, -S(O)H, -S (O) R', -SO₂NHR' , -SO₂N(R')₂, -NHS(O)₂H ou -NHS(O)₂R'; et où ledit aliphatique carbocyclyle ou hétérocyclyle peut en outre être facultativement substitué par =O, =S, =NNHR', =NN(R')₂, =N-OR', =NNHCOR', =NNHCO₂R', =NNHSO₂R', =N-CN, ou =NR'; ou bien où R₂ et R₃ pris ensemble avec les atomes intervenants forment facultativement un cycle de cinq- à neuf- membres qui est condensé au cycle de pyridazinyle de la formule I, ledit cycle condensé ayant 0-2 hétéroatomes;
R est sélectionné parmi H; aliphatique sélectionné parmi linéaire ou branché: alkyle C₁-C₁₂, alcényleC₂-C₁₂ et alkynyleC₂-C₁₂; carbocyclyleC₃-C₁₂; hétérocyclyle sélectionné parmi 3-1H-benzimidazol-2-one, 3-1H-alkyl-benzimidazol-2-one, 2-tétrahydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-pipérazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle, aryle (phényle, 1-naphthyle, 2-naphthyle, 1-anthracyle et 2-anthracyle; aryle sélectionné parmi phényle, 1-naphthyle, 2-naphthyle, 1-anthracyle et 2-anthracyle, tétrahydronaphthyle; aralkyle; aralcényle; aralkynyle; hétéroaryle; sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tetrazolyle, 2-triazolyle, 5-triazolyle, 2-thiényle et 3-thiényle; hétéroaralkyle; hétéroaralcényle; hétéroaralkynyle; où chaque membre de R, excepté H, est facultativement substitué par halogène, CF₃, -R', -OR', -OH, -SH, -SR', acyloxy, -NO₂, -CN, -NH₂, -NHR', -N(R')₂, -NHCOR', -NHCONH₂, -NHCONHR', -NHCON(R')₂, -NRCOR', -NHCO₂H, -NHCO₂R', -CO₂R', -CO₂H, -COR', -CONH₂, -CONHR', -CON(R')₂, -S(O)₂H, -S(O)₂R', -SO₂NH₂, -S(O)H, -S(O)R', -SO₂NHR', -SO₂N(R')₂, -NHS(O)₂H ou -NHS(O)₂R' ; et où ledit aliphatique, carbocyclyle ou hétérocyclyle peut en outre être facultativement substitué par =O, =S, =NNHR', =NN(R')₂, =N-OR', =NNHCOR', =NNHCO₂R', =NNHSO₂R', =N-CN ou =NR';
R' est indépendamment sélectionné parmi H; aliphatique sélectionné parmi linéaire ou branché: alkylC₂-C₁₂, alcényleC₂-C₁₂ et alkynyleC₂-C₁₂; carbocyclyleC₃-C₁₂; hétérocyclyle sélectionné parmi 3-1H-benzimidazol-2-one, 3-1H- alkyl-benzimidazol-2-one, 2-tétrahydrofuranyle, 3-tétrahydrofuranyle, 2-tétrahydrothiophényle, 3-tétrahydrothiophényle, 2-morpholino, 3-morpholino, 4-morpholino, 2-thiomorpholino, 3-thiomorpholino, 4-thiomorpholino, 1-pyrrolidinyle, 2-pyrrolidinyle, 3-pyrrolidinyle, 1-piperazinyle, 2-pipérazinyle, 1-pipéridinyle, 2-pipéridinyle, 3-pipéridinyle, 4-pipéridinyle, 4-thiazolidinyle, diazolonyle, diazolonyle N-substitué, 1-phthalimidinyle, benzoxane, benzotriazol-1-yle, benzopyrrolidine, benzopipéridine, benzoxolane, benzothiolane, benzothiane, aziranyle, oxiranyle, azétidinyle, pyrrolinyle, dioxolanyle, imidazolinyle, imidazolidinyle, pyrazolinyle, pyrazolidinyle, pyranyle, dioxanyle, dithianyle, trithianyle, quinuclidinyle, oxépanyle et thiépanyle; aryle sélectionné parmi phényle, 1-naphthyle, 2-naphthyle, 1-anthracyle et 2-anthracyle, tétrahydronaphthyle; aralkyle; aralcényle; aralkynyle; hétéroaryle sélectionné parmi benzimidazolyle, benzothiényle, benzofuranyle, indolyle, quinolinyle, benzothiazolyle, benzoxazolyle, benzimidazolyle, isoquinolinyle, isoindolyle, acridinyle, benzoisoxazolyle, 2-furanyle, 3-furanyle, N-imidazolyle, 2-imidazolyle, 4-imidazolyle, 5-imidazolyle, 3-isoxazolyle, 4-isoxazolyle, 5-isoxazolyle, 2-oxadiazolyle, 5-oxadiazolyle, 2-oxazolyle, 4-oxazolyle, 5-oxazolyle, 2-pyrrolyle, 3-pyrrolyle, 2-pyridyle, 3-pyridyle, 4-pyridyle, 2-pyrimidyle, 4-pyrimidyle, 5-pyrimidyle, 3-pyridazinyle, 2-thiazolyle, 4-thiazolyle, 5-thiazolyle, 5-tétrazolyle, 2-triazolyle, 5-triazolyle, 2-thiényle et 3-thiényle; hétéroaralkyle; hétéroaralcényle ou hétéroaralkynyle; et chaque R' est facultativement substitué par halogène, nitro, cyano, amino, -NH-(aliphatique non substitué), -N-(aliphatique non substitué)₂, carboxy, carbamoyle, hydroxy, -0-(aliphatique non substitué), -SH, -S-(aliphatique non substitué), CF₃, -SO₂NH₂, aliphatique non substitué, carbocyclyle non substitué, hétérocyclyle non substitué, aryle non substitué, aralkyle non substitué, hétéroaryle non substitué ou hétéroaralkyle non substitué; et
n est 1 ou 2; ou
ou une composition pharmaceutique comprenant ledit composé et un support, adjuvant ou véhicule pharmaceutiquement acceptable;
pour utilisation dans l'inhibition de l'activité de la GSK-3 dans un patient.

16. Utilisation selon la revendication 14 pour stimuler la synthèse du glycogène ou pour diminuer les niveaux sanguins du glucose dans un patient qui en a besoin.

17. Utilisation selon la revendication 16, où le patient est traité pour le diabète.

18. Utilisation selon la revendication 14, pour l'inhibition de la production de la protéine Tau hyperphosphorylée dans un patient qui en a besoin.

19. Utilisation selon la revendication 18, où le patient est traité pour la maladie d'Alzheimer.

20. Utilisation selon la revendication 14 pour l'inhibition de la phosphorylation de la β-caténine dans un patient qui en a besoin.

21. Utilisation selon la revendication 20, où le patient est traité pour la schizophrénie.

22. Utilisation selon l'une quelconque des revendications 14, 16, 18 et 20, où ledit composé ou composition est administré avec un agent thérapeutique additionnel.

23. Composé ou composition selon la revendication 15, pour utilisation dans la stimulation de la synthèse du glycogène ou pour la diminution des niveaux sanguins du glucose dans un patient qui en a besoin.

24. Composé ou composition selon la revendication 23, où le patient est traité pour le diabète.

25. Composé ou composition selon la revendication 15 pour utilisation dans l'inhibition de la production de la protéine Tau hyperphosphorylée dans un patient qui en a besoin.

26. Composé ou composition selon la revendication 25, où le patient est traité pour la maladie d'Alzheimer.

27. Composé ou composition selon la revendication 15 pour utilisation dans l'inhibition de la phosphorylation de la p-cathénine dans un patient qui en a besoin.

28. Composé ou composition selon la revendication 27, où le patient est traité pour la schizophrénie.

29. Composé ou composition selon l'une quelconque des revendications 15, 23, 25 et 27, où ledit composé ou composition est administré avec un agent thérapeutique additionnel.

30. Méthode in vitro pour inhiber l'activité de la GSK-3 dans un échantillon biologique, comprenant l'étape consistant à mettre en contact l'échantillon biologique avec un composé tel que défini dans la revendication 14 ou une composition comprenant ledit composé et un support, adjuvant ou véhicule.


### SEQUENCE LISTING

<110> VERTEX PHARMACEUTICALS, INC.
   TER HAAR, ERNST
   SWENSON, LOVORKA
   GREEN, JEREMY
   ARNOST, MICHAEL J.
<120> INHIBITORS OF GSK-3 AND CRYSTAL STRUCTURES OF GSK-3B
   PROTEIN AND PROTEIN COMPLEXES
<130> VPI-01-02 PCT
<140>
   <141>
<150> 60/361,899
   <151> 2002-02-27
<150> 60/297,094
   <151> 2001-06-08
<150> 60/287,366
   <151> 2001-04-30
<160> 1
<170> PatentIn Ver. 2.1
<210> 1
   <211> 420
   <212> PRT
   <213> Homo sapiens
<400> 1
   Met Ser Gly Arg Pro Arg Thr Thr Ser Phe Ala Glu Ser Cys Lys Pro
   1               5                   10                  15
   Val Gln Gln Pro Ser Ala Phe Gly Ser Met Lys Val Ser Arg Asp Lys
               20                  25                  30
   Asp Gly Ser Lys Val Thr Thr Val Val Ala Thr Pro Gly Gln Gly Pro
           35                  40                  45
   Asp Arg Pro Gln Glu Val Ser Tyr Thr Asp Thr Lys Val Ile Gly Asn
       50                  55                  60
   Gly Ser Phe Gly Val Val Tyr Gln Ala Lys Leu Cys Asp Ser Gly Glu
   65                  70                  75                  80
   Leu Val Ala Ile Lys Lys Val Leu Gln Asp Lys Arg Phe Lys Asn Arg
                   85                  90                  95
   Glu Leu Gln Ile Met Arg Lys Leu Asp His Cys Asn Ile Val Arg Leu
               100                 105                 110
   Arg Tyr Phe Phe Tyr Ser Ser Gly Glu Lys Lys Asp Glu Val Tyr Leu
           115                 120                 125
   Asn Leu Val Leu Asp Tyr Val Pro Glu Thr Val Tyr Arg Val Ala Arg
       130                 135                 140
   His Tyr Ser Arg Ala Lys Gln Thr Leu Pro Val Ile Tyr Val Lys Leu
   145                 150                 155                 160
   Tyr Met Tyr Gln Leu Phe Arg Ser Leu Ala Tyr Ile His Ser Phe Gly
                   165                 170                 175
   Ile Cys His Arg Asp Ile Lys Pro Gln Asn Leu Leu Leu Asp Pro Asp
               180                 185                 190
   Thr Ala Val Leu Lys Leu Cys Asp Phe Gly Ser Ala Lys Gln Leu Val
           195                 200                 205
   Arg Gly Glu Pro Asn Val Ser Tyr Ile Cys Ser Arg Tyr Tyr Arg Ala
       210                 215                 220
   Pro Glu Leu Ile Phe Gly Ala Thr Asp Tyr Thr Ser Ser Ile Asp Val
   225                 230                 235                 240
   Trp Ser Ala Gly Cys Val Leu Ala Glu Leu Leu Leu Gly Gln Pro Ile
                   245                 250                 255
   Phe Pro Gly Asp Ser Gly Val Asp Gln Leu Val Glu Ile Ile Lys Val
               260                 265                 270
   Leu Gly Thr Pro Thr Arg Glu Gln Ile Arg Glu Met Asn Pro Asn Tyr
           275                 280                 285
   Thr Glu Phe Lys Phe Pro Gln Ile Lys Ala His Pro Trp Thr Lys Val
       290                 295                 300
   Phe Arg Pro Arg Thr Pro Pro Glu Ala Ile Ala Leu Cys Ser Arg Leu
   305                 310                 315                 320
   Leu Glu Tyr Thr Pro Thr Ala Arg Leu Thr Pro Leu Glu Ala Cys Ala
                   325                 330                 335
   His Ser Phe Phe Asp Glu Leu Arg Asp Pro Asn Val Lys Leu Pro Asn
               340                 345                 350
   Gly Arg Asp Thr Pro Ala Leu Phe Asn Phe Thr Thr Gln Glu Leu Ser
           355                 360                 365
   Ser Asn Pro Pro Leu Ala Thr Ile Leu Ile Pro Pro His Ala Arg Ile
       370                 375                 380
   Gln Ala Ala Ala Ser Thr Pro Thr Asn Ala Thr Ala Ala Ser Asp Ala
   385                 390                 395                 400
   Asn Thr Gly Asp Arg Gly Gln Thr Asn Asn Ala Ala Ser Ala Ser Ala
                   405                 410                 415
   Ser Asn Ser Thr
               420